# EUROPEAN PATENT SPECIFICATION

(11) **EP 1 558 607 B1**
(45) Date of publication and mention of the grant of the patent: **05.05.2010**
(21) Application number: 03781448.0
(22) Date of filing: 30.10.2003
(51) Int. Cl.: C07D 409/04, C07D 417/04, C07D 417/14, C07D 409/14

(54) **COMPOSITIONS USEFUL AS INHIBITORS OF ROCK AND OTHER PROTEIN KINASES**
ZUSAMMENSETZUNGEN VERWENDBAR FÜR DIE HEMMUNG VON ROCK UND ANDEREN KINASEN
COMPOSITIONS UTILES EN TANT QU'INHIBITEURS DE ROCK ET D'AUTRES PROTEINES KINASES

(30) Priority: 30.10.2002 US 422441 P; 06.06.2003 US 476433 P; 06.06.2003 US 476691 P; 19.06.2003 US 479903 P
(43) Date of publication of application: 03.08.2005
(73) Proprietor: VERTEX PHARMACEUTICALS INCORPORATED, Cambridge, MA 02139-4242 (US)
(72) Inventor: CAO, Jingrong, Newton, MA 02165 (US); GAO, Huai, Lincoln Massachusetts 01773 (US); GREEN, Jeremy, Burlington, MA 01803 (US); MARHEFKA, Craig, Belmont, MA 02478 (US)
(74) Representative: Hedley, Nicholas James Matthew
(86) International application number: PCT/US2003/034319
(87) International publication number: WO 2004/041813

(56) References cited:
- EP-A- 1 205 478
- EP-A- 1 256 578
- WO-A-00/26202
- WO-A-00/26203
- WO-A-01/17995
- WO-A-02/14311
- WO-A-02/96905

## Description

### TECHNICAL FIELD OF INVENTION

The present invention relates to compounds useful as inhibitors of protein kinases. The invention also provides pharmaceutically acceptable compositions comprising the compounds of the invention and methods of using the compositions in the treatment of various disorders.

### BACKGROUND OF THE INVENTION

The search for new therapeutic agents has been greatly aided in recent years by a better understanding of the structure of enzymes and other biomolecules associated with diseases. One important class of enzymes that has been the subject of extensive study is protein kinases.

Protein kinases constitute a large family of structurally related enzymes that are responsible for the control of a variety of signal transduction processes within the cell. (See, Hardie, G. and Hanks, S. The Protein Kinase Facts Book, I and II, Academic Press, San Diego, CA: 1995). Protein kinases are thought to have evolved from a common ancestral gene due to the conservation of their structure and catalytic function. Almost all kinases contain a similar 250-300 amino acid catalytic domain. The kinases may be categorized into families by the substrates they phosphorylate (e.g., protein-tyrosine, protein-serine/threonine, lipids, etc.). Sequence motifs have been identified that generally correspond to each of these kinase families (See, for example, Hanks, S.K., Hunter, T., FASEB J. 1995, 9, 576-596; Knighton et al., Science 1991, 253, 407-414; Hiles et al., Cell 1992, 70, 419-429; Kunz et al., Cell 1993, 73, 585-596; Garcia-Bustos et al., EMBO J. 1994, 13, 2352-2361).

Many diseases are associated with abnormal cellular responses triggered by protein kinase-mediated events. These diseases include autoimmune diseases, inflammatory diseases, bone diseases, metabolic diseases, neurological and neurodegenerative diseases, cancer, cardiovascular diseases, allergies and asthma, Alzheimer's disease and hormone-related diseases. Accordingly, there has been a substantial effort in medicinal chemistry to find protein kinase inhibitors that are effective as therapeutic agents.

One kinase family of interest is Rho-associated coiled-coil forming protein serine/threonine kinase (ROCK), which is believed to be an effector of Ras-related small GTPase Rho. The ROCK family includes p160ROCK (ROCK-1) (Ishizaki et al., EMBO J. 1996, 15, 1885-1893) and ROKα/Rho-kinase/ROCK-II (Leung et al., J. Biol. Chem. 1995, 270, 29051-29054; Matsui et al., EMBO J. 1996, 15, 2208-2216; Nakagawa et al., FEBS Lett. 1996, 392, 189-193), protein kinase PKN (Amano et al., Science 1996, 271, 648-650; Watanabe et al., Science 1996, 271, 645-648), and citron and citron kinase (Madaule et al. Nature, 1998, 394, 491-494; Madaule et al., FEBS Lett. 1995, 377, 243-248). The ROCK family of kinases have been shown to be involved in a variety of functions including Rho-induced formation of actin stress fibers and focal adhesions (Leung et al., Mol. Cell Biol. 1996, 16, 5313-5327; Amano et al., Science, 1997, 275, 1308-1311; Ishizaki et al., FEBS Lett. 1997, 404, 118-124) and in downregulation of myosin phosphatase (Kimura et al., Science, 1996, 273, 245-248), platelet activation (Klages et al., J. Cell. Biol., 1999, 144, 745-754), aortic smooth muscle contraction by various stimuli (Fu et al., FEBS Lett., 1998, 440, 183-187), thrombin-induced responses of aortic smooth muscle cells (Seasholtz et al., Cir. Res., 1999, 84, 1186-1193), hypertrophy of cardiomyocytes (Kuwahara et al., FEBS Lett., 1999, 452, 314-318), bronchial smooth muscle contraction (Yoshii et al., Am. J. Respir. Cell Mol. Biol., 1999, 20, 1190-1200), smooth muscle contraction and cytoskeletal reorganization of non-muscle cells (Fukata et al., Trends in Pharm. Sci 2001, 22, 32-39), activation of volume-regulated anion channels (Nilius et al., J. Physiol., 1999, 516, 67-74), neurite retraction (Hirose et al., J. Cell. Biol., 1998, 141, 1625-1636), neutrophil chemotaxis (Niggli, FEBS Lett., 1999, 445, 69-72), wound healing (Nobes and Hall, J. Cell. Biol., 1999, 144, 1235-1244), tumor invasion (Itoh et al., Nat. Med., 1999, 5, 221-225) and cell transformation (Sahai et al., Curr. Biol., 1999, 9, 136-145). More specifically, ROCK has been implicated in various diseases and disorders including hypertension (Satoh et al., J. Clin. Invest. 1994, 94, 1397-1403; Mukai et al., FASEB J. 2001, 15, 1062-1064; Uehata et al., Nature 1997, 389, 990-994; Masumoto et al., Hypertension, 2001, 38, 1307-1310), cerebral vasospasm (Sato et al., Circ. Res. 2000, 87, 195-200; Miyagi et al., J. Neurosurg. 2000, 93, 471-476; Tachibana et al., Acta Neurochir (Wien) 1999, 141, 13-19), coronary vasospasm (Shimokawa et al., Jpn. Cir. J. 2000, 64, 1-12; Kandabashi et al., Circulation 2000, 101, 1319-1323; Katsumata et al., Circulation 1997, 96, 4357-4363; Shimokawa et al., Cardiovasc. Res. 2001, 51, 169-177; Utsunomiya et al., J. Pharmacol. 2001, 134, 1724-1730; Masumoto et al., Circulation 2002, 105, 1545-1547), bronchial asthma (Chiba et al., Comp. Biochem. Physiol. C Pharmacol. Toxicol. Endocrinol. 1995, 11, 351-357; Chiba et al., Br. J. Pharmacol. 1999, 127, 597-600; Chiba et al., Br. J. Pharmacol. 2001, 133, 886-890; Iizuka et al., Eur. J. Pharmacol. 2000, 406, 273-279), preterm labor (Niro et al., Biochem. Biophys. Res. Commun. 1997, 230, 356-359; Tahara et al., Endocrinology 2002, 143, 920-929; Kupittayanant et al., Pflugers Arch. 2001, 443, 112-114), erectile dysfunction (Chitaley et al., Nat. Med. 2001, 7, 119-122; Mills et al., J. Appl. Physiol. 2001, 91, 1269-1273), glaucoma (Honjo et al., Arch. Ophthalmol. 2001, 1171-1178; Rao et al., Invest. Ophthalmol. Vis. Sci. 2001, 42, 1029-1037), vascular smooth muscle cell proliferation (Shimokawa et al., Cardiovasc. Res. 2001, 51, 169-177; Morishige et al., Arterioscler. Thromb. Vasc. Biol. 2001, 21, 548-554; Eto et al., Am. J. Physiol. Heart Circ. Physiol. 2000, 278, H1744-H1750; Sawada et al., Circulation 2000, 101, 2030-2023; Shibata et al., Circulation 2001, 103, 284-289), myocardial hypertrophy (Hoshijima et al., J. Biol. Chem. 1998, 273, 7725-77230; Sah et al., J. Biol. Chem. 1996, 271, 31185-31190; Kuwahara et al., FEBS Lett. 1999, 452, 314-318; Yanazume et al., J. Biol. Chem. 2002, 277, 8618-8625), malignoma (Itoh et al., Nat. Med. 1999, 5, 221-225; Genda et al., Hepatology 1999, 30, 1027-1036; Somlyo et al., Biochem. Biophys. Res. Commun. 2000, 269, 652-659), ischemia/reperfusion-induced injury (Ikeda et al., J. of Surgical Res. 2003, 109, 155-160; Miznuma et al. Transplantation 2003, 75, 579-586), endothelial dysfunction (Hernandez-Perera et al., Circ. Res. 2000, 87, 616-622; Laufs et al., J. Biol. Chem. 1998, 273, 24266-24271; Eto et al., Circ. Res. 2001, 89, 583-590), Crohn's Disease and colitis (Segain et al. Gastroenterology 2003, 124(5), 1180-1187), neurite outgrowth (Fournier et al. J. Neurosci. 2003, 23, 1416-1423), Raynaud's Disease (Shimokawa et al. J. Cardiovasc. Pharmacol. 2002, 39, 319-327), and atherosclerosis (Retzer et al. FEBS Lett. 2000, 466, 70-74; Ishibashi et al. Biochim. Biophys. Acta 2002, 1590, 123-130). Accordingly, the development of inhibitors of ROCK kinase would be useful as therapeutic agents for the treatment of disorders implicated in the ROCK kinase pathway.

ERK2 (extracellular signal regulated kinase) is a member of the mammalian mitogen-activated protein (MAP)1 kinase family. (MAP)1 kinases are serine/threonine kinases that mediate intracellular signal transduction pathways (Cobb and Goldsmith, J Biol. Chem., 1995, 270, 14843; Davis, Mol. Reprod. Dev. 1995, 42, 459) and are activated by mitogens and growth factors (Bokemeyer et al.. Kidney Int. 1996, 49, 1187). Members of the MAP kinase family share sequence similarity and conserved structural domains, and, in addition to ERK2, include the JNK (Jun N-terminal kinase), and p38 kinases. JNKs and p38 kinases are activated in response to the pro-inflammatory cytokines TNF-alpha and interleukin-1, and by cellular stress such as heat shock, hyperosmolarity, ultraviolet radiation, lipopolysaccharides and inhibitors of protein synthesis (Derijard et al., Cell 1994, 76, 1025; Han et al., Science 1994, 265, 808; Raingeaud et al., J Biol. Chem. 1995, 270, 7420; Shapiro and Dinarello, Proc. Natl. Acad. Sci. USA 1995, 92, 12230). In contrast, ERKs are activated by mitogens and growth factors (Bokemeyer et al., Kidney Int. 1996, 49, 1187).

ERK2 is a widely distributed protein kinase that achieves maximum activity when both Thr183 and Tyr185 are phosphorylated by the upstream MAP kinase kinase, MEK1 (Anderson et al., Nature 1990, 343, 651; Crews et al., Science 1992, 258, 478). Upon activation, ERK2 phosphorylates many regulatory proteins, including the protein kinases Rsk90 (Bjorbaek et al., J. Biol. Chem. 1995, 270, 18848) and MAPKAP2 (Rouse et al., Cell 1994, 78, 1027), and transcription factors such as ATF2 (Raingeaud et al., Mol. Cell Biol. 1996, 16, 1247), Elk-1 (Raingeaud et al., Mol. Cell Biol. 1996, 16, 1247), c-Fos (Chen et al., Proc. Natl. Acad. Sci. USA 1993, 90, 10952), and c-Myc (Oliver et al., Proc. Soc. Exp. Biol. Med. 1995, 210, 162). ERK2 is also a downstream target of the Ras/Raf dependent pathways (Moodie et al., Science 1993, 260, 1658) and may help relay the signals from these potentially oncogenic proteins. ERK2 has been shown to play a role in the negative growth control of breast cancer cells (Frey and Mulder, Cancer Res. 1993, 57, 628) and hyperexpression of ERK2 in human breast cancer has been reported (Sivaraman et al., J Clin. Invest. 1997, 99, 1478). Activated ERK2 has also been implicated in the proliferation of endothelin-stimulated airway smooth muscle cells, suggesting a role for this kinase in asthma (Whelchel et al., Am. J. Respir. Cell Mol. Biol. 1997, 16, 589).

Glycogen synthase kinase-3 (GSK-3) is a serine/threonine protein kinase comprised of α and β isoforms that are each encoded by distinct genes [Coghlan et al., Chemistry & Biology 2000, 7, 793-803; and Kim and Kimmel, Curr. Opinion Genetics Dev., 2000 10, 508-514]. GSK-3 has been implicated in various diseases including diabetes, Alzheimer's disease, CNS disorders such as manic depressive disorder and neurodegenerative diseases, and cardiomyocyte hypertrophy [PCT Application Nos.: WO 99/65897 and WO 00/38675; and Haq et al., J. Cell Biol. 2000, 151, 117-130]. These diseases are associated with the abnormal operation of certain cell signaling pathways in which GSK-3 plays a role. GSK-3 has been found to phosphorylate and modulate the activity of a number of regulatory proteins. These proteins include glycogen synthase, which is the rate limiting enzyme necessary for glycogen synthesis, the microtubule associated protein Tau, the gene transcription factor β-catenin, the translation initiation factor e1F2B, as well as ATP citrate lyase, axin, heat shock factor-1, c-*Jun*, *c-myc*, c-*myb*, CREB, and CEPBα. These diverse protein targets implicate GSK-3 in many aspects of cellular metabolism, proliferation, differentiation, and development.

In a GSK-3 mediated pathway that is relevant for the treatment of type II diabetes, insulin-induced signaling leads to cellular glucose uptake and glycogen synthesis. Along this pathway, GSK-3 is a negative regulator of the insulin-induced signal. Normally, the presence of insulin causes inhibition of GSK-3 mediated phosphorylation and deactivation of glycogen synthase. The inhibition of GSK-3 leads to increased glycogen synthesis and glucose uptake [Klein et al., PNAS 1996, 93, 8455-8459; Cross et al., Biochem. J. 1994, 303, 21-26); Cohen, Biochem. Soc. Trans. 1993, 21, 555-567; and Massillon et al., Biochem J. 1994, 299, 123-128]. However, in a diabetic patient, where the insulin response is impaired, glycogen synthesis and glucose uptake fail to increase despite the presence of relatively high blood levels of insulin. This leads to abnormally high blood levels of glucose with acute and long-term effects that may ultimately result in cardiovascular disease, renal failure and blindness. In such patients, the normal insulin-induced inhibition of GSK-3 fails to occur. It has also been reported that in patients with type II diabetes, GSK-3 is overexpressed [see, PCT Application: WO 00/38675]. Therapeutic inhibitors of GSK-3 are therefore potentially useful for treating diabetic patients suffering from an impaired response to insulin.

GSK-3 activity is also associated with Alzheimer's disease. This disease is characterized by the well-known β-amyloid peptide and the formation of intracellular neurofibrillary tangles. Aβ peptides are derived from the amyloid precursor protein (APP) by sequential proteolysis, catalysed by the aspartyl protease BACE2, followed by presenilin-dependent γ-secretase cleavage. It has been demonstrated that antibodies against β-amyloid plaques can slow cognitive decline in patients with Alzheimer's disease (Hock et al., Neuron, 2003, 38, 547-554), and thus other β-amyloid-lowering strategies (e.g., the development of agents capable of inhibiting β-amyloid peptide) would be useful in the treatment of Alzherimer's disease and other psychotic and neurodegenerative disorders. Additionally, the neurofibrillary tangles contain hyperphosphorylated Tau protein, in which Tau is phosphorylated on abnormal sites, and thus agents capble of inhibiting the hyperphosphorylation of Tau protein would be useful in the treatment of Alzherimer's disease and other psychotic and neurodegenerative disorders.

GSK-3 is known to phosphorylate these abnormal sites in cell and animal models. Furthermore, inhibition of GSK-3 has been shown to prevent hyperphosphorylation of Tau in cells [Lovestone et al., Current Biology 1994, 4, 1077-86; and Brownlees et al., Neuroreport 1997, 8, 3251-55]. Therefore, GSK-3 activity promotes generation of the neurofibrillary tangles and the progression of Alzheimer's disease. It has also been shown that GSK-3 facilitates APP processing and that a GSK-3 inhibitor (lithium) inhibits of the generation of Aβ peptides through the inhibition of GSK-3 (Phiel et al. Nature 2003, 423, 435-439). Thus, the development of inhibitors of GSK-3 would be useful for the reduction of the formation of amyloid plaques and neurofibrillry tangles, the pathological hallmarks of Alzheimer's Disease, and would also be useful for the treament of other psychotic and neurodegenerative disorders.

Another substrate of GSK-3 is β-catenin, which is degradated after phosphorylation by GSK-3. Reduced levels of β-catenin have been reported in schizophrenic patients and have also been associated with other diseases related to increase in neuronal cell death [Zhong et al., Nature 1998, 395, 698-702; Takashima et al., PNAS 1993, 90, 7789-93; and Pei et al., J. Neuropathol. Exp 1997, 56, 70-78].

GSK-3 activity is also associated with stroke [Wang et al., Brain Res 2000, 859, 381-5; Sasaki et al., Neurol Res 2001, 23, 588-92; Hashimoto et al., J. Biol. Chem 2002, 277, 32985-32991].

The AGC sub-family of kinases phosphorylate their substrates at serine and threonine residues and participate in a variety of well-known signaling processes, including, but not limited to cyclic AMP signaling, the response to insulin, apoptosis protection, diacylglycerol signaling, and control of protein translation (Peterson et al., Curr. Biol. 1999, 9, R521). This sub-family includes PKA, PKB (c-Akt), PKC, PRK1, 2, p70^{S6K}, and PDK.

AKT (also known as PKB or Rac-PK beta), a serine/threonine protein kinase, has been shown to be overexpressed in several types of cancer and is a mediator of normal cell functions [(Khwaja, A., Nature 1999, 401, 33-34); (Yuan, Z.Q., et al., Oncogene 2000, 19, 2324-2330); (Namikawa, K., et al., J Neurosci. 2000, 20, 2875-2886,)]. AKT comprises an N-terminal pleckstrin homology (PH) domain, a kinase domain and a C-terminal "tail" region. Three isoforms of human AKT kinase (AKT-1, -2 and -3) have been reported so far [(Cheng, J.Q., Proc. Nail. Acad. Sci. USA 1992, 89, 9267-9271); (Brodbeck, D. et al., J. Biol. Chem. 1999, 274, 9133-9136)]. The PH domain binds 3-phosphoinositides, which are synthesized by phosphatidyl inositol 3-kinase (PI3K) upon stimulation by growth factors such as platelet derived growth factor (PDGF), nerve growth factor (NGF) and insulin-like growth factor (IGF-1) [(Kulik et al., Mol. Cell. Biol., 1997, 17, 1595-1606,); (Hemmings, B.A., Science, 1997, 275, 628-630)]. Lipid binding to the PH domain promotes translocation of AKT to the plasma membrane and facilitates phosphorylation by another PH-domain-containing protein kinases, PDK1 at Thr308, Thr309, and Thr305 for the AKT isoforms 1, 2 and 3, respectively. A second, as of yet unknown, kinase is required for the phosphorylation of Ser473, Ser474 or Ser472 in the C-terminal tails of AKT-1, -2 and -3 respectively, in order to yield a fully activated AKT enzyme.

Once localized to the membrane, AKT mediates several functions within the cell including the metabolic effects of insulin (Calera, M.R. et al., J. Biol. Chem. 1998, 273, 7201-7204) induction of differentiation and/or proliferation, protein synthesis and stress responses (Alessi, D.R. et al., Curr. Opin. Genet. Dev. 1998, 8, 55-62,).

Manifestations of altered AKT regulation appear in both injury and disease, the most important role being in cancer. The first account of AKT was in association with human ovarian carcinomas where expression of AKT was found to be amplified in 15% of cases (Cheng, J.Q. et al., Proc. Natl. Acad Sci. U.S.A. 1992, 89, 9267-9271). It has also been found to be overexpressed in 12% of pancreatic cancers (Cheng, J. Q. et al., Proc. Natl. Acad. Sci. U.S.A. 1996, 93, 3636-3641). It was demonstrated that AKT-2 was overexpressed in 12% of ovarian carcinomas and that amplification of AKT was especially frequent in 50% of undifferentiated tumours, suggesting that AKT may also be associated with tumour aggressiveness (Bellacosa, et al., Int. J. Cancer 1995, 64, 280-285).

PKA (also known as cAMP-dependent protein kinase) has been shown to regulate many vital functions including energy metabolism, gene transcription, proliferation, differentiation, reproductive function, secretion, neuronal activity, memory, contractility and motility (Beebe, S.J., Semin. Cancer Biol. 1994, 5, 285-294). PKA is a tetrameric holoenzyme, which contains two catalytic subunits bound to a homo-dimeric regulatory subunit (which acts to inhibit the catalytic sub-units). On binding of cAMP (enzyme activation), the catalytic subunits dissociate from the regulatory subunits to yield the active serine/threonine kinase (McKnight, G.S. et al., Recent Prog. Horm. Res. 1988, 44, pp. 307). Three isoforms of the catalytic subunit (C-α, C-β and C-γ) have been reported to date (Beebe, S.J. et al., J. Biol. Chem. 1992, 267, 25505-25512) with the C-α subunit being the most extensively studied, primarily because of its elevated expression in primary and metastatic melanomas (Becker, D. et al., Oncogene 1990, 5, 1133). To date, strategies to modulate the activity of the C-α subunit involve the use of antibodies, molecules that block PKA activity by targeting regulatory dimers and antisense oligonucleotides expression.

The ribosomal protein kinases p70^{S6K}-1 and -2 are also members of the AGC subfamily of protein kinases and catalyze the phosphorylation and subsequent activation of the ribosomal protein S6, which has been implicated in the translational up-regulation of mRNAs coding for the components of the protein synthetic apparatus. These mRNAs contain an oligopyrimidine tract at their 5' transcriptional start site, termed a 5TOP, which has been shown to be essential for their regulation at the translational level (Volarevic, S. et al., Prog. Nucleic Acid Res. Mol. Biol. 2001, 65, 101-186). p70^{S6K} dependent S6 phosphorylation is stimulated in response to a variety of hormones and growth factors primarily via the PI3K pathway (Coffer, P.J. et al., Biochem. Biophys. Res. Commun, 1994 198, 780-786), which may be under the regulation of mTOR, since rapamycin acts to inhibit p70^{S6K} activity and blocks protein synthesis, specifically as a result of a down-regulation of translation of these mRNA's encoding ribosomal proteins (Kuo, C.J. et al., Nature 1992, 358, 70-73).

*In vitro* PDK1 catalyses the phosphorylation of Thr252 in the activation loop of the p70 catalytic domain, which is indispensable for p70 activity (Alessi, D.R., Curr. Biol., 1998, 8, 69-81). The use of rapamycin and gene deletion studies of dp70S6K from Drosophila and p70^{S6K}1 from mouse have established the central role p70 plays in both cell growth and proliferation signaling.

The 3-phosphoinositide-dependent protein kinase-1 (PDK1) plays a key role in regulating the activity of a number of kinases belonging to the AGC subfamily of protein kinases (Alessi, D. et al., Biochem. Soc. Trans 2001, 29, 1). These include isoforms of protein kinase B (PKB, also known as AKT), p70 ribosomal S6 kinase (S6K) (Avruch, J. et al., Prog. Mol. Subcell. Biol. 2001, 26, 115), and p90 ribosomal S6 kinase (Frödin, M. et al., EMBO J. 2000, 19, 2924-2934). PDK1 mediated signaling is activated in response to insulin and growth factors and as a consequence of attachment of the cell to the extracellular matrix (integrin signaling). Once activated these enzymes mediate many diverse cellular events by phosphorylating key regulatory proteins that play important roles controlling processes such as cell survival, growth, proliferation and glucose regulation [(Lawlor, M.A. et al., J. Cell Sci. 2001, 114, 2903-2910), (Lawlor, M.A. et al., EMBO J. 2002, 21, 3728-3738)]. PDK1 is a 556 amino acid protein, with an N-terminal catalytic domain and a C-terminal pleckstrin homology (PH) domain, which activates its substrates by phosphorylating these kinases at their activation loop (Belham, C. et al., Curr. Biol. 1999, 9, R93-R96). Many human cancers including prostate and NSCL have elevated PDK1 signaling pathway function resulting from a number of distinct genetic events such as PTEN mutations or over-expression of certain key regulatory proteins [(Graff, J.R., Expert Opin. Ther. Targets 2002, 6, 103-113), (Brognard, J., et al., Cancer Res. 2001, 61, 3986-3997)]. Inhibition of PDK1 as a potential mechanism to treat cancer was demonstrated by transfection of a PTEN negative human cancer cell line (U87MG) with antisense oligonucleotides directed against PDK1. The resulting decrease in PDK1 protein levels led to a reduction in cellular proliferation and survival (Flynn, P., et al., Curr. Biol. 2000, 10, 1439-1442). Consequently the design of ATP binding site inhibitors of PDK1 offers, amongst other treatments, an attractive target for cancer chemotherapy.

The diverse range of cancer cell genotypes has been attributed to the manifestation of the following six essential alterations in cell physiology: self-sufficiency in growth signaling, evasion of apoptosis, insensitivity to growth-inhibitory signaling, limitless replicative potential, sustained angiogenesis, and tissue invasion leading to metastasis (Hanahan, D. et al., Cell 2000, 100, 57-70). PDK1 is a critical mediator of the PI3K signalling pathway, which regulates a multitude of cellular function including growth, proliferation and survival. Consequently, inhibition of this pathway could affect four or more of the six defining requirements for cancer progression. As such it is anticipated that a PDK1 inhibitor will have an effect on the growth of a very wide range of human cancers.

Specifically, increased levels of PI3K pathway activity has been directly associated with the development of a number of human cancers, progression to an aggressive refractory state (acquired resistance to chemotherapies) and poor prognosis. This increased activity has been attributed to a series of key events including decreased activity of negative pathway regulators such as the phosphatase PTEN, activating mutations of positive pathway regulators such as Ras, and overexpression of components of the pathway itself such as PKB, examples include: brain (gliomas), breast, colon, head and neck, kidney, lung, liver, melanoma, ovarian, pancreatic, prostate, sarcoma, thyroid [(Teng, D.H. et al., Cancer Res., 1997 57, 5221-5225), (Brognard, J. et al., Cancer Res., 2001, 61, 3986-3997), (Cheng, J.Q. et al., Proc. Natl. Acad. Sci. 1996, 93, 3636-3641*),* (Int. J. Cancer 1995, 64, 280), (Graff, J.R., Expert Opin. Ther. Targets 2002, 6, 103-113), (Am. J. Pathol. 2001, 159, 431)].

Additionally, decreased pathway function through gene knockout, gene knockdown, dominant negative studies, and small molecule inhibitors of the pathway have been demonstrated to reverse many of the cancer phenotypes *in vitro* (some studies have also demonstrated a similar effect *in vivo*) such as block proliferation, reduce viability and sensitize cancer cells to known chemotherapies in a series of cell lines, representing the following cancers: pancreatic [(Cheng, J.Q. et al., Proc. Natl. Acad. Sci. 1996, 93, 3636-3641), (Neoplasia 2001, 3, 278)], lung [(Brognard, J. et al., Cancer Res. 2001, 61, 3986-3997), (Neoplasia 2001, 3, 278)], ovarian [(Hayakawa, J. et al., Cancer Res. 2000, 60, 5988-5994), (Neoplasia 2001, 3, 278)], breast (Mol. Cancer Ther. 2002, 1, 707), colon [(Neoplasia 2001, 3, 278), (Arico, S. et al., J. Biol. Chem. 2002, 277, 27613-27621)], cervical (Neoplasia 2001, 3, 278), prostate [(Endocrinology 2001, 142, 4795), (Thakkar, H. et al. J. Biol. Chem. 2001, 276, 38361-38369), (Chen, X. et al., Oncogene 2001, 20, 6073-6083)] and brain (glioblastomas) [(Flynn, P. et al., Curr. Biol. 2000, 10, 1439-1442)].

Accordingly, there is a great need to develop inhibitors of ROCK, ERK, GSK, and members of the AGC sub-family of protein kinases (e.g., PKA, PDK, p70^{S6K}-1 and -2, and PKB) that would be useful in treating various diseases or conditions associated with ROCK, ERK or GSK activation, or activation of the AGC sub-family of protein kinases (e.g., PKA, PDK, p70^{S6K}-1 and -2, and PKB), particularly given the inadequate treatments currently available for the majority of these disorders.

WO 02/14311 describes N-thiazolyl-N'-aryl ureas, wherein the thiazolyl group is substituted by pyridyl groups. These protein kinase inhibitors are described as useful in the treatment of proliferative disorders, neurological disorders and apoptosis.

WO 00/26202 discloses 2-acylamino-thiazoles, described as useful for treating cell proliferative disorders associated with an altered cell dependent kinase activity.

EP 1205478 discloses 1,3 thiazole compounds, which are substituted by a pyridyl group at the 5 position. These compounds are described as inhibitors of p38/MAP kinase activity and inhibitors of TNF-α production.

WO 00/26203 discloses 2-ureido-1,3-thiazole derivatives, described as useful for the treatment of cell proliferative disorders associated with an altered cell dependent kinase activity.

JP 2002 053566 discloses thiazole compounds which are described as being protein kinase C inhibitors and described as having a sedative effect.

### SUMMARY OF THE INVENTION

It has now been found that compounds of this invention, and pharmaceutically acceptable compositions thereof, are effective as inhibitors of ROCK, ERK, GSK, and members of the AGC sub-family of protein kinases (e. g., PKA, PDK, p70^{S6K}-1 and -2, and PKB). These compounds have the general formula I: 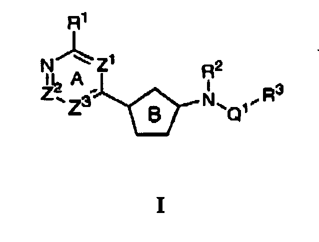 or a pharmaceutically acceptable derivative thereof, wherein ring B, R¹, R², R³, Z¹, Z², Z³, and Q¹ are as defined below.

These compounds, and pharmaceutically acceptable compositions thereof, are useful for treating or lessening the severity of a variety of disorders, including allergic disorders such as asthma and atopic dermatitis, autoimmune diseases such as SLE lupus and psoriasis, conditions associated with organ transplantation, proliferative disorders such as cancer, inflammatory diseases, destructive bone disorders, hypertension, angina pectoris, cerebrovascular contraction, asthma, peripheral circulation disorder, premature birth, arteriosclerosis, spasm, retinopathy, erectile dysfunction (ED), Alzheimer's Disease, reperfusion/ischemia induced injury (e.g., stroke), and AIDS, to name a few.

The compounds provided by this invention are also useful for the study of kinases in biological and pathological phenomena; the study of intracellular signal transduction pathways mediated by such kinases, and the comparative evaluation of new kinase inhibitors.

### DETAILED DESCRIPTION OF THE INVENTION

*I. General Description of Compounds of the Invention:*

The present invention relates to a compound of formula **I:** 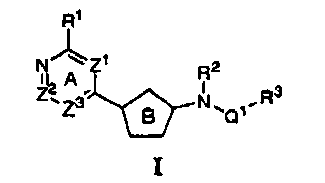 or a pharmaceutically acceptable salt thereof, wherein:
wherein 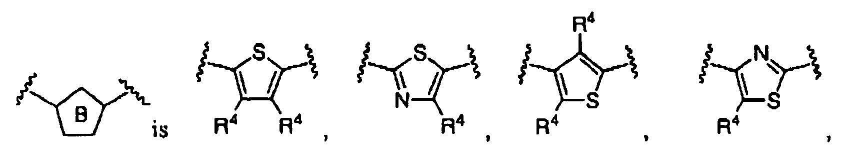 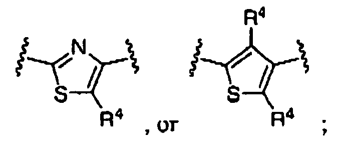 R¹ is halogen, CN, NO₂, or VₘR;
Z¹ and Z³ are each CR^{Z} and Z² is CR¹;
each occurrence of R^{Z} is independently halogen, CN, NO₂, or UₙR';
R² is UₙR';
each occurrence of R⁴ is independently halogen, CN, NO₂, or VₘR;
each occurrence of U or V is independently an optionally substituted C₁₋₆ alkylidene chain, wherein up to two methylene units of the chain are optionally and independently replaced by -NR-, -S-, -O-, -CS-, -CO₂-, -OCO-, -CO-, -COCO-, -CONR-, -NRCO-, -NRCO₂-, -SO₂NR-, -NRSO₂-, -CONRNR-, -NRCONR-, -OCONR-, -NRNR-, -NRSO₂NR-, -SO-, -SO₂-, -PO-, -PO₂-, or -POR-;
m and n are each independently 0 or 1;
each occurrence of R is independently hydrogen or an optionally substituted C₁₋₆ aliphatic group; and each occurrence of R^{'} is independently hydrogen or an optionally substituted C₁₋₆ aliphatic group, a 3-8-membered saturated, partially unsaturated, or fully unsaturated monocyclic ring having 0-3 heteroatoms independently selected from nitrogen, oxygen, or sulfur, or an 8-12 membered saturated, partially unsaturated, or fully unsaturated bicyclic ring system having 0-5 heteroatoms independently selected from nitrogen, oxygen, or sulfur; or R and R^{'}, two occurrences of R, or two occurrences of R^{'}, arc taken together with the atom(s) to which they are bound to form an optionally substituted 3-12 membered saturated, partially unsaturated, or fully unsaturated monocyclic or bicyclic ring having 0-4 heteroatoms independently selected from nitrogen, oxygen, or sulfur;
Q¹ is -CO-;
R³ is Q²-Ar¹, wherein Q² is -(CHR⁶)_{q}-, where q is 1, 2, or 3,
or R² and Q¹-R³, taken together with the intervening nitrogen atom, form the cyclic group: 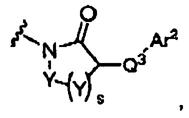 where s is 1 or 2, each occurrence of Y is independently, as valency and stability permit, -CO-, -CS-, -SO₂-, -O-, -S-, -NR⁵-, or -C(R⁵)₂-, and R⁵ is UₙR';
Q³ is a bond or a C₁₋₆ alkylidene chain, wherein up to two methylene units of the chain are each optionally and independently replaced by -NR'-, -S-, -O-, -CS-, -CO₂-, -OCO-, -CO-, - COCO-, -CONR'-, -NR'CO-, -NR'CO₂-, -SO₂NR'-, -NR'SO₂-, -CONR'NR'-, -NR'CONR'-, -OCONR'-, -NR'NR'-, -NR'SO₂NR'-, -SO-, -SO₂-, -PO-, -PO₂-, or-POR'-; and wherein any carbon atom in the one or more methylene units is optionally substituted with one or two occurrences of R⁶, wherein each occurrence of R⁶ is independently halogen, CN, NO₂, or UₙR', or two occurrences of R⁶, or R' and R⁶, taken together with the atoms to which they are bound, form an optionally substituted 3-6-membered cycloalkyl, heterocyclyl, aryl or heteroaryl ring; and
Ar¹ is 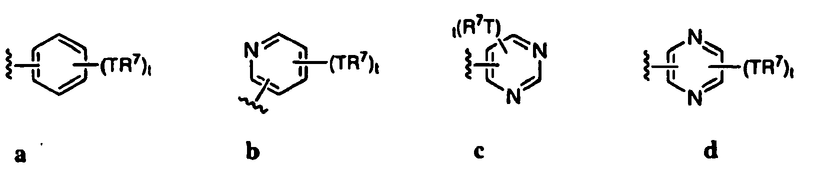 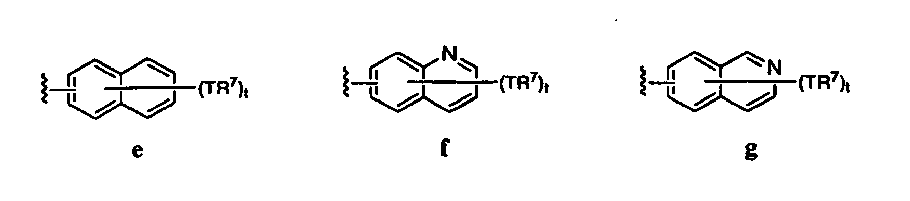 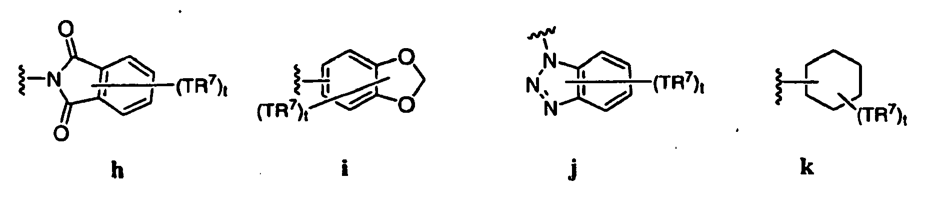 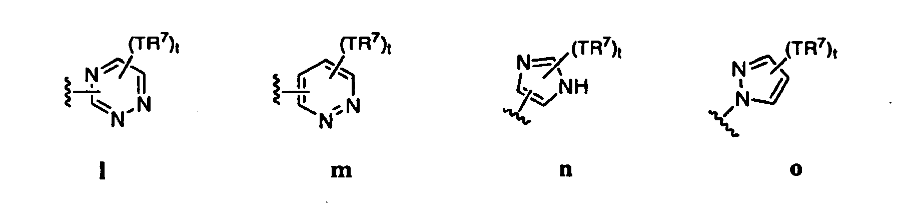 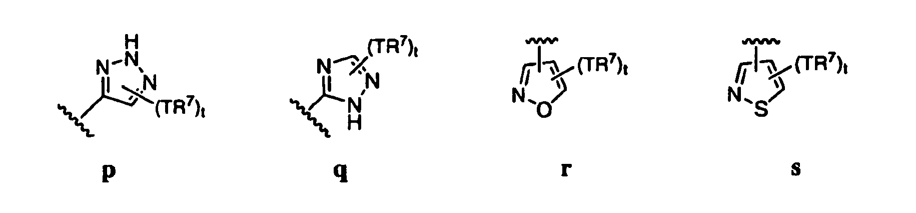 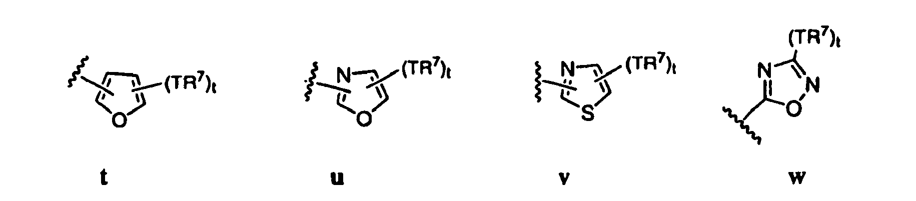 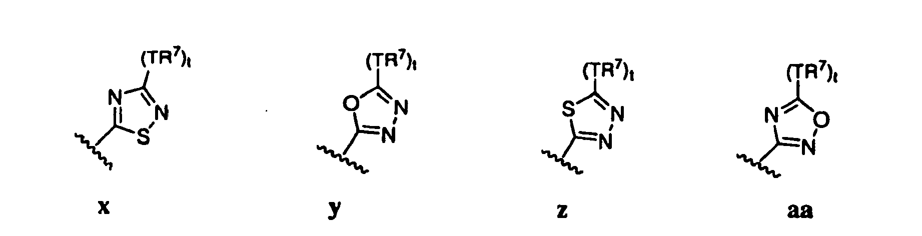 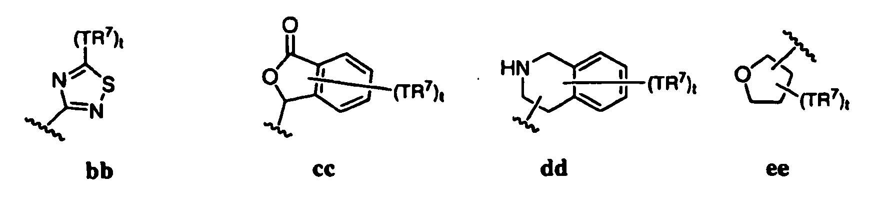 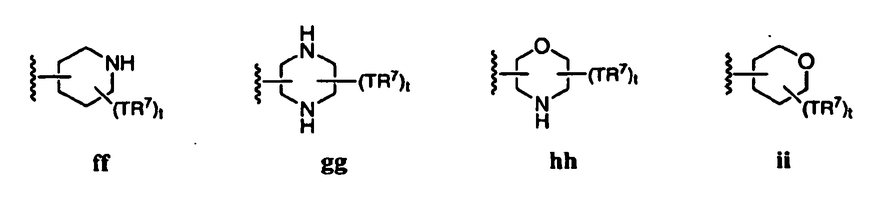 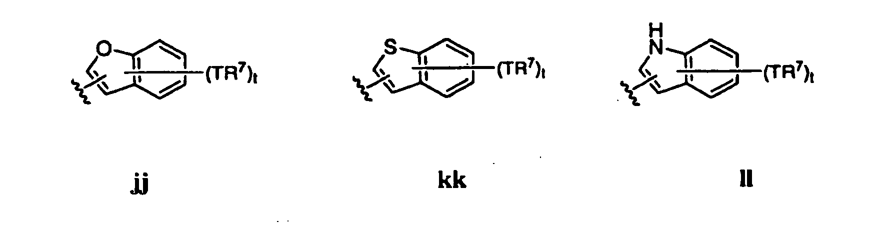 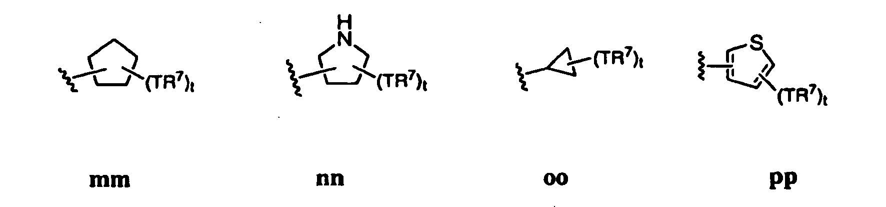 wherein t is 0, 1, 2, 3, 4 or 5, and wherein any Ar¹ is bonded to Q² through any substitutable nitrogen or carbon atom, and wherein one or more hydrogen atoms on any substitutable nitrogen or carbon atom is substituted with one or more independent occurrences of TR⁷, wherein TR⁷ is defined generally below; and Ar² is a 5-8 membered saturated, partially unsaturated, or fully unsaturated monocyclic ring having 0-3 heteroatoms independently selected from nitrogen, oxygen, or sulfur, or an 8-12 membered saturated, partially unsaturated, or fully unsaturated bicyclic ring system having 0-5 heteroatoms independently selected from nitrogen, oxygen, or sulfur; wherein Ar¹ and Ar² are each optionally substituted with 0-5 independent occurrences of TR⁷; wherein T is a bond or is a C₁-C₆ alkylidene chain wherein up to two methylene units of T are optionally and independently replaced by -NR-, -S-, -O-,
-CS-, -CO₂-, -OCO-, -CO-, -COCO-, -CONR-, -NRCO-, -NRCO₂-, -SO₂NR-, -NRSO₂-, -CONRNR-, -NRCONR-, -OCONR-, -NRNR-, -NRSO₂NR-, -SO-, -SO₂-, -PO-, -PO₂-, or -POR-; and each occurrence of R⁷ is independently R', halogen, NO₂, or CN;
provided that: for compounds having the structure: 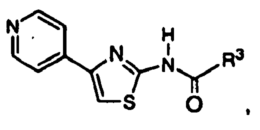 R³ is not any one of the following groups: -CH₂(3-NHCOPh-phenyl), -CH₂-pyrrolidine, unsubstituted benzyl, -CH₂-naphthyl, -CH₂CH₂-3-(4-Cl-phenyl)-1-phenyl-1-H-pyrazol-4-yl, or -CH₂(1,3-dioxoisoindole).

2. *Compounds and Definitions:*

Compounds of this invention include those described generally above, and are further illustrated by the classes, subclasses, and species disclosed herein. As used herein, the following definitions shall apply unless otherwise indicated. For purposes of this invention, the chemical elements are identified in accordance with the Periodic Table of the Elements, CAS version, Handbook of Chemistry and Physics, 75th Ed. Additionally, general principles of organic chemistry are described in "Organic Chemistry", Thomas Sorrell, University Science Books, Sausalito: 1999, and "March's Advanced Organic Chemistry", 5th Ed., Ed.: Smith, M.B. and March, J., John Wiley & Sons, New York: 2001, the entire contents of which are hereby incorporated by reference.

As described herein, compounds of the invention may optionally be substituted with one or more substituents, such as are illustrated generally above, or as exemplified by particular classes, subclasses, and species of the invention. It will be appreciated that the phrase "optionally substituted" is used interchangeably with the phrase "substituted or unsubstituted." In general, the term "substituted", whether preceded by the term "optionally" or not, refers to the replacement of hydrogen radicals in a given structure with the radical of a specified substituent. Unless otherwise indicated, an optionally substituted group may have a substituent at each substitutable position of the group, and when more than one position in any given structure may be substituted with more than one substituent selected from a specified group, the substituent may be either the same or different at every position. Combinations of substituents envisioned by this invention are preferably those that result in the formation of stable or chemically feasible compounds. The term "stable", as used herein, refers to compounds that are not substantially altered when subjected to conditions to allow for their production, detection, and preferably their recovery, purification, and use for one or more of the purposes disclosed herein. In some embodiments, a stable compound or chemically feasible compound is one that is not substantially altered when kept at a temperature of 40°C or less, in the absence of moisture or other chemically reactive conditions, for at least a week.

The term "aliphatic" or "aliphatic group", as used herein, means a straight-chain (i.e., unbranched) or branched, substituted or unsubstituted hydrocarbon chain that is completely saturated or that contains one or more units of unsaturation, or a monocyclic hydrocarbon or bicyclic hydrocarbon that is completely saturated or that contains one or more units of unsaturation, but which is not aromatic (also referred to herein as "carbocycle" "cycloaliphatic" or "cycloalkyl"), that has a single point of attachment to the rest of the molecule. Unless otherwise specified, aliphatic groups contain 1-20 aliphatic carbon atoms. In some embodiments, aliphatic groups contain 1-10 aliphatic carbon atoms. In other embodiments, aliphatic groups contain 1-8 aliphatic carbon atoms. In still other embodiments, aliphatic groups contain 1-6 aliphatic carbon atoms, and in yet other embodiments aliphatic groups contain 1-4 aliphatic carbon atoms. In some embodiments, "cycloaliphatic" (or "carbocycle" or "cycloalkyl") refers to a monocyclic C₃-C₈ hydrocarbon or bicyclic C₈-C₁₂ hydrocarbon that is completely saturated or that contains one or more units of unsaturation, but which is not aromatic, that has a single point of attachment to the rest of the molecule wherein any individual ring in said bicyclic ring system has 3-7 members. Suitable aliphatic groups include, but are not limited to, linear or branched, substituted or unsubstituted alkyl, alkenyl, alkynyl groups and hybrids thereof such as (cycloalkyl)alkyl, (cycloalkenyl)alkyl or (cycloalkyl)alkenyl.

The term "heteroaliphatic", as used herein, means aliphatic groups wherein one or two carbon atoms are independently replaced by one or more of oxygen, sulfur, nitrogen, phosphorus, or silicon. Heteroaliphatic groups may be substituted or unsubstituted, branched or unbranched, cyclic or acyclic, and include "heterocycle", "heterocyclyl", "heterocycloaliphatic", or "heterocyclic" groups.

The term "heterocycle", "heterocyclyl", "heterocycloaliphatic", or "heterocyclic" as used herein means non-aromatic, monocyclic, bicyclic, or tricyclic ring systems in which one or more ring members are an independently selected heteroatom. In some embodiments, the "heterocycle", "heterocyclyl", "heterocycloaliphatic", or "heterocyclic" group has three to fourteen ring members in which one or more ring members is a heteroatom independently selected from oxygen, sulfur, nitrogen, or phosphorus, and each ring in the system contains 3 to 7 ring members.

The term "heteroatom" means one or more of oxygen, sulfur, nitrogen, phosphorus, or silicon (including, any oxidized form of nitrogen, sulfur, phosphorus, or silicon; the quaternized form of any basic nitrogen or; a substitutable nitrogen of a heterocyclic ring, for example N (as in 3,4-dihydro-2*H*-pyrrolyl), NH (as in pyrrolidinyl) or NR⁺ (as in N-substituted pyrrolidinyl)).

The term "unsaturated", as used herein, means that a moiety has one or more units of unsaturation.

The term "alkoxy", or "thioalkyl", as used herein, refers to an alkyl group, as previously defined, attached to the principal carbon chain through an oxygen ("alkoxy") or sulfur ("thioalkyl") atom.

The terms "haloalkyl", "haloalkenyl" and "haloalkoxy" means alkyl, alkenyl or alkoxy, as the case may be, substituted with one or more halogen atoms. The term "halogen" means F, Cl, Br, or I.

The term "aryl" used alone or as part of a larger moiety as in "aralkyl", "aralkoxy", or "aryloxyalkyl", refers to monocyclic, bicyclic, and tricyclic ring systems having a total of five to fourteen ring members, wherein at least one ring in the system is aromatic and wherein each ring in the system contains 3 to 7 ring members. The term "aryl" may be used interchangeably with the term "aryl ring". The term "aryl" also refers to heteroaryl ring systems as defined hereinbelow.

The term "heteroaryl", used alone or as part of a larger moiety as in "heteroaralkyl" or "hetemarylalkoxy", refers to monocyclic, bicyclic, and tricyclic ring systems having a total of five to fourteen ring members, wherein at least one ring in the system is aromatic, at least one ring in the system contains one or more heteroatoms, and wherein each ring in the system contains 3 to 7 ring members. The term "heteroaryl" may be used interchangeably with the term "heteroaryl ring" or the term "heteroaromatic".

An aryl (including aralkyl, aralkoxy, aryloxyalkyl and the like) or heteroaryl (including heteroaralkyl and heteroarylalkoxy and the like) group may contain one or more substituents and thus may be "optionally substituted". Unless otherwise defined above and herein, suitable substituents on the unsaturated carbon atom of an aryl or heteroaryl group are generally selected from halogen; -R°; -OR°; -SR°; phenyl (Ph) optionally substituted with R°; -O(Ph) optionally substituted with R°; -(CH₂)₁₋₂(Ph), optionally substituted with R°; - CH=CH(Ph), optionally substituted with R°; -NO₂; -CN; -N(R°)₂; -NR°C(O)R°; - NR°C(S)R°; -NR°C(O)N(R°)₂; -NR°C(S)N(R°)₂; -NR°CO₂R°; -NR°NR°C(O)R°; -NR°NR°C(O)N(R°)₂; -NR°NR°CO₂R°; -C(O)C(O)R°; -C(O)CH₂C(O)R°; -CO₂R°; - C(O)R°: -C(S)R°: -C(O)N(R°)₂; -C(S)N(R°)₂; -OC(O)N(R°)₂; -OC(O)R°; -C(O)N(OR°) R°; -C(NOR°) R°; -S(O)₂R°; -S(O)₃R°; -SO₂N(R°)₂; -S(O)R°; -NR°SO₂N(R°)₂; -NR°SO₂R°; -N(OR°)R°; -C(=NH)-N(R°)₂; -P(O)₂R°; -PO(R°)_{2;} -OPO(R°)₂; -(CH₂)₀₋₂NHC(O)R°; phenyl (Ph) optionally substituted with R°; -O(Ph) optionally substituted with R°; -(CH₂)₁₋₂(Ph), optionally substituted with R°; or -CH=CH(Ph), optionally substituted with R°; wherein each independent occurrence of R° is selected from hydrogen, optionally substituted C₁₋₆ aliphatic, an unsubstituted 5-6 membered heteroaryl or heterocyclic ring, phenyl, -O(Ph), or -CH₂(Ph), or, notwithstanding the definition above, two independent occurrences of R°, on the same substituent or different substituents, taken together with the atom(s) to which each R° group is bound, to form an optionally substituted 3-12 membered saturated, partially unsaturated, or fully unsaturated monocyclic or bicyclic ring having 0-4 heteroatoms independently selected from nitrogen, oxygen, or sulfur.

. Optional substituents on the aliphatic group of R° are selected from NH₂, NH(C₁₋₄aliphatic), N(C₁₋₄aliphatic)₂, halogen, C₁₋₄aliphatic, OH, O(C₁₋₄aliphatic), NO₂, CN, CO₂H, CO₂(C₁₋₄aliphatic), O(haloC₁₋₄ aliphatic), or haloC₁₋₄aliphatic, wherein each of the foregoing C₁₋₄aliphatic groups of R° is unsubstituted.

An aliphatic or heteroaliphatic group, or a non-aromatic heterocyclic ring may contain one or more substituents and thus may be "optionally substituted". Unless otherwise defined above and herein, suitable substituents on the saturated carbon nf an aliphatic or heteroaliphatic group, or of a non-aromatic heterocyclic ring are selected from those listed above for the unsaturated carbon of an aryl or heteroaryl group and additionally include the following =O, =S, =NNHR^{*}, =NN(R^{*})₂, =NNHC(O)R⁺, =NNHCO₂(alkyl), =NNHSO₂(alkyl), or =NR⁺, where each R⁺ is independently selected from hydrogen or an optionally substituted C₁₋₆ aliphatic group.

Unless otherwise defined above and herein, optional substituents on the nitrogen of a non-aromatic heterocyclic ring are generally selected from -R⁺, -N(R⁺)2, -C(O)R⁺, -CO₂R⁺, -C(O)C(O)R⁺, -C(O)CH₂C(O)R⁺, -SO₂R⁺, -SO₂N(R⁺)₂, -C(=S)N(R⁺¹)₂, -C(=NH)-N(R⁺)₂, or -NR⁺SO₂R⁺; wherein R⁺ is hydrogen, an optionally substituted C₁₋₆ aliphatic, optionally substituted phenyl, optionally substituted -O(Ph), optionally substituted -CH₂(Ph), optionally substituted -(CH₂)₁₋₂(Ph); optionally substituted -CH=CH(Ph); or an unsubstituted 5-6 membered heteroaryl or heterocyclic ring having one to four heteroatoms independently selected from oxygen, nitrogen, or sulfur, or, notwithstanding the definition above, two independent occurrences of R⁺, on the same substituent or different substituents, taken together with the atom(s) to which each R⁺ group is bound, form an optionally substituted 3-12 membered saturated, partially unsaturated, or fully unsaturated monocyclic or bicyclic ring having 0-4 heteroatoms independently selected from nitrogen, oxygen, or sulfur.

Optional substituents on the aliphatic group or the phenyl ring of R⁺ are selected from -NH₂, -NH(C₁₋₄ aliphatic), -N(C₁₋₄ aliphatic)₂, halogen, C₁₋₄ aliphatic, -OH, -O(C₁₋₄ aliphatic), -NO₂, -CN, -CO₂H, -CO₂(C₁₋₄ aliphatic), -O(halo C₁₋₄ aliphatic), or halo(C₁₋₄ aliphatic), wherein each of the foregoing C₁₋₄aliphatic groups of R⁺ is unsubstituted.

The term "alkylidene chain" refers to a straight or branched carbon chain that may be fully saturated or have one or more units of unsaturation and has two points of attachment to the rest of the molecule.

As detailed above, in some embodiments, two independent occurrences of R° (or R⁺, R, R' or any other variable similarly defined herein), are taken together with the atom(s) to which they are bound to form an optionally substituted 3-12 membered saturated, partially unsaturated, or fully unsaturated monocyclic or bicyclic ring having 0-4 heteroatoms independently selected from nitrogen, oxygen, or sulfur.

Exemplary rings that are formed when two independent occurrences of R° (or R⁺, R, R' or any other variable similarly defined herein), are taken together with the atom(s) to which each variable is bound include, but are not limited to the following: a) two independent occurrences of R° (or R⁺, R, R' or any other variable similarly defined herein) that are bound to the same atom and are taken together with that atom to form a ring, for example, N(R°)₂, where both occurrences of R° are taken together with the nitrogen atom to form a piperidin-1-yl, piperazin-1-yl, or mopholin-4-yl group; and b) two independent occurrences of R° (or R⁺, R, R' or any other variable similarly defined herein) that are bound to different atoms and are taken together with both of those atoms to form a ring, for example where a phenyl group is substituted with two occurrences of OR° 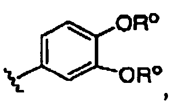 these two occurrences of R° are taken together with the oxygen atoms to which they are bound to form a fused 6-membered oxygen containing ring: 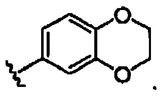 It will be appreciated that a variety of other rings can be formed when two independent occurrences of R° (or R⁺, R, R' or any other variable similarly defined herein) are taken together with the atom(s) to which each variable is bound and that the examples detailed above are not intended to be limiting.

Unless otherwise stated, structures depicted herein are also meant to include all isomeric (e.g., enantiomeric, diastereomeric, and geometric (or conformational)) forms of the structure; for example, the R and S configurations for each asymmetric center, (Z) and (E) double bond isomers, and (Z) and (E) conformational isomers. Therefore, single stereochemical isomers as well as enantiomeric, diastereomeric, and geometric (or conformational) mixtures of the present compounds are within the scope of the invention. Unless otherwise stated, all tautomeric forms of the compounds of the invention are within the scope of the invention. Additionally, unless otherwise stated, structures depicted herein are also meant to include compounds that differ only in the presence of one or more isotopically enriched atoms. For example, compounds having the present structures except for the replacement of hydrogen by deuterium or tritium, or the replacement of a carbon by a ¹³C- or ¹⁴C-enriched carbon are within the scope of this invention. Such compounds are useful, for example, as analytical tools or probes in biological assays.

*3. Description of Exemplary Compounds:*

In certain embodiments, compounds of formula I-A, I-B, or I-C are provided: 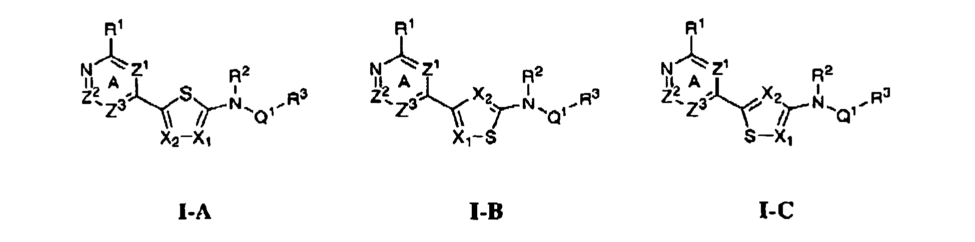 wherein X₁ and X₂ arc each independently CR⁴ or N, and the mig is selected from one of:

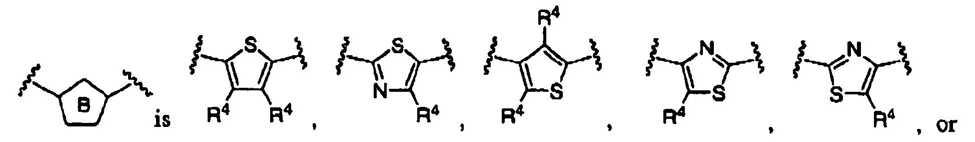

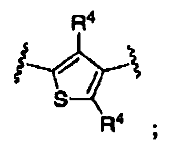

As also described generally above for compounds of formula **I**, R³ is Q²-Ar¹, or R² and Q¹-R³, taken together with the nitrogen atom, form the cyclic group: 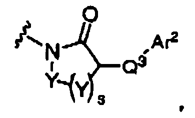 where s is 1 or 2, each occurrence of Y is independently, as valency and stability permit, - CO-, -CS-, -SO₂-, -O-, -S-, -NR⁵-, or -C(R⁵)₂-, and R⁵ is UₙR'.

Accordingly, in one embodiment, R³ is Q²-Ar¹ and compounds of formula **I-A-i, I-B-i,** and **I-C-i** are provided. 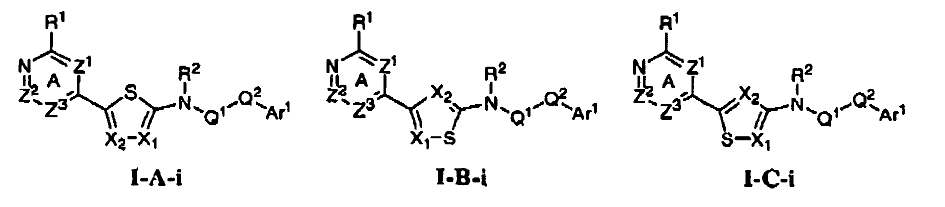 wherein X₁ and X₂ are each independently CR⁴ or N, and the ring is selected from one of:

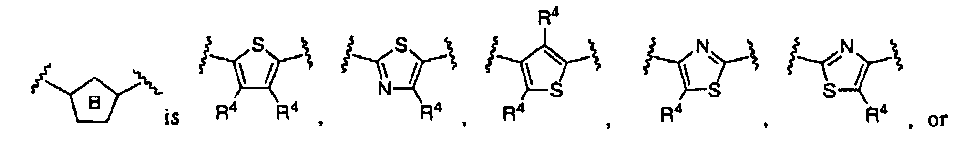

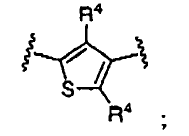

In general, for compounds of formula I (and compounds of formula **I-A-i, I-B-i,** and **I-C-i),** R² is UₙR'. In certain embodiments, R² is hydrogen, or is UₙR', where n is I, and U is a C₁₋₆ alkylidene chain wherein one or two methylene units are optionally and independently replaced by -O-, -NR-, -S-, or -CO-. In other embodiments, U is -CH₂-, -CH₂CH₂-, -CH₂CH₂CH₂-, -CH₂CH₂CH₂CH₂-, -CH₂O-, -CH₂S-, -CH₂NR-, -CH₂CH₂O-, -CH₂CH₂S-, -CH₂CH₂NR-, -CH₂CH₂CH₂O-, -CH₂CH₂CH₂S-, -CH₂CH₂CH₂NR-, -CH₂CH₂CH₂CH₂O-, -CH₂CH₂CH₂CH₂S-, -CH₂CH₂CH₂CH₂NR-, -CH₂CH₂OCH₂CH₂-, -(CH₂)₄NHCH₂-, -(CH₂)₃NHCH₂CH₂-, or -CH₂CH₂NHCH₂CH₂-, and exemplary R' groups are hydrogen, C₁-C₄alkyl, optionally substituted tetrahydropyranyl, pyrrolidinyl, piperidinyl, piperazinyl, morpholinyl, thiomorpholinyl, pyridinyl, phenyl, or cyclohexyl, or R and R', taken together with the nitrogen atom to which they are bound, form an optionally substituted 5- or 6-membered saturated, partially unsaturated, or unsaturated heterocyclyl ring.

As described generally above, for compounds of formula **I** (and compounds of formula **I-A-i, I-B-i,** and **I-C-i),** Q¹ is -CO-, -SO₂-, -CONR-, or -SO₂NR-. In some embodiments, Q¹ is -CO- or -SO₂NR-. In other embodiments, Q¹ is -CO-.

For compounds of general formula **I** (and compounds of formula **I-A-i, I-B-i,** and **I-C-i),** Q² is a bond or a C₁₋₆ alkylidene chain, wherein up to two methylene units of the chain are each optionally and independently replaced by -NR'-, -S-, -O-, -CS-, -CO₂-, -OCO-, - CO-, -COCO-, -CONR'-, -NR'CO-, -NR'CO₂-, -SO₂NR'-, -NR'SO₂-, -CONR'NR'-,-NR'CONR'-, -OCONR'-, -NR'NR'-, -NR'SO₂NR'-, -SO-, -SO₂-, -PO-, -PO₂-, or -POR'-; and wherein any carbon atom in the one or more methylene units is optionally substituted with one or two occurrences of R⁶, wherein each occurrence of R⁶ is independently halogen, CN, NO₂, or UₙR', or two occurrences of R⁶, or R' and R⁶, taken together with the atoms to which they are bound, form an optionally substituted 3-6-membered cycloalkyl, heterocyclyl, aryl or heteroaryl ring. In some embodiments, Q² is a direct bond, or is -(CHR⁶)_{q}-, - (CHR⁶)_{q}O-, -(CHR⁶)_{q}S-, -(CHR⁶)_{q}S(O)₂-, -(CHR⁶)_{q}S(O)- , -(CHR⁶)_{q}NR-, or -(CHR⁶)_{q}C(O)-, wherein q is 0, 1, 2, or 3. In certain other embodiments, R⁶ is R', -N(R)(R'), -(CH₂)₁. ₄N(R)(R'). -OR', -(CH₂)₁₋₄OR', -NR(CH₂)₁₋₄N(R)(R'), -NR(CH₂)₁₋₄SO₂R', -NR(CH₂)₁₋₄COOR', or -NR(CH₂)₁₋₄COR', or two occurrences of R⁶, taken together with the atoms to which they are bound, form an optionally substituted 3-6-membered saturated, partially unsaturated, or fully unsaturated ring. Examples of such R⁶ groups include, but are not limited to CH₂OH, CH₂CH₂OH, OH, OMe, OEt, NH₂, NH(Me), NH(Et), N(Me)(Me), CH₂NH₂, CH₂CH₂NH₂, NHCO₂*t*-butyl, phenyl, cyclopentyl, methyl, ethyl, isopropyl, cyclopropyl, NH(CH₂)₃NH₂, NH(CH₂)₂NH₂, NH(CH₂)₂NHEt, NHCH₂pyridyl, NHSO₂phenyl, NHC(O)CH₂C(O)O*t*-butyl, NHC(O)CH₂NH₃, and NHCH₂-imidazol-4-yl.

In certain embodiments, Ar¹ is **a, b, e, g, h, i, j, k, r, cc, dd, ff, jj, ll,** or **pp.** As described generally above, Ar¹ is optionally substituted with 0-5 independent occurrences of TR⁷; wherein T is a bond or is a C₁-C₆ alkylidene chain wherein up to two methylene units of T are optionally replaced by -NR-, -S-, -O-, -CS-, -CO₂-, -OCO-, -CO-, -COCO-, -CONR-, - NRCO-, -NRCO₂-, -SO₂NR-, -NRSO₂-, -CONRNR-, -NRCONR-, -OCONR-, -NRNR-,-NRSO₂NR-, -SO-, -SO₂-, -PO-, -PO₂-, or -POR-; and each occurrence of R⁷ is independently R', halogen, NO₂, or CN. In certain embodiments, T is a bond or is an optionally substituted C₁₋₆ alkylidene chain wherein one or two methylene units are optionally and independently replaced by --O-, -NR-, -S-, -SO₂-,-COO-, -CO-, -OSO₂-, -NRSO₂, -CONR-, or -SO₂NR-, and R⁷ is R' or halogen. In other embodiments, each occurrence of TR⁷ is independently - C₁₋₃alkyl, -OR', -SR', -CF₃, -OCF₃, -SCF₃, -F, -Cl, I, -Br, -COOR', -COR', - O(CH₂)₄N(R)(R'), -O(CH₂)₃N(R)(R'), -O(CH₂)₂N(R)(R'), -O(CH₂)N(R)(R'), - O(CH₂)₄CON(R)(R'), -O(CH₂)₃CON(R)(R'), -O(CH₂)₂CON(R)(R'), -O(CH₂)CON(R)(R'), - C(O)N(R)(R'), -(CH₂)₄OR', -(CH₂)₃OR', -(CH₂)₂OR', -CH₂OR', optionally substituted phenyl or benzyl, -N(R)(R'), -(CH₂)₄N(R)(R), -(CH₂)₃N(R)(R'), -(CH₂)₂N(R)(R'), - (CH₂)N(R)(R'), or SO₂N(R)(R'), NRSO₂R', CON(R)(R'), or -OSO₂R', where, as defined generally above, each occurrence of R is independently hydrogen or an optionally substituted C₁₋₆ aliphatic group; and each occurrence of R' is independently hydrogen or an optionally substituted C₁₋₆ aliphatic group, a 3-8-membered saturated, partially unsaturated, or fully unsaturated monocyclic ring having 0-3 heteroatoms independently selected from nitrogen, oxygen, or sulfur, or an 8-12 membered saturated, partially unsaturated, or fully unsaturated bicyclic ring system having 0-5 heteroatoms independently selected from nitrogen, oxygen, or sulfur; or R and R^{'}, two occurrences of R, or two occurrences of R^{'}, are taken together with the atom(s) to which they are bound to form an optionally substituted 3-12 membered saturated, partially unsaturated, or fully unsaturated monocyclic or bicyclic ring having 0-4 heteroatoms independently selected from nitrogen, oxygen, or sulfur.

In another embodiment, R³ is Q²-Ar¹, or R² and Q¹-R³, taken together with the nitrogen atom, form the cyclic group: 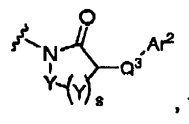 where s is 1 or 2, each occurrence of Y is independently, as valency and stability permit, -CO-, -CS-. -SO₂-, -O-, -S-, -NR⁵-, or - C(R⁵)₂-, and R⁵ is UₙR', and compounds of formula **I-A-ii, I-B-ii,** and **I-C-ii** are provided: 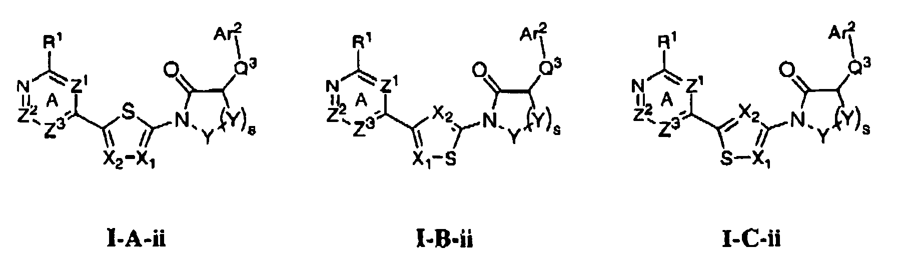 wherein X₁ and X₂ are each independently CR⁴ or N, and the ring is selected from one of:

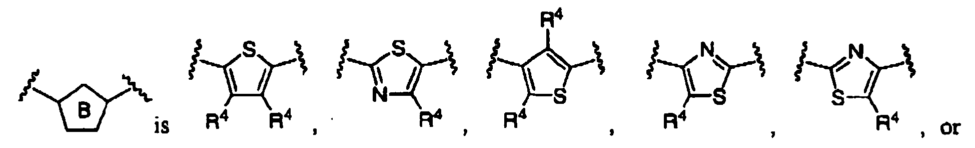

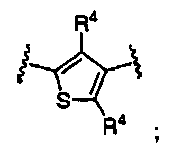

For compounds of formula **I-A-ii, I-B-ii,** and **I-C-ii,** Q³ is a bond or a C₁₋₆ alkylidene chain, wherein up to two methylene units of the chain are each optionally and independently replaced by -NR'-, -S-, -O-, -CS-, -CO₂-, -OCO-, -CO-, -COCO-, -CONR'-, - NR'CO-, -NR'CO₂-, -SO₂NR'-, -NR'SO₂-, -CONR'NR'-, -NR'CONR'-, -OCONR'-, NR'NR'-, -NR'SO₂NR'-, -SO-, -SO₂-, -PO-, -PO₂-, or -POR'-; and wherein any carbon atom in the one or more methylene units is optionally substituted with one or two occurrences of R⁶, wherein each occurrence of R⁶ is independently halogen, CN, NO₂, or UₙR', or two occurrences of R⁶, or R' and R⁶, taken together with the atoms to which they are bound, form an optionally substituted 3-6-membered cycloalkyl, heterocyclyl, aryl or heteroaryl ring. In some embodiments, Q³ is a direct bond, or is -(CHR⁶)_{q}-, -(CHR⁶)_{q}O-, -(CHR⁶)_{q}S-, - (CHR⁶)_{q}S(O)₂-, -(CHR⁶)_{q}S(O)- , -(CHR⁶)_{q}NR-, or -(CHR⁶)_{q}C(O)-, wherein q is 0, 1, 2, or 3. In certain other embodiments, R⁶ is R', -N(R)(R'), -(CH₂)₁₋₄N(R)(R'), -OR', -(CH₂)₁₋₄OR', - NR(CH₂)₁₋₄N(R)(R'), -NR(CH₂)₁₋₄SO₂R', -NR(CH₂)₁₋₄COOR', or -NR(CH₂)₁₋₄COR', or two occurrences of R⁶, taken together with the atoms to which they are bound, form an optionally substituted 3-6-membered saturated, partially unsaturated, or fully unsaturated ring. Examples of such R⁵ groups include, but are not limited to CH₂OH, CH₂CH₂OH, OH, OMe, OEt, NH₂, NH(Me), NH(Et), N(Me)(Me), CH₂NH₂, CH₂CH₂NH₂, NHCO₂*t*-butyl, phenyl, cyclopentyl, methyl, ethyl, isopropyl, cyclopropyl, NH(CH₂)₃NH₂, NH(CH₂)₂NH₂, NH(CH₂)₂NHEt, NHCH₂pyridyl, NHSO₂phenyl, NHC(O)CH₂C(O)O*t*-butyl, NHC(O)CH₂NH₃, and NHCH₂-imidazol-4-yl.

For compounds of general formula **I-A-ii, I-B-ii,** and **I-C-ii,** exemplary Ar² groups are: 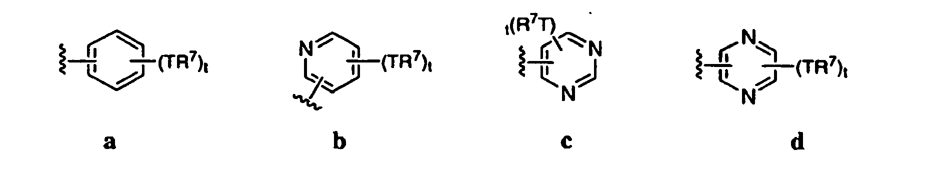 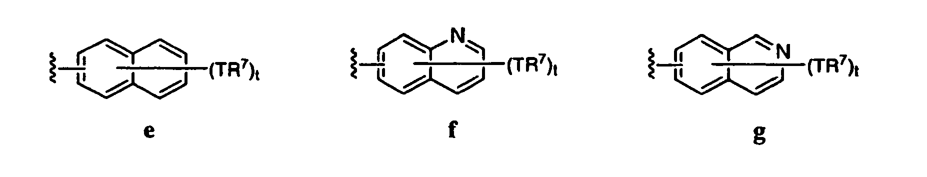 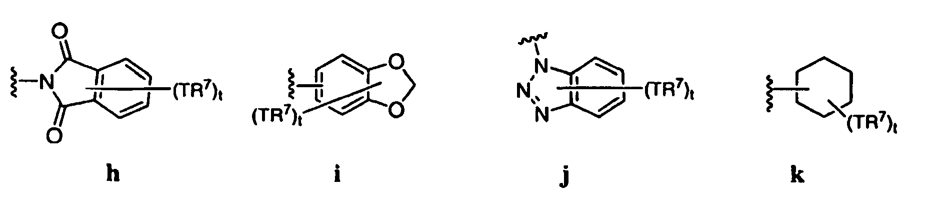 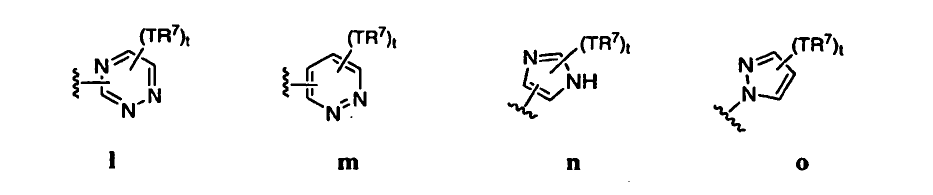 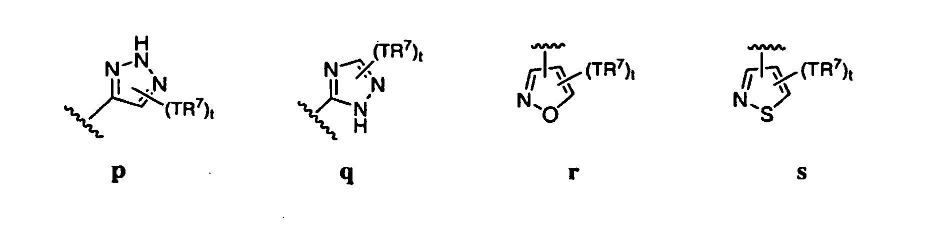 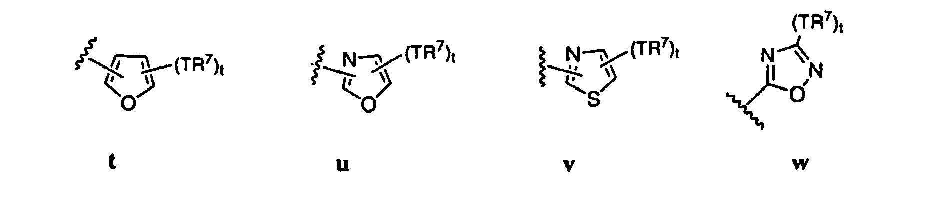 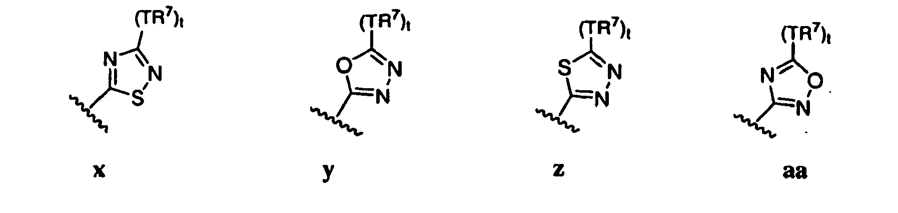 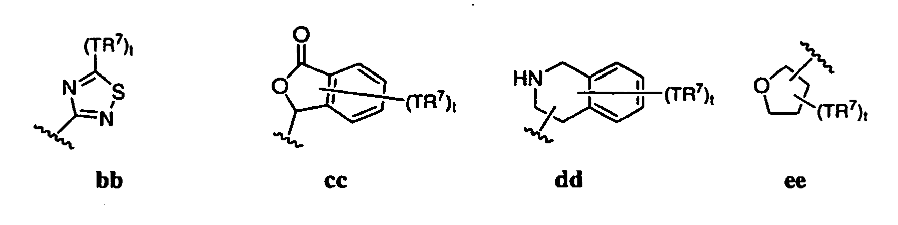 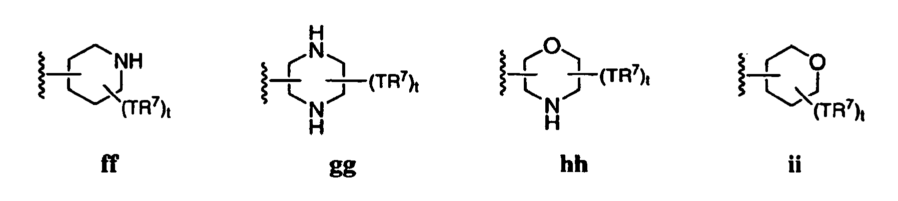 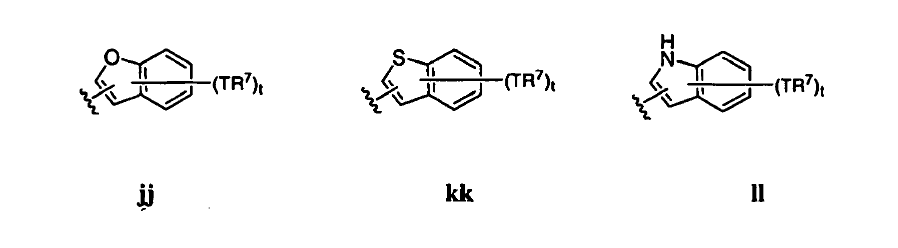 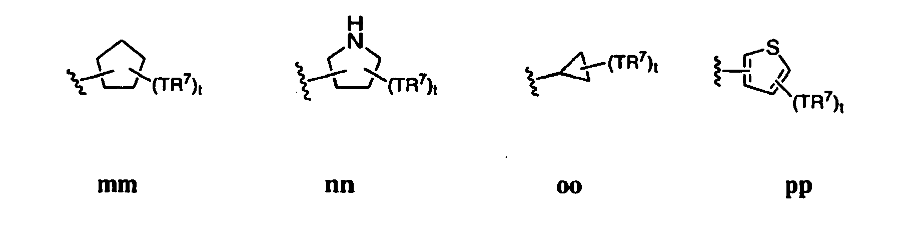 wherein t is 0, 1, 2, 3, 4 or 5, and wherein any Ar² is bonded to Q³ through any substitutable nitrogen or carbon atom, and wherein one or more hydrogen atoms on any substitutable nitrogen or carbon atom is substituted with one or more independent occurrences of TR⁷, wherein TR⁷ is defined generally above.

In more preferred embodiments, Ar² is **a, b, e, g, h, i, j, k, n, r, cc, dd, ff, jj, ll,** or **pp.**

As described generally above, Ar² is optionally substituted with 0-5 independent occurrences of TR⁷; wherein T is a bond or is a C₁-C₆ alkylidene chain wherein up to two methylene units of T are optionally replaced by -NR-, -S-, -O-, -CS-, -CO₂-, -OCO-, -CO-, - COCO-, -CONR-, -NRCO-, -NRCO₂-, -SO₂NR-, -NRSO₂-, -CONRNR-, -NRCONR-, - OCONR-, -NRNR-, -NRSO₂NR-, -SO-, -SO₂-, -PO-, -PO₂-, or -POR-; and each occurrence of R⁷ is independently R', halogen, NO₂, or CN. In certain embodiments, T is a bond or is an optionally substituted C₁₋₆ alkylidene chain wherein one or two methylene units are optionally and independently replaced by -O-, -NR-, -S-, -SO₂-, -COO-, -CO-, -OSO₂-, - NRSO₂, -CONR-, or -SO₂NR-, and R⁷ is R' or halogen. In other embodiments, each occurrence of TR⁷ is independently -C₁₋₃alkyl, -OR', -SR', -CF₃, -OCF₃, -SCF₃, -F, -Cl, I, - Br, -COOR', -COR', -O(CH₂)₄N(R)(R'), -O(CH₂)₃N(R)(R'), -O(CH₂)₂N(R)(R'), - O(CH₂)N(R)(R'), -O(CH₂)₄CON(R)(R'), -O(CH₂)₃CON(R)(R'), -O(CH₂)₂CON(R)(R'), - O(CH₂)CON(R)(R'), -C(O)N(R)(R'), -(CH₂)₄OR', -(CH₂)₃OR', -(CH₂)₂OR', -CH₂OR', optionally substituted phenyl or benzyl, -N(R)(R'), -(CH₂)₄N(R)(R'), -(CH₂)₃N(R)(R'), - (CH₂)₂N(R)(R'), -(CH₂)N(R)(R'), or SO₂N(R)(R'), NRSO₂R', CON(R)(R'), or -OSO₂R', where, as defined generally above, each occurrence of R is independently hydrogen or an optionally substituted C₁₋₆ aliphatic group; and each occurrence of R^{'} is independently hydrogen or an optionally substituted C₁₋₆ aliphatic group, a 3-8-membered saturated, partially unsaturated, or fully unsaturated monocyclic ring having 0-3 heteroatoms independently selected from nitrogen, oxygen, or sulfur, or an 8-12 membered saturated, partially unsaturated, or fully unsaturated bicyclic ring system having 0-5 heteroatoms independently selected from nitrogen, oxygen, or sulfur; or R and R^{'}, two occurrences of R, or two occurrences of R^{'}, are taken together with the atom(s) to which they are bound to form an optionally substituted 3-12 membered saturated, partially unsaturated, or fully unsaturated monocyclic or bicyclic ring having 0-4 heteroatoms independently selected from nitrogen, oxygen, or sulfur.

In certain embodiments, for compounds of formula **I-b,** R⁵ is hydrogen, (CH₂)₃OR', (CH₂)₂OR', (CH₂)OR', (CH₂)₃N(R')₂, (CH₂)₂N(R')₂, (CH₂)N(R')₂, or C₁₋₄aliphatic.

As described generally above, for compounds of formula **I, I-A-i, I-B-i, I-C-i, I-A-ii, I-B-ii,** and **I-C-ii,** X¹ and X² are each independently CR⁴ or N, and thus compounds of formulas **II, III, IV, V, VI, VII, VIII, IX, X, XI, XII,** and **XIII** are provided:

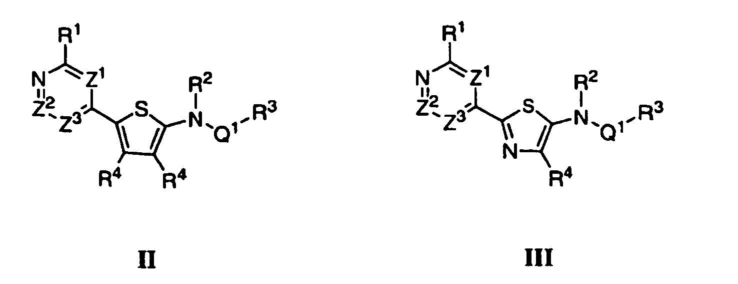

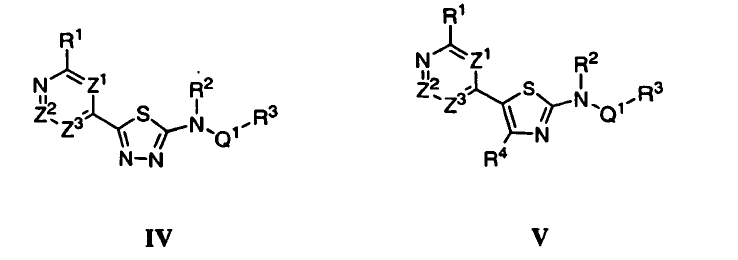

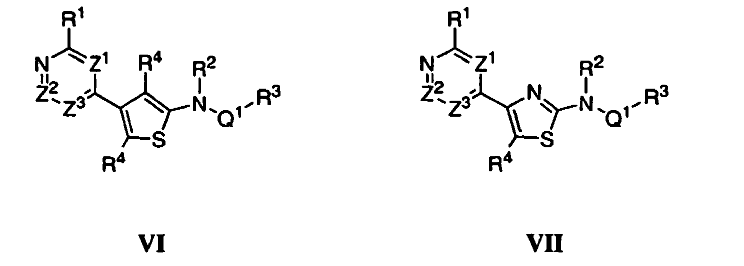

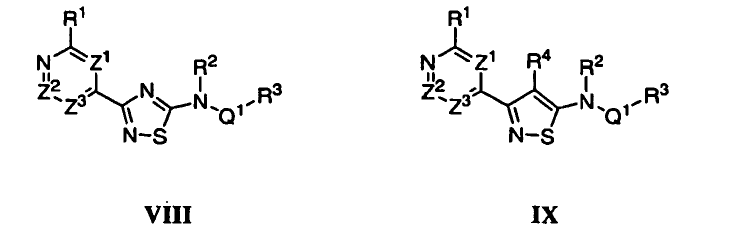

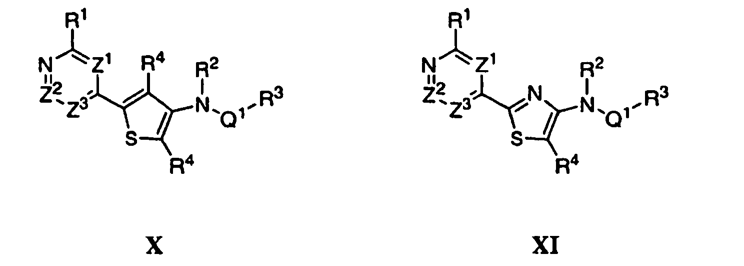

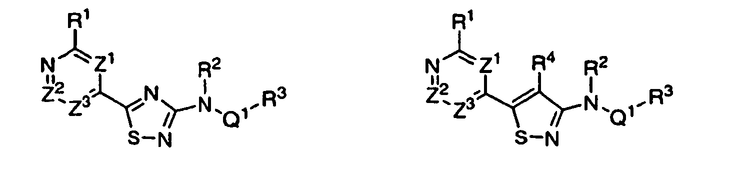

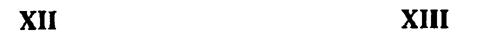

In certain embodiments, for compounds of formula **I, I-A-i, I-B-i, I-C-i, I-A-ii, I-B-ii,** and **I-C-ii,** ring **A** is a pyridyl, pyrimidinyl, triazinyl, or pyridazinyl group. Accordingly, in certain embodiments, compounds have one of the structures of formulas **II-A, II-B, II-C, II-D, II-E, II-F, III-A, III-B, III-C, III-D, III-E, III-F, IV-A, IV-B, IV-C, IV-D, IV-E, IV-F, V-A, V-B, V-C, V-D, V-E, V-F, VI-A, VI-B, VI-C, VI-D, VI-E, VI-F, VII-A, VII-B, VII-C, VII-D, VII-E, VII-F, VIII-A, VIII-B, VIII-C, VIII-D, VIII-E, VIII-F, IX-A, IX-B, IX-C, IX-D, IX-E, IX-F, X-A, X-B, X-C, X-D, X-E, X-F, XI-A, XI-B, XI-C, XI-D, XI-E, XI-F, XII-A, XII-B, XII-C-, XII-D, XII-E, XII-F, XIII-A, XIII-B, XIII-C, XIII-D, XIII-E,** and **XIII-F** depicted below:

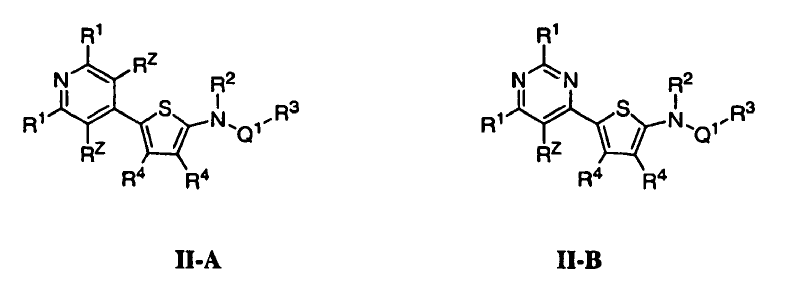

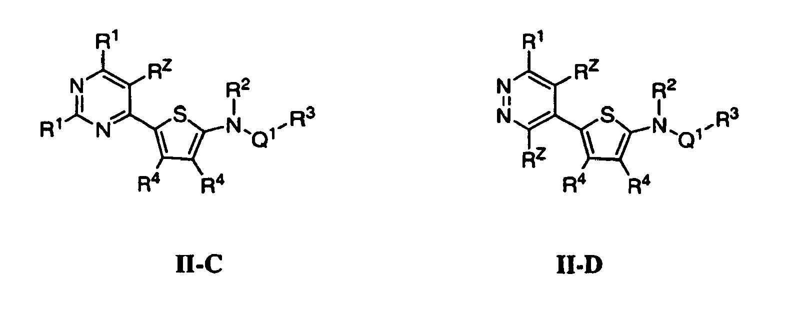

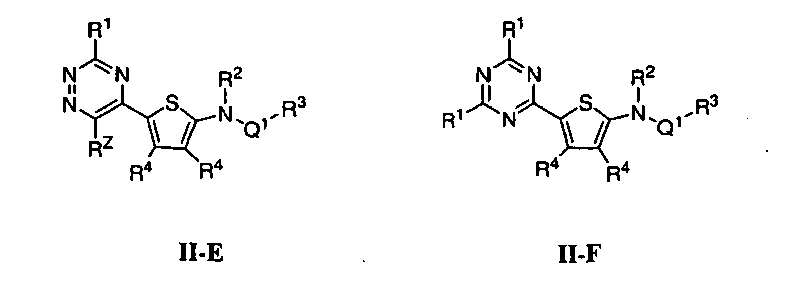

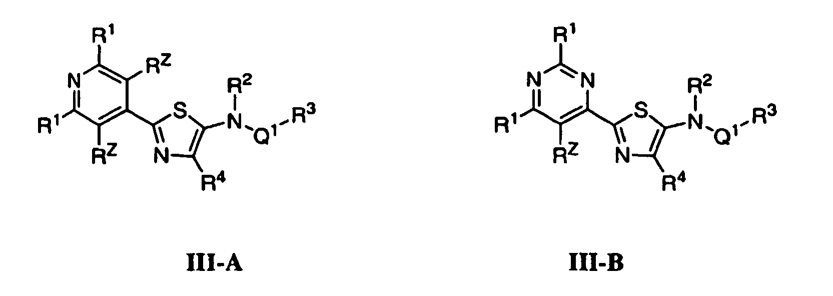

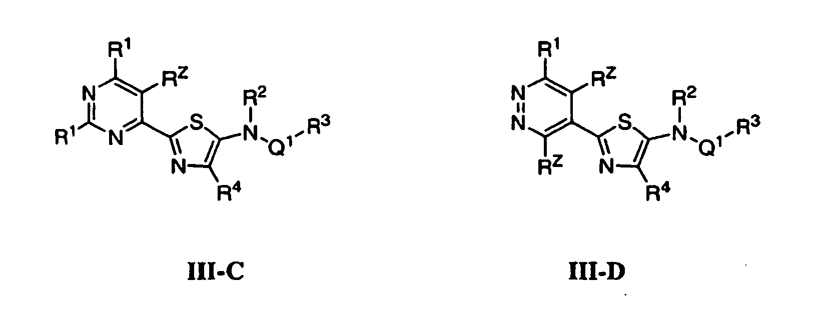

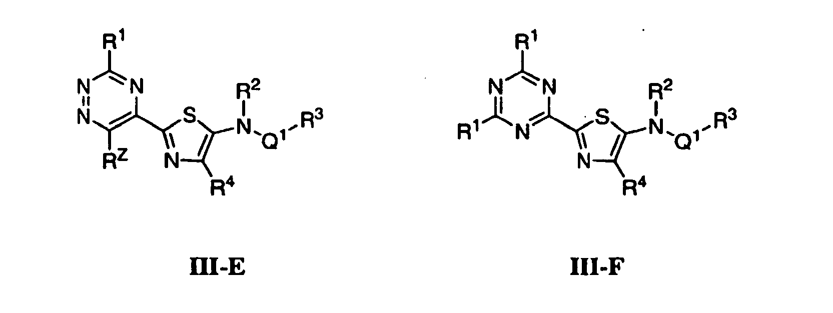

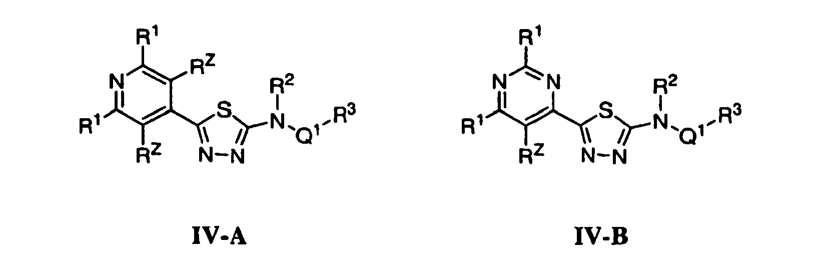

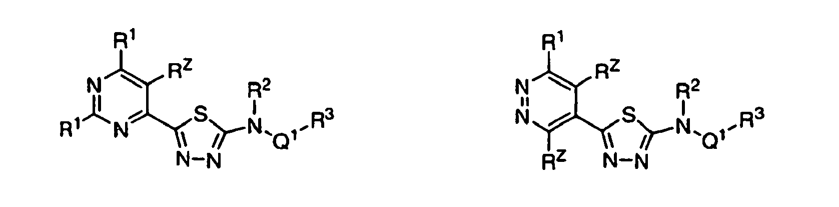

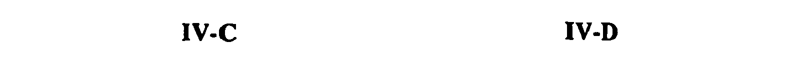

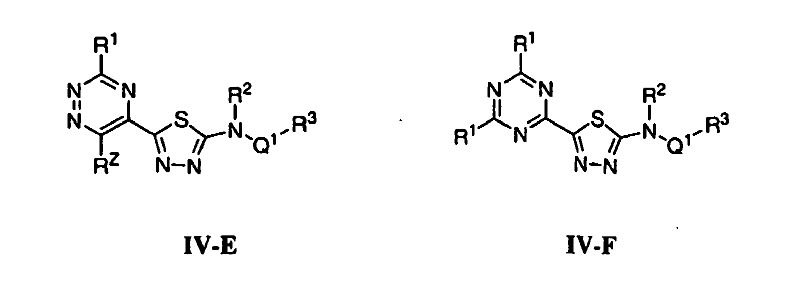

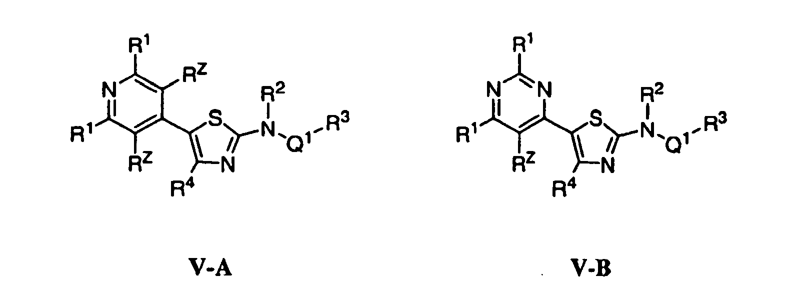

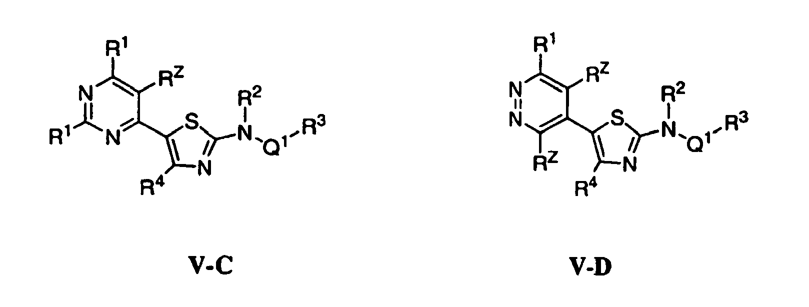

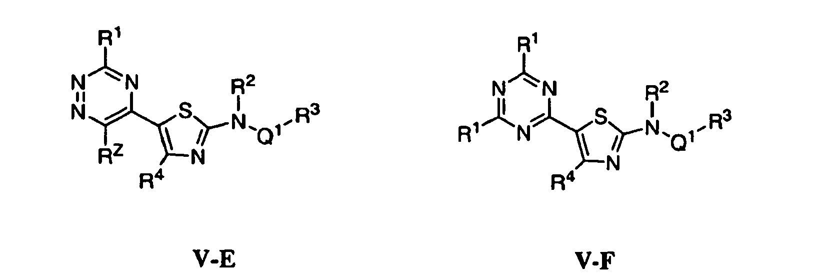

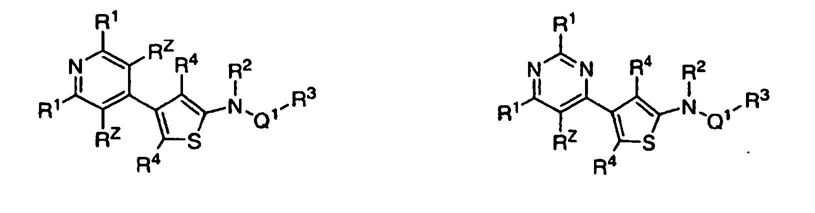

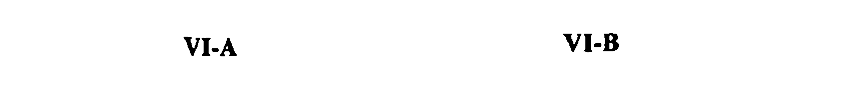

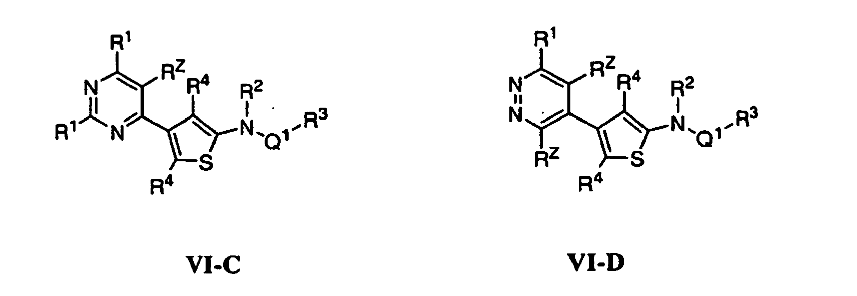

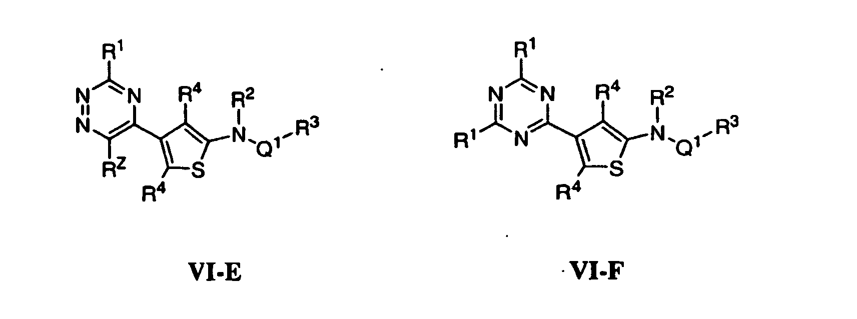

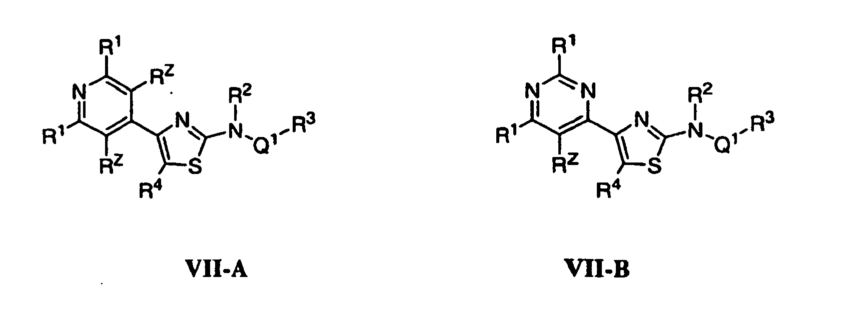

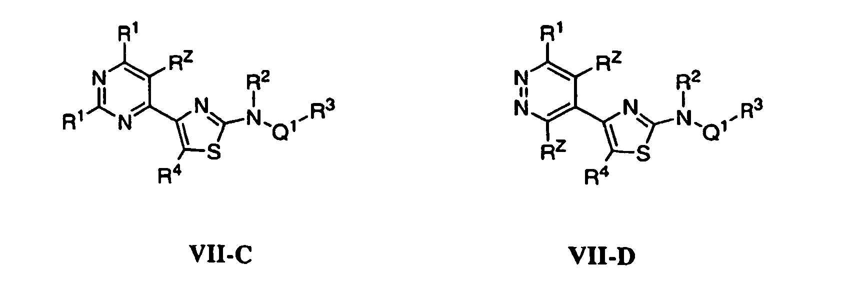

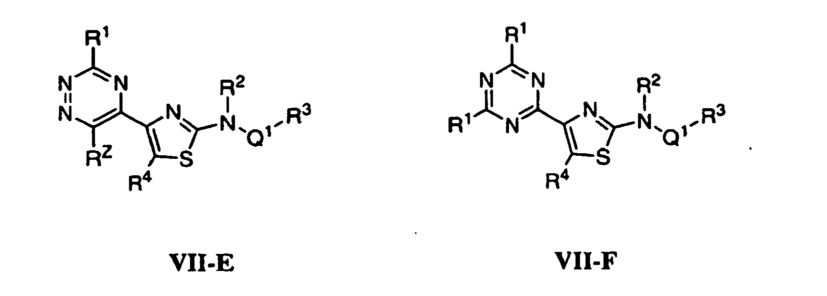

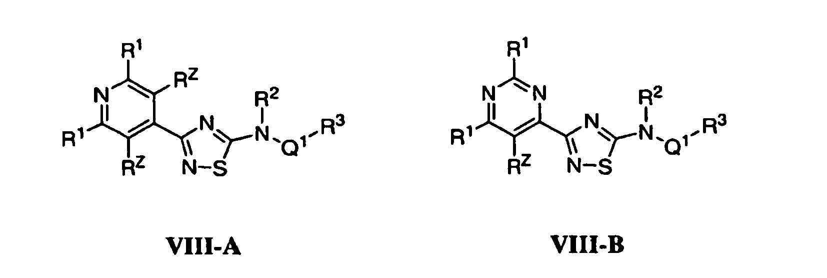

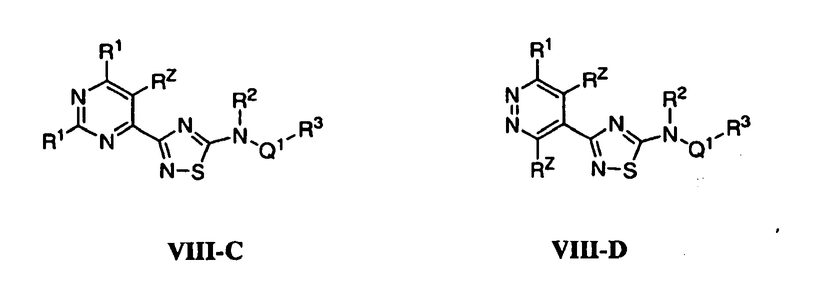

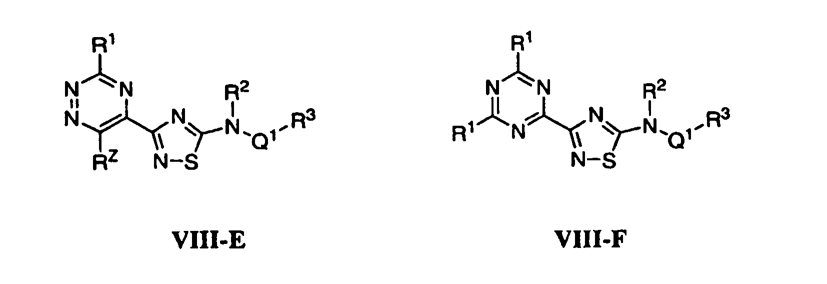

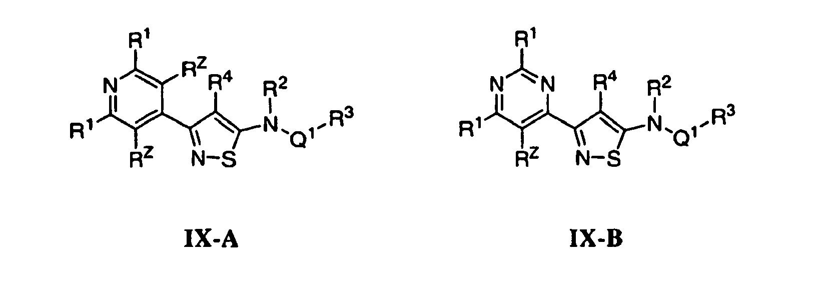

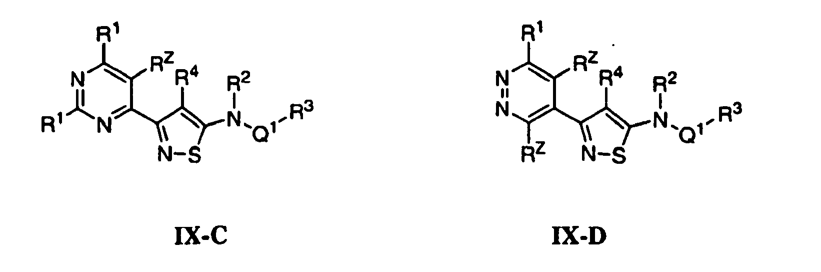

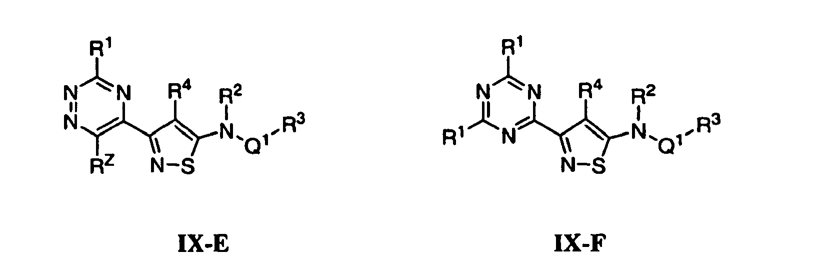

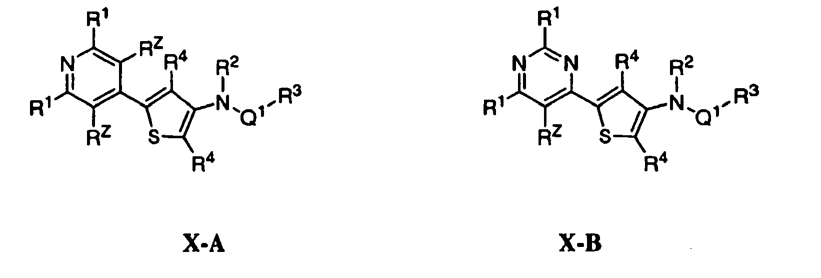

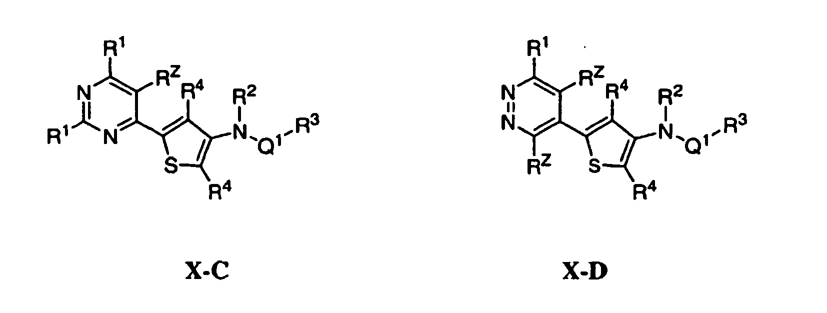

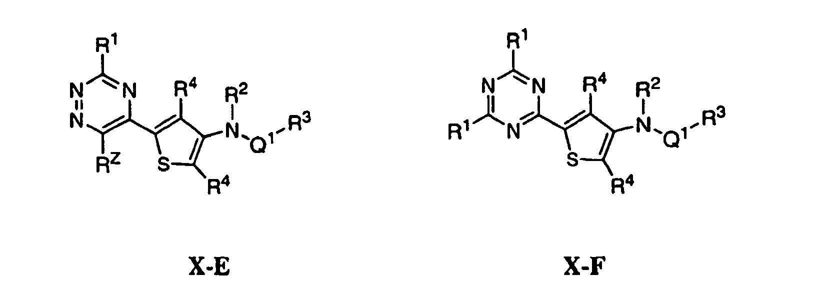

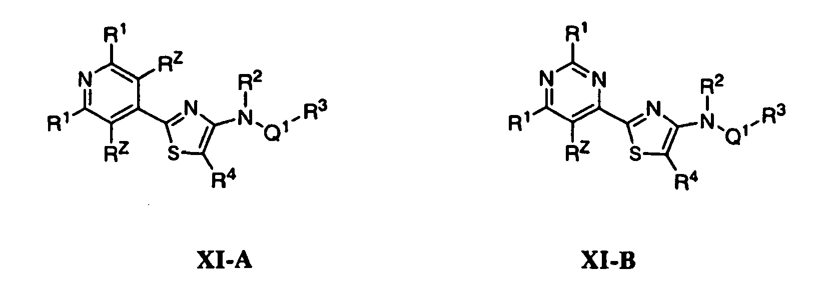

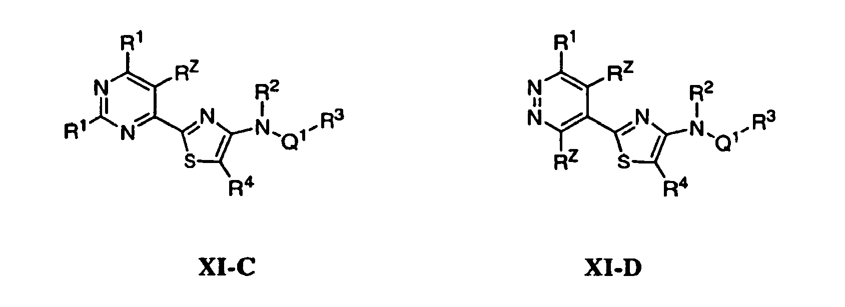

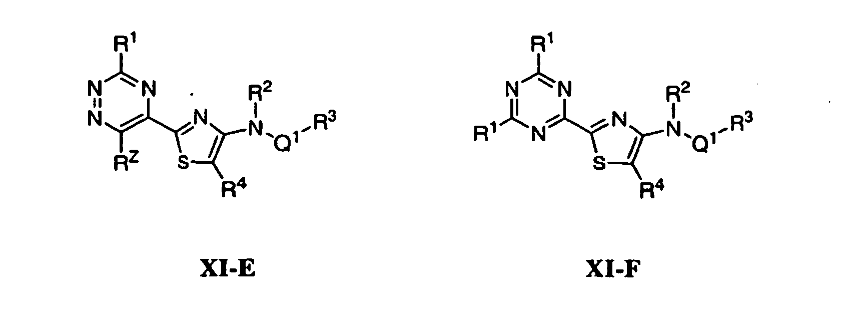

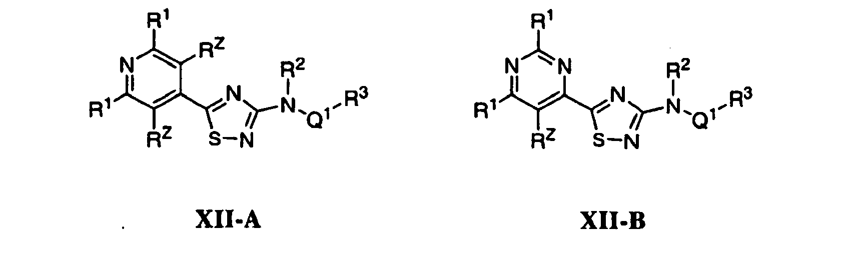

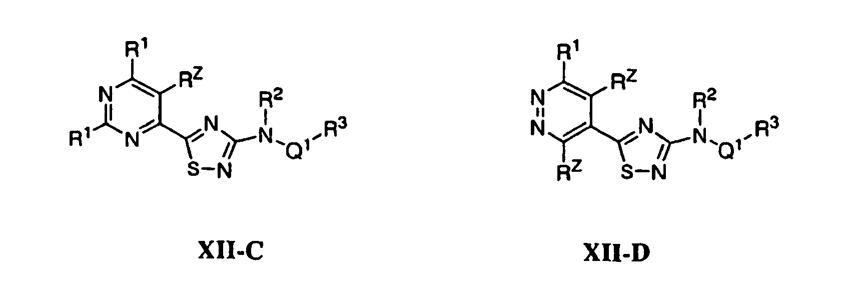

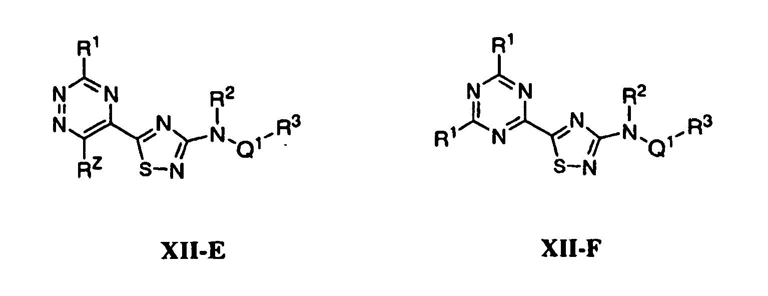

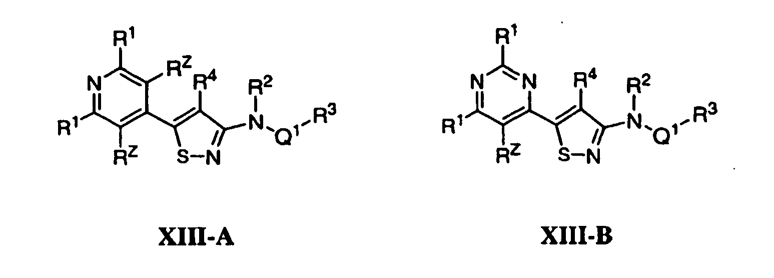

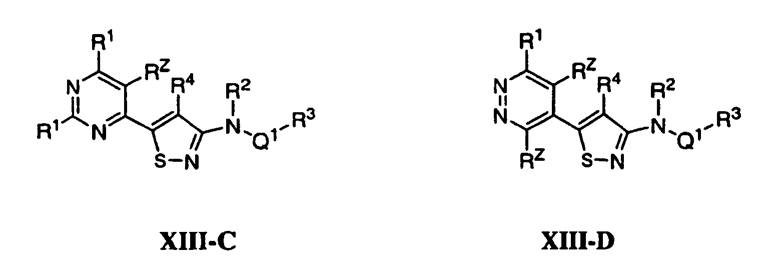

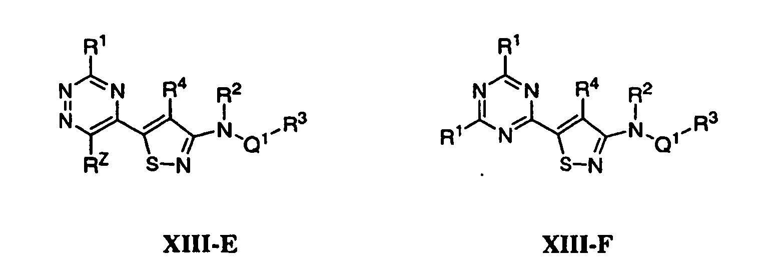

In general, for compounds of formulas **I, I-A-i, I-B-i, I-C-i, I-A-ii, I-B-ii,** and **I-C-ii,** (and subsets of formula **II-A, II-B, II-C, II-D, II-E, II-F, III-A, III-B, III-C, III-D, III-E, III-F, IV-A, IV-B, IV-C, IV-D, IV-E, IV-F, V.A, V-B, V-C, V-D, V-E, V-F, VI-A, VI-B, VI-C, VI-D, VI-E, VI-F, VII-A, VII-B, VII-C, VII-D, VII-E, VII-F, VIII-A, VIII-B, VIII-C, VIII-D, VIII-E, VIII-F, IX-A, IX-B, IX-C, IX-D, IX-E, IX-F, X-A, X-B, X-C, X-D, X-E, X-F, XI-A, XI-B, XI-C, XI-D, XI-E, XI-F, XII-A, XII-B, XII-C-, XII-D, XII-E, XII-F, XIII-A, XIII-B, XIII-C, XIII-D, XIII-E,** and **XIII-F)** each occurrence of R¹ is independently halogen, CN, NO₂, or VₘR, and each occurrence of R^{Z} is independently halogen, CN, NO₂, or UₙR'. In certain embodiments, R¹ groups are hydrogen, halogen, optionally substituted C₁-C₄aliphatic, OH, OR, SR, or N(R)₂. In other embodiments R¹ groups are hydrogen, halogen, -CH₃, -CH₂CH₃, -OH, -OCH₃, -SCH₃, -NH₂, -N(CH₃)₂, - N(CH₂CH₃)₂, NH(CH₂)₂NHCH₃, NH(cyclopropyl), NH(CH₂)cyclopropyl, or NH(CH₂)₂N(CH₃)₂. Exemplary R^{Z} groups are each independently hydrogen, halogen, C₁-C₄aliphatic, OH, OR', or N(R)(R'). In certain embodiments, R^{Z} groups are each independently hydrogen, halogen, Me, OH, OMe, NH₂, or N(CH₃)₂.

As described generally above, the thiadiazole, thiazole, thiophene, and isothiazole rings are each optionally substituted with zero, one or two occurrences of R⁴, as valency permits, wherein each occurrence of R⁴ is independently halogen, CN, NO₂, or VₘR. In some embodiments, R⁴ groups are each independently hydrogen, C₁₋₆aliphatic, -CN, -COR, - COOR, CON(R)₂, or halogen.

In certain embodiments, for thiophene compounds of general formula **II,** one occurrence of R⁴ is hydrogen and the other occurrence of R⁴ is CN and compounds have the general structure **II-a:**

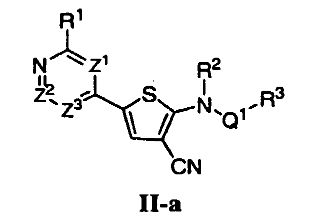

In yet other embodiments, for thiazole compounds of general formula **III,** R⁴ is hydrogen and compounds have the general structure **III-a:**

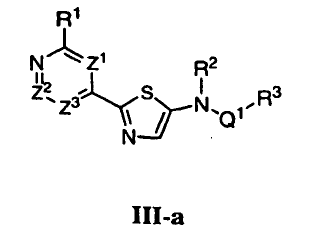

In certain embodiments, for thiophene compounds of general formula **VI,** one occurrence of R⁴ is hydrogen and the other occurrence of R⁴ is -COOR and compounds have the general structure **VI-a:**

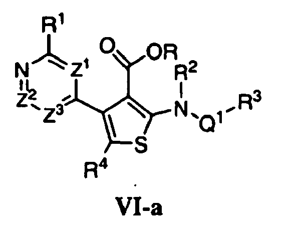

In yet other embodiments, for thiazole compounds of general formula VII, R⁴ is hydrogen and compounds have the general structure **VII-a:**

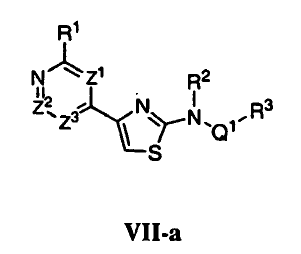

In certain other embodiments, for thiophene compounds of general formula **X**, one occurrence of R⁴ is hydrogen and the other occurrence of R⁴ is C(=O)OR and compounds have the general structure **X-a:**

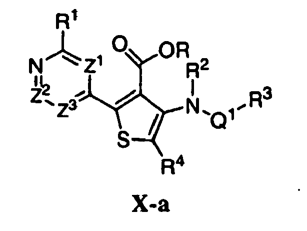

In yet other preferred embodiments, for thiazole compounds of general formula **XI,** R⁴ is hydrogen and compounds have the general structure **XI-a:**

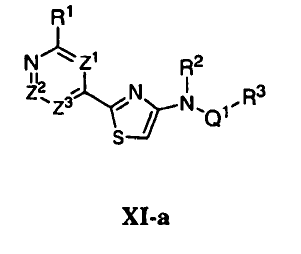

It will also be appreciated that for each of the above-described compounds **I,** and subsets of formula **II-A, II-B, II-C, II-D, II-E, II-F, III-A, III-B, III-C, III-D, III-E, III-F, IV-A, IV-B, IV-C. IV-D, IV-E, IV-F, V-A, V-B, V-C, V-D, V-E, V-F, VI-A, VI-B, VI-C, VI-D, VI-E, VI-F, VII-A, VII-B, VII-C, VII-D, VII-E, VII-F, VIII-A, VIII-B, VIII-C, VIII-D, VIII-E, VIII-F, IX-A, IX-B, IX-C, IX-D, IX-E, IX-F, X-A, X-B, X-C, X-D, X-E, X-F, XI-A, XI-B, XI-C, XI-D, XI-E, XI-F, XII-A, XII-B, XII-C-, XII-D, XII-E, XII-F, XIII-A, XIII-B, XIII-C, XIII-D, XIII-E, XIII-F, II-a, III-a, VI-a, VII-a, X-a,** and **XI-a,** in some embodiments R³ is Q²-Ar¹, wherein Q² and Ar¹ are described generally and in subsets above and herein. In other exemplary embodiments, for each of the above-described classes and subclasses of compounds, R² and Q¹-R³, taken together with the nitrogen atom, form the cyclic group: 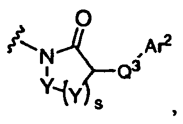 where s is 1 or 2, each occurrence of Y is independently, as valency and stability permit, -CO-, -CS-, -SO₂-, -O-, -S-, -NR⁵-, or -C(R⁵)₂-, and R⁵ is UₙR', wherein Q³, Ar², and R⁵ are described generally above and in classes and subclasses above and herein.

It will be appreciated that for compounds as described above, certain additional compounds are of special interest. For example, in certain exemplary embodiments, thiophene compounds are provided where Q¹ is -CO-, Q² is CHR⁶, q is 1 2, or 3, and compounds have one of formulas **XIV, XV**, or **XVI:**

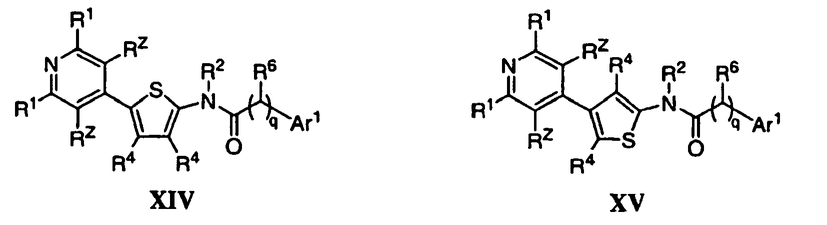

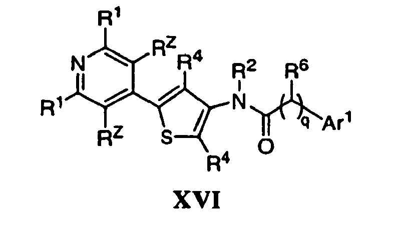

In other embodiments, thiazole compounds are provided where Q¹ is -CO-, Q² is CHR⁶, q is 1, 2 or 3, and compounds have one of formulas XVII, XVIII, or **XIX:**

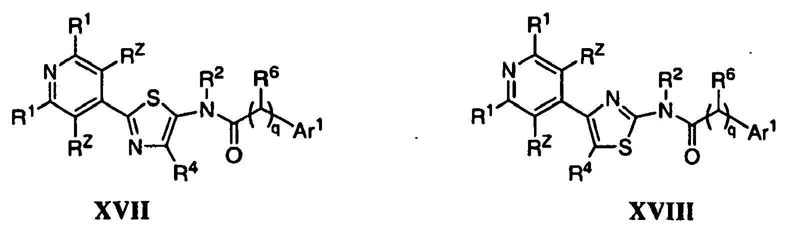

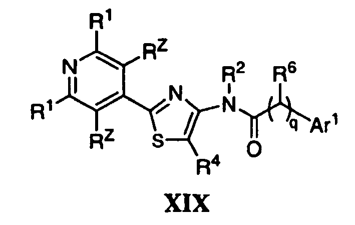

In certain embodiments, for compounds of formulas **XIV, XV, XVI, XVII, XVIII,** or **XIX,** compound variables are selected from one of more of the following groups:
a) each occurrence of R¹ is independently hydrogen, halogen, optionally substituted C₁-C₄aliphatic, OR, SR, or N(R)₂;
b) each occurrence of R¹ is independently hydrogen, halogen, -CH₃, -CH₂CH₃, -OH, - OCH₃, -SCH₃, -NH₂, -N(CH₃)₂, -N(CH₂CH₃)₂, NH(CH₂)₂NHCH₃, NH(cyclopropyl), NH(CH₂)cyclopropyl, or NH(CH₂)₂N(CH₃)₂;
c) each occurrence of R^{Z} is independently hydrogen, halogen, optionally substituted C₁-C₄aliphatic, OH, O(R'), or N(R)(R');
d) each occurrence of R^{Z} is independently hydrogen, halogen, Me, OH, OMe, NH₂, or N(Me)₂;
e) R² is hydrogen, or is UₙR', where n is 1, and U is-CH₂-, -CH₂CH₂-, -CH₂CH₂CH₂-, -CH₂CH₂CH₂CH₂-, -CH₂O-, -CH₂S-, -CH₂NR-, -CH₂CH₂O-, -CH₂CH₂S-, -CH₂CH₂NR-, -CH₂CH₂CH₂O-, -CH₂CH₂CH₂S-, -CH₂CH₂CH₂NR-, -CH₂CH₂CH₂CH₂O-, -CH₂CH₂CH₂CH₂S-, -CH₂CH₂CH₂CH₂NR-, -CH₂CH₂OCH₂CH₂-, -(CH₂)₄NHCH₂-, -(CH₂)₃NHCH₂CH₂-, or -CH₂CH₂NHCH₂CH₂-, and R' groups are hydrogen, C₁-C₄alkyl, optionally substituted tetrahydropyranyl, pyrrolidinyl, piperidinyl, piperazinyl, morpholinyl, thiomorpholinyl, pyridinyl, phenyl, or cyclohexyl, or R and R', taken together with the nitrogen atom to which they are bound, form an optionally substituted 5- or 6-membered heterocyclyl ring;
f) each occurrence of R⁴ is independently hydrogen, C₁₋₆aliphatic, CN, COR, COOR, CON(R)₂, or halogen;
g) q is 1, 2, or 3;
h) R⁶ is R', -N(R)(R'), -(CH₂)₁₋₄N(R)(R'), -OR', -(CH₂)₁₋₄OR', -NR(CH₂)₁₋₄N(R)(R'), -NR(CH₂)₁₋₄SO₂R', -NR(CH₂)₁₋₄COOR', or -NR(CH₂)₁₋₄COR', or two occurrences of R⁶, taken together with the atoms to which they are bound, form an optionally substituted 3-6-membered saturated, partially unsaturated, or fully unsaturated ring;
i) R⁶ is CH₂OH, CH₂CH₂OH, OH, OMe, OEt, NH₂, NH(Me), NH(Et), N(Me)(Me), CH₂NH₂, CH₂CH₂NH₂, NHCO₂*t*-butyl, phenyl, cyclopentyl, methyl, ethyl, isopropyl, cyclopropyl, NH(CH₂)₃NH₂, NH(CH₂)₂NH₂, NH(CH₂)₂NHEt, NHCH₂pyridyl, NHSO₂phenyl, NHC(O)CH₂C(O)O*t*-butyl, NHC(O)CH₂NH₃, and NHCH₂-imidazol-4-yl;
j) Ar¹ is ring **a, b, e, g, h, i, j, k, r, cc, dd, ff, jj, ll,** or pp, wherein t is 0, 1, 2, or 3, and T is a bond or is an optionally substituted C₁₋₆ alkylidene chain wherein one or two methylene units are optionally and independently replaced by -0-, -NR-, -S-, -SO₂-,-COO-, - CO-, -OSO₂-, -NRSO₂, -CONR-, or -SO₂NR-, and R⁷ is R' or halogen; or
k) Ar¹ is ring **a, b, e, g, h, i, j, k, r, cc, dd, ff, jj, ll,** or **pp,** wherein t is 0, 1, 2, or 3, and each occurrence of TR⁷ is independently -C₁₋₃alkyl, -OR', -SR', -CF₃, -OCF₃, -SCF₃, -F, -Cl, I, -Br, -COOR', -COR', -O(CH₂)₄N(R)(R'), -O(CH₂)₃N(R)(R'), -O(CH₂)₂N(R)(R'), - O(CH₂)N(R)(R'), -C(O)N(R)(R'), -(CH₂)₄OR', -(CH₂)₃OR', -(CH₂)₂OR', -CH₂OR', optionally substituted phenyl or benzyl, -N(R)(R'), -(CH₂)₄N(R)(R'), -(CH₂)₃N(R)(R'), - (CH₂)₂N(R)(R'), -(CH₂)N(R)(R'), or SO₂N(R)(R'), NRSO₂R', CON(R)(R'), or -OSO₂R'.

In other embodiments, for the thiophene and thiazole compounds of formulas **XIV** through **XIX,** q is l, and Ar¹ is optionally substituted phenyl and compounds of general formula **XIV-A** through **XIX-A** are provided: 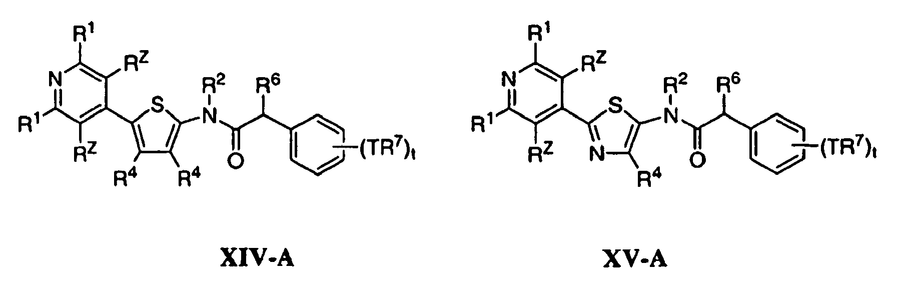 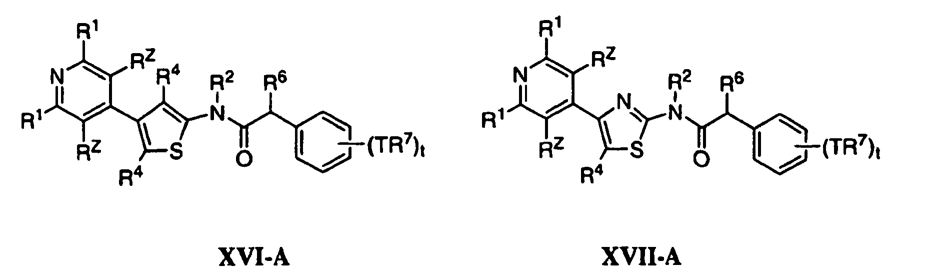 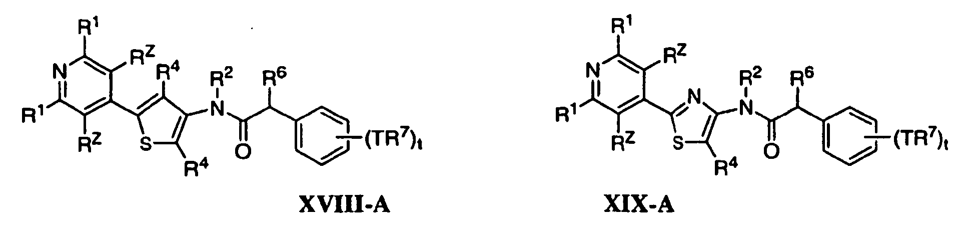 wherein R¹, R^{Z}, R², R⁴, R⁶, T, R⁷ and t are as defined generally and in classes and subclasses above and herein.

In preferred embodiments, for compounds of formula **XIV-A** through **XIX-A:**
each occurrence of R¹ is hydrogen;
each occurrence of R^{Z} is hydrogen;
R² is hydrogen, or is UₙR', where n is 1, and U is-CH₂-, -CH₂CH₂-, -CH₂CH₂CH₂-, -CH₂CH₂CH₂CH₂-, -CH₂O-, -CH₂S-, -CH₂NR-, -CH₂CH₂O-, -CH₂CH₂S-, -CH₂CH₂NR-, -CH₂CH₂CH₂O-, -CH₂CH₂CH₂S-, -CH₂CH₂CH₂NR-, -CH₂CH₂CH₂CH₂O-, -CH₂CH₂CH₂CH₂S-, -CH₂CH₂CH₂CH₂NR-, -CH₂CH₂OCH₂CH₂-, -(CH₂)₄NHCH₂-, -(CH₂)₃NHCH₂CH₂-, or -CH₂CH₂NHCH₂CH₂-, and R' groups are hydrogen, C₁-C₄alkyl, optionally substituted tetrahydropyranyl, pyrrolidinyl, piperidinyl, piperazinyl, morpholinyl, thiomorpholinyl, pyridinyl, phenyl, or cyclohexyl, or R and R', taken together with the nitrogen atom to which they are bound, form an optionally substituted 5- or 6-membered heterocyclyl ring;
each occurrence of R⁴ is independently hydrogen, C₁₋₆aliphatic, CN, COR, COOR, CON(R)₂, or halogen;
R⁶ is R', -N(R)(R'), -(CH₂)₁₋₄N(R)(R'), -OR', -(CH₂)₁₋₄OR', -NR(CH₂)₁₋₄N(R)(R'), - NR(CH₂)₁₋₄SO₂R', -NR(CH₂)₁₋₄COOR', or -NR(CH₂)₁₋₄COR'; and
t is 0, 1, 2, or 3, and each occurrence of TR⁷ is independently -C₁₋₃alkyl, -OR', -SR', -CF₃, -OCF₃, -SCF₃, -F, -Cl, I, -Br, -COOR', -COR', -O(CH₂)₄N(R)(R'), -O(CH₂)₃N(R)(R'), -O(CH₂)₂N(R)(R'), -O(CH₂)N(R)(R'), -O(CH₂)₄CON(R)(R'), -O(CH₂)₃CON(R)(R'), - O(CH₂)₂CON(R)(R'), -O(CH₂)CON(R)(R'), -C(O)N(R)(R'), -(CH₂)₄OR', -(CH₂)₃OR', - (CH₂)₂OR', -CH₂OR', optionally substituted phenyl or benzyl, -N(R)(R'), -(CH₂)₄N(R)(R'), - (CH₂)₃N(R)(R'), -(CH₂)₂N(R)(R'), -(CH₂)N(R)(R'), or SO₂N(R)(R'), NRSO₂R', CON(R)(R'), or -OSO₂R'.

Other subsets include those compounds where R² and Q'-R³, taken together with the atoms to which they are bound form a 5-membered cyclic group, and compounds have the general formula **XX** through **XXV:**

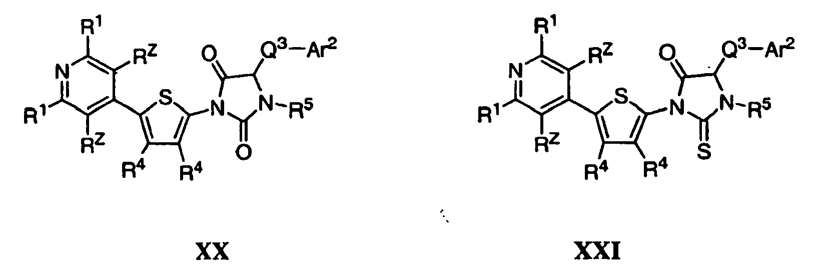

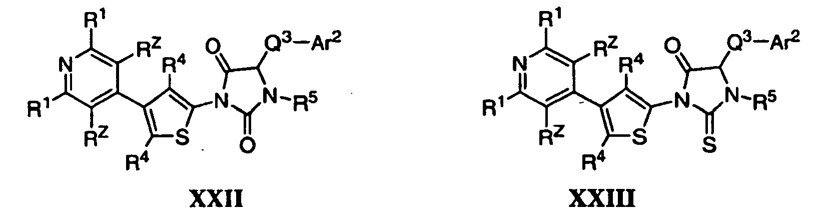

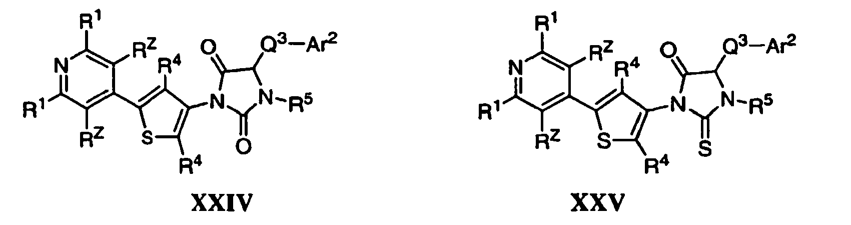

In other embodiments, thiazole compounds are provided where R² and Q¹-R³, taken together with the atoms to which they are bound form a 5-membered cyclic group, and compounds have the general formula **XXVI** through **XXXI:**

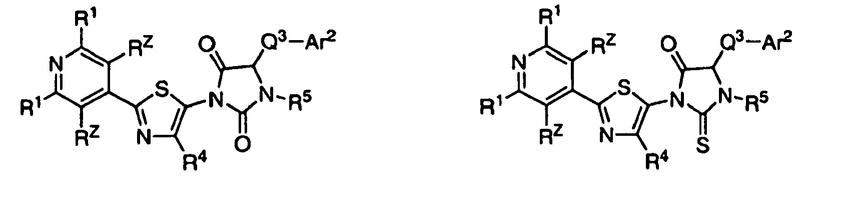

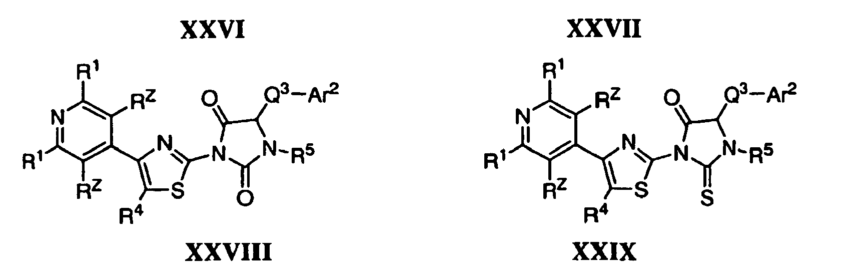

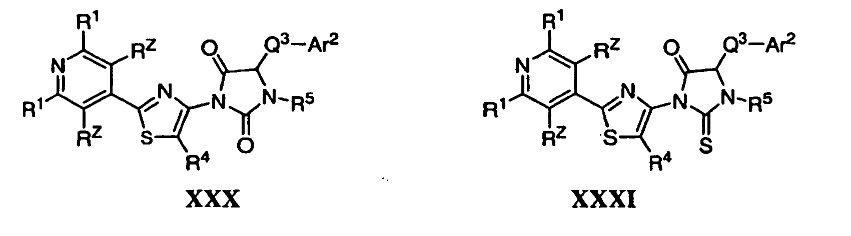

In still other embodiments, thiophene and thiazole compounds are provided where R² and Q¹-R³, taken together with the atoms to which they are bound form a 6-membered cyclic group, and compounds have the general formula **XXXII** through **XXXVII:** 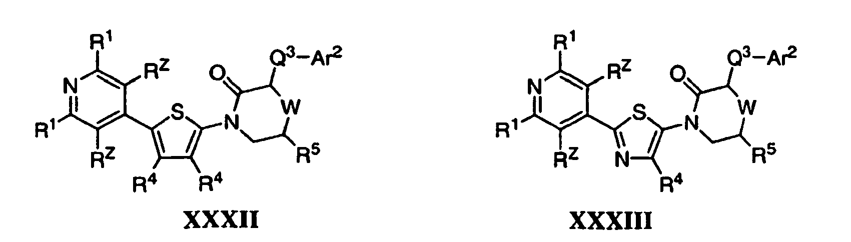 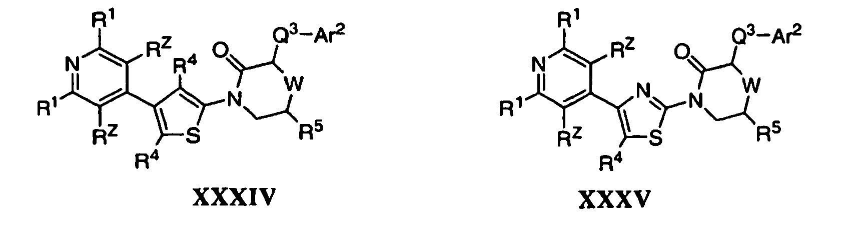 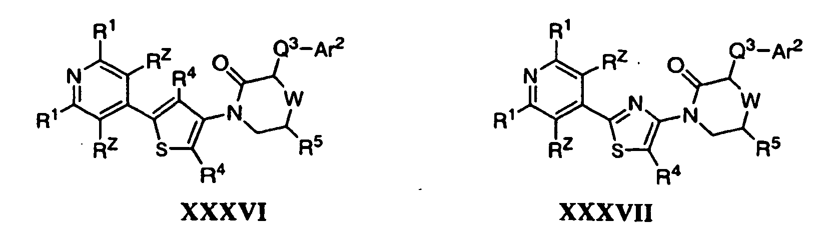 wherein W is O, NR⁵, or CHR⁵.

In certain embodiments, for compounds of formulas **XX** through **XXXVII** compound variables are selected from one of more of the following groups:
a) each occurrence of R¹ is hydrogen, halogen, optionally substituted C₁-C₄aliphatic, OR, SR, or N(R)₂;
b) each occurrence of R^{Z} is independently hydrogen, halogen, optionally substituted C₁-C₄aliphatic, OH, OR' or N(R)(R');
c) each occurrence of R⁴ is independently hydrogen, C₁₋₆aliphatic, CN, COR, COOR, CON(R)₂, or halogen;
d) R⁵ is hydrogen, (CH₂)₃OR', (CH₂)₂OR', (CH₂)OR', (CH₂)₃N(R')₂, (CH₂)₂N(R')₂, (CH₂)N(R')₂, or C₁₋₄aliphatic;
e) Q³ is a direct bond, or is -(CHR⁶)_{q}-, -(CHR⁶)_{q}O-, -(CHR⁶)_{q}S-, -(CHR⁶)_{q}S(O)₂-, - (CHR⁶)_{q}S(O)- , -(CHR⁶)_{q}NR-, or -(CHR⁶)_{q}C(O)-, wherein q is 0, 1, 2, or 3; and
f) Ar² is ring **a, b, e, g, h, i, j, k, n, r, cc, dd, ff, jj, ll,** or **pp,** wherein t is 0, 1, 2, or 3, and T is a bond or is an optionally substituted C₁₋₆ alkylidene chain wherein one or two methylene units are optionally and independently replaced by -0-, -NR-, -S-, -SO₂-,-COO-, - CO-, -OSO₂-, -NRSO₂, -CONR-, or -SO₂NR-, and R⁷ is R' or halogen.

In certain other embodiments, for compounds of formulas **XX** through **XXXVII** compound variables are selected from one of more of the following groups:
a) each occurrence of R¹ is independently hydrogen, halogen, -CH₃, -CH₂CH₃, -OH, - OCH₃, -SCH₃, -NH₂, -N(CH₃)₂, -N(CH₂CH₃)₂, NH(CH₂)₂NHCH₃, NH(cyclopropyl), NH(CH₂)cyclopropyl, or NH(CH₂)₂N(CH₃)₂;
b) each occurrence of R^{Z} is independently hydrogen, halogen, Me, OH, OMe, NH₂, or N(Me)₂;
c) each occurrence of R⁴ is independently hydrogen, C₁₋₆aliphatic, CN, COR, COOR, CON(R)₂, or halogen;
d) R⁵ is hydrogen, (CH₂)₃OR', (CH₂)₂OR', (CH₂)OR', (CH₂)₃N(R')₂, (CH₂)₂N(R')₂. (CH₂)N(R')₂, or C₁₋₄aliphatic;
e) Q³ is a direct bond, or is -(CHR⁶)_{q}-, -(CHR⁶)_{q}O-, -(CHR⁶)_{q}S-, -(CHR⁶)_{q}S(O)₂-, - (CHR⁶)_{q}S(O)- , -(CHR⁶)_{q}NR-, or -(CHR⁶)_{q}C(O)-, wherein q is 0, 1, 2, or 3; and
f) Ar² is ring **a, b, e, g, h, i, j, k, n, r, cc, dd, ff, jj, ll,** or **pp,** wherein t is 0, 1, 2, or 3, and each occurrence of TR⁷ is independently -C₁₋₃alkyl, -OR', -SR', -CF₃, -OCF₃, -SCF₃, -F, -Cl, I, -Br, -COOR', -COR', -O(CH₂)₄N(R)(R'), -O(CH₂)₃N(R)(R'), -O(CH₂)₂N(R)(R'), - O(CH₂)N(R)(R'), -O(CH₂)₄CON(R)(R'), -O(CH₂)₃CON(R)(R'), -O(CH₂)₂CON(R)(R'), - O(CH₂)CON(R)(R'), -C(O)N(R)(R'), -(CH₂)₄OR', -(CH₂)₃OR', -(CH₂)₂OR', -CH₂OR', optionally substituted phenyl or benzyl, -N(R)(R'), -(CH₂)₄N(R)(R'), -(CH₂)₃N(R)(R'), - (CH₂)₂N(R)(R'), -(CH₂)N(R)(R'), or SO₂N(R)(R'), NRSO₂R', CON(R)(R'), or -OSO₂R'.

In other embodiments, for the thiophene and thiazole compounds of formulas as described above, Ar² is optionally substituted phenyl and compounds of general formula **XX-A,** through **XXXVII** are provided: 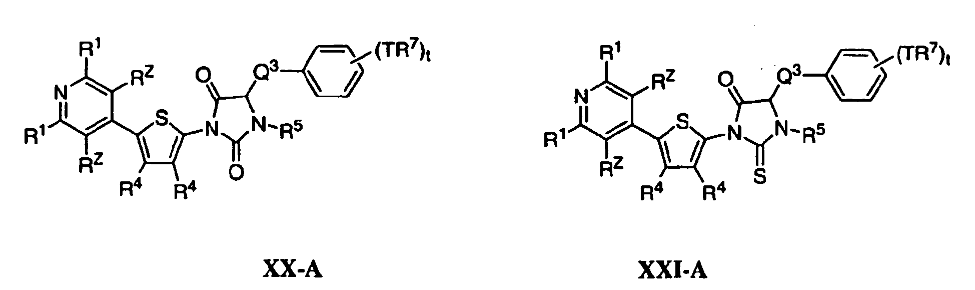 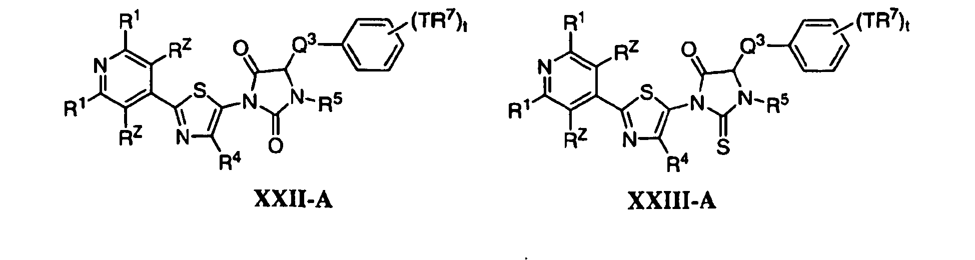 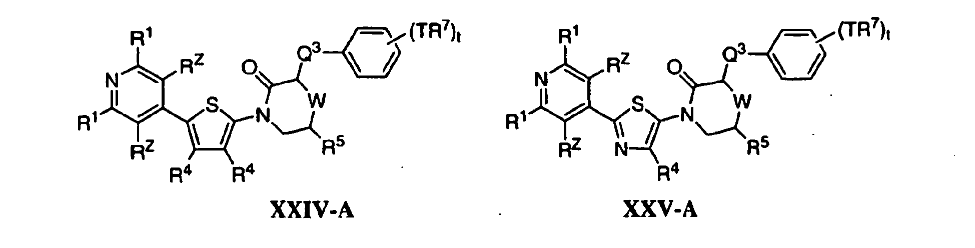 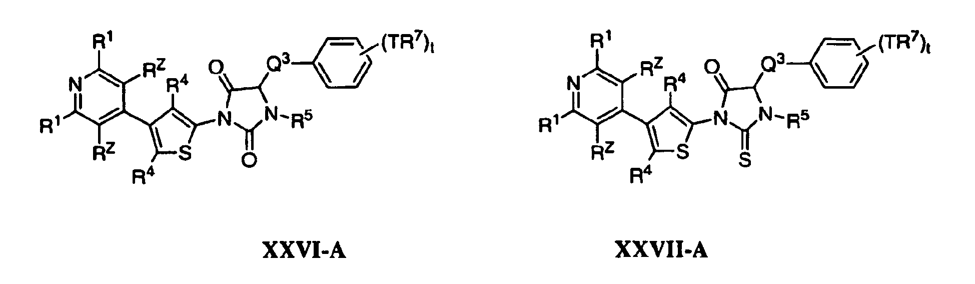 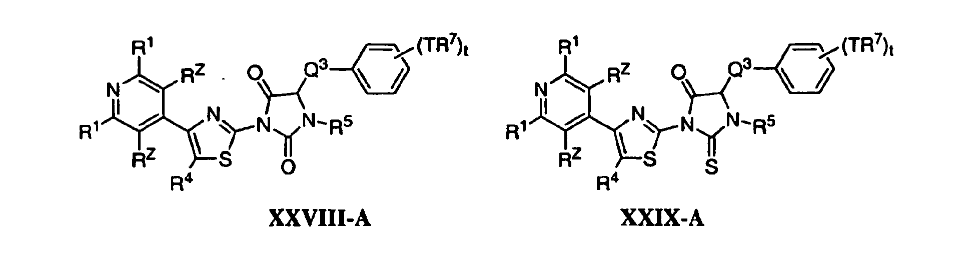 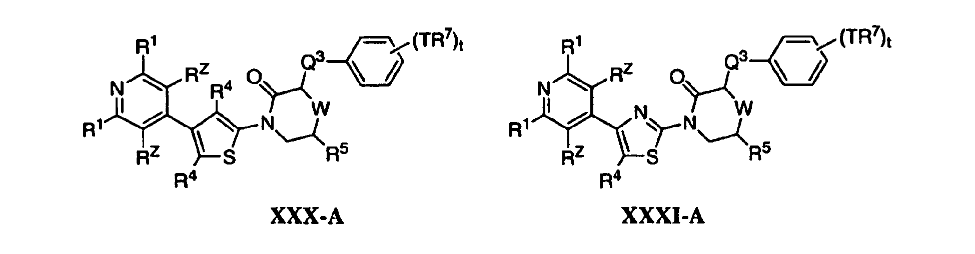 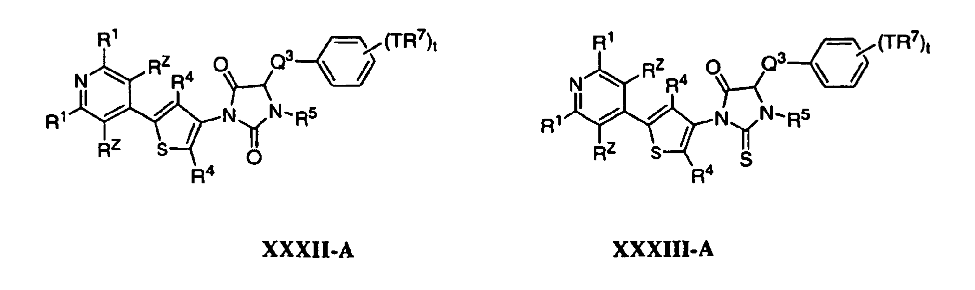 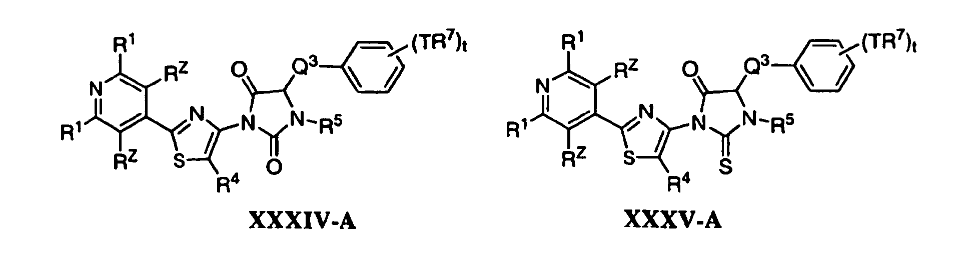 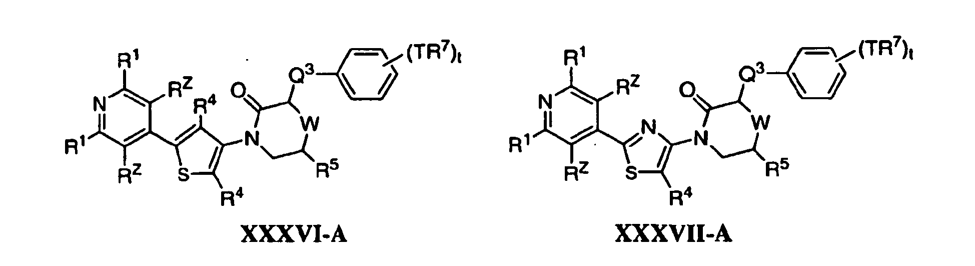 wherein R¹, R², R⁴, R⁵, Q³ T, R⁷, t, and W are as defined generally and in classes and subclasses above and herein.

In preferred embodiments, for compounds of formula **XX-A** through **XXXVII-A:**
each occurrence of R¹ is hydrogen;
each occurrence of R^{Z} is hydrogen;
each occurrence of R⁴ is independently hydrogen, C₁₋₆aliphatic, CN, COR, COOR, CON(R)₂, or halogen;
R⁵ is hydrogen, (CH₂)₃OR', (CH₂)₂OR', (CH₂)OR', (CH₂)₃N(R')₂, (CH₂)₂N(R')₂, (CH₂)N(R')₂, or C₁₋₄aliphatic;
Q³ is a direct bond, or is -(CHR⁶)_{q}-, -(CHR⁶)_{q}O-, -(CHR⁶)_{q}S-, -(CHR⁶)_{q}S(O)₂-, - (CHR⁶)_{q}S(O)- , -(CHR⁶)_{q}NR-, or -(CHR⁶)_{q}C(O)-, wherein q is 0, 1, 2, or 3; and
t is 0, 1, 2, or 3, and each occurrence of TR⁷ is independently -C₁₋₃alkyl, -OR', -SR', -CF₃, -OCF₃, -SCF₃, -F, -Cl, I, -Br, -COOR', -COR', -O(CH₂)₄N(R)(R'), -O(CH₂)₃N(R)(R'), -O(CH₂)₂N(R)(R'), -O(CH₂)N(R)(R'), -O(CH₂)₄CON(R)(R'), -O(CH₂)₃CON(R)(R'), - O(CH₂)₂CON(R)(R'), -O(CH₂)CON(R)(R'), -C(O)N(R)(R'), -(CH₂)₄OR', -(CH₂)₃OR', - (CH₂)₂OR', -CH₂OR', optionally substituted phenyl or benzyl, -N(R)(R'), -(CH₂)₄N(R)(R'), - (CH₂)₃N(R)(R'), -(CH₂)₂N(R)(R'), -(CH₂)N(R)(R'), or SO₂N(R)(R'), NRSO₂R', CON(R)(R'), or -OSO₂R'.

Representative examples of compounds of formula **I-A** are set forth below in Table 1 below.

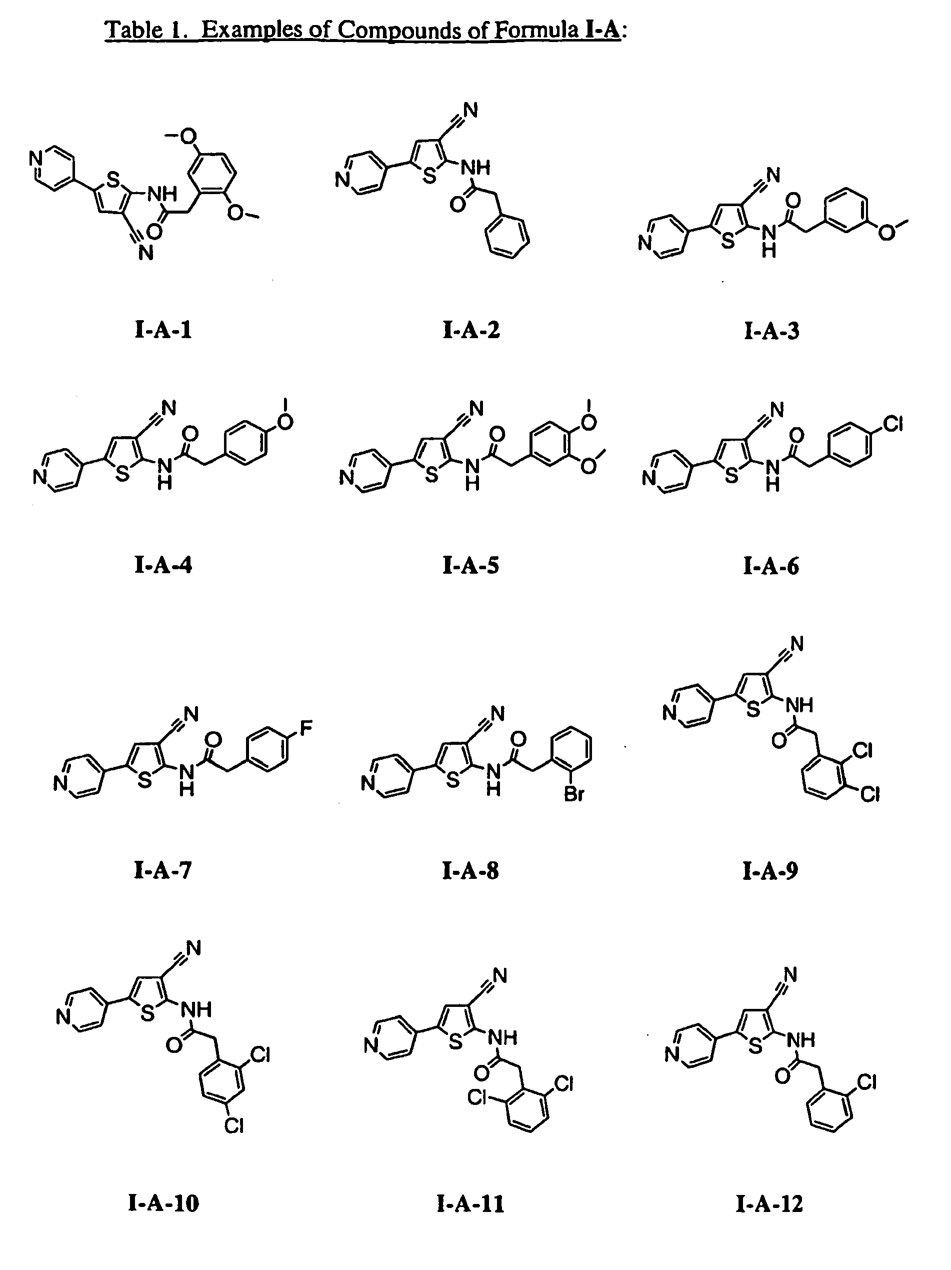

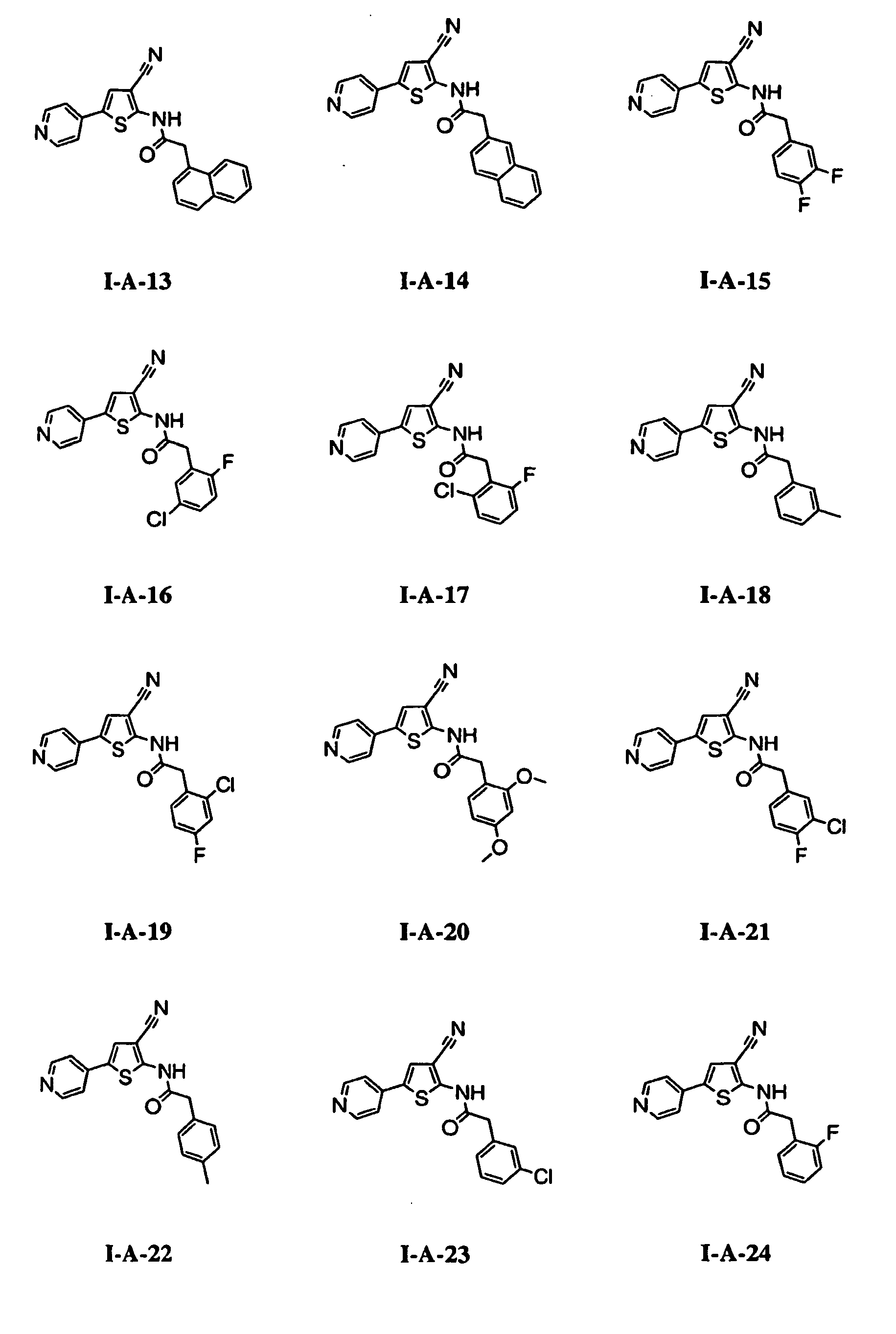

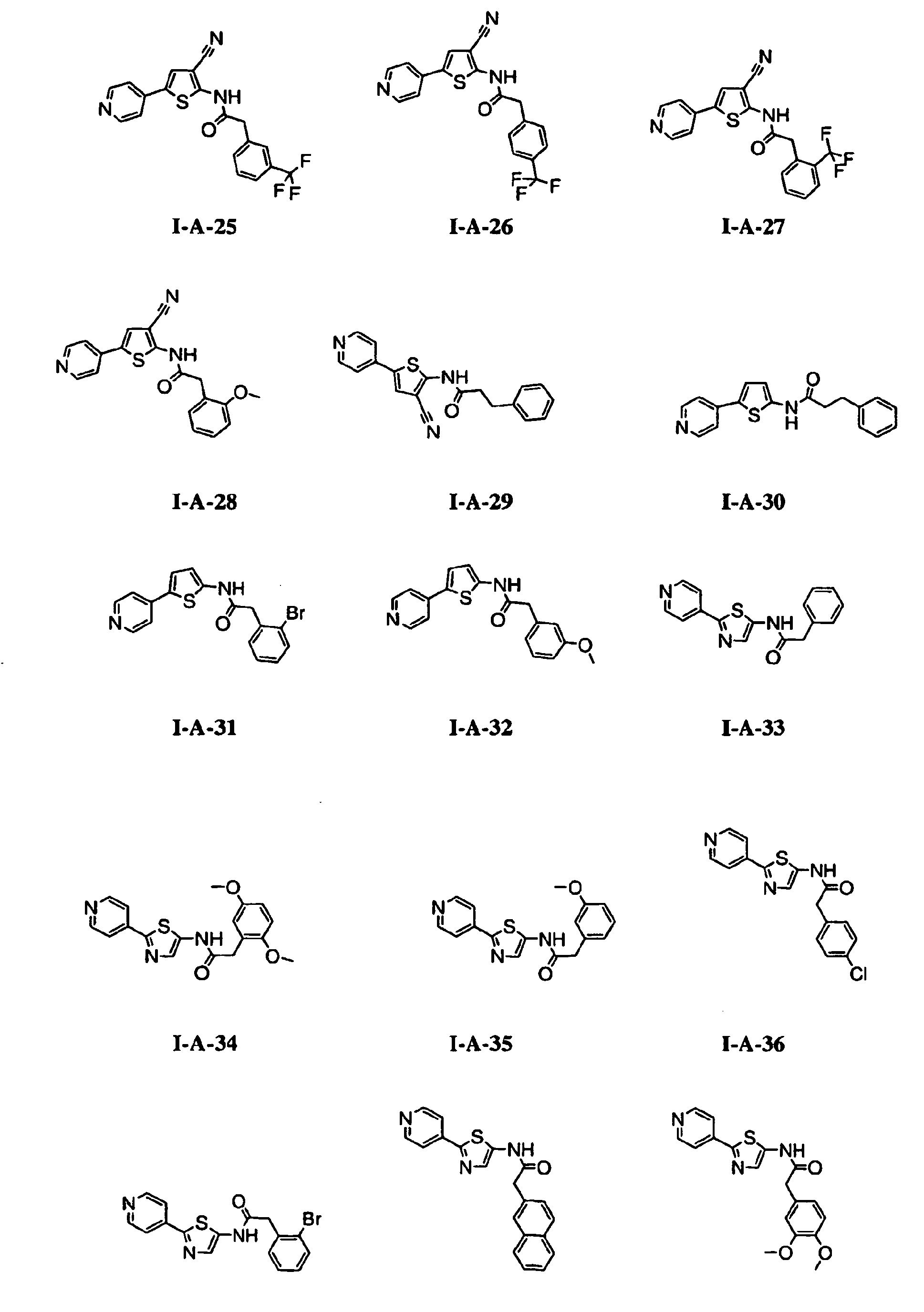

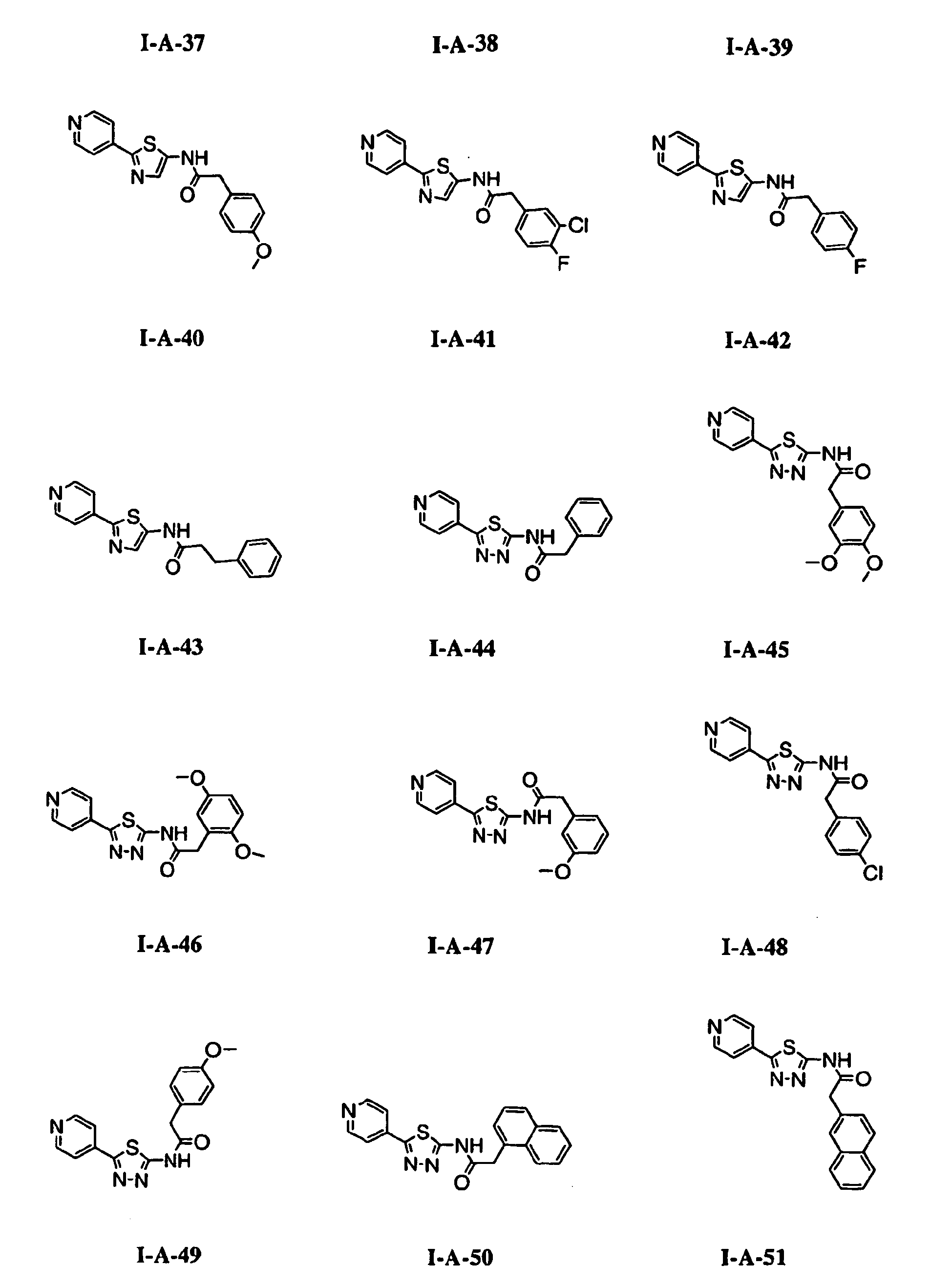

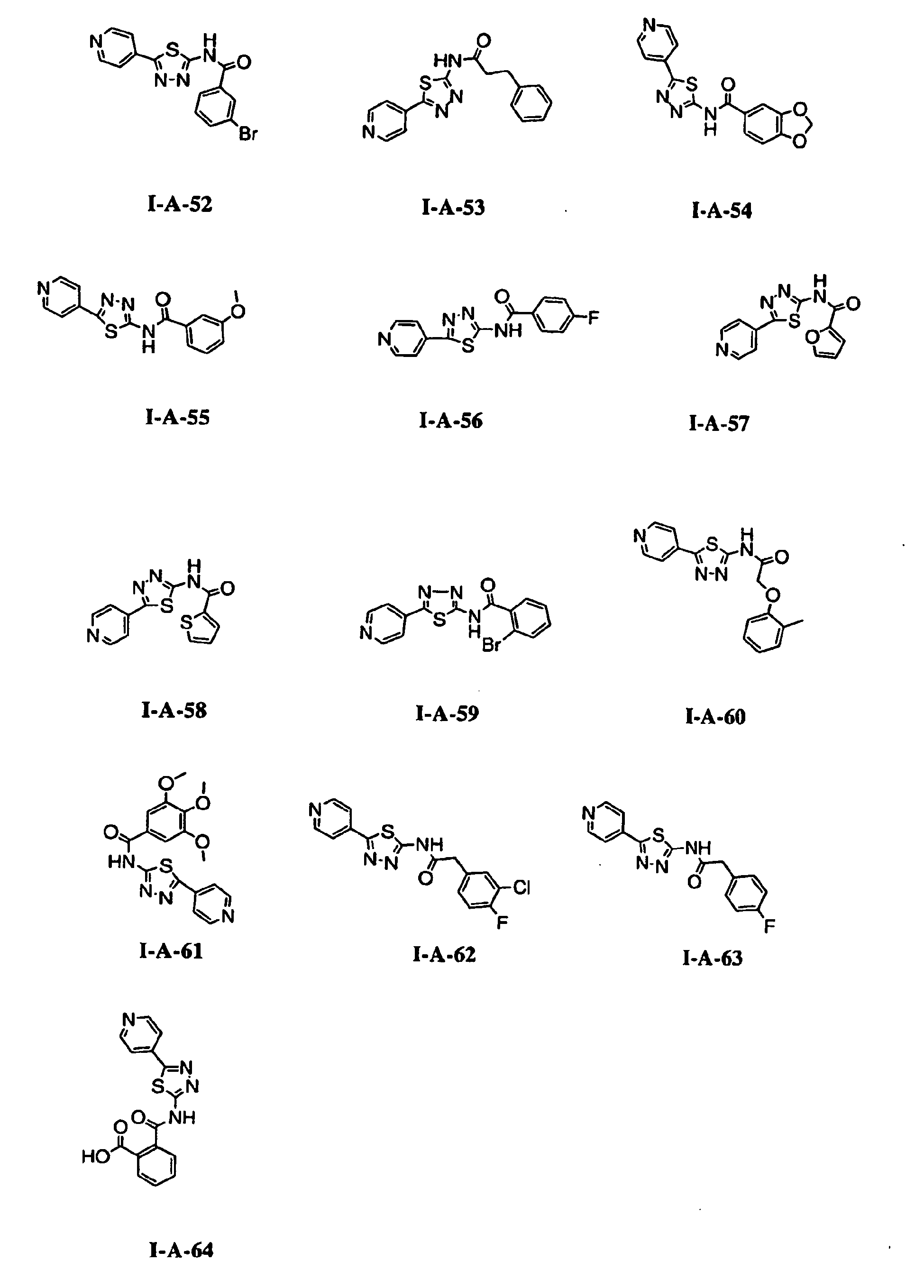

Representative examples of compounds of formula **I-B** are set forth below in Table 2 below.

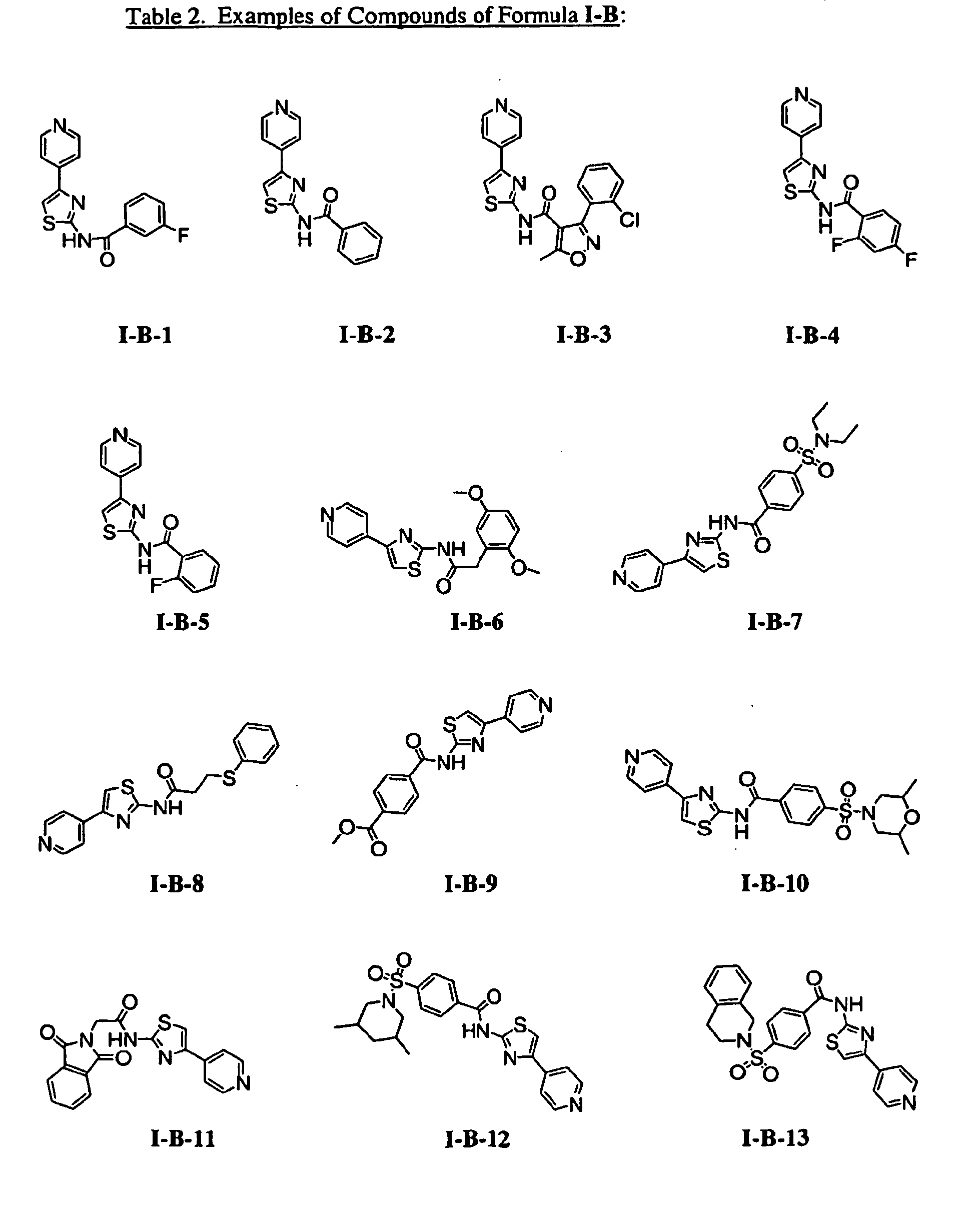

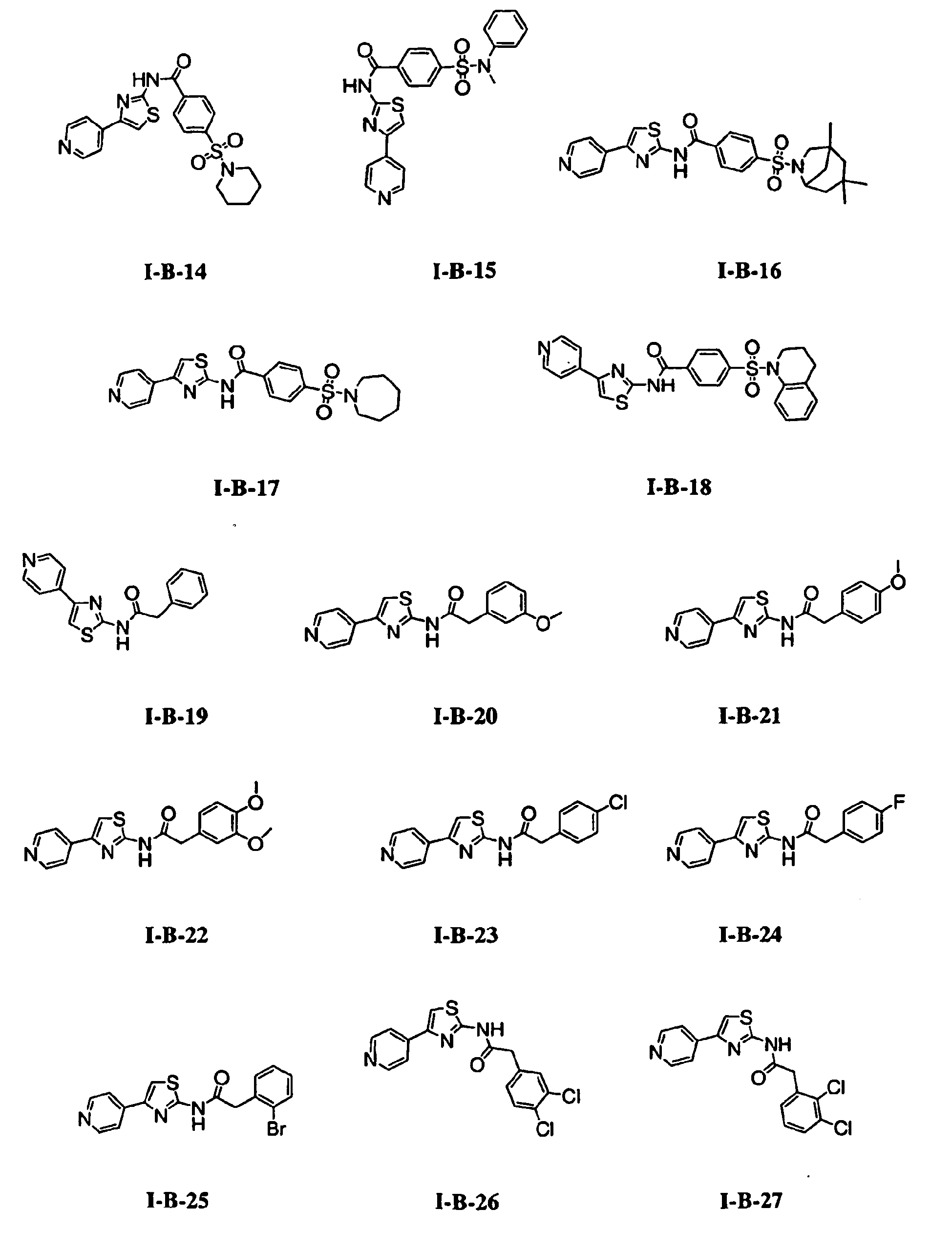

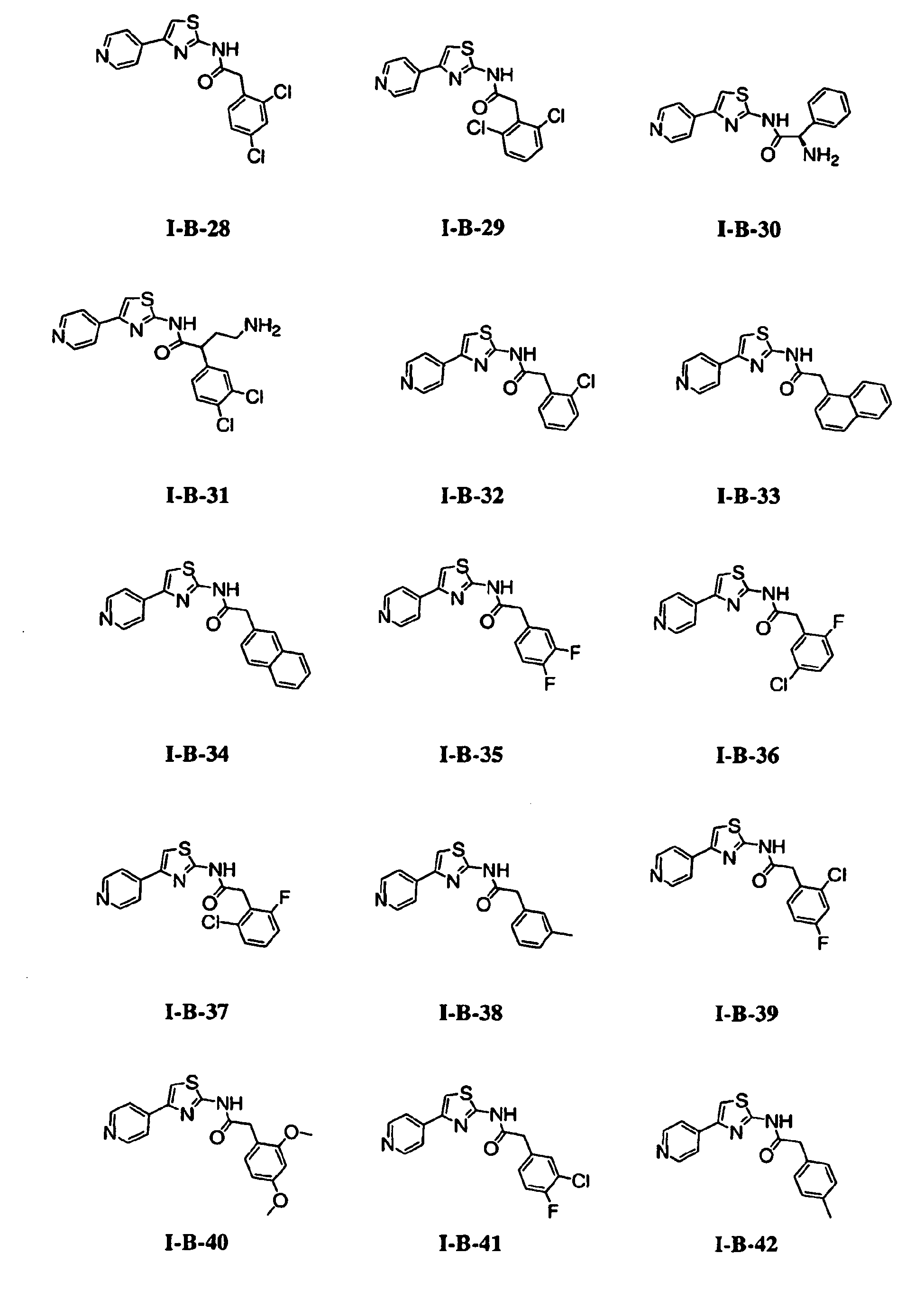

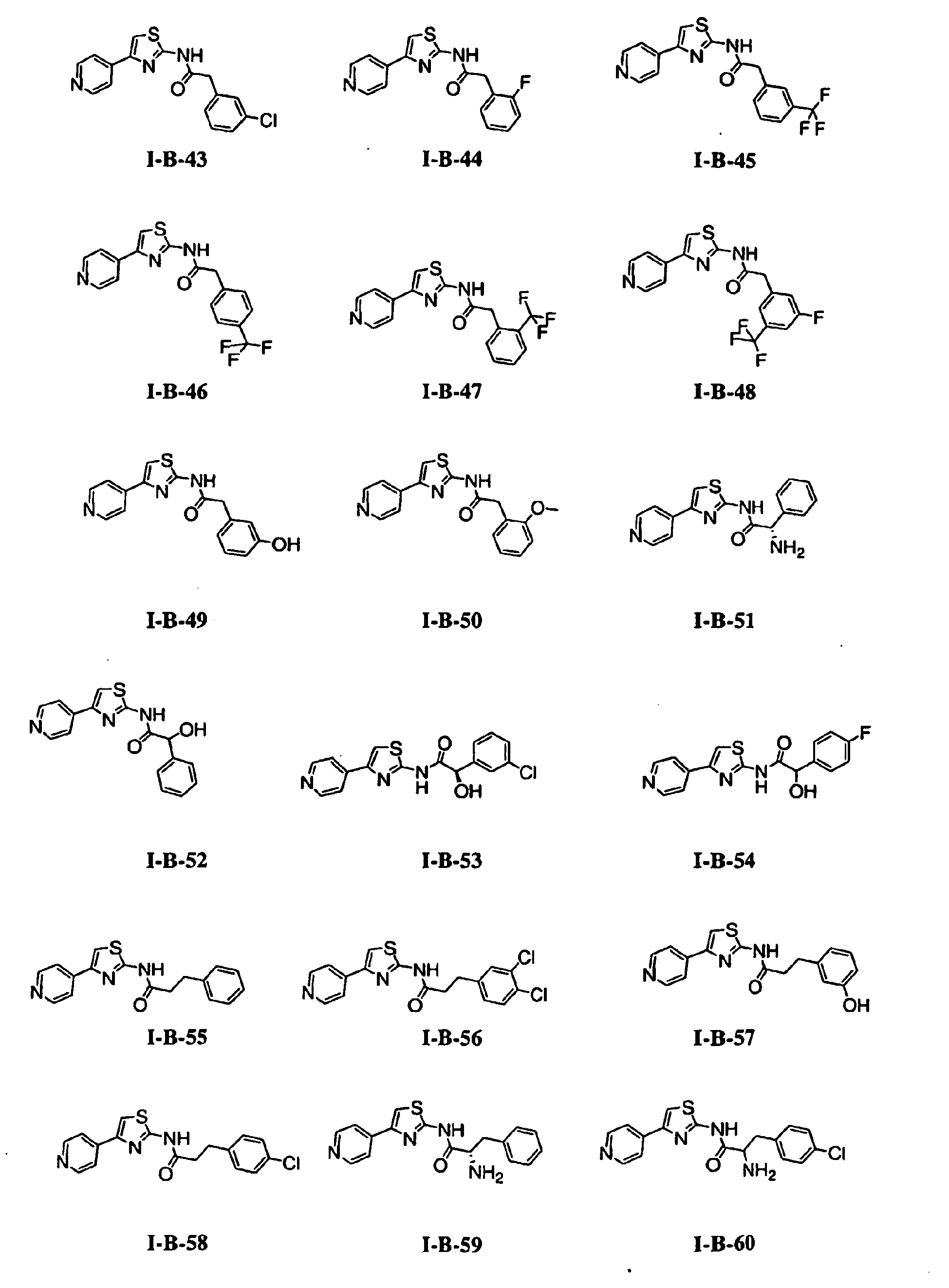

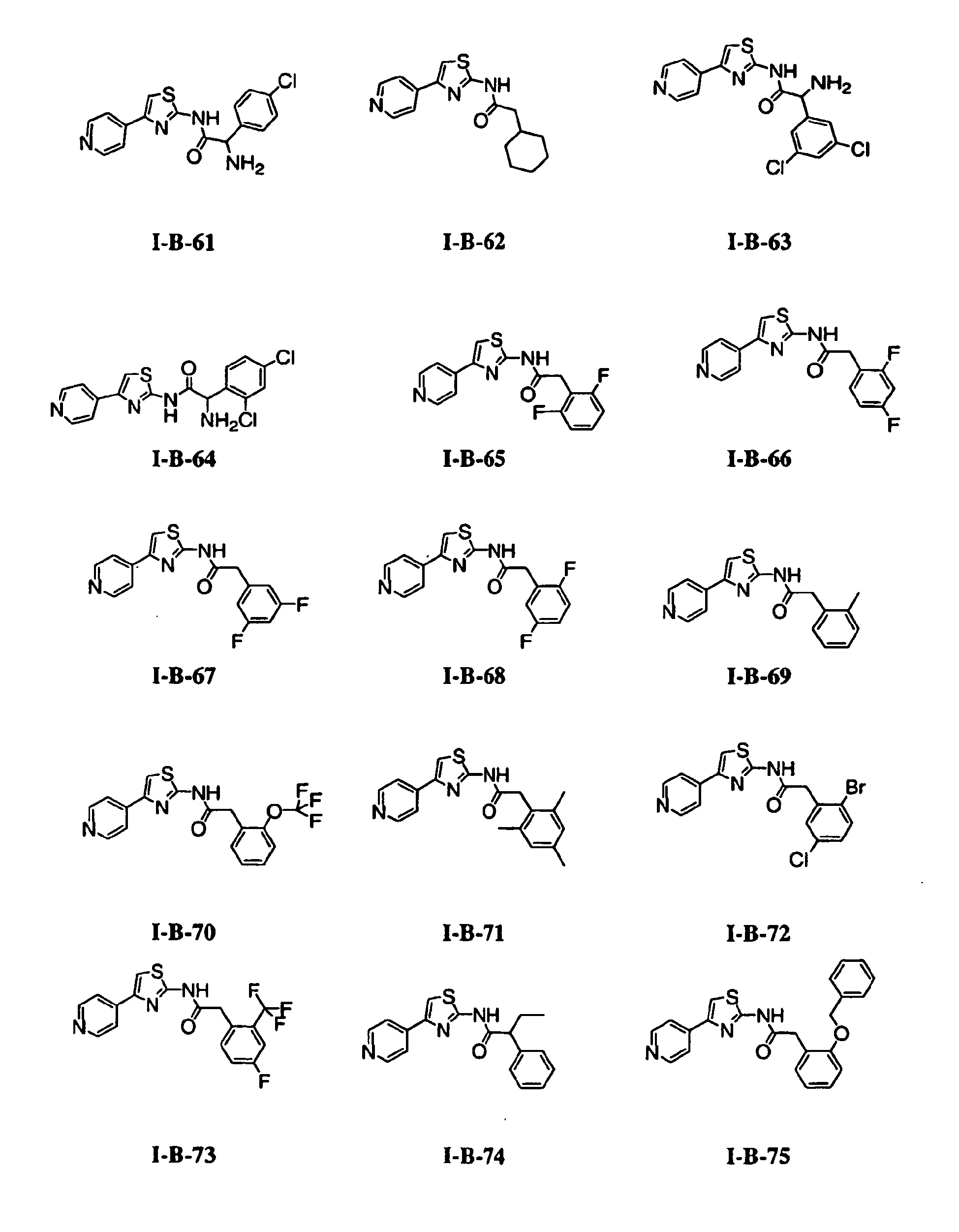

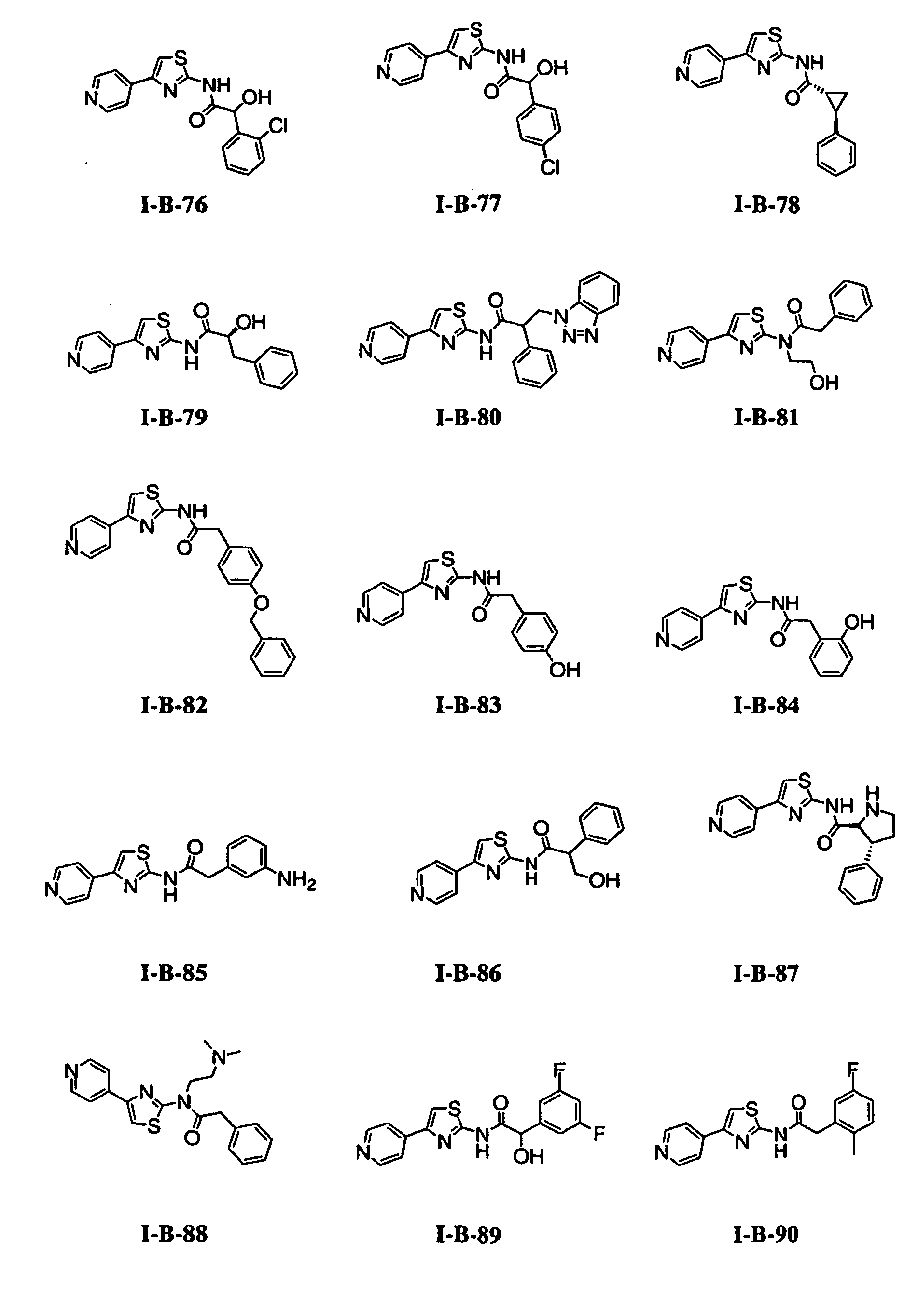

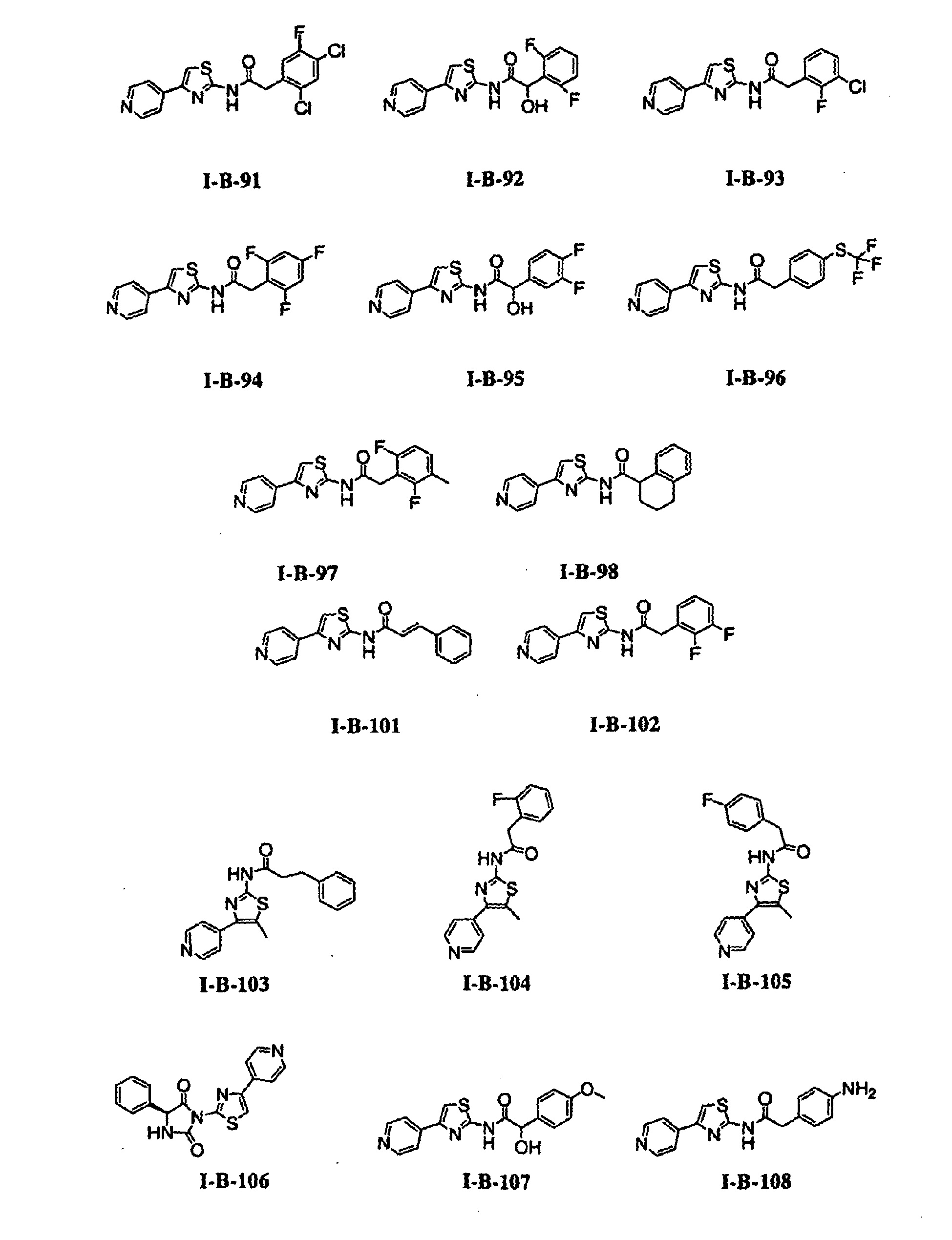

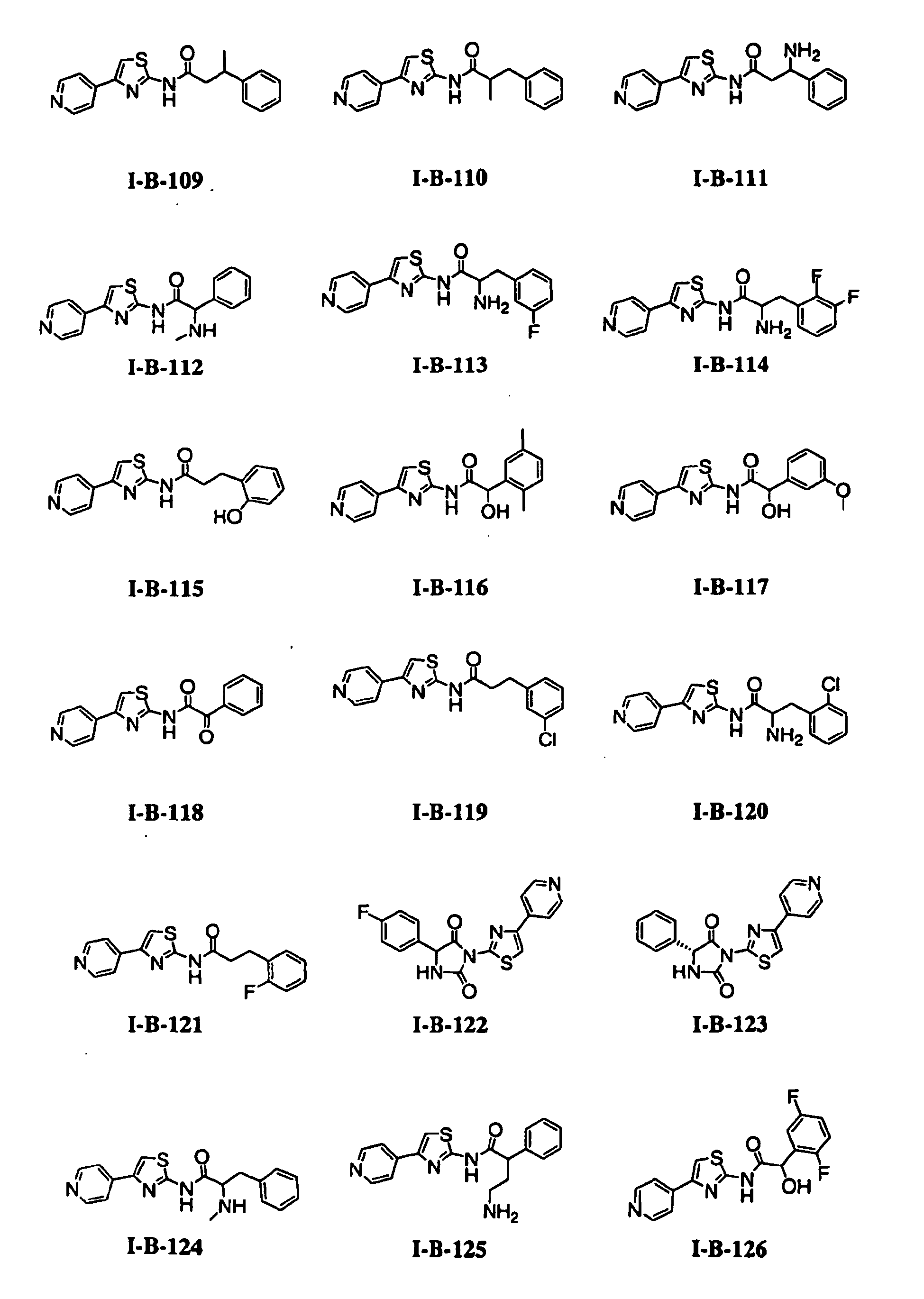

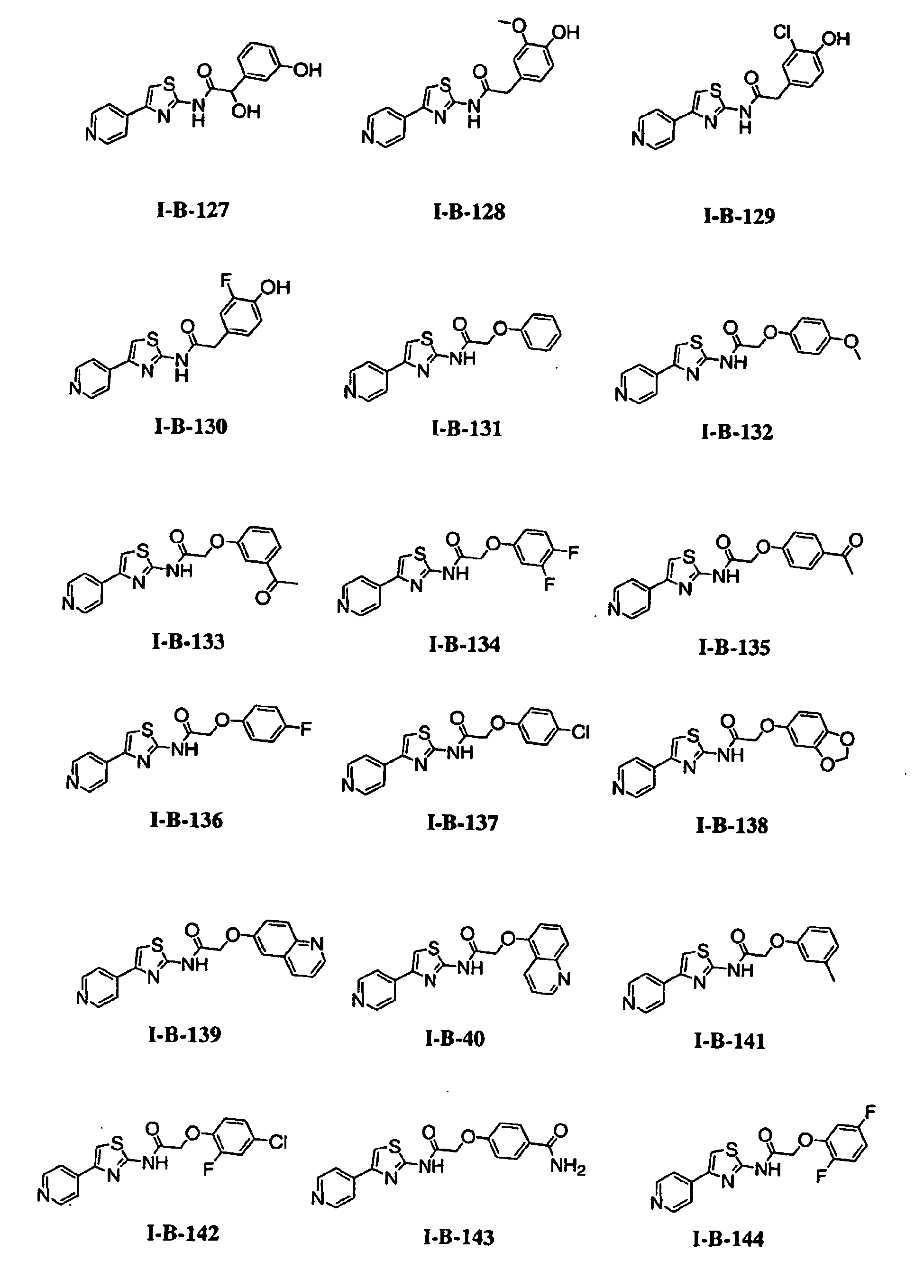

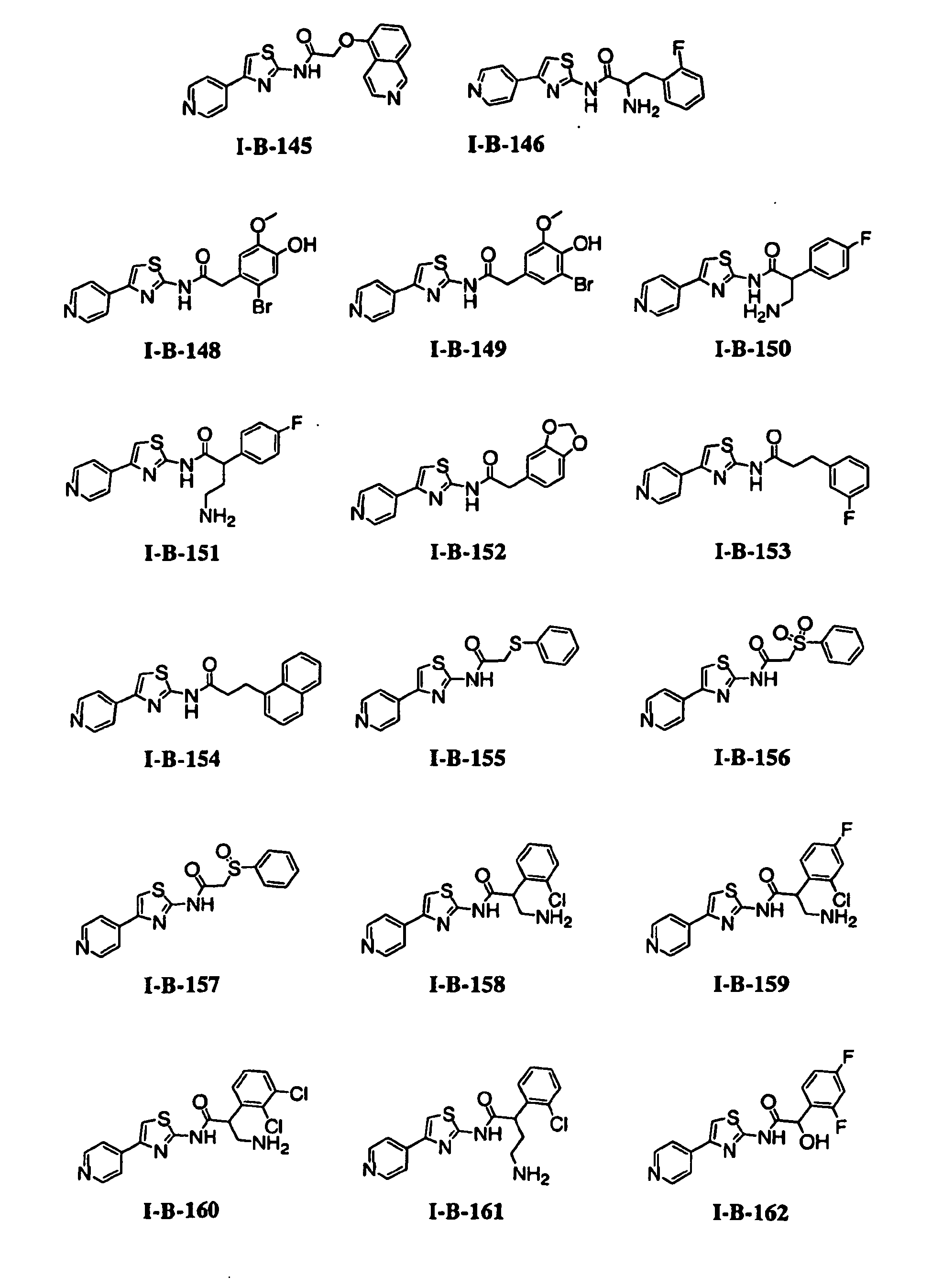

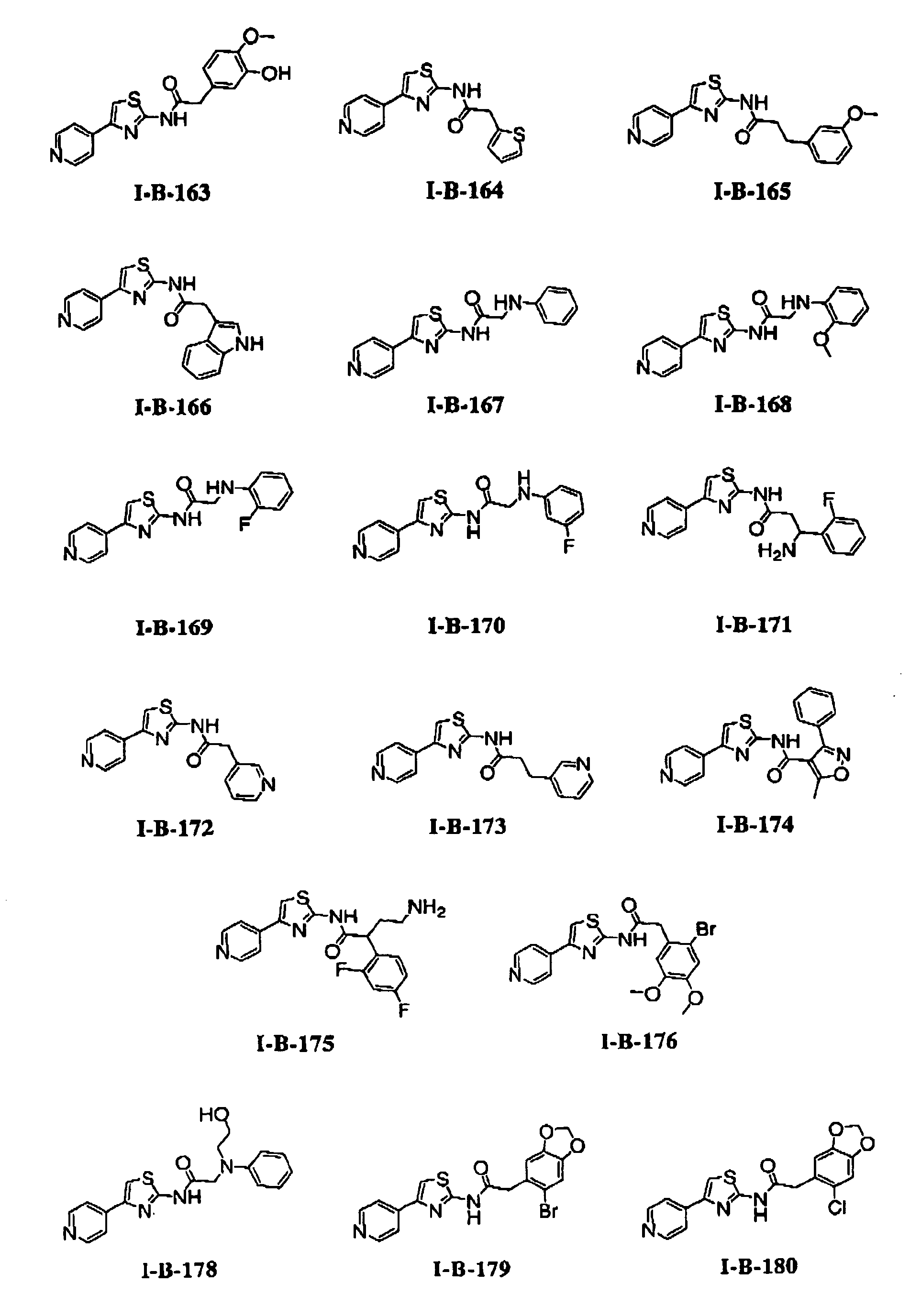

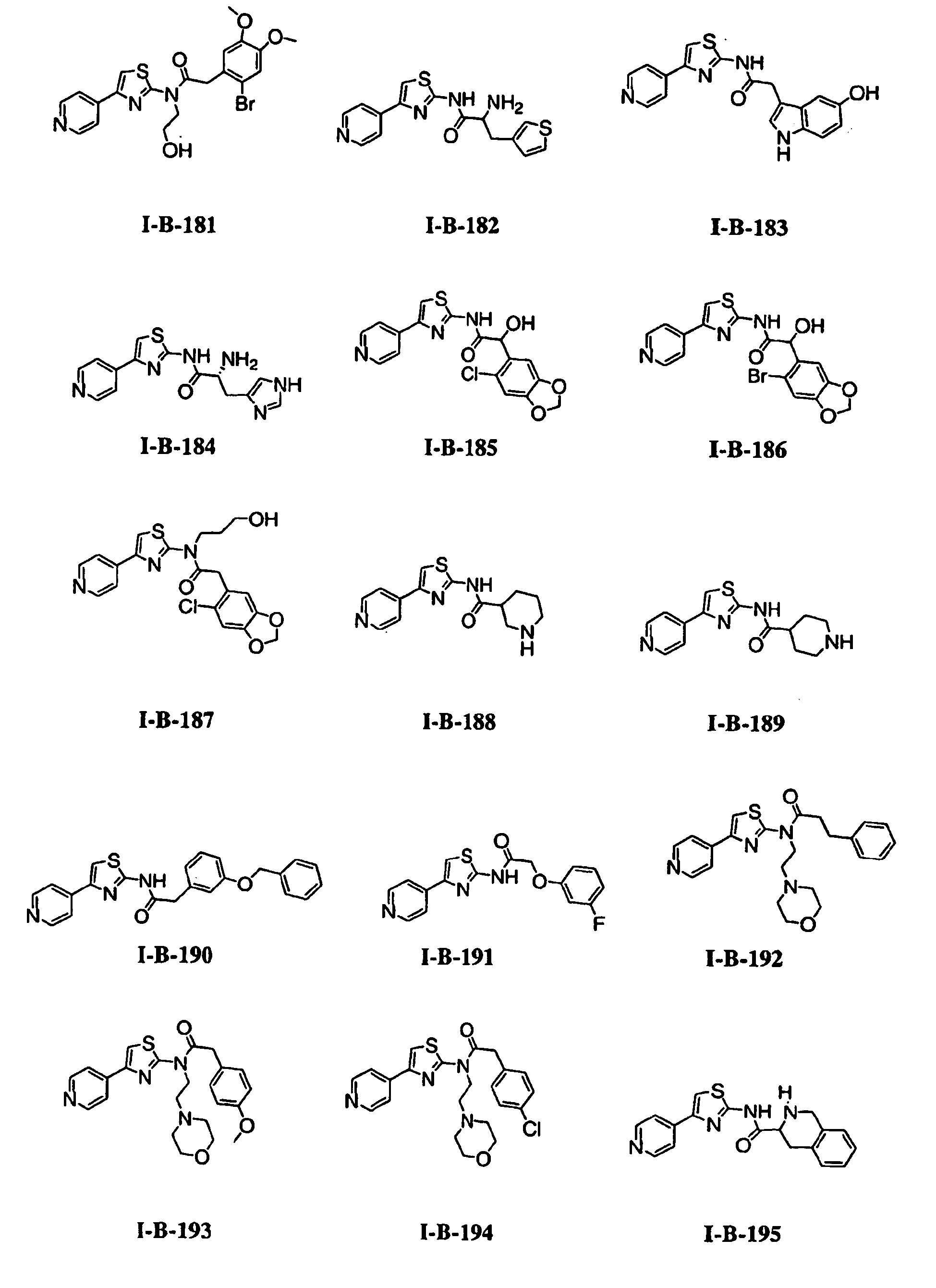

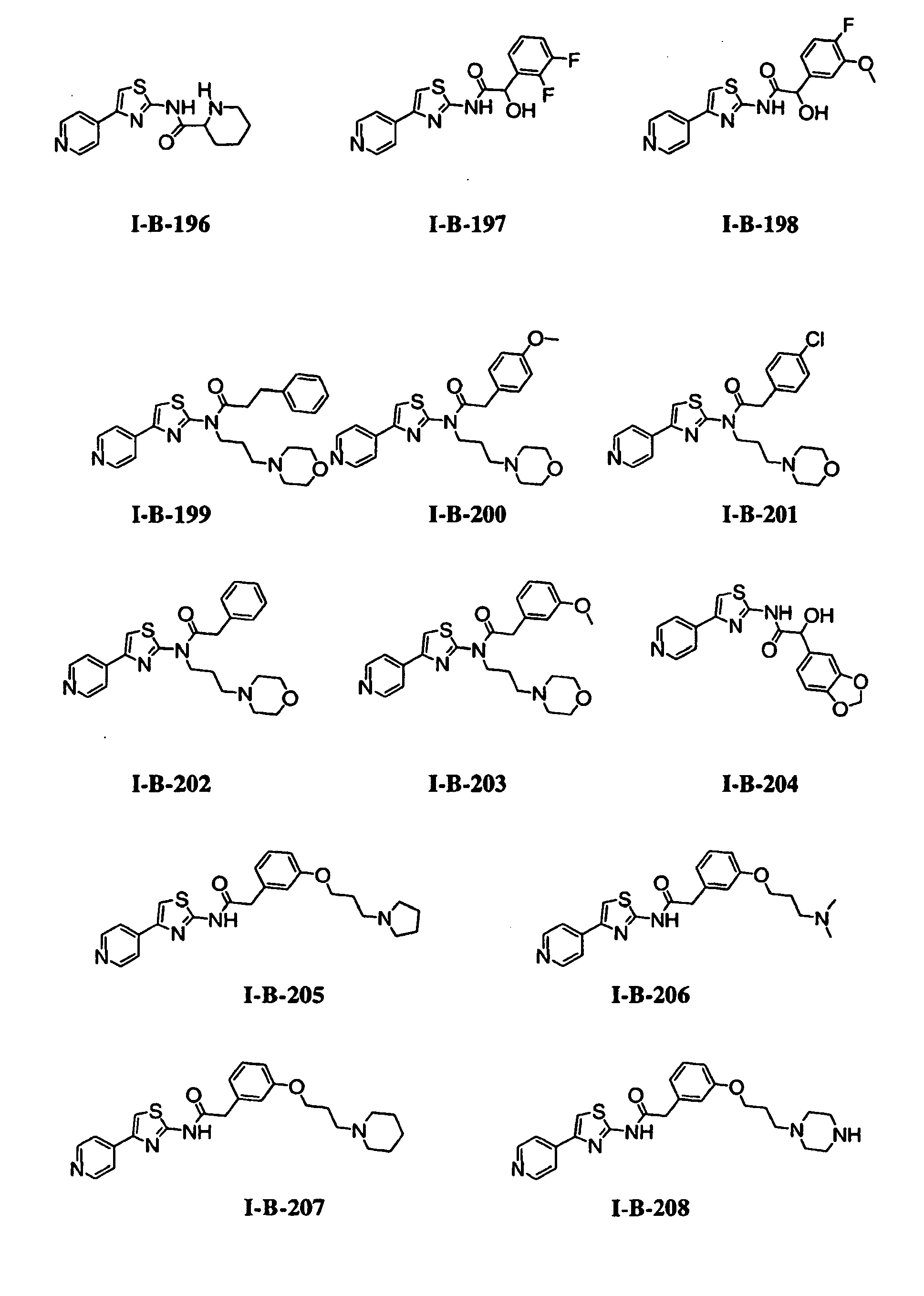

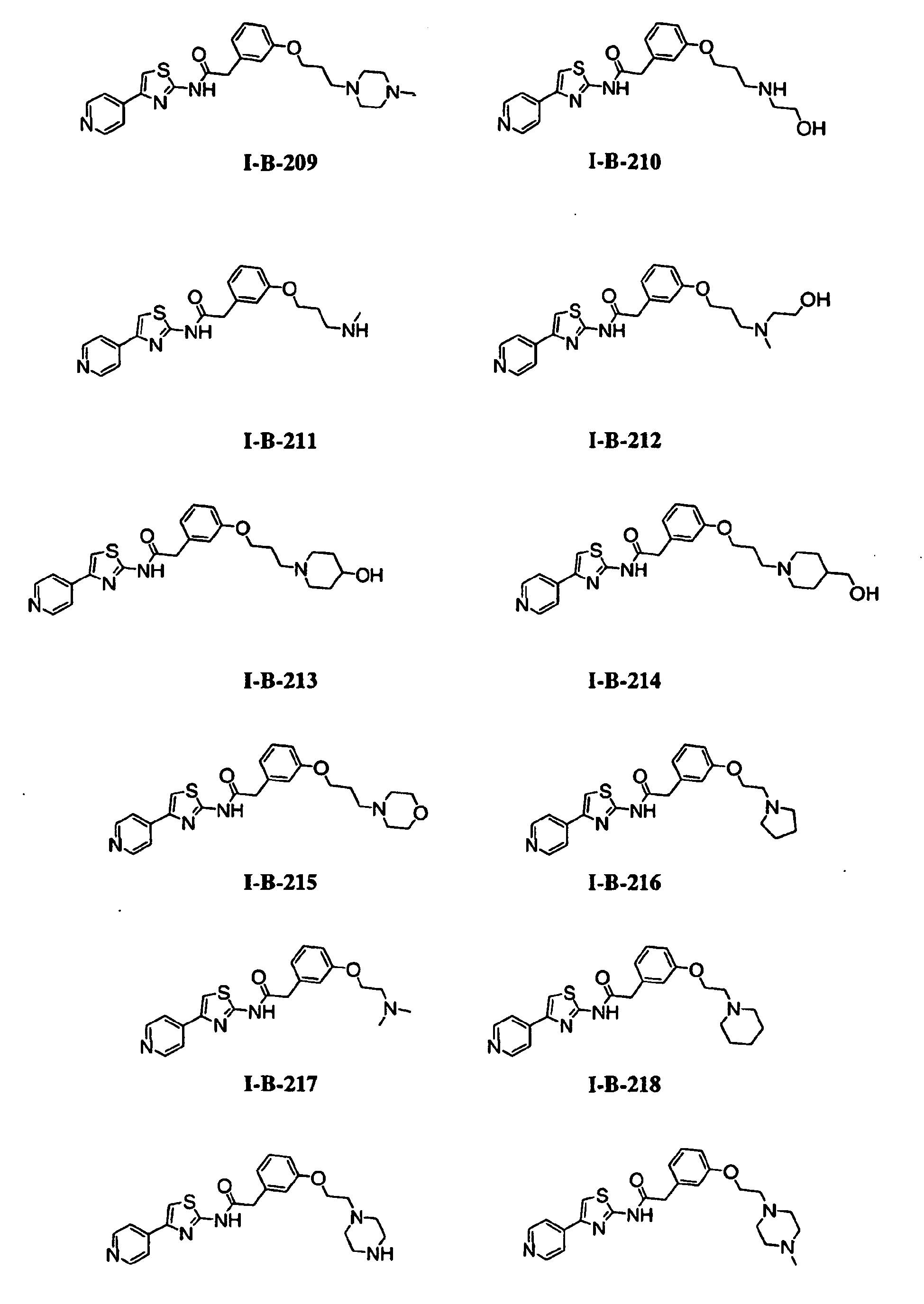

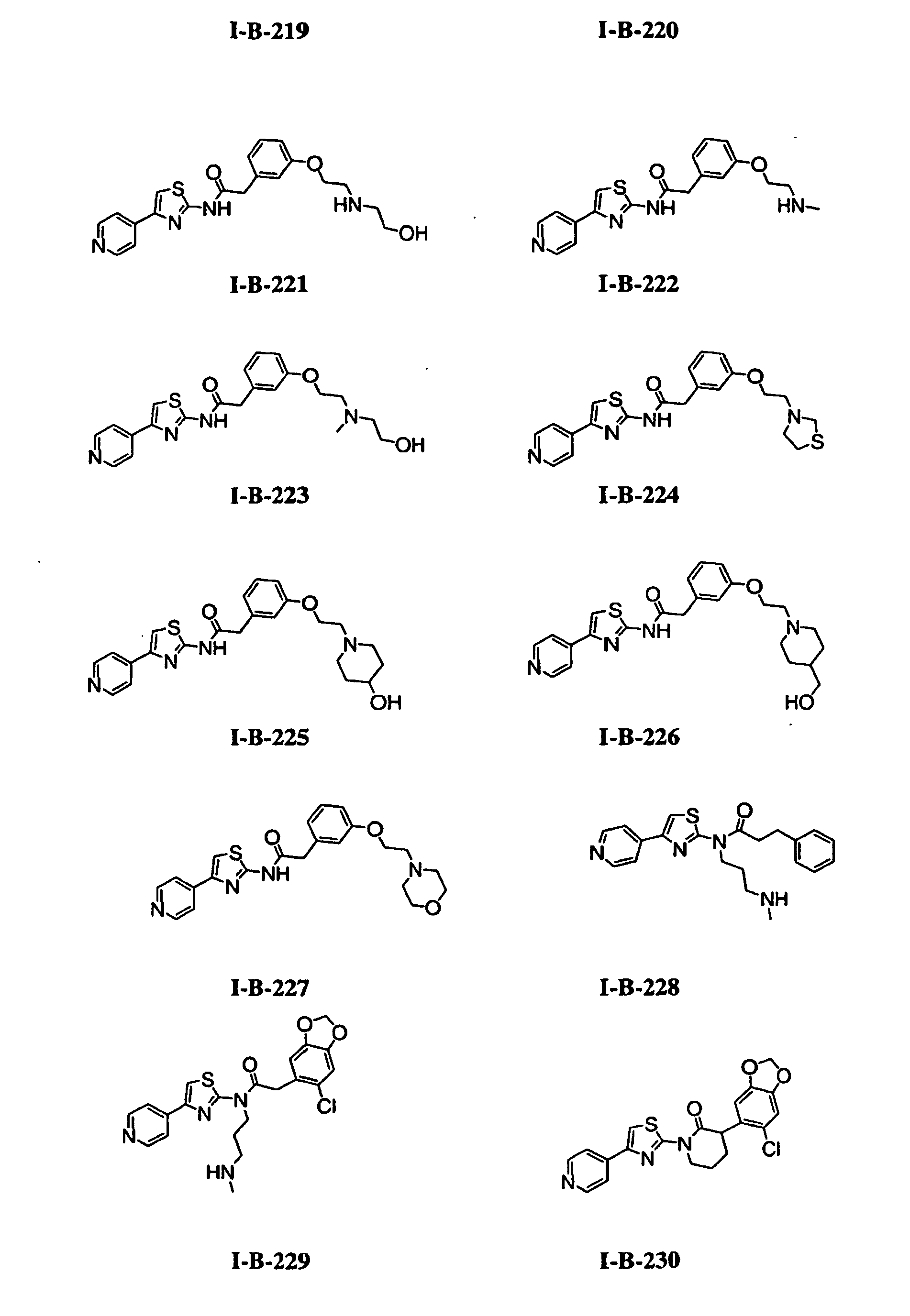

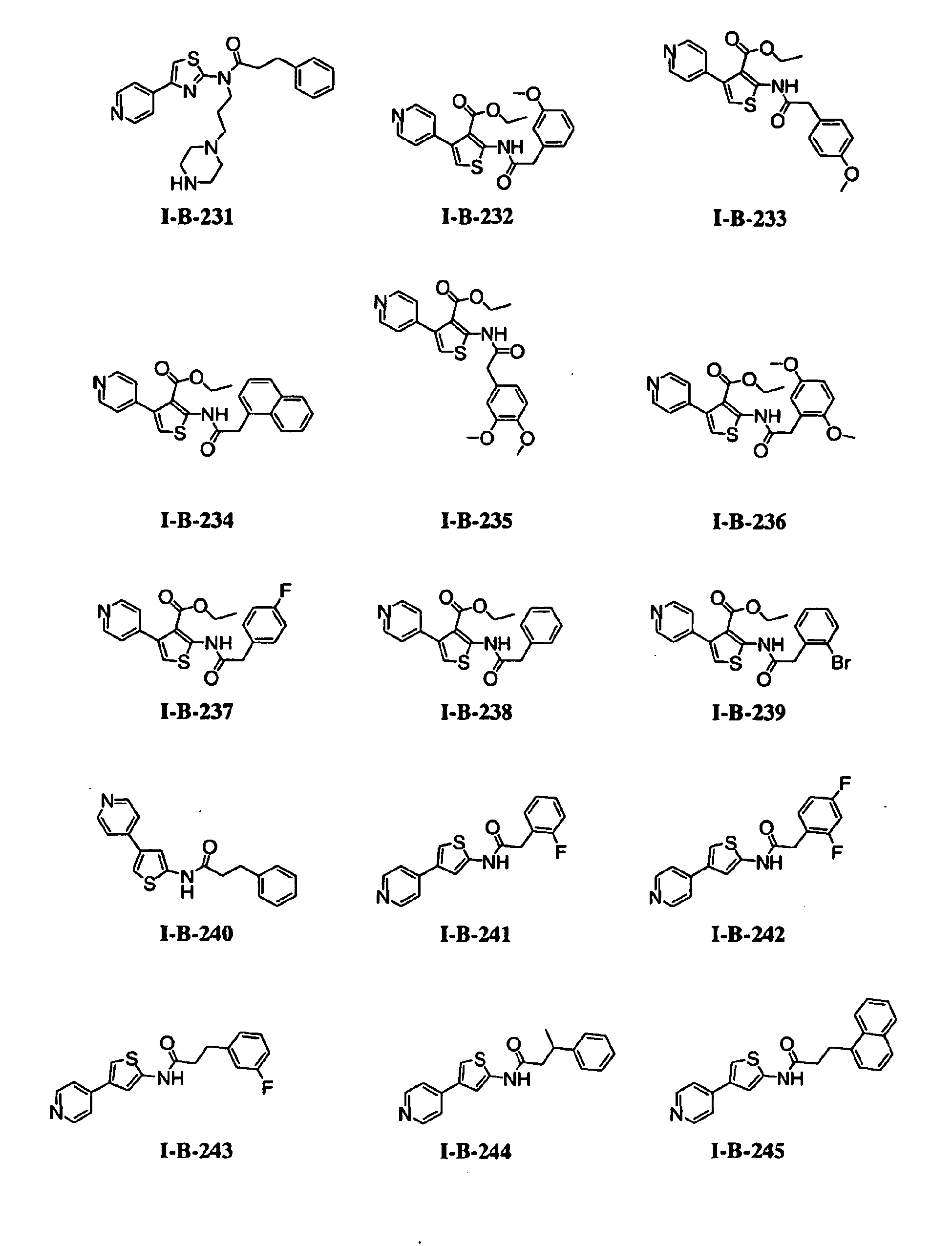

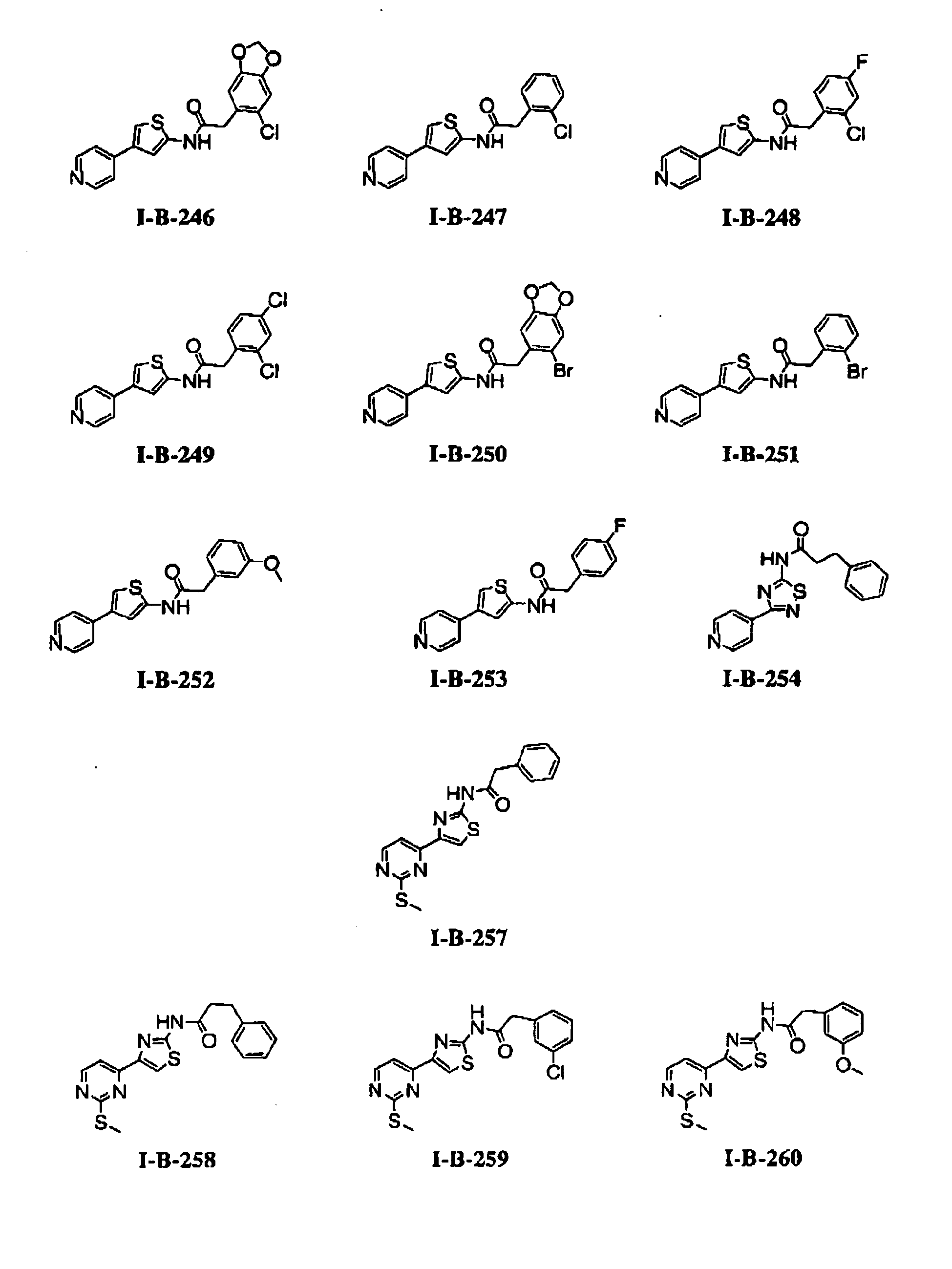

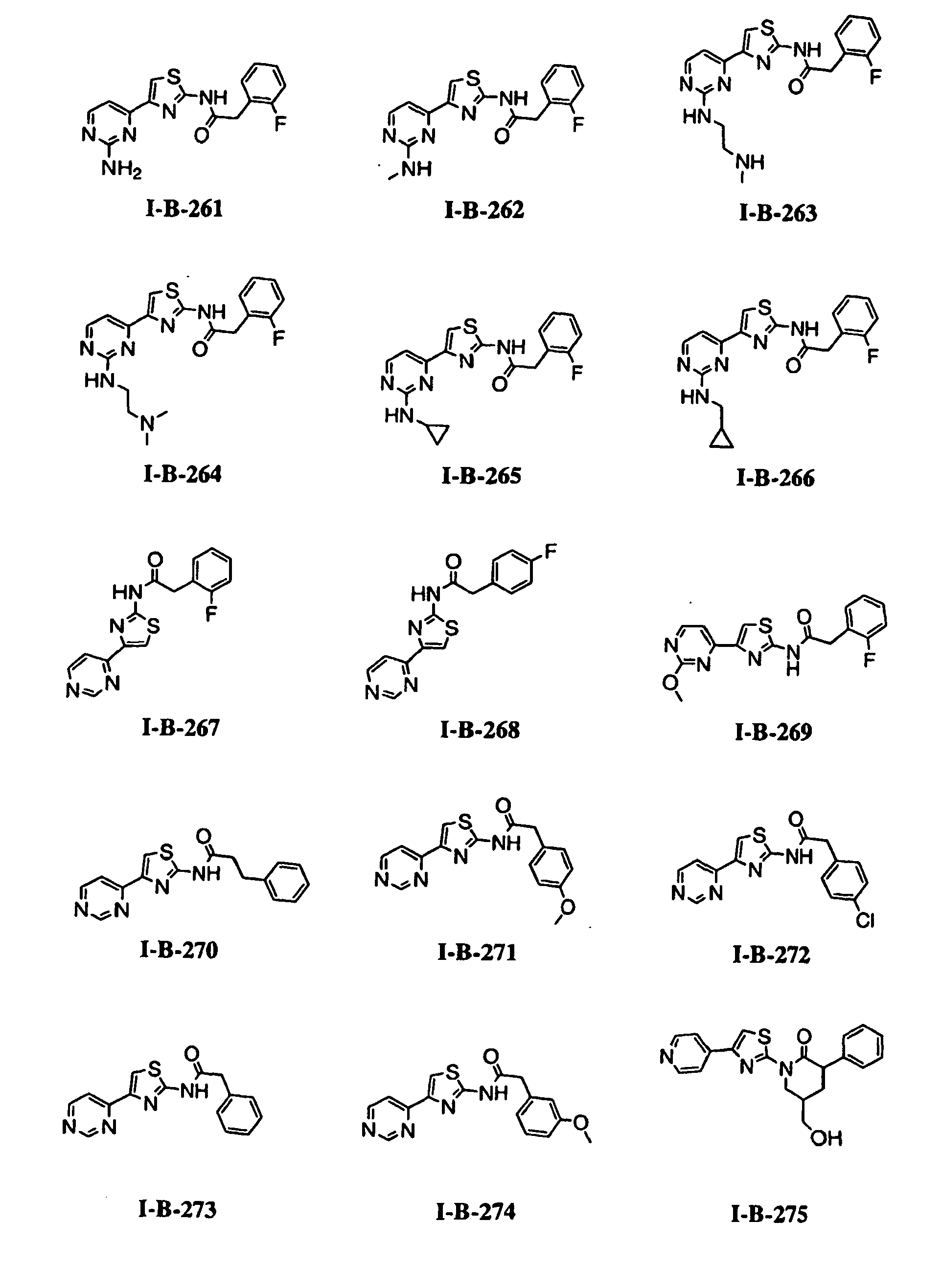

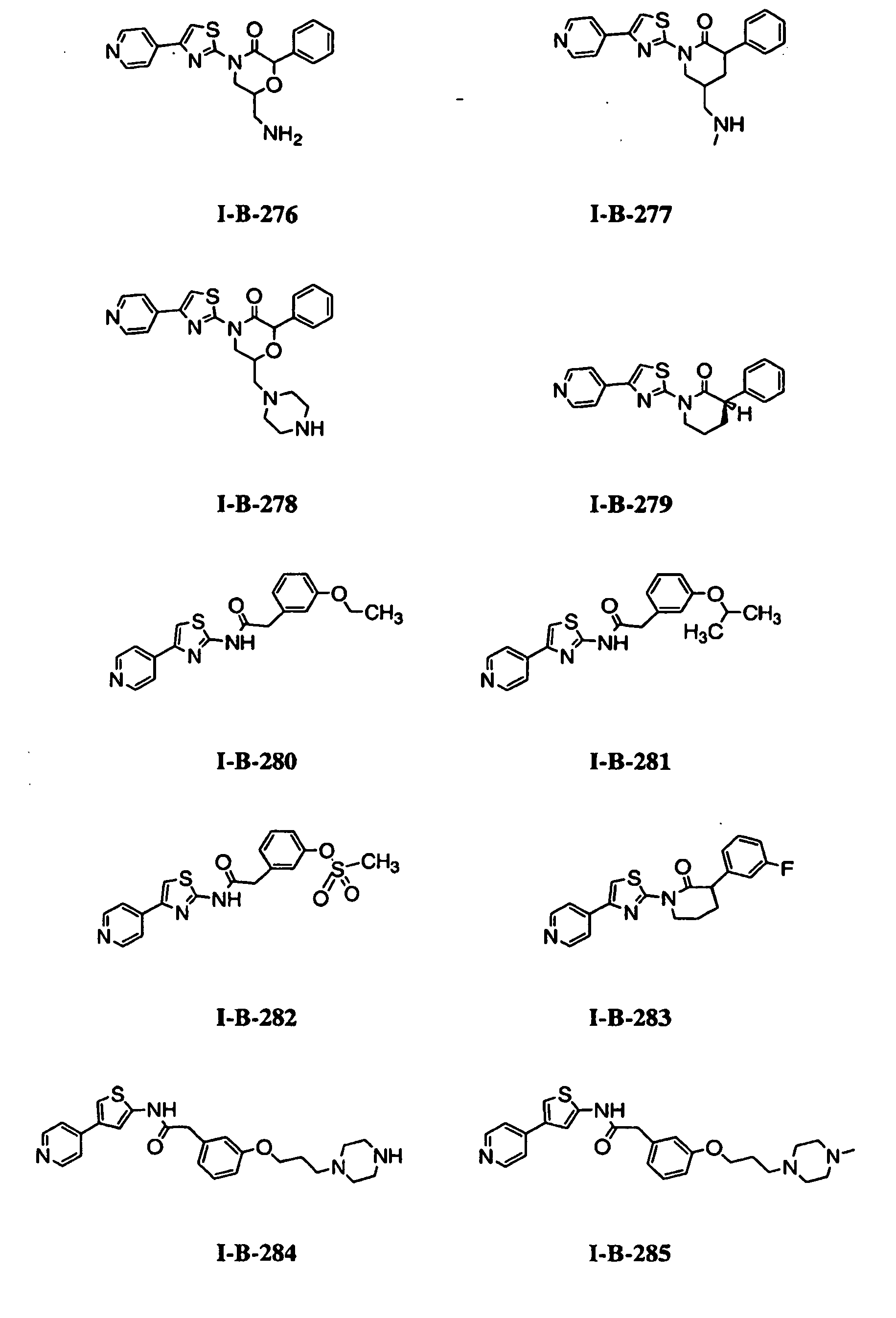

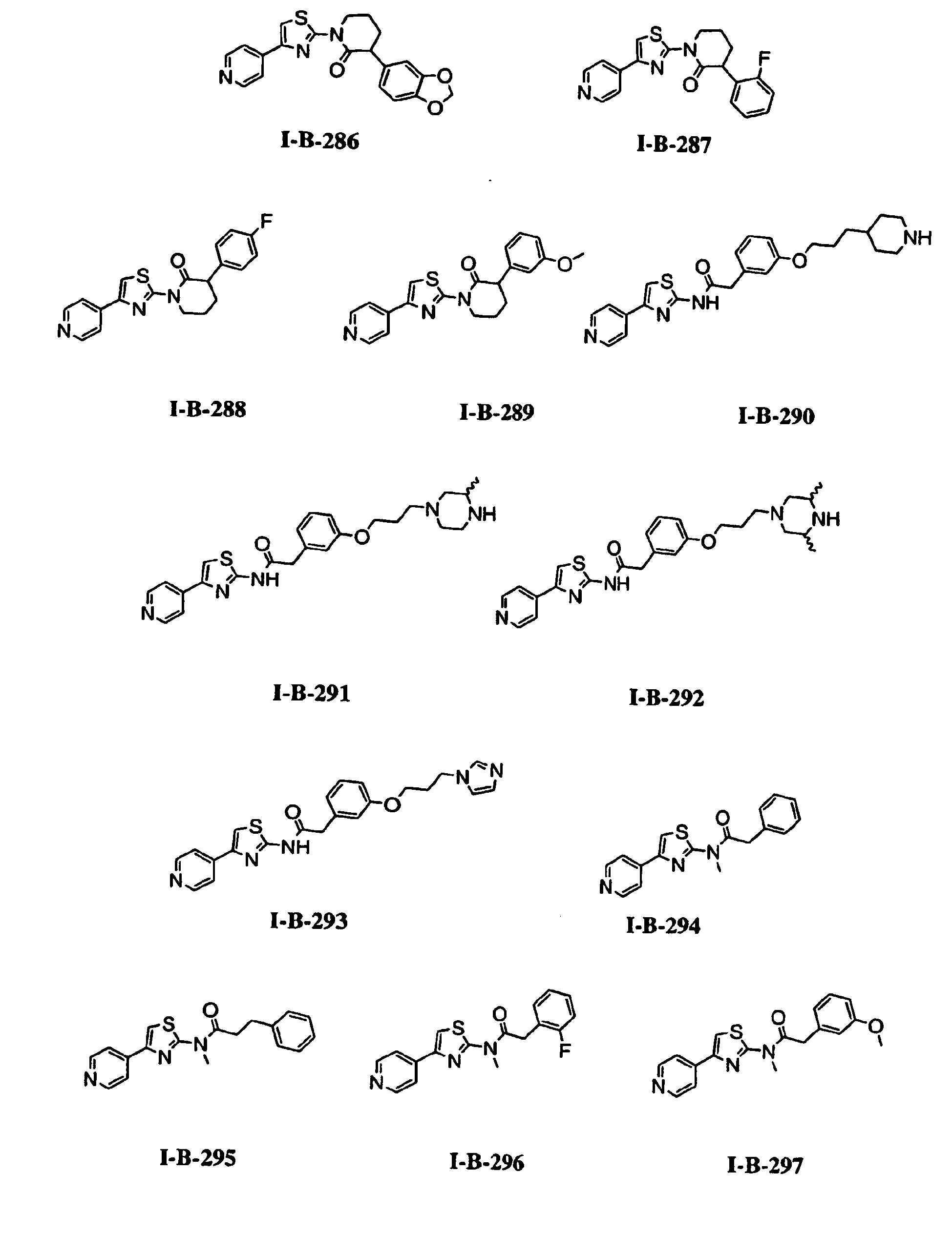

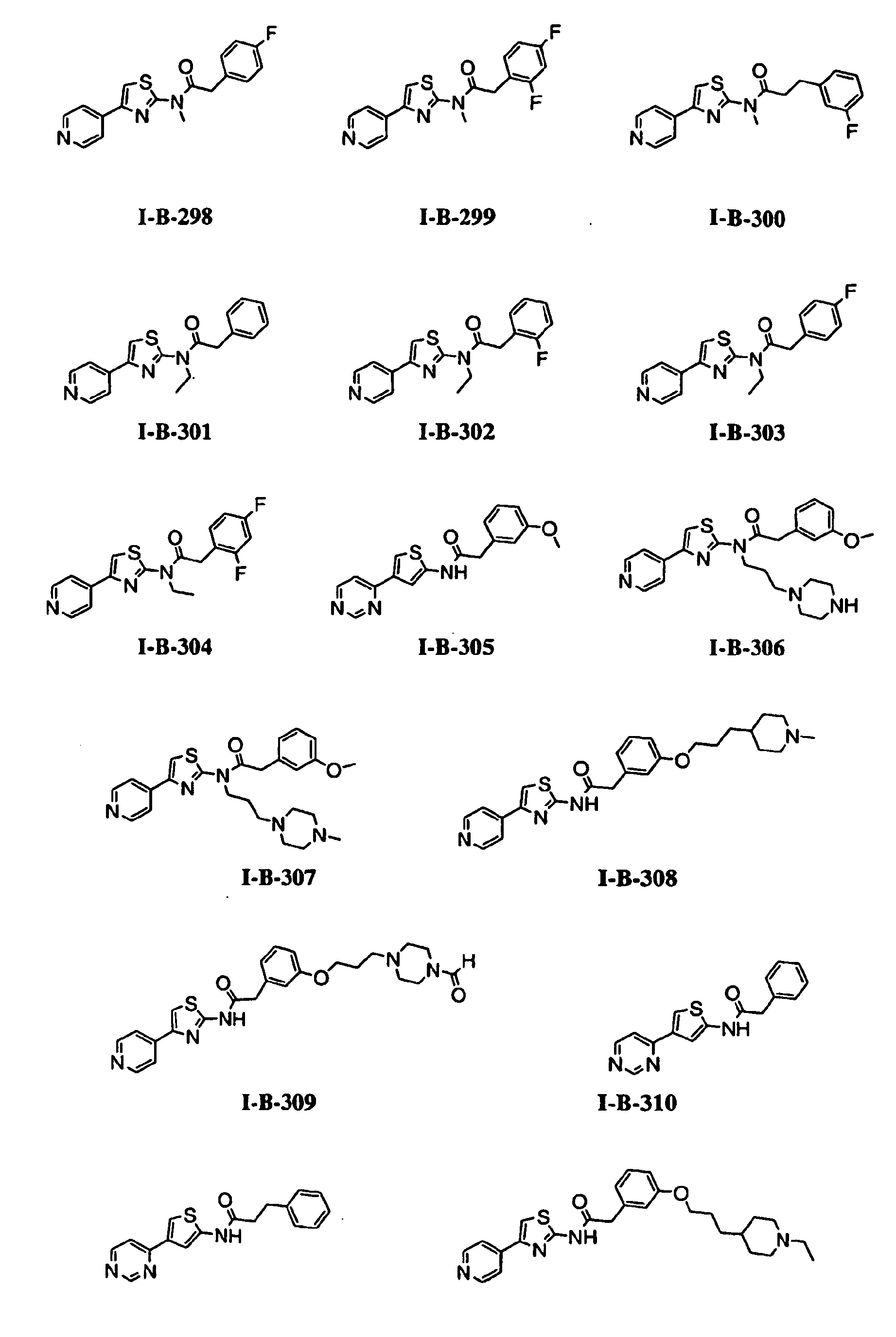

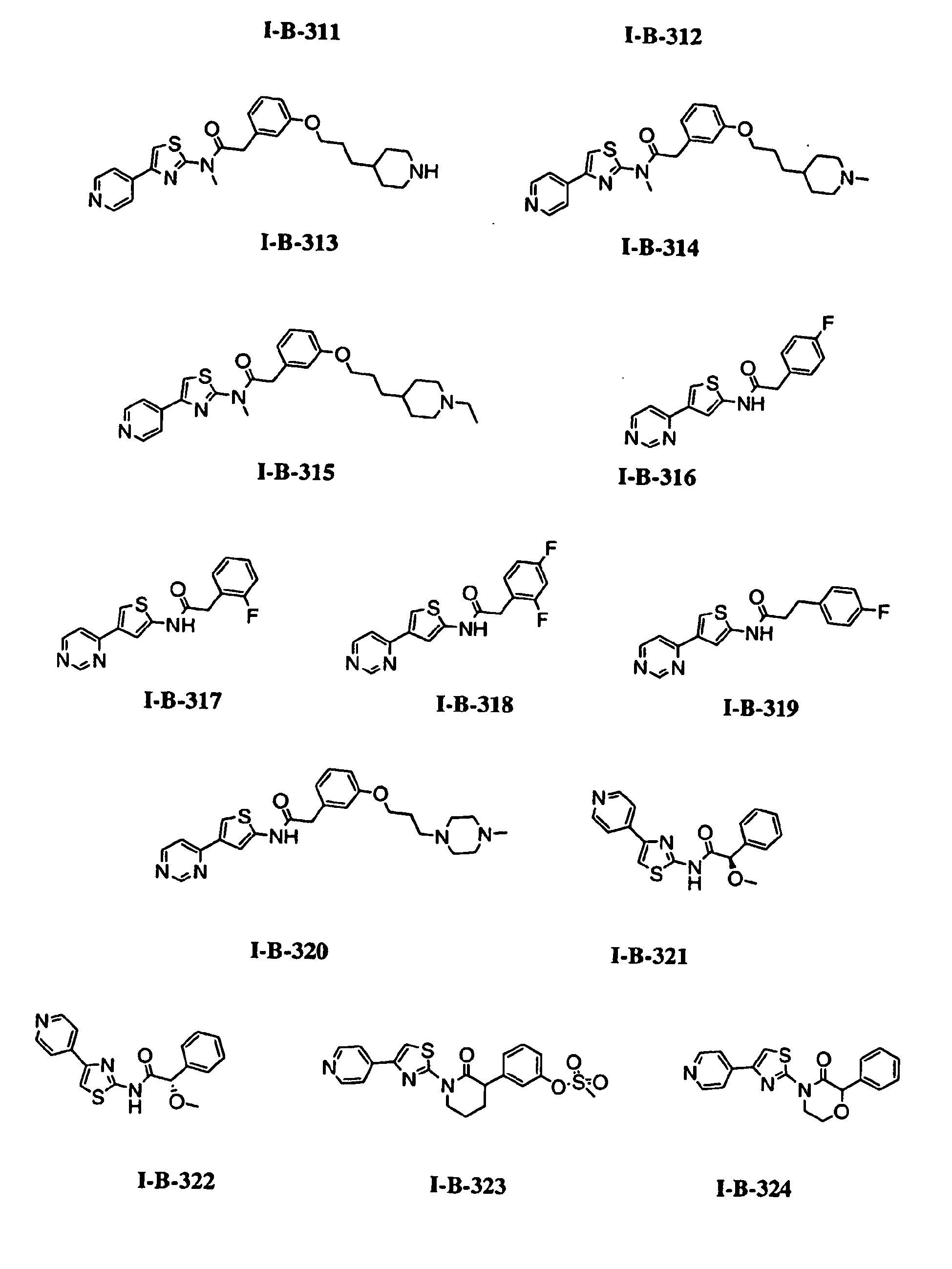

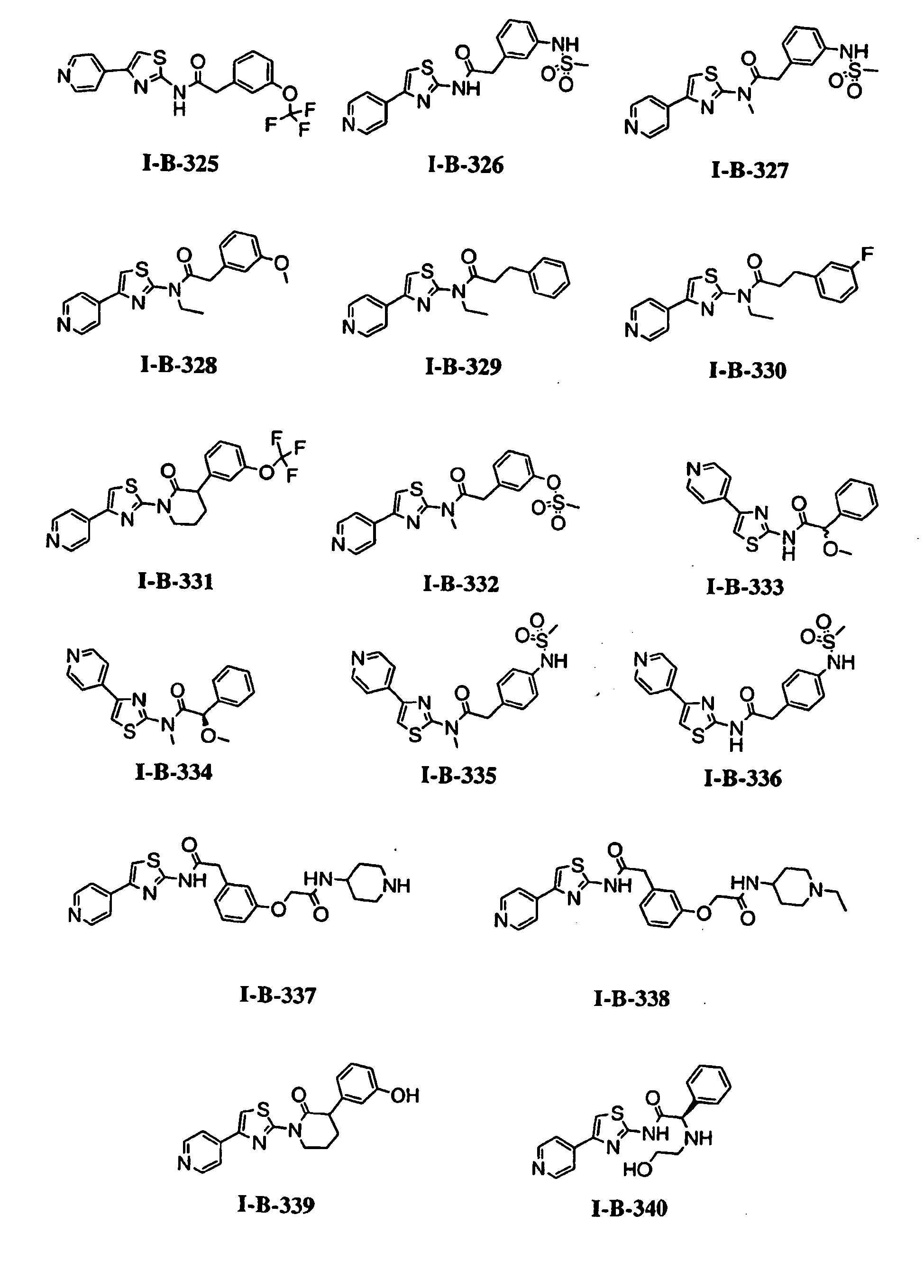

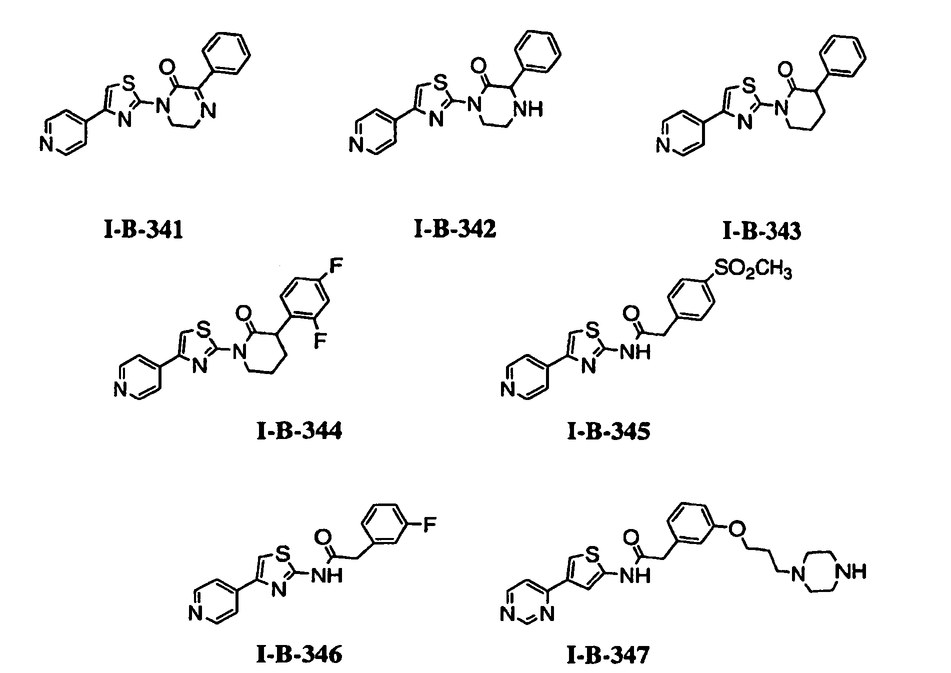

Representative examples of compounds of formula **I-C** are set forth below in Table 3 below.

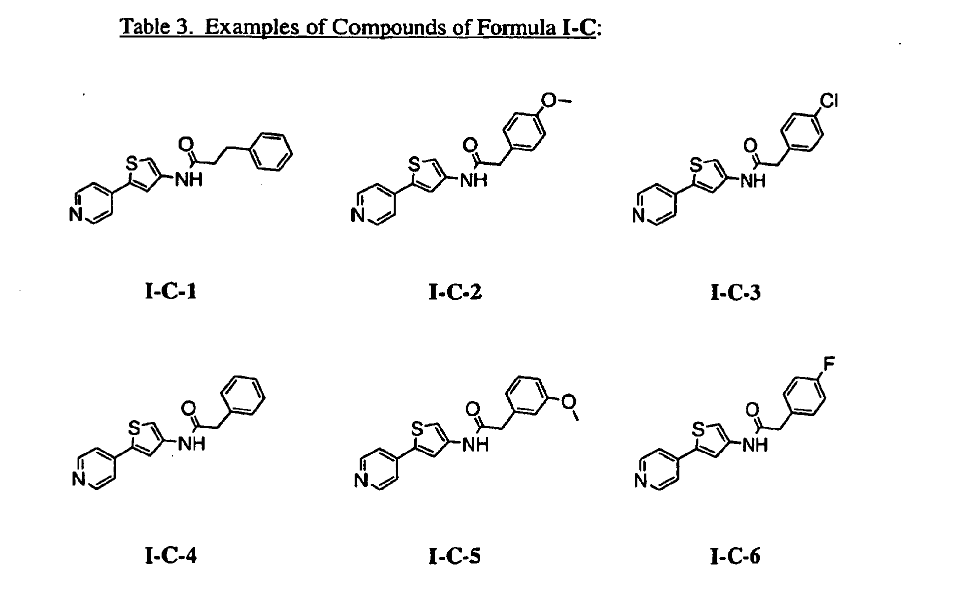

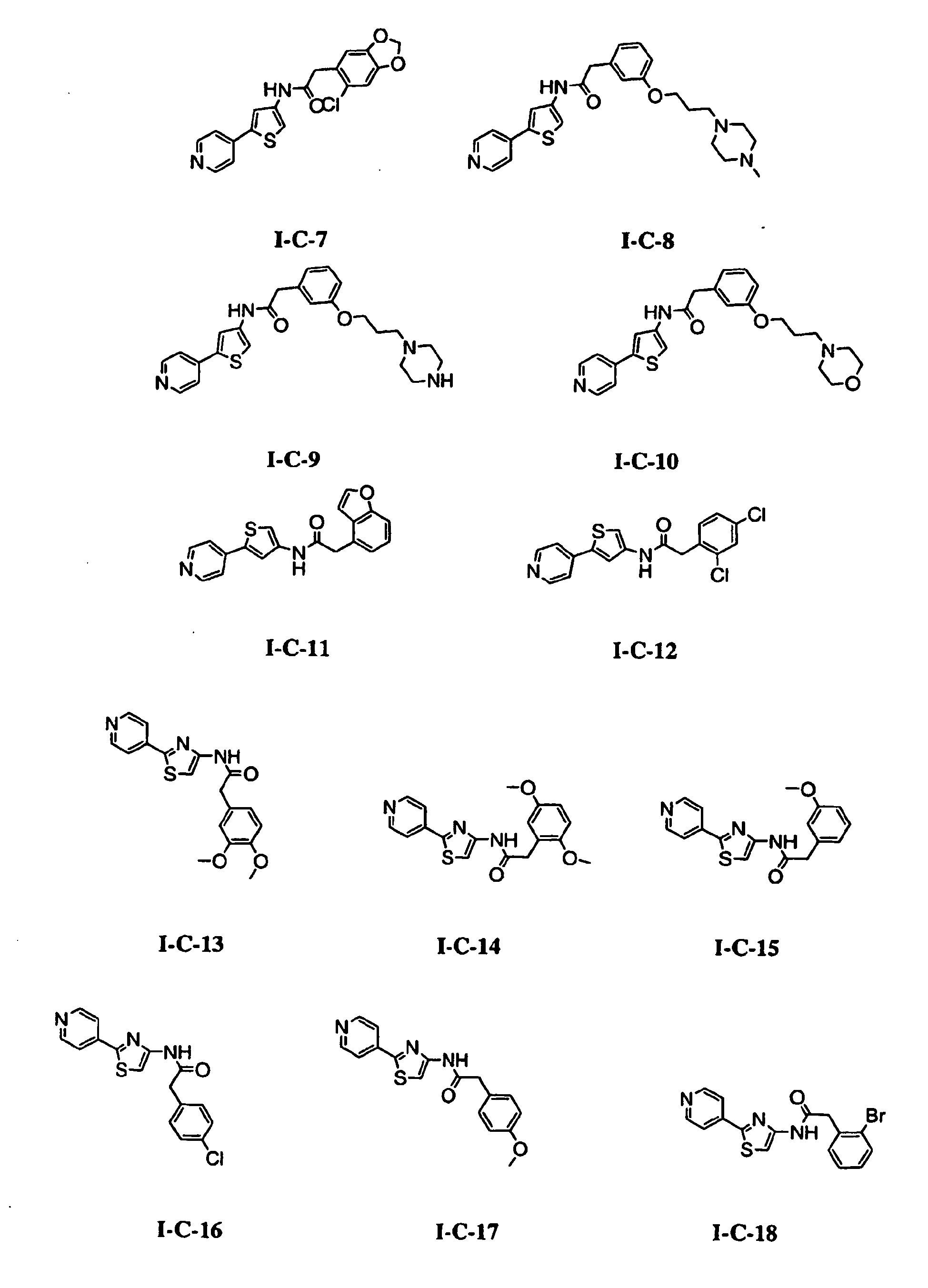

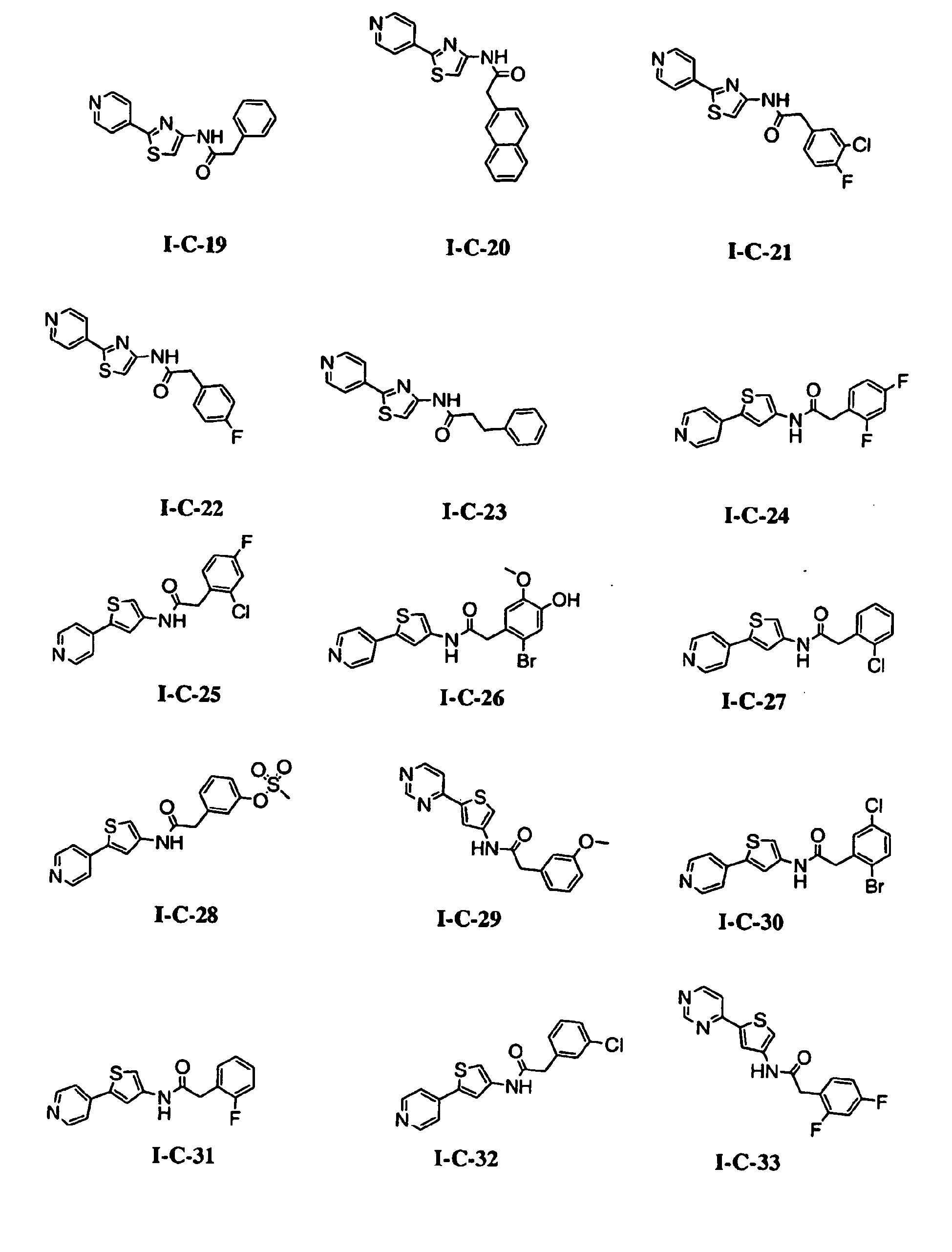

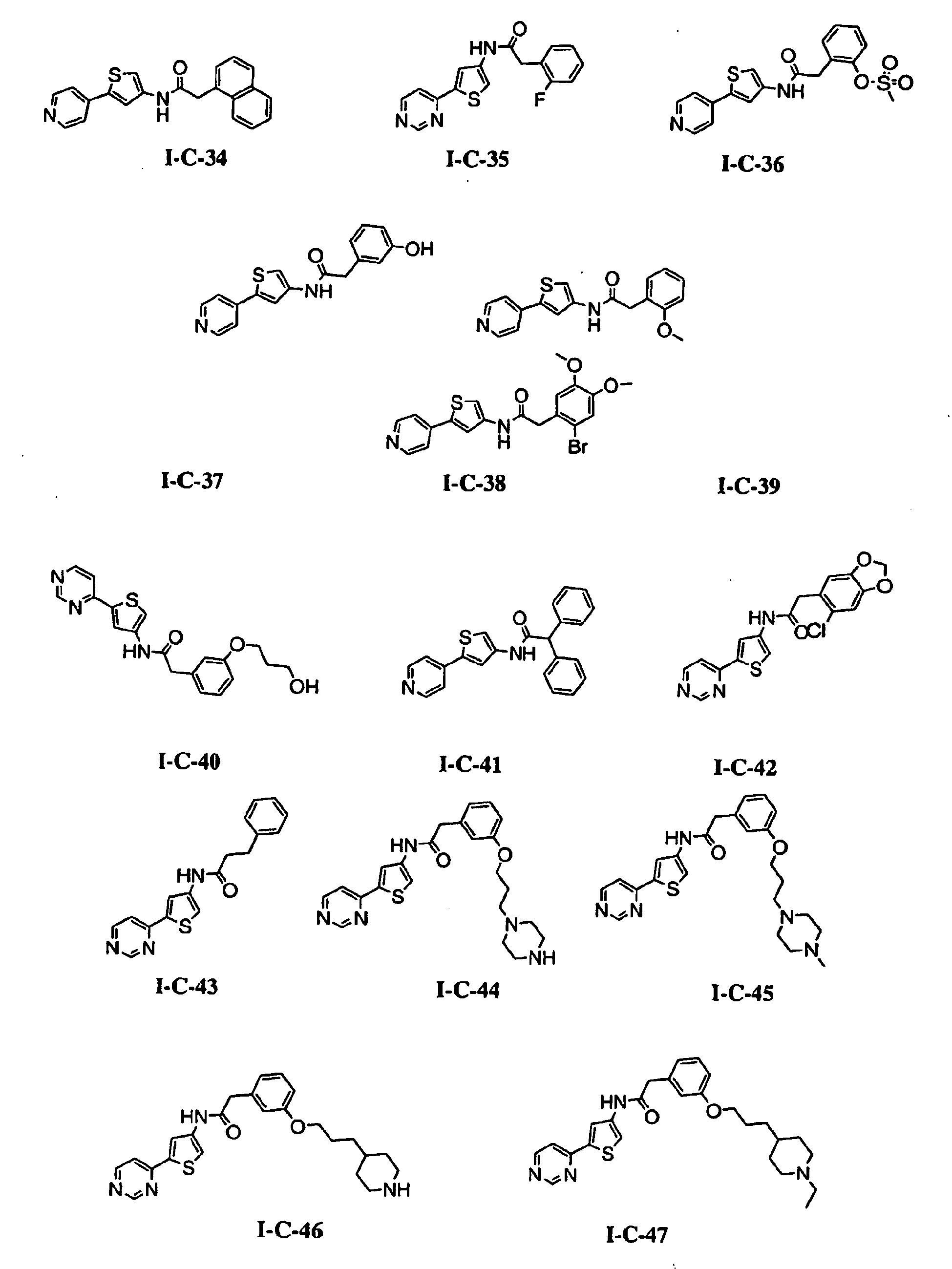

*4. General Synthetic Methodology:*

The compounds of this invention may be prepared in general by methods known to those skilled in the art for analogous compounds, as illustrated by the general schemes below, and the preparative examples that follow.

**Scheme I** below shows a general method for preparing compounds of formula I-A.

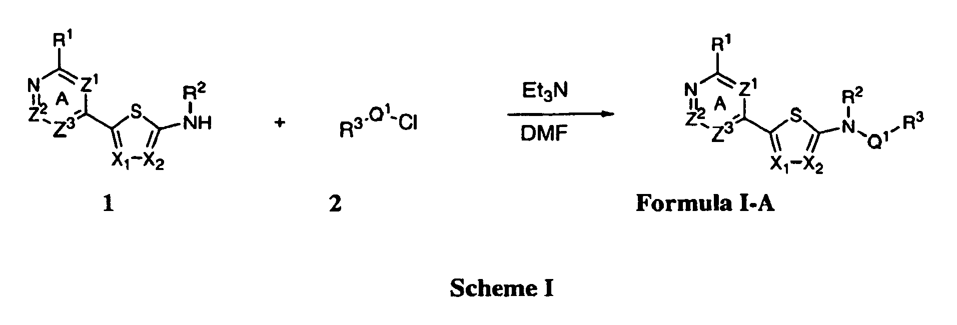

Specifically, as shown in **Scheme I,** the intermediate amine **1** is reacted with a desired acid chloride **2** in the presence of dimethylformamide (DMF) and triethylamine (Et₃N) to yield desired compounds of formula **I** as described generally and in classes, subclasses and species herein.

In certain embodiments, for compounds of formula **I-A,** Q¹ is CHR⁶, wherein R⁶ is defined generally and in classes, subclasses and species herein. Scheme **2** below depicts a general procedure for the preparation of compounds where Q¹ is CHR⁶:

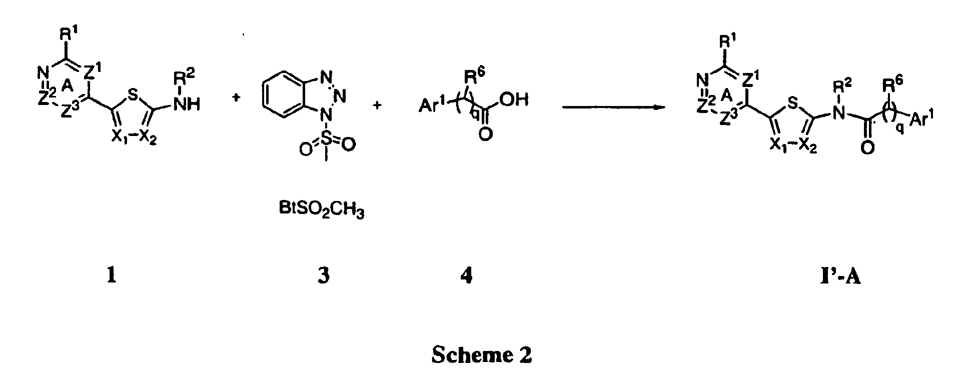

Specifically, as shown in **Scheme 2,** the intermediate amine **1** is reacted with BtSO₂CH₃ **3** and a desired acid **4** in the presence of the presence of triethylamine (Et₃N) to yield desired compounds of formula **I'-A** as described generally and in classes, subclasses and species herein.

**Scheme 3** below shows a general method for preparing compounds of formula **I-B.**

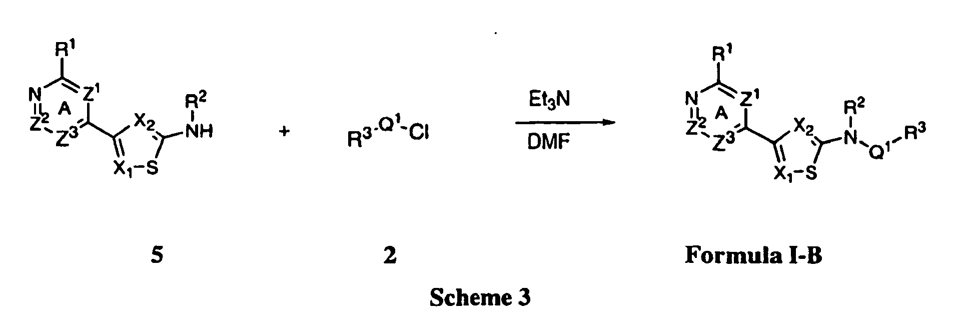

Specifically, as shown in **Scheme 3**, the intermediate amine **5** is reacted with a desired acid chloride **2** in the presence of dimethylformamide (DMF) and triethylamine (Et₃N) to yield desired compounds of formula **I-B** as described generally and in classes, subclasses and species herein.

In certain embodiments, for compounds of formula **I-B,** Q¹ is CHR⁶, wherein R⁶ is defined generally and in classes, subclasses and species herein. Scheme 4 below depicts a general procedure for the preparation of compounds where Q¹ is CHR⁶:

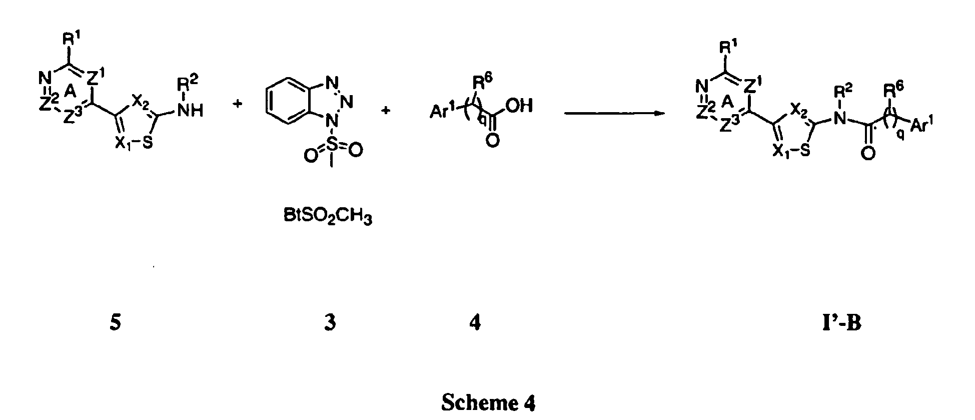

Specifically, as shown in **Scheme 4,** the intermediate amine **5** is reacted with BtSO₂CH₃ **3** and a desired acid **4** in the presence of the presence of triethylamine (Et₃N) to yield desired compounds of formula **I'-B** as described generally and in classes, subclasses and species herein.

**Scheme 5** below shows a general method for preparing compounds of formula **I-C.**

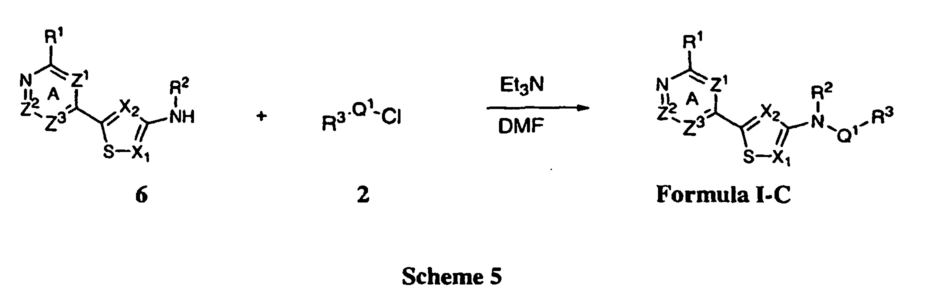

Specifically, as shown in **Scheme 5**, the intermediate amine **6** is reacted with a desired acid chloride **2** in the presence of dimethylformamide (DMF) and triethylamine (Et₃N) to yield desired compounds of formula **I-C** as described generally and in classes, subclasses and species herein.

In certain embodiments, for compounds of formula **I-C,** Q¹ is CHR⁶, wherein R⁶ is defined generally and in classes, subclasses and species herein. Scheme 6 below depicts a general procedure for the preparation of compounds where Q¹ is CHR⁶:

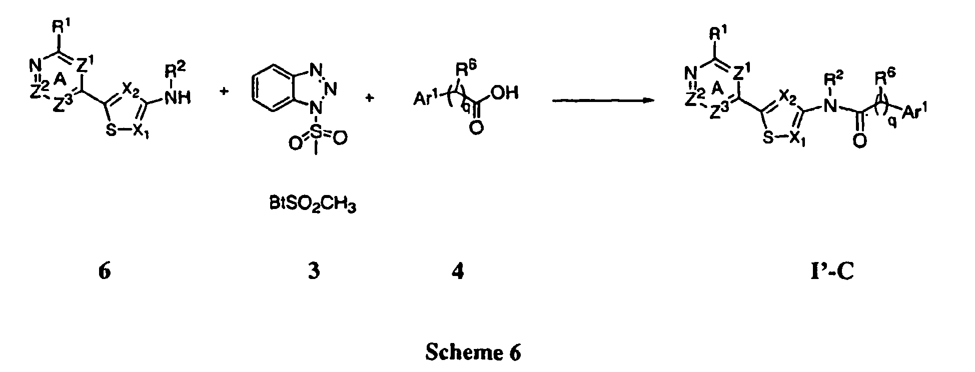

Specifically, as shown in **Scheme 6**, the intermediate amine **6** is reacted with BtSO₂CH₃ **3** and a desired acid **4** in the presence of the presence of triethylamine (Et₃N) to yield desired compounds of formula **I'-C** as described generally and in classes, subclasses and species herein.

Although certain exemplary embodiments are depicted and described above and herein, it will be appreciated that compounds of the invention can be prepared according to the methods described generally above using appropriate starting materials by methods generally available to one of ordinary skill in the art. Additional embodiments are exemplified in more detail herein.

*5. Uses, Formulation and Administration*

*Pharmaceutically acceptable compositions*

As discussed above, the present invention provides compounds that are inhibitors of protein kinases, and thus the present compounds are useful for the treatment of diseases, disorders, and conditions including, but not limited to a proliferative disorder, a cardiac disorder, a neurodegenerative disorder, psychotic disorders, an autoimmune disorder, a condition associated with organ transplant, an inflammatory disorder, an immunologically mediated disorder, a viral disease, or a bone disorder. In preferred embodiments, the compounds are useful for the treatment of allergy, asthma, diabetes, Alzheimer's disease, Huntington's disease, Parkinson's disease, AIDS-associated dementia, amyotrophic lateral sclerosis (AML, Lou Gehrig's disease), multiple sclerosis (MS), schizophrenia, cardiomyocyte hypertrophy, reperfusion/ischemia (e.g., stroke), baldness, cancer, hepatomegaly, cardiovascular disease including cardiomegaly, cystic fibrosis, viral disease, autoimmune diseases, atherosclerosis, restenosis, psoriasis, inflammation, hypertension, angina pectoris, cerebrovascular contraction, peripheral circulation disorder, premature birth, arteriosclerosis, vasospasm (cerebral vasospasm, coronary vasospasm), retinopathy, erectile dysfunction (ED), AIDS, osteoporosis, Crohn's Disease and colitis, neurite outgrowth, and Raynaud's Disease. In preferred embodiments, the disease, condition, or disorder is atherosclerosis, hypertension, erectile dysfunction (ED), reperfusion/ischemia (e.g., stroke), or vasospasm (cerebral vasospasm and coronary vasospasm).

Accordingly, in another aspect of the present invention, pharmaceutically acceptable compositions are provided, wherein these compositions comprise any of the compounds as described herein, and optionally comprise a pharmaceutically acceptable carrier, adjuvant or vehicle. In certain embodiments, these compositions optionally further comprise one or more additional therapeutic agents.

It will also be appreciated that certain of the compounds of present invention can exist in free form for treatment, or where appropriate, as a pharmaceutically acceptable derivative thereof. According to the present invention, a pharmaceutically acceptable derivative includes, but is not limited to, pharmaceutically acceptable prodrugs, salts, esters, salts of such esters, or any other adduct or derivative which upon administration to a patient in need is capable of providing, directly or indirectly, a compound as otherwise described herein, or a metabolite or residue thereof.

As used herein, the term "pharmaceutically acceptable salt" refers to those salts which are, within the scope of sound medical judgement, suitable for use in contact with the tissues of humans and lower animals without undue toxicity, irritation, allergic response and the like, and are commensurate with a reasonable benefit/risk ratio. A "pharmaceutically acceptable salt" means any non-toxic salt or salt of an ester of a compound of this invention that, upon administration to a recipient, is capable of providing, either directly or indirectly, a compound of this invention or an inhibitorily active metabolite or residue thereof. As used herein, the term "inhibitorily active metabolite or residue thereof' means that a metabolite or residue thereof is also an inhibitor of a ROCK, ERK, or GSK kinase, or members of the AGC sub-family of protein kinases (e.g., PKA, PDK, p70^{S6K}-1 and -2, and PKB).

Pharmaceutically acceptable salts are well known in the art. For example, S. M. Berge *et al.,* describe pharmaceutically acceptable salts in detail in J. Pharmaceutical Sciences, 1977, 66, 1-19, incorporated herein by reference. Pharmaceutically acceptable salts of the compounds of this invention include those derived from suitable inorganic and organic acids and bases. Examples of pharmaceutically acceptable, nontoxic acid addition salts are salts of an amino group formed with inorganic acids such as hydrochloric acid, hydrobromic acid, phosphoric acid, sulfuric acid and perchloric acid or with organic acids such as acetic acid, oxalic acid, maleic acid, tartaric acid, citric acid, succinic acid or malonic acid or by using other methods used in the art such as ion exchange. Other pharmaceutically acceptable salts include adipate, alginate, ascorbate, aspartate, benzenesulfonate, benzoate, bisulfate, borate, butyrate, camphorate, camphorsulfonate, citrate, cyclopentanepropionate, digluconate, dodecylsulfate, ethanesulfonate, formate, fumarate, glucoheptonate, glycerophosphate, gluconate, hemisulfate, heptanoate, hexanoate, hydroiodide, 2-hydroxy-ethanesulfonate, lactobionate, lactate, laurate, lauryl sulfate, malate, maleate, malonate, methanesulfonate, 2-naphthalenesulfonate, nicotinate, nitrate, oleate, oxalate, palmitate, pamoate, pectinate, persulfate, 3-phenylpropionate, phosphate, picrate, pivalate, propionate, stearate, succinate, sulfate, tartrate, thiocyanate, p-toluenesulfonate, undecanoate, valerate salts, and the like. Salts derived from appropriate bases include alkali metal, alkaline earth metal, ammonium and N⁺(C₁₋₄alkyl)₄ salts. This invention also envisions the quaternization of any basic nitrogen-containing groups of the compounds disclosed herein. Water or oil-soluble or dispersable products may be obtained by such quaternization. Representative alkali or alkaline earth metal salts include sodium, lithium, potassium, calcium, magnesium, and the like. Further pharmaceutically acceptable salts include, when appropriate, nontoxic ammonium, quaternary ammonium, and amine cations formed using counterions such as halide, hydroxide, carboxylate, sulfate, phosphate, nitrate, loweralkyl sulfonate and aryl sulfonate.

As described above, the pharmaceutically acceptable compositions of the present invention additionally comprise a pharmaceutically acceptable carrier, adjuvant, or vehicle, which, as used herein, includes any and all solvents, diluents, or other liquid vehicle, dispersion or suspension aids, surface active agents, isotonic agents, thickening or emulsifying agents, preservatives, solid binders, lubricants and the like, as suited to the particular dosage form desired. Remington's Pharmaceutical Sciences, Sixteenth Edition, E. W. Martin (Mack Publishing Co., Easton, Pa., 1980) discloses various carriers used in formulating pharmaceutically acceptable compositions and known techniques for the preparation thereof. Except insofar as any conventional carrier medium is incompatible with the compounds of the invention, such as by producing any undesirable biological effect or otherwise interacting in a deleterious manner with any other component(s) of the pharmaceutically acceptable composition, its use is contemplated to be within the scope of this invention. Some examples of materials which can serve as pharmaceutically acceptable carriers include, but are not limited to, ion exchangers, alumina, aluminum stearate, lecithin, serum proteins, such as human serum albumin, buffer substances such as phosphates, glycine, sorbic acid, or potassium sorbate, partial glyceride mixtures of saturated vegetable fatty acids, water, salts or electrolytes, such as protamine sulfate, disodium hydrogen phosphate, potassium hydrogen phosphate, sodium chloride, zinc salts, colloidal silica, magnesium trisilicate, polyvinyl pyrrolidone, polyacrylates, waxes, polyethylene-polyoxypropylene-block polymers, wool fat, sugars such as lactose, glucose and sucrose; starches such as com starch and potato starch; cellulose and its derivatives such as sodium carboxymethyl cellulose, ethyl cellulose and cellulose acetate; powdered tragacanth; malt; gelatin; talc; excipients such as cocoa butter and suppository waxes; oils such as peanut oil, cottonseed oil; safflower oil; sesame oil; olive oil; corn oil and soybean oil; glycols; such a propylene glycol or polyethylene glycol; esters such as ethyl oleate and ethyl laurate; agar; buffering agents such as magnesium hydroxide and aluminum hydroxide; alginic acid; pyrogen-free water; isotonic saline; Ringer's solution; ethyl alcohol, and phosphate buffer solutions, as well as other non-toxic compatible lubricants such as sodium lauryl sulfate and magnesium stearate, as well as coloring agents, releasing agents, coating agents, sweetening, flavoring and perfuming agents, preservatives and antioxidants can also be present in the composition, according to the judgment of the formulator.

*Uses of Compounds and Pharmaceutically acceptable compositions*

In yet another aspect, a method for the treatment or lessening the severity of a proliferative disorder, a cardiac disorder, a neurodegenerative disorder, a psychotic disorder, an autoimmune disorder, a condition associated with organ transplant, an inflammatory disorder, an immunologically mediated disorder, a viral disease, or a bone disorder is provided comprising administering an effective amount of a compound, or a pharmaceutically acceptable composition comprising a compound to a subject in need thereof. In certain embodiments of the present invention an "effective amount" of the compound or pharmaceutically acceptable composition is that amount effective for treating or lessening the severity of a proliferative disorder, a cardiac disorder, a neurodegenerative disorder, a psychotic disorder, an autoimmune disorder, a condition associated with organ transplant, an inflammatory disorder, an immunologically mediated disorder, a viral disease, or a bone disorder. The compounds and compositions, according to the method of the present invention, may be administered using any amount and any route of administration effective for treating or lessening the severity of a proliferative disorder, a cardiac disorder, a neurodegenerative disorder, an autoimmune disorder, a condition associated with organ transplant, an inflammatory disorder, an immunologically mediated disorder, a viral disease, or a bone disorder. The exact amount required will vary from subject to subject, depending on the species, age, and general condition of the subject, the severity of the infection, the particular agent, its mode of administration, and the like. The compounds of the invention are preferably formulated in dosage unit form for ease of administration and uniformity of dosage. The expression "dosage unit form" as used herein refers to a physically discrete unit of agent appropriate for the patient to be treated. It will be understood, however, that the total daily usage of the compounds and compositions of the present invention will be decided by the attending physician within the scope of sound medical judgment. The specific effective dose level for any particular patient or organism will depend upon a variety of factors including the disorder being treated and the severity of the disorder; the activity of the specific compound employed; the specific composition employed; the age, body weight, general health, sex and diet of the patient; the time of administration, route of administration, and rate of excretion of the specific compound employed; the duration of the treatment; drugs used in combination or coincidental with the specific compound employed, and like factors well known in the medical arts. The term "patient", as used herein, means an animal, preferably a mammal, and most preferably a human.

The pharmaceutically acceptable compositions of this invention can be administered to humans and other animals orally, rectally, parenterally, intracisternally, intravaginally, intraperitoneally, topically (as by powders, ointments, or drops), bucally, as an oral or nasal spray, or the like, depending on the severity of the infection being treated. In certain embodiments, the compounds of the invention may be administered orally or parenterally at dosage levels of about 0.01 mg/kg to about 50 mg/kg and preferably from about 1 mg/kg to about 25 mg/kg, of subject body weight per day, one or more times a day, to obtain the desired therapeutic effect.

Liquid dosage forms for oral administration include, but are not limited to, pharmaceutically acceptable emulsions, microemulsions, solutions, suspensions, syrups and elixirs. In addition to the active compounds, the liquid dosage forms may contain inert diluents commonly used in the art such as, for example, water or other solvents, solubilizing agents and emulsifiers such as ethyl alcohol, isopropyl alcohol, ethyl carbonate, ethyl acetate, benzyl alcohol, benzyl benzoate, propylene glycol, 1,3-butylene glycol, dimethylformamide, oils (in particular, cottonseed, groundnut, corn, germ, olive, castor, and sesame oils), glycerol, tetrahydrofurfuryl alcohol, polyethylene glycols and fatty acid esters of sorbitan, and mixtures thereof. Besides inert diluents, the oral compositions can also include adjuvants such as wetting agents, emulsifying and suspending agents, sweetening, flavoring, and perfuming agents.

Injectable preparations, for example, sterile injectable aqueous or oleaginous suspensions may be formulated according to the known art using suitable dispersing or wetting agents and suspending agents. The sterile injectable preparation may also be a sterile injectable solution, suspension or emulsion in a nontoxic parenterally acceptable diluent or solvent, for example, as a solution in 1,3-butanediol. Among the acceptable vehicles and solvents that may be employed are water, Ringer's solution, U.S.P. and isotonic sodium chloride solution. In addition, sterile, fixed oils are conventionally employed as a solvent or suspending medium. For this purpose any bland fixed oil can be employed including synthetic mono- or diglycerides. In addition, fatty acids such as oleic acid are used in the preparation of injectables.

The injectable formulations can be sterilized, for example, by filtration through a bacterial-retaining filter, or by incorporating sterilizing agents in the form of sterile solid compositions which can be dissolved or dispersed in sterile water or other sterile injectable medium prior to use.

In order to prolong the effect of a compound of the present invention, it is often desirable to slow the absorption of the compound from subcutaneous or intramuscular injection. This may be accomplished by the use of a liquid suspension of crystalline or amorphous material with poor water solubility. The rate of absorption of the compound then depends upon its rate of dissolution that, in turn, may depend upon crystal size and crystalline form. Alternatively, delayed absorption of a parenterally administered compound form is accomplished by dissolving or suspending the compound in an oil vehicle. Injectable depot forms are made by forming microencapsule matrices of the compound in biodegradable polymers such as polylactide-polyglycolide. Depending upon the ratio of compound to polymer and the nature of the particular polymer employed, the rate of compound release can be controlled. Examples of other biodegradable polymers include poly(orthoesters) and poly(anhydrides). Depot injectable formulations are also prepared by entrapping the compound in liposomes or microemulsions that are compatible with body tissues.

Compositions for rectal or vaginal administration are preferably suppositories which can be prepared by mixing the compounds of this invention with suitable non-irritating excipients or carriers such as cocoa butter, polyethylene glycol or a suppository wax which are solid at ambient temperature but liquid at body temperature and therefore melt in the rectum or vaginal cavity and release the active compound.

Solid dosage forms for oral administration include capsules, tablets, pills, powders, and granules. In such solid dosage forms, the active compound is mixed with at least one inert, pharmaceutically acceptable excipient or carrier such as sodium citrate or dicalcium phosphate and/or a) fillers or extenders such as starches, lactose, sucrose, glucose, mannitol, and silicic acid, b) binders such as, for example, carboxymethylcellulose, alginates, gelatin, polyvinylpyrrolidinone, sucrose, and acacia, c) humectants such as glycerol, d) disintegrating agents such as agar--agar, calcium carbonate, potato or tapioca starch, alginic acid, certain silicates, and sodium carbonate, e) solution retarding agents such as paraffin, f) absorption accelerators such as quaternary ammonium compounds, g) wetting agents such as, for example, cetyl alcohol and glycerol monostearate, h) absorbents such as kaolin and bentonite clay, and i) lubricants such as talc, calcium stearate, magnesium stearate, solid polyethylene glycols, sodium lauryl sulfate, and mixtures thereof. In the case of capsules, tablets and pills, the dosage form may also comprise buffering agents.

Solid compositions of a similar type may also be employed as fillers in soft and hard-filled gelatin capsules using such excipients as lactose or milk sugar as well as high molecular weight polyethylene glycols and the like. The solid dosage forms of tablets, dragees, capsules, pills, and granules can be prepared with coatings and shells such as enteric coatings and other coatings well known in the pharmaceutical formulating art. They may optionally contain opacifying agents and can also be of a composition that they release the active ingredient(s) only, or preferentially, in a certain part of the intestinal tract, optionally, in a delayed manner. Examples of embedding compositions that can be used include polymeric substances and waxes. Solid compositions of a similar type may also be employed as fillers in soft and hard-filled gelatin capsules using such excipients as lactose or milk sugar as well as high molecular weight polethylene glycols and the like.

The active compounds can also be in micro-encapsulated form with one or more excipients as noted above. The solid dosage forms of tablets, dragees, capsules, pills, and granules can be prepared with coatings and shells such as enteric coatings, release controlling coatings and other coatings well known in the pharmaceutical formulating art. In such solid dosage forms the active compound may be admixed with at least one inert diluent such as sucrose, lactose or starch. Such dosage forms may also comprise, as is normal practice, additional substances other than inert diluents, e.g., tableting lubricants and other tableting aids such a magnesium stearate and microcrystalline cellulose. In the case of capsules, tablets and pills, the dosage forms may also comprise buffering agents. They may optionally contain opacifying agents and can also be of a composition that they release the active ingredient(s) only, or preferentially, in a certain part of the intestinal tract, optionally, in a delayed manner. Examples of embedding compositions that can be used include polymeric substances and waxes.

Dosage forms for topical or transdermal administration of a compound of this invention include ointments, pastes, creams, lotions, gels, powders, solutions, sprays, inhalants or patches. The active component is admixed under sterile conditions with a pharmaceutically acceptable carrier and any needed preservatives or buffers as may be required. Ophthalmic formulation, ear drops, and eye drops are also contemplated as being within the scope of this invention. Additionally, the present invention contemplates the use of transdermal patches, which have the added advantage of providing controlled delivery of a compound to the body. Such dosage forms can be made by dissolving or dispensing the compound in the proper medium. Absorption enhancers can also be used to increase the flux of the compound across the skin. The rate can be controlled by either providing a rate controlling membrane or by dispersing the compound in a polymer matrix or gel.

As described generally above, the compounds of the invention are useful as inhibitors of protein kinases. In one embodiment, the compounds and compositions of the invention are inhibitors of one or more of ROCK, ERK, or GSK kinase, or members of the AGC sub-family of protein kinases (e.g., PKA, PDK, p70^{S6K}-1 and -2, and PKB), and thus, without wishing to be bound by any particular theory, the compounds and compositions are particularly useful for treating or lessening the severity of a disease, condition, or disorder where activation of one or more of ROCK, ERK, or GSK kinase, or members of the AGC sub-family of protein kinases (e.g., PKA, PDK, p70^{S6K}-1 and -2, and PKB) is implicated in the disease, condition, or disorder. When activation of ROCK, ERK, or GSK kinase, or members of the AGC sub-family of protein kinases (e.g., PKA, PDK, p70^{S6K}-1 and -2, and PKB) is implicated in a particular disease, condition, or disorder, the disease, condition, or disorder may also be referred to as "ROCK, ERK, GSK, AGC (e.g., PKA, PDK, p70^{S6K}-1 and -2, and PKB) -mediated disease" or disease symptom. Accordingly, in another aspect, the present invention provides a method for treating or lessening the severity of a disease, condition, or disorder where activation or one or more of ROCK, ERK, or GSK kinase, or members of the AGC sub-family of protein kinases (e.g., PKA, PDK, p70^{S6K}-1 and -2, and PKB) is implicated in the disease state.

The activity of a compound utilized in this invention as an inhibitor of ROCK, ERK, or GSK kinase, or members of the AGC sub-family of protein kinases (e.g., PKA, PDK, p70^{S6K}-1 and -2, and PKB), may be assayed *in vitro, in vivo* or in a cell line. *In vitro* assays include assays that determine inhibition of either the phosphorylation activity or ATPase activity of activated ROCK, ERK, or GSK kinase, or members of the AGC subfamily of protein kinases (e.g., PKA, PDK, p70^{S6K}-1 and -2, and PKB). Alternate *in vitro* assays quantitate the ability of the inhibitor to bind to ROCK, ERK, or GSK kinase, or members of the AGC sub-family of protein kinases (e.g., PKA, PDK, p70^{S6K}-1 and -2, and PKB). Inhibitor binding may be measured by radiolabelling the inhibitor prior to binding, isolating the inhibitor/ ROCK, inhibitor/ERK, inhibitor/GSK kinase, or inhibitor/AGC (e.g., PKA, PDK, p70^{S6K}-1 and -2, and PKB) complex and determining the amount of radiolabel bound. Alternatively, inhibitor binding may be determined by running a competition experiment where new inhibitors are incubated with ROCK, ERK, or GSK kinase, or members of the AGC sub-family of protein kinases (e.g., PKA, PDK, p70^{S6K}-1 and -2, and PKB) bound to known radioligands.

The term "measurably inhibit", as used herein means a measurable change in ROCK, ERK, or GSK kinase, or members of the AGC sub-family of protein kinases (e.g., PKA, PDK, p70^{S6K}-1 and -2, and PKB) activity between a sample comprising said composition and a ROCK, ERK, or GSK kinase, or members of the AGC sub-family of protein kinases (e.g., PKA, PDK, p70^{S6K}-1 and -2, and PKB) kinase and an equivalent sample comprising ROCK, ERK, or GSK kinase, or members of the AGC sub-family of protein kinases (e.g., PKA, PDK, p70^{S6K}-1 and -2, and PKB) kinase in the absence of said composition.

The terms "AKT-mediated disease" or "AKT-mediated condition", as used herein, mean any disease or other deleterious condition in which AKT is known to play a role. The terms "AKT-mediated disease" or "AKT-mediated condition" also mean those diseases or conditions that are alleviated by treatment with a AKT inhibitor. AKT-mediated diseases or conditions include, but are not limited to, proliferative disorders, cancer, and neurodegenerative disorders. The association of AKT, also known as protein kinase B, with various diseases has been described [Khwaja, A. Nature 1999, 401, 33-34; Yuan, Z.Q. et al., Oncogene 2000, 19, 2324-2330; Namikawa, K. et al., The Journal of Neuroscience 2000, 20, 2875-2886].

The term "PDK1-mediated condition" or "disease", as used herein, means any disease or other deleterious condition in which PDK1 is known to play a role. The term "PDK1-mediated condition" or "disease" also means those diseases or conditions that are alleviated by treatment with a PDK1 inhibitor. PDK1-mediated diseases or conditions include, but are not limited to, proliferative disorders, and cancer. Preferably, said cancer is selected from pancreatic, prostate, or ovarian cancer.

The term "PKA-mediated condition" or "disease", as used herein, means any disease or other deleterious condition in which PKA is known to play a role. The term "PKA-mediated condition" or "disease" also means those diseases or conditions that are alleviated by treatment with a PKA inhibitor. PKA-mediated diseases or conditions include, but are not limited to, proliferative disorders and cancer.

The term "p70^{S6K}-mediated condition" or "disease", as used herein, means any disease or other deleterious condition in which p70^{S6K} is known to play a role. The term "p70S6K-mediated condition" or "disease" also means those diseases or conditions that are alleviated by treatment with a p70^{S6K} inhibitor. p70^{S6K}-mediated diseases or conditions include, but are not limited to, proliferative disorders, such as cancer and tuberous sclerosis.

The terms "ERK-mediated disease" or "ERK-mediated condition", as used herein mean any disease or other deleterious condition in which ERK is known to play a role. The terms "ERK-2-mediated disease" or "ERK-2-mediated condition" also mean those diseases or conditions that are alleviated by treatment with an ERK-2 inhibitor. Such conditions include, without limitation, cancer, stroke, diabetes, hepatomegaly, cardiovascular disease including cardiomegaly, Alzheimer's disease, cystic fibrosis, viral disease, autoimmune diseases, atherosclerosis, restenosis, psoriasis, allergic disorders including asthma, inflammation, neurological disorders, and hormone-related diseases. The term "cancer" includes, but is not limited to the following cancers: breast, ovary, cervix, prostate, testis, genitourinary tract, esophagus, larynx, glioblastoma, neuroblastoma, stomach, skin, keratoacanthoma, lung, epidermoid carcinoma, large cell carcinoma, small cell carcinoma, lung adenocarcinoma, bone, colon, adenoma, pancreas, adenocarcinoma, thyroid, follicular carcinoma, undifferentiated carcinoma, papillary carcinoma, seminoma, melanoma, sarcoma, bladder carcinoma, liver carcinoma and biliary passages, kidney carcinoma, myeloid disorders, lymphoid disorders, Hodgkin's, hairy cells, buccal cavity and pharynx (oral), lip, tongue, mouth, pharynx, small intestine, colon-rectum, large intestine, rectum, brain and central nervous system, and leukemia. ERK-2 protein kinase and its implication in various diseases has been described [Bokemeyer et al., Kidney Int. 1996, 49, 1187; Anderson et al., Nature 1990, 343, 651; Crews et al., Science 1992, 258, 478; Bjorbaek et al., J. Biol. Chem. 1995, 270, 18848; Rouse et al., Cell 1994, 78, 1027; Raingeaud et al., Mol. Cell Biol. 1996, 16, 1247; Chen et al., Proc. Natl. Acad. Sci. USA 1993, 90, 10952; Oliver et al., Proc. Soc. Exp. Biol. Med. 1995, 210, 162; Moodie et al., Science 1993, 260, 1658; Frey and Mulder, Cancer Res. 1997, 57, 628; Sivaraman et al., J Clin. Invest. 1997, 99, 1478; Whelchel et al., Am. J. Respir. Cell Mol. Biol. 1997, 16, 589].

The term "GSK-3-mediated disease" as used herein, means any disease or other deleterious condition or disease in which GSK-3 is known to play a role. Such diseases or conditions include, without limitation, autoimmune diseases, inflammatory diseases, metabolic, neurological and neurodegenerative diseases (e.g., Alzheimer's disease, Huntington's disease, Parkinson's disease and basal ganglia movement disorders, chorea, dystonia, Wilson Disease, Pick Disease, frontal lobe degeneration, progessive supranuclear palsy (PSP), Creutzfeldt-Jakob Disease, taupathology and corticobasal degeneration (CBD)), psychotic disorders (e.g., schizophrenia, AIDS-associated dementia, depression, bipolar disorder, and anxiety disorders), cardiovascular diseases, allergy, asthma, diabetes, amyotrophic lateral sclerosis (AML, Lou Gehrig's disease), multiple sclerosis (MS), cardiomyocyte hypertrophy, reperfusion/ischemia, stroke, and baldness.

The term "ROCK-mediated condition" or "disease", as used herein, means any disease or other deleterious condition in which ROCK is known to play a role. The term "ROCK-mediated condition" or "disease" also means those diseases or conditions that are alleviated by treatment with a ROCK inhibitor. Such conditions include, without limitation, hypertension, angina pectoris, cerebrovascular contraction, asthma, peripheral circulation disorder, premature birth, cancer, erectile dysfunction, arteriosclerosis, spasm (cerebral vasospasm and coronary vasospasm), retinopathy (e.g., glaucoma), inflammatory disorders, autoimmune disorders, AIDS, osteoporosis, myocardial hypertrophy, ischemia/reperfusion-induced injury, and endothelial dysfunction.

In other embodiments, the invention relates to a method of enhancing glycogen synthesis and/or lowering blood levels of glucose in a patient in need thereof, comprising administering to said patient a therapeutically effective amount of a composition comprising a compound of formula **I.** This method is especially useful for diabetic patients.

In yet another embodiment, the invention relates to a method of inhibiting the production of hyperphosphorylated Tau protein in a patient in need thereof, comprising administering to said patient a therapeutically effective amount of a composition comprising a compound of formula **I.** This method is especially useful in halting or slowing the progression of Alzheimer's disease.

In still another embodiments, the invention relates to a method of inhibiting the phosphorylation of β-catenin in a patient in need thereof, comprising administering to said patient a therapeutically effective amount of a composition comprising a compound of formula **I.** This method is especially useful for treating schizophrenia.

It will also be appreciated that the compounds and pharmaceutically acceptable compositions of the present invention can be employed in combination therapies, that is, the compounds and pharmaceutically acceptable compositions can be administered concurrently with, prior to, or subsequent to, one or more other desired therapeutics or medical procedures. The particular combination of therapies (therapeutics or procedures) to employ in a combination regimen will take into account compatibility of the desired therapeutics and/or procedures and the desired therapeutic effect to be achieved. It will also be appreciated that the therapies employed may achieve a desired effect for the same disorder (for example, an inventive compound may be administered concurrently with another agent used to treat the same disorder), or they may achieve different effects (e.g., control of any adverse effects). As used herein, additional therapeutic agents that are normally administered to treat or prevent a particular disease, or condition, are known as "appropriate for the disease, or condition, being treated".

For example, chemotherapeutic agents or other anti-proliferative agents may be combined with the compounds of this invention to treat proliferative diseases and cancer. Examples of known chemotherapeutic agents include, but are not limited to, For example, other therapies or anticancer agents that may be used in combination with the inventive anticancer agents of the present invention include surgery, radiotherapy (in but a few examples, gamma.-radiation, neutron beam radiotherapy, electron beam radiotherapy, proton therapy, brachytherapy, and systemic radioactive isotopes, to name a few), endocrine therapy, biologic response modifiers (interferons, interleukins, and tumor necrosis factor (TNF) to name a few), hyperthermia and cryotherapy, agents to attenuate any adverse effects (e.g., antiemetics), and other approved chemotherapeutic drugs, including, but not limited to, alkylating drugs (mechlorethamine, chlorambucil, Cyclophosphamide, Melphalan, Ifosfamide), antimetabolites (Methotrexate), purine antagonists and pyrimidine antagonists (6-Mercaptopurine, 5-Fluorouracil, Cytarabile, Gemcitabine), spindle poisons (Vinblastine, Vincristine, Vinorelbine, Paclitaxel), podophyllotoxins (Etoposide, Irinotecan, Topotecan), antibiotics (Doxorubicin, Bleomycin, Mitomycin), nitrosoureas (Carmustine, Lomustine), inorganic ions (Cisplatin, Carboplatin), enzymes (Asparaginase), and hormones (Tamoxifen, Leuprolide, Flutamide, and Megestrol), Gleevec™, adriamycin, dexamethasone, and cyclophosphamide. For a more comprehensive discussion of updated cancer therapies see, http://www.nci.nih.gov/, a list of the FDA approved oncology drugs at http://www.fda.gov/cder/cancer/druglistframe.htm, and The Merck Manual, Seventeenth Ed. 1999, the entire contents of which are hereby incorporated by reference.

Other examples of agents the inhibitors of this invention may also be combined with include, without limitation: treatments for Alzheimer's Disease such as Aricept^{®} and Excelon^{®}; treatments for Parkinson's Disease such as L-DOPA/carbidopa, entacapone, ropinrole, pramipexole, bromocriptine, pergolide, trihexephendyl, and amantadine; agents for treating Multiple Sclerosis (MS) such as beta interferon (e.g., Avonex^{®} and Rebif^{®}), Copaxone^{®}, and mitoxantrone; treatments for asthma such as albuterol and Singulair^{®}; agents for treating schizophrenia such as zyprexa, risperdal, seroquel, and haloperidol; anti-inflammatory agents such as corticosteroids, TNF blockers, IL-1 RA, azathioprine, cyclophosphamide, and sulfasalazine; immunomodulatory and immunosuppressive agents such as cyclosporin, tacrolimus, rapamycin, mycophenolate mofetil, interferons, corticosteroids, cyclophosphamide, azathioprine, and sulfasalazine; neurotrophic factors such as acetylcholinesterase inhibitors, MAO inhibitors, interferons, anti-convulsants, ion channel blockers, riluzole, and anti-Parkinsonian agents; agents for treating cardiovascular disease such as beta-blockers, ACE inhibitors, diuretics, nitrates, calcium channel blockers, and statins; agents for treating liver disease such as corticosteroids, cholestyramine, interferons, and anti-viral agents; agents for treating blood disorders such as corticosteroids, anti-leukemic agents, and growth factors; and agents for treating immunodeficiency disorders such as gamma globulin.

The amount of additional therapeutic agent present in the compositions of this invention will be no more than the amount that would normally be administered in a composition comprising that therapeutic agent as the only active agent. Preferably the amount of additional therapeutic agent in the presently disclosed compositions will range from about 50% to 100% of the amount normally present in a composition comprising that agent as the only therapeutically active agent.

The compounds of this invention or pharmaceutically acceptable compositions thereof may also be incorporated into compositions for coating implantable medical devices, such as prostheses, artificial valves, vascular grafts, stents and catheters. Accordingly, the present invention, in another aspect, includes a composition for coating an implantable device comprising a compound of the present invention as described generally above, and in classes and subclasses herein, and a carrier suitable for coating said implantable device. In still another aspect, the present invention includes an implantable device coated with a composition comprising a compound of the present invention as described generally above, and in classes and subclasses herein, and a carrier suitable for coating said implantable device.

Vascular stents, for example, have been used to overcome restenosis (renarrowing of the vessel wall after injury). However, patients using stents or other implantable devices risk clot formation or platelet activation. These unwanted effects may be prevented or mitigated by pre-coating the device with a pharmaceutically acceptable composition comprising a kinase inhibitor. Suitable coatings and the general preparation of coated implantable devices are described in US Patents 6,099,562; 5,886,026; and 5,304,121. The coatings are typically biocompatible polymeric materials such as a hydrogel polymer, polymethyldisiloxane, polycaprolactone, polyethylene glycol, polylactic acid, ethylene vinyl acetate, and mixtures thereof. The coatings may optionally be further covered by a suitable topcoat of fluorosilicone, polysaccarides, polyethylene glycol, phospholipids or combinations thereof to impart controlled release characteristics in the composition.

Another aspect of the invention relates to inhibiting ROCK, ERK, GSK, or AGC (e.g., PKA, PDK, p70^{S6K}-1 and -2, and PKB) activity in a biological sample or a patient, which method comprises administering to the patient, or contacting said biological sample with a compound of formula **I** or a composition comprising said compound. The term "biological sample", as used herein, includes, without limitation, cell cultures or extracts thereof; biopsied material obtained from a mammal or extracts thereof; and blood, saliva, urine, feces, semen, tears, or other body fluids or extracts thereof.

Inhibition of ROCK, ERK, GSK, or AGC (e.g., PKA, PDK, p70^{S6K}-1 and -2, and PKB) kinase activity in a biological sample is useful for a variety of purposes that are known to one of skill in the art. Examples of such purposes include, but are not limited to, blood transfusion, organ-transplantation, biological specimen storage, and biological assays.

### EXAMPLES

GENERAL EXPERIMENTAL PROCEDURES:

As depicted in Schemes 7, 8, 9, 10, and 11 below, in certain exemplary embodiments, compounds are prepared according to the following general procedures. It will be appreciated that although the general methods depict the synthesis of compounds of general formula **VII,** the following general methods can be applied to all compounds and subclasses and species of each of these compounds, as described herein.

General Method A: Acylation of amines 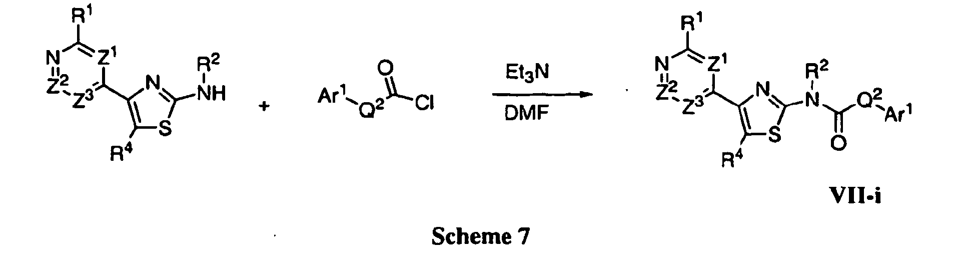 0.25 mmol of amine, and 0.5 mmol of acid chloride were dissolved in 2 mL of anhydrous DMF. 0.75 mmol of Et₃N was then added to the reaction mixture, and the mixture was stirred at RT for overnight. After completion of the reaction, EtOAc was added, the organic layer was washed with H₂O and brine, and was then dried over Na₂SO₄. Removal of the solvent gave a solid, **VII-i,** which was further purified by preparative HPLC.

General Method B: Acylation of amines 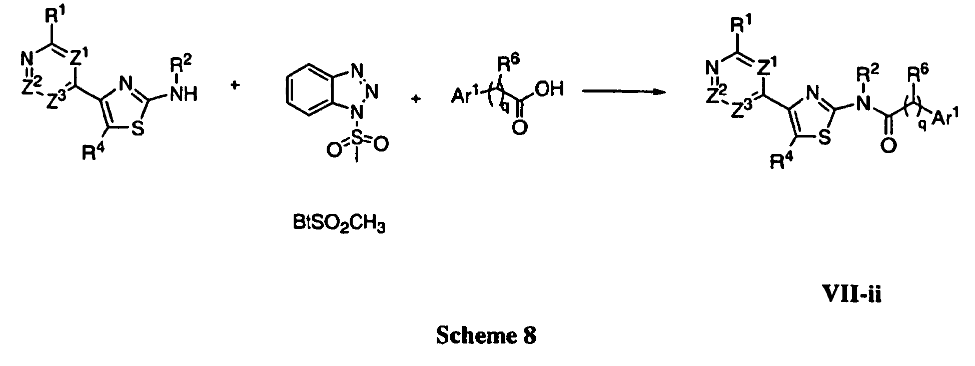 A mixture of BtSO₂CH₃ (preparation described below) (0.25 mmol), acid (0.25 mmol), and Et₃N (0.35mmol) was refluxed in dry THF for about 20 min. Amine (0.25 mmol) was then added to the reaction mixture, and the mixture was refluxed for 18 h. After the mixture was concentrated, EtOAc (5 mL) was added, and the organic phase was washed with 2 M NaOH and dried over anhydrous MgSO₄. Removal of the solvent gave a solid, **VII-ii,** which was purified by preparative HPLC.

General Method C: Acylation of amines

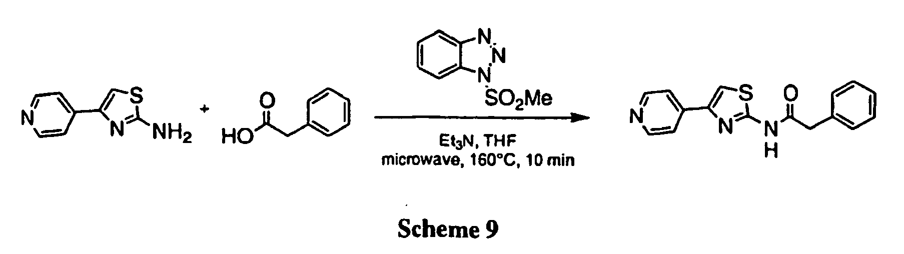

Amine (1 mmol), carboxylic acid (1.2 mmol) and Bt-SO₂Me (1.2 mmol) are combined in a microwave reaction vessel. Anhydrous THF (2 mL) is added followed by triethylamine (2 mmol) and the mixture heated by microwave irradiation at 160°C for 10 minutes. Product is isolated by precipitation following addition of acetonitrile, or by preparative HPLC.

Standard protection and deprotection of amino and hydroxyl functionalities:

General Method D: Protection of amino groups

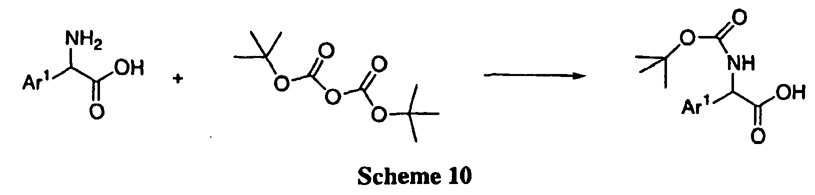

0.25 mmol of amine, 0.25 mmol of Boc anhydride were mixed in 2 mL of anhydrous CH₂Cl₂. To the reaction mixture, 0.75 mmol of Et₃N was added and the mixture was stirred at RT for overnight. The solvent was evaporated to give the Boc protected amine.

General Method E: Deprotection of Boc-protected amines

To the Boc protected amine (0.25 mmol) in a vial, 2 mL 4N HCl in dioxane was added and the reaction mixture was stirred at RT for 30 min. The solvent was evaporated to give the free amine product.

General Method F: Protection of phenols and alcohols

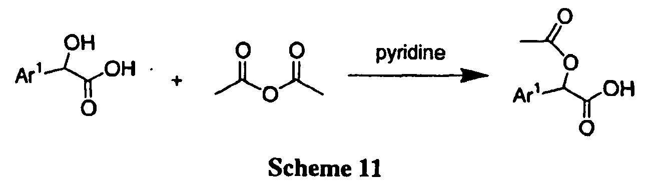

Hydroxy acid (2.5 mol) was stirred with acetic anhydride (0.57 mL, 6 mol) in pyridine (5mL) overnight and then evaporated in vacuo. The resulting oil was partitioned between EtOAc and 1N HCl and the resulting organic layer washed successively with 1 N HCl, water and brine, dried over MgSO₄, and evaporated to dryness.

General Method G: Deprotection of acetylated phenols and alcohols

The acetyl-protected alcohol or phenol (0.25 mmol) was dissolved in EtOH, 0.5 mL 2N NaOH was added and the mixture was stirred at RT for 1h. The solvent was evaporated and redissolved in DMF/CH₃CN/H₂O, and subjected to preparative HPLC for purification.

General Method H: Preparation of phenylacetic acids

Substituted benzaldehyde (5 mmol) and zinc iodide (10 mg) were dissolved or suspended in anhydrous acetonitrile (5 - 10 mL). Trimethylsilyl cyanide (12 mmol) was added dropwise and the mixture stirred at room temperature overnight. The mixture was rotary evaporated and the residue dissolved in glacial acetic acid (2 mL) and concentrated hydrochloric acid (3 mL). Tin (II) chloride dihydrate (12 mmol) was added and the mixture heated to reflux for 1 - 2 hours. To the cooled mixture was added water (20 mL) and the mixture was extracted with methylene chloride (3 x 15 mL). The extracts were washed with water (x 2) and brine and dried over MgSO₄. The solution is concentrated and the product precipitated by addition of hexane.

General Method I: Preparation of α-hydroxyphenylacetic acids

Substituted benzaldehyde (5 mmol) and zinc iodide (10 mg) were dissoved or suspended in anhydrous acetonitrile (5 - 10 mL). Trimethylsilyl cyanide (12 mmol) was added dropwise and the mixture stirred at room temperature overnight. The mixture was rotary evaporated and the residue dissolved in glacial acetic acid (2 mL) and concentrated hydrochloric acid (3 mL) and the mixture heated to reflux for 1 - 2 hours. To the cooled mixture was added water (20 mL) and the mixture was extracted with methylene chloride (3 x 15 mL). The extracts were washed with water (x 2) and brine and dried over MgSO₄. The solution is concentrated and the product precipitated by addition of hexane.

Although the preparation of certain amines are described below, it will be appreciated that a variety of alternate amines can be prepared as described generally below and can be utilized in the preparation of compounds of the invention.

EXPERIMENTAL PROCEDURES:

Preparation of N-(1-Methanesulfonyl)benzotriazole (BtSO₂CH₃):

To an ice-cold solution of benzotriazole (11.9g, 0.10 mol) and pyridine (12.0 g, 0.16 mol) in dry toluene (120 mL) was added methylsulfonyl chloride (9.3 mL, 0.12 mol) in toluene (30 mL) dropwise. The mixture was then stirred overnight at room temperature. EtOAc (150 mL) and H₂O (100 mL) were added, the organic layer was separated, successively washed with water and brine, and dried over anhydrous MgSO₄. Removal of solvents in vacuo gave BtSO₂CH₃ as a white solid.

The synthesis of certain exemplary amines (described generally above) are described more specifically below. It will be appreciated that a variety of alternate amines can be prepared according to methods known in the art and can be utilized in the preparation of compounds of the invention.

Preparation of 5-Pyridin-4-yl-[1,3,4]thiadiazol-2-ylamine

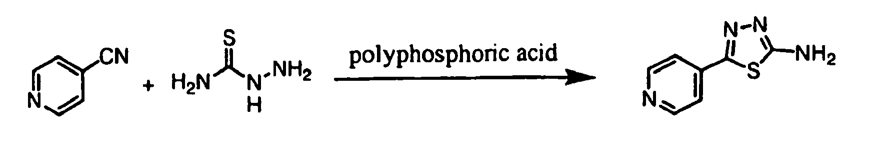

A mixture of 4-cyanopyridine (5.2 g, 50 mmol) and thiosemicarbazide (6.37 g, 70 mmol) was heated in polyphosphoric acid at 100°C overnight. The reaction mixture was poured onto 200 g ice and the pH was adjusted to approximately 7.5 by addition of 6N NaOH. The product precipitated and was filtered and dried to afford 5-pyridin-4-yl-[1,3,4]thiadiazol-2-ylamine (4.59 g, 51%). ¹H NMR CD₃OD: 7.8 (d, 2H), 8.62 (d, 2H).

Preparation of 2-Pyridin-4-yl-thiazol-5-ylamine

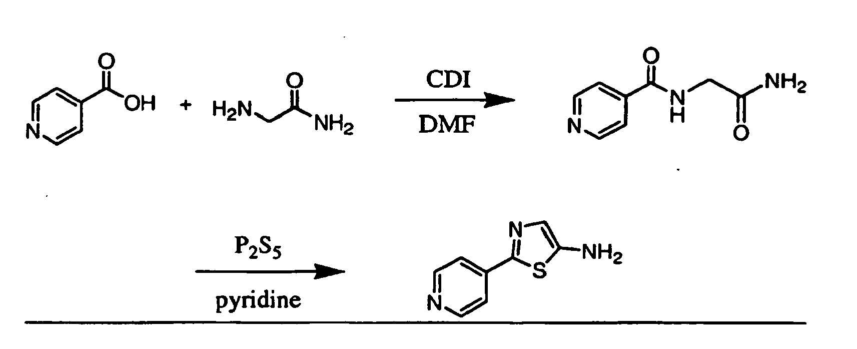

**N-Carbamoylmethyl-isonicotinamide:** To 200 mL DMF was added isonicotinic acid (12.3 g, 0.1mol), and carbonyl diimidazole. The mixture was stirred at room temperature for 1h then glycineamide hydrochloride and 200 mL THF were added and the mixture was stirred overnight. To the mixture was added 300 mL acetonitrile to precipitate the product, which was filtered, washed and dried to afford N-carbamoylmethyl-isonicotinamide (13.0 g, 72.6%). ¹H NMR d⁶-DMSO: 3.85 (d, 2H), 7.08 (br s, 1H), 7.46 (br s, 1H), 7.8 (d, 2H), 8.73 (d, 2H), 9.0 (br, 1H).

**2-Pyridin-4-yl-thiazol-5-ylamine:** A mixture of N-carbamoylmethyl-isonicotinamide (10.8 g, 0.06mol), and phosphorus pentasulfide (13.40 g, 0.06mol) in pyridine (250 mL) was heated at 100°C for 6h. The mixture was poured into to NaHCO₃ aqueous solution, the product was extracted into ethyl acetate, the solvent was removed under reduced pressure, the product was purified by silica gel flash chromatography to afford 2-pyridin-4-yl-thiazol-5-ylamine in 20% yield. ¹H NMR d6-DMSO: 6.36 (s, 2H), 6.95 (s, 1H), 7.59 (d, 2H), 8.54 (d, 2H).

Preparation of 5-Pyridin-4-yl-thiophen-2-ylamine

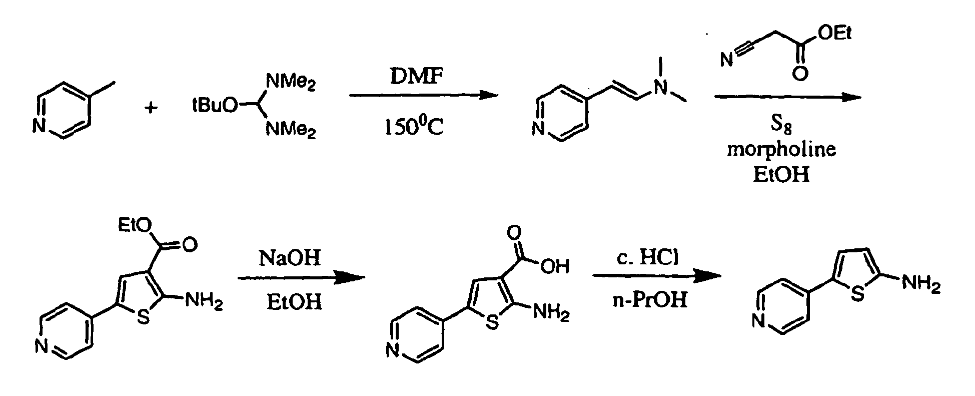

**Dimethyl-(2-pyridin-4-yl-vinyl)-amine:** 4-Methyl-pyridine (50 mmol) and Bredereck's reagent (C-tert-Butoxy-N,N,N',N'-tetramethyl-methanediamine, 62.5 mmol) were dissolved in 12.5 mL of DMF in a sealed tube and the reaction mixture was heated to 150□C overnight. The solvent was evaporated to give a brown solid that was carried on to the next step.

**2-Amino-5-pyridin-4-yl-thiophene-3-carboxylic** acid **ethyl ester:** A stirred solution of dimethyl-(2-pyridin-4-yl-vinyl)-amine and ethyl cyanoacetate (50 mmoL) in 60mL EtOH was treated with 50 mmol of elemental sulfur and 2 mL morphline at room temperature and stirred overnight. Precipitation formed. Cooled to -20°C and filtered off the solid, washed with hexane to give yellow solid. (60% yield). ¹H NMR CDCl₃: 8.48 (2H, m), 7.50 (1H, s), 7.29 (2H, m), 6.30 (2H, br), 4.32 (2H, q), 1.37 (3H, t).

**2-Amino-5-pyridin-4-yl-thiophene-3-carboxylic acid:** To 2-amino-5-pyridin-4-yl-thiophene-3-carboxylic acid ethyl ester (1 mmol) in 4 mL EtOH was added 1 mL 2N NaOH and the reaction mixture was refluxed for 2 hours, then cooled to room temperature. A precipitate formed, which was filtered. The filtrate was diluted with 2mL H₂O and neutralized with dilute H₂SO₄ until more precipitate formed. The mixture was cooled and the solid filtered. The combined solids were used without further purification. MS [M+H] = 221. ¹H NMR CD₃OD: 8.22 (2H, m), 7.83 (1H, s), 7.65 (2H, m).

**5-Pyridin-4-yl-thiophen-2-ylamine:** 2-Amino-5-pyridin-4-yl-thiophene-3-carboxylic acid was dissolved in 4mL of n-propanol, 2mL of conc. HCl was added and the reaction mixture was stirred at 70□C for 24 hours. The reaction mixture was cooled and the solid filtered, to give 5-pyridin-4-yl-thiophen-2-ylamine as a yellow solid (82%). MS [M+H] = 177.

Preparation of 4-Pyridin-4-yl-thiophen-2-ylamine:

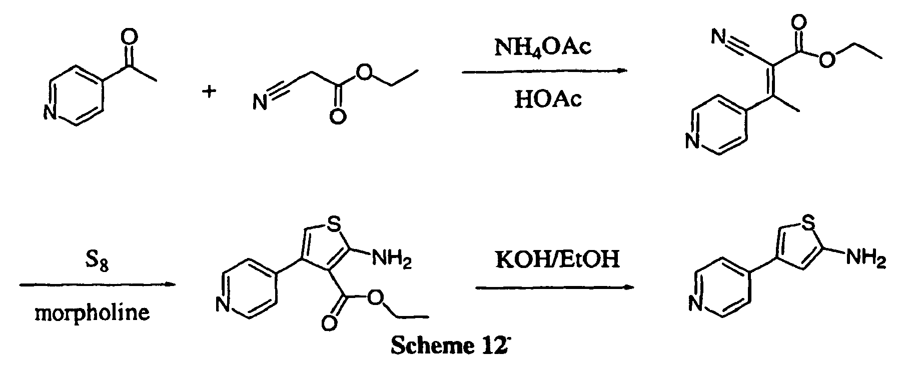

**2-Cyano-3-pyridin-4-yl-but-2-enoic acid ethyl ester:** Ethyl cyanoacetate (60 mmol, 6.78 g) and 4-pyridylacetophenone (60 mmol, 7.26g) were dissolved in 35 mL of dry benzene to which 7 mmol of ammonium acetate and 1.5 mL glacial acetic acid were added. The mixture was refluxed under a Dean-Stark trap until the formation of H₂O ceased. The mixture was cooled, diluted with benzene, and washed with H₂O. The organic layer was dried with Na₂SO₄, filtered, and concentrated in vacuo. The reaction mixture was carried on for next step without purification.

**2-Amino-4-pyridin-4-yl-thiophene-3-carboxylic acid ethyl ester.** To a stirred solution of 2-Cyano-3-pyridin-4-yl-but-2-enoic acid ethyl ester in 100mL EtOH was added 60 mmol of sulfur and 1 mL morpholine. The mixture was stirred at room temperature overnight. The precipitate was filtered, washed with cold EtOH and hexane to give a light yellow solid. The filtrate was concentrated and redissolved in cold ethanol, filtered and washed with hexane to give more product. Overall yield is 60%. NMR 500MHz, CDCl₃: 8.57 (2H, m), 7.31 (2H, m), 6.25 (2H, br), 6.17 (1H, s), 4.10 (2H, q), 0.99 (3H, t).

**4-Pyridin-4-yl-thiophen-2-ylamine:** 2.49g of 2-Amino-4-pyridin-4-yl-thiophene-3-carboxylic acid ethyl ester (10 mmol) was dissolved into a mixture of 10 mL 20% KOH and EtOH (10 mL) and the reaction mixture was refluxed for 18 hours. The mixture was cooled to room temperature and 10 mL H₂O was added and the mixture was stirred at room temperature overnight. The precipitate was filtered, washed with H₂O, and dried. To afford the product as a light yellow solid (65% yield). NMR 500MHz. CD₃OD: 8.45 (2H, m), 7.60 (2H, m), 7.07 (1H, s), 6.54 (1H, s).

Preparation of 3-Pyridin-4-yl-[1,2.4]thiadiazol-5-ylamine

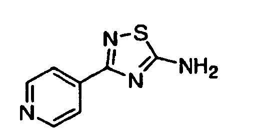

3-Pyridin-4-yl-[1,2,4]thiadiazol-5-ylamine was prepared as described in EP 0455356. NMR 500MHz, d6-DMSO: 8.7 (br s, 2H), 8.2 (br s, 2H), 7.9 (m, 2H).

Preparation of 3-Pyridin-4-yl-isothiazol-5-ylamine

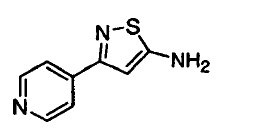

3-Pyridin-4-yl-isothiazol-5-ylamine was prepared according to the following scheme as described in EP129407.

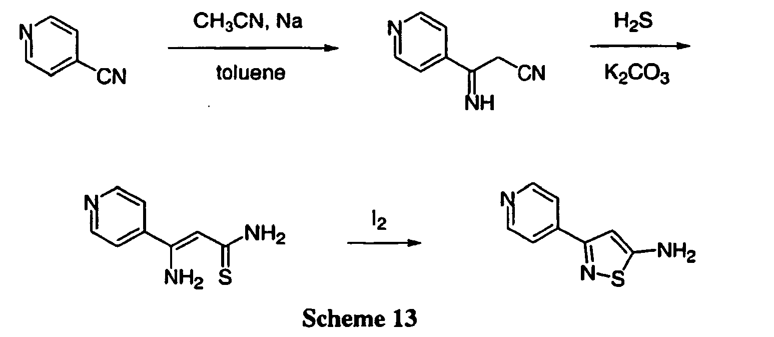

Preparation of 4-Pyrimidin-4yl-thiophen-2-ylamine

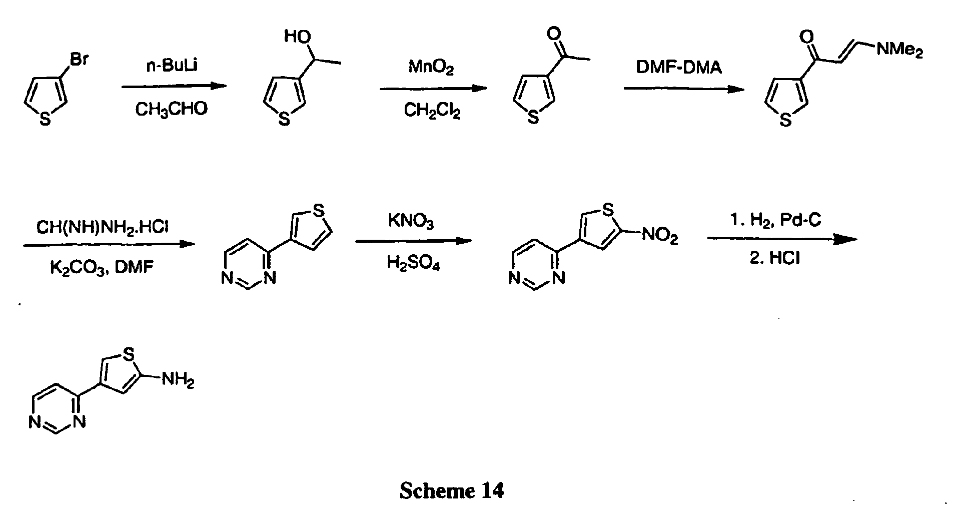

**1-(3-Thienyl)-ethanol.** In a 3 L four-necked round-bottomed flask equipped with an overhead stirrer, addition funnel, and low-temperature thermometer, 202.4 g (1.24 mol) of 3-bromothiophene was dissolved in 1L of 10% THF-hexane. The solution was cooled to - 10°C using a dry-ice/acetone bath. n-BuLi was added dropwise via the addition funned. A white solid precipitated during the addition. The reaction was stirred for one hour and 80 mL (excess) of acetaldehyde was added. The reaction was stirred for 10 minutes, poured into 1N HCl and extracted with diethyl ether. The extract was dried (MgSO₄) and filtered over a plug of silica gel. The plug was eluted with diethyl ether and the filtrate was evaporated to afford 82.84 g (53%) of a light yellow oil that was shown by ¹H NMR to be a 3:1 mixture of the desired product and the isomeric alcohol at the 2-position of the thiophene. The mixture was used in the next step. 3-isomer: ¹H NMR (500 MHz, CDCl₃) δ 7.29 (dd, 1H), 7.18 (d, 1H), 7.09 (d, 1H), 4.96 (m, 1H), 1.52 (d, 3H). 2-isomer: ¹H NMR (500 MHz, CDCl₃) δ 7.22 (d, 1H), 6.96 (m, 1H), 6.92 (d, 1H), 5.23 (m, 1H), 1.57 (d, 3H).

3-Acetylthiophene. The mixture of alcohols from the previous procedure was dissolved in 700 mL of toluene. Manganese oxide (131.82 g, excess) was added and the mixture was stirred at reflux overnight. The mixture was cooled, filtered, and evaporated in vacuo. The oil was dissolved in CH₂Cl₂ and filtered over a plug of silica gel. The plug was eluted with CH₂Cl₂ and the filtrate was evaporated in vacuo to afford the product as a yellow solid. The product was a 3:1 mixture of the 3-acetyl and 2-acetylthiophene. 3-isomer: ¹H NMR (500 MHz, CDCl₃) δ 8.05 (dd, 1H), 7.53 (d, 1H), 7.31 (dd, 1H). 2.52 (s, 3H). 2-isomer: ¹H NMR (500 MHz, CDCl₃) δ 7.65 (dd, 1H), 7.51 (d, 1H), 7.10 (d, 1H).

**3-Dimethylamino-1-thiophen-3-yl-propenone.** The crude 3-acetylthiophene (42.81 g, 339 mmol) was mixed with 250 mL of dimethylformamide dimethyl acetal and heated to reflux overnight. The red solution was evaporated in vacuo. The resulting oil was dissolved in CH₂Cl₂ and washed with water. The organic solution was dried (MgSO₄), and evaporated in vacuo to afford 61.04 g (99%) of a light yellow oil that crystallized upon standing. ¹H NMR (500 MHz, CDCl₃) δ 7.89 (d, 1H), 7.74 (d, 1H), 7.52 (d, 1H), 7.25 (dd, 1H), 5.56 (d, 1H), 3.05 (br s, 3H), 2.91 (br s, 3H).

**4-Thiophen-3-yl-pyrimidine.** 3-Dimethylamino-1-thiophen-3-yl-propenone (61.04 g, 337 mmol) was dissolved in 500 mL of DMF. Formamidine hydrochloride (44.65 g, 555 mmol) was added along with 62.3 g (451 mmol) of K₂CO₃. The mixture was heated to 80°C overnight. The mixture was poured into water and extracted with diethyl ether. The extract was dried (MgSO₄) and evaporated in vacuo to afford a brown solid. The solid was dissolved in CH₂Cl₂ and filtered over a plug of silica gel. The plug was eluted with CH₂Cl₂ and the filtrate was evaporated in vacuo to afford 44.7 g (82%) of 4-thiophen-3-yl-pyrimidine. ¹H NMR (500 MHz, CDCl₃) δ 9.18 (s, 1H), 8.70 (d, 1H), 8.13 (d, 1H), 7.69 (d, 1H), 7.53 (d, 1H), 7.43 (dd, 1H).

**4-(5-Nitro-thiophen-3-yl)-pyrimidine.** 4-Thiophen-3-yl-pyrimidine (640 mg, 3.8 mmol) was dissolved in 10 mL of 98% sulfuric acid forming a red solution. The mixture was cooled to 0°C and 390 mg (3.86 mmol) of KNO₃ was added. The mixture was stirred for 10 minutes at 0°C and then one hour at room temperature. The mixture was poured into water and extracted numerous times with CH₂Cl₂. The extract was dried and filtered over a plug of silica gel. The plug was eluted with CH₂Cl₂ and the filtrate was evaporated to afford 4-(5-nitro-thiophen-3-yl)-pyrimidine as a yellow solid. ¹H NMR (500 MHz, CDCl₃) δ 9.22 (s, 1H), 8.81 (d, 1H), 8.49 (s, 1H), 8.33 (s, 1H), 7.57 (d, 1H).

**4-Pyrimidin-4-yl-thiophen-2-ylamine hydrochloride.** To a solution of 4-(5-nitro-thiophen-3-yl)-pyrimidine (0.20 g, 0.97mmol) in 4 mL of 3:1 EtOAc - MeOH was added 50 mg of 10% palladium on carbon. The reaction was stirred at ambient temperature under 1 atmosphere of hydrogen gas for 4 hours, until no starting material remained by tlc analysis. The catalyst was filtered and washed with EtOAc and the filtrate was cooled to 0°C. The volume of the filtrate volume was doubled with Et₂O, and then to the solution was added a solution of 4N HCl in dioxane (500 µL, 2 mmol). A light yellow solid precipitated immediately, which was stirred for approximately 5 minutes at 0°C, and then filtered. The solids were washed with copious amounts of Et₂O with care taken not to expose the compound to air, and quickly transferred to high vacuum for drying. The solid was dried *in vacuo* to give 160 mg (77%) of 4-pyrimidin-4-yl-thiophen-2-ylamine hydrochloride as a pale yellow solid.
¹H NMR (500 MHz, DMSO-d6) δ 9.17 (t, 1H, J=1.92H2), 8.82-8.79 (m, 1H), 8.10 (s, 1H), 7.94-7.90 (m, 1H),7.3 (bs, 2H). FIA/MS [M+H]⁺ = 308.

Preparation of 5-Pyrimidin-4-yl-thiophen-3-ylamine

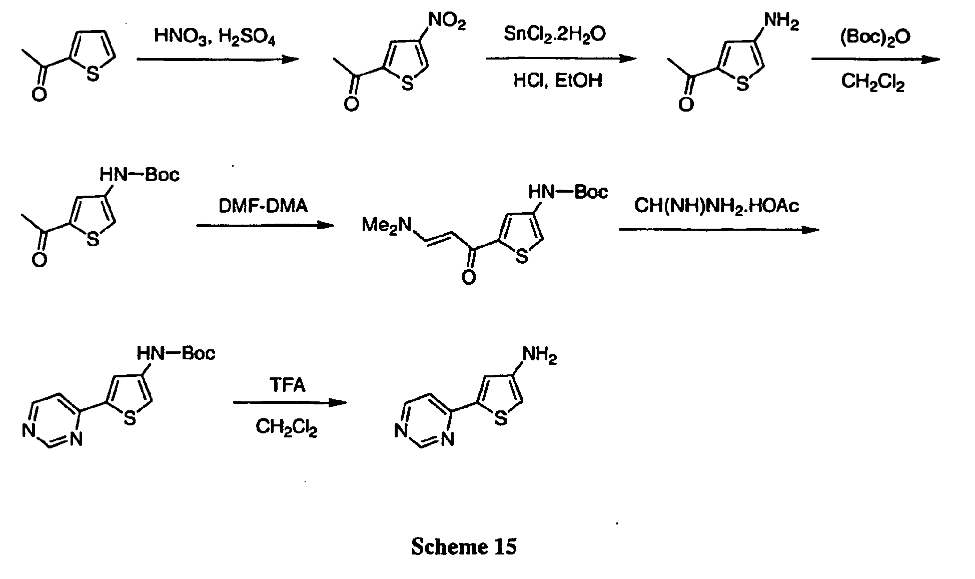

**2-Acetyl-4-nitrothiophene:** Concentrated sulfuric acid (42.4 g, 0.44mol) was cooled to -10 °C and 2-acetylthiophene (20.2 g, 0.16mol) slowly added over 2 hours. Then a cold (-10 °C) mixture of 90% nitric acid (37.8 g, 0.60 mol) and concentrated sulfuric acid (28.1 g, 0.28 mol) was slowly added over 2 hours and the reaction mixture was stirred at -10 °C for another hour. The reaction mixture was slowly poured onto 300 g ice and the product precipitated out. The crude 2-acetyl-4-nitrothiophene was washed with ether and filtered to afford 8.5g pure product. ¹H NMR (500 MHz, CDCl₃) δ 2.6 (s, 3H), 8.14 (d, 1H), 8.5 (d, 1H).

**2-Acetyl-4-aminothiophene:** To an ethanol suspension of 0.524 g (0.003 mol) 2-acetyl-4-nitrothiophene and 2.1 g (0.009 mol) tin(II) chloride dihydrate was added 3 mL 6N HCl (0.018mol). The reaction mixture was stirred at 70°C for 1 hour, then cooled to room temperature. The reaction mixture was adjusted to pH10 by addition of 6N NaOH, and the product was extracted into ethyl acetate. The product was purified by silica gel chromatography, to afford 0.20 g 2-acetyl-4-aminothiophene (47%). ¹H NMR (500 MHz, CDCl₃) δ 2.52 (s, 3H), 6.5 (d, 1H), 7.23 (d, 1H).

**2-Acetyl-4-(t-butoxycarbonylamino)-thiophene:** A solution of 2-acetyl-4-aminothiophene (1.2 g, 8.5 mmol) and di-tert-butyl dicarbonate (2.78 g, 12.7 mmol) in dichloromethane was stirred at room temperature over night. The solvent was removed by rotary evaporation and the product was purified by silica gal chromatography to afford 2-acetyl-4-(t-butoxycarbonylamino)-thiophene (1.48 g, 72%). ¹H NMR (500 MHz, CDCl₃) δ 1.5 (s, 9H), 2.5 (s, 3H), 6.66 (s, 1H), 7.36 (s, 1H), 7.65 (s, 1H).

**2-(3-Dimethylaminopropenoyl)-4-(t-butoxycarbonylamino)-thiophene:** A mixture of 2-acetyl-4-(t-butoxycarbonylamino)-thiophene (0.15 g, 0.622 mmol) and *N,N-*dimethylforamide dimethyl acetal (0.296 g, 2.49mmol) was stirred at 75°C for 24 h. The reaction mixture was rotary evaporated. ¹H NMR of the crude product indicated 90% product and 10% starting material. ¹H NMR (500 MHz, CDCl₃) δ 1.45 (s, 9H), 2.85 (s, 3H), 3.05 (s, 3H), 5.47 (d, 1H), 6.58 (s, 1H), 7.47 (s, 1H), 7.7 (d, 1H).

**N-t-Butoxycarbonyl-5-pyrimidin-4-yl-thiophen-3-ylamine:** 2-(3-Dimethylamino-propenoyl)-4-(t-butoxycarbonylamino)-thiophene (0.184 g, 0.622mmol) and formamidine acetate (0.388 g, 3.73 mmol) were heated at 115°C for 6 h. The mixture was cooled to room temperature and ethyl acetate and brine added. The organic phase was dried with MgSO₄ and the product was purified by silica gel chromatography to afford N-t-butoxycarbonyl-5-pyrimidin-4-yl-thiophen-3-ylamine (0.1 g, 58%) ¹H NMR (500 MHz, CDCl₃) δ 1.48 (s, 9H), 6.68 (s, 1H), 7.21 (s, 1H), 7.45 (dd, 1H), 7.7 (s, 1H), 8.58 (d, 1H), 9.04 (d, 1H).

**5-Pyrimidin-4-yl-thiophen-3-ylamine:** To a solution of N-t-butoxycarbonyl-5-pyrimidin-4-yl-thiophen-3-ylamine (0.1 g, 0.36mmol) in methylene chloride was added 0.5 mL TFA. The reaction mixture was stirred at room temperature for 3 h, then rotary evaporated. The product was purified by silica gel chromatography to afford 5-pyrimidin-4-yl-thiophen-3-ylamine (60 mg, 93%). ¹H NMR (500 MHz, CDCl₃) δ 6.95 (s, 1H), 7.56 (s, 2H), 8.69 (s, 1H), 9.10 (s, 1H).

Preparation of 2-(Pyrid-4-yl)-4-amino-thiazole

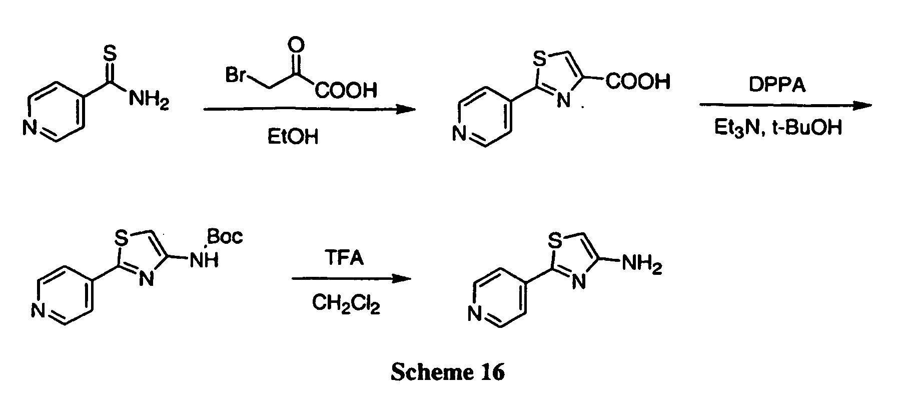

**2-(Pyrid-4-yl)-thiazole-4-carboxylic acid**

Thioisonicotinamide (22.06 g, 0.16 mol) was suspended in reagent alcohol (280 mL) and warmed to 40 °C. Bromopyruvic acid (28.3 g, 0.16 mol) was dissolved in reagent alcohol (100 mL) and added via addition funnel. The reaction was refluxed for 2.5 h, then cooled to 4°C. The precipitate was filtered, washed with reagent alcohol and dried. The product was suspended in reagent alcohol (100 mL) and aqueous 2 N NaOH (80 mL) added. After stirring for 1 h the mixture was diluted with water (500 mL) and extracted with EtOAc (2 x 100 mL). The aqueous solution was acidified with 20% aqueous citric acid solution (500 mL) and the resulting precipitate was filtered, washed with water and dried. Yield 15.1 g. MS: [M+H] = 207; 1H NMR (d6-DMSO) 8.77 (2H, d), 8.67 (1H, s), 7.96 (2H, d), 1.50 (9H, s).

**2-(Pyrid-4-yl)-4-(t-butoxycarbonylamino)-thiazole**

2-(Pyrid-4-yl)-thiazole-4-carboxylic acid (16.96 g, 82 mmol) was suspended in t-butanol (250 mL) at 30 °C. Triethylamine (18 mL, 129 mmol) was added, followed by dropwise addition of diphenylphosphorylazide (23.5 mL, 109 mmol). The solution was brought to reflux for 5 h, then allowed to cool. Solvent was partially removed by rotary evaporation, whereupon a gelatinous mass formed. Ethyl acetate (300 mL) was added to give a clear, brown solution. Upon standing a solid precipitated, which was filtered, washed with EtOAc and dried. The filtrate was diluted to 1000 mL total volume with EtOAc and washed with water, saturated aqueous NaHCO₃ (x2), water, 5% aqueous citric acid (x2), water and brine. The solution was dried over Na₂SO₄ and evaporated to dryness. The solid was combined with that isolated previously and recrystallized from hot methanol. Yield 11.9 g. MS: [M+H] = 278.1; 1H NMR (d6-DMSO) 10.45 (1H, br s), 8.72 (2H, d), 7.83 (2H, d), 7.49 (1H, s), 1.50 (9H, s).

**2-(Pyrid-4-yl)-4-amino-thiazole**

2-(Pyrid-4-yl)-4-(t-butoxycarbonylamino)-thiazole (12.49 g, 45.0 mmol) was suspended in methylene chloride (50 mL) arid trifluoroacetic acid (60 mL) added. The solution was stirred at room temperature for 2 h, then evaporated to dryness. Methylene chloride (80 mL) was added and evaporated (x3). The product was triturated under anhydrous diethyl ether, filtered, washed with ether and dried. Yield 12.33 g. MS: [M+H] = 178.1; 1H NMR (d6-DMSO) 8.75 (2H, d), 8.01 (2H, d), 6.35 (1H, s).

Preparation of 5-Pyridin-4-yl-thiophen-3-ylamine

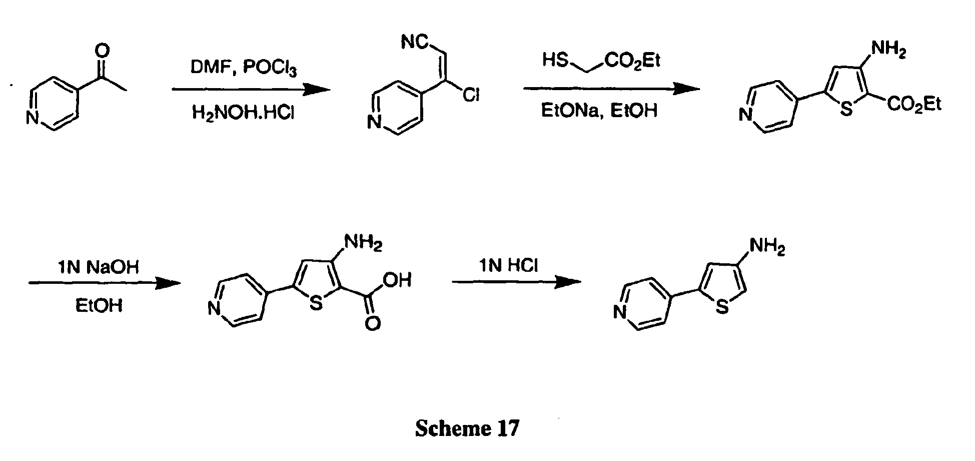

**3-Chloro-3-pyridin-4-yl-acrylonitrile:** To DMF (29.2 g, 0.4 mol) was added phosphorus oxychloride (30.66 g, 0.2 mol) dropwise at 0 - 6 °C over 1.5 hours, then to this mixture was added 4-acetylpyridine (12.1 g, 0.1 mol) room temperature over 3.5 hours, the internal temperature was below 60 °C, then to the reaction mixture was added hydroxylamine hydrochloride suspended in DMF over 4 hours (the reaction is extremely exothermic), then the reaction mixture was stirred at 80 °C for 4 hours. The reaction mixture was neutralized with saturated NaHCO₃ solution, the product was extracted into ethyl acetate, the organic solvent was removed, the product was purified by silica gel chromatography to afford 3-chloro-3-pyridin-4-yl-acrylonitrile (5.0 g, 30%). ¹H NMR (500 MHz, DMSO-d6) δ7.25 (s, 1H), 7.8 (d, 2H), 8.8 (d, 2H).

**3-Anidno-5-pyridin-4-yl-thiophene-2-carboxylic acid ethyl ester:** To a solution containing 3-chloro-3-pyridin-4-yl-acrylonitrile (8.3 g, 0.0504 mol) and ethyl 2-mercaptoacetate (7.27 g, 0.0605 mol) in ethanol was added sodium ethoxide (8.23 g, 0.121 mol). The reaction mixture was refluxed for 20 hours, ethyl acetate and brine added, and the organic phase was dried with MgSO₄. The solvent was removed under reduced pressure, the product was purified by crystallization from methylene chloride and hexanes, (10.0 g, 80%). ¹H NMR (500 MHz, DMSO-d6) δ 1.3 (t, 3H), 4.23 (q, 2H), 6.63 (s, 1H), 7.60 (d, 2H), 8.60 (d, 2H).

**3-Amino-5-pyridin-4-yl-thiophene-2-carboxylic acid:** 3-Amino-5-pyridin-4-yl-thiophene-2-carboxylic acid ethyl ester (6.0 g, 0.0242 mol) was dissolved in hot ethanol (20 mL), and to the solution was addedl 1N NaOH (24 mL). The reaction mixture was heated at 85 °C for 6 hours, then cooled to room temperature. The precipitated solid was filtered, washed with water and dried (4.6g, 86%). ¹H NMR (500 MHz, DMSO-d6) δ 5.96 (s, br, 2H), 7.09 (s, 1H), 7.48 (d, 2H), 8.52 (d, 2H).

**5-Pyridin-4-yl-thiophen-3-ylamine:** To 3-Amino-5-pyridin-4-yl-thiophene-2-carboxylic acid (4.6 g, 0.0209 mol) was added 1N HCl (50 mL) and the suspension was heated at 90 °C. The solid went into solution and after 30 min no more gas was formed. The reaction mixture was cooled to room temperature and neutralized by addition of 6N sodium hydroxide. The solid precipitate was filtered, washed and dried to afford 5-Pyridin-4-yl-thiophen-3-ylamine (2.5g, 67%). ¹H NMR (500 MHz, DMSO-d6) δ 4.99 (s, br, 2H), 6.17 (s, 1H), 7.18 (s, 1H), 7.47 (d, 2H), 8.5 (d, 2H).

The synthesis of certain exemplary acids (for reaction with the amines described generally above) is described below. It will be appreciated that a variety of acids can be prepared according to the general methods described below.

Preparation of 3-(3-(N-Boc-piperidin-4-yl)-propoxy)-phenylacetic acid

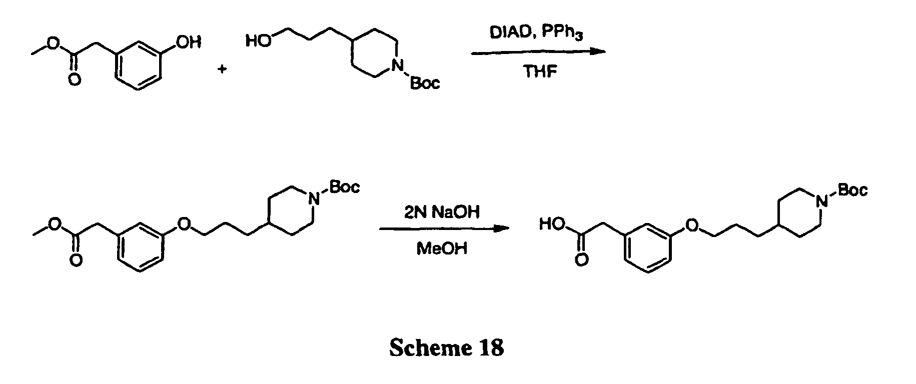

**Methyl 3-hydroxyphenylacetate:** 3-Hydroxyphenylacetic acid (75.3 g, 0.5 mol) was dissolved in methanol (900 mL). Concentrated sulfuric acid (2 mL) was added and the mixture refluxed for 5 hours. The solvent was evaporated and the residue dissolved in ethyl acetate (1000 mL) and washed with water (2 x 600 mL) and brine, and dried (MgS04). Solvent was evaporated to afford methyl 3-hydroxyphenylacetate as an oil (82 g, quantitative yield). ¹H NMR (500 MHz, CDCl₃) δ 7.2 (1H, t), 6.9 - 6.75 (3H, m), 5.5 (1H, br), 3.75 (3H, s), 3.63 (2H, s).

**Methyl 3-(3-(N-Boc-piperidin-4-yl)-propoxy)-phenylacetate:** To THF solution of 0.409g (2.4 mmol) methyl 3-hydroxyphenylacetate, 0.50 g (20.5 mmol) N-Boc-piperidin-4-yl-propanol and 0.645 g (24.6 mmol) triphenylphosphine was added diisopropyl azodicarboxylate at 0 °C slowly, then the ice bath was removed and the reaction mixture was stirred at room temperature overnight. The solvent was removed by rotary evaporation, the residue was dissolved in 2 mL methylene chloride and was loaded on a silica gel column and, the product eluted with 80% hexane and 20% ethyl acetate. Methyl 3-(3-(N-Boc-piperidin-4-yl)-propoxy)-phenylacetate (0.5 g, 62%) was obtained. ¹H NMR (500 MHz, CDCl₃) δ 1.1 (m, 2H), 1.4 (m, 2H), 1.46 (s, 9H), 1.66 (d, 2H), 1.7 8(m, 2H), 2.67 (t, 2H), 3.58 (s, 2H), 3.68 (s, 3H), 4.05 (m, 2H), 6.75 (m, 3H), 7.18 (dd, 1H).

**3-(3-(N-Boc-piperidin-4-yl)-propoxy)-phenylacetic acid:** Methyl 3-(3-(N-Boc-piperidin-4-yl)-propoxy)-phenylacetate (0.5 g, 1.3 mmol) was dissolved in methanol, and 2N NaoH (3 mL) added. The reaction was stirred at 60°C for 2h, then the solution was adjusted to pH 6.5, the product was extracted into ethyl acetate and the organic phase was dried by MgSO₄. Removal of solvent revealed 3-(3-(N-Boc-piperidin-4-yl)-propoxy)-phenylacetic acid (0.30 g). ¹H NMR (500 MHz, CDCl₃) δ 1.02 (m, 2H), 1.25 (m, 2H), 1.55 (m, 2H), 1.65 (m, 2H), 2.57 (m, 2H), 3.33 (m, 1H), 3.75 (s, 2H), 3.95 (m, 2H), 6.63 (m, 3H), 6.98 (m, 1H).

Preparation of 3-(3-chloro-propoxy)-phenylacetic acid

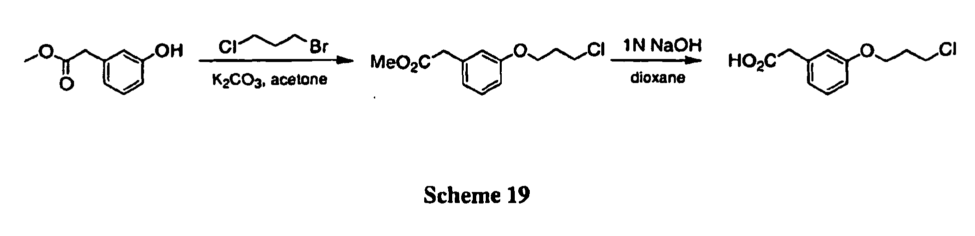

**Methyl 3-(3-chloro-propoxy)-phenylacetate:** Methyl 3-hydroxyphenylacetate ( 87 g, 0.52 mol) was dissolved in acetone (500 mL). 1-Bromo-3-chloropropane (55 mL, 0.56 mol) was added, followed by potassium carbonate (73 g, 0.53 mol) and acetone (100 mL). The reaction was heated to reflux. After 24 hours, more 1-bromo-3-chloropropane (5 mL, 50 mmol) was added and the reaction refluxed for a further 24 hours. The mixture was cooled, filtered and rotary evaporated. The product was purified by passage over a short column of silica gel (650 g: 135 mm diameter column) eluted with hexane, and 30% ethyl acetate in hexane, to afford methyl 3-(3-chloro-propoxy)-phenylacetate (120g, 95%) as an oil. ¹H NMR (500 MHz, CDCl₃) δ 7.25 (1H, dd), 6.93 - 6.85 (3H, m), 4.16 (2H, t), 3.79 (2H, t), 3.73 (3H, s), 3.62 (2H, s), 2.28 (2H, m).

**3-(3-Chloro-propoxy)-phenylacetic acid:** Methyl 3-(3-chloro-propoxy)-phenylacetate (12.7 g, 52.3 mmol) was dissolved in dioxane (25 mL) and 1N NaOH (53 mL) was added. The mixture was stirred at room temperature for 45 minutes then acidified by addition of 1N hydrochloric acid (60 mL). A white precipitae formed which was filtered, washed with 1N HCl, water and dried. 3-(3-Chloro-propoxy)-phenylacetic acid (11.7 g, 98 %). ¹H NMR (500 MHz, CDCl₃) δ 7.25 (1H, dd), 6.93 - 6.85 (3H, m), 4.11 (2H, t), 3.79 (2H, t), 3.70 (2H, s), 2.25 (2H, m).

Preparation of 3-(2-chloro-ethoxy)-phenylacetic acid

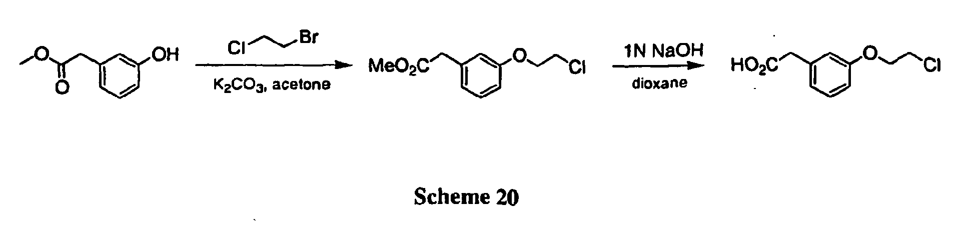

**Methyl 3-(2-chloroethoxy)-phenylacetate:** Methyl 3-hydroxyphenylacetate ( 10.8 g, 65 mmol) was dissolved in acetone (120 mL). 1-Bromo-2-chloroethane (5.5 mL, 66 mmol) was added, followed by potassium carbonate (10.1 g, 73.6 mmol). The reaction was heated to reflux. After 24 hours, more 1-bromo-2-chloroethane (11 mL, 132 mmol) was added and the reaction refluxed for a further 24 hours. The mixture was cooled, filtered and rotary evaporated. The product was purified by passage over a short column of silica gel eluted with hexane, and 30% ethyl acetate in hexane, to afford methyl 3-(3-chloroethoxy)-phenylacetate as an oil.

**3-(2-Chloroethoxy)-phenylacetic acid:** Methyl 3-(2-chloro-ethoxy)-phenylacetate (7.0 g, 32.9 mmol) was dissolved in methanol (40 mL) and 6N NaOH (5.5 mL) was added. The mixture was stirred at room temperature overnight then acidified by addition of 6N hydrochloric acid (5.5 mL). A white precipitae formed which was filtered, washed with 1N HCl, water and dried. 3-(3-Chloroethoxy)-phenylacetic acid (6.5 g, 99 %). ¹H NMR (500 MHz, CDCl₃) δ3.55 (s, 2H), 3.75 (t, 2H), 4.15 (t, 2H), 6.78 (dd, 1H), 6.80 (d, 1H), 6.84 (dd, 1H), 7.16 (dd, 1H).

Preparation of 3-Ethoxyphenylacetic acid

**Methyl 3-ethoxyphenylacetate:** Methyl 3-hydroxyphenylacetate (6.4 g, 38.5 mmol) was dissolved in acetone (50 mL). Ethyl bromide (3.5 mL, 46.9 mmol) was added, followed by potassium carbonate (6.37 g, 46 mmol). The reaction was heated to reflux. After 24 hours, more ethyl bromide (3.55 mL, 46.9 mmol) was added and the reaction refluxed for a further 24 hours. The mixture was cooled, filtered and rotary evaporated. The product was dissolved in ethyl acetate and the solution washed with saturated sodium bicarbonate (2 x 50 mL) and brine, and dried (MgS04). Removal of solvent revealed methyl 3-ethoxyphenylacetate as an oil that crystallized upon standing. ¹H NMR (500 MHz, CDCl₃) δ 7.25 (1H, dd), 6.87 (3H, m), 4.08 (2H, q), 3.73 (3H, s), 3.65 (2H, s), 1.45 (3H, t).

**3-Ethoxyphenylacetic acid:** Methyl 3-ethoxyphenylacetate (7.5 g, 38.6 mmol) was dissolved in ethanol (15 mL) and 1N NaOH (40 mL) was added. The mixture was stirred at room temperature for 30 minutes then acidified by addition of 1N hydrochloric acid (45 mL). A white precipitate formed which was filtered, washed with 1N HCl, water and dried. 3-Ethoxyphenylacetic acid (6.4 g, 92 %). ¹H NMR (500 MHz, CDCl₃) δ 7.20 (1H, dd), 6.8 (3H, m), 4.0 (2H, q), 3.6 (2H, s), 1.4 (3H, t).

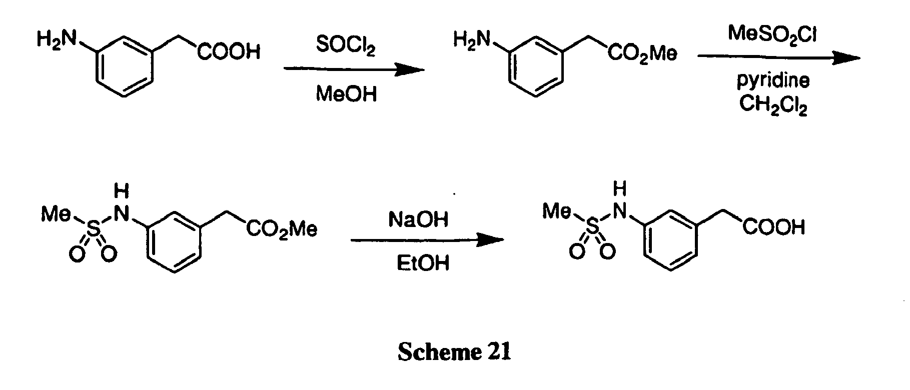

Preparation of 3-(Methanesulfonyl)-phenylacetic acid

**Methyl 3-aminophenylacetate:** 3-Aminophenylacetic acid (15.5 g, 0.10 mol) was suspended in methanol (150 mL) and cooled to 0 °C. Thionyl chloride (11.2 mL, 0.15 mol) was added dropwise under stirring. A clear orange solution was obtained, which was stirred for 4 hours, then evaporated. The solid residue was partitioned between ethyl acetate (150 mL) and saturated sodium bicarbonate (150 mL) and the organic phase washed with saturated sodium bicarbonate (100 mL), and brine and dried (Na₂SO₄). Methyl 3-aminophenylacetate was isolated as a brown oil. (14.1g, 83%). ¹H NMR (500 MHz, CDCl₃) δ 7.12 (1H, dd), 6.7-6.6 (3H, m), 3.71 (3H, s), 3.55 (2H, s).

**Methyl 3-(methanesulfonyl)phenylacetate:** Methyl 3-aminophenylacetate (2.26 g, 13.7 mmol) was dissolved in dry methylene chloride (20 mL) and cooled to 0 °C. Pyridine (2.2 mL, 27.2 mmol) was added followed by dropwise addition of methanesulfonyl chloride (1.3 mL, 16.8 mmol). The mixture was stirred at 0 °C for 1 hour and at room temperature for 3 hours, then poured into 100 mL of saturated sodium bicarbonate solution. The organic layer was washed with saturated sodium bicarbonate (100 mL), 1N HCl (2 x 100 mL) and brine. Dried over MgSO₄. Solvent was evaporated to reveal methyl 3-(methanesulfonyl)phenylacetate. (3.36 g, 100%). ¹H NMR (500 MHz, CDCl₃) δ 7.32 (1H, dd), 7.2 -7.1 (3H, m), 6.57 (1H, s), 3.72 (3H, s), 3.64 (2H, s), 3.02 (3H, s).

**3-(Methanesulfonyl)phenylacetic acid:** Methyl 3-(methanesulfonyl)phenylacetate (3.36 g, 13.8 mmol) was dissolved in ethanol (16 mL) and 1N NaOH (30 mL) added. The reaction was stirred for 1 hour, then 1N HCl (50 mL) and water (50 mL) were added. The product was extracted into ethyl acetate (3 x 50 mL) and the combined extracts were washed with water and brine and dried (MgSO₄). Removal of solvent afforded 3-(methanesulfonyl)phenylacetic acid (2.90 g, 92%). ¹H NMR (500 MHz, DMSO-d6) δ 12.32 (1H, br), 9.69 (1H, br), 7.26 (1H, dd), 7.10 (2H, m), 7.00 (1H, d), 6.57 (1H, s), 3.54 (2H, s), 2.97 (3H, s).

Preparation of 3-(3-(N-Boc-piperidin-4-yl)-propoxy)-phenylacetic acid

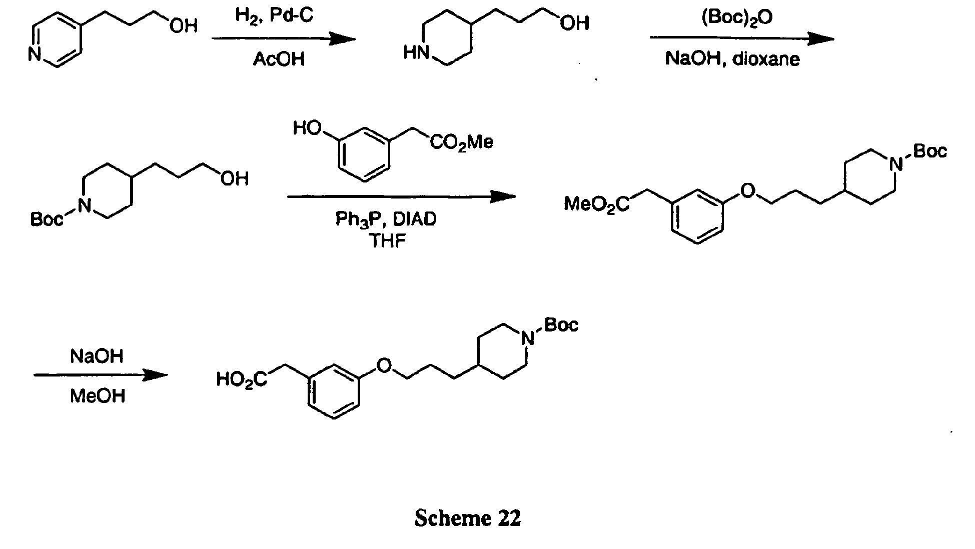

**3-Piperidin-4-yl-propan-1-ol:** 4-Pyridinepropanol (10.0 g, 73 mmol) was dissolved in glacial acetic acid (50 mL). 10% Palladium on carbon (1.1 g) was added and the mixture hydrogenated under 50 psi hydrogen gas for 6 days. The mixture was filtered through Celite and the solvent removed by rotary evaporation. The crude product 3-piperidin-4-yl-propan-1-ol (acetic acid salt) was used as obtained. ¹H NMR (500 MHz, CDCl₃) δ 6.3 (br), 3.65 (2H, t), 3.36 (2H, m), 2.79 (2H, dt), 2.01 (3H, s), 1.85 (2H, m), 1.7 - 1.3 (7H, m).

**3-(N-Boc-Piperidin-4-yl)-propan-1-ol:** The crude 3-pipelidin-4-yl-propan-1-ol (73 mmol) was dissolved in dioxane (100 mL) and 3N NaOH (25 mL) was added to give a pH9 solution. Di-tert-butyl dicarbonate (16.0 g, 73 mmol) in dioxane (35 mL) was added dropwise, with simultaneous addition of 3N NaOH to maintain the solution at approximately pH9. After 2 hours no residual amine was visible by TLC (ninhydrin stain) and the reaction was diluted with water (200 mL)and extracted with ethyl acetate (3 x 100 mL). The combined extracts were washed with water and brine and dried (MgSO₄). Removal of solvent afforded 20 g crude product which was purified by silica gel chromatography (200 g silica) in a sintered glass funnel (L.M. Harwood, Aldrichimica Acta, 1985, 18, 25) eluted with 500 mL each of hexane, 20%, 40%, 60% and 80% ethyl acetate in hexane. 3-(N-Boc-Piperidin-4-yl)-propan-1-ol was isolated as a clear, colorless oil (14.5 g, 82%). ¹H NMR (500 MHz, CDCl₃) δ 4.09 (2H, m), 3.66 (2H, t), 2.69 (2H, dt), 1.7-1.5 (4H, m), 1.47 (9H, s), 1.4-1.3 (5H, m), 1.12 (2H, m).

**Methyl 3-(3-(N-Boc-piperidin-4-yl)-propoxy)-phenylacetate:** To a solution of methyl 3-hydroxyphenylacetate (0.409 g, 2.4 mmol), 3-(N-Boc-piperidin-4-yl)-propan-1-ol (0.50 g, 20.5 mmol) and triphenylphosphine (0.645, 24.6 mmol) in THF, was added diisopropyl azodicarboxylate at 0 °C slowly, then the ice bath was removed and the reaction mixture was stirred at room temperature overnight. The solvent was removed and the residue was dissolved in methylene chloride (2 mL) and loaded on a silica gel column. The product was eluted with 20% ethyl acetate in hexane, to afford methyl 3-(3-(N-Boc-piperidin-4-yl)-propoxy)-phenylacetate (0.5 g, 62%). ¹H NMR (500 MHz, CDCl₃) δ1.1 (m, 2H), 1.4 (m, 2H), 1.46 (s, 9H), 1.66 (d, 2H), 1.78 (m, 2H), 2.67 (t, 2H), 3.58 (s, 2H), 3.68 (s, 3H), 4.05 (m, 2H), 6.75 (m, 3H), 7.18 (dd, 1H).
**3-(3-(N-Boc-piperidin-4-yl)-propoxy)-phenylacetic acid:** Methyl 3-(3-(N-Boc-piperidin-4-yl)-propoxy)-phenylacetate (0.5 g, 1.3 mmol) was dissolved in methanol and 2N NaOH (3 mL) added. The reaction was stirred at 60°C for 2 hours then the solution was adjusted to pH 6.5. The product was extracted into ethyl acetate, and the organic phase was dried by MgSO₄. The solvent was evaporated to afford 3-(3-(N-Boc-piperidin-4-yl)-propoxy)-phenylacetic acid (0.30 g). ¹H NMR (500 MHz, CDCl₃) δ 1.02 (m, 2H), 1.25 (m, 2H), 1.55 (m, 2H), 1.65 (m, 2H), 2.57 (m, 2H), 3.33 (m, 1H), 3.75 (s, 2H), 3.95 (m, 2H), 6.63 (m, 3H), 6.98 (m, 1H).

PREPARATION OF EXEMPLARY COMPOUNDS

Preparation of 2-amino-4-(4-pyridyl)thiazoles

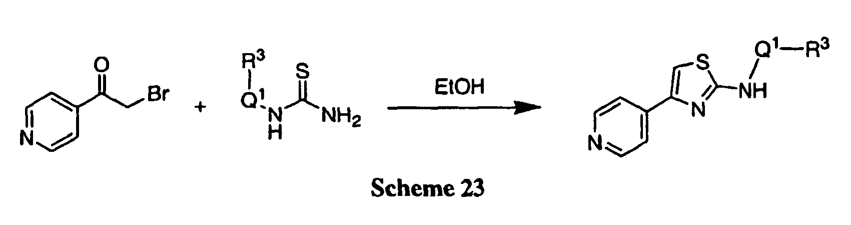

**4-Pyridin-4-yl-thiazol-2-ylamine:** To 4-(bromoacetyl)-pyridine hydrobromide (Can. J. Chem., 1970, 7, 1137) (97.5 g, 0.35 mol) and thiourea (26.5 g, 0.35 mol) was added ethanol (900 mL) and the mixture heated to reflux for 2 hours. After cooling to 4 °C the product was filtered, washed with ethanol and diethyl ether and dried under suction. The solid 4-pyridin-4-yl-thiazol-2-ylamine dihydrobromide (88.7 g) was dissolved in warm water (500 mL) and the desired 4-Pyridin-4-yl-thiazol-2-ylamine obtained as a light brown solid upon addition of 7% aqueous ammonium hydroxide (800 mL). 43.5 g, 71%. ¹H NMR (500 MHz, DMSO-d6) δ 8.53 (2H, d), 7.71 (2H, d), 7.38 (1H, s), 7.16 (2H, br).

**Methyl-(4-pyridin-4-yl-thlazol-2-yl)-amine:** To 4-(bromoacetyl)-pyridine hydrobromide (Can. J. Chem., 1970, 7, 1137) (16.7 g, 59 mmol) and N-methylthiourea (5.4 g, 60 mmol) was added ethanol (160 mL) and the mixture heated to reflux for 1 hour. A thick solid formed. After cooling to 4 °C the product was filtered, washed with ethanol and diethyl ether and dried under suction. The solid methyl-4-pyridin-4-yl-thiazol-2-ylamine dihydrobromide (15 g) was stirred in 1N NaOH (100 mL) for 30 min then filtered, washed with 1N NaOH and water, and dried.to afford methyl-4-pyridin-4-yl-thiazol-2-ylamine (7.6 g, 67%). [M+H]+ = 192. ¹H NMR (500 MHz, DMSO-d6) δ 8.55 (2H, d), 7.76 (2H, d), 7.67 (1H, br), 7.42 (1H, s), 2.90(3H, d).

**Ethyl-(4-pyridin-4-yl-thiazol-2-yl)-amine:** To 4-(bromoacetyl)-pyridine hydrobromide (Can. J. Chem., 1970, 7, 1137) (16.7 g, 59 mmol) and N-ethylthiourea (6.3 g, 61 mmol) was added ethanol (160 mL) and the mixture heated to reflux for 1 hour. A thick solid formed. After cooling to 4 °C the product was filtered, washed with ethanol and diethyl ether and dried under suction. The solid ethyl-4-pyridin-4-yl-thiazol-2-ylamine dihydrobromide (13.7 g) was stirred in 1N NaOH (100 mL) for 30 min then filtered, washed with 1N NaOH and water, and dried.to afford ethyl-4-pyridin-4-yl-thiazol-2-ylamine (6.7 g, 56%). [M+H]+ = 206. ¹H NMR (500 MHz, DMSO-d6) δ 8.56 (2H, d), 7.75 (3H, m), 7.40 (1H, s), 3.3 (2H, obscured), 1.20 (3H, t).

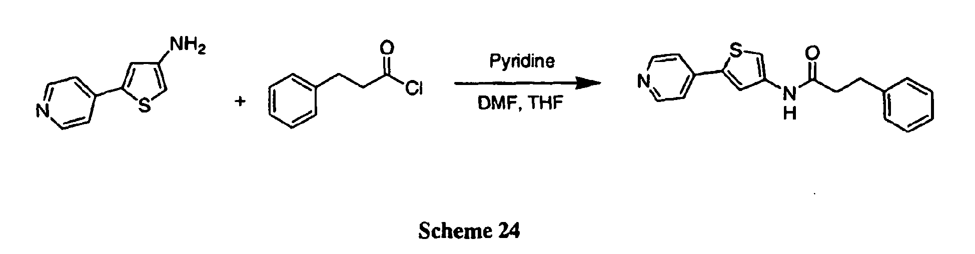

**3-Phenyl-N-(5-pyridin-4-yl-thiophen-3-yl)-propionamide:** A DMF/THF solution containing 5-pyridin-4-yl-ihiophen-3-ylamine (60mg, 0.341 mmol), hydrocinnamyl chloride (76.8 mg, 0.411 mmol), and pyridine (32 mg, 0.411 mmol) was stirred at 70 °C for 3 hours, then the solvent was removed, the residue was dissolved in MeOH and purified by preparative HPLC. ¹H NMR (500 MHz, DMSO-d6) δ 2.64 (t, 2H), 2.92(t, 2H), 7.17 (m, 1H), 7.28 (m, 4H), 7.8 (s, 1H), 7.85 (s, 1H), 7.93 (d, 2H), 8.73 (d, 2H), 10.52 (s, 1H). LC-MS (10-90% CH₃CN in H₂O), Rt = 2.30 min, [M+H]⁺ = 309, [M-H]⁻ = 307.

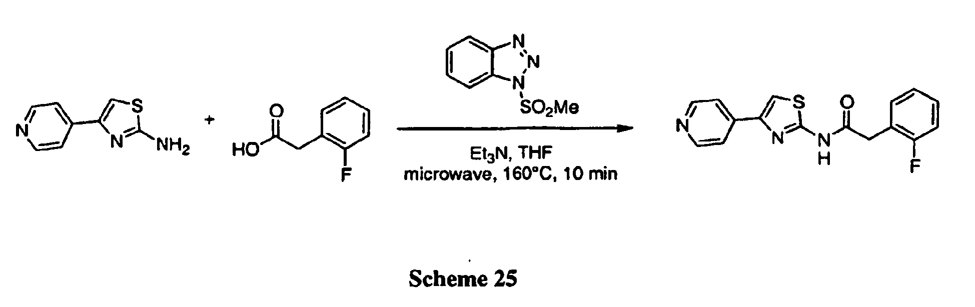

**2-(2-Fluorophenyl)-N-(4-pyridin-4-yl-thiazol-2-yl)-acetamide:** 4-(4-Pyridyl)-2-aminothiazole (329 mg, 1.86 mmol), 2-fluorophenylacetic acid (377 mg, 2.25 mmol) and N-(1-methanesulfonyl)benzotriazole (440 mg, 2.23 mmol) were placed in a microwave reaction vessel (Personal Chemistry, Uppsala, Sweden). THE (2 mL) was added followed by triethylamine (0.52 mL, 3.73 mmol) and the mixture heated in the sealed tube at 160 °C for 10 minutes. Upon cooling to room temperature the product 2-(2-fluorophenyl)-N-(4-pyridin-4-yl-thiazol-2-yl)-acetamide precipitated, was filtered, washed with acetonitrile and dried. (462 mg, 76%). ¹H NMR (500 MHz, DMSO-d6) δ 12.68 (1H, s), 8.63 (2H, d), 8.00 (1H, s), 7.84 (2H, d), 7.43-7.17 (4H, m), 3.90 (2H, s). LC-MS Rt = 1.9 min, [M+H]⁺ = 314, [M-H]⁻ = 312.

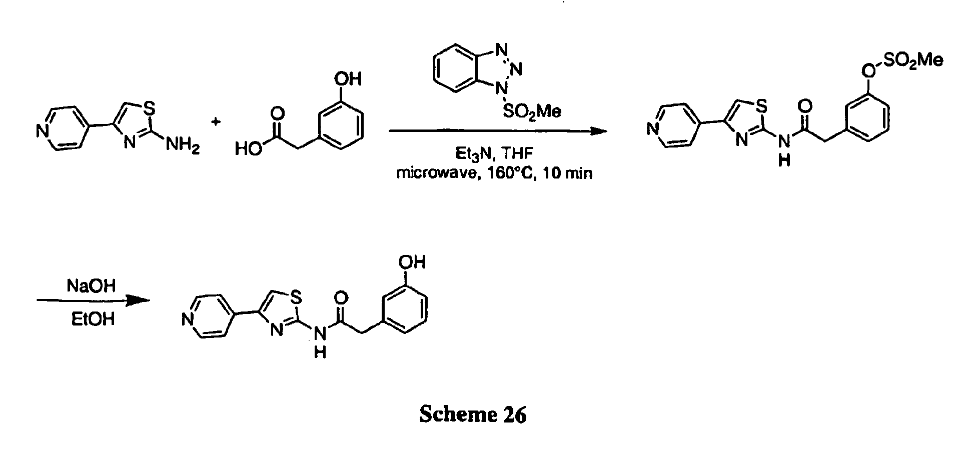

**Methanesulfonic acid 3-[(4-pyridin-4-yl-thiazol-2-ylcarbamoyl)-methyl]-phenyl ester:** 4-(4-Pyridyl)-2-aminothiazole (317 mg, 1.79 mmol), 3-hydroxyphenylacetic acid (343 mg, 2.25 mmol) and N-(1-methanesulfonyl)benzotriazole (927 mg, 4.70 mmol) were placed in a microwave reaction vessel (Personal Chemistry, Uppsala, Sweden). THF (2 mL) was added followed by triethylamine (1.24 mL, 8.93 mmol) and the mixture heated in the sealed tube at 160 °C for 10 minutes. Upon cooling to room temperature the solvent was concentrated and ethanol added. The mixture was stored at -20°C and then the precipitated product was filtered, washed with ethanol and dried. (910 mg, 65%). ¹H NMR (500 MHz, DMSO-d6) δ 12.62 (1H, s), 8.63 (2H, d), 7.98 (1H, s), 7.84 (2H, d), 7.47 (1H, m), 7.36 (2H, m), 7.27 (1H, m), 3.89 (2H, s), 3.40 (3H, s). LC-MS Rt = 2.1 min, [M+H]⁺ = 390, [M-H]⁻ = 388.

**2-(3-Hydroxyphenyl)-N-(4-pyridin-4-yl-thiazol-2-yl)-acetamide:** Methanesulfonic acid 3-[(4-pyridin-4-yl-thiazol-2-ylcarbamoyl)-methyl]-phenyl ester (400 mg) was suspended in ethanol (6 mL) and 1N NaOH (2 mL) added. The mixture was stirred at 50°C. After 15 hours 2N NaOH (2mL) was added and the reaction stirred for a further 5 hours at 50°C. 1N Hydrochloric acid was added to precipitate the product. ¹H NMR (500 MHz, DMSO-d6) δ 12.7 (1H, s), 9.45 (1H, br s), 8.88 (2H, d), 8.35 (1H, s), 8.20 (2H, d), 7.21 (1H, t), 6.85 (2H, m), 6.70 (1H, d), 3.80 (2H, s). LC-MS Rt = 1.5 min, [M+H]⁺ = 312, [M-H]⁻ = 310.

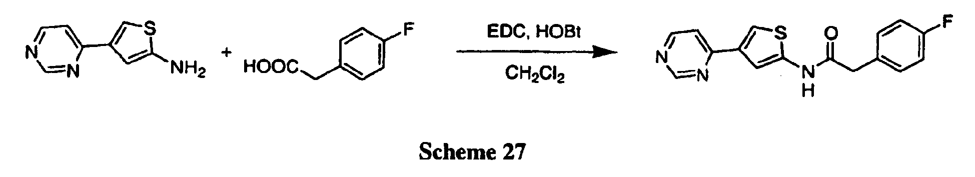

**2-(4-Fluorophenyl)-*N*-(4-pyrimidin-4-yl-thiophen-2-yl)-acetamide:** To a solution of 4-pyrimidin-4-yl-thiophen-2-ylamine (0.010g, 0.047mmol) in DCM (1mL) was added 1-ethyl-(dimethylaminopropyl)carbodiimide hydrochloride (0.025g, 0.13mmol), 1-hydroxybenzotriazole (0.015g, 0.11mmol), and 4-fluorophenylacetic acid (0.025g, 0.16mmol). The material was stirred for 5 minutes at RT, and then triethylamine (0.20mL, 1.43mmol) was added drop-wise over one minute. The reaction was stirred for one hour, then partitioned between EtOAc and water and the organic layer was dried with sodium sulfate. The solution was evaporated and stripped to a glassy residue which was purified by flash chromatography on silica gel by eluting with 50% EtOAc-hexane. The product 2-(4-fluorophenyl)-*N*-(4-pyrimidin-4-yl-thiophen-2-yl)-acetamide is obtained (6.4mg, 43%) as colorless glass. ¹H NMR (500 MHz, DMSO-d6) δ 9.06 (s, 1H), 8.62 (d, 1H), 8.00 (s, 1H), 7.57 (d, 1H),7.41 (d, 1H), 7.42-7.22 (m, 2H), 7.11-7.00 (m, 2H), 3.69 (s, 2H) LC-MS (10-90% CH₃CN in H₂O), Rt = 2.90min, [M+H]⁺ = 314, [M-H]⁻ = 312.1.

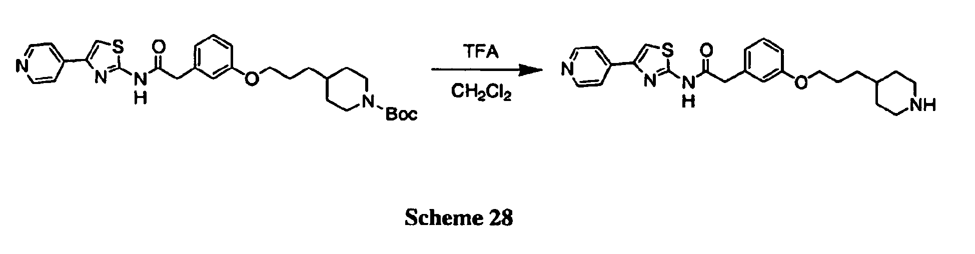

**2-(3-(3-(Piperidin-4-yl)-propoxy)-phenyl)-N-(4-pyridin-4-yl-thiazol-2-yl)-**acetamide: 2-(3-(3-(N-Boc-piperidin-4-yl)-propoxy)-phenyl)-N-(4-pyridin-4-yl-thiazol-2-yl)-acetamide was dissolved in methylene chloride and TFA added. After stirring for 4 hours at room temperature the solvents were evaporated and the residue purified by HPLC. ¹H NMR (500 MHz, CD₃OD) δ 1.4 (m, 2H), 1.5 (m, 2H), 1.67 (m, 1H), 1.81 (m, 2H), 1.95 (dd, 2H), 2.94 (t, 2H), 3.35 (dd, 2H), 3.78 (s, 2H), 4.0 (t, 2H), 6.83 (dd, 1H), 6.9 (dd, 1H), 6.92 (d, 1H), 7.24 (dd, 1H), 8.19 (s, 1H), 8.4 (d, 2H), 8.74 (d, 2H). LC-MS (5-45% CH₃CN in H₂O, Rt = 2.39 min, [M+H]⁺ = 437, [M-H]⁻ = 435.

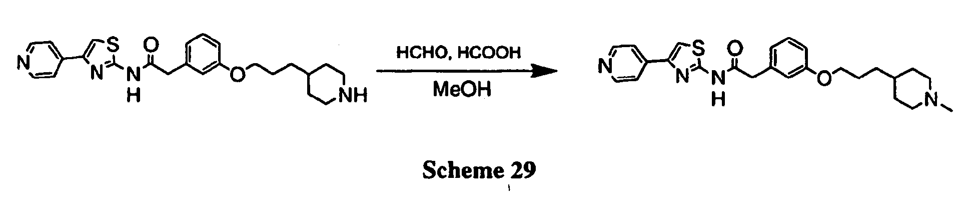

**2-(3-(3-(N-Methyl-Piperidin-4-yl)-propoxy)-phenyl)-N-(4-pyridin-4-yl-thiazol-2-yl)-acetamide:** 2-[3-(3-Piperidin-4-yl-propoxy)-phenyl]-*N*-(4-pyridin-4-yl-thiazol-2-yl)-acetamide (1 equiv.), formaldehyde (30 equiv.) and formic acid (30 equiv.) in methanol solution were heated in a sealed tube at 80 °C for 48 hours. The reaction mixture was diluted with ethyl acetate and brine, and the organic phase was dried over MgSO₄. The solvent was removed by rotary evaporation and the product was purified by HPLC. ¹H NMR (500 MHz, CD₃OD) δ 1.43 (m, 1H), 1.5 (m, 2H), 1.62(m, 1H),1.82 (m 2H), 2.05 (d, 2H), 2.84 (s, 3H), 2.95 (t, 2H), 3.46 (d, 2H), 3.79 (s, 2H), 4.02 (t, 2H), 6.82 (d, 1H), 6.9 (d, 1H), 6.9 (s, 1H), 7.24 (dd, 1H), 8.05 (s, 1H), 8.24 (d, 2H), 8.68 (d, 2H). LC-MS (5-45% CH₃CN in H₂O, Rt = 2.68 min, [M+H]⁺ = 451, [M-H]⁻ = 449.

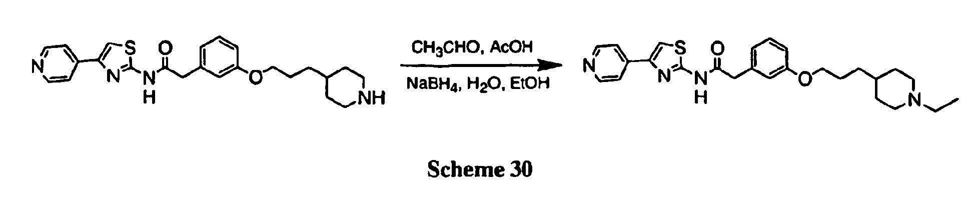

**2-(3-(3-(N-Ethyl-Piperidin-4-yl)-propoxy)-phenyl)-N-(4-pyridin-4-yl-thiazol-2-yl)-acetamide:** 2-[3-(3-Piperidin-4-yl-propoxy)-phenyl]-*N*-(4-pyridin-4-yl-thiazol-2-yl)-acetamide (1 equiv.), acetaldehyde (30 equiv.) and acetic acid (30 equiv.) in ethanol solution were treated with water (4 drops) and sodium borohydride. The reaction was stirred at room temperature for 5 minutes. The reaction mixture was diluted with ethyl acetate and brine, and the organic phase was dried over MgSO₄. The solvent was removed by rotary evaporation and the product was purified by HPLC. ¹H NMR (500 MHz, CD₃OD) δ 8.75 (d, 2H), 8.43 (d, 2H), 8.23 (s, 1H),7.23 (dd, 1H), 6.93 (d, 2H), 6.84 (d, 1H), 3.98 (q, 2H), 3.79 (s, 2H), 3.55 (d, 2H), 3.14 (q, 2H), 2.87 (t, 2H), 2.05 (d, 2H), 1.83 (m, 2H), 1.65 (m, 1H), 1.46 (m, 2H), 1.40 (d, 2H), 1.32 (t, 3H). LC-MS (5-45% CH₃CN in H₂O, Rt = 2.48 min, [M+H]⁺ = 465, [M-H]⁻ = 463.

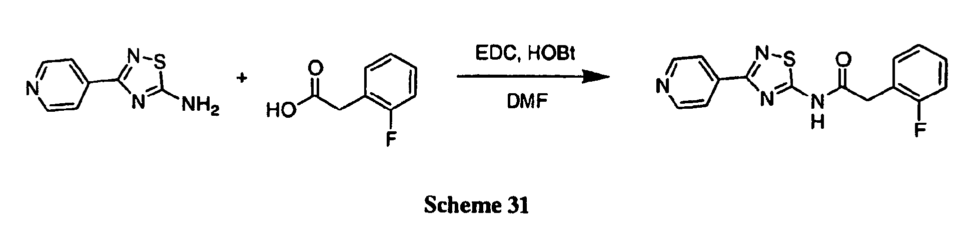

**2-(2-Fluorophenyl)-N-(3-pyridin-4-yl-[1,2,4]thiadiazol-5-yl)-acetamide: 2-**Fluorophenylacetic acid (86 mg, 0.56 mmol), was dissolved in DMF (2mL). 1-Hydroxybenzotriazole (89 mg, 0.66 mmol) and 1-ethyl-(dimethylaminopropyl)carbodiimide hydrochloride (115 mg, 0.6 mmol) were added and the mixture stirred at room temperature for 10 minutes. 3-Pyridin-4-yl-[1,2,4]thiadiazol-5-ylamine (100 mg, 0.56 mmol), was added and stirring continued for 4 hours. The DMF was evaporated and the residue washed with water. The crude product was then purified by preparative thin layer chromatography (5% methanol in methylene chloride), affording 5 mg of 2-(2-fluorophenyl)-N-(3-pyridin-4-yl-[1,2,4]thiadiazol-5-yl)-acetamide. ¹H NMR (500 MHz, CD₃OD) δ: 8.8 (m, 1H), 8.7 (m, 1H), 8.2 (m, 1H), 7.8 (m, 1H), 7.3 (m, 2H), 7.1 (m, 2H), 4.2 (s, 1H), 4.0 (s, 1H). LC-MS (10-90% CH3CN in H2O), Rt = 2.31 min, [M+H]⁺ = 315, [M-H]⁻ = 313.

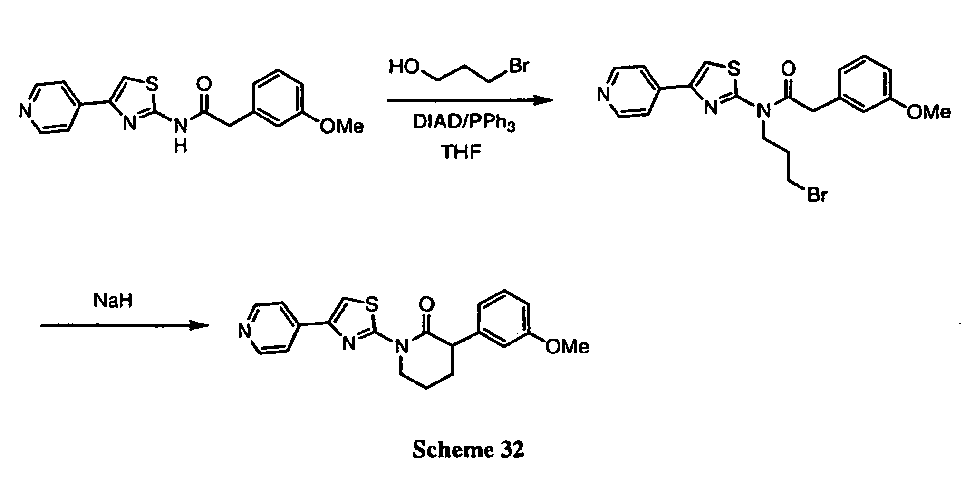

**3-(3-Methoxy-phenyl)-1-(4-pyridin-4-yl-thiazol-2-yl)-piperidin-2-one:** 2-(3-Methoxy-phenyl)-N-(4-pyridin-4-yl-thiazol-2-yl)-acetamide (prepared according to General Method A from 4-(4-pyridyl)-2-aminothiazole and 3-methoxyphenylacetic acid: 1 mmol) , triphenylphosphine (1.2 mmol), diisopropyl azodicarboxylate (1.2 mmol) and THF were were stirred overnight at room temperature. The mixture was then cooled to 0 C and NaH (1.2 mmol) added and reaction mixture was stirred at 0 C for 30 minutes. MeOH was added to quench the reaction. The solvent was evaporated and the residue dissolved in ethyl acetate and washed with H₂O and dried over Na₂SO₄. The product was purified by flash column chromatography on silica gel to afford 3-(3-methoxyphenyl)-1-(4-pyridin-4-yl-thiazol-2-yl)-piperidin-2-one in 60% yield. ¹H NMR (500 MHz, DMSO-d6) δ 8.67 (d, 2H), 7.80 (d, 2H), 7.46 (s, 1H). 7.30 (m, 1H), 6.85 (m, 3H), 4.57 (m, 1H), 4.32 (m, 1H), 3.93 (m, 1H), 3.82 (s, 3H), 2.36 (m, 1H), 2.25 (m, 1H), 2.15 (m, 2H). LC-MS (10-90% CH3CN in H2O), Rt = 2.40 min, [M+H]⁺ = 366, [M-H]⁻ = 364.

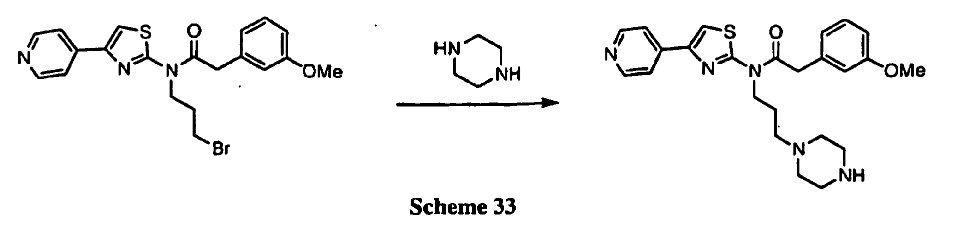

**2-(3-Methoxy-phenyl)-N-(3-piperazin-1-yl-propyl)-N-(4-pyridin-4-yl-thiazol-2-yl)-acetamide:** 2-(3-Methoxyphenyl)-N-(4-pyridin-4-yl-thiazol-2-yl)-acetamide (prepared according to General Method A from 4-(4-pyridyl)-2-aminothiazole and 3-methoxyphenylacetic acid: 1 mmol) , triphenylphosphine (1.2 mmol), diisopropyl azodicarboxylate (1.2 mmol) and THF were were stirred overnight at room temperature. Piperazine (3 mmol) was added and reaction mixture was heated to 60 C for 1 hour. The solvent was evaporated and the residue dissolved in ethyl acetate and washed with H₂O and dried over Na₂SO₄. The product was purified by flash column chromatography on silica gel to afford 2-(3-methoxyphenyl)-N-(3-piperazin-1-yl-propyl)-N-(4-pyridin-4-yl-thiazol-2-yl)-acetamide 70 % yield. ¹H NMR (500 MHz, CD₃OD) δ 8.78 (d, 2H), 8.47 (d, 2H), 8.32 (s, 1H), 7.30 (m, 1H), 6.90 (m, 3H), 4.43 (t, 2H), 4.16 (s, 2H), 3.78 (s, 3H), 3.30 (m, 4H obscured), 2.87 (br, 4H), 2.85 (t, 2H), 2.06 (m, 2H). LC-MS (5-45% CH₃CN in H₂O, Rt = 1.80 min, [M+H]⁺ = 452, [M-H]⁻ = 450.

Preparation of N, O, and S-linked acetamides

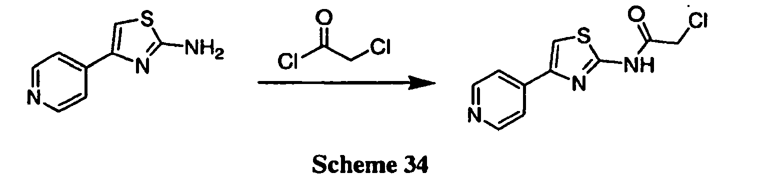

**2-Chloro-N-(4-pyridin-4-yl-thiazol-2-yl)-acetamide:** A solution of 4-pyridin-4-yl-thioazol-2-ylamine (3.64 g, 0.02 mol) and chloroacetyl chloride (3.39 g, 0.03 mol) in dioxane was refluxed overnight then cooled to room temperature. The solid precipitate was filtered, then the filtration cake was suspended in saturated KHCO₃, then filtered again. The filtration cake was washed with water dried in dessicator over P₂O₅. 2-Chloro-N-(4-pyridin-4-yl-thiazol-2-yl)-acetamide (4.3 g, 85%). ¹H NMR (500 MHz, DMSO-d6) δ 4.42 (s, 2H), 7.9 (d, 2H), 8.77 (d, 2H).

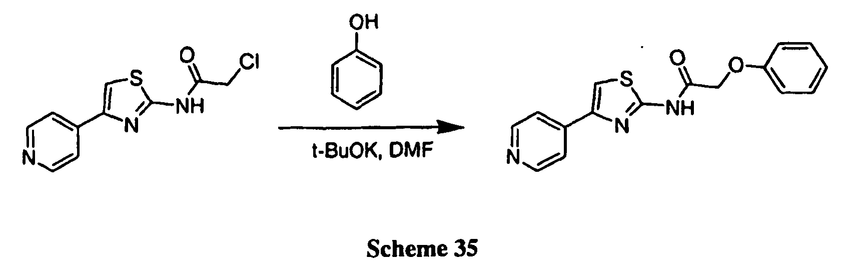

**2-Phenoxy-N-(4-pyridin-4-yl-thiazol-2-yl)-acetamide:** 2-Chloro-N-(4-pyridin-4-yl-thiazol-2-yl)-acetamide (1 mmol) was added to a stirred DMF solution (45 °C, 2h) containing phenol (3 mmol) and t-BuOK (3 mmol). The reaction mixture was stirred at 80°C for 8 h. To the reaction mixture was added ethyl acetate and brine, and the organic phase was dried with MgSO₄. The product was purified by HPLC. ¹H NMR (500 MHz, DMSO-d6) δ 4.92(s, 2H), 7.0(m, 3H), 7.35(dd, 2H), 8.17(d, 2H), 8.33(s, 1H), 8.8(d, 2H), 12.7(s, 1H). LC-MS (10-90% CH3CN in H2O), Rt = 1.67 min, [M+H]⁺ = 312, [M-H]⁻ = 310.

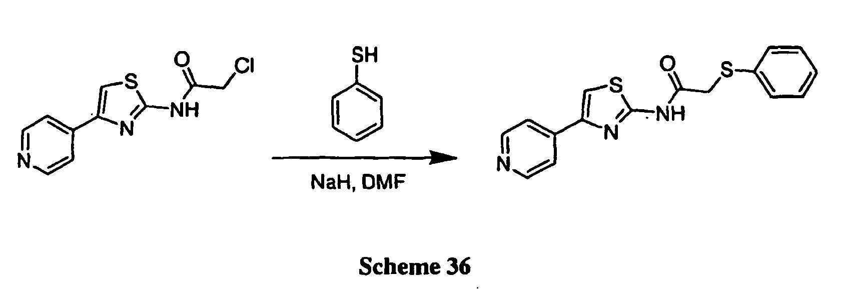

**2-Phenylsulfanyl-N-(4-pyridin-4-yl-thiazol-2-yl)-acetamide:** To a flask containing NaH (65%: 0.53 g, 1.32 mmol) was added a DMF solution of thiophenol (0.146 g, 1.32 mmol). The reaction mixture was stirred at room temperature until no more gas was released. To this was added 2-chloro-N-(4-pyridin-4-yl-thiazol-2-yl)-acetamide (0.112 g, 0.4 mmol) in DMF and the reaction mixture was stirred at 60 °C for 5 hours. To the reaction mixture was added ethyl acetate and brine, and the organic phase was dried with MgSO₄. The solvent was removed, and the product was purified by silica gel chromatography. 2-Phenylsulfanyl-N-(4-pyridin-4-yl-thiazol-2-yl)-acetamide (0.080g , 55%). ¹H NMR (500 MHz, DMSO-d6) δ 4.0 (s, 2H), 7.24 (m, 1H), 7.35 (dd, 2H), 7.40 (d, 2H), 8.83 (d, 2H), 8.02 (s, 1H), 8.62 (d, 2H), 12.65 (s, 1H). LC-MS (10-90% CH3CN in H2O), Rt = 5.09 min, [M+H]⁺ = 328, [M-H]⁻ = 326.

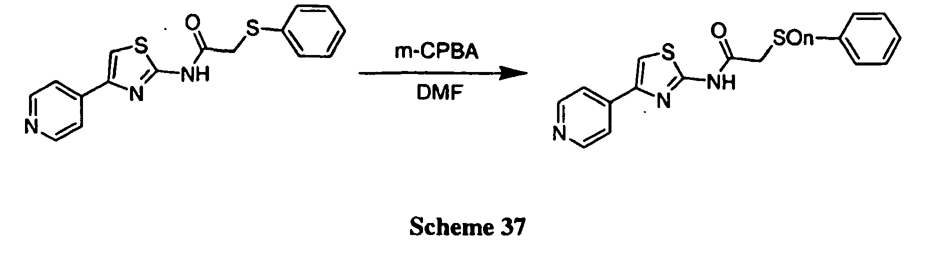

**2-Benzenesulfonyl-N-(4-pyridin-4-yl-thiazol-2-yl)-acetamide (Scheme 37: n = 2):** To 2-phenyl-sulfanyl-N-(4-pyridin-4-yl-thiazol-2-yl)-acetamide (0.08 g, 0.244 mmol) in DMF was added m-CPBA (77%: 0.081 g, 0.366 mmol), and the reaction mixture was stirred at 40 °C for 1 h, TLC indicated 2 new spots and no starting material.. To the reaction mixture was added ethyl acetate and brine, and the organic phase was dried with MgSO₄. 2-Benzenesulfonyl-*N*-(4-pyridin-4-yl-thiazol-2-yl)-acetamide was isolated following by preparative HPLC. ¹H NMR (500 MHz, DMSO-d6) δ 4.7 (s, 2H), 7.68 (dd, 2H), 7.80 (dd, 1H), 7.94 (d, 2H), 8.15 (d, 2H), 8.35 (s, 1H), 8.82 (d, 2H), 12.82 (s, 1H). LC-MS (10-90% CH₃CN in H₂O), Rt = 3.77 min, [M+H]⁺ = 360, [M-H]⁻ = 358.

**2-Benzenesulfinyl-N-(4-pyridin-4-yl-thiazol-2-yl)-acetamide (Scheme 37: n = 1):** To 2-phenylsulfanyl-N-(4-pyridin-4-yl-thiazol-2-yl)-acetamide (30 mg, 91.7 mmol) in DMF was added m-CPBA (77%: 20 g, 91.7 mmol), and the reaction mixture was stirred at 40 °C for 1h, then diluted with water. 2-Benzenesulfinyl-N-(4-pyridin-4-yl-thiazol-2-yl)-acetamide was isolated following by preparative HPLC (20 mg, 64%). ¹H NMR (500 MHz, DMSO-d6) δ 4.07 (d, 1H), 4.29 (d, 1H), 7.60 (m, 3H), 7.75 (d, 1H), 8.20 (d, 2H), 8.41(s, 1H), 8.84 (d, 2H), 12.74 (s, 1H). LC-MS (10-90% CH3CN in H2O), Rt = 3.36 min, [M+H]⁺ = 344, [M-H]⁻ = 342.

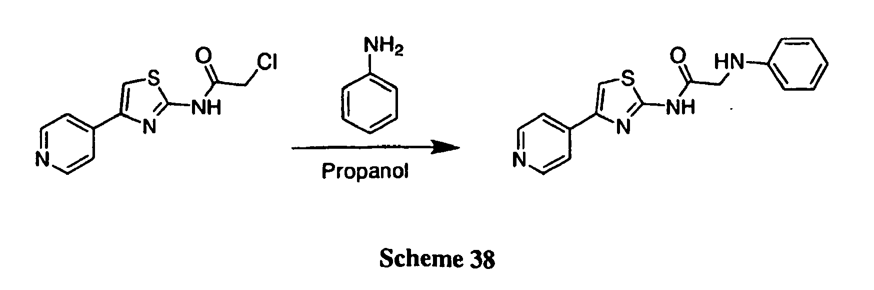

**2-Phenylamino-N-(4-pyridin-4-yl-thiazol-2-yl)-acetamide:** A suspension of of 2-chloro-N-(4-pyridin-4-yl-thiazol-2-yl)-acetamide (1 mmol) and aniline (4 mmol) in n-propanol was stirred at 75 °C overnight. The reaction mixture was filtered and the filtrate was injected into preparative HPLC. ¹H NMR (500 MHz, CD₃OD) δ 4.3 (s, 2H), 6.73 (dd, 1H), 6.78 (d, 2H), 7.2 (dd, 2H), 8.3 (s, 1H), 8.47 (d, 2H), 8.79 (d, 2H). LC-MS (10-90% CH3CN in H2O), Rt = 3.36 min, [M+H]⁺ = 311, [M-H]⁻ = 309.

**2-(Methyl-phenyl-amino)-N-(4-pyridin-4-yl-thiazol-2-yl)-acetamide:** 2-Chloro-N-(4-pyridin-4-yl-thiazol-2-yl)-acetamide (1mmol) and N-methylaniline (3 mmol) were suspended in dimethylacetamide and stirred at 70 °C overnight. The reaction mixture was filtered and the filtrate was injected into preparative HPLC. ¹H NMR (500 MHz, CD₃OD) δ 3.18 (s, 3H), 4.17 (s, 2H), 6.8 (d, 2H), 6.95 (dd, 1H), 7.32 (dd, 2H), 7.85 (s, 1H), 8.22 (d, 2H), 8.8 (d, 2H). LC-MS (10-90% CH₃CN in H₂O), Rt = 3.36 min, [M+H]⁺ = 329, [M-H]⁻ = 327.

Preparation of 3-Phenyl-1-(4-pyridin-4-yl-thiazol-2-yl)-piperazin-2-one

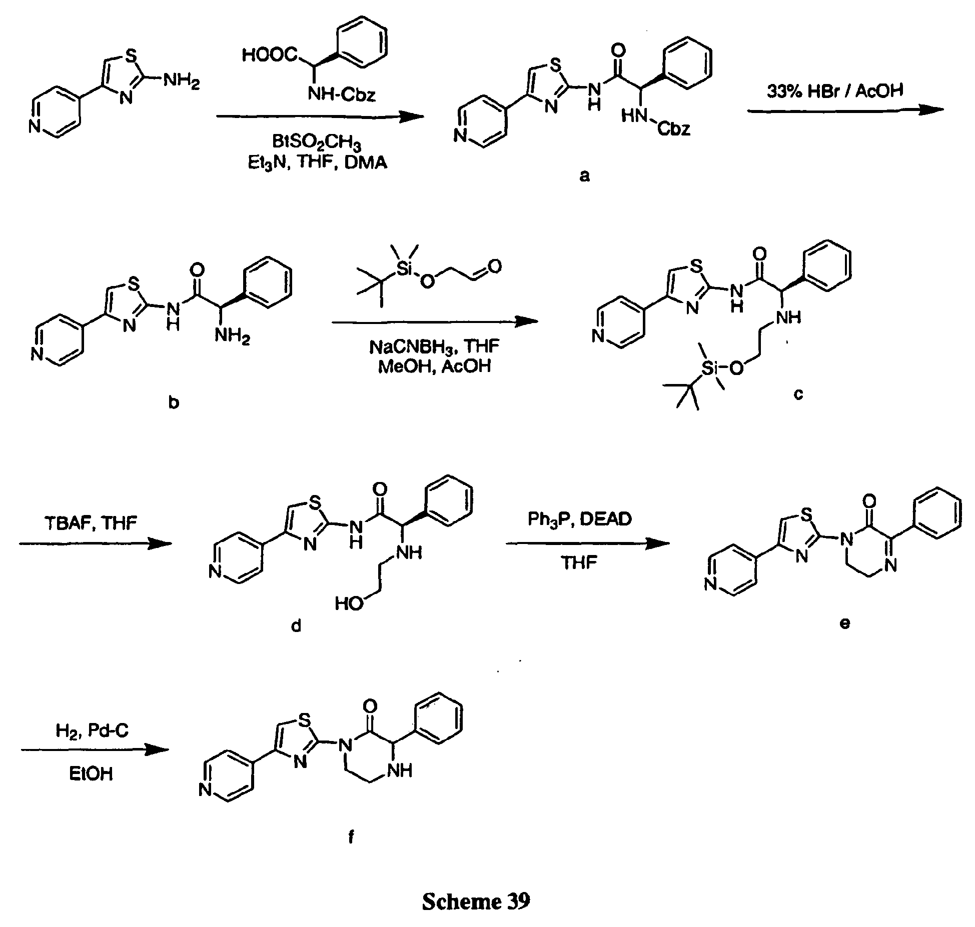

**[Phenyl-(4-pyridin-4-yl-thiazol-2-ylcarbamoyl)-methyl]-carbamic** acid benzyl ester: To a solution of 4-pyridin-4-yl-thiazol-2-ylamine; di-hydrobromide (1.0 g, 2.95 mmol) in 60 mL THF (tetrahydrofuran), was added 1-methanesulfonyl-1*H*-benzotriazole (1.0 g, 5.08 mmol), triethylamine (1.5 mL, 10.8 mmol), and benzyloxycarbonylamino-phenylacetic acid (1.0 g, 3.5 mmol) at ambient temperature. The mixture was heated using an oil bath to reflux for 12 hours. After the reaction was cooled the THF was removed under vacuum, and then the reaction was diluted with 100 mL of water and extracted with ethyl acetate (2 x 125 mL). The organic extracts were combined and washed with 10% citric acid, saturated sodium hydrogen carbonate aqueous, and brine. The organic layer was dried with sodium sulfate and concentrated *in vacuo* to give 1.7 g as a tan solid. The material was purified by crystallization from hot EtOAc-DCM (9:1: 40 mL) to give 0.7 g (54% of theory) of [phenyl-(4-pyridin-4-yl-thiazol-2-ylcarbamoyl)-methyl]-carbamic acid benzyl ester as a pale yellow solid. ¹H NMR (CD3CN) δ 10.4 (bs,1H), 8.65 (d,2H), 7.8 (d,2H), 7.7 (s,1H), 7.6-7.3 (m,5H) 6.65 (bs,1H), 5.65 (m,1H), 5.15 (s,1H). This material was used as is in the next step.

**2-Amino-2-phenyl-*N*-(4-pyridin-4-yl-thiazol-2-yl)-acetamide; hydrobromide (b):** A mixture of a (0.275g, 0.62mmol) in 5 mL of 33% HBr in acetic acid was prepared The mixture was heated at 100 °C, and the mixture turned a homogeneous. After stirring for 1.5 hours, the excess HBr in acetic acid was removed under vacuum to give quantitative conversion to **3** as a burnish red glass which turned to a foam under high vacuum. LC-MS (10-90% CH₃CN in H₂O), Rt = 0.31min, [M+H]⁺ = 311, [M-H]⁻ = 309.2. This material was used as is in the next step.

**2-[2-(tert-Butyl-dimethyl-silanyloxy)-ethylamino]-2-phenyl-N-(4-pyridin-4-yl-thiazol-2-yl)-acetamide (c):** To a solution of b (0.132g, 0.28mmol) in MeOH (6mL) was added (*tert*-Butyl-dimethyl-silanyloxy)-acetaldehyde (0.059 mL, 0.308mmol), 1M sodium cyanoborohydride in THF (0.4mL, 0.4mmol), and AcOH (0.05mL). The reaction was stirred at ambient temperature for 5 hours, and then the reaction was reduced to a solid under vacuum. The solid was purified by flash chromatography on silica gel by eluting with 25-100% EtOAc-Hexanes. The product c obtained (40mgs, 30% of theory) as colorless glass, and used as is without further purification.

**2-(2-Hydroxy-ethylamino)-2-phenyl-*N*-(4-pyridin-4-yl-thiazol-2-yl)-acetamide (d):** To a solution of c (0.14g, 0.297mmol) in THF (2mL) was added 1M tetrabutylammonium fluoride solution (1.0mL, 1mmol), and the reaction was stirred for 1.0 hr. The solvent was removed and the residue was purified by flash chromatography on silica gel by eluting with 10% MeOH-EtOAc. The product d obtained (67mgs, 64% of theory) as brown glass. ¹H NMR (500MHz, CDCl₃, ppm) δ 8.53 (d, 2H, J=6.19Hz), 7.60 (d, 2H, J=6.18Hz), 7.36-7.20 (m, 6H), 4.06-4.02 (m, 1H), 3.76-3.73 (m, 2H), 2.90-2.71 (m, 03H), 1.55 (m, 1H), 1.30-1.67 (m, 3H), 0.87 (t, 1H, J=7.65Hz). FIA/MS [M+H]⁺ = 355, [M-H]⁻ = 353. This material was used as is in the next step.

**3-Phenyl-1-(4-pyridin-4-yl-thiazol-2-yl)-5,6-dihydro-1*H*-pyrazin-2-one (e):** To a solution of **d** (35 mg, 0.10 mmol) in anhydrous THF was added triphenylphosphine (0.0314 g, 0.12 mmol), and diethyl azodicarboxylate (0.025 mL, 0.22 mmol). The reaction was stirred at room temperature for 1 hour or until the reaction showed no **d** remaining by HPLC. Solvent was removed from the reaction and then the material was taken up in EtOAc, and washed with brine. The residue was purified by preparative TLC chromatography on silica gel, eluting with 5% MeOH-EtOAc. The product was obtained as a colorless solid (16 mgs, 46% of theory). ¹H NMR (500MHz, CDCl₃) δ 8.60 (d, 2H, J=5.43Hz), 7.88 (m, 2H), 7.71 (m,2H), 7.46-7.36 (m, 4H), 4.55 (t, 2H, J=6.18), 4.15 (t, 2H, J=6.18). LC-MS (10-90% CH₃CN in H₂O), Rt = 2.1min, [M+H]⁺ = 335.

**3-Phenyl-1-(4-pyridin-4-yl-thiazol-2-yl)-piperazin-2-one (f):** To a solution of e (0.010g, 0.03mmol) in EtOH was added a catalytic amount of 10% palladium on carbon, and then the reaction was stirred under on atmosphere of hydrogen for 1.5 hour. The reaction was filtered through celite and purified by flash chromatography on silica gel by eluting with 5% MeOH-EtOAc. The product **f** obtained as a colorless glass (6.0 mgs, 60% of theory). ¹H NMR (500MHz, CDCl₃) δ 8.58 (d, 2H, J=5.97Hz), 7.71 (m, 2H), 7.46-7.18 (m, 6H), 4.78 (s, 1H), 4.50 (m, 1H), 4.20 (m, 1H), 3.40 (m, 1H), 3.25 (m, 1H). LC-MS (10-90% CH₃CN in H₂O), Rt = 0.25min, [M+H]⁺ = 337(strong).

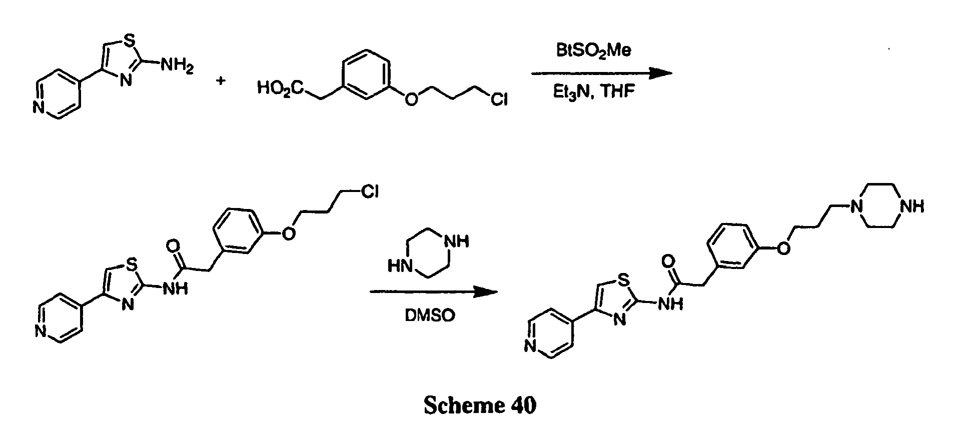

**2-[3-(3-Chloropropoxy)-phenyl]-N-(4-pyridin-4-yl-thiazol-2-yl)-acetamide:** A suspension of 1-methylsulfonylbenzentriazole (2.87 g, 0.0146 mol), [3-(3-chloro-propoxy)-phenyl]-acetic acid (3.33 g, 0.0146 mol) and triethylamine (2.94 g, 0.0291 mol) was stirred at room temperature for 3 hours, then to the suspension was added 4-pyridin-4-yl-thiazol-2-ylamine, and the reaction mixture was refluxed for 20h, then cooled to room temperature. The precipitated solid was filtered, washed and dried to afford 2-[3-(3-chloropropoxy)-phenyl]-N-(4-pyridin-4-yl-thiazol-2-yl)-acetamide, (2.5g, 64%). ¹H NMR (500 MHz, CD₃OD) δ 2.18 (m, 2H), 3.78 (s, 2H), 3.80 (t, 2H), 4.05 (t, 2H), 6.85 (dd, 1H), 6.9 (dd, 1H), 6.95 (d, 1H), 7.26 (dd, 1H), 7.85 (d, 2H), 8.02 (s, 1H), 8.63 (d, 2H).
**2-[3-(3-Piperazin-1-yl-propoxy)-phenyl]-N-(4-pyridin-4-yl-thiazol-2-yl)-acetamide:** A DMSO solution of 2-[3-(3-chloropropoxy)-phenyl]-N-(4-pyridin-4-yl-thiazol-2-yl)-acetamide (2.39 g, 6.2 mmol) and piperizine (2.12 g, 24.6 mol) was stirred at 60 °C overnight. The reactiom mixture was diluted with water, then purified by preparative HPLC to afford 2-[3-(3-piperazin-1-yl-propoxy)-phenyl]-N-(4-pyridin-4-yl-thiazol-2-yl)-acetamide, 1.9g. ¹H NMR (500 MHz, DMSO-d6) δ 2.1 (t, 2H), 3.2 (t, 2H), 3.38 (m, 8H), 3.8 (s, 2H), 4.04 (t, 2H), 6.85 (dd, 1H), 6.92 (s, 1H), 6.93 (d, 1H), 7.28 (dd, 1H), 8.17 (d, 2H), 8.33 (s, 1H), 8.8 (d,2H), 12.65 (s, br, 1H). LC-MS (5-45% CH₃CN in H₂O, Rt = 1.34 min, [M+H]⁺ = 438, [M-H]⁻ = 436.

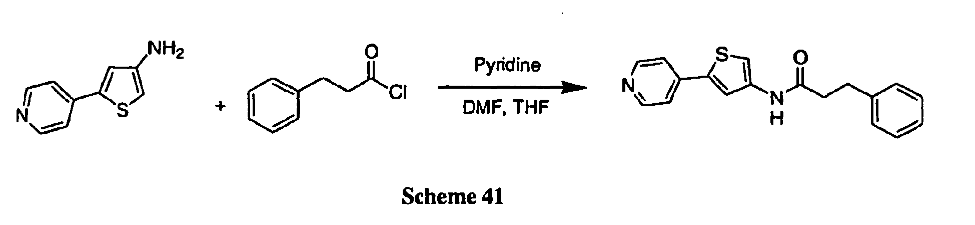

**3-Phenyl-N-(5-pyridin-4-yl-thiophen-3-yl)-propionamide:** A DMF/THF solution containing 5-pyridin-4-yl-thiophen-3-ylamine (60mg, 0.341 mmol), hydrocinnamyl chloride (76.8 mg, 0.411 mmol), and pyridine (32 mg, 0.411 mmol) was stirred at 70 °C for 3 hours, then the solvent was removed, the residue was dissolved in MeOH and purified by preparative HPLC. ¹H NMR (500 MHz, DMSO-d6) δ 2.64 (t, 2H), 2.92(t, 2H), 7.17 (m, 1H), 7.28 (m, 4H), 7.8 (s, 1H), 7.85 (s, 1H), 7.93 (d, 2H), 8.73 (d, 2H), 10.52 (s, 1H). LC-MS (10-90% CH₃CN in H₂O), Rt = 2.30 min, [M+H]⁺ = 309, [M-H]⁻ = 307.

EXEMPLARY COMPOUNDS: It will be appreciated that a variety of compounds can be prepared according to the general methods described above. Tables 4, 5, and 6 include exemplary data for certain compounds prepared according to the general methods described above.

**Table 4**

| **COMPOUND** | **LC MASS PLUS** | **LC MASS RT** |
|---|---|---|
| **I-A-1** | 380 | 1.12 |
| **I-A-2** | 320 | 1.66 |
| **I-A-3** | 350 | 1.09 |
| **I-A-4** | 350 | 1.11 |
| **I-A-5** | 380 | |
| **I-A-6** | 354 | 1.24 |
| **I-A-7** | 338 | 2.3 |
| **I-A-8** | 398 | 1.56 |
| **I-A-13** | 370 | 1.63 |
| **I-A-14** | 370 | 1.3 |
| **I-A-21** | 372.1 | 2.6 |
| **I-A-29** | 334.13 | 2.08 |
| **I-A-30** | 309.1 | 1.79 |
| **I-A-31** | 372.9 | 2.17 |
| **I-A-32** | 325 | 2.04 |
| **I-A-33** | 296 | 1.61 |
| **I-A-34** | 356 | 1.62 |
| **I-A-35** | 326 | 1.64 |
| **I-A-36** | 330 | 1.7 |
| **I-A-37** | 374 | 1.66 |
| **I-A-38** | 346 | 1.76 |
| **I-A-39** | 356 | 0.94 |
| **I-A-40** | 326 | 1.11 |
| **I-A-41** | 347.99 | 2.09 |
| **I-A-42** | 314.09 | 1.73 |
| **I-A-43** | 310.11 | 1.86 |
| **I-A-44** | 297 | 1.58 |
| **I-A-45** | 357 | 1.53 |
| **I-A-46** | 357 | 1.58 |
| **I-A-47** | 327 | 1.59 |
| **I-A-48** | 331 | 1.69 |
| **I-A-49** | 327 | 1.57 |
| **I-A-50** | 347 | 1.74 |
| **I-A-51** | 347 | 1.74 |
| **I-A-53** | 311.1 | 2.23 |
| **I-A-62** | 348.98 | 2.49 |
| **I-A-63** | 315 | 1.42 |

**Table 5**

| **COMPOUND** | **LC MASS PLUS** | **LC MASS RT** |
|---|---|---|
| **I-B-6** | 356 | 1.67 |
| **I-B-19** | 296 | 1.65 |
| **I-B-20** | 326.1 | 1.66 |
| **I-B-21** | 326 | 1.65 |
| **I-B-22** | 356 | 0.98 |
| **I-B-23** | 330 | 1.22 |
| **I-B-24** | 314 | 1.66 |
| **I-B-25** | 374 | 1.7 |
| **I-B-33** | 346 | 1.79 |
| **I-B-34** | 346 | 1.81 |
| **I-B-44** | 314 | 1.9 |
| **I-B-49** | 312.25 | 1.49 |
| **I-B-53** | 346.12 | 2.05 |
| **I-B-54** | 330.16 | 1.87 |
| **I-B-55** | 310 | 2.1 |
| **I-B-62** | 302.1 | 2.04 |
| **I-B-65** | 332.1 | 1.65 |
| **I-B-66** | 332.2 | 1.68 |
| **I-B-67** | 332.1 | 1.82 |
| **I-B-68** | 332.1 | 1.67 |
| **I-B-69** | 310.2 | 1.73 |
| **I-B-70** | 380.1 | 2.19 |
| **I-B-71** | 338.2 | 2.18 |
| **I-B-72** | 409.9 | 2.13 |
| **I-B-73** | 382.1 | 2.05 |
| **I-B-74** | 324.2 | 2.05 |
| **I-B-75** | 402.2 | 2.24 |
| **I-B-76** | 346 | 5.9 |
| **I-B-77** | 346 | 5.29 |
| **I-B-78** | 322.1 | 2.02 |
| **I-B-79** | 326 | 4.62 |
| **I-B-80** | 427.2 | 2.1 |
| **I-B-81** | 340.06 | 1.7 |
| **I-B-82** | 402.1 | 2.47 |
| **I-B-83** | 312 | 3.53 |
| **I-B-85** | 311 | 3.49 |
| **I-B-86** | 326 | 4.45 |
| **I-B-87** | 351.1 | 3.29 |
| **I-B-88** | 367.2 | |
| **I-B-89** | 390.10348 | 1.72 |
| **I-B-90** | 328.1 | 1.9 |
| **I-B-91** | 381.9 | 2.23 |
| **I-B-92** | 348 | 1.3 |
| **I-B-93** | 348 | 1.94 |
| **I-B-94** | 350.1 | 1.8 |
| **I-B-95** | 348 | 1.66 |
| **I-B-96** | 396 | 2.41 |
| **I-B-97** | 346.1 | 1.92 |
| **I-B-98** | 336.1 | |
| **I-B-99** | 336.1 | 1.84 |
| **I-B-100** | 352.1 | 1.46 |
| **I-B-101** | 308.1 | 2.02 |
| **I-B-102** | 332.1 | 1.77 |
| **I-B-103** | 324.1 | 2.3 |
| **I-B-104** | 328 | 1.75 |
| **I-B-105** | 328.1 | 2.11 |
| **I-B-106** | 337.1 | 1.5 |
| **I-B-107** | 342.1 | 1.36 |
| **I-B-108** | 311 | 3.13 |
| **I-B-109** | 324.1 | 1.99 |
| **I-B-110** | 324.1 | 1.97 |
| **I-B-111** | 325 | 3.9 |
| **I-B-112** | 325 | 3.92 |
| **I-B-113** | 343.1 | 1.38 |
| **I-B-114** | 361.1 | 1.56 |
| **I-B-115** | 326.1 | 1.66 |
| **I-B-116** | 340.1 | 1.75 |
| **I-B-117** | 342.1 | 1.39 |
| **I-B-118** | 310.1 | 1.82 |
| **I-B-119** | 344 | 2.17 |
| **I-B-120** | 359 | 1.83 |
| **I-B-121** | 328.1 | 1.92 |
| **I-B-122** | 355.1 | 1.58 |
| **I-B-123** | 337.1 | 1.58 |
| **I-B-124** | 339.2 | 3.84 |
| **I-B-125** | 339.1 | 4.32 |
| **I-B-126** | 348 | 3.88 |
| **I-B-127** | 328 | 2.89 |
| **I-B-128** | 342 | 3.54 |
| **I-B-129** | 345.9 | 4.33 |
| **I-B-130** | 330 | 3.68 |
| **I-B-131** | 312 | 1.67 |
| **I-B-132** | 342 | 1.71 |
| **I-B-133** | 354.1 | 1.67 |
| **I-B-134** | 348 | 1.92 |
| **I-B-135** | 354.1 | 1.64 |
| **I-B-136** | 330 | 1.75 |
| **I-B-137** | 346 | |
| **I-B-138** | 356.1 | 1.71 |
| **I-B-139** | 363.1 | 4.88 |
| **I-B-140** | 363.1 | 4.84 |
| **I-B-141** | 326 | 1.96 |
| **I-B-142** | 364 | 2.05 |
| **I-B-143** | 355.1 | 1.25 |
| **I-B-144** | 348 | 1.8 |
| **I-B-145** | 363 | 4.92 |
| **I-B-146** | 343 | 2.13 |
| **I-B-147** | 351.1 | 2.77 |
| **I-B-148** | 421.9 | 1.96 |
| **I-B-149** | 421.9 | 1.95 |
| **I-B-150** | 343 | |
| **I-B-151** | 357 | 4.88 |
| **I-B-152** | 340 | 2.01 |
| **I-B-153** | 328.1 | 2.31 |
| **I-B-154** | 360.1 | 2.71 |
| **I-B-155** | 328 | 5.09 |
| **I-B-156** | 360 | 3.77 |
| **I-B-157** | 343.9 | 3.36 |
| **I-B-158** | 359 | 2.64 |
| **I-B-159** | 377 | 2.89 |
| **I-B-160** | 392.9 | 3.8 |
| **I-B-161** | 373 | 3.3 |
| **I-B-162** | 348 | 3.93 |
| **I-B-163** | 342.1 | 3.81 |
| **I-B-164** | 302 | 1.62 |
| **I-B-165** | 340 | 2.1 |
| **I-B-166** | 335 | 1.9 |
| **I-B-167** | 311 | 1.88 |
| **I-B-168** | 341 | 2.25 |
| **I-B-169** | 329.1 | 2.21 |
| **I-B-170** | 329.2 | 1.84 |
| **I-B-171** | 343 | 1.1 |
| **I-B-174** | 363 | 2.1 |
| **I-B-175** | 375.2 | 1.78 |
| **I-B-176** | 435.9 | |
| **I-B-177** | 325 | 2.1 |
| **I-B-178** | 355.1 | 2.21 |
| **I-B-179** | 417.8 | 2.2 |
| **I-B-180** | 373.9 | 2.1 |
| **I-B-181** | 477.9 | |
| **I-B-183** | 351 | 2.2 |
| **I-B-185** | 389.9 | 1.8 |
| **I-B-186** | 433.8 | 1.8 |
| **I-B-187** | 431.9 | 2.4 |
| **I-B-190** | 402 | 2.7 |
| **I-B-191** | 329.9 | 2.5 |
| **I-B-192** | 423.1 | 0.4 |
| **I-B-193** | 439.1 | 0.86 |
| **I-B-194** | 443 | 1.5 |
| **I-B-197** | 347.9 | 2.8 |
| **I-B-198** | 360 | 2.9 |
| **I-B-199** | 437.1 | 2.4 |
| **I-B-200** | 453.1 | 2.15 |
| **I-B-201** | 457 | 2.4 |
| **I-B-202** | 423.2 | 1.8 |
| **I-B-203** | 453.2 | 2.24 |
| **I-B-204** | 356 | 1.4 |
| **I-B-205** | 423.1 | 2.17 |
| **I-B-206** | 397 | 2.03 |
| **I-B-207** | 437.1 | 2.3 |
| **I-B-208** | 438.6 | 1.34 |
| **I-B-209** | 452.1 | 1.75 |
| **I-B-210** | 412.9 | 1.96 |
| **I-B-211** | 383.1 | 1.92 |
| **I-B-212** | 427.1 | 0.75 |
| **I-B-213** | 453.4 | 2.14 |
| **I-B-214** | 467.2 | 2.03 |
| **I-B-215** | 439.2 | 1.97 |
| **I-B-216** | | 1.99 |
| **I-B-217** | 383.1 | 1.63 |
| **I-B-218** | 423.1 | 2.1 |
| **I-B-219** | 424.1 | 1.09 |
| **I-B-220** | 438.2 | 1.55 |
| **I-B-221** | 399.1 | 1.56 |
| **I-B-222** | 369.1 | 1.62 |
| **I-B-223** | 413.1 | 1.5 |
| **I-B-224** | 427 | 2.13 |
| **I-B-225** | 439.1 | 1.24 |
| **I-B-226** | 451.3 | 1.13 |
| **I-B-227** | 425.1 | 1.12 |
| **I-B-228** | 381.1 | |
| **I-B-229** | 445 | 2.2 |
| **I-B-230** | 414 | 2.6 |
| **I-B-231** | 436.1 | 1.4 |
| **I-B-232** | 397 | 1.61 |
| **I-B-233** | 397 | 1.59 |
| **I-B-234** | 417 | 1.75 |
| **I-B-235** | 427 | 1.12 |
| **I-B-236** | 427 | 1.23 |
| **I-B-237** | 385 | 1.22 |
| **I-B-238** | 367 | 1.21 |
| **I-B-239** | 447 | 1.28 |
| **I-B-241** | 313.1 | 1.8 |
| **I-B-242** | 331.1 | 2.06 |
| **I-B-243** | 327.1 | 2.17 |
| **I-B-244** | 323.1 | 2.23 |
| **I-B-245** | 359.1 | 2.57 |
| **I-B-246** | 373 | 2.13 |
| **I-B-247** | 329 | 2.11 |
| **I-B-248** | 347 | 2.18 |
| **I-B-249** | 363 | 2.42 |
| **I-B-250** | 416.9 | 2.19 |
| **I-B-251** | 372.9 | 2.16 |
| **I-B-252** | 325.1 | 2.01 |
| **I-B-253** | 313.1 | 2.05 |
| **I-B-257** | 343 | 6.74 |
| **I-B-258** | 357.1 | 3.84 |
| **I-B-261** | 330 | 4.84 |
| **I-B-262** | 344 | 5.44 |
| **I-B-263** | 387.1 | 4.98 |
| **I-B-264** | 400.7 | 5.42 |
| **I-B-265** | 370.1 | 5.69 |
| **I-B-265** | 384.1 | 6.96 |
| **I-B-267** | 315.1 | 2.91 |
| **I-B-268** | 315.1 | 2.96 |
| **I-B-269** | 344.9 | 3.3 |
| **I-B-270** | 311 | 3.13 |
| **I-B-271** | 327 | 2.84 |
| **I-B-272** | 330.9 | 3.25 |
| **I-B-273** | 297 | 2.88 |
| **I-B-274** | 327 | 2.88 |
| **I-B-275** | 340 | 2.2 |
| **I-B-276** | 354.1 | 2.3 |
| **I-B-277** | 389.9 | 1.9 |
| **I-B-278** | 314 | |
| **I-B-280** | 340.0 | 2.20 |
| **I-B-281** | 354.1 | 2.3 |
| **I-B-282** | 389.9 | 1.9 |
| **I-B-283** | 354.1 | 2.3 |
| **I-B-286** | 380.1 | 2.5 |
| **I-B-287** | 354.1 | 2.4 |
| **I-B-288** | 354.1 | 2.5 |
| **I-B-289** | 366.10 | 2.40 |
| **I-B-290** | 437.2 | 2.39 |
| **I-B-292** | 466.2 | 1.65 |
| **I-B-293** | 420.2 | 2.11 |
| **I-B-299** | 346.1 | 3.17 |
| **I-B-306** | 452.2 | 1.8 |
| **I-B-307** | 466.2 | 1.9 |
| **I-B-308** | 451.2 | 2.68 |
| **I-B-309** | 466.2 | 1.4 |
| **I-B-310** | 296.0 | 3.3 |
| **I-B-311** | 310.0 | 3.5 |
| **I-B-312** | 465.2 | 2.48 |
| **I-B-315** | 479.2 | 3.76 |
| **I-B-316** | 314.0 | 2.9 |
| **I-B-317** | 314.0 | 2.8 |
| **I-B-318** | 332.0 | 2.9 |
| **I-B-319** | 328.0 | 3.1 |
| **I-B-320** | 320.0 | 1.58 |
| **I-B-321** | 326.2 | 1.89 |
| **I-B-323** | 430.2 | 2.29 |
| **I-B-324** | 338.2 | 2.16 |
| **I-B-325** | 380.2 | 2.54 |
| **I-B-326** | 389.2 | 1.72 |
| **I-B-327** | 403.2 | 1.92 |
| **I-B-328** | 354.3 | 2.47 |
| **I-B-329** | 338.2 | 2.65 |
| **I-B-330** | 356.3 | 2.79 |
| **I-B-331** | 420.1 | 2.8 |
| **I-B-332** | 404.2 | 2.0 |
| **I-B-333** | 326.0 | 1.9 |
| **I-B-334** | 340.0 | 2.0 |
| **I-B-335** | 403.1 | 1.89 |
| **I-B-336** | 389.1 | 1.72 |
| **I-B-339** | 352.2 | 1.9 |
| **I-B-340** | 335.0 | 0.25 |
| **I-B-341** | 335.1 | 2.1 |
| **I-B-342** | 336.98 | 0.25 |
| **I-B-343** | 336 | 2.3 |
| **I-B-344** | 372.1 | 2.4 |
| **I-B-345** | 374.0 | 1.0 |
| **I-B-346** | 314.00 | 2.0 |
| **I-B-347** | 437.3 | 2.13 |

**Table 6**

| **COMPOUND** | **LC MASS PLUS** | **LC MASS RT** |
|---|---|---|
| **I-C-1** | 309 | 2.3 |
| **I-C-2** | 325 | 2.05 |
| **I-C-3** | 329 | 2.67 |
| **I-C-4** | 295 | 1.92 |
| **I-C-5** | 325 | 2.17 |
| **I-C-6** | 313 | 2.21 |
| **I-C-7** | 372.9 | 2.5 |
| **I-C-8** | 351.2 | 1.17 |
| **I-C-9** | 437.2 | 0.88 |
| **I-C-10** | 438.1 | 1.79 |
| **I-C-11** | 335 | 2.1 |
| **I-C-12** | 363 | 2.5 |
| **I-C-13** | 356 | 1.51 |
| **I-C-14** | 356 | 1.63 |
| **I-C-15** | 326 | 1.65 |
| **I-C-6** | 330 | 1.74 |
| **I-C-17** | 326 | 1.63 |
| **I-C-18** | 374 | 1.71 |
| **I-C-19** | 296 | 1.42 |
| **I-C-20** | 346 | 1.61 |
| **I-C-21** | 348 | 2.47 |
| **I-C-22** | 314.04 | 1.99 |
| **I-C-23** | 310.1 | |
| **I-C-24** | 331.0 | 1.99 |
| **I-C-26** | 420.9 | 1.86 |
| **I-C-27** | 329.0 | 2.10 |
| **I-C-28** | 389.1 | 1.87 |
| **I-C-29** | 326.1 | 2.84 |
| **I-C-30** | 408.95 | 2.42 |
| **I-C-31** | 313.0 | 1.92 |
| **I-C-32** | 239.0 | 2.3 |
| **I-C-33** | 332.0 | 3.0 |
| **I-C-34** | 345.1 | 2.34 |
| **I-C-35** | 314.0 | 4.63 |
| **I-C-36** | 389.1 | 1.77 |
| **I-C-37** | 311.0 | 1.50 |
| **I-C-38** | 325 | 1.9 |
| **I-C-39** | 435.0 | 2.10 |
| **I-C-40** | 370.20 | |
| **I-C-41** | 371.0 | 2.7 |
| **I-C-42** | 374.2 | 3.23 |
| **I-C-43** | 310.0 | 3.16 |
| **I-C-44** | 438.4 | 1.39 |
| **I-C-45** | 452.4 | 2.42 |
| **I-C-46** | 437.1 | 3.34 |
| **I-C-47** | 465.2 | 3.35 |

BIOLOGICAL TESTING

Example 1: ROCK INHIBITION ASSAY

Compounds were screened for their ability to inhibit ROCK I (AA 6-553) activity using a standard coupled enzyme system (Fox et al. (1998) Protein Sci. 7, 2249). Reactions were carried out in a solution containing 100 mM HEPES (pH 7.5), 10 mM MgCl₂, 25 mM NaCl, 2 mM DTT and 1.5% DMSO. Final substrate concentrations in the assay were 45 µM ATP (Sigma Chemicals, St Louis, MO) and 200 µM peptide (American Peptide, Sunnyvale, CA). Reactions were carried out at 30 °C and 45 nM ROCK I. Final concentrations of the components of the coupled enzyme system were 2.5 mM phosphoenolpyruvate, 350 µM NADH, 30 µg/ml pyruvate kinase and 10 µg/ml lactate dehydrogenase.

Compounds of the invention were found to inhibit ROCK. In certain embodiments, compounds were shown to have a Kᵢ of less than 1 µM for ROCK

Example 2: ERK INHIBITION ASSAY

Compounds were assayed for the inhibition of ERK2 by a spectrophotometric coupled-enzyme assay (Fox et al (1998) Protein Sci 7, 2249). In this assay, a fixed concentration of activated ERK2 (10 nM) was incubated with various concentrations of the compound in DMSO (2.5 %) for 10 min. at 30°C in 0.1 M HEPES buffer, pH 7.5, containing 10 mM MgCl₂, 2.5 mM phosphoenolpyruvate, 200 µM NADH, 150 µg/mL pyruvate kinase, 50 µg/mL lactate dehydrogenase, and 200 µM erktide peptide. The reaction was initiated by the addition of 65 µM ATP. The rate of decrease of absorbance at 340 nM was monitored. The Kᵢ was determined from the rate data as a function of inhibitor concentration.

Compounds of the invention were found to inhibit ERK2. In certain embodiments, compounds were shown to have a Kᵢ of less than 1 µM for ERK2

Example 3: GSK INHIBITION ASSAY

Compounds were screened for their ability to inhibit GSK-3β (AA 1-420) activity using a standard coupled enzyme system (Fox et al. (1998) Protein Sci. 7, 2249). Reactions were carried out in a solution containing 100 mM HEPES (pH 7.5), 10 mM MgCl₂, 25 mM NaCl, 300 µM NADH, 1 mM DTT and 1.5% DMSO. Final substrate concentrations in the assay were 20 µM ATP (Sigma Chemicals, St Louis, MO) and 300 µM peptide (American Peptide, Sunnyvale, CA). Reactions were carried out at 30 °C and 20 nM GSK-3β. Final concentrations of the components of the coupled enzyme system were 2.5 mM phosphoenolpyruvate, 300 µM NADH, 30 µg/ml pyruvate kinase and 10 µg/ml lactate dehydrogenase.

An assay stock buffer solution was prepared containing all of the reagents listed above with the exception of ATP and the test compound of interest. The assay stock buffer solution (175 µl) was incubated in a 96 well plate with 5 µl of the test compound of interest at final concentrations spanning 0.002 µM to 30 µM at 30 °C for 10 min. Typically, a 12 points titration was conducted by preparing serial dilutions (from 10 mM compound stocks) with DMSO of the test compounds in daughter plates. The reaction was initiated by the addition of 20 µl of ATP (final concentration 20 µM). Rates of reaction were obtained using a Molecular Devices Spectramax plate reader (Sunnyvale, CA) over 10 min at 30°C. The Kᵢ values were determined from the rate data as a function of inhibitor concentration.

Compounds of the invention were found to inhibit GSK3. In certain embodiments, compounds were shown to have a Kᵢ of less than 1 µM for GSK3.

Example 4: PKA Inhibition Assay

Compounds were screened for their ability to inhibit PKA using a standard coupled enzyme assay (Fox et al., Protein Sci, 1998, 7, 2249). Assays were carried out in a mixture of 100 mM HEPES (pH 7.5), 10 mM MgCl₂, 25 mM NaCl , 1 mM DTT and 3% DMSO. Final substrate concentrations in the assay were 50 µM ATP (Sigma Chemicals) and 80 µM peptide (Kemptide, American Peptide, Sunnyvale, CA). Assays were carried out at 30 °C and 18 nM PKA. Final concentrations of the components of the coupled enzyme system were 2.5 mM phosphoenolpyruvate, 300 µM NADH, 30 µg/ml pyruvate kinase and 10 µg/ml lactate dehydrogenase.

An assay stock buffer solution was prepared containing all of the reagents listed above, with the exception of ATP, and the test compound of the present invention. 55 µl of the stock solution was placed in a 96 well plate followed by addition of 2 µl of DMSO stock containing serial dilutions of the test compound of the present invention (typically starting from a final concentration of 5µM). The plate was preincubated for 10 minutes at 30°C and the reaction initiated by addition of 5 µl of ATP (final concentration 50 µM). Initial reaction rates were determined with a Molecular Devices SpectraMax Plus plate reader over a 15 minute time course. IC₅₀ and Kᵢ data were calculated from non-linear regression analysis using the Prism software package (GraphPad Prism version 3.0a for Macintosh, GraphPad Software, San Diego California, USA).

Compounds of the invention were found to inhibit PKA. In certain embodiments, compounds were shown to have a Kᵢ of less than 1 µM for PKA.

## Claims

1. A compound of formula **I:** 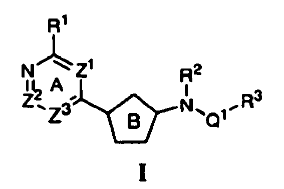 or a pharmaceutically acceptable salt thereof, wherein: wherein 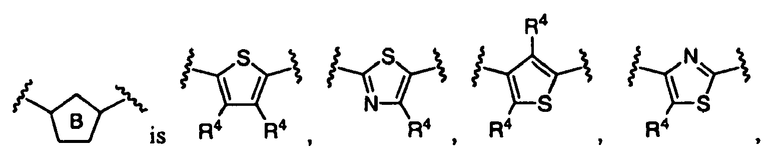 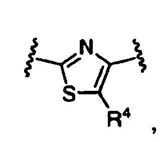 or 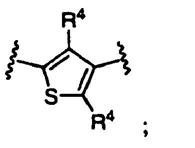 R¹ is halogen, CN, NO₂, or VₘR;
Z¹ and Z³ are each CR^{Z} and Z² is CR¹;
each occurrence of R^{Z} is independently halogen, CN, NO₂, or UₙR';
R² is UₙR';
each occurrence of R⁴ is independently halogen, CN, NO₂, or VₘR;
each occurrence of U or V is independently an optionally substituted C₁₋₆ alkylidene chain, wherein up to two methylene units of the chain are optionally and independently replaced by -NR-, -S-, -O-, -CS-, -CO₂-, -OCO-, -CO-, -COCO-, -CONR-, -NRCO-, -NRCO₂-, -SO₂NR-, -NRSO₂-, -CONRNR-, -NRCONR-, -OCONR-, -NRNR-, -NRSO₂NR-, -SO-, -SO₂-, -PO-, -PO₂-, or -POR-;
m and n are each independently 0 or 1;
each occurrence of R is independently hydrogen or an optionally substituted C₁₋₆ aliphatic group; and each occurrence of R^{'} is independently hydrogen or an optionally substituted C₁₋₆ aliphatic group, a 3-8-membered saturated, partially unsaturated, or fully unsaturated monocyclic ring having 0-3 heteroatoms independently selected from nitrogen, oxygen, or sulfur, or an 8-12 membered saturated, partially unsaturated, or fully unsaturated bicyclic ring system having 0-5 heteroatoms independently selected from nitrogen, oxygen, or sulfur; or R and R^{'}, two occurrences of R, or two occurrences of R^{'}, are taken together with the atom(s) to which they are bound to form an optionally substituted 3-12 membered saturated, partially unsaturated, or fully unsaturated monocyclic or bicyclic ring having 0-4 heteroatoms independently selected from nitrogen, oxygen, or sulfur;
Q¹ is -CO-;
R³ is Q²-Ar¹, wherein Q² is -(CHR⁶)_{q}-, where q is 1, 2, or 3,
or R² and Q¹-R³, taken together with the intervening nitrogen atom, form the cyclic group: 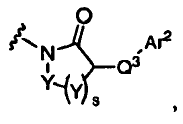 where s is 1 or 2, each occurrence of Y is independently, as valency and stability permit, -CO-, -CS-, -SO₂-, -O-, -S-, -NR⁵-, or -C(R⁵)₂-, and R⁵ is UₙR';
Q³ is a bond or a C₁₋₆ alkylidene chain, wherein up to two methylene units of the chain are each optionally and independently replaced by -NR'-, -S-, -O-, -CS-, -CO₂-, -OCO-, -CO-, -COCO-, -CONR'-, -NR'CO-, -NR'CO₂-, -SO₂NR'-, -NR'SO₂-, -CONR'NR'-, -NR'CONR'-, -OCONR'-, -NR'NR'-, -NR'SO₂NR'-, -SO-, -SO₂-, -PO-, -PO₂-, or -POR'-; and wherein any carbon atom in the one or more methylene units is optionally substituted with one or two occurrences of R⁶, wherein each occurrence of R⁶ is independently halogen, CN, NO₂, or UₙR', or two occurrences of R⁶, or R' and R⁶, taken together with the atoms to which they are bound, form an optionally substituted 3-6-membered cycloalkyl, heterocyclyl, aryl or heteroaryl ring; and
Ar¹ is: 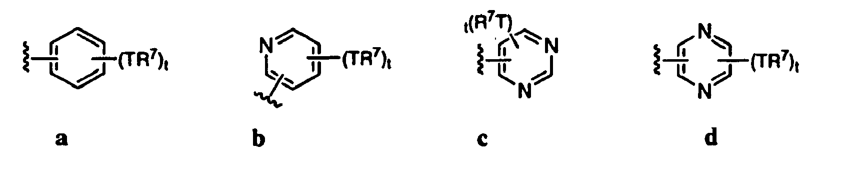 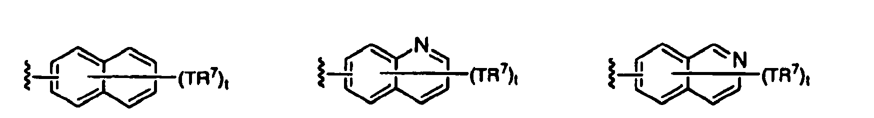 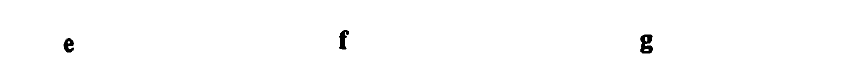 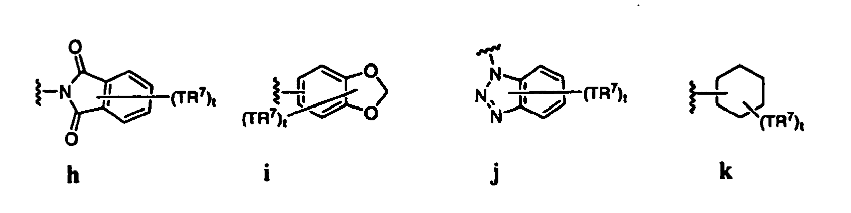 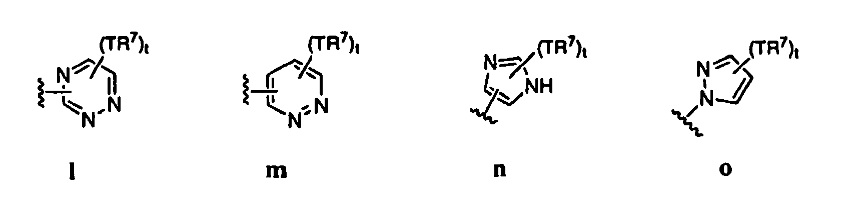 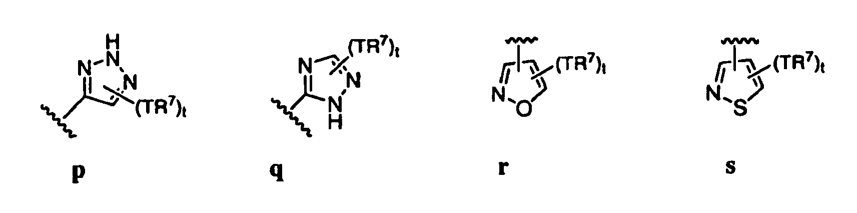 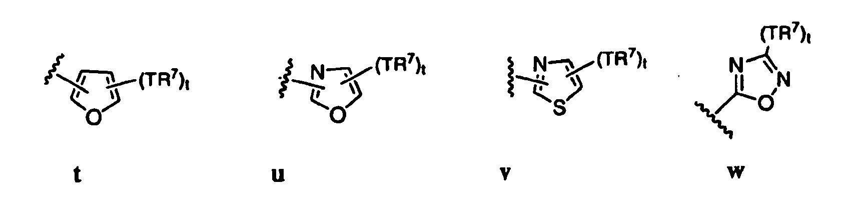 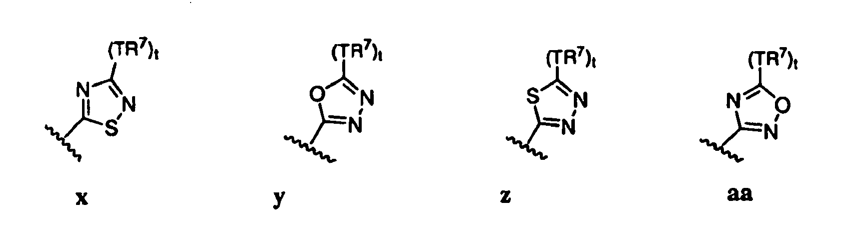 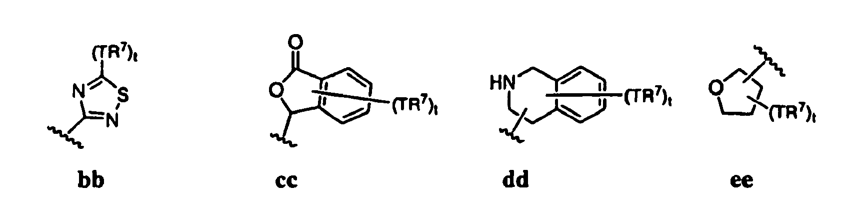 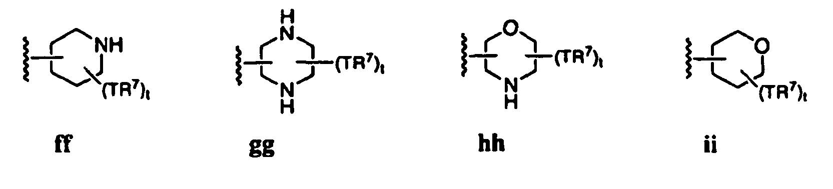 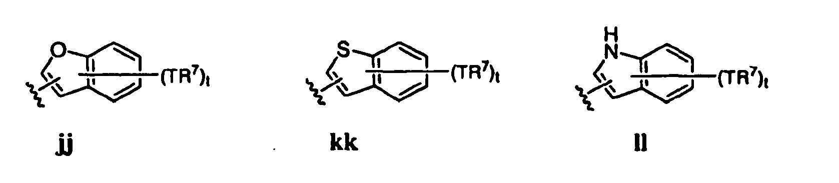 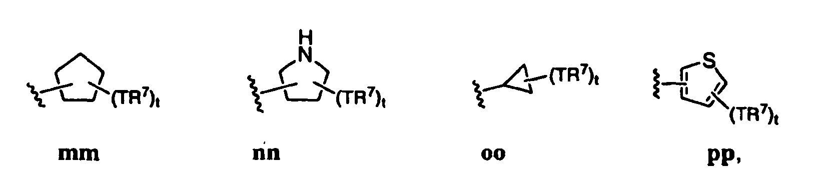 wherein t is 0, 1, 2, 3, 4 or 5, and wherein any Ar¹ is bonded to Q² through any substitutable nitrogen or carbon atom, and wherein one or more hydrogen atoms on any substitutable nitrogen or carbon atom is substituted with one or more independent occurrences of TR⁷;
and Ar² is a 5-8 membered saturated, partially unsaturated, or fully unsaturated monocyclic ring having 0-3 heteroatoms independently selected from nitrogen, oxygen, or sulfur, or an 8-12 membered saturated, partially unsaturated, or fully unsaturated bicyclic ring system having 0-5 heteroatoms independently selected from nitrogen, oxygen, or sulfur; wherein Ar¹ and Ar² are each optionally substituted with 0-5 independent occurrences of TR⁷; wherein T is a bond or is a C₁-C₆ alkylidene chain wherein up to two methylene units of T are optionally and independently replaced by -NR-, -S-, -O-, -CS-, -CO₂-, -OCO-, -CO-, -COCO-, -CONR-, -NRCO-, -NRCO₂-, -SO₂NR-, -NRSO₂-, -CONRNR-, -NRCONR-, -OCONR-, -NRNR-, -NRSO₂NR-, -SO-, -SO₂-, -PO-, -PO₂-, or -POR-; and each occurrence of R⁷ is independently R', halogen, NO₂, or CN;
provided that:
for compounds having the structure: 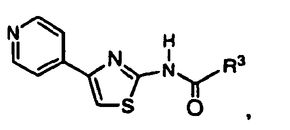 R³ is not any one of the following groups: -CH₂(3-NHCOPh-phenyl), -CH₂-pyrrolidine, unsubstituted benzyl, -CH₂-naphthyl, -CH₂CH₂-3-(4-Cl-phenyl)-1-phenyl-1-H-pyrazol-4-yl, or -CH₂(1,3-dioxoisoindole).

2. The compound of claim 1, wherein:
(1) R² is hydrogen, or is UₙR', where n is 1, and U is a C₁₋₆ alkylidene chain wherein one or two methylene units are optionally and independently replaced by O, NR, S, or C(O),
preferably, wherein U is -CH₂-, -CH₂CH₂-, -CH₂CH₂CH₂-,
-CH₂CH₂CH₂CH₂-, -CH₂O-, -CH₂S-, -CH₂NR-, -CH₂CH₂O-, -CH₂CH₂S-,
-CH₂CH₂NR-, -CH₂CH₂CH₂O-, -CH₂CH₂CH₂S-, -CH₂CH₂CH₂NR-,
-CH₂CH₂CH₂CH₂O-, -CH₂CH₂CH₂CH₂S-, -CH₂CH₂CH₂CH₂NR-,
-CH₂CH₂OCH₂CH₂-, -(CH₂)₄NHCH₂-, -(CH₂)₃NHCH₂CH₂-, or
-CH₂CH₂NHCH₂CH₂-, and, preferably, R' is selected from hydrogen, C₁-C₄alkyl, optionally substituted tetrahydropyranyl, pyrrolidinyl, piperidinyl, piperazinyl, morpholinyl, thiomorpholinyl, pyridinyl, phenyl, or cyclohexyl, or R and R', taken together with the nitrogen atom to which they are bound, form an optionally substituted 5- or 6-membered heterocyclyl ring; or
(2) Ar¹ is: 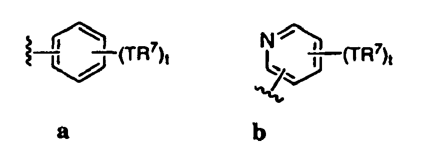 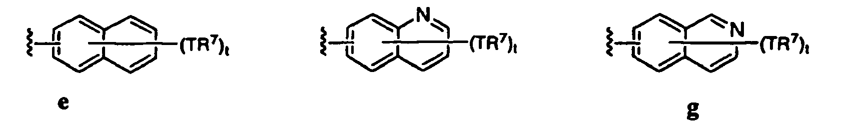 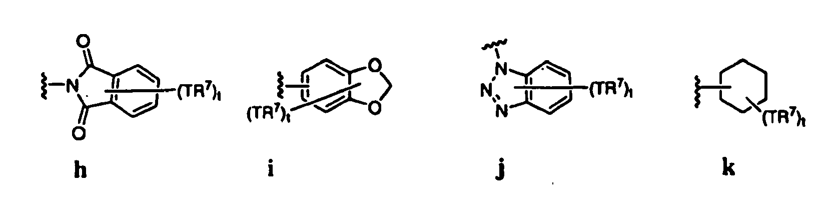 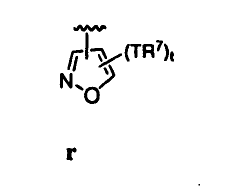 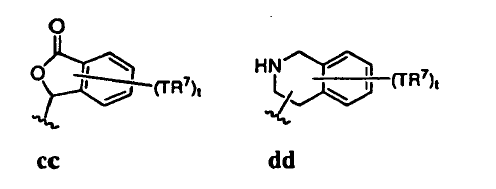 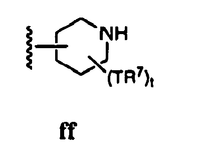 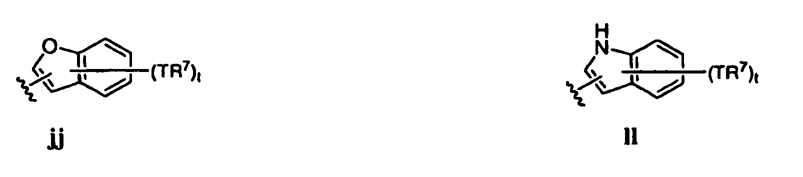 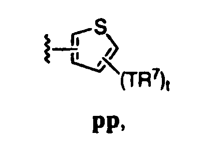 wherein t is 0, 1, 2, 3, 4 or 5, and wherein any Ar¹ is bonded to Q² through any substitutable nitrogen or carbon atom, and wherein one or more hydrogen atoms on any substitutable nitrogen or carbon atom is substituted with one or more independent occurrences of TR⁷;
wherein:
(a) T is a bond or is an optionally substituted C₁₋₆ alkylidene chain wherein one or two methylene units are optionally and independently replaced by -O-, -NR-, -S-, -SO₂-,-COO-, -CO-, -OSO₂-, -NRSO₂, -CONR-, or -SO₂NR-, and R⁷ is R' or halogen; or
(b) each occurrence of TR⁷ is independently -C₁₋₃alkyl, -OR', -SR', -CF₃, -OCF₃, -SCF₃, -F, -Cl, I, -Br, -COOR', -COR', -O(CH₂)₄N(R)(R'), - O(CH₂)₃N(R)(R'), -O(CH₂)₂N(R)(R'), -O(CH₂)N(R)(R'), -O(CH₂)₄CON(R)(R'), - O(CH₂)₃CON(R)(R'), -O(CH₂)₂CON(R)(R'), -O(CH₂)CON(R)(R'), - C(O)N(R)(R'), -(CH₂)₄OR', -(CH₂)₃OR', -(CH₂)₂OR', -CH₂OR', optionally substituted phenyl or benzyl, -N(R)(R'), -(CH₂)₄N(R)(R'), -(CH₂)₃N(R)(R'), - (CH₂)₂N(R)(R'), -(CH₂)N(R)(R'), or SO₂N(R)(R'), NRSO₂R', CON(R)(R'), or-OSO₂R'.

3. The compound of claim 1, wherein R² and Q¹-R³, taken together with the intervening nitrogen atom, form the cyclic group: 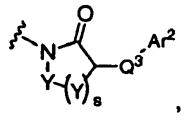 where s is 1 or 2, each occurrence of Y is independently, as valency and stability permit, -CO-, -CS-, -SO₂-, -O-, -S-, -NR⁵-, or -C(R⁵)₂-, and R⁵ is UₙR', and compounds of formula **I-A-ii, I-B-ii,** and **I-C-ii** are provided: 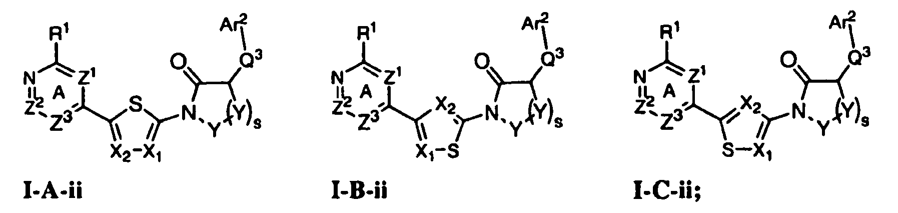 wherein:
(1) Q³ is a direct bond, or is -(CHR⁶)_{q}-, -(CHR⁶)_{q}O-, -(CHR⁶)_{q}S-, - (CHR⁶)_{q}S(O)₂-, -(CHR⁶)_{q}S(O)- , -(CHR⁶)_{q}NR-, or -(CHR⁶)_{q}C(O)-, wherein q is 0, 1, 2, or 3, and R⁶ is R', -N(R)(R'), -(CH₂)₁₋₄N(R)(R'), -OR', -(CH₂)₁₋₄OR', -NR(CH₂)₁₋₄N(R)(R'), -NR(CH₂)₁₋₄SO₂R', -NR(CH₂)₁₋₄COOR', or -NR(CH₂)₁₋₄COR', or two occurrences of R⁶, taken together with the atoms to which they are bound, form an optionally substituted 3-6-membered saturated, partially unsaturated, or fully unsaturated ring; for example wherein: R⁶ is CH₂OH, CH₂CH₂OH, OH, OMe, OEt, NH₂, NH(Me), NH(Et), N(Me)(Me), CH₂NH₂, CH₂CH₂NH₂. NHCO₂*t*-butyl, phenyl, cyclopentyl, methyl, ethyl, isopropyl, cyclopropyl, NH(CH₂)₃NH₂, NH(CH₂)₂NH₂, NH(CH₂)₂NHEt, NHCH₂pyridyl, NHSO₂phenyl, NHC(O)CH₂C(O)O*t*-butyl, NHC(O)CH₂NH₃, and NHCH₂-imidazol-4-yl;
(2) Ar² is: 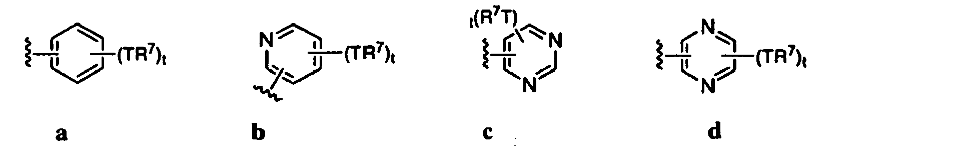 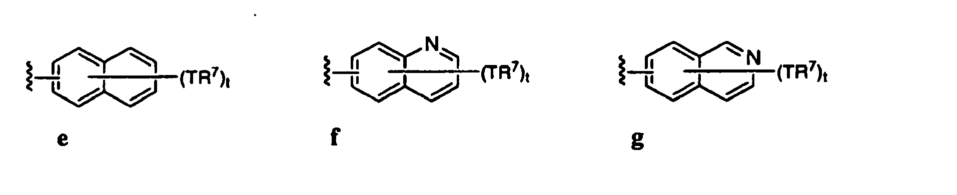 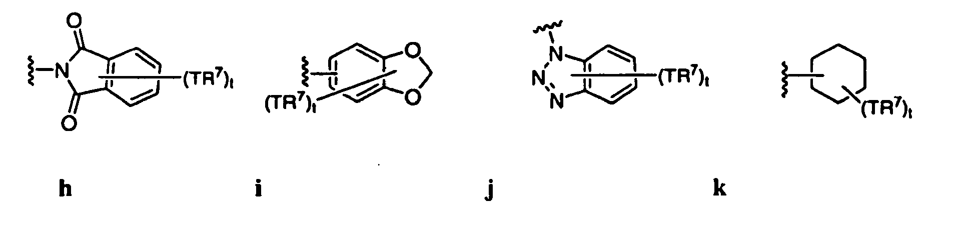 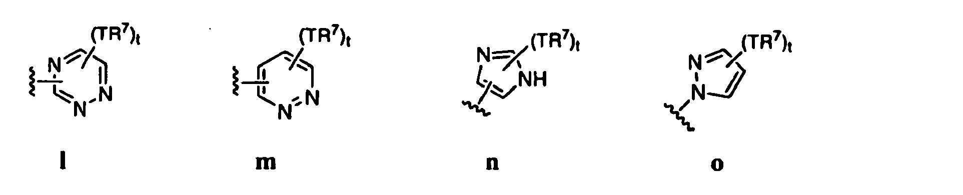 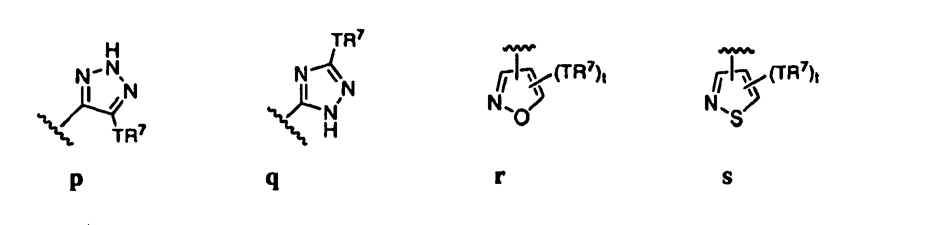 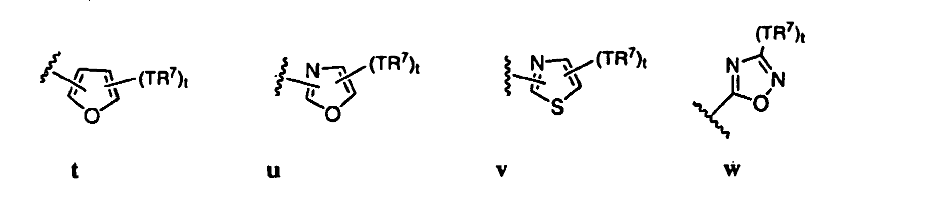 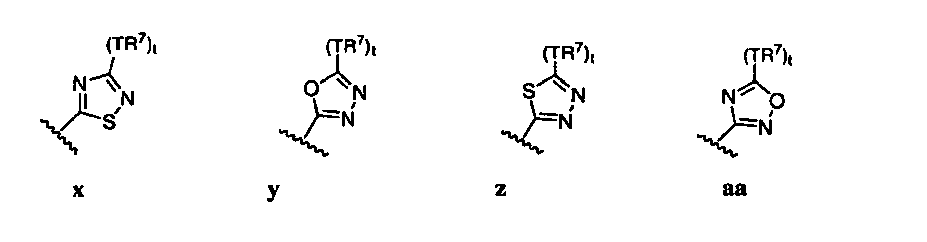 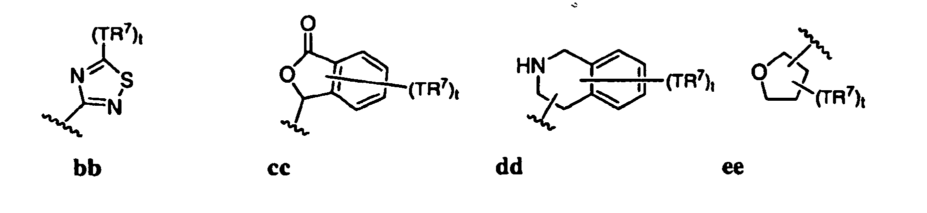 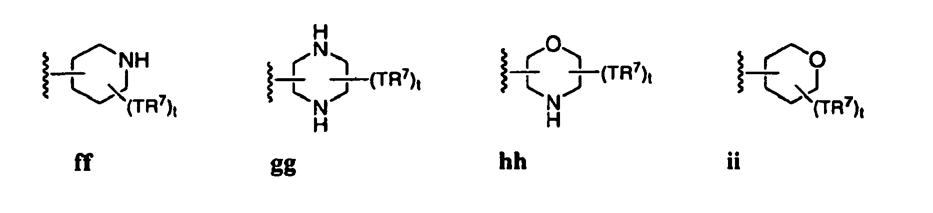 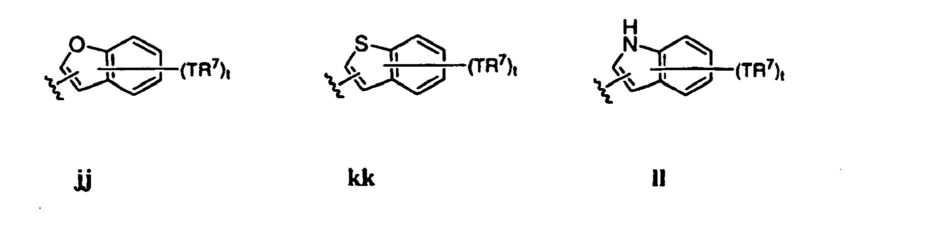 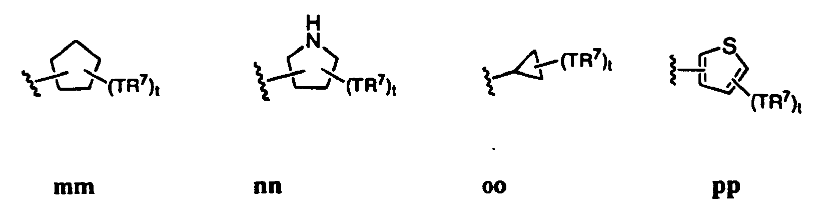 wherein t is 0, 1, 2, 3, 4 or 5, and wherein any Ar² is bonded to Q³ through any substitutable nitrogen or carbon atom, and wherein one or more hydrogen atoms on any substitutable nitrogen or carbon atom is substituted with one or more independent occurrences of TR⁷; for example wherein Ar² is a, **b, e, g, h, i, j, k, n, r, cc, dd, ff, jj, ll,** or **pp;**
wherein:
(a) T is a bond or is an optionally substituted C₁₋₆ alkylidene chain wherein one or two methylene units are optionally and independently replaced by -O-, -NR-, -S-, -SO₂-,-COO-, -CO-, -OSO₂-, -NRSO₂, -CONR-, or -SO₂NR-, and R⁷ is R' or halogen; or
(b) each occurrence of TR⁷ is independently -C₁₋₃alkyl, -OR', -SR', -CF₃, -OCF₃, -SCF₃, -F, -Cl, I, -Br, -COOR', -COR', -O(CH₂)₄N(R)(R'), -O(CH₂)₃N(R)(R'), -O(CH₂)₂N(R)(R'), -O(CH₂)N(R)(R'), -O(CH₂)₄CON(R)(R'), -O(CH₂)₃CON(R)(R'), -O(CH₂)₂CON(R)(R'), -O(CH₂)CON(R)(R'), -C(O)N(R)(R'), -(CH₂)₄OR', -(CH₂)₃OR', -(CH₂)₂OR', -CH₂OR', optionally substituted phenyl or benzyl, -N(R)(R'), -(CH₂)₄N(R)(R'), -(CH₂)₃N(R)(R'), -(CH₂)₂N(R)(R'), -(CH₂)N(R)(R'), or SO₂N(R)(R'), NRSO₂R', CON(R)(R'), or -OSO₂R'; or
(3) R⁵ is hydrogen, (CH₂)₃OR', (CH₂)₂OR', (CH₂)OR', (CH₂)₃N(R')₂, (CH₂)₂N(R')₂, (CH₂)N(R')₂, or C₁₋₄aliphatic.

4. The compound of claim 1, wherein each occurrence of R¹ is independently hydrogen, halogen, optionally substituted C₁-C₄aliphatic, OR, SR, or N(R)₂; preferably wherein each occurrence of R¹ is independently hydrogen, halogen, -CH₃, -CH₂CH₃, -OH, -OCH₃, -SCH₃, -NH₂, -N(CH₃)₂, -N(CH₂CH₃)₂, NH(CH₂)₂NHCH₃, NH(cyclopropyl), NH(CH₂)cyclopropyl, or NH(CH₂)₂N(CH₃)₂.

5. The compound of claim 1, wherein each occurrence of R^{Z} is independently hydrogen, halogen, C₁-C₄aliphatic, OH, OR', or N(R)(R');
preferably wherein each occurrence of R^{Z} is independently hydrogen, halogen, Me, OH, OMe, NH₂, or N(Me)₂.

6. The compound of claim 1, wherein R⁴ groups are each independently hydrogen, C₁₋₆aliphatic, CN, COR, C(=O)OR, C(=O)N(R)₂, or halogen.

7. The compound of claim 1, wherein 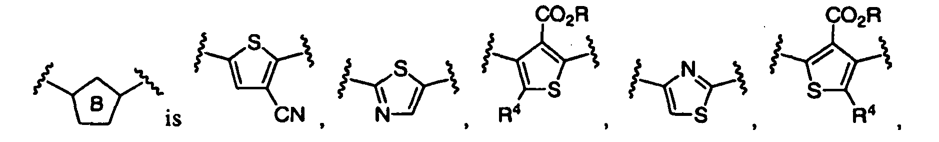 or

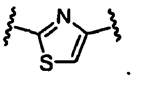

8. The compound of claim 1, wherein said compound has one of formulas **XIV, XV, XVI, XVII, XVIII,** or **XIX:**

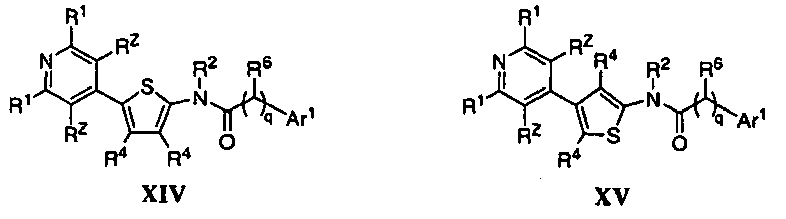

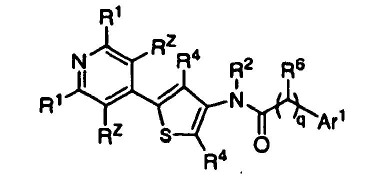

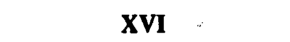

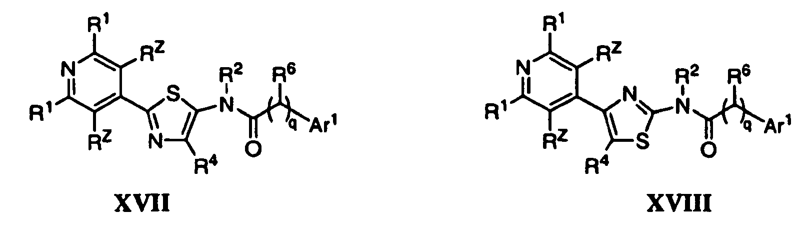

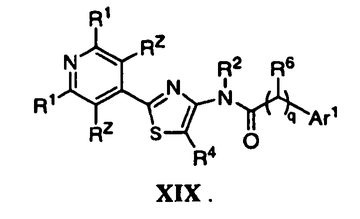

9. The compound of claim 8, wherein compound variables are selected from one of more of the following groups:
a) each occurrence of R¹ is independently hydrogen, halogen, optionally substituted C₁-C₄aliphatic, OR, SR, or N(R)₂;
b) each occurrence of R¹ is independently hydrogen, halogen, -CH₃, -CH₂CH₃, -OH, -OCH₃, -SCH₃, -NH₂, -N(CH₃)₂, -N(CH₂CH₃)₂, NH(CH₂)₂NHCH₃, NH(cyclopropyl), NH(CH₂)cyclopropyl, or NH(CH₂)₂N(CH₃)₂;
c) each occurrence of R^{Z} is independently hydrogen, halogen, optionally substituted C₁-C₄aliphatic, OH, O(R'), or N(R)(R');
d) each occurrence of R^{Z} is independently hydrogen, halogen, Me, OH, OMe, NH₂, or N(Me)₂;
e) R² is hydrogen, or is UₙR', where n is 1, and U is-CH₂-, -CH₂CH₂-, -CH₂CH₂CH₂-, -CH₂CH₂CH₂CH₂-, -CH₂O-, -CH₂S-, -CH₂NR-, -CH₂CH₂O-, -CH₂CH₂S-, -CH₂CH₂NR-, -CH₂CH₂CH₂O-, -CH₂CH₂CH₂S-, -CH₂CH₂CH₂NR-, -CH₂CH₂CH₂CH₂O-, -CH₂CH₂CH₂CH₂S-, -CH₂CH₂CH₂CH₂NR-, -CH₂CH₂OCH₂CH₂-, -(CH₂)₄NHCH₂-, -(CH₂)₃NHCH₂CH₂-, or -CH₂CH₂NHCH₂CH₂-, and R' groups are hydrogen, C₁-C₄alkyl, optionally substituted tetrahydropyranyl, pyrrolidinyl, piperidinyl, piperazinyl, morpholinyl, thiomorpholinyl, pyridinyl, phenyl, or cyclohexyl, or R and R', taken together with the nitrogen atom to which they are bound, form an optionally substituted 5- or 6-membered heterocyclyl ring;
f) each occurrence of R⁴ is independently hydrogen, C₁₋₆aliphatic, CN, COR, COOR, CON(R)₂, or halogen;
g) q is 1, 2, or 3;
h) R⁶ is R', -N(R)(R'), -(CH₂)₁₋₄N(R)(R'), -OR', -(CH₂)₁₋₄OR', -NR(CH₂)₁₋₄N(R)(R'), -NR(CH₂)₁₋₄SO₂R', -NR(CH₂)₁₋₄COOR', or -NR(CH₂)₁₋₄COR', or two occurrences of R⁶, taken together with the atoms to which they are bound, form an optionally substituted 3-6-membered saturated, partially unsaturated, or fully unsaturated ring;
i) R⁶ is CH₂OH, CH₂CH₂OH, OH, OMe, OEt, NH₂, NH(Me), NH(Et), N(Me)(Me), CH₂NH₂, CH₂CH₂NH₂, NHCO₂*t*-butyl, phenyl, cyclopentyl, methyl, ethyl, isopropyl, cyclopropyl, NH(CH₂)₃NH₂, NH(CH₂)₂NH₂, NH(CH₂)₂NHEt, NHCH₂pyridyl, NHSO₂phenyl, NHC(O)CH₂C(O)O*t*-butyl, NHC(O)CH₂NH₃, and NHCH₂-imidazol-4-yl;
j) Ar¹ is ring **a, b, e, g, h, i, j, k, r, cc, dd, ff, jj, ll,** or **pp**, wherein t is 0, 1, 2, or 3, and T is a bond or is an optionally substituted C₁₋₆ alkylidene chain wherein one or two methylene units are optionally and independently replaced by -0-, -NR-, -S-, -SO₂-,-COO-, -CO-, -OSO₂-, -NRSO₂, -CONR-, or -SO₂NR-, and R⁷ is R' or halogen; or
k) Ar¹ is ring **a, b, e, g, h, i, j, k, r, cc, dd, ff, jj, ll,** or **pp,** wherein t is 0, 1, 2, or 3, and each occurrence of TR⁷ is independently -C₁₋₃alkyl, -OR', -SR', -CF₃, -OCF₃, -SCF₃, -F, -Cl, I, -Br, -COOR', -COR', -O(CH₂)₄N(R)(R'), -O(CH₂)₃N(R)(R'), -O(CH₂)₂N(R)(R'), -O(CH₂)N(R)(R'), -O(CH₂)₄CON(R)(R'), -O(CH₂)₃CON(R)(R'), -O(CH₂)₂CON(R)(R'), -O(CH₂)CON(R)(R'). -C(O)N(R)(R'), -(CH₂)₄OR', -(CH₂)₃OR', -(CH₂)₂OR', -CH₂OR', optionally substituted phenyl or benzyl, -N(R)(R'), -(CH₂)₄N(R)(R'), -(CH₂)₃N(R)(R'). -(CH₂)₂N(R)(R'), -(CH₂)N(R)(R'), or SO₂N(R)(R'), NRSO₂R', CON(R)(R'), or -OSO₂R'.

10. The compound of claim 8, wherein q is 1, and Ar¹ is optionally substituted phenyl and a compound of general formula **XIV-A** through **XIX-A** is provided: 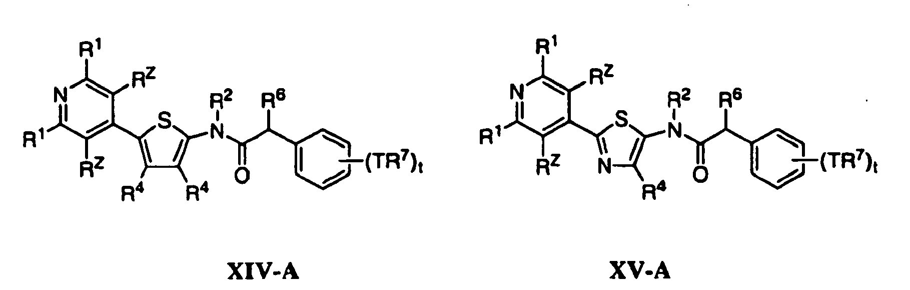 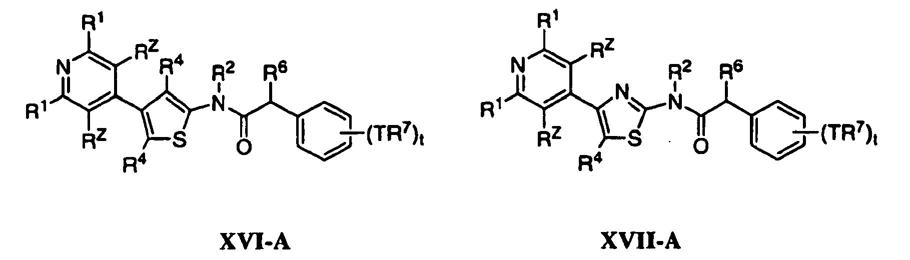 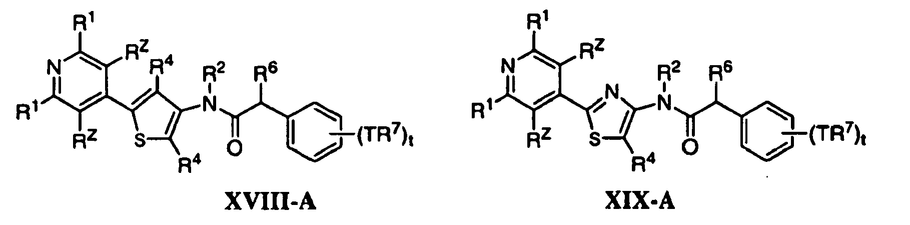 wherein:
each occurrence of R¹ is hydrogen;
each occurrence of R^{Z} is hydrogen;
R² is hydrogen, or is UₙR', where n is 1, and U is -CH₂-, -CH₂CH₂-, -CH₂CH₂CH₂-, -CH₂CH₂CH₂CH₂-, -CH₂O-, -CH₂S-, -CH₂NR-, -CH₂CH₂O-, -CH₂CH₂S-, -CH₂CH₂NR-, -CH₂CH₂CH₂O-, -CH₂CH₂CH₂S-, -CH₂CH₂CH₂NR-, -CH₂CH₂CH₂CH₂O-, -CH₂CH₂CH₂CH₂S-, -CH₂CH₂CH₂CH₂NR-, -CH₂CH₂OCH₂CH₂-, -(CH₂)₄NHCH₂-, -(CH₂)₃NHCH₂CH₂-, or -CH₂CH₂NHCH₂CH₂-, and R' groups are hydrogen, C₁-C₄alkyl, optionally substituted tetrahydropyranyl, pyrrolidinyl, piperidinyl, piperazinyl, morpholinyl, thiomorpholinyl, pyridinyl, phenyl, or cyclohexyl, or R and R', taken together with the nitrogen atom to which they are bound, form an optionally substituted 5- or 6-membered heterocyclyl ring;
each occurrence of R⁴ is independently hydrogen, C₁₋₆aliphatic, CN, COR, COOR, CON(R)₂, or halogen;
R⁶ is R', -N(R)(R'), -(CH₂)₁₋₄N(R)(R'), -OR', -(CH₂)₁₋₄OR', -NR(CH₂)₁₋₄N(R)(R'), -NR(CH₂)₁₋₄SO₂R'. -NR(CH₂)₁₋₄COOR', or -NR(CH₂)₁₋₄COR'; and
t is 0, 1, 2, or 3, and each occurrence of TR⁷ is independently -C₁₋₃alkyl, -OR', -SR', -CF₃, -OCF₃, -SCF₃, -F, -Cl, I, -Br, -COOR', -COR', -O(CH₂)₄N(R)(R'), -O(CH₂)₃N(R)(R'), -O(CH₂)₂N(R)(R'), -O(CH₂)N(R)(R'), -O(CH₂)₄CON(R)(R'). -O(CH₂)₃CON(R)(R'), -O(CH₂)₂CON(R)(R'), -O(CH₂)CON(R)(R'), -C(O)N(R)(R'), -(CH₂)₄OR', -(CH₂)₃OR', -(CH₂)₂OR', -CH₂OR', optionally substituted phenyl or benzyl, -N(R)(R'), -(CH₂)₄N(R)(R'), -(CH₂)₃N(R)(R'), -(CH₂)₂N(R)(R'), -(CH₂)N(R)(R'), or SO₂N(R)(R'), NRSO₂R', CON(R)(R'), or -OSO₂R'.

11. The compound of claim 1, wherein
(1) R² and Q¹-R³, taken together with the atoms to which they are bound form a 5-membered cyclic group, and compounds have the general formula **XX** through **XXXI:** 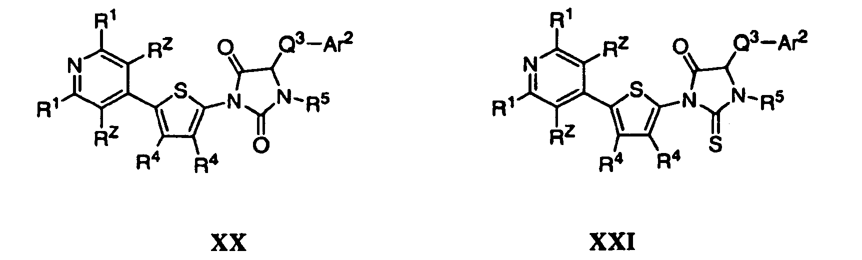 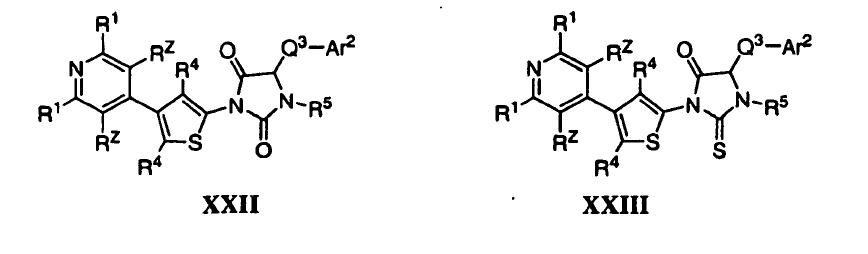 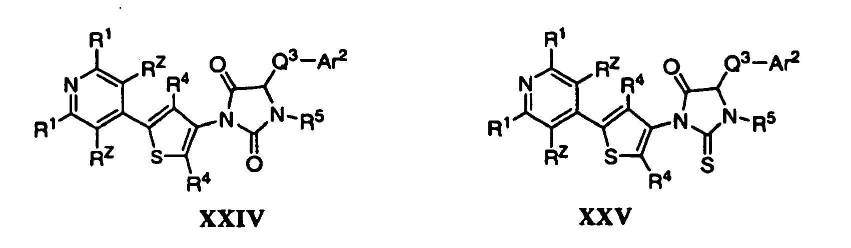 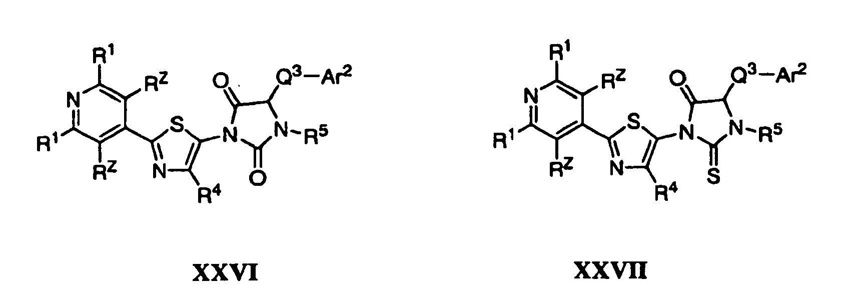 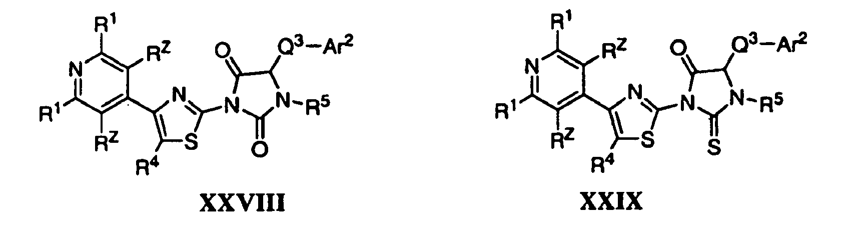 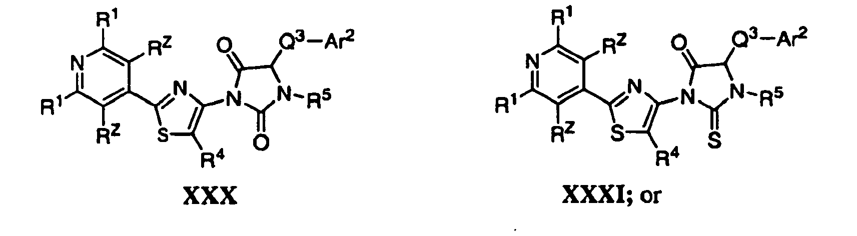
(2) R² and Q¹-R³, taken together with the atoms to which they are bound form a 6-membered cyclic group, and compounds have the general formula **XXXII** through **XXXVII:** 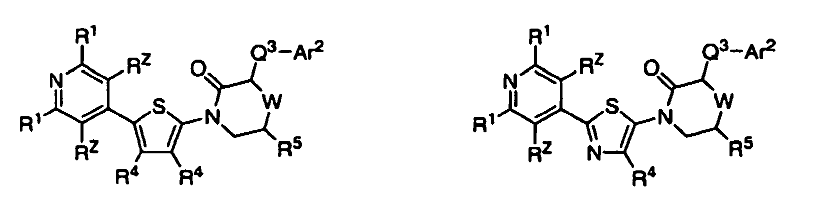 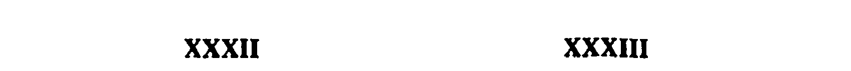 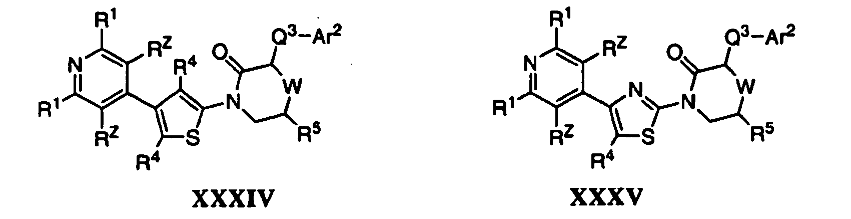 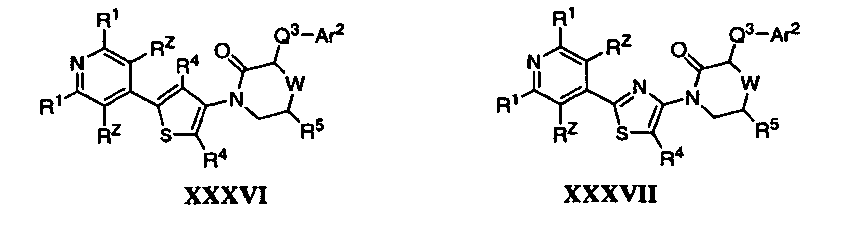
wherein W is O, NR⁵, or CHR⁵.

12. The compound of claim 11 wherein compound variables are selected from one of more of the following groups:
a) each occurrence of R¹ is independently hydrogen, halogen, optionally substituted C₁-C₄aliphatic, OR, SR, or N(R)₂;
b) each occurrence of R^{Z} is independently hydrogen, halogen, optionally substituted C₁-C₄aliphatic, OH, OR' or N(R)(R');
c) each occurrence of R⁴ is independently hydrogen, C₁₋₆aliphatic, CN, COR, COOR, CON(R)₂, or halogen;
d) R⁵ is hydrogen, (CH₂)₃OR', (CH₂)₂OR', (CH₂)OR', (CH₂)₃N(R')₂, (CH₂)₂N(R')₂, (CH₂)N(R')₂, or C₁₋₄aliphatic;
e) Q³ is a direct bond, or is -(CHR⁶)_{q}-, -(CHR⁶)_{q}O-, -(CHR⁶)_{q}S-, -(CHR⁶)_{q}S(O)₂-, -(CHR⁶)_{q}S(O)- , -(CHR⁶)_{q}NR-, or -(CHR⁶)_{q}C(O)-, wherein q is 0, 1, 2, or 3; and
f) Ar² is ring **a, b, e, g, h, i, j, k, n, r, cc, dd, ff, jj, ll,** or **pp**, where in t is 0, 1, 2, or 3, and T is a bond or is an optionally substituted C₁₋₆ alkylidene chain wherein one or two methylene units are optionally and independently replaced by -0-, -NR-, -S-, -SO₂-,-COO-, -CO-, -OSO₂-, -NRSO₂, -CONR-, or -SO₂NR-, and R⁷ is R' or halogen;

13. The compound of claim 11 , wherein compound variables are selected from one of more of the following groups:
a) each occurrence of R¹ is independently hydrogen, halogen, -CH₃, -CH₂CH₃, -OH, -OCH₃, -SCH₃, -NH₂, -N(CH₃)₂, -N(CH₂CH₃)₂, NH(CH₂)₂NHCH₃, NH(cyclopropyl), NH(CH₂)cyclopropyl, or NH(CH₂)₂N(CH₃)₂;
b) each occurrence of R^{Z} is independently hydrogen, halogen, Me, OH, OMe, NH₂, or N(Me)₂;
c) each occurrence of R⁴ is independently hydrogen, C₁₋₆aliphatic, CN, COR, COOR, CON(R)₂, or halogen;
d) R⁵ is hydrogen, (CH₂)₃OR', (CH₂)₂OR', (CH₂)OR', (CH₂)₃N(R')₂, (CH₂)₂N(R')₂, (CH₂)N(R')₂, or C₁₋₄aliphatic;
e) Q³ is a direct bond, or is -(CHR⁶)_{q}-, -(CHR⁶)_{q}O-, -(CHR⁶)_{q}S-, -(CHR⁶)_{q}S(O)₂-, -(CHR⁶)_{q}S(O)-, -(CHR⁶)_{q}NR-, or -(CHR⁶)_{q}C(O)-, wherein q is 0, 1, 2, or 3; and
f) Ar² is ring **a, b, e, g, h, i, j, k, n, r, cc, dd, ff, jj, ll,** or **pp**, wherein t is 0, 1, 2, or 3, and each occurrence of TR⁷ is independently -C₁₋₃alkyl, -OR', -SR', -CF₃, -OCF₃, -SCF₃, -F, -Cl, I, -Br, -COOR', -COR', -O(CH₂)₄N(R)(R'), -O(CH₂)₃N(R)(R'). -O(CH₂)₂N(R)(R'). -O(CH₂)N(R)(R'), -O(CH₂)₄CON(R)(R'). -O(CH₂)₃CON(R)(R'), -O(CH₂)₂CON(R)(R'). -O(CH₂)CON(R)(R'), -C(O)N(R)(R'). -(CH₂)₄OR', -(CH₂)₃OR', -(CH₂)₂OR', -CH₂OR', optionally substituted phenyl or benzyl, -N(R)(R'). -(CH₂)₄N(R)(R'). -(CH₂)₃N(R)(R'). -(CH₂)₂N(R)(R'), -(CH₂)N(R)(R'), or SO₂N(R)(R'), NRSO₂R', CON(R)(R'), or -OSO₂R'.

14. The compound of claim 11 wherein Ar² is optionally substituted phenyl and compounds of general formula **XX-A,** through **XXXVII** are provided:

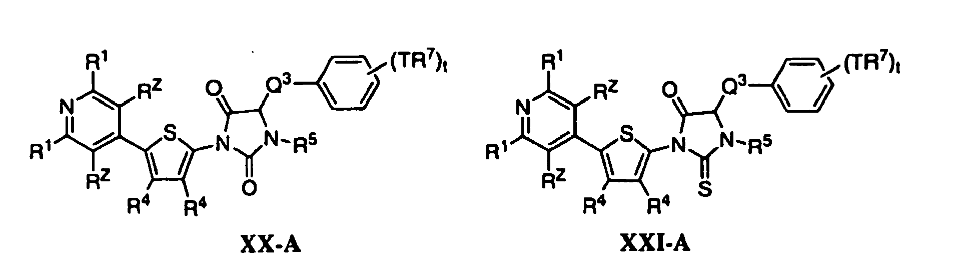

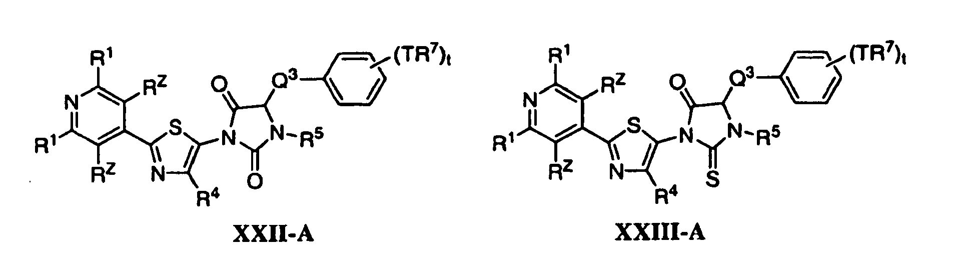

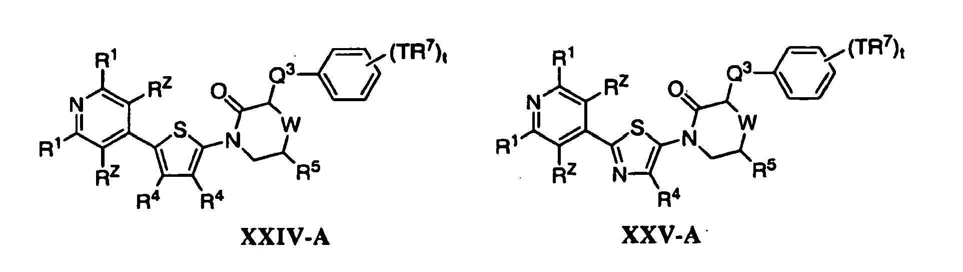

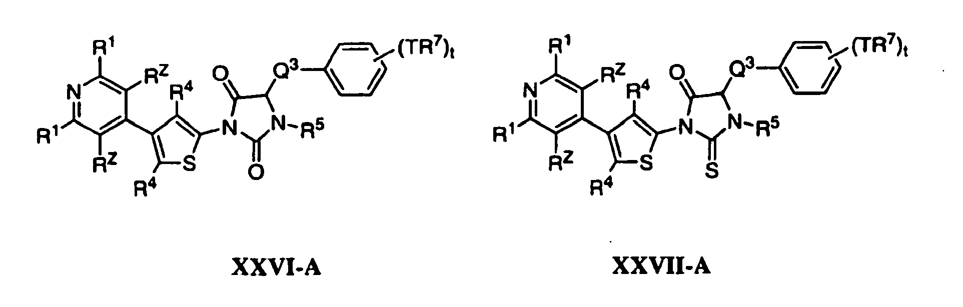

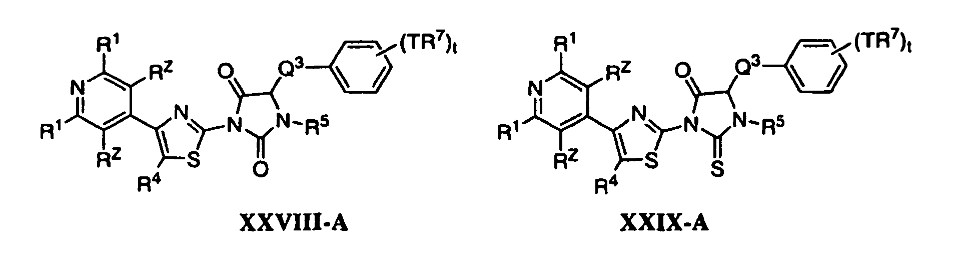

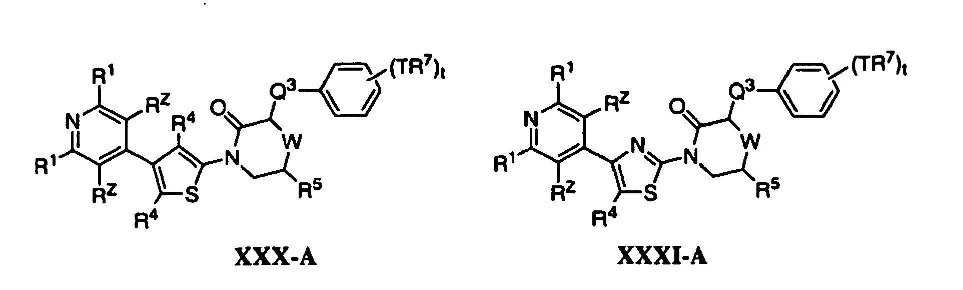

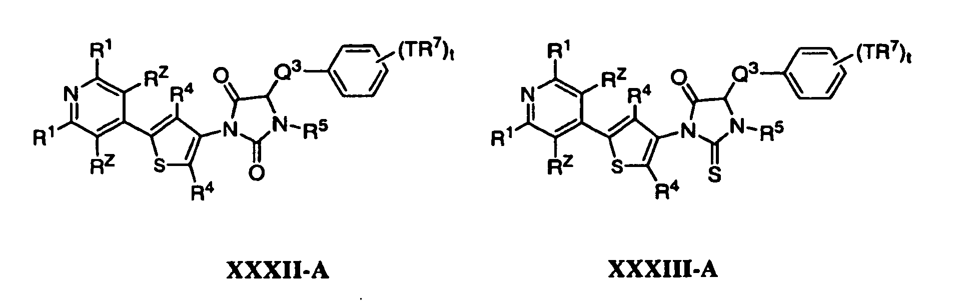

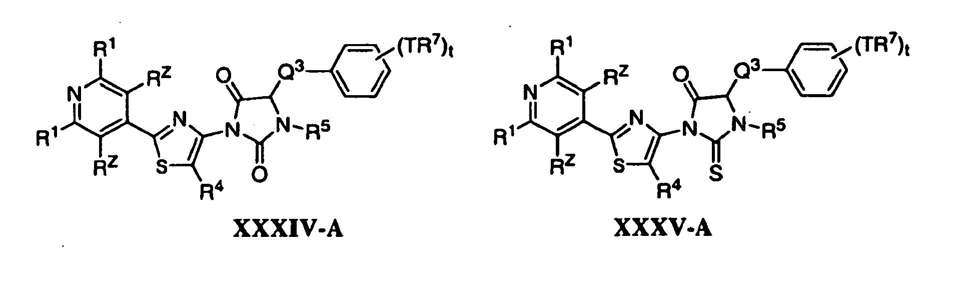

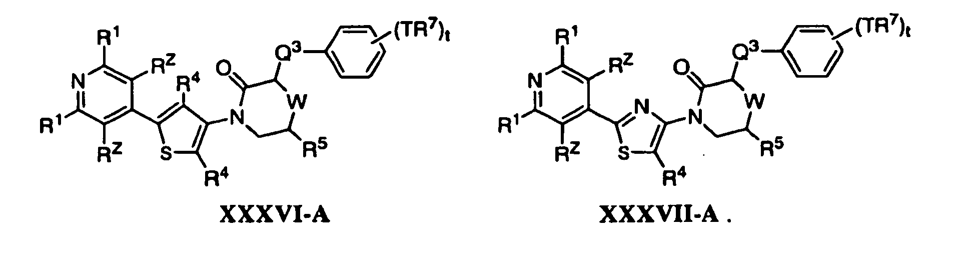

15. The compound of claim 14, wherein compound variables are selected from:
each occurrence of R¹ is hydrogen;
each occurrence of R^{Z} is hydrogen;
each occurrence of R⁴ is independently hydrogen, C₁₋₆aliphatic, CN, COR, COOR, CON(R)₂, or halogen;
R⁵ is hydrogen, (CH₂)₃OR', (CH₂)₂OR', (CH₂)OR', (CH₂)₃N(R')₂, (CH₂)₂N(R')₂, (CH₂)N(R')₂. or C₁₋₄aliphatic;
Q³ is a direct bond, or is -(CHR⁶)_{q}-, -(CHR⁶)_{q}O-, -(CHR⁶)_{q}S-, -(CHR⁶)_{q}S(O)₂-, -(CHR⁶)_{q}S(O)- , -(CHR⁶)_{q}NR-, or -(CHR⁶)_{q}C(O)-, wherein q is 0, 1, 2, or 3; and
t is 0, 1, 2, or 3, and each occurrence of TR⁷ is independently -C₁₋₃alkyl, -OR', -SR', -CF₃, -OCF₃, -SCF₃, -F, -Cl, I, -Br, -COOR', -COR', -O(CH₂)₄N(R)(R'), -O(CH₂)₃N(R)(R'), -O(CH₂)₂N(R)(R'). -O(CH₂)N(R)(R'), -O(CH₂)₄CON(R)(R'). -O(CH₂)₃CON(R)(R'), -O(CH₂)₂CON(R)(R'), -O(CH₂)CON(R)(R'), -C(O)N(R)(R'), -(CH₂)₄OR', -(CH₂)₃OR', -(CH₂)₂OR', -CH₂OR', optionally substituted phenyl or benzyl, -N(R)(R'), -(CH₂)₄N(R)(R'), -(CH₂)₃N(R)(R'), -(CH₂)₂N(R)(R'), -(CH₂)N(R)(R'), or SO₂N(R)(R'), NRSO₂R', CON(R)(R'), or -OSO₂R'.

16. The compound of claim 1, having one of the structures:

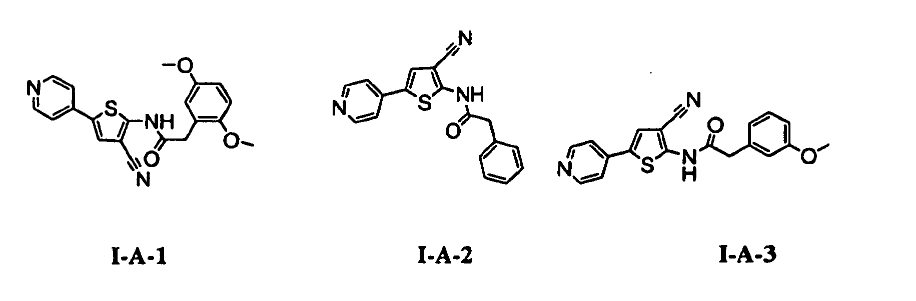

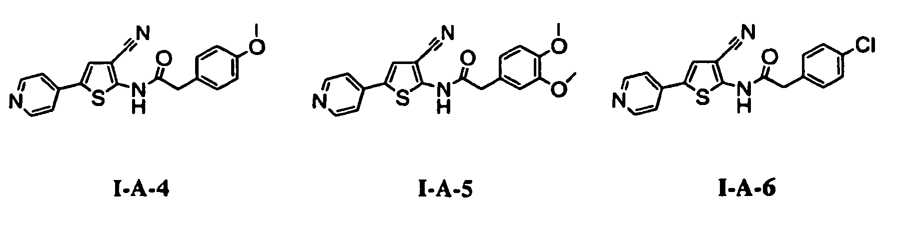

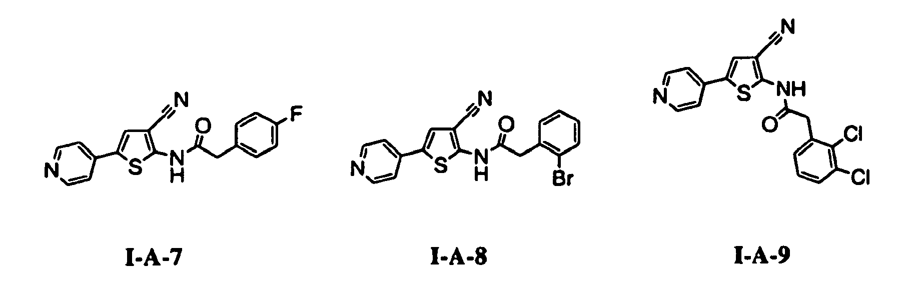

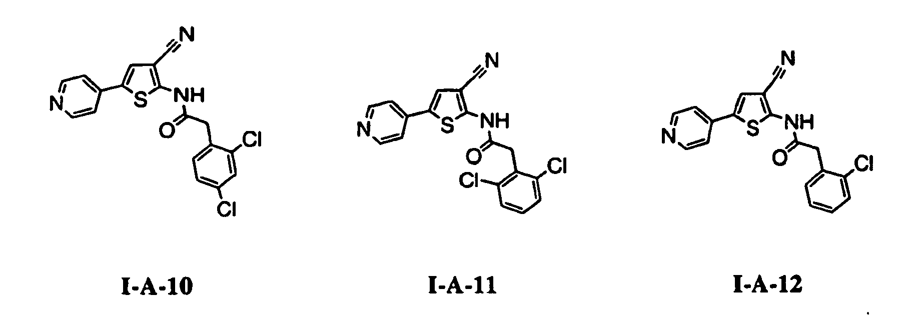

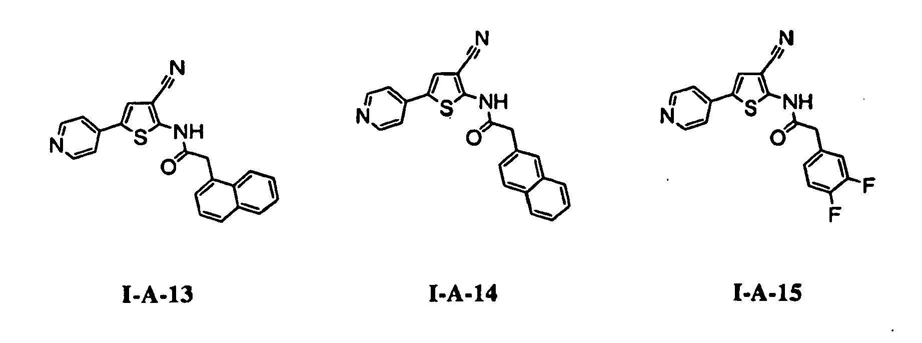

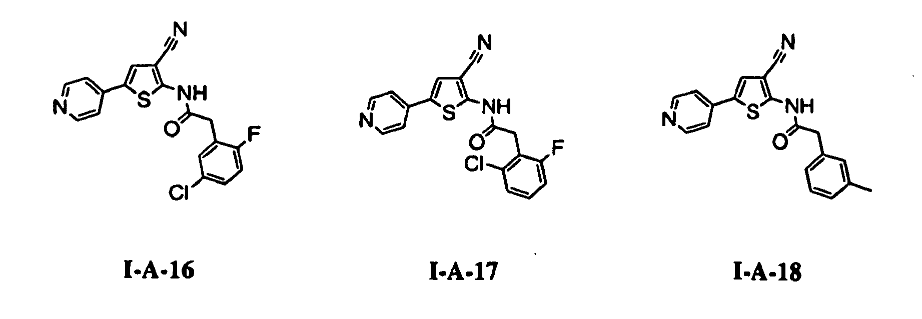

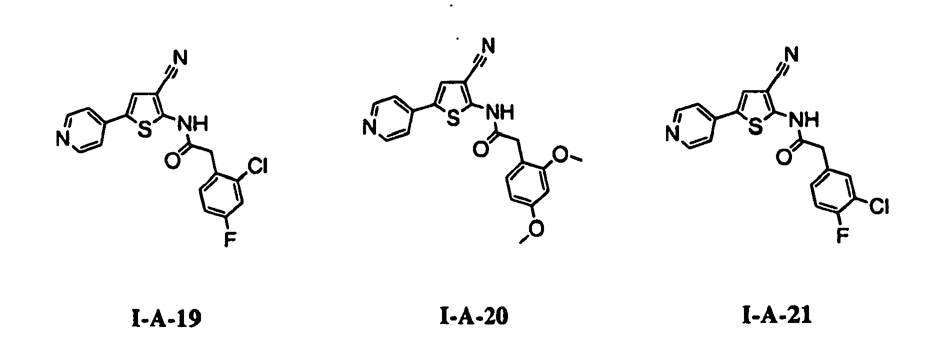

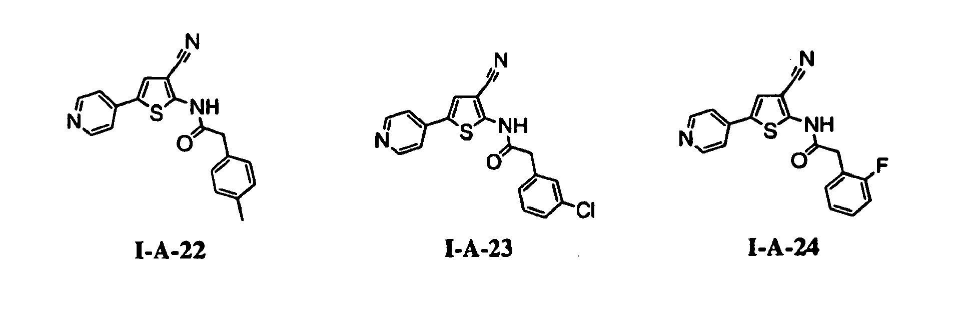

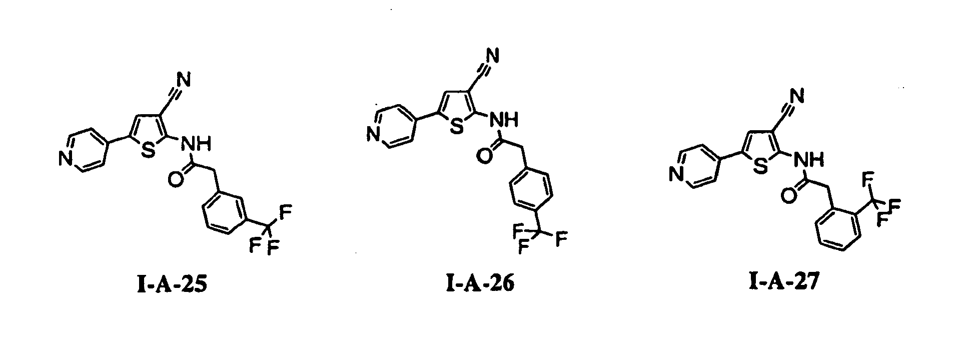

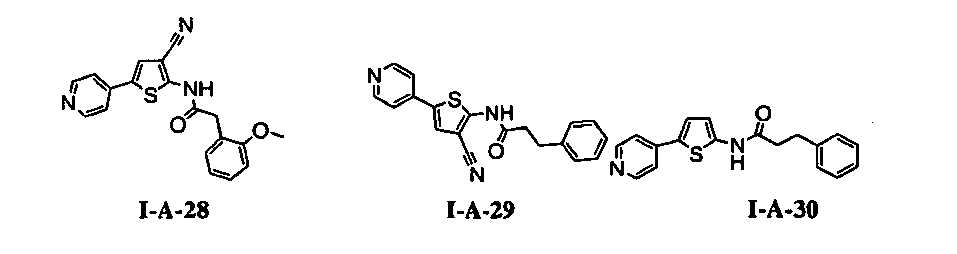

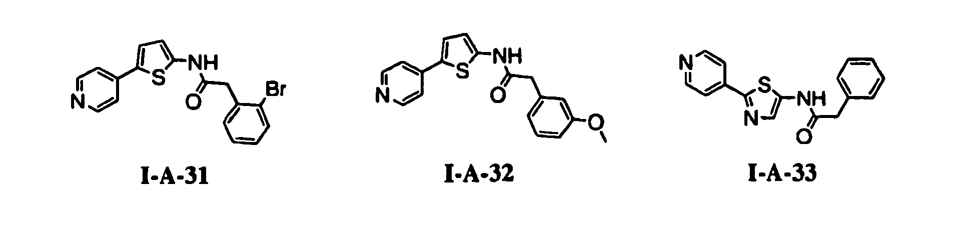

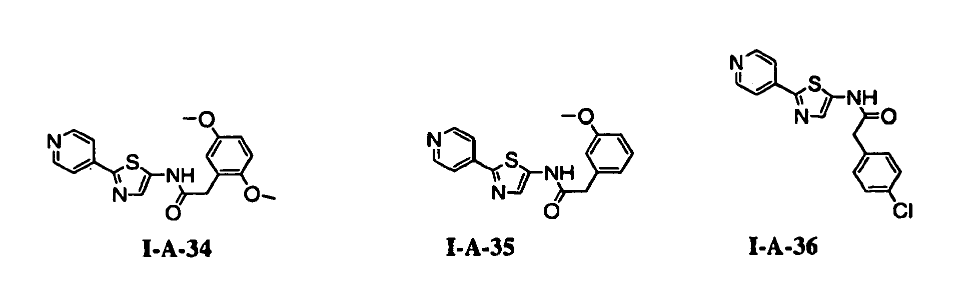

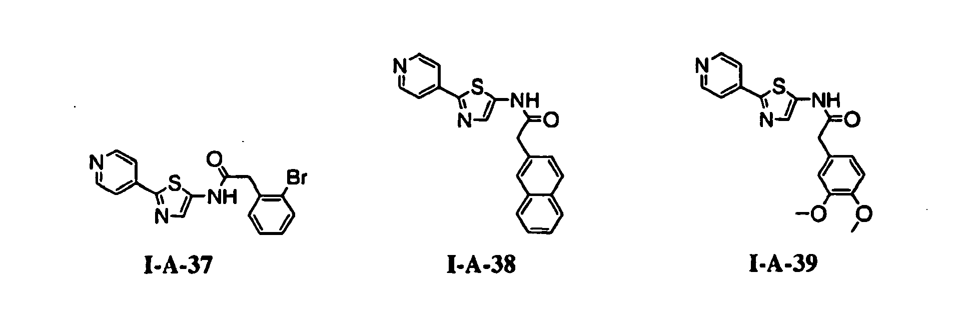

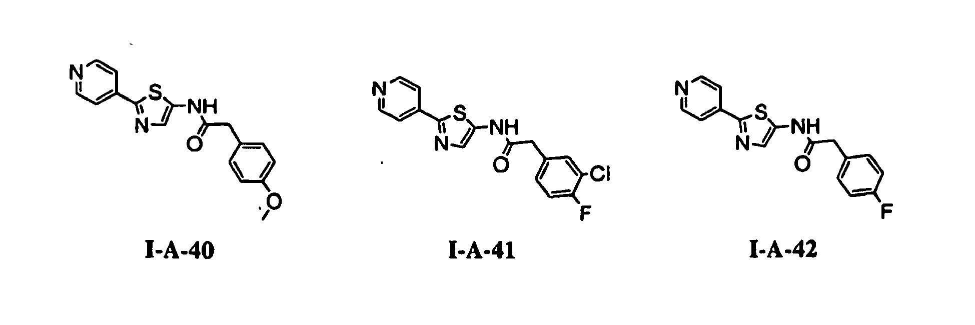

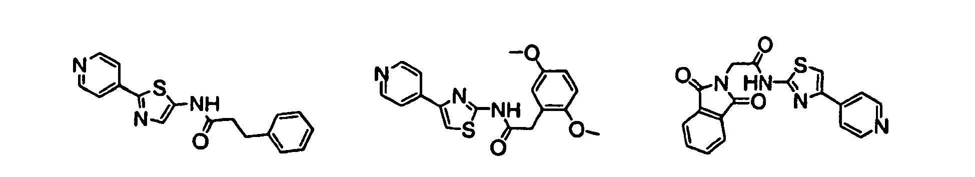

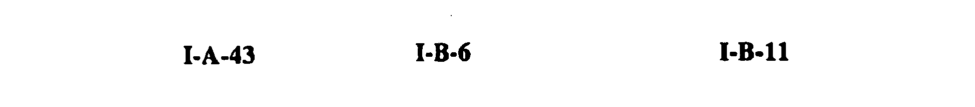

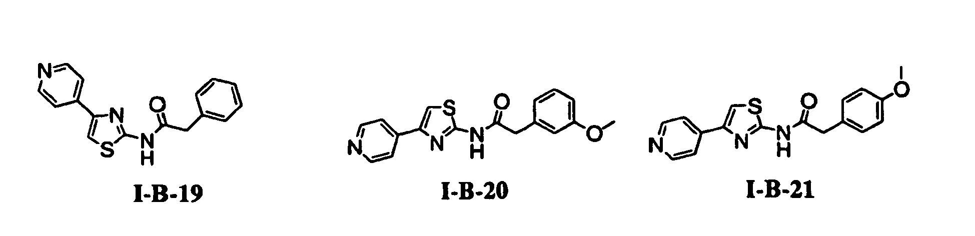

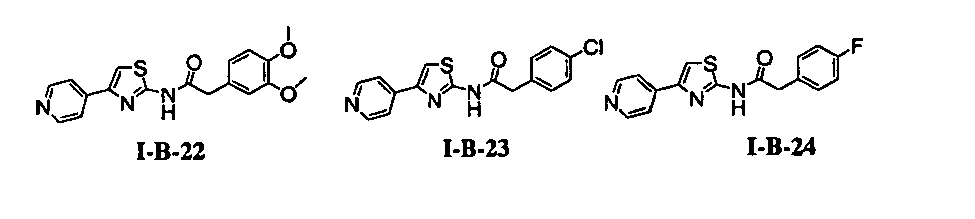

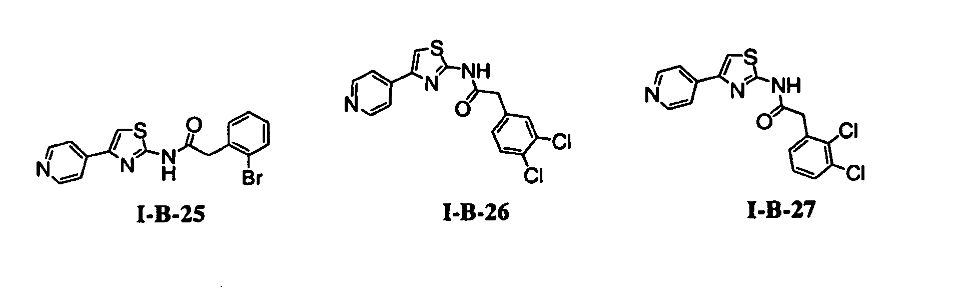

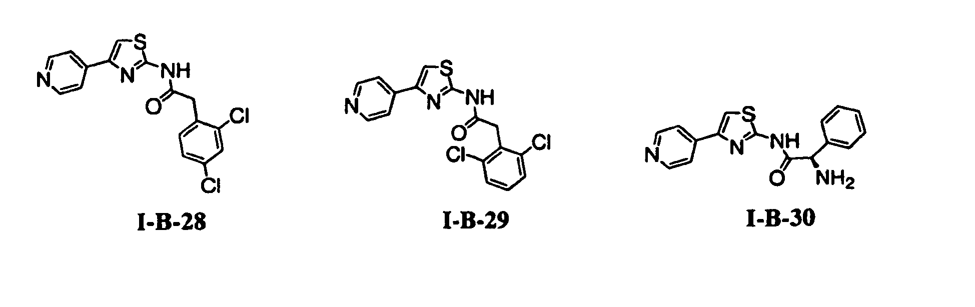

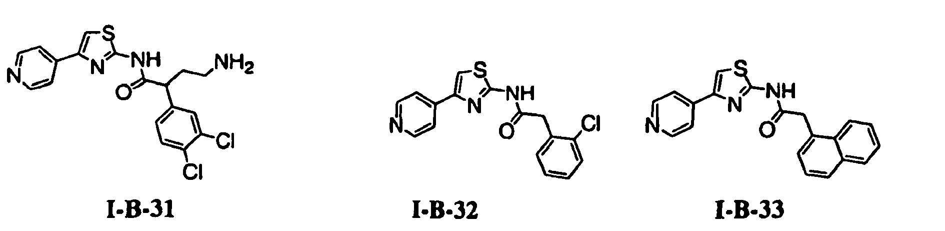

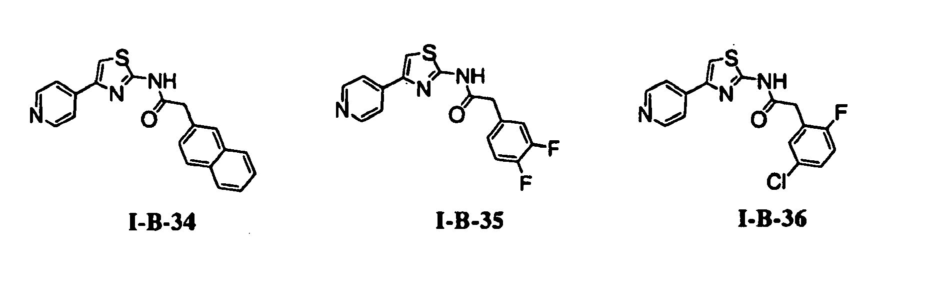

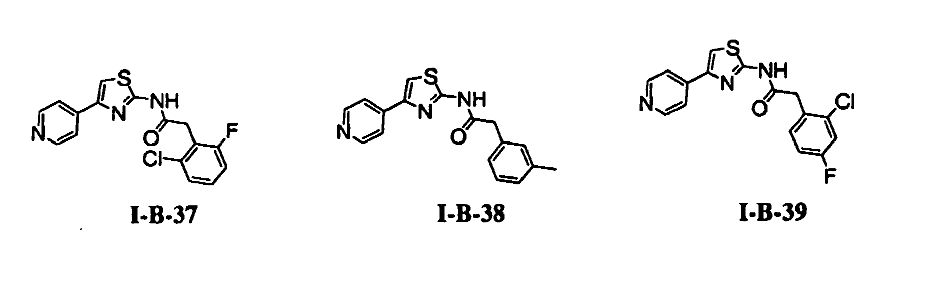

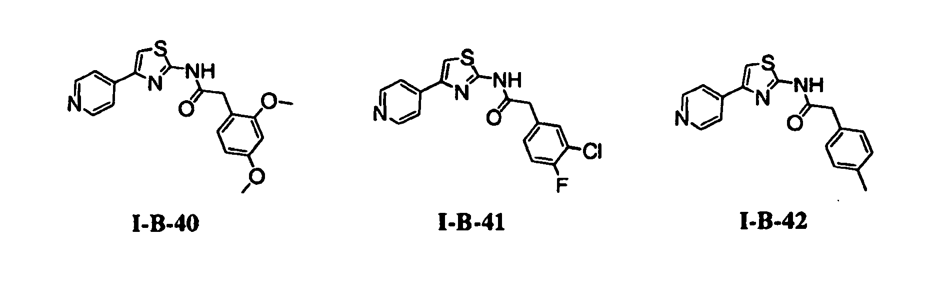

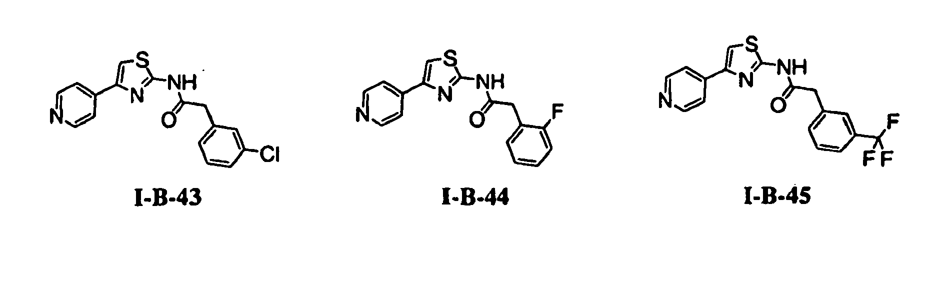

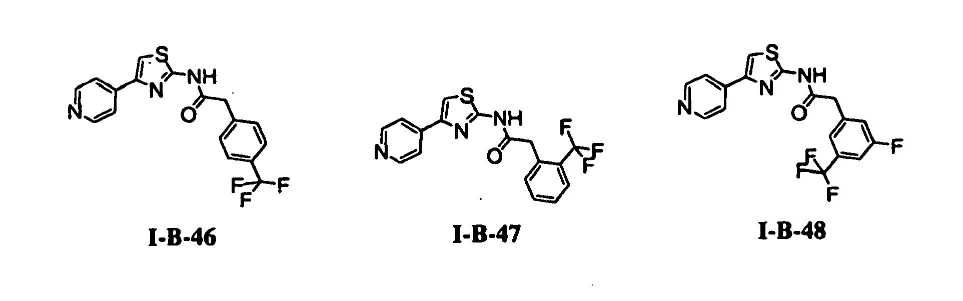

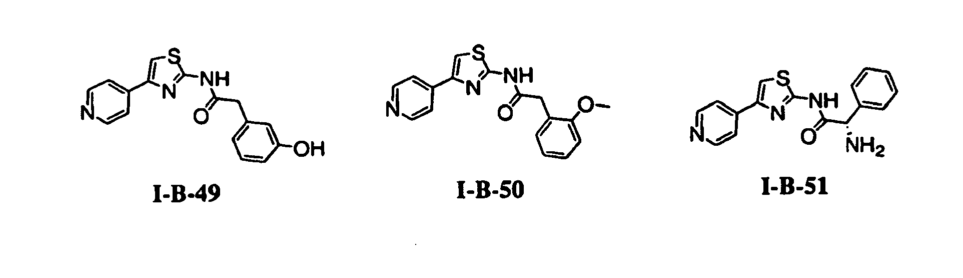

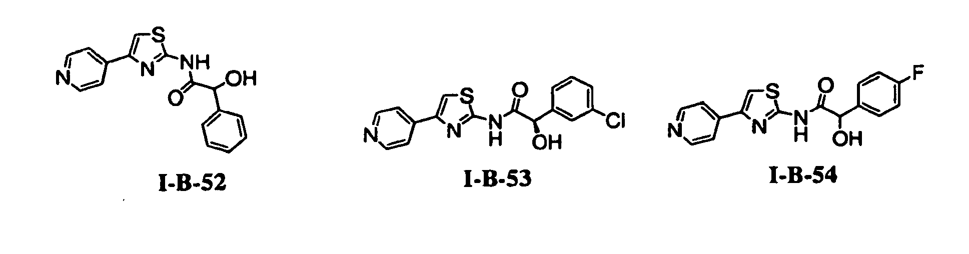

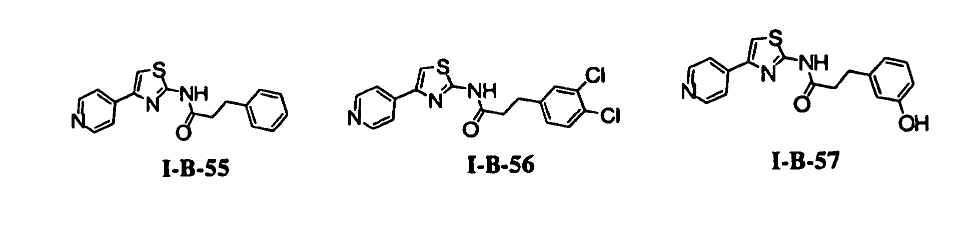

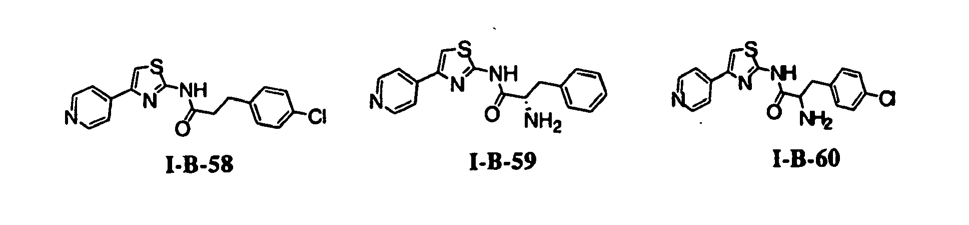

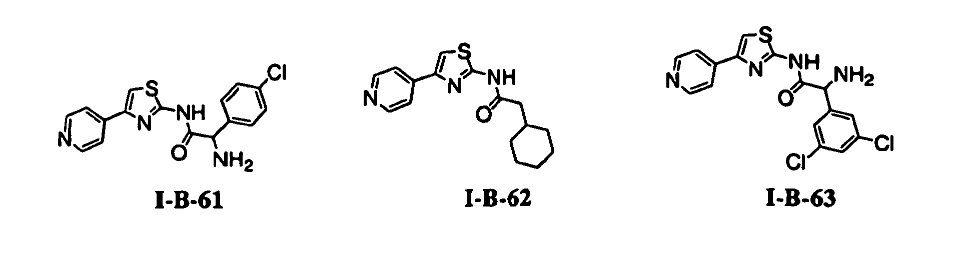

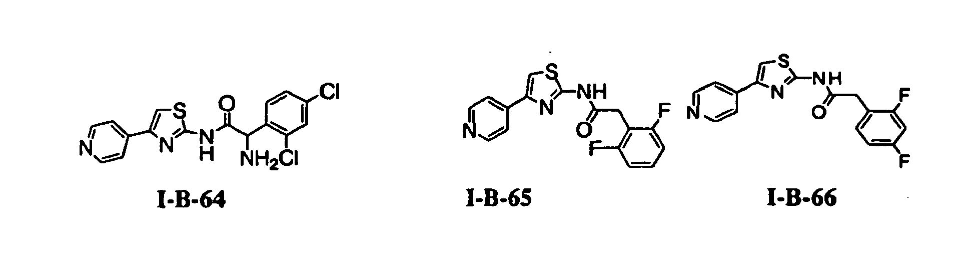

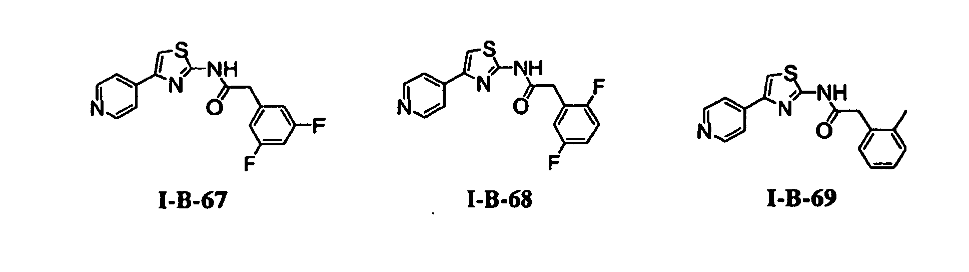

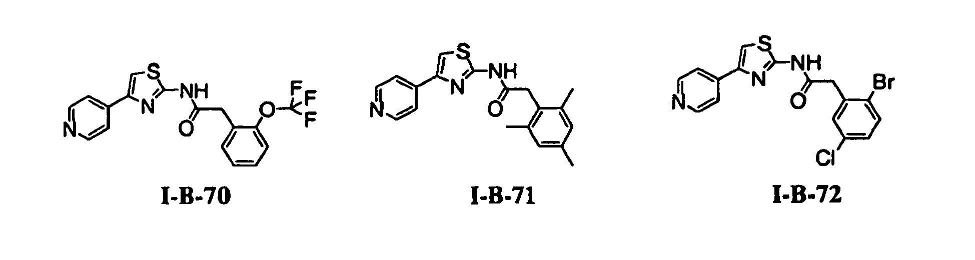

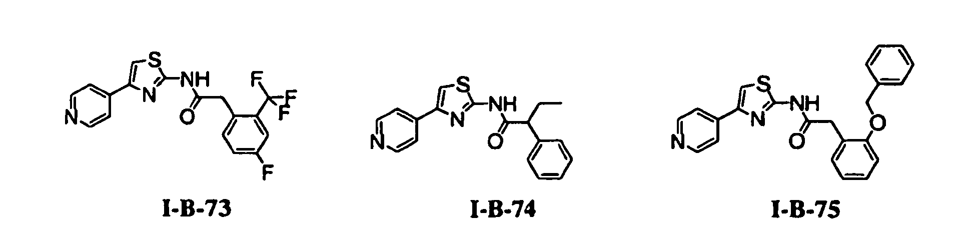

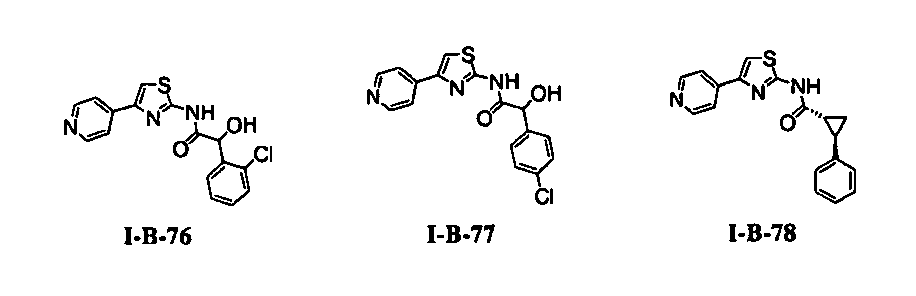

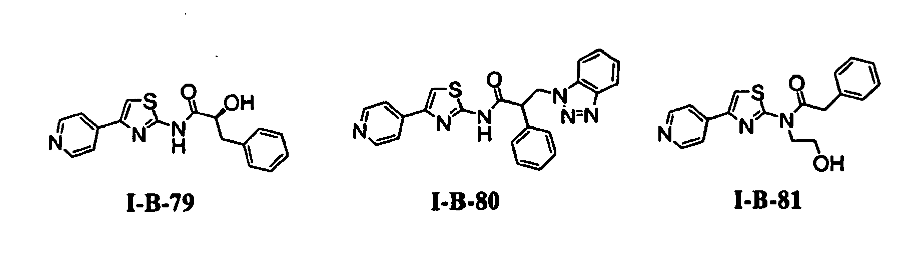

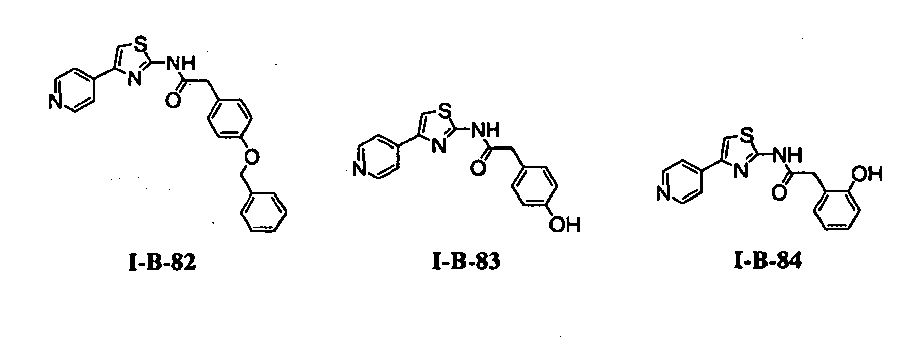

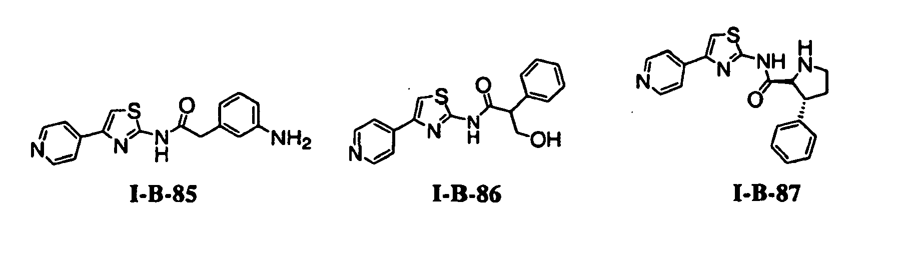

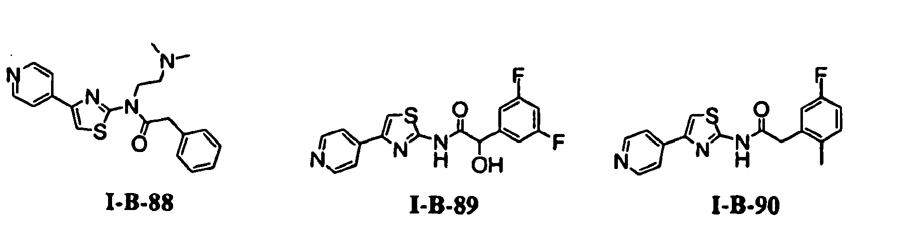

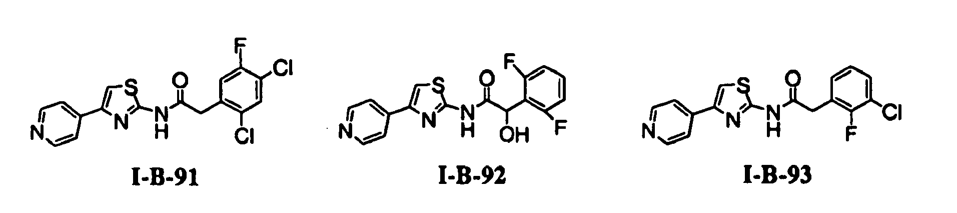

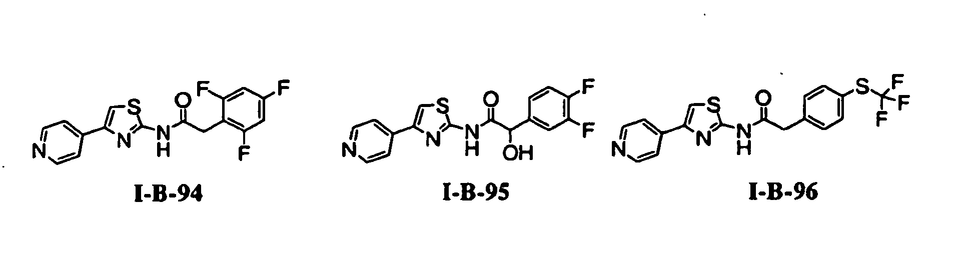

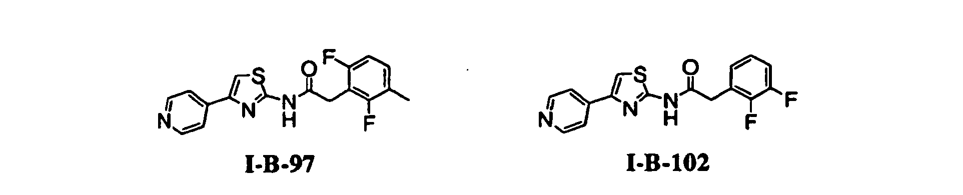

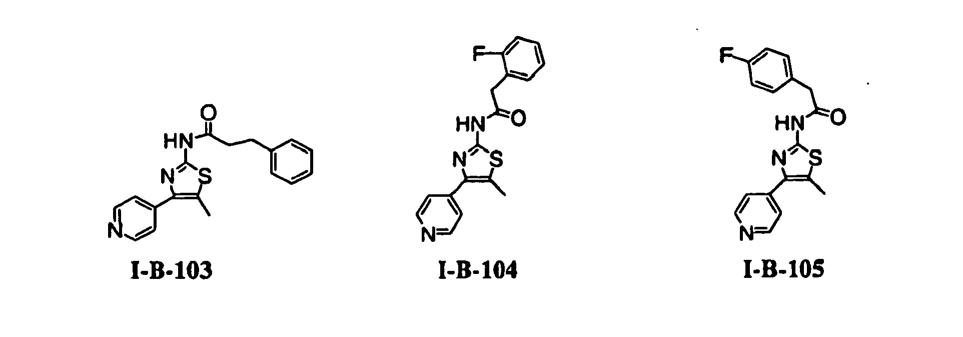

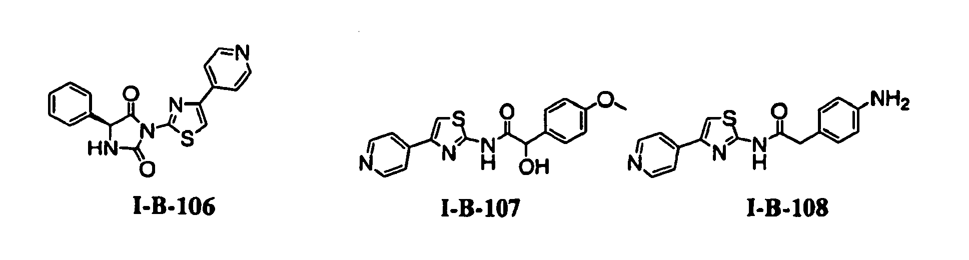

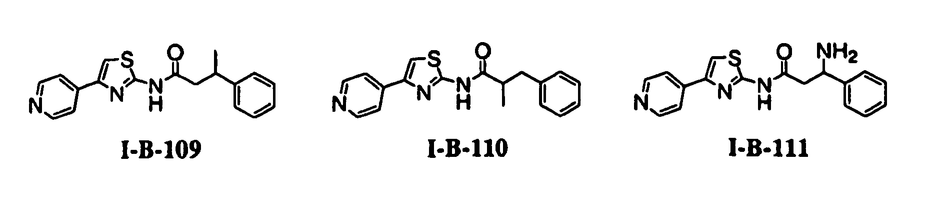

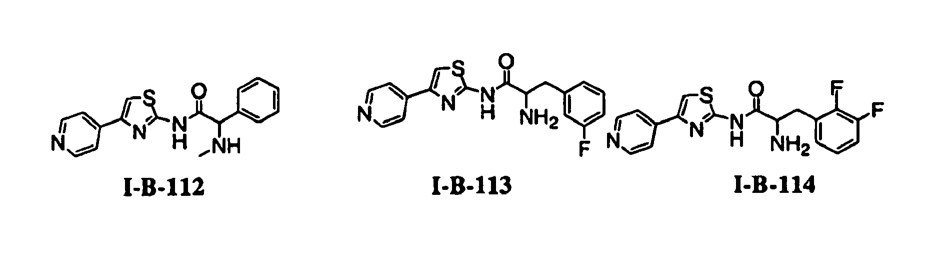

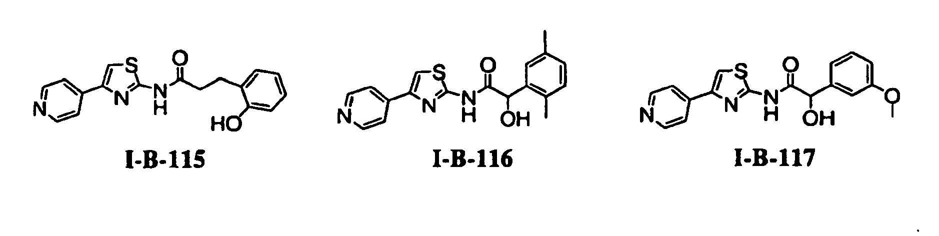

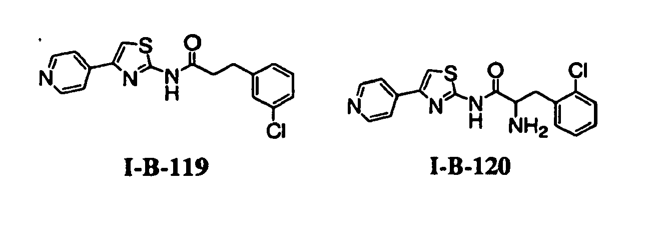

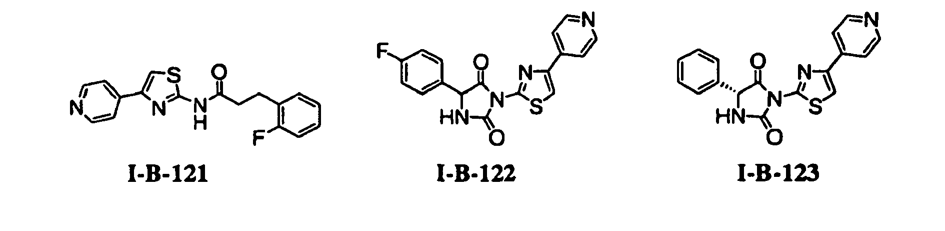

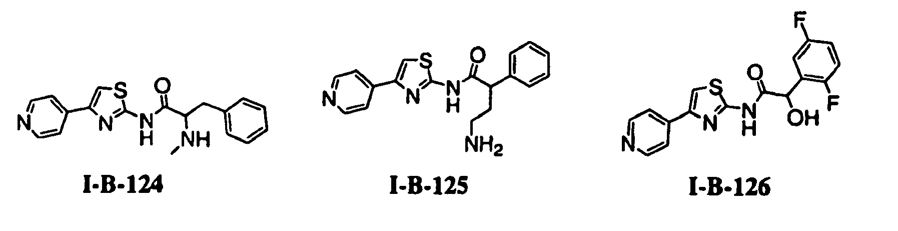

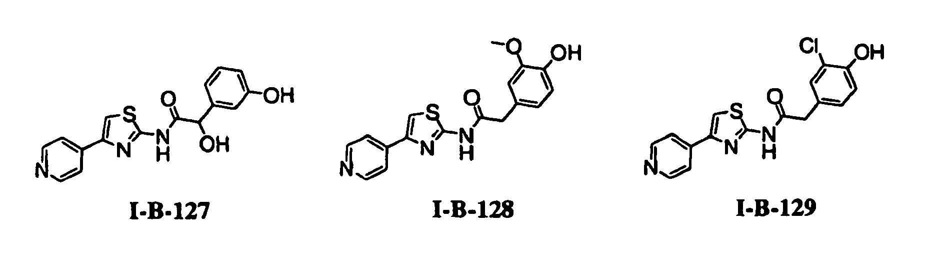

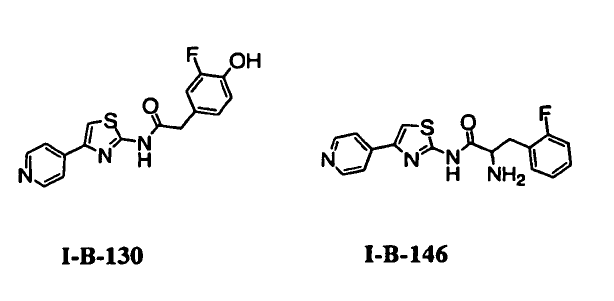

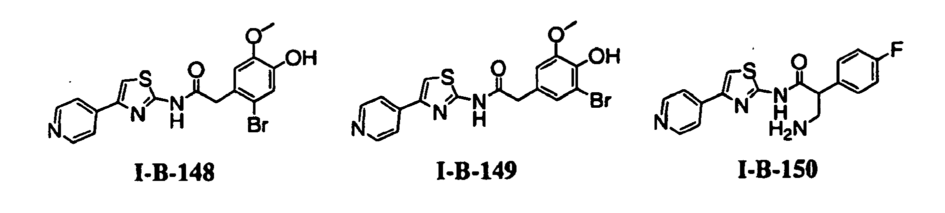

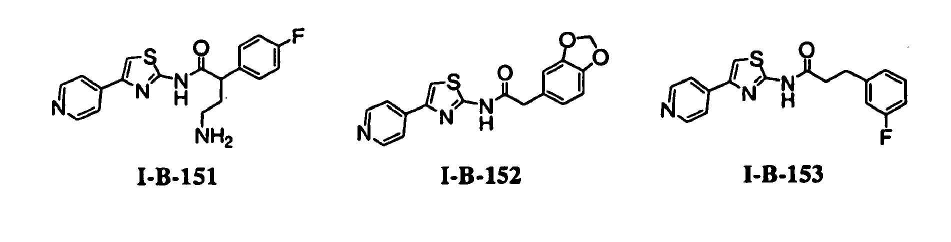

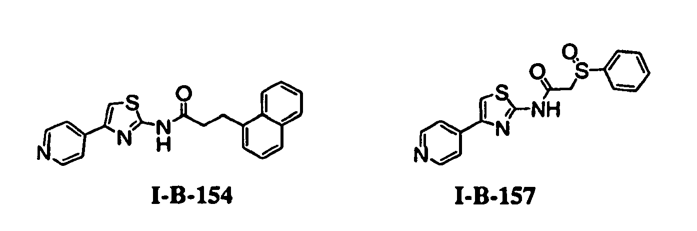

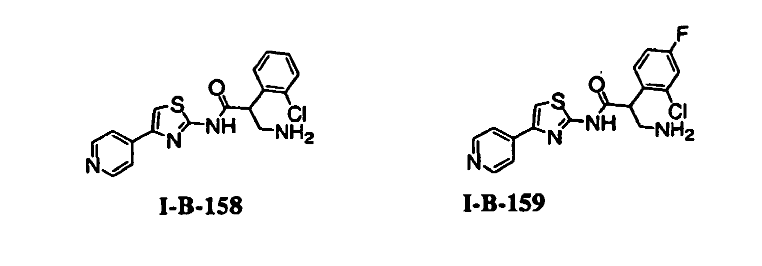

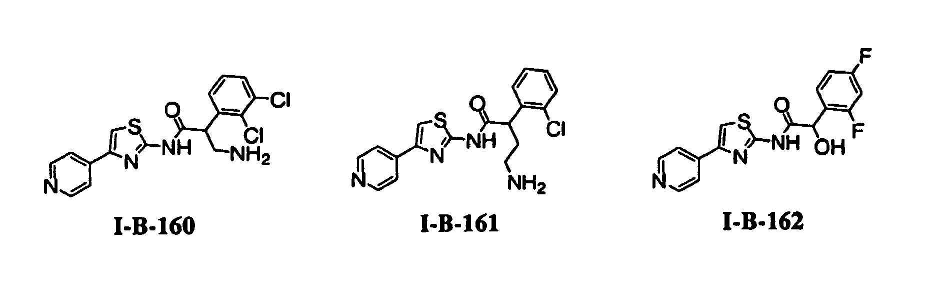

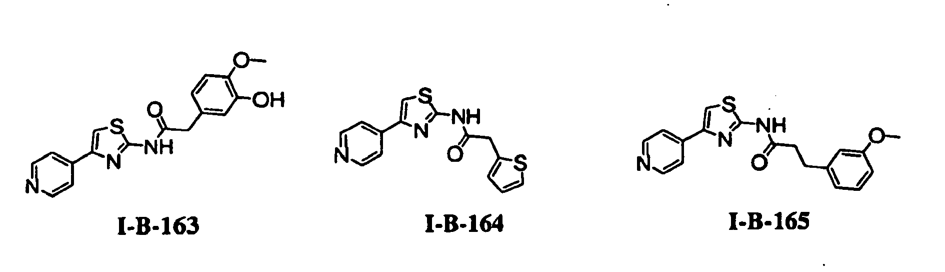

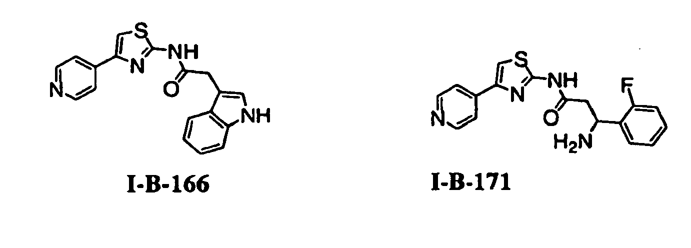

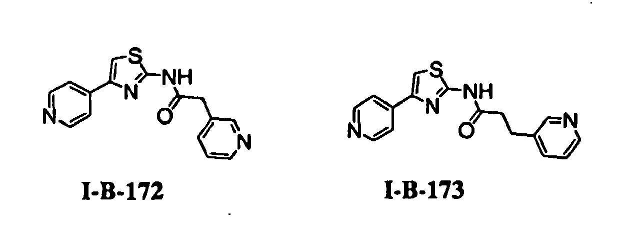

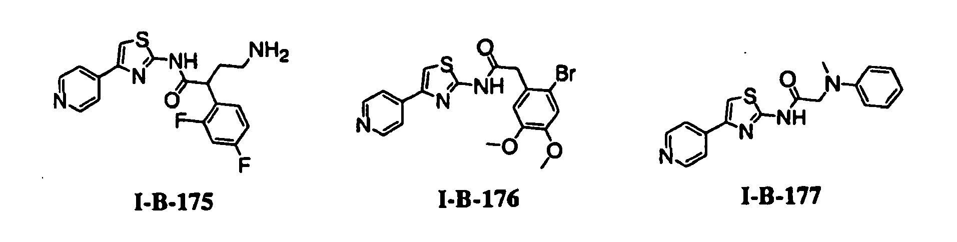

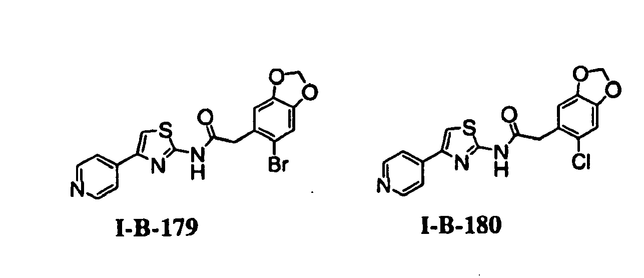

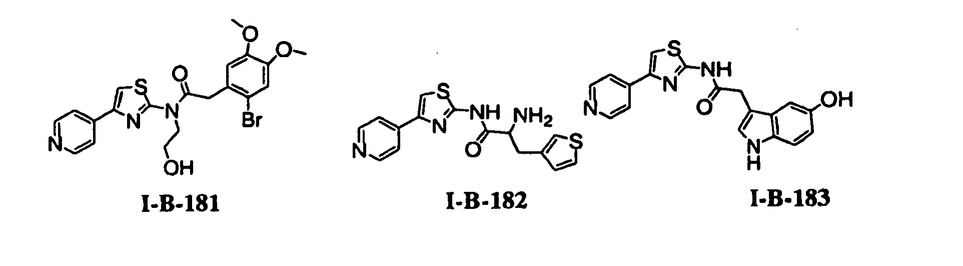

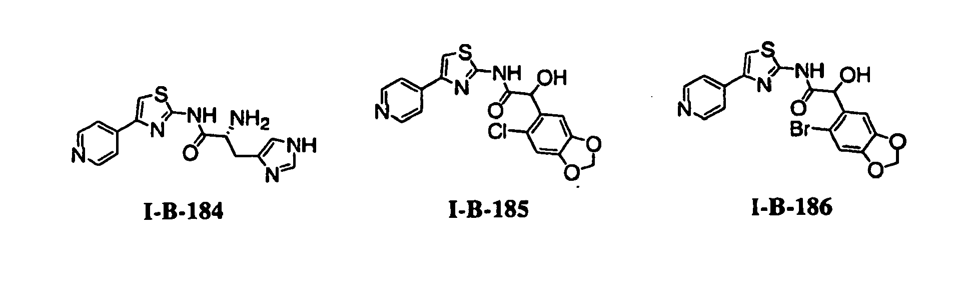

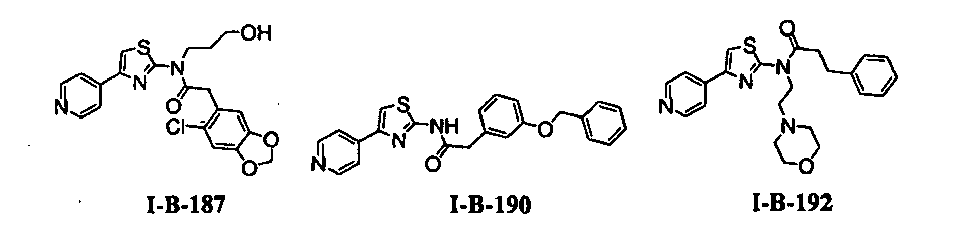

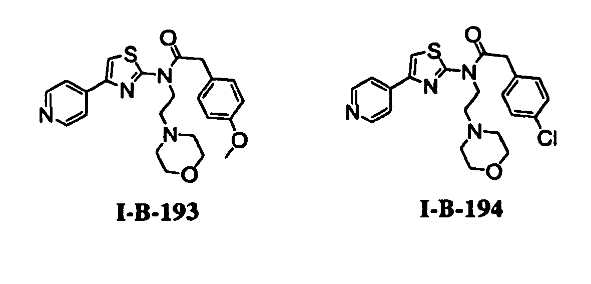

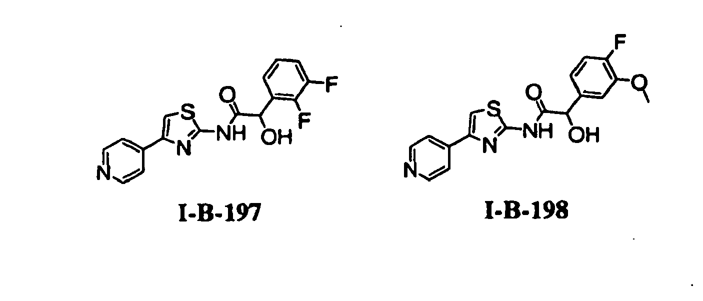

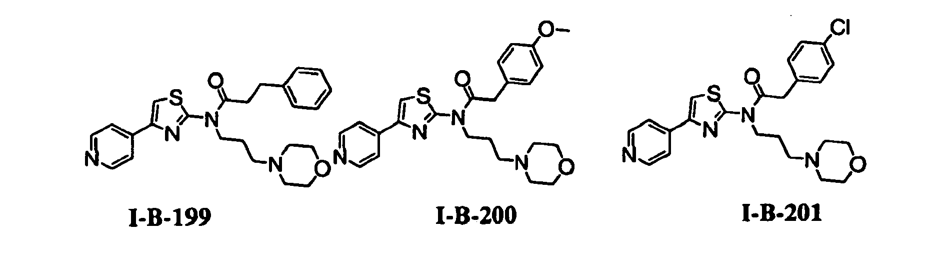

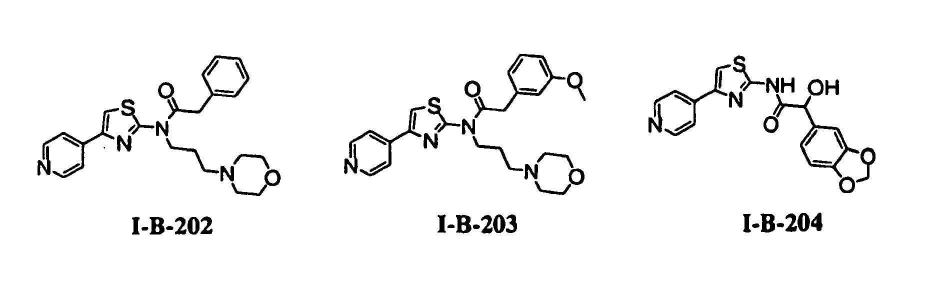

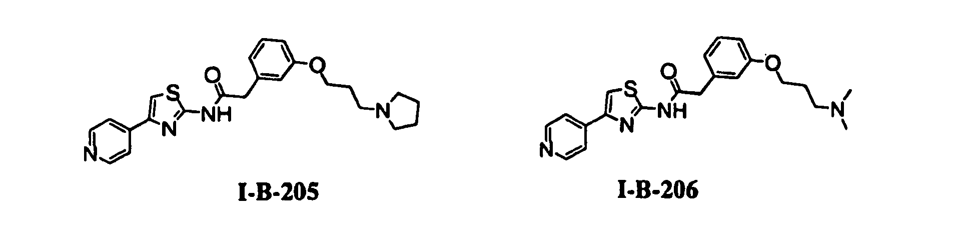

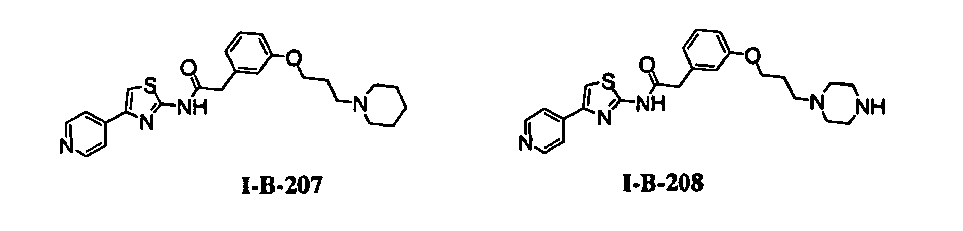

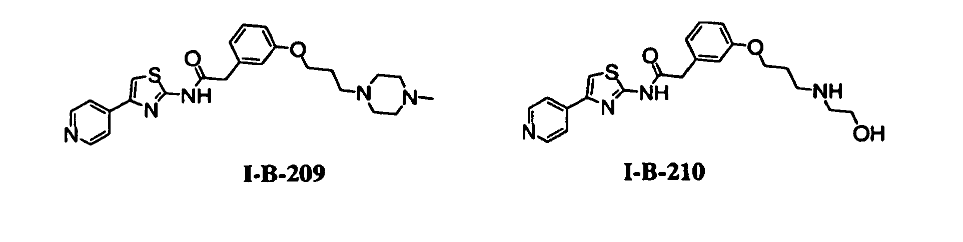

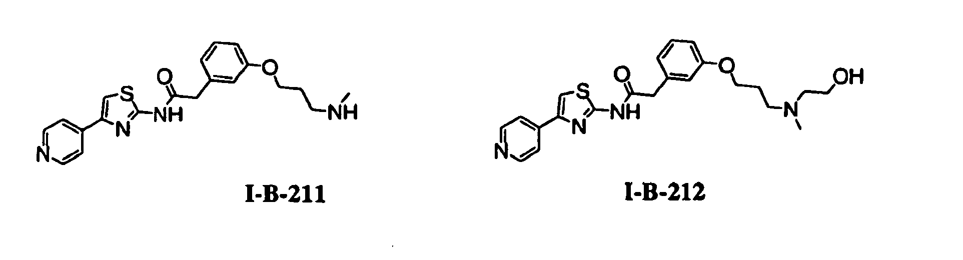

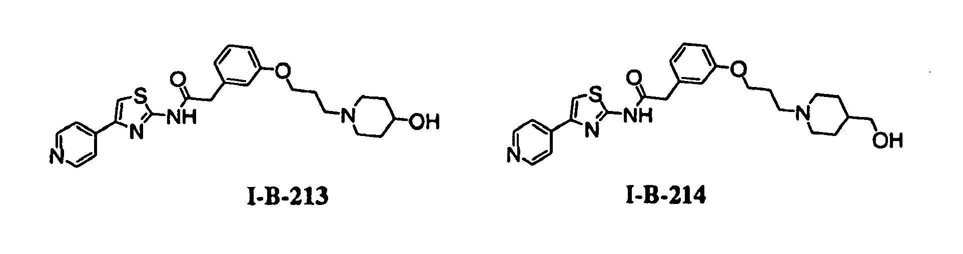

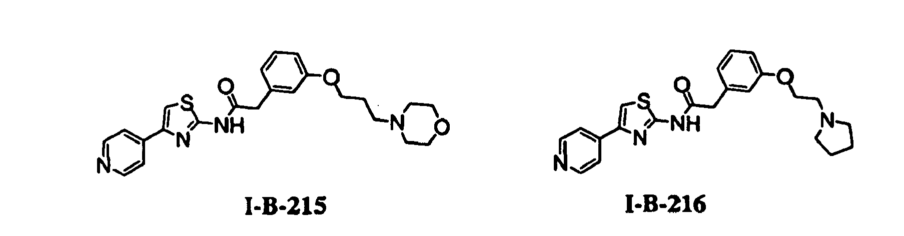

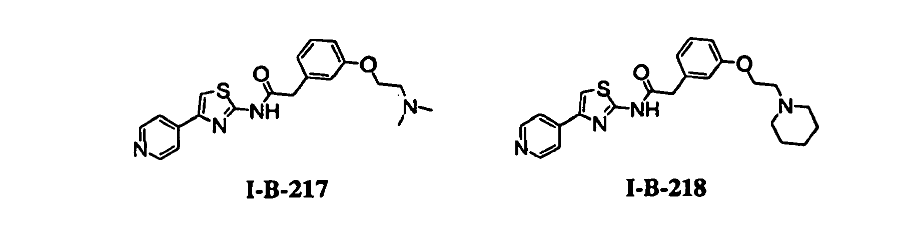

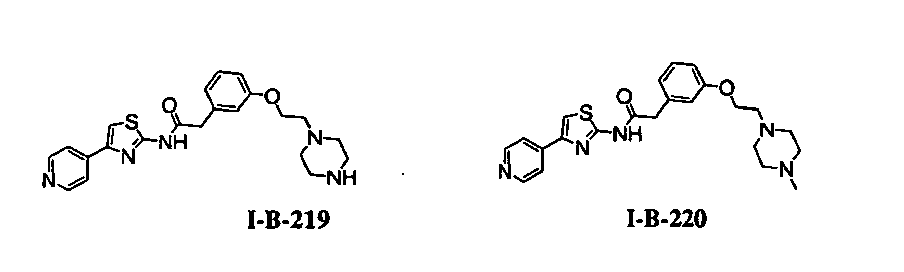

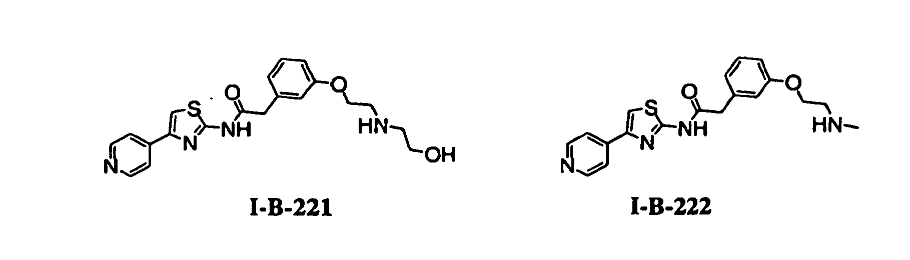

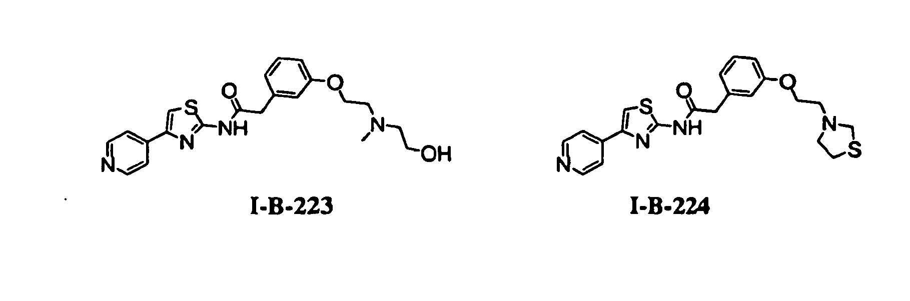

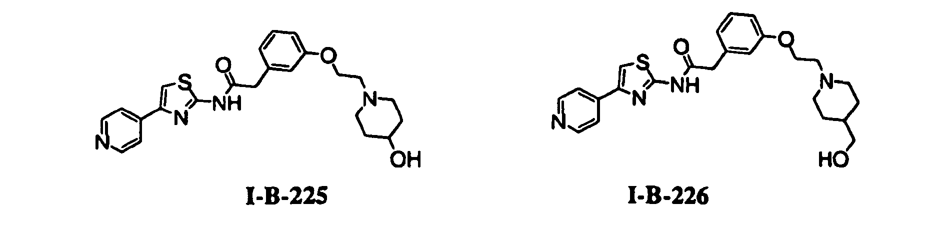

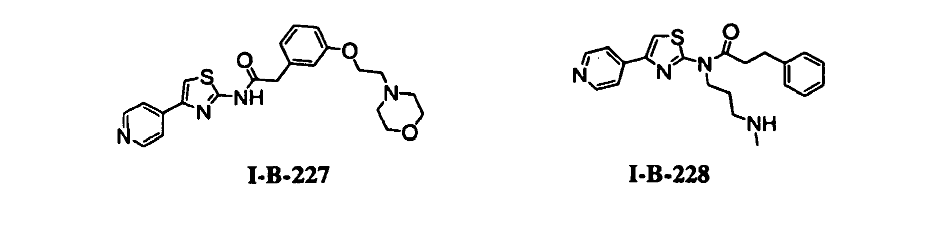

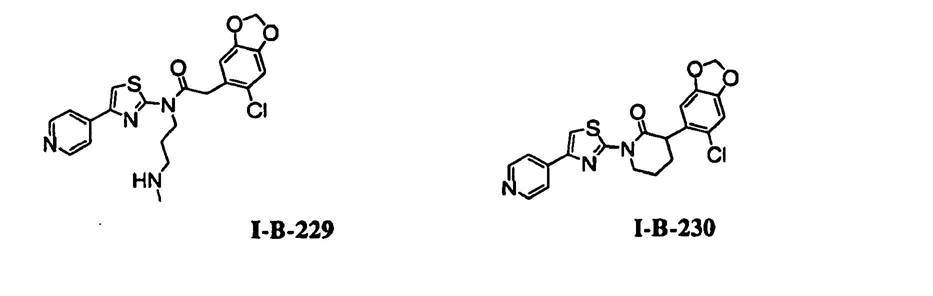

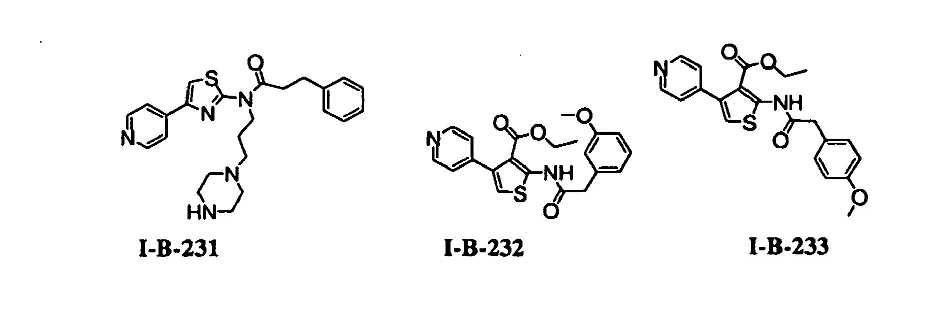

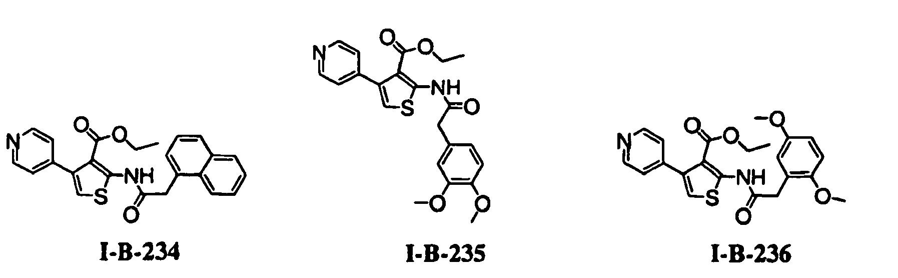

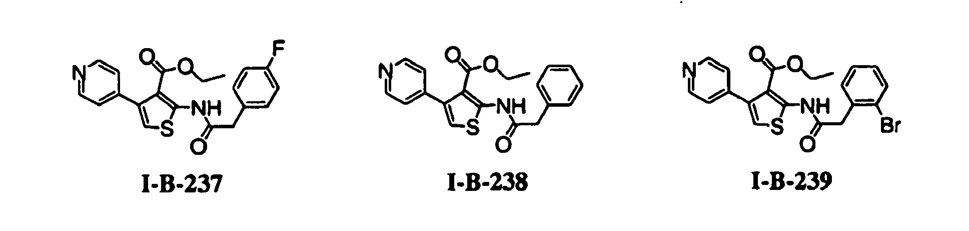

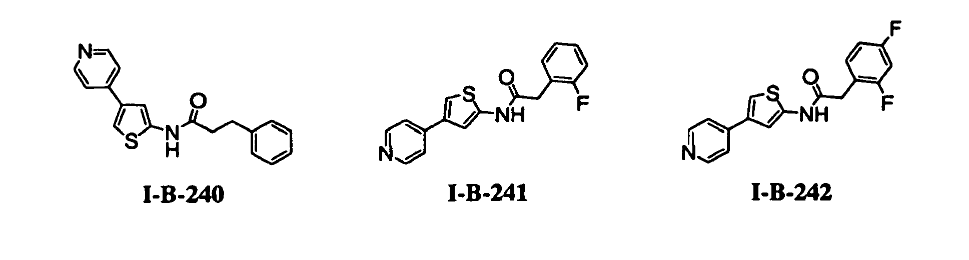

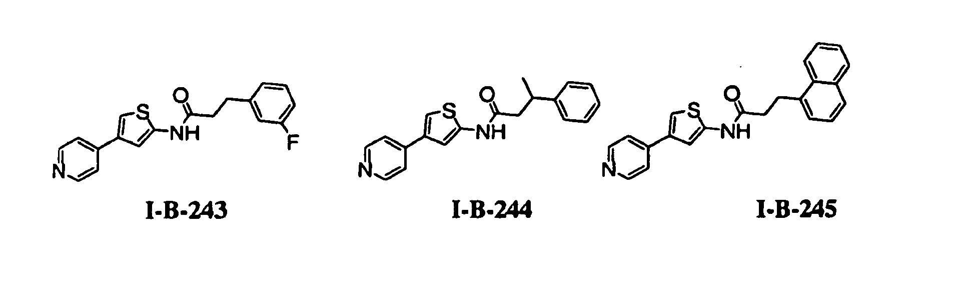

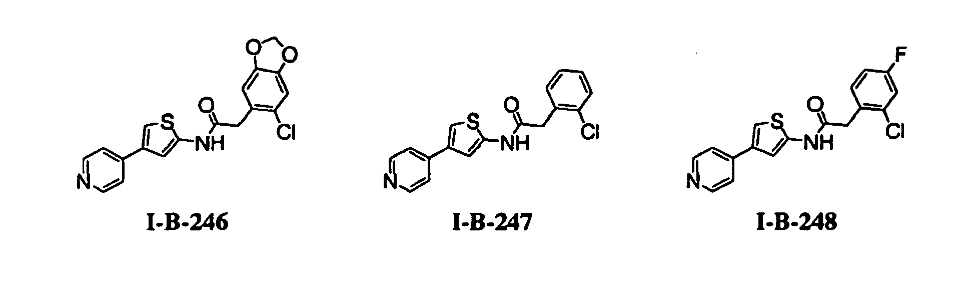

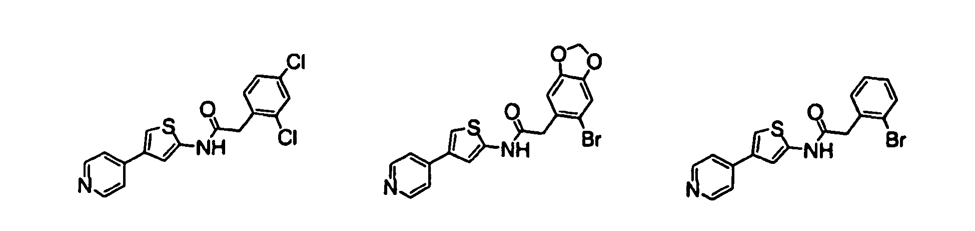

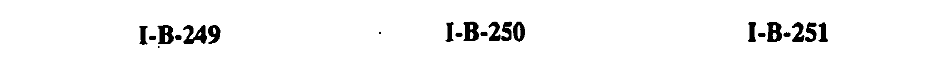

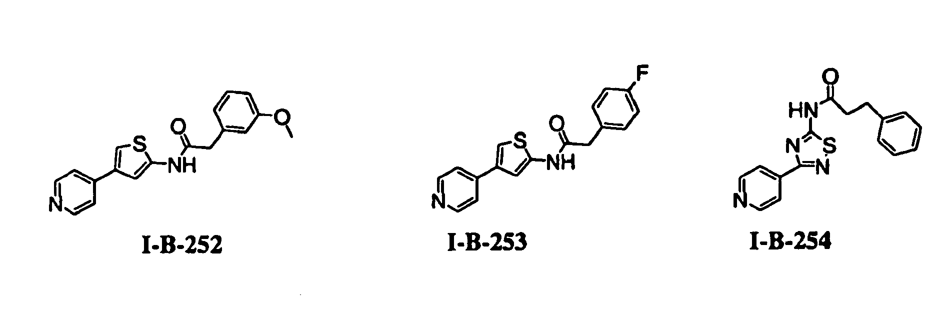

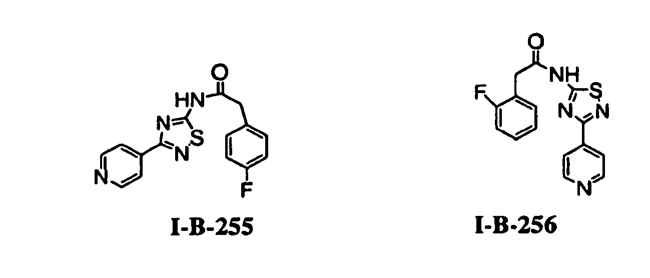

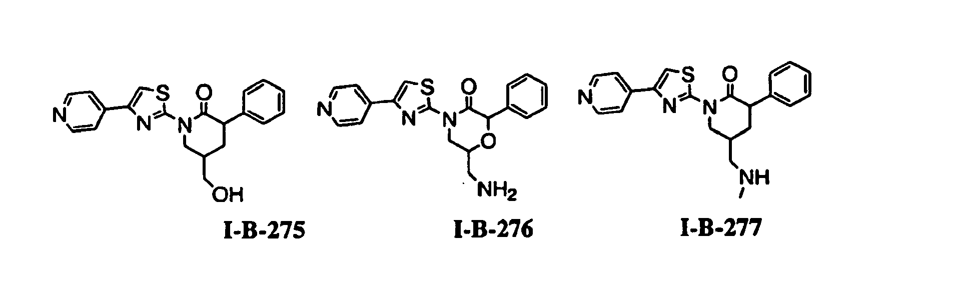

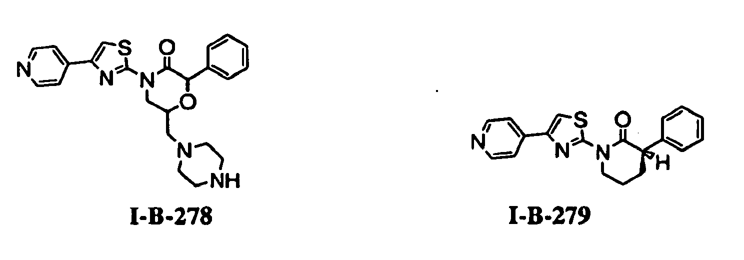

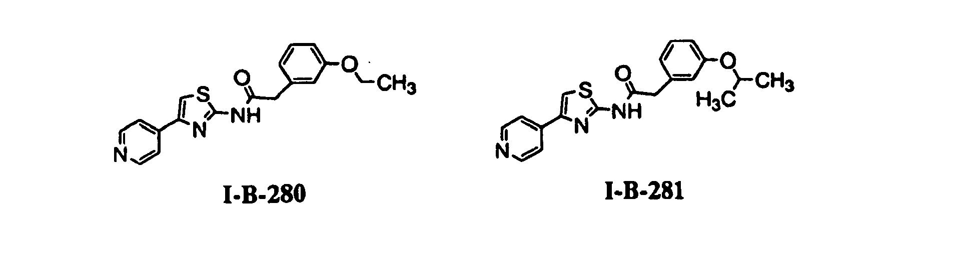

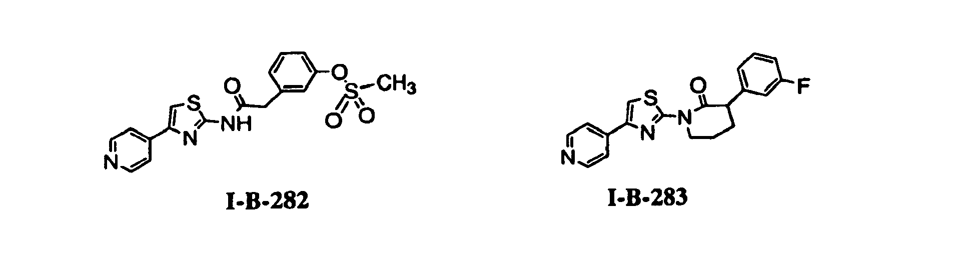

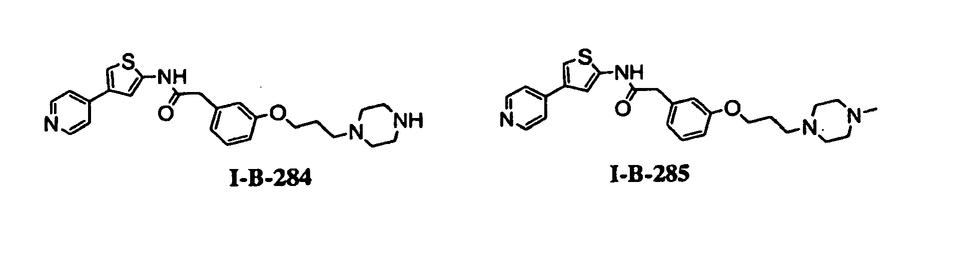

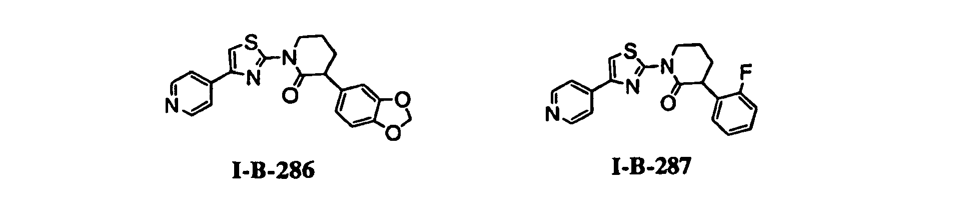

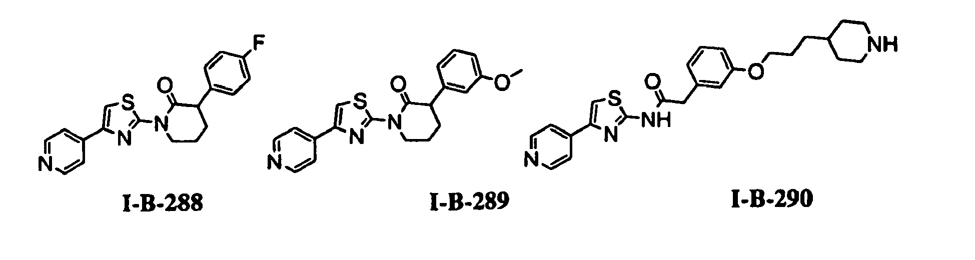

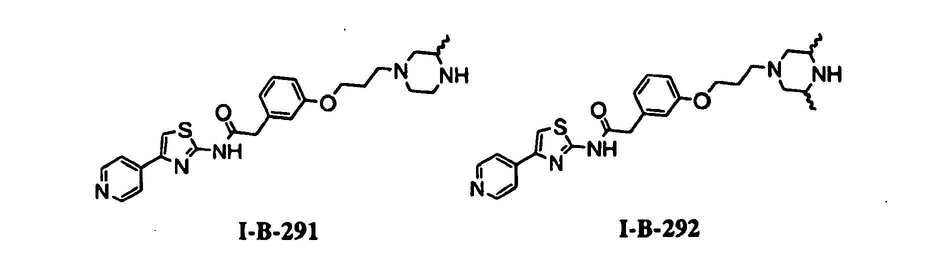

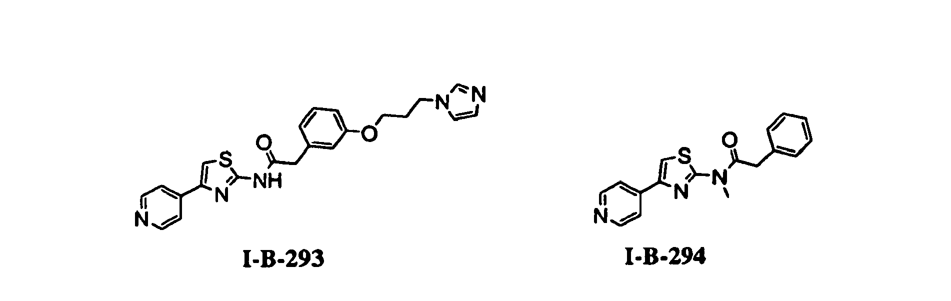

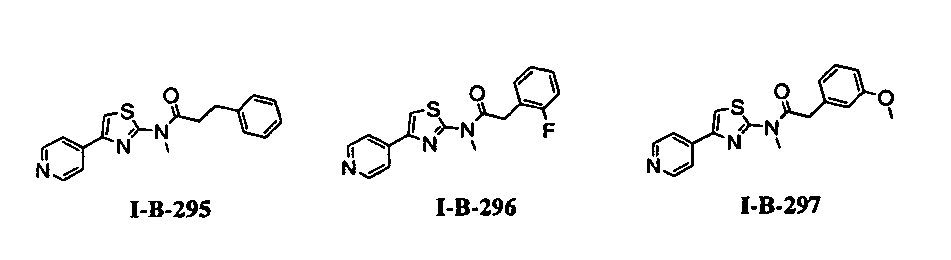

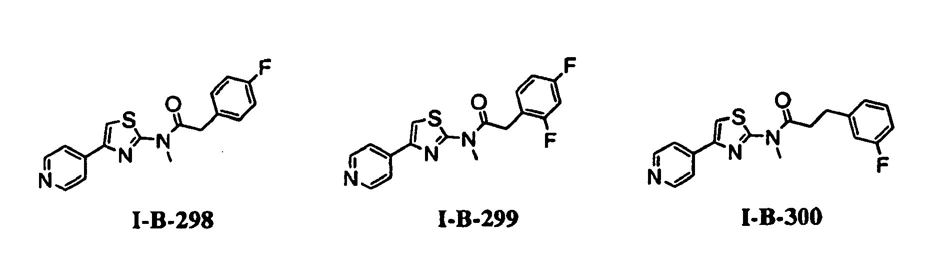

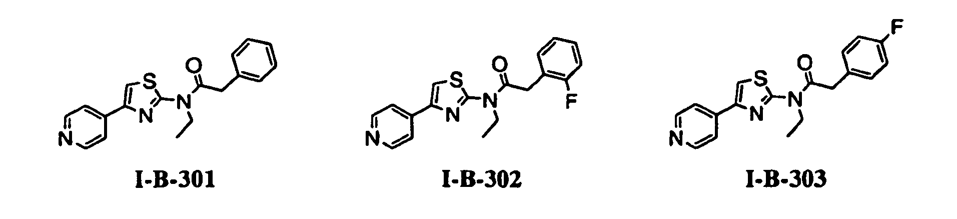

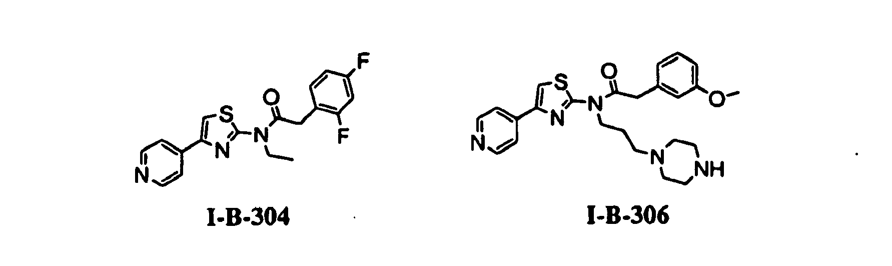

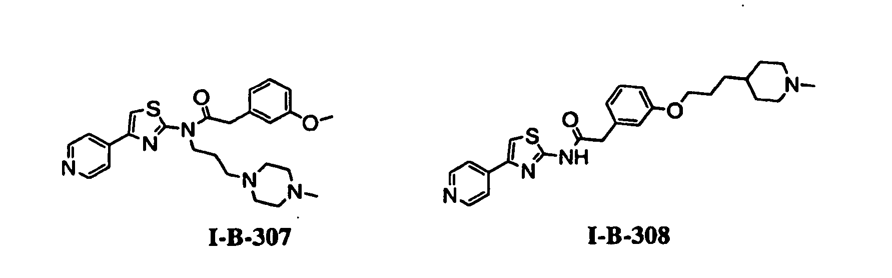

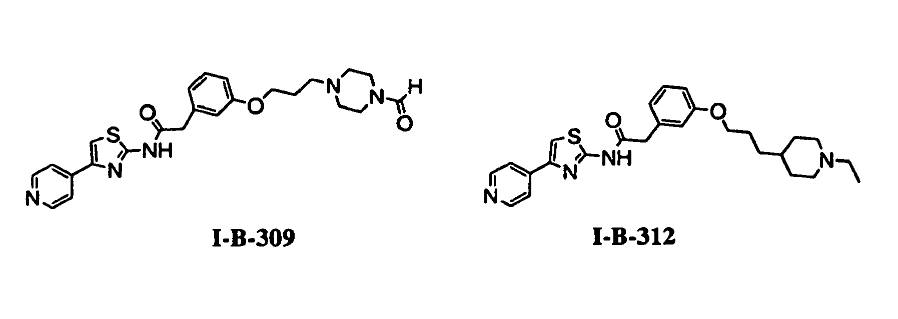

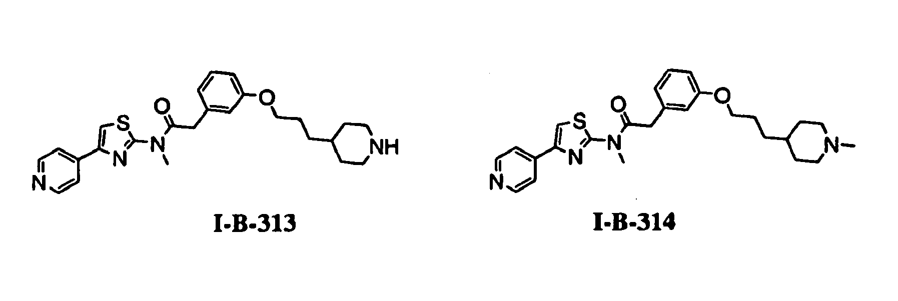

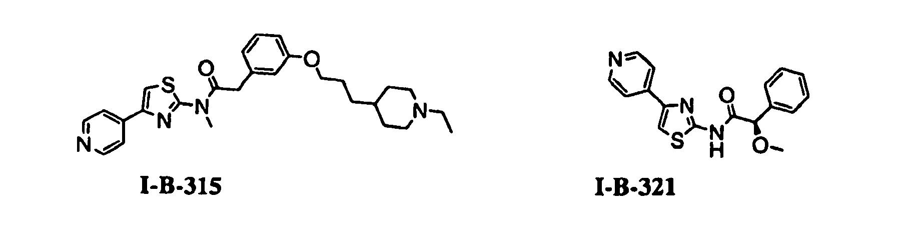

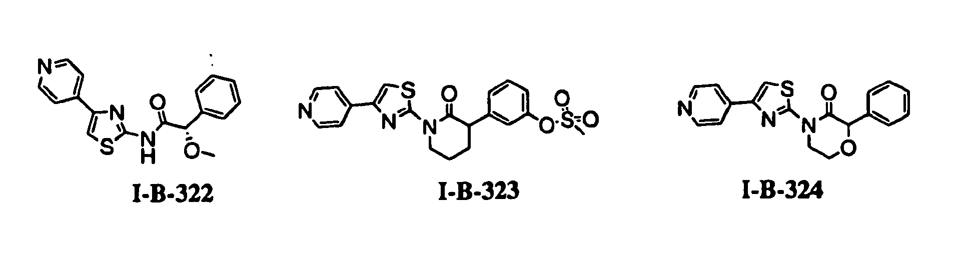

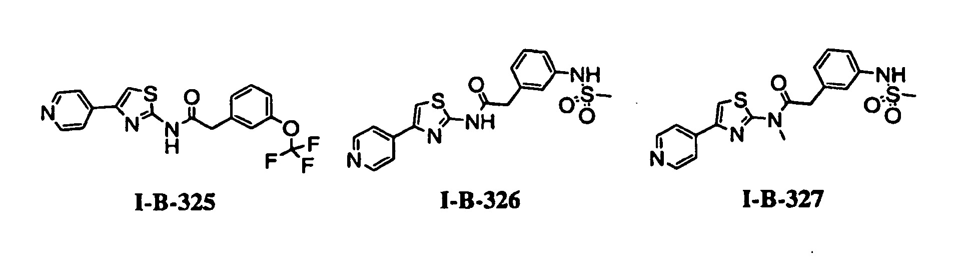

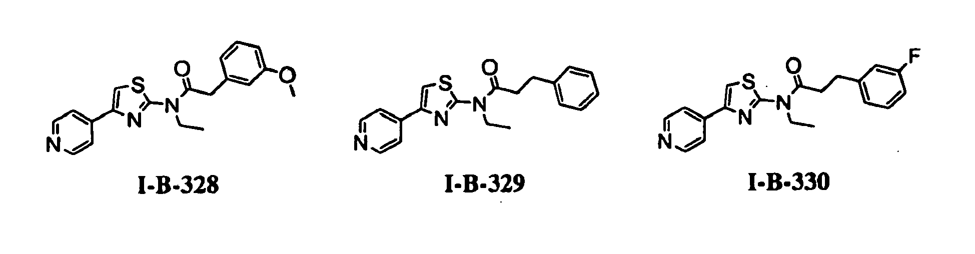

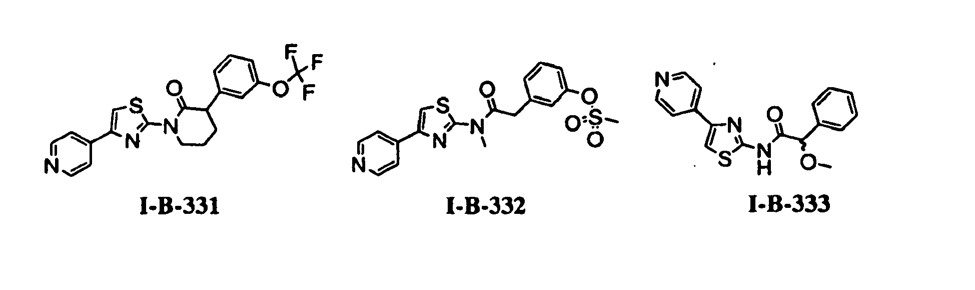

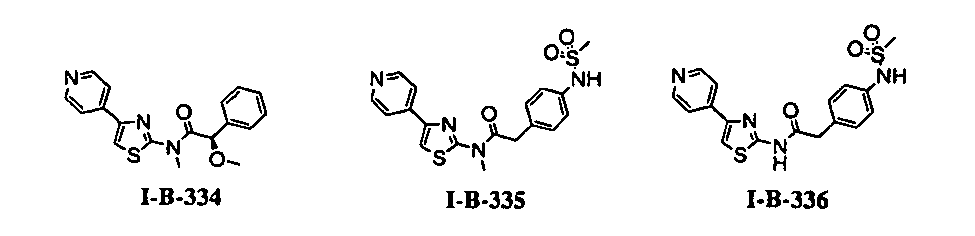

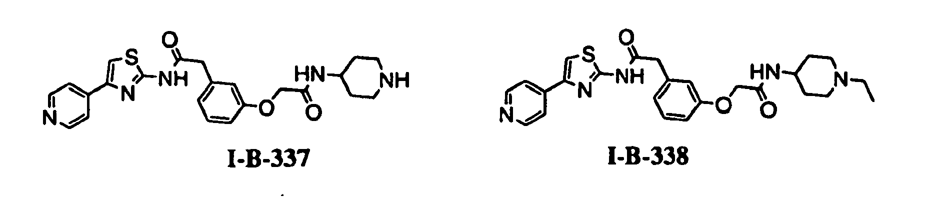

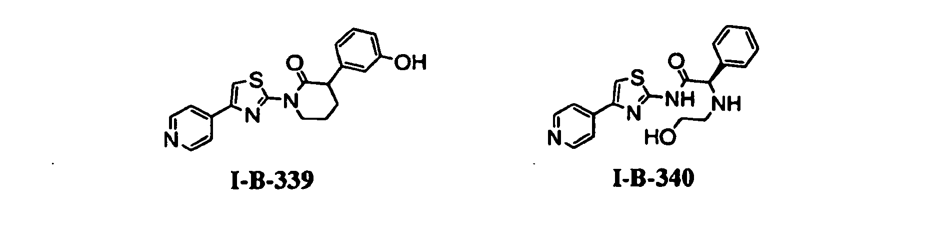

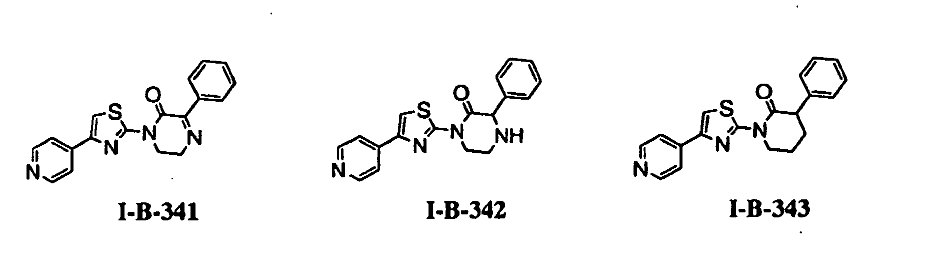

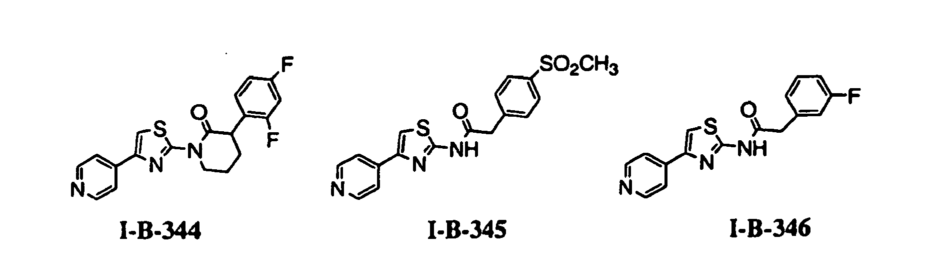

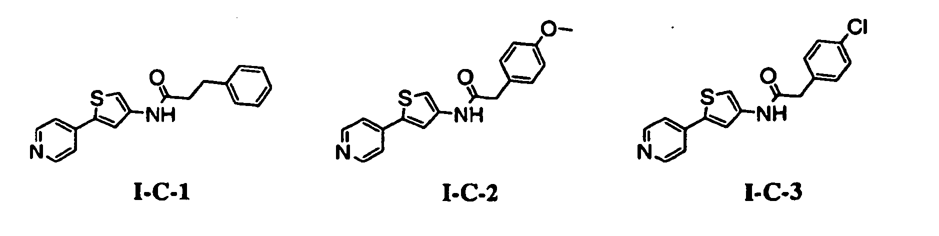

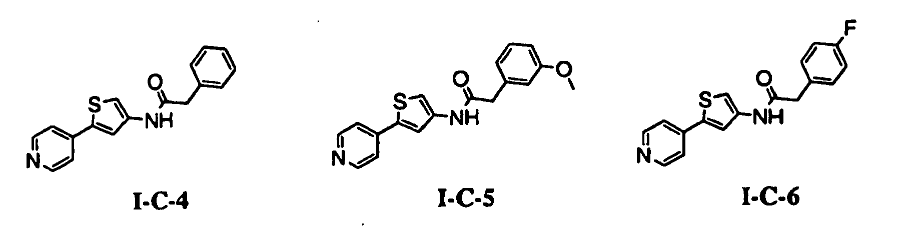

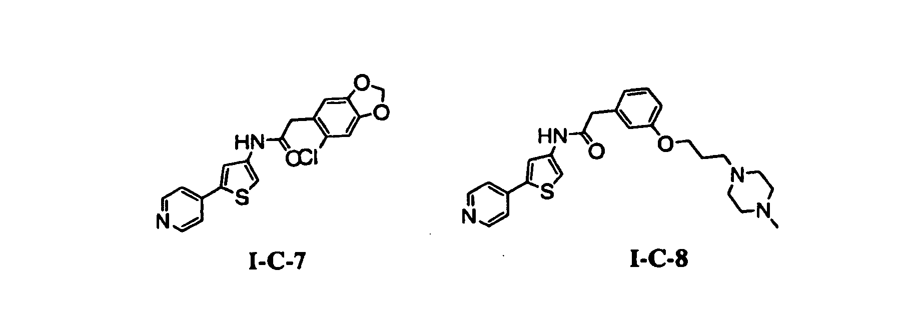

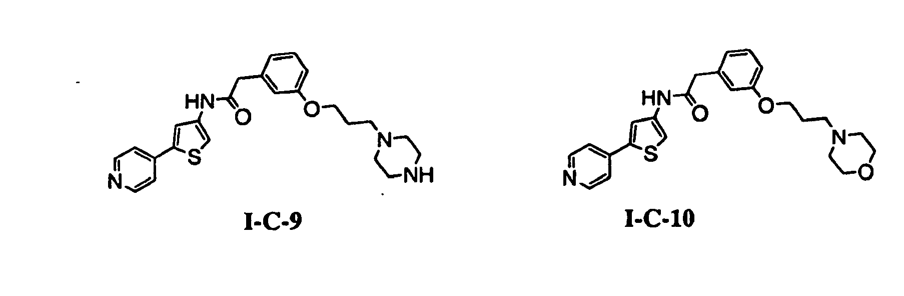

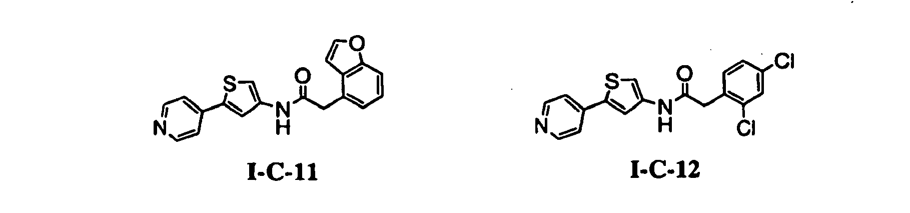

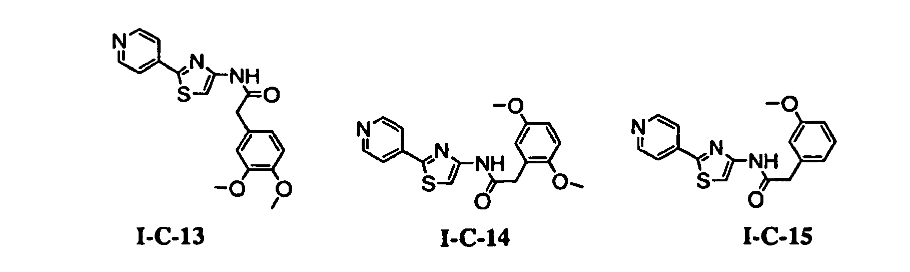

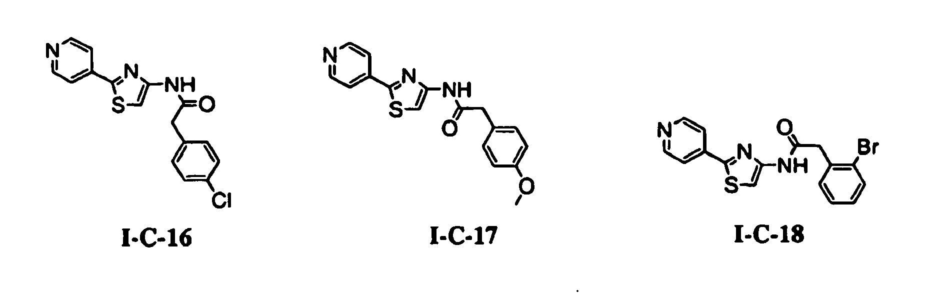

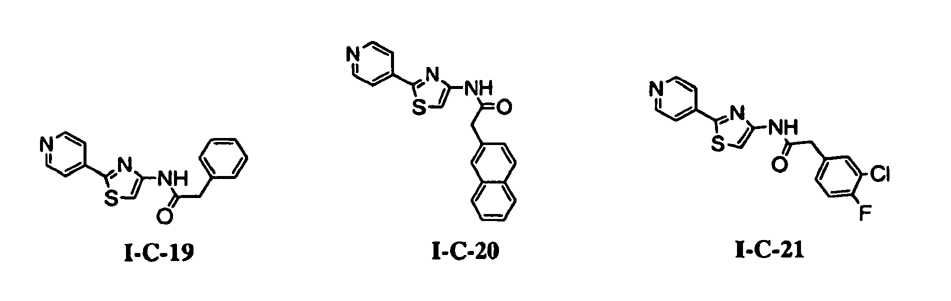

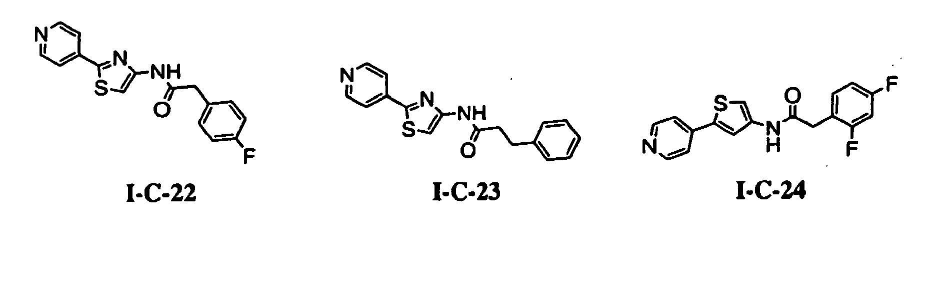

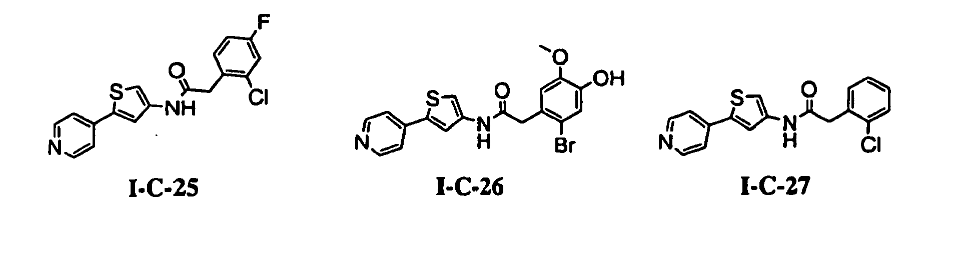

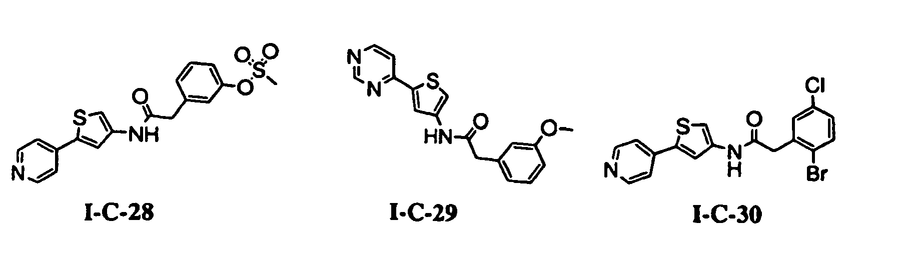

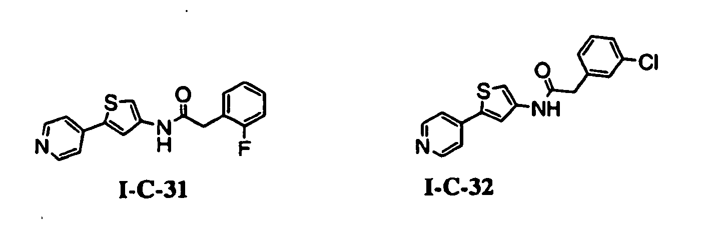

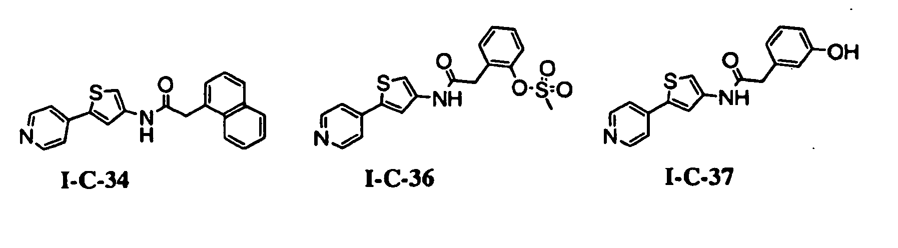

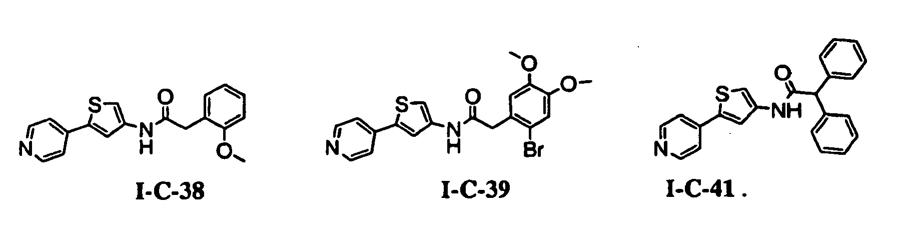

17. A composition comprising an effective amount of compound of any one of claims 1 to 16, and a pharmaceutically acceptable carrier, adjuvant, or vehicle.

18. The composition of claim 17, additionally comprising a therapeutic agent selected from a chemotherapeutic or anti-proliferative agent, an anti-inflammatory agent, an immunomodulatory or immunosuppressive agent, a neurotrophic factor, an agent for treating cardiovascular disease, an agent for treating destructive bone disorders, an agent for treating liver disease, an anti-viral agent, an agent for treating blood disorders, an agent for treating diabetes, or an agent for treating immunodeficiency disorders.

19. An *in vitro* method using a compound having the formula: 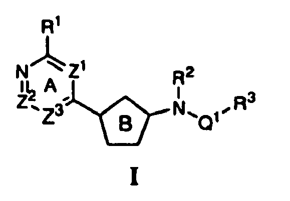 or a pharmaceutically acceptable salt thereof, wherein 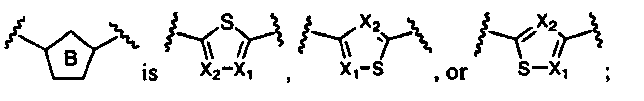 R¹ is halogen, CN, NO₂, or VₘR;
Z¹ and Z³ are each independently N or CR^{Z}, and Z² is N or CH¹, provided that Z¹, Z² and Z³ are not simultaneously N;
each occurrence of R^{Z} is independently halogen, CN, NO₂, or UₙR';
R² is UₙR';
X¹ and X² are each independently CR⁴ or N;
each occurrence of R⁴ is independently halogen, CN, NO₂, or VₘR;
each occurrence of U or V is independently an optionally substituted C₁₋₆ alkylidene chain, wherein up to two methylene units of the chain are optionally and independently replaced by -NR-, -S-, -O-, -CS-, -CO₂-, -OCO-, -CO-, -COCO-, - CONR-, -NRCO-, -NRCO₂-, -SO₂NR-, -NRSO₂-, -CONRNR-, -NRCONR-, - OCONR-, -NRNR-, -NRSO₂NR-, -SO-, -SO₂-, -PO-, -PO₂-, or -POR-;
m and n are each independently 0 or 1;
each occurrence of R is independently hydrogen or an optionally substituted C₁₋₆ aliphatic group; and each occurrence of R^{'} is independently hydrogen or an optionally substituted C₁₋₆ aliphatic group, a 3-8-membered saturated, partially unsaturated, or fully unsaturated monocyclic ring having 0-3 heteroatoms independently selected from nitrogen, oxygen, or sulfur, or an 8-12 membered saturated, partially unsaturated, or fully unsaturated bicyclic ring system having 0-5 heteroatoms independently selected from nitrogen, oxygen, or sulfur; or R and R^{'}, two occurrences of R, or two occurrences of R^{'}, are taken together with the atom(s) to which they are bound to form an optionally substituted 3-12 membered saturated, partially unsaturated, or fully unsaturated monocyclic or bicyclic ring having 0-4 heteroatoms independently selected from nitrogen, oxygen, or sulfur;
Q¹ is -CO-, -SO₂-, -CONR-, or -SO₂NR-;
R³ is Q²-Ar¹,
or R² and Q¹-R³, taken together with the nitrogen atom, form the cyclic group: 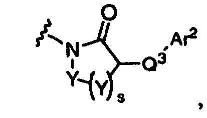 where s is 1 or 2, each occurrence of Y is independently, as valency and stability permit, -CO-, -CS-, -SO₂-, -O-, -S-, -NR⁵-, or -C(R⁵)₂-, and R⁵ is UₙR';
Q² and Q³ are each independently a bond or a C₁₋₆ alkylidene chain, wherein up to two methylene units of the chain are each optionally and independently replaced by -NR'-, -S-, -O-, -CS-, -CO₂-, -OCO-, -CO-, -COCO-, -CONR'-, -NR'CO-, -NR'CO₂-, -SO₂NR'-, -NR'SO₂-, -CONR'NR'-, -NR'CONR'-, -OCONR'-, -NR'NR'-, -NR'SO₂NR'-, -SO-, -SO₂-, -PO-, -PO₂-, or-POR'-; and wherein any carbon atom in the one or more methylene units is optionally substituted with one or two occurrences of R⁶, wherein each occurrence of R⁶ is independently halogen, CN, NO₂, or UₙR', or two occurrences of R⁶, or R' and R⁶, taken together with the atoms to which they are bound, form an optionally substituted 3-6-membered cycloalkyl, heterocyclyl, aryl or heteroaryl ring; and
Ar¹ and Ar² are each independently a 5-8 membered saturated, partially unsaturated, or fully unsaturated monocyclic ring having 0-3 heteroatoms independently selected from nitrogen, oxygen, or sulfur, or an 8-12 membered saturated, partially unsaturated, or fully unsaturated bicyclic ring system having 0-5 heteroatoms independently selected from nitrogen, oxygen, or sulfur; wherein Ar¹ and Ar² are each optionally substituted with 0-5 independent occurrences of TR⁷; wherein T is a bond or is a C₁-C₆ alkylidene chain wherein up to two methylene units of T are optionally and independently replaced by -NR-, -S-, -O-, -CS-, -CO₂-, -OCO-, -CO-, -COCO-, -CONR-, -NRCO-, -NRCO₂-, -SO₂NR-, -NRSO₂-, -CONRNR-, -NRCONR-, -OCONR-, -NRNR-, -NRSO₂NR-, -SO-, -SO₂-, -PO-, -PO₂-, or -POR-; and each occurrence of R⁷ is independently R', halogen, NO₂, or CN;
or a pharmaceutically acceptable salt or composition thereof, for inhibiting ROCK, ERK, GSK or AGC kinase activity in a biological sample.

20. An *in vitro* method as claimed in claim 19 for use in inhibiting ROCK activity.

21. A compound having the formula: 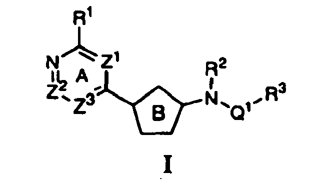 wherein 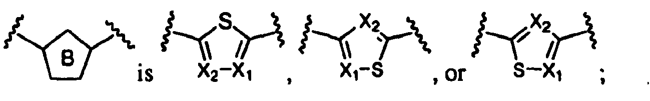 R¹ is halogen, CN, NO₂, or VₘR;
Z¹ and Z³ are each independently N or CR^{Z}, and Z² is N or CR¹, provided that Z¹, Z² and Z³ are not simultaneously N;
each occurrence of R^{Z} is independently halogen, CN, NO₂, or UₙR';
R² is UₙR';
X¹ and X² are each independently CR⁴ or N;
each occurrence of R⁴ is independently halogen, CN, NO₂, or VₘR;
each occurrence of U or V is independently an optionally substituted C₁₋₆ alkylidene chain, wherein up to two methylene units of the chain are optionally and independently replaced by -NR-, -S-, -O-, -CS-, -CO₂-, -OCO-, -CO-, -COCO-, - CONR-, -NRCO-, -NRCO₂-, -SO₂NR-, -NRSO₂-, -CONRNR-, -NRCONR-,-OCONR-, -NRNR-, -NRSO₂NR-, -SO-, -SO₂-, -PO-, -PO₂-, or -POR-;
m and n are each independently 0 or 1;
each occurrence of R is independently hydrogen or an optionally substituted C₁₋₆ aliphatic group; and each occurrence of R^{'} is independently hydrogen or an optionally substituted C₁₋₆ aliphatic group, a 3-8-membered saturated, partially unsaturated, or fully unsaturated monocyclic ring having 0-3 heteroatoms independently selected from nitrogen, oxygen, or sulfur, or an 8-12 membered saturated, partially unsaturated, or fully unsaturated bicyclic ring system having 0-5 heteroatoms independently selected from nitrogen, oxygen, or sulfur; or R and R^{'}, two occurrences of R, or two occurrences of R^{'}, are taken together with the atom(s) to which they are bound to form an optionally substituted 3-12 membered saturated, partially unsaturated, or fully unsaturated monocyclic or bicyclic ring having 0-4 heteroatoms independently selected from nitrogen, oxygen, or sulfur;
Q¹ is -CO-, -SO₂-, or -SO₂NR-;
R³ is Q²-Ar¹,
or R² and Q¹-R³, taken together with the nitrogen atom, form the cyclic group: 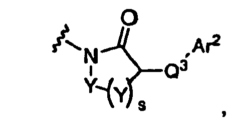 where s is 1 or 2, each occurrence of Y is independently, as valency and stability permit, -CO-, -CS-, -SO₂-, -O-, -S-, -NR⁵-, or -C(R⁵)₂-, and R⁵ is UₙR';
Q² and Q³ are each independently a bond or a C₁₋₆ alkylidene chain, wherein up to two methylene units of the chain are each optionally and independently replaced by -S-, -O-, -CS-, -CO₂-, -OCO-, -CO-, -COCO-, -CONR'-, -NR'CO-, -NR'CO₂-, -SO₂NR'-, -NR'SO₂-, -CONR'NR'-, -NR'CONR'-, -OCONR'-, -NR'NR'-, -NR'SO₂NR'-, -SO-, -SO₂-, -PO-, -PO₂-, or -POR'-; and wherein any carbon atom in the one or more methylene units is optionally substituted with one or two occurrences of R⁶, wherein each occurrence of R⁶ is independently halogen, CN, NO₂, or UₙR', or two occurrences of R⁶, or R' and R⁶, taken together with the atoms to which they are bound, form an optionally substituted 3-6-membered cycloalkyl, heterocyclyl, aryl or heteroaryl ring; and
Ar¹ and Ar² are each independently a 5-8 membered saturated, partially unsaturated, or fully unsaturated monocyclic ring having 0-3 heteroatoms independently selected from nitrogen, oxygen, or sulfur, or an 8-12 membered saturated, partially unsaturated, or fully unsaturated bicyclic ring system having 0-5 heteroatoms independently selected from nitrogen, oxygen, or sulfur; wherein Ar¹ and Ar² are each optionally substituted with 0-5 independent occurrences of TR⁷; wherein T is a bond or is a C₁-C₆ alkylidene chain wherein up to two methylene units of T are optionally and independently replaced by -NR-, -S-, -O-, -CS-, -CO₂-, -OCO-, -CO-, -COCO-, -CONR-, -NRCO-, -NRCO₂-, -SO₂NR-, -NRSO₂-, -CONRNR-, -NRCONR-, -OCONR-, -NRNR-, -NRSO₂NR-, -SO-, -SO₂-, -PO-, -PO₂-, or -POR-; and each occurrence of R⁷ is independently R', halogen, NO₂, or CN;
or a pharmaceutically acceptable salt or composition thereof for use in treating or lessening the severity of a disease condition or disorder selected from a proliferative disorder, a cardiac disorder, a neurodegenerative disorder, a psychotic disorder, an autoimmune disorder, a condition associated with organ transplant, an inflammatory disorder, an immunologically mediated disorder, a viral disease, or a bone disorder.

22. The compound, pharmaceutically acceptable salt or composition thereof of claim 21 for use with an additional therapeutic agent selected from a chemotherapeutic or anti-proliferative agent, an anti-inflammatory agent, an immunomodulatory or immunosuppressive agent, a neurotrophic factor, an antipsychotic agent, an agent for treating cardiovascular disease, an agent for treating destructive bone disorders, an agent for treating liver disease, an anti-viral agent, an agent for treating blood disorders, an agent for treating diabetes, or an agent for treating immunodeficiency disorders.

23. The compound, pharmaceutically acceptable salt or composition thereof, of claim 21, for use in treating or lessening the severity of a disease, condition or disorder, wherein disease, condition, or disorder is allergy, asthma, diabetes, Alzheimer's disease, Huntington's disease, Parkinson's disease, AIDS-associated dementia, amyotrophic lateral sclerosis (AML, Lou Gehrig's disease), multiple sclerosis (MS), schizophrenia, cardiomyocyte hypertrophy, reperfusion/ischemia (e.g., stroke), baldness, cancer, hepatomegaly, cardiovascular disease including cardiomegaly, cystic fibrosis, viral disease, autoimmune diseases, atherosclerosis, restenosis, psoriasis, inflammation, hypertension, angina pectoris, cerebrovascular contraction, peripheral circulation disorder, premature birth, arteriosclerosis, vasospasm (cerebral vasospasm, coronary vasospasm), retinopathy, erectile dysfunction (ED), AIDS, osteoporosis, Crohn's Disease and colitis, neurite outgrowth, or Raynaud's Disease; particularly atherosclerosis, hypertension, erectile dysfunction (ED), reperfusion/ischemia (e.g., stroke), or vasospasm (cerebral vasospasm and coronary vasospasm).

24. Use of a compound according to any of claims 1-16 in the manufacture of a medicament for treating or lessening the severity of a disease, condition or disorder selected from a proliferative disorder, a cardiac disorder, a neurodegenerative disorder, a psychotic disorder, an autoimmune disorder, a condition associated with organ transplant, an inflammatory disorder, an immunologically mediated disorder, a viral disease, or a bone disorder; such as allergy, asthma, diabetes, Alzheimer's disease, Huntington's disease, Parkinson's disease, AIDS-associated dementia, amyotrophic lateral sclerosis, (AML, Lou Gehrig's disease), multiple sclerosis (MS), schizophrenia, cardiomyocyte hypertrophy, reperfusion/ischemia (e.g. stroke), baldness, cancer, hepatomegaly, cardiovascular disease including cardiomegaly, cystic fibrosis, viral disease, autoimmune diseases, atherosclerosis, restenosis, psoriasis, inflammation, hypertension, angina pectoris, cerebrovascular contraction, peripheral circulation disorder, premature birth, arteriosclerosis, vasospasm (cerebral vasospasm, coronary vasospasm), retinopathy, erectile dysfunction (ED), AIDS, osteoporosis, Crohn's Disease and colitis, neurite outgrowth, or Raynaud's Disease;
particularly atherosclerosis, hypertension, erectile dysfunction (ED), reperfusion/ischemia (e.g., stroke), or vasospasm (cerebral vasospasm and coronary vasospasm).

## Patentansprüche

1. Verbindung der Formel I: 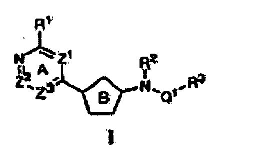 oder eines pharmazeutisch annehmbaren Salzes davon, wobei: 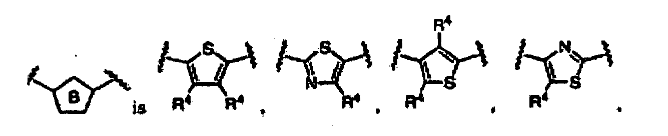 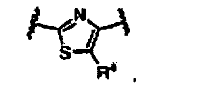 oder 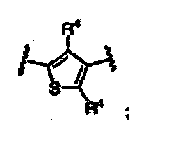 ist;
R¹ Halogen, CN, NO₂ oder VₘR ist;
Z¹ und Z³ jeweils CR^{Z} sind und Z² CR¹ ist;
jede Präsenz von R^{Z} unabhängig Halogen, CN, NO₂ oder UₙR' ist;
R² UₙR' ist;
jede Präsenz von R⁴ unabhängig Halogen, CN, NO₂ oder VₘR ist;
jede Präsenz von U oder V unabhängig eine optional substituierte C₁₋₆-Alkylidenkette ist, wobei bis zu zwei Methyleneinheiten der Kette optional und unabhängig durch -NR-, -S-, -O-, -CS-, -CO₂-, -OCO-, -CO-, -COCO-, -CONR-, -NRCO-, - NRCO₂-, -SO₂NR-, -NRSO₂-, -CONRNR-, -NRCONR-, -OCONR-, -NRNR-, - NRSO₂NR-, -SO-, -SO₂-, -PO-, -PO₂-, oder -POR- ersetzt sind;
m und n jeweils unabhängig 0 oder 1 sind;
jede Präsenz von R unabhängig Wasserstoff oder eine optional substituierte, aliphatische C₁₋₆-Gruppe ist; und jede Präsenz von R' unabhängig Wasserstoff oder eine optional substituierte, aliphatische C₁₋₆-Gruppe, ein 3- bis 8-gliedriger, gesättigter, teilweise ungesättigter oder vollständig ungesättigter, monocyclischer Ring mit 0 bis 3 Heteroatomen ist, die unabhängig ausgewählt sind aus Stickstoff, Sauerstoff oder Schwefel, oder ein 8-bis 12-gliedriges, gesättigtes, teilweise ungesättigtes oder vollständig ungesättigtes, bicyclisches Ringsystem mit 0 bis 5 Heteroatomen, die unabhängig ausgewählt sind aus Stickstoff, Sauerstoff oder Schwefel; oder R und R', zweifache Präsenzen von R oder zweifache Präsenzen von R', gemeinsam mit dem Atom oder den Atomen genommen werden, an die sie gebunden sind, um einen optional substituierten, 3- bis 12-gliedrigen, gesättigten, teilweise ungesättigten oder vollständig ungesättigten, monocyclischen oder bicyclischen Ring mit 0 bis 4 Heteroatomen zu bilden, die unabhängig ausgewählt sind aus Stickstoff, Sauerstoff oder Schwefel;
Q¹ -CO- ist;
R³ Q²-Ar¹ ist, wobei Q² -(CHR⁶)_{q}- ist, wobei q 1, 2 oder 3 ist,
oder R² und Q¹-R³, gemeinsam genommen mit dem intervenierenden Stickstoffatom, die cyclische Gruppe 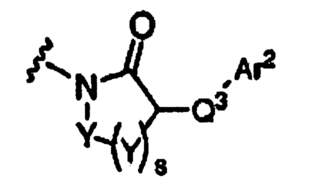 bilden, wobei s 1 oder 2 ist, jede Präsenz von Y unabhängig, wie Valenz und Stabilität zulassen, -CO-, -CS-, -SO₂-, -O-, -S-, NR⁵- oder -C(R⁵)₂- ist und R⁵ UₙR' ist;
Q³ eine Bindung oder eine C₁₋₆-Alkylidenkette ist, wobei bis zu zwei Methyleneinheiten der Kette jeweils optional und unabhängig durch -NR'-, -S-, -O-, -CS-, -CO₂-, -OCO-, -CO-, - COCO-, -CONR'-, -NR'CO-, NR'CO₂-, -SO₂NR'-, -NR'SO₂-, -CONR'NR'-, -NR'CONR', -OCONR'-, -NR'NR'-, -NR'SO₂NR'-, -SO-, -SO₂-, -PO-, -PO₂- oder -POR' ersetzt sind; und wobei jedes Kohlenstoffatom in der einen oder den mehreren Methyleneinheiten optional mit einer oder mehreren Präsenzen von R⁶ substituiert ist, wobei jede Präsenz von R⁶ unabhängig Halogen, CN, NO₂ oder UₙR' ist, oder zwei Präsenzen von R⁶ oder R' und R⁶ gemeinsam genommen mit den Atomen, an die sie gebunden sind, einen optional substituierten, 3-bis 6-gliedrigen Cycloalkyl-, Heterocyclyl-, Aryl- oder Heteroarylring bilden; und
Ar¹ Folgendes ist: 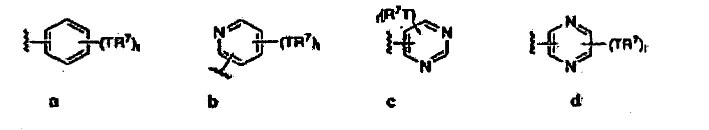 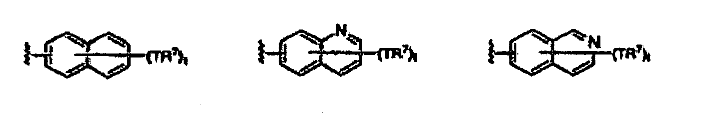 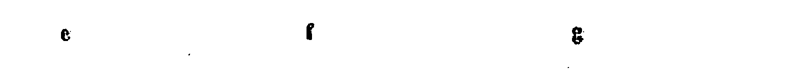 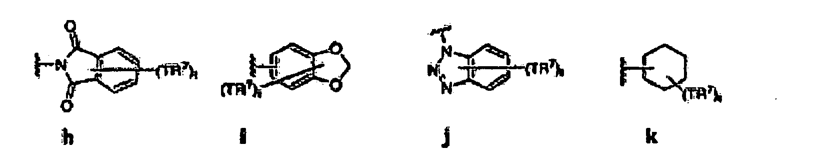 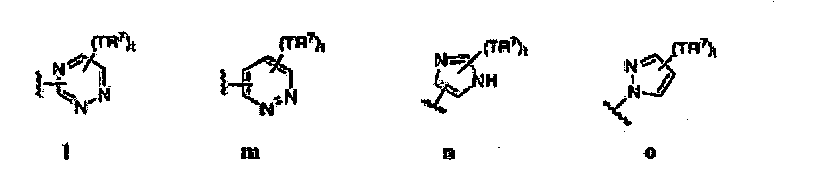 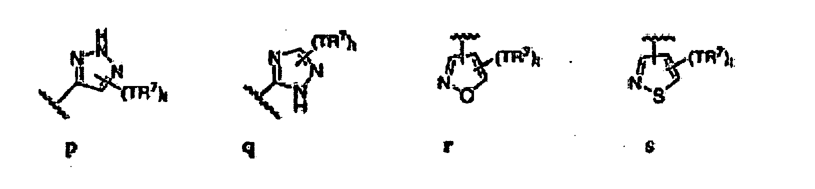 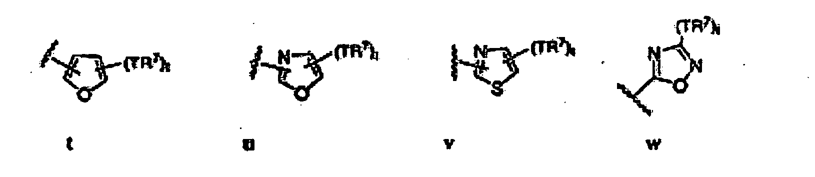 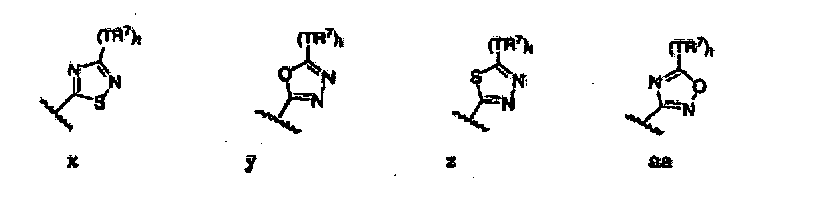 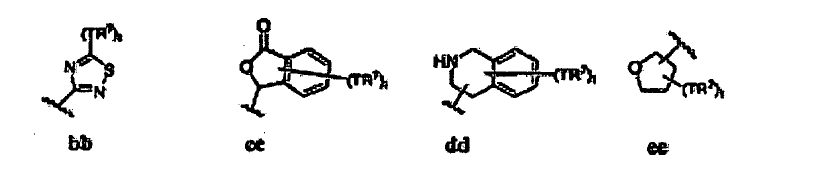 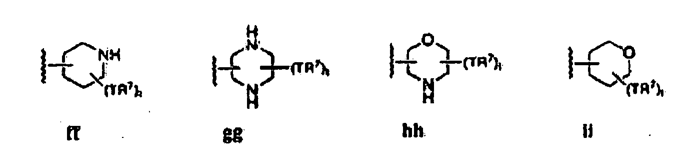 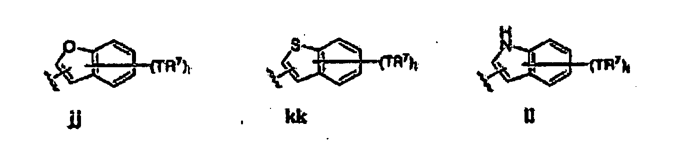 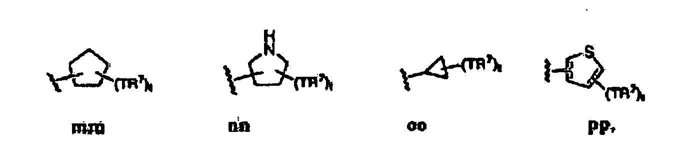 wobei t 0, 1, 2, 3, 4 oder 5 ist und wobei jedes Ar¹ an Q² durch ein substituierbares Stickstoff- oder Kohlenstoffatom gebunden ist, und wobei ein oder mehrere Wasserstoffatome auf jedem substituierbaren Stickstoff- oder Wasserstoffatom mit einer oder mehreren unabhängigen Präsenzen von TR⁷ substituiert ist beziehungsweise sind;
und Ar² ein 5- bis 8-gliedriger, gesättigter, teilweise ungesättigter oder vollständig ungesättigter, monocyclischer Ring mit 0 bis 3 Heteroatomen ist, unabhängig ausgewählt aus Stickstoff, Sauerstoff oder Schwefel, oder ein 8- bis 12-gliedriges, gesättigtes, teilweise ungesättigtes oder vollständig ungesättigtes, bicyclisches Ringsystem mit 0 bis 5 Heteroatomen, die unabhängig ausgewählt sind aus Stickstoff, Sauerstoff oder Schwefel; wobei Ar¹ und Ar² jeweils optional mit 0 bis 5 unabhängigen Präsenzen von TR⁷ substituiert sind; wobei T eine Bindung oder eine C₁-C₆-Alkylidenkette ist, wobei bis zu zwei Methyleneinheiten von T optional und unabhängig durch -NR-, -S-, -O-, -CS-, -CO₂-, -OCO-, -CO-, -COCO-, -CONR-, -NRCO-, -NRCO₂-, - SO₂NR-, -NRSO₂-, -CONRNR-, -NRCONR-, -OCONR-, -NRNR-, -NRSO₂NR-, -SO-, -SO₂-, -PO-, -PO₂- oder -POR- ersetzt sind; und jede Präsenz von R⁷ unabhängig R', Halogen, NO₂ oder CN ist;
vorausgesetzt, dass:
für Verbindungen mit der Struktur 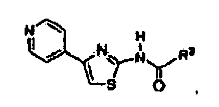 R³ nicht eine der folgende Gruppen ist: -CH₂(3-NHCOPh-phenyl), - CH₂-pyrrolidin, unsubstituiertes Benzyl, -CH₂-naphthyl, -CH₂CH₂-3-(4-Cl-phenyl)-1-phenyl-1-H-pyrazol-4-yl oder -CH₂(1,3-dioxoisoindol).

2. Verbindung nach Anspruch 1, wobei:
(1) R² Wasserstoff ist oder UₙR' ist, wobei n 1 ist und U eine C₁₋₆-Alkylidenkette ist, wobei eine oder zwei Methyleneinheiten optional und unabhängig durch O, NR, S oder C(O) ersetzt sind,
wobei vorzugsweise U -CH₂-, -CH₂CH₂-, -CH₂CH₂CH₂-, -CH₂CH₂CH₂CH₂'-, -CH₂O-, -CH₂S-, -CH₂NR-, -CH₂CH₂O-, -CH₂CH₂S-, -CH₂CH₂NR-, - CH₂CH₂CH₂O-, -CH₂CH₂CH₂S-, -CH₂CH₂CH₂NR-, -CH₂CH₂CH₂CH₂O-, - CH₂CH₂CH₂CH₂S-, -CH₂CH₂CH₂CH₂NR-, -CH₂CH₂OCH₂CH₂-, -(CH₂)₄NHCH₂-, - (CH₂)₃NHCH₂CH₂- oder -CH₂CH₂NHCH₂CH₂- ist und vorzugsweise R' ausgewählt ist aus Wasserstoff, C₁-C₄-Alkyl, optional substituiertem Tetrahydropyranyl, Pyrrolidinyl, Piperidinyl, Piperazinyl, Morpholinyl, Thiomorpholinyl, Pyridinyl, Phenyl oder Cyclohexyl, oder R und R', gemeinsam genommen mit dem Stickstoffatom, an das sie gebunden sind, einen optional substituierten 5- oder 6-gliedrigen heterocyclischen Ring bilden; oder
(2) Ar¹ Folgendes ist: 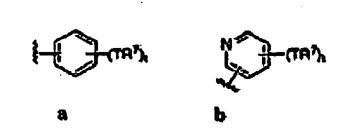 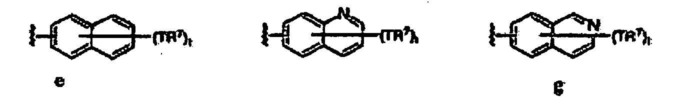 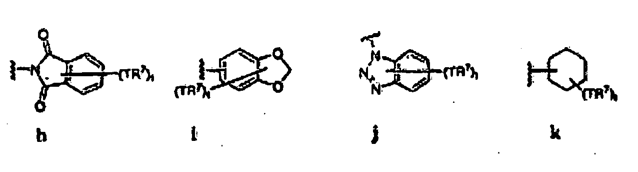 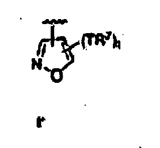 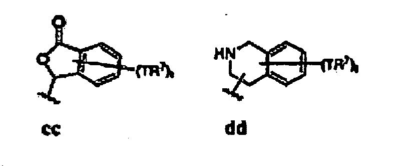 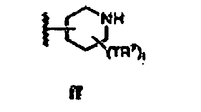 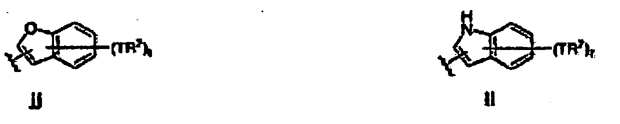 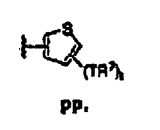
wobei t 0, 1, 2, 3, 4 oder 5 ist und wobei jedes Ar¹ an Q² durch ein substituierbares Stickstoff- oder Kohlenstoffatom gebunden ist, und wobei ein oder mehrere Wasserstoffatome auf jedem substituierbaren Stickstoff- oder Wasserstoffatom mit einer oder mehreren unabhängigen Präsenzen von TR⁷ substituiert ist beziehungsweise sind;
wobei
(a) T eine Bindung oder eine optional substituierte C₁₋₆-Alkylidenkette ist, wobei eine oder zwei Methyleneinheiten optional und unabhängig durch -O-, -NR-, -S-, -SO₂-, -COO-, -CO-, -OSO₂-, -NRSO₂-, -CONR- oder -SO₂NR- ersetzt sind, und R⁷R' oder Halogen ist; oder
(b) jede Präsenz von TR⁷ unabhängig -C₁₋₃-Alkyl, -OR', -SR', -CF₃, -OCF₃, -SCF₃, -F, -Cl, I, -Br, -COOR', -COR', - O(CH₂)₄N(R)(R'), -O(CH₂)₃N(R)(R'), -O(CH₂)₂N(R)(R'), - O(CH₂)N(R)(R'), -O(CH₂)₄CON(R)(R'), -O(CH₂)₃CON(R)(R'), - O(CH₂)₂CON(R)(R'), -O(CH₂)CON(R)(R'), -C(O)N(R)(R'), -(CH₂)₄OR', - (CH₂)₃OR', (CH₂)₂OR', -CH₂OR', optional substituiertes Phenyl oder Benzyl, -N(R)(R'), -(CH₂)₄N(R)(R'), -(CH₂)₃N(R)(R'), - (CH₂)₂N(R)(R'), -(CH₂)N(R)(R') oder SO₂N(R)(R'), NRSO₂R', CON(R)(R') oder -OSO₂R' ist.

3. Verbindung nach Anspruch 1, wobei R² und Q¹-R³, gemeinsam genommen mit dem intervenierenden Stickstoffatom die cyclische Gruppe 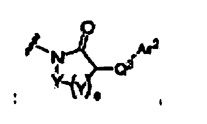 bilden, wobei s 1 oder 2 ist, jede Präsenz von Y unabhängig, wie Valenz und Stabilität zulassen, -CO-, -CS-, -SO₂-, -O-, -S-, NR⁵- oder -C(R⁵)₂- ist und R⁵ UₙR' ist; und Verbindungen der Formel I-A-ii, I-B-ii und I-C-ii bereitgestellt sind: 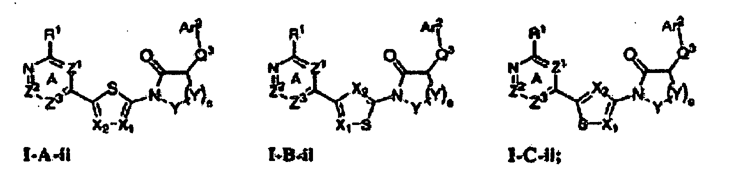 wobei
(1) Q³ eine direkte Bindung ist oder -(CHR⁶)_{q}-, -(CHR⁶)_{q}O-, -(CHR⁶)_{q}S-, -(CHR⁶)_{q}S(O)₂-, -(CHR⁶)_{q}S(O)-, -(CHR⁶)_{q}NR- oder - (CHR⁶)_{q}C(O)- ist, wobei q 0, 1, 2 oder 3 ist, und R⁶ R', - N(R)(R'), -(CH₂)₁₋₄N(R)(R'), -OR', -(CH₂)₁₋₄OR', -NR(CH₂)₁₋₄N(R)(R'), -NR(CH₂)₁₋₄SO₂R', NR(CH₂)₁₋₄COOR' oder NR(CH₂)₁₋₄COR' ist, oder zwei Präsenzen von R⁶, gemeinsam genommen mit den Atomen, an die sie gebunden sind, einen 3- bis 6-gliedrigen, gesättigten, teilweise ungesättigten oder vollständig ungesättigten Ring bilden; wobei zum Beispiel R⁶ CH₂OH, CH₂CH₂OH, OH, OMe, OEt, NH₂, NH(Me), NH(Et), N(Me)(Me), CH₂NH₂, CH₂CH₂NH₂, NHCO₂t-Butyl, Phenyl, Cyclopentyl, Methyl, Ethyl, Isopropyl, Cyclopropyl, NH(CH₂)₃NH₂, NH(CH₂)₂NH₂, NH(CH₂)₂NHEt, NHCH₂pyridyl, NHSO₂phenyl, NHC(O)CH₂C(O)Ot-butyl, NHC(O)CH₂NH₃ und NHCH₂-Imidazol-4-yl ist;
(2) Ar² Folgendes ist: 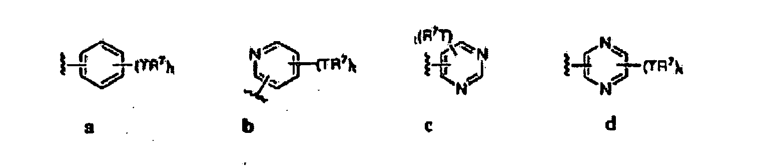 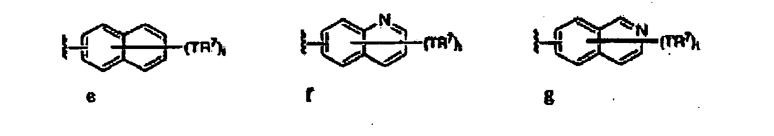 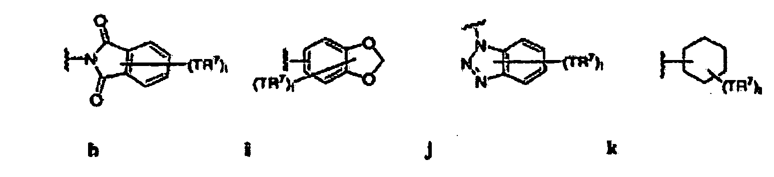 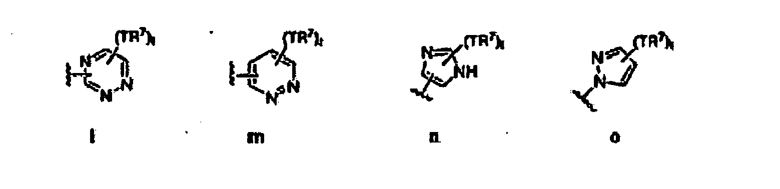 wobei t 0, 1, 2, 3, 4 oder 5 ist und wobei jedes jedes Ar² an Q³ durch ein substituierbares Stickstoff- oder Kohlenstoffatom gebunden ist, und wobei ein oder mehrere Wasserstoffatome auf 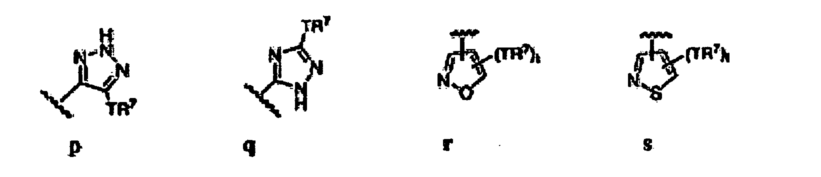 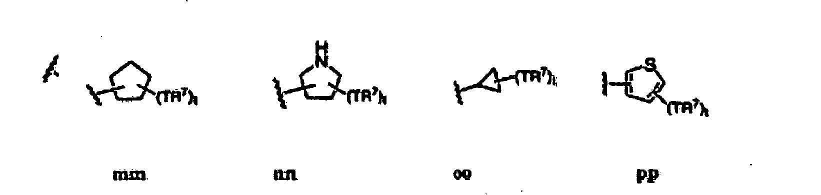 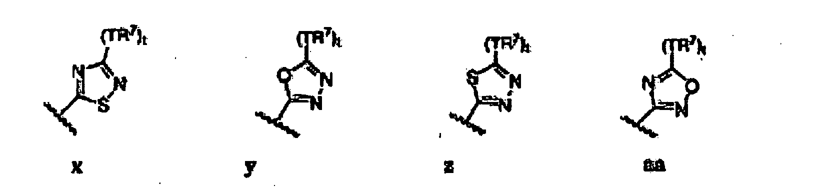 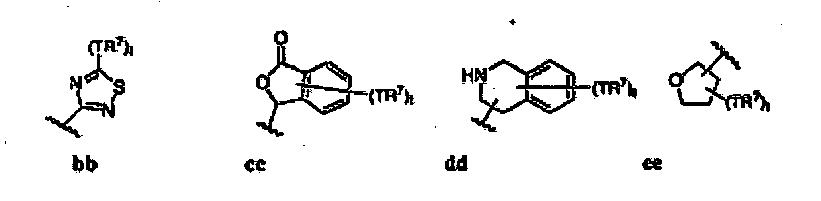 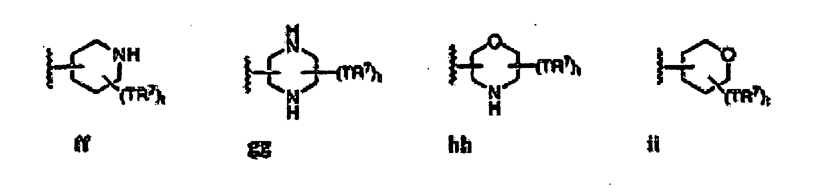 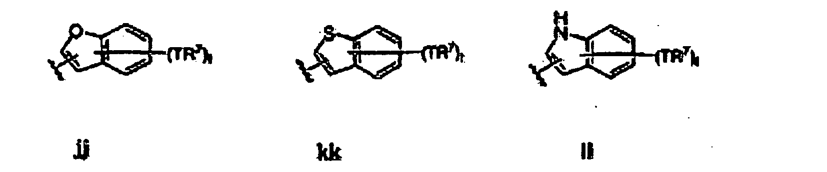 jedem substituierbaren Stickstoff- oder Wasserstoffatom mit einer oder mehreren unabhängigen Präsenzen von TR⁷ substituiert ist beziehungsweise sind; wobei zum Beispiel Ar² a, b, e, g, h, i, j, k, n, r, cc, dd, ff, jj, 11 oder pp ist; wobei:
(a) T eine Bindung oder eine optional substituierte C₁₋₆-Alkylidenkette ist, wobei eine oder zwei Methyleneinheiten optional und unabhängig durch -O-, -NR-, -S-, -SO₂-, -COO-, -CO-, -OSO₂-, -NRSO₂-, -CONR- oder -SO₂NR- ersetzt sind, und R⁷ R' oder Halogen ist; oder
(b) jede Präsenz von TR⁷ unabhängig -C₁₋₃-Alkyl, -OR', -SR', -CF₃, -OCF₃, -SCF₃, -F, -Cl, I, -Br, -COOR', -COR', - O(CH₂)₄N(R)(R'), -O(CH₂)₃N(R)(R'), -O(CH₂)₂N(R)(R'), - O(CH₂)N(R)(R'), -O(CH₂)₄CON(R)(R'), -O(CH₂)₃CON(R)(R'), - O(CH₂)₂CON(R)(R'), -O(CH₂)CON(R)(R'), -C(O)N(R)(R'), -(CH₂)₄OR', - (CH₂)₃OR', -(CH₂)₂OR', -CH₂OR', optional substituiertes Phenyl oder Benzyl, -N(R)(R'), -(CH₂)₄N(R)(R'), -(CH₂)₃N(R)(R'), - (CH₂)₂N(R)(R'), -(CH₂)N(R)(R') oder SO₂N(R)(R'), NRSO₂R', CON(R)(R') oder -OSO₂R' ist; oder
(3) R⁵ Wasserstoff, (CH₂)₃OR', (CH₂)₂OR', (CH₂)OR', (CH₂)₃N(R')₂, (CH₂)₂N(R')₂, (CH₂)N(R')₂ oder C₁₋₄-aliphatisch ist.

4. Verbindung nach Anspruch 1, wobei jede Präsenz von R¹ unabhängig Wasserstoff, Halogen, optional substituiert C₁-C₄-aliphatisch, OR, SR oder N(R)₂ ist; wobei vorzugsweise jede Präsenz von R¹ unabhängig Wasserstoff, Halogen, -CH₃-, CH₂CH₃-, - OH, -OCH₃, -SCH₃, -SCH₃, -NH₂, -N(CH₃)₂, -N(CH₂CH₃)₂, NH(CH₂)₂NHCH₃, NH(cyclopropyl, NH(CH₂)cyclopropyl oder NH(CH₂)₂N(CH₃)₂ ist.

5. Verbindung nach Anspruch 1, wobei jede Präsenz von R^{Z} unabhängig Wasserstoff, Halogen, C₁-C₄-aliphatisch, OH, OR' oder N(R)(R') ist;
wobei vorzugsweise jede Präsenz von R^{Z} unabhängig Wasserstoff, Halogen, Me, OH, OMe, NH₂ oder N(Me)₂ ist.

6. Verbindung nach Anspruch 1, wobei R⁴-Gruppen jeweils unabhängig Wasserstoff, C₁-C₆-aliphatisch, CN, COR, C(=0)OR, C(=O)N(R)₂ oder Halogen sind.

7. Verbindung nach Anspruch 1, wobei 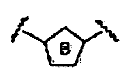 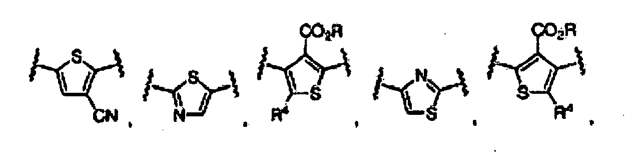 oder 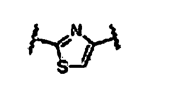 ist.

8. Verbindung nach Anspruch 1, wobei die Verbindung eine der Formeln XIV, XV, XVI, XVII, XVIII oder XIX hat:

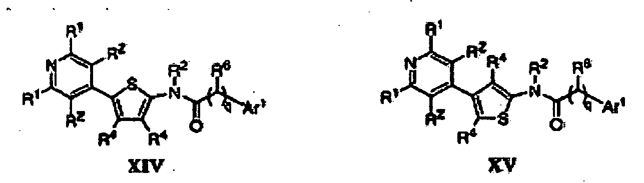

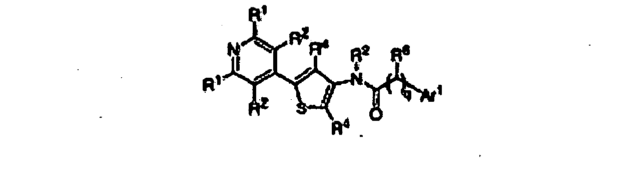

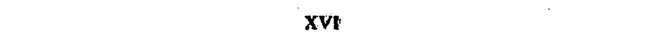

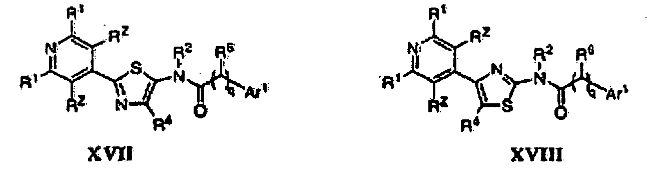

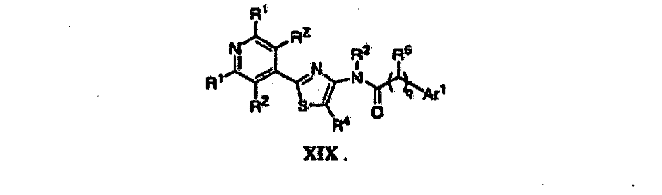

9. Verbindung nach Anspruch 8, wobei Verbindungsvariable aus einer oder mehreren der folgenden Gruppen ausgewählt sind:
a) jede Präsenz von R¹ ist unabhängig Wasserstoff, Halogen, optional substituiert C₁-C₄-aliphatisch, OR, SR oder N(R)₂;
b) jede Präsenz von R¹ ist unabhängig Wasserstoff, Halogen, -CH₃-, CH₂CH₃, -OH, -OCH₃, -SCH₃, -NH₂, -N(CH₃)₂, - N(CH₂CH₃)₂, NH(CH₂)₂NHCH₃, NH(cyclopropyl, NH(CH₂)cyclopropyl oder NH(CH₂)₂N(CH₃)₂;
c) jede Präsenz von R^{Z} ist unabhängig Wasserstoff, Halogen, optional substituiert C₁-C₄-aliphatisch, OH, O(R') oder N(R)(R');
d) jede Präsenz von R^{Z} ist unabhängig Wasserstoff, Halogen, Me, OH, OMe, NH₂ oder N(Me)₂;
e) R² ist Wasserstoff oder UₙR', wobei n 1 ist und U -CH₂-, -CH₂CH₂-, -CH₂CH₂CH₂-, -CH₂CH₂CH₂CH₂-, -CH₂O-, -CH₂S-, -CH₂NR-, - CH₂CH₂O-, -CH₂CH₂S-, -CH₂CH₂NR-, -CH₂CH₂CH₂O-, -CH₂CH₂CH₂S-, - CH₂CH₂CH₂NR-, -CH₂CH₂CH₂CH₂O-, -CH₂CH₂CH₂CH₂S-, -CH₂CH₂CH₂CH₂NR-, - CH₂CH₂OCH₂CH₂-, -(CH₂)₄NHCH₂-, -(CH₂)₃NHCH₂CH₂- oder -CH₂CH₂NHCH₂CH₂- ist, und R'-Gruppen Wasserstoff, C₁₋₄-Alkyl, optional substituiertes Tetrahydropyranyl, Pyrrolidinyl, Piperidinyl, Piperazinyl, Morpholinyl, Thiomorpholinyl, Pyridinyl, Phenyl oder Cyclohexyl sind, oder R und R', gemeinsam genommen mit dem Stickstoffatom, an das sie gebunden sind, einen optional substituierten 5- oder 6-gliedrigen, heterocyclischen Ring bilden;
f) jede Präsenz von R⁴ unabhängig Wasserstoff, C₁-C₆-aliphatisch, CN, COR, COOR, CON(R)₂ oder Halogen ist;
g) q 1, 2 oder 3 ist;
h) R⁶ R', -N(R)(R'), -(CH₂)₁₋₄N(R)(R'), -OR', -(CH₂)₁₋₄OR', - NR(CH₂)₁₋₄N(R)(R'), -NR (CH₂)₁₋₄SO₂R', -NR(CH₂)₁₋₄COOR' oder -NR(CH₂)₁₋₄COR' ist, oder zwei Präsenzen von R⁶, gemeinsam genommen mit den Atomen, an die sie gebunden sind, einen 3- bis 6-gliedrigen, gesättigten, teilweise ungesättigten oder vollständig ungesättigten Ring bilden;
i) R⁶ CH₂OH, CH₂CH₂OH, OH, OMe, OEt, NH₂, NH(Me), NH(Et), N(Me)(Me), CH₂NH₂, CH₂CH₂NH₂, NHCO₂t-Butyl, Phenyl, Cyclopentyl, Methyl, Ethyl, Isopropyl, Cyclopropyl, NH(CH₂)₃NH₂, NH(CH₂)₂NH₂, NH(CH₂)₂NHEt, NHCH₂pyridyl, NHSO₂phenyl, NHC(O)CH₂C(O)Ot-butyl, NHC(O)CH₂NH₃ und NHCH₂-Imidazol-4-yl ist;
j) Ar¹ Ring a, b, e, g, h, i, j, k, r, cc, dd, ff, jj, 11 oder pp ist, wobei t 0, 1, 2 oder 3 ist und T eine Bindung oder eine optional substituierte C₁₋₆-Alkylidenkette ist, wobei eine oder zwei Methyleneinheiten optional und unabhängig durch -O-, - NR-, -S-, -SO₂-, -COO-, -CO-, -OSO₂-, -NRSO₂-, -CONR- oder -SO₂NR- ersetzt sind, und R⁷ R' oder Halogen ist; oder
k) Ar¹ Ring a, b, e, g, h, i, j, k, r, cc, dd, ff, jj, 11 oder pp ist, wobei t 0, 1, 2 oder 3 ist und jede Präsenz von TR⁷ unabhängig -C₁₋₃-Alkyl, -OR', -SR', -CF₃, -OCF₃, -SCF₃, -F, -Cl, I, -Br, -COOR', -COR', -O(CH₂)₄N(R)(R'), -O(CH₂)₃N(R)(R'), - O(CH₂)₂N(R)(R'), -O(CH₂)N(R)(R'), -O(CH₂)₄CON(R)(R'), - O(CH₂)₃CON(R)(R'), -O(CH₂)₂CON(R)(R'), -O(CH₂)CON(R)(R'), - C(O)N(R)(R'), -(CH₂)₄OR', -(CH₂)₃OR', -(CH₂)₂OR', -CH₂OR', optional substituiertes Phenyl oder Benzyl, -N(R)(R'), -(CH₂)₄N(R)(R'), - (CH₂)₃N(R)(R'), -(CH₂)₂N(R)(R'), -(CH₂)N(R)(R') oder SO₂N(R)(R'), NRSO₂R', CON(R)(R') oder -OSO₂R' ist.

10. Verbindung nach Anspruch 8, wobei q 1 ist und Ar¹ optional substituiertes Phenyl ist und eine Verbindung der allgemeinen Formel XIV-A bis XIX-A bereitgestellt ist: 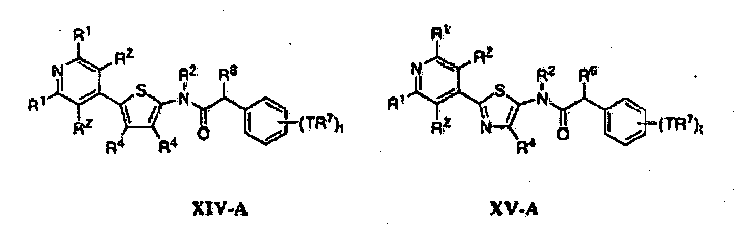 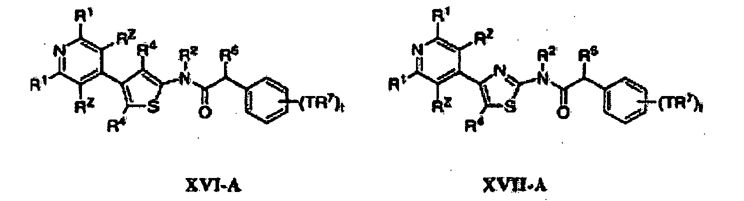 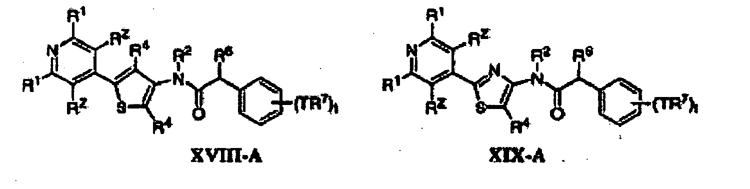 wobei:
jede Präsenz von R¹ Wasserstoff ist;
jede Präsenz von R^{Z} Wasserstoff ist;
R² Wasserstoff ist oder UₙR' ist, wobei n 1 ist und U -CH₂-, -CH₂CH₂-, -CH₂CH₂CH₂-, -CH₂CH₂CH₂CH₂-, -CH₂O-, -CH₂S-, -CH₂NR-, - CH₂CH₂O-, -CH₂CH₂S-, -CH₂CH₂NR-, -CH₂CH₂CH₂O-, -CH₂CH₂CH₂S-, - CH₂CH₂CH₂NR-, -CH₂CH₂CH₂CH₂O-, -CH₂CH₂CH₂CH₂S-, -CH₂CH₂CH₂CH₂NR-, - CH₂CH₂OCH₂CH₂-, -(CH₂)₄NHCH₂-, -(CH₂)₃NHCH₂CH₂- oder -CH₂CH₂NHCH₂CH₂- ist und R'-Gruppen Wasserstoff, C₁-C₄-Alkyl, optional substituiertem Tetrahydropyranyl, Pyrrolidinyl, Piperidinyl, Piperazinyl, Morpholinyl, Thiomorpholinyl, Pyridinyl, Phenyl oder Cyclohexyl sind, oder R und R', gemeinsam genommen mit dem Stickstoffatom, an das sie gebunden sind, einen optional substituierten 5- oder 6-gliedrigen heterocyclischen Ring bilden;
jede Präsenz von R⁴ unabhängig Wasserstoff, C₁-C₄-aliphatisch, CN, COR, COOR, CON(R)₂ oder Halogen ist;
R⁶ R', -N(R)(R'), -(CH₂)₁₋₄N(R)(R'), -OR', -(CH₂)₁₋₄OR', - NR(CH₂)₁₋₄N(R)(R'), -NR(CH₂)₁₋₄SO₂R', NR(CH₂)₁₋₄COOR' oder NR(CH₂)₁₋₄COR' ist; und
t 0, 1, 2 oder 3 ist und jede Präsenz von TR⁷ unabhängig -C₁₋₃-Alkyl, -OR', -SR', -CF₃, -OCF₃, -SCF₃, -F, -Cl, I, -Br, -COOR', -COR', -O(CH₂)₄N(R)(R'), -O(CH₂)₃N(R)(R'), -O(CH₂)₂N(R)(R'), - O(CH₂)N(R)(R'), -O(CH₂)₄CON(R)(R'), -O(CH₂)₃CON(R)(R'), - O(CH₂)₂CON(R)(R'), -O(CH₂)CON(R)(R'), -C(O)N(R)(R'), -(CH₂)₄OR', - (CH₂)₃OR', -(CH₂)₂OR', -CH₂OR', optional substituiertes Phenyl oder Benzyl, -N(R)(R'), -(CH₂)₄N(R)(R'), -(CH₂)₃N(R)(R'), - (CH₂)₂N(R)(R'), - (CH₂)N(R)(R') oder SO₂N(R)(R'), NRSO₂R', CON(R)(R') oder -OSO₂R' ist.

11. Verbindung nach Anspruch 1, wobei
(1) R² und Q¹-R³, gemeinsam genommen mit den Atomen, an die sie gebunden sind, eine 5-gliedrige cyclische Gruppe bilden, und Verbindungen haben die allgemeine Formel XX bis XXXI: 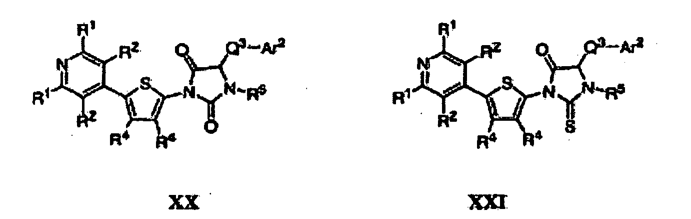 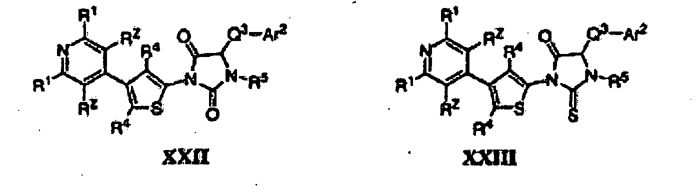
(2) R² und Q¹-R³, gemeinsam genommen mit den Atomen, an die sie gebunden sind, eine 6-gliedrige cyclische Gruppe bilden, und Verbindungen haben die allgemeine Formel XXXII bis XXXVII: 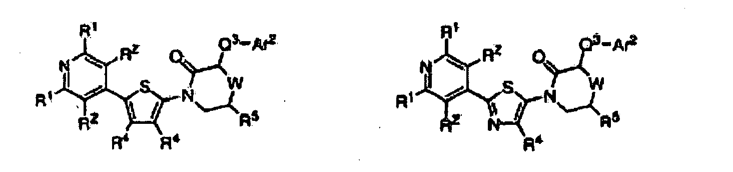 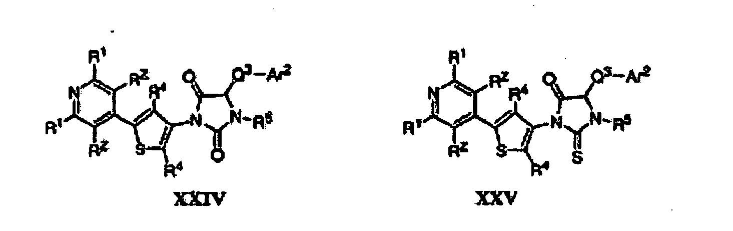 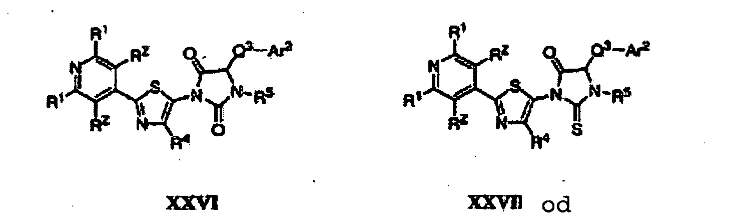 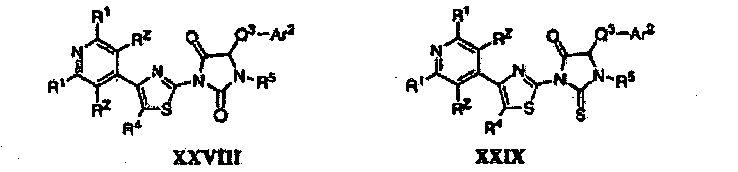 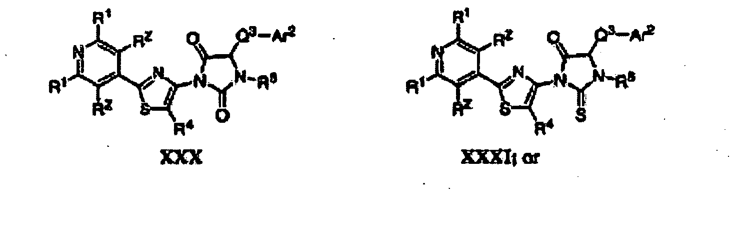
wobei W O, NR⁵ oder CHR⁵ ist.

12. Verbindung nach Anspruch 11, wobei Verbindungsvariable aus einer oder mehreren der folgenden Gruppen ausgewählt sind:
a) jede Präsenz von R¹ ist unabhängig Wasserstoff, Halogen, optional substituiert C₁-C₄-aliphatisch, OR, SR oder N(R)₂;
b) jeder Präsenz von R^{z} ist unabhängig Wasserstoff, Halogen, optional substituiert C₁-C₄-aliphatisch, OH, OR' oder 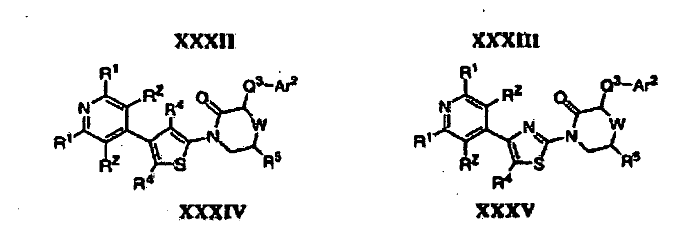 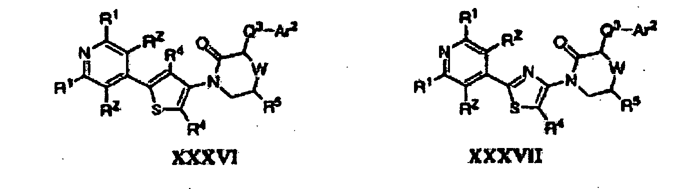 N(R)(R');
c) jede Präsenz von R⁴ ist unabhängig Wasserstoff, C₁-C₆-aliphatisch, CN, COR, COOR, CON(R)₂ oder Halogen;
d) R⁵ ist Wasserstoff, (CH₂)₃OR', (CH₂)₂OR', (CH₂)OR', (CH₂)₃N(R')₂, (CH₂)₂N(R')₂, (CH₂)N(R')₂ oder C₁₋₄-aliphatisch;
e) Q³ ist eine direkte Bindung oder ist -(CHR⁶)_{q}-, - (CHR⁶)_{q}O-, -(CHR⁶)_{q}S-, -(CHR⁶)_{q}S(O)₂-, -(CHR⁶)_{q}S(O)-, -(CHR⁶)_{q}NR- oder -(CHR⁶)_{q}C(O)-, wobei q 0, 1, 2 oder 3 ist; und
f) Ar² ist a, b, e, g, h, i, j, k, n, r, cc, dd, ff, jj, 11 oder pp, wobei t 0, 1, 2 oder 3 ist, und T eine Bindung oder eine optional substituierte C₁-C₆-Alkylidenkette ist, wobei eine oder zwei Methyleneinheiten optional und unabhängig durch -O-, -NR-, -S-, -SO₂-, -COO-, -CO-, OSO₂-, NRSO₂-, -CONR- oder -SO₂NR- ersetzt sind, und R⁷ R' oder Halogen ist.

13. Verbindung nach Anspruch 11, wobei Verbindungsvariable aus einer oder mehreren der folgenden Gruppen ausgewählt sind:
a) jede Präsenz von R¹ ist unabhängig Wasserstoff, Halogen, -CH₃-, -CH₂CH₃-, -OH, -OCH₃, -SCH₃, NH₂, -N(CH₃)₂, - N(CH₂CH₃)₂, NH(CH₂)₂NHCH₃, NH(cyclopropyl), NH(CH₂)cyclopropyl oder NH(CH₂)₂N(CH₃)₂;
b) jede Präsenz von R^{Z} ist unabhängig Wasserstoff, Halogen, Me, OH, OMe, NH₂ oder N(Me)₂;
c) jede Präsenz von R⁴ ist unabhängig Wasserstoff ist C₁-C₆-aliphatisch, CN, COR, COOR, CON(R)₂ oder Halogen;
d) R⁵ ist Wasserstoff (CH₂)₃OR', (CH₂)₂OR', (CH₂)OR', (CH₂)₃N(R')₂, (CH₂)₂N(R')₂, (CH₂)N(R')₂ oder C₁₋₄-aliphatisch;
e) Q³ ist eine direkte Bindung oder ist -(CHR⁶)_{q}-, - (CHR⁶)_{q}O-, (CHR⁶)_{q}S-, -(CHR⁶)_{q}S(O)₂-, -(CHR⁶)_{q}S(O)-, -(CHR⁶)_{q}NR- oder -(CHR⁶)_{q}C(O)-, wobei q 0, 1, 2 oder 3 ist; und
f) Ar² ist Ring a, b, e, g, h, i, j, k, n, r, cc, dd, ff, jj, 11 oder pp, wobei t 0, 1, 2 oder 3 ist, und jede Präsenz von TR⁷ ist unabhängig -C₁₋₃-Alkyl, -OR', -SR', -CF₃, -OCF₃, -SCF₃, -F, -Cl, I, -Br, -COOR', -COR', -O(CH₂)₄N(R)(R'), -O(CH₂)₃N(R)(R'), - O(CH₂)₂N(R)(R'), -O(CH₂)N(R)(R'), -O(CH₂)₄CON(R)(R'), - O(CH₂)₃CON(R)(R'), -O(CH₂)₂CON(R)(R'), -O(CH₂)CON(R)(R'), - C(O)N(R)(R'), -(CH₂)₄OR', -(CH₂)₃OR', -(CH₂)₂OR', -CH₂OR', optional substituiertes Phenyl oder Benzyl, -N(R)(R'), -(CH₂)₄N(R)(R'), - (CH₂)₃N(R)(R'), -(CH₂)₂N(R)(R'), -(CH₂)N(R)(R') oder SO₂N(R)(R'), NRSO₂R', CON(R)(R') oder -OSO₂R'.

14. Verbindung nach Anspruch 11, wobei Ar² optional substituiertes Phenyl ist und Verbindungen der allgemeinen Formel XX-A bis XXXVII bereitgestellt sind:

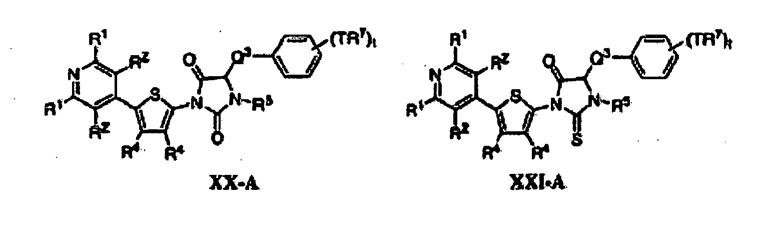

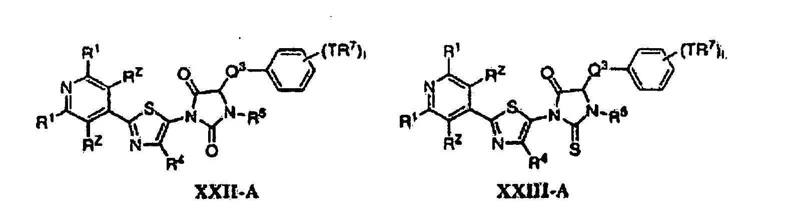

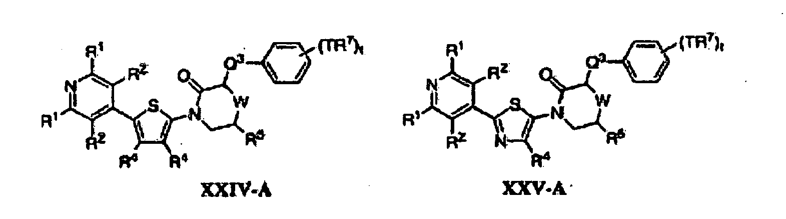

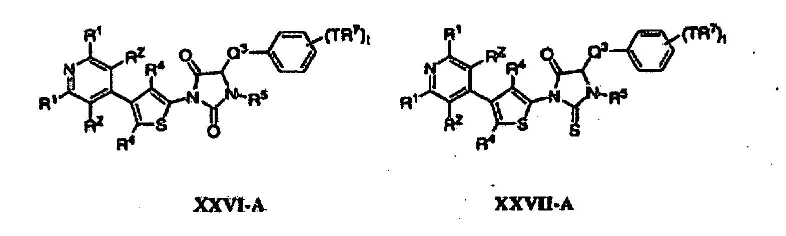

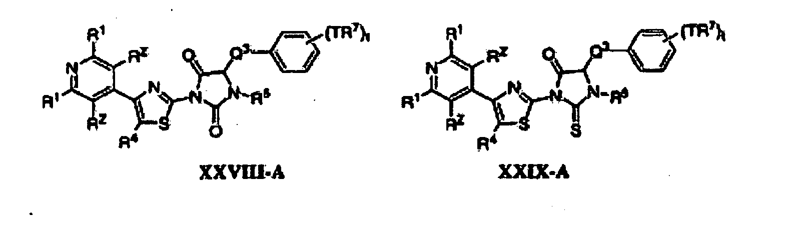

15. Verbindung nach Anspruch 14, wobei Verbindungsvariable ausgewählt sind aus:
jede Präsenz von R¹ ist Wasserstoff;
jede Präsenz von R^{Z} ist Wasserstoff;
jede Präsenz von R⁴ ist unabhängig Wasserstoff, C₁-C₆-aliphatisch, CN, COR, COOR, CON(R)₂ oder Halogen;
R⁵ ist Wasserstoff (CH₂)₃OR', (CH₂)₂OR¹, (CH₂)OR', (CH₂)₃N(R')₂, (CH₂)₂N(R')₂, (CH₂)N(R')₂ oder C₁₋₄-aliphatisch; 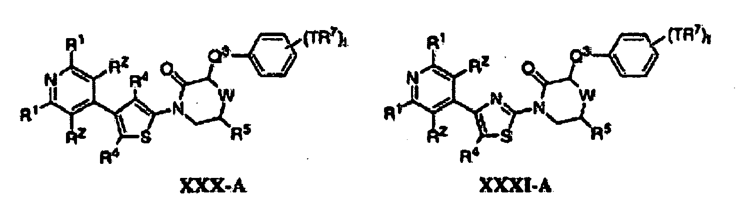 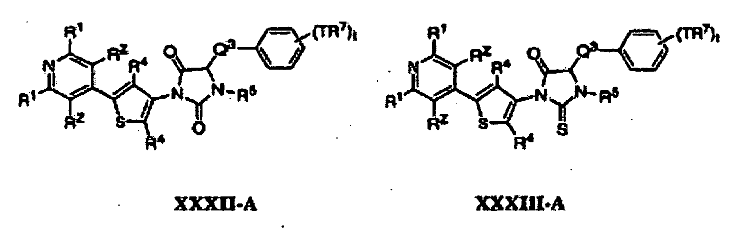 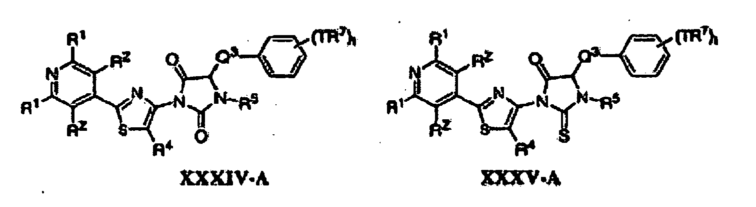 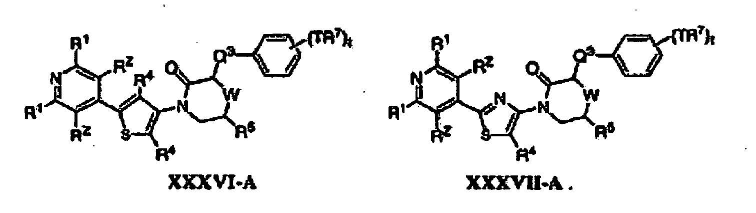
Q³ ist eine direkte Bindung oder ist -(CHR⁶)_{q}-, -(CHR⁶)_{q}O-, -(CHR⁶)_{q}S-, -(CHR⁶)_{q}S(O)₂-, -(CHR⁶)_{q}S(O)-, -(CHR⁶)_{q}NR- oder - (CHR⁶)_{q}C(O)- ist, wobei q 0, 1, 2 oder 3 ist; und
t ist 0, 1, 2 oder 3, und jede Präsenz von TR⁷ ist unabhängig -C₁₋₃-Alkyl, -OR', -SR', -CF₃, -OCF₃, -SCF₃-, -F, -Cl, I, -Br, - COOR', -COR', -O(CH₂)₄N(R)(R'), -O(CH₂)₃N(R)(R'), - O(CH₂)₂N(R)(R'), -O(CH₂)N(R)(R'), -O(CH₂)₄CON(R)(R'), - O(CH₂)₃CON(R)(R'), -O(CH₂)₂CON(R)(R'), -O(CH₂)CON(R)(R'), - C(O)N(R)(R'), -(CH₂)₄OR', -(CH₂)₃OR', -(CH₂)₂OR', -CH₂OR', optional substituiertes Phenyl oder Benzyl, -N(R)(R'), -(CH₂)₄N(R)(R'), - (CH₂)₃N(R)(R'), -(CH₂)₂N(R)R'), -(CH₂)N(R)(R') oder SO₂N(R)(R'), NRSO₂R', CON(R)(R') oder -OSO₂R'.

16. Verbindung nach Anspruch 1 mit einer der folgenden Strukturen:

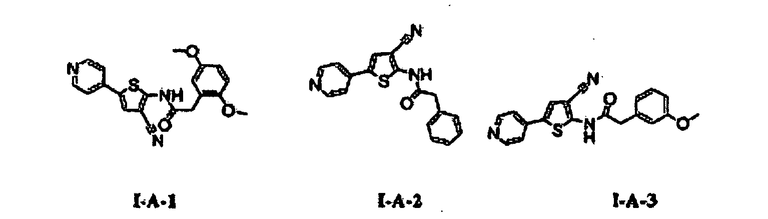

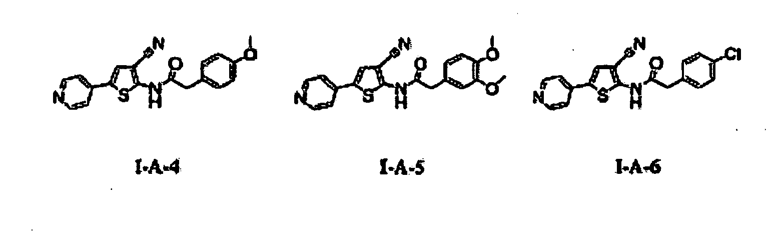

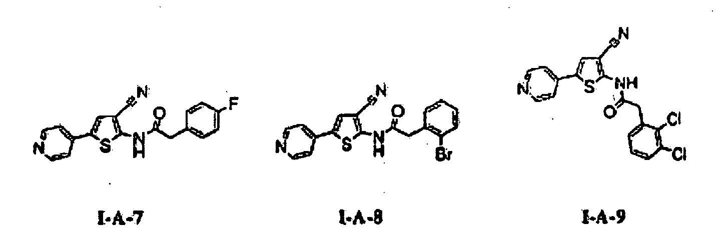

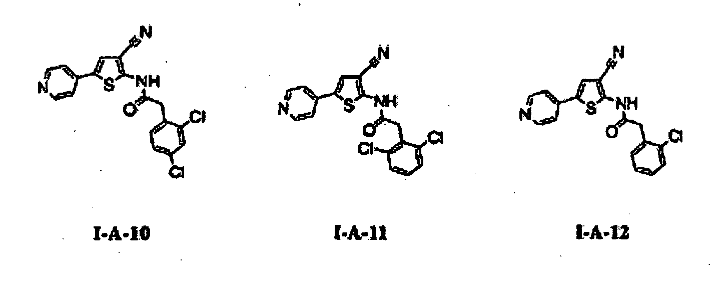

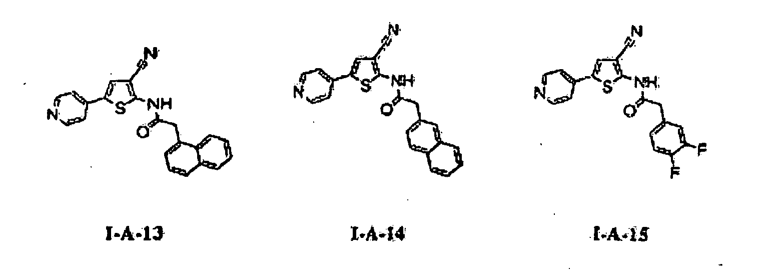

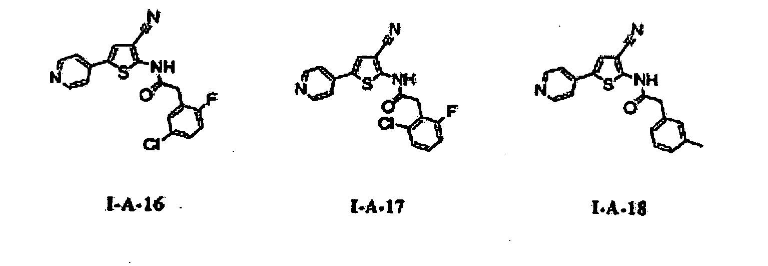

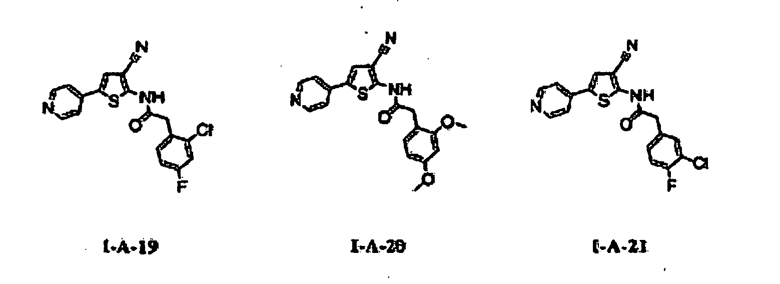

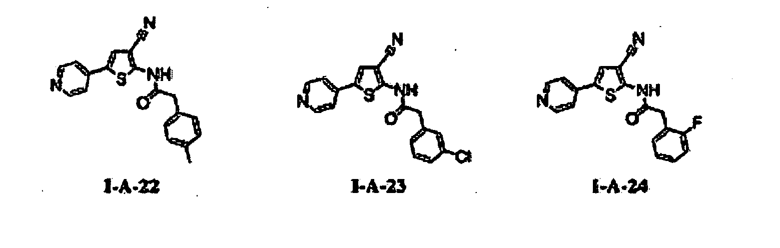

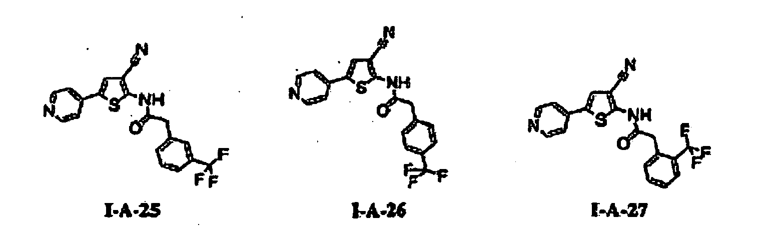

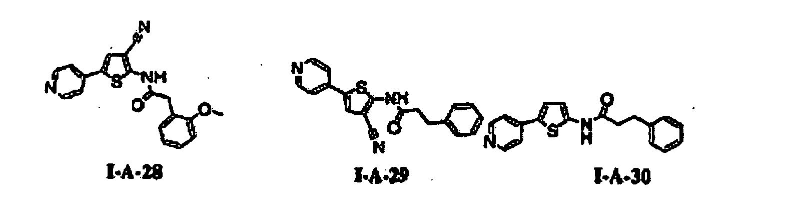

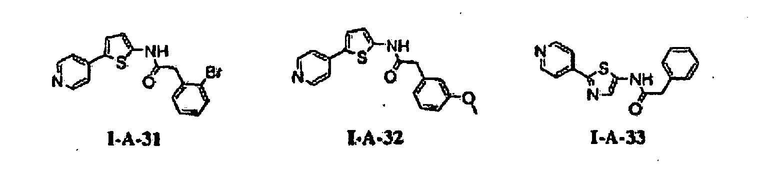

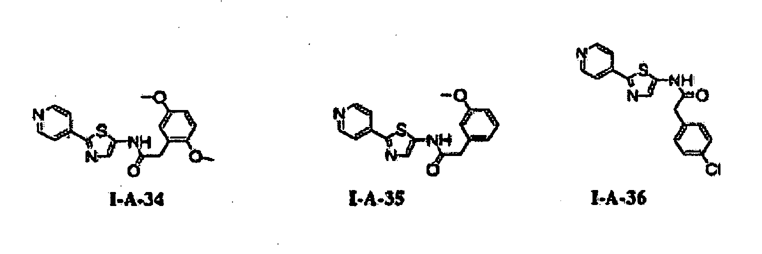

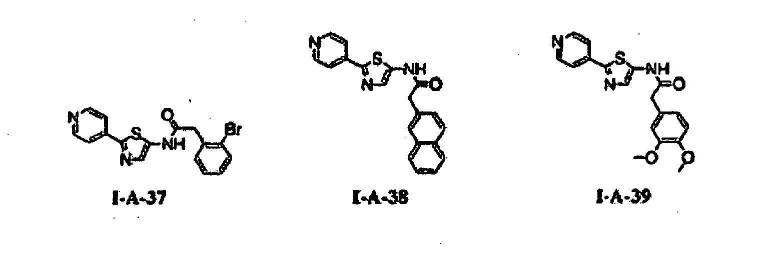

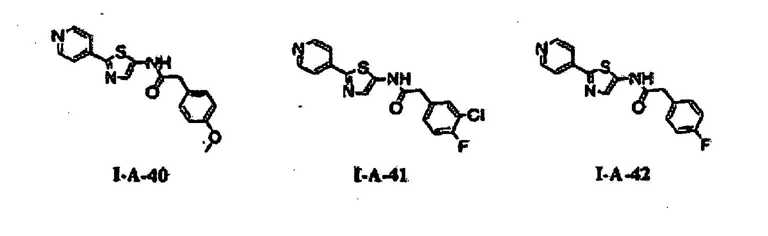

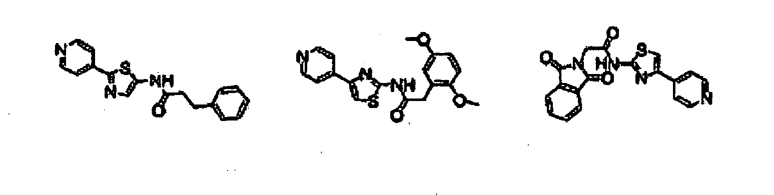

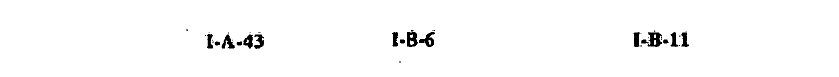

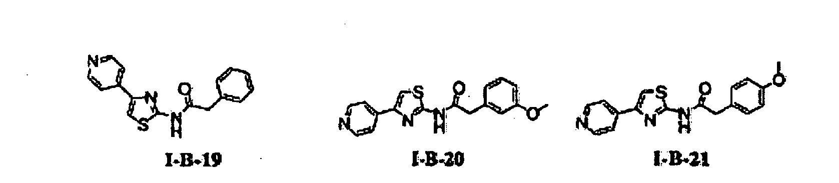

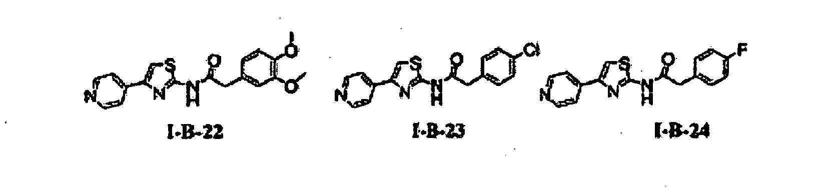

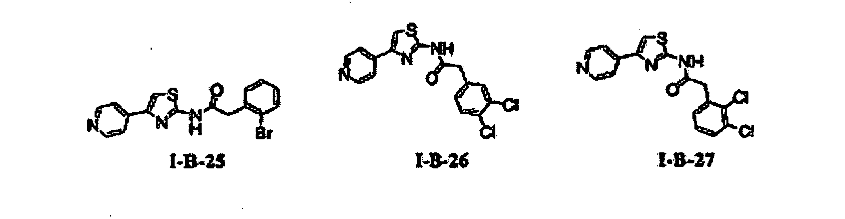

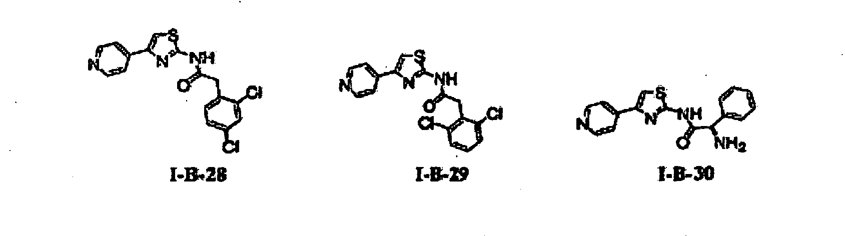

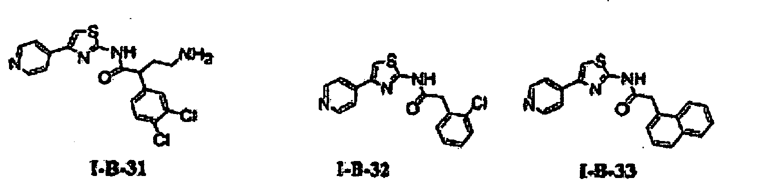

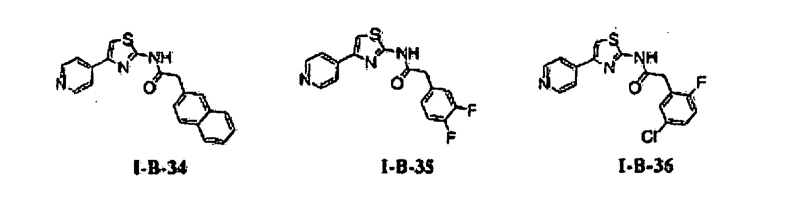

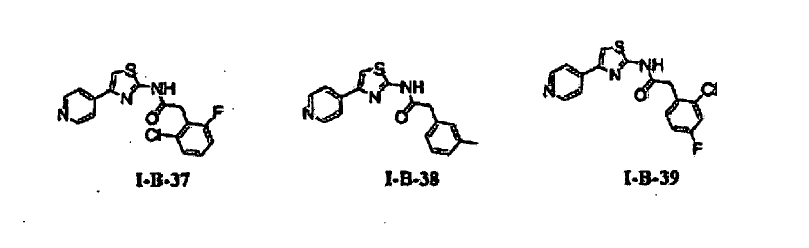

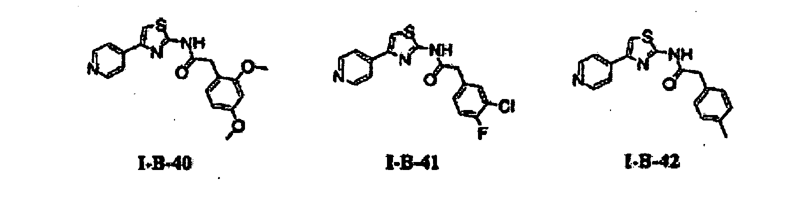

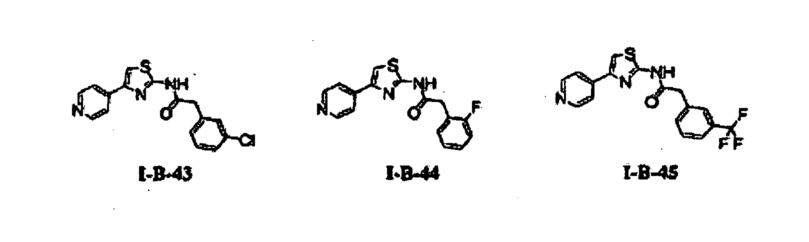

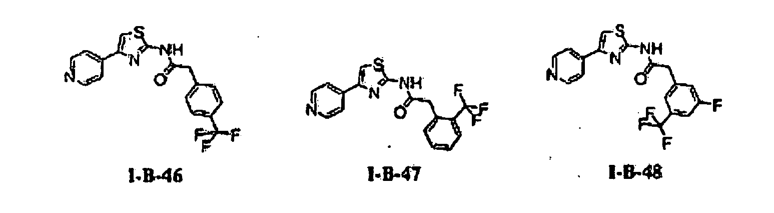

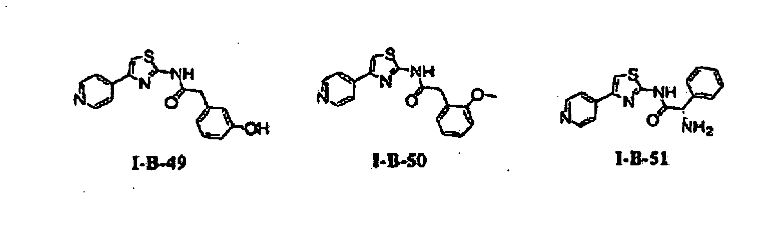

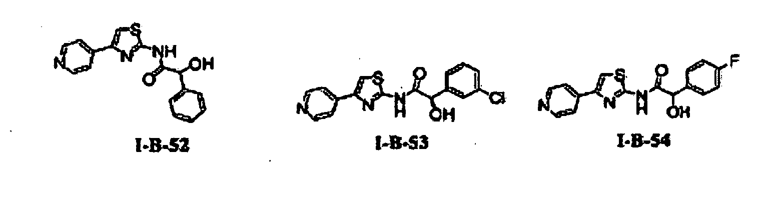

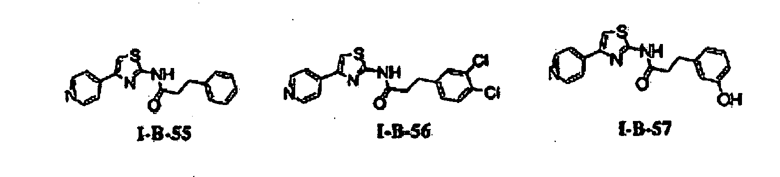

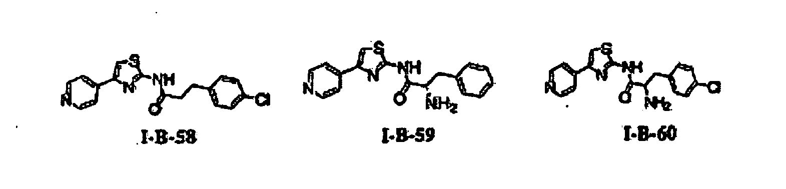

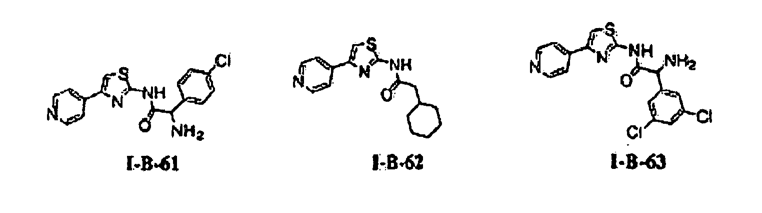

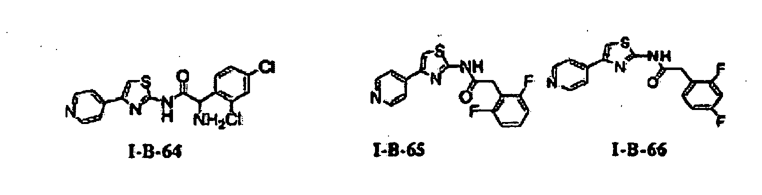

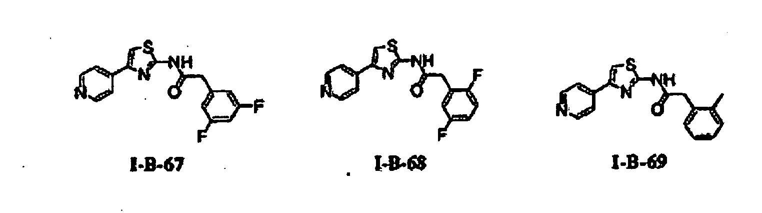

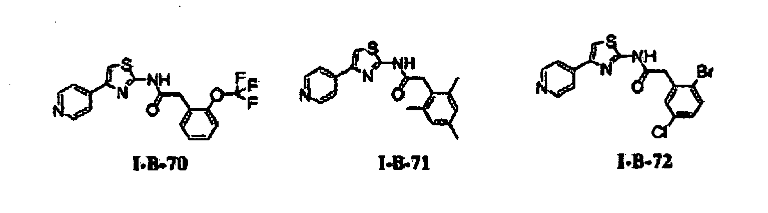

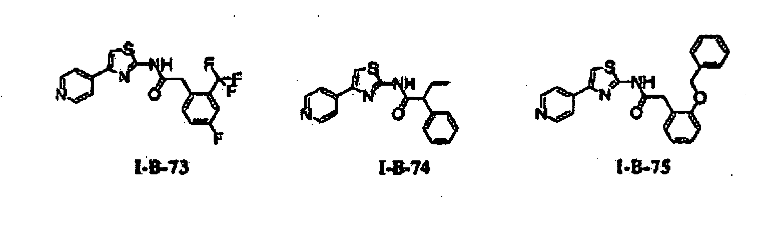

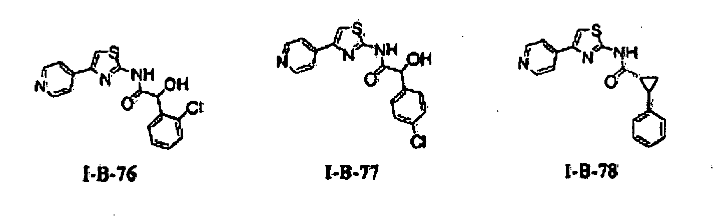

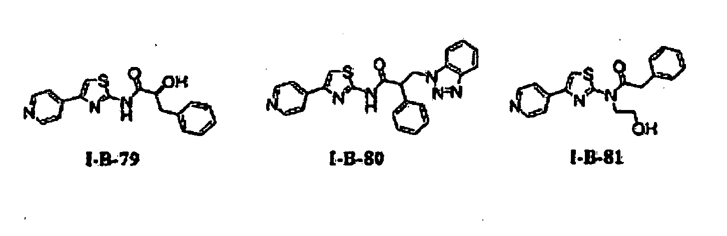

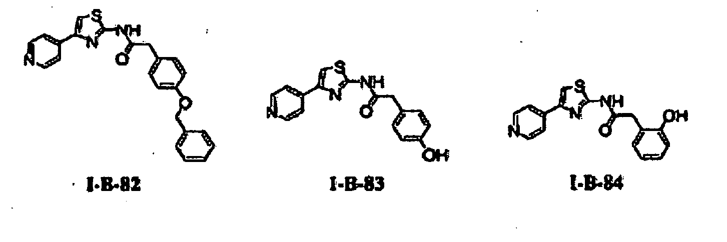

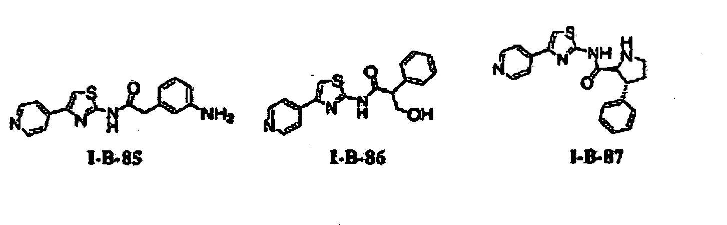

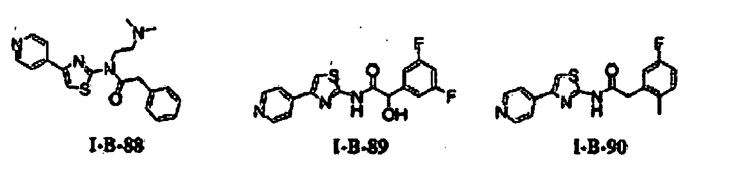

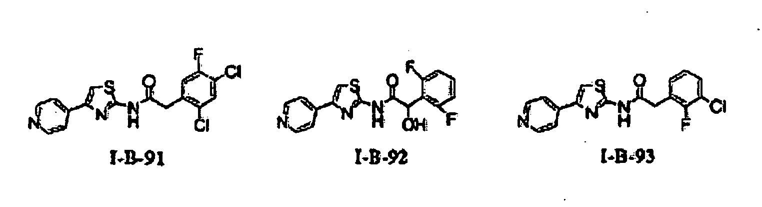

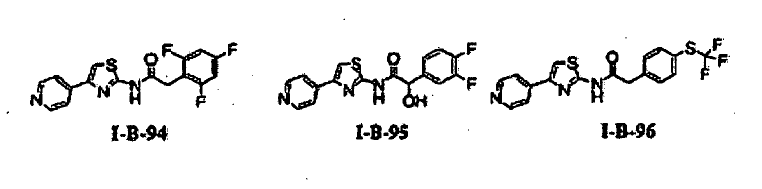

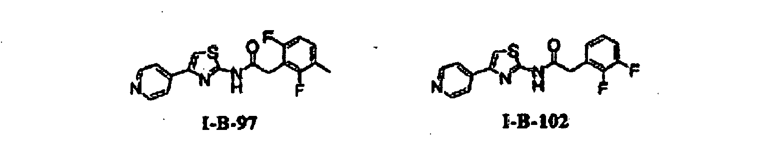

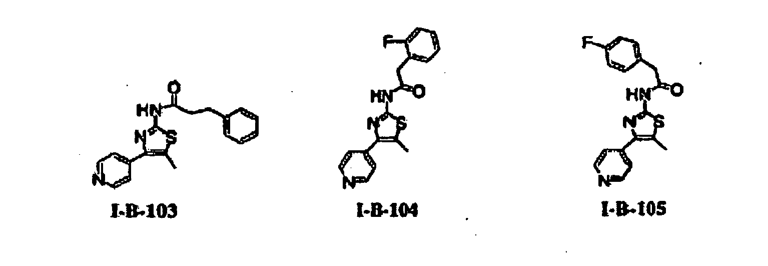

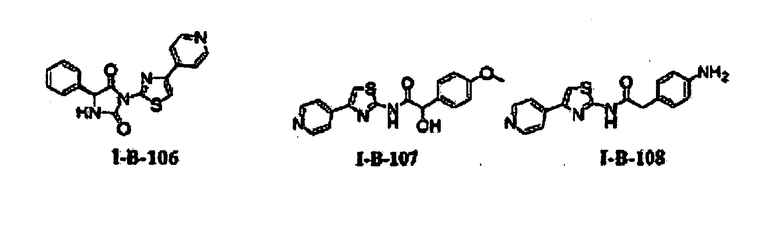

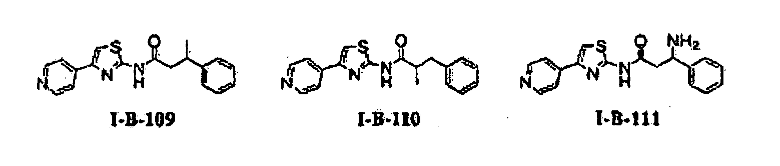

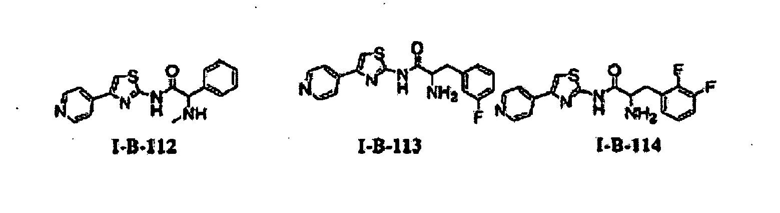

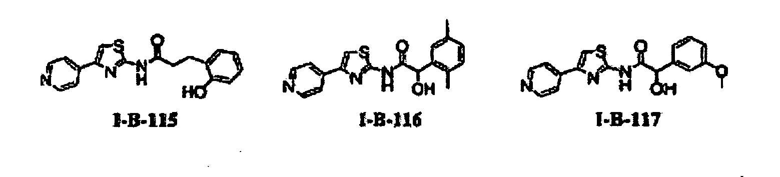

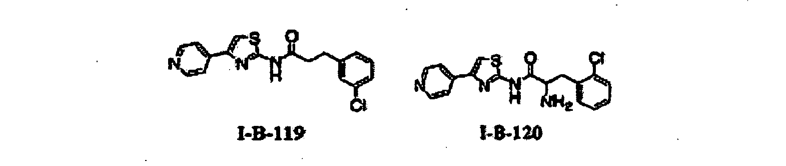

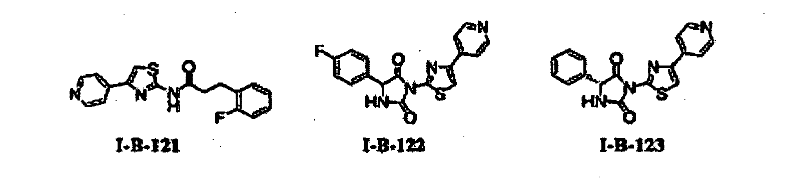

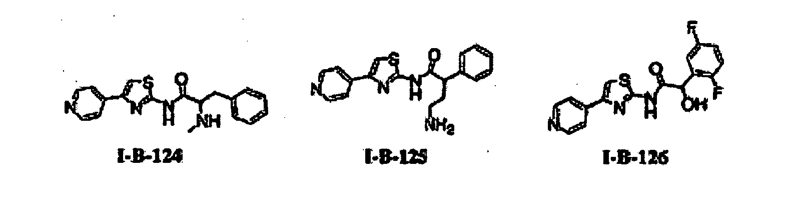

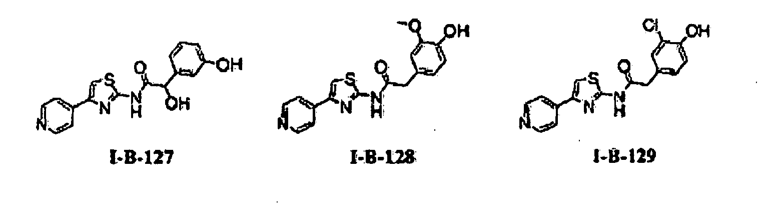

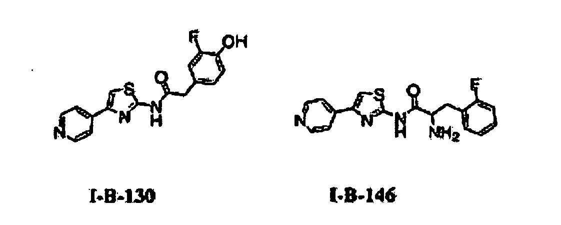

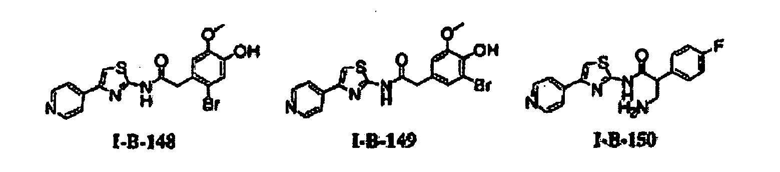

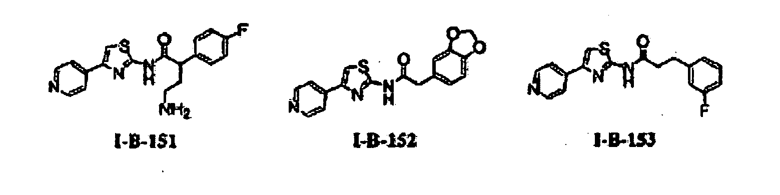

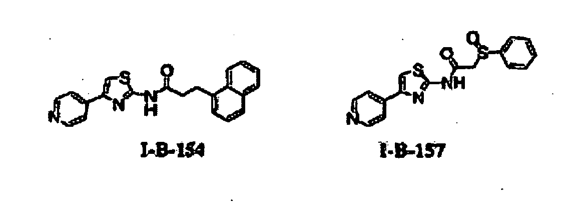

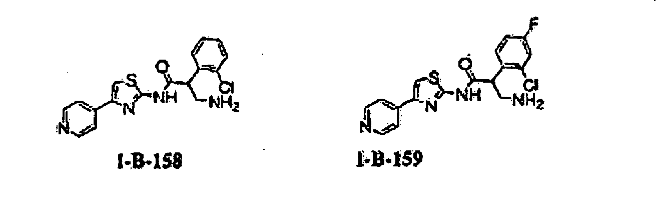

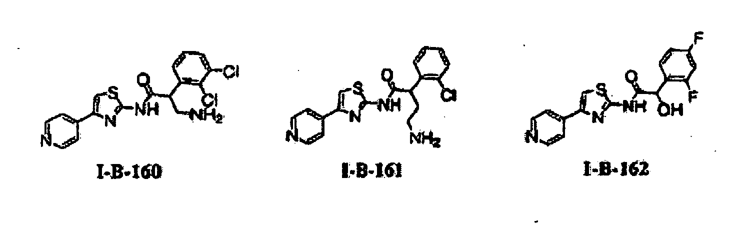

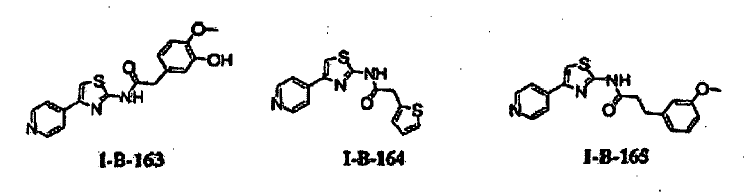

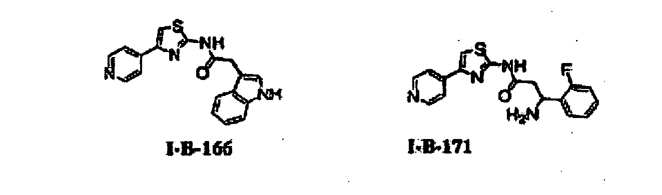

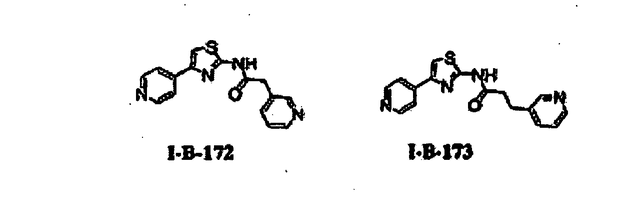

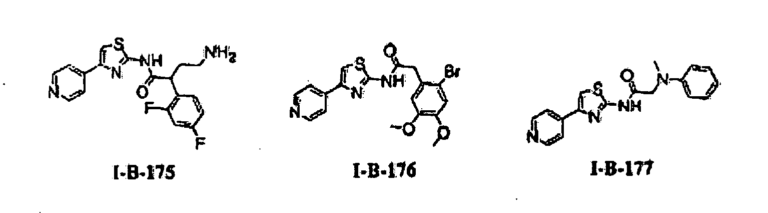

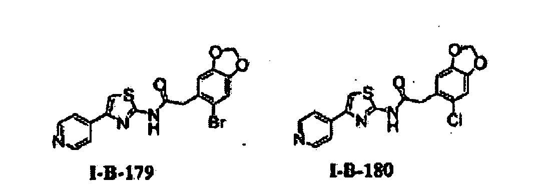

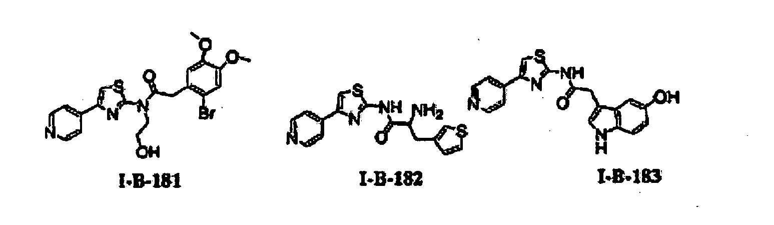

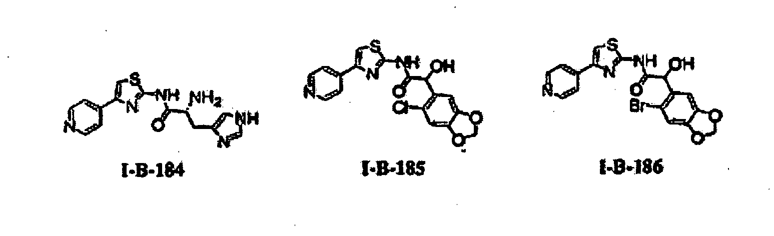

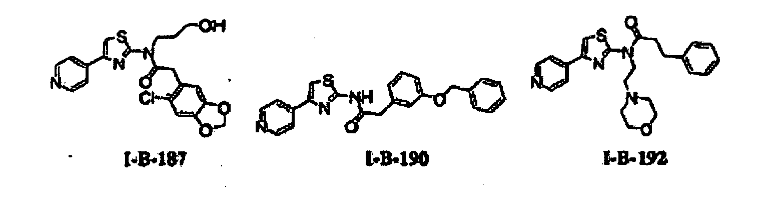

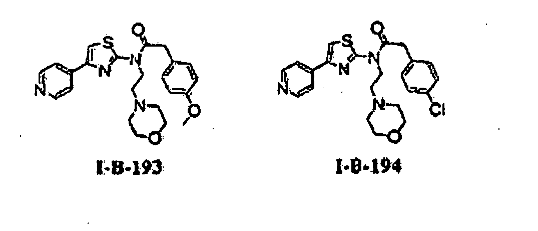

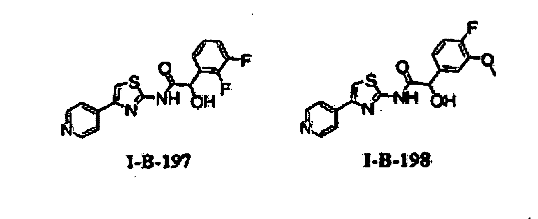

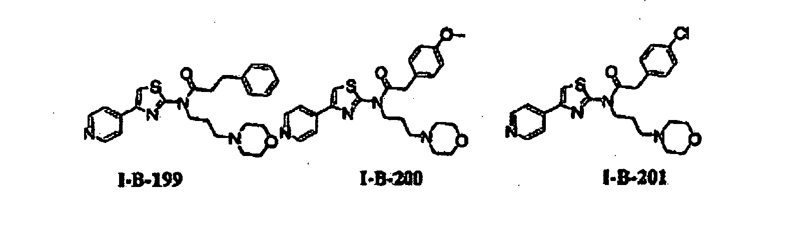

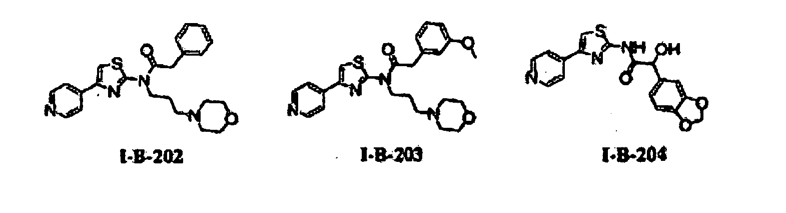

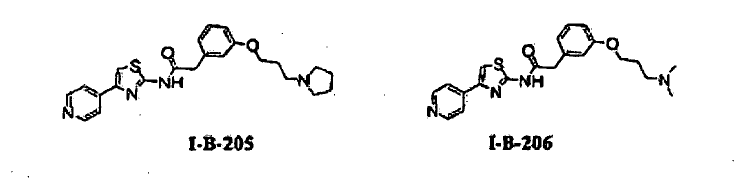

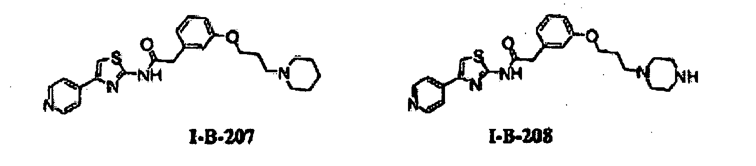

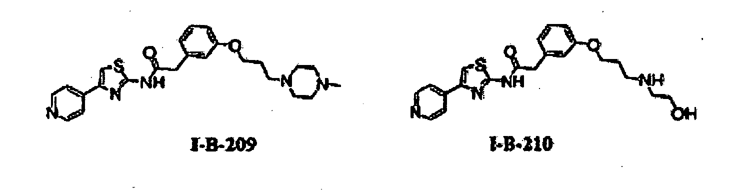

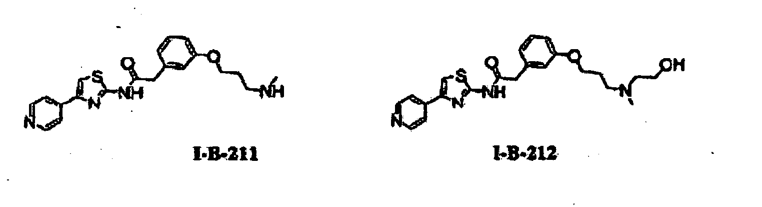

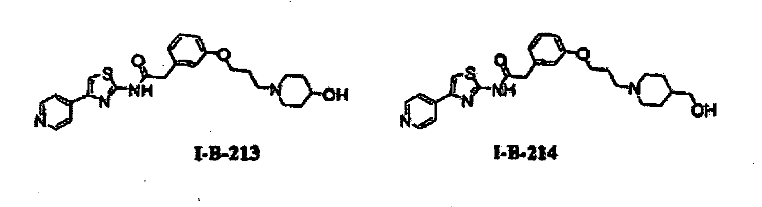

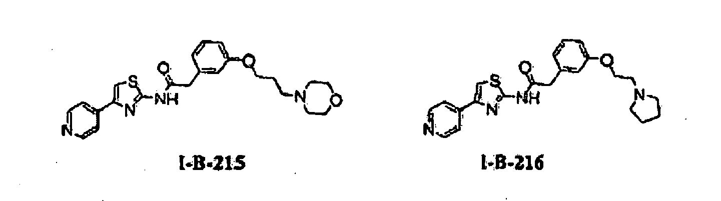

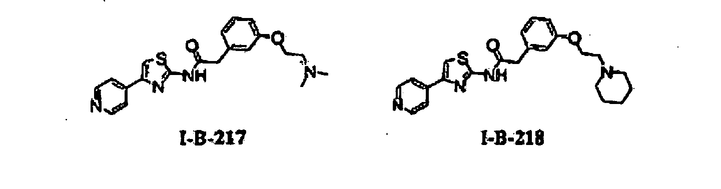

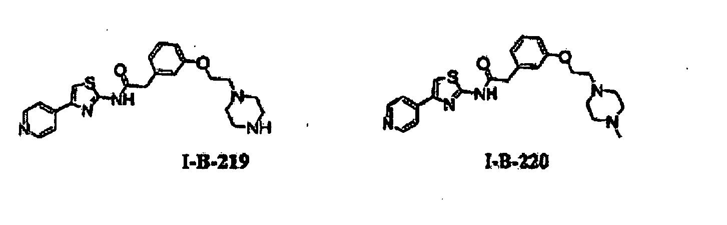

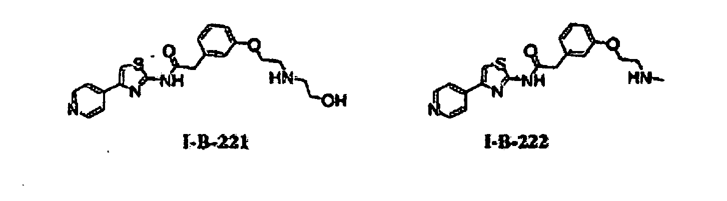

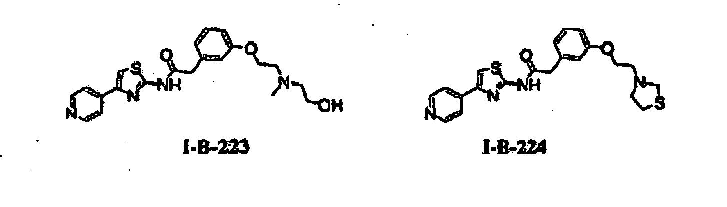

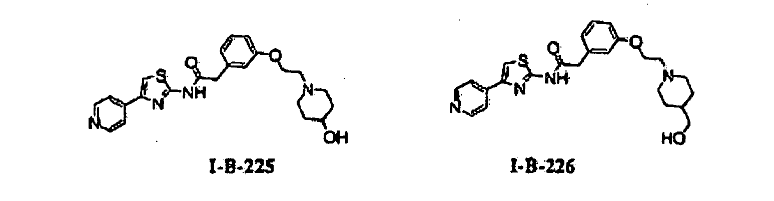

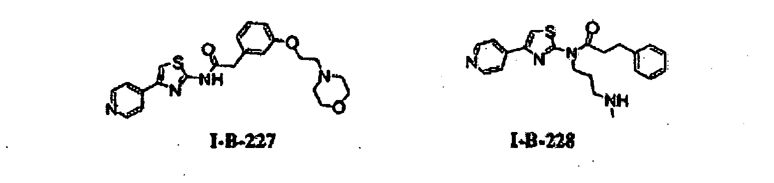

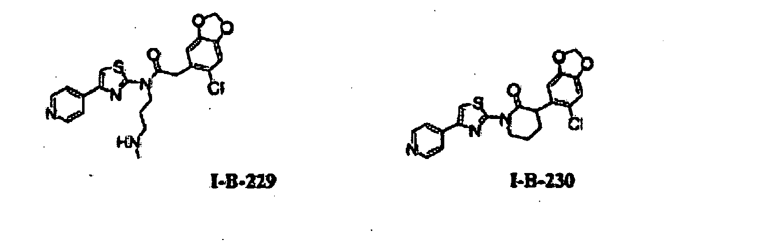

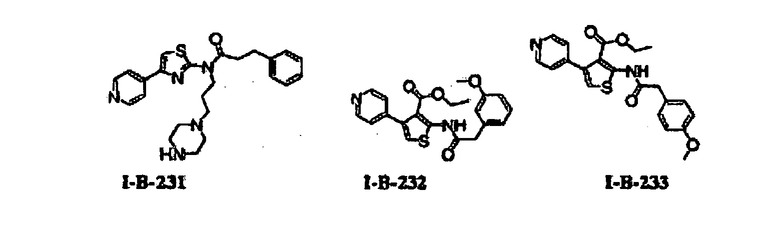

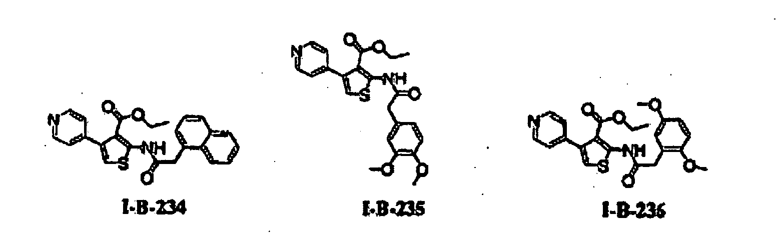

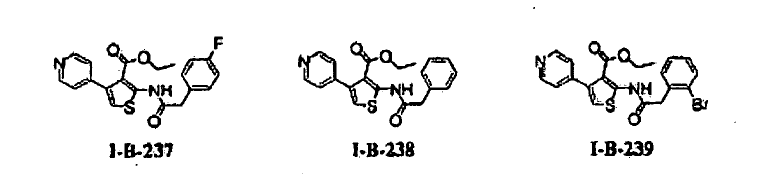

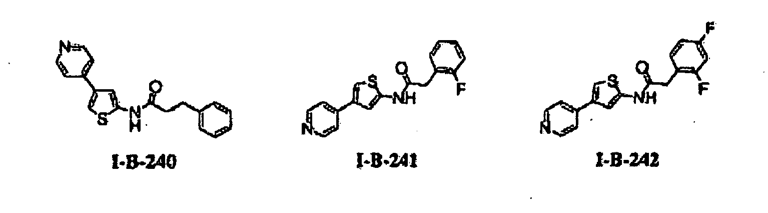

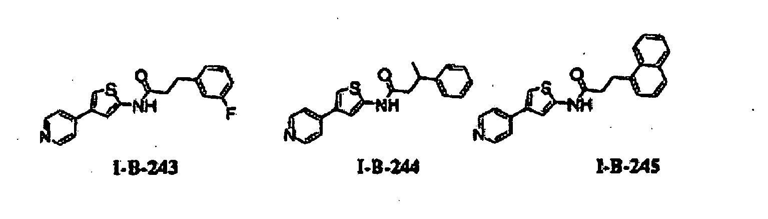

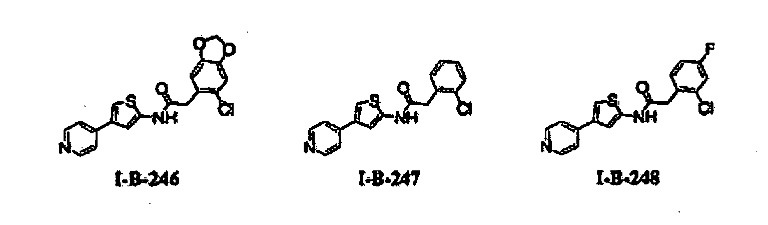

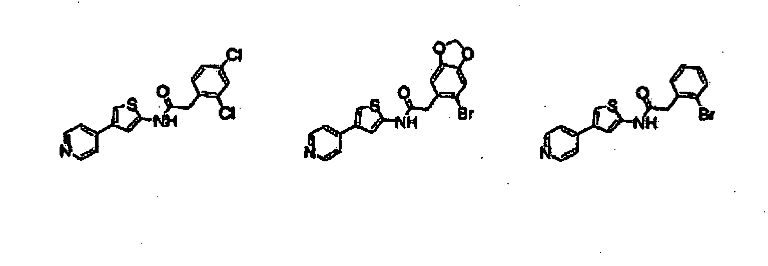

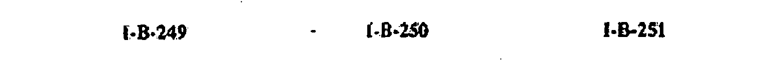

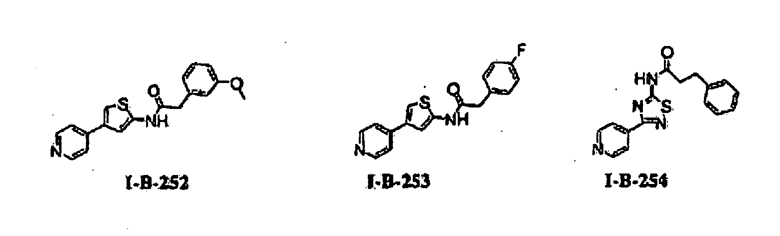

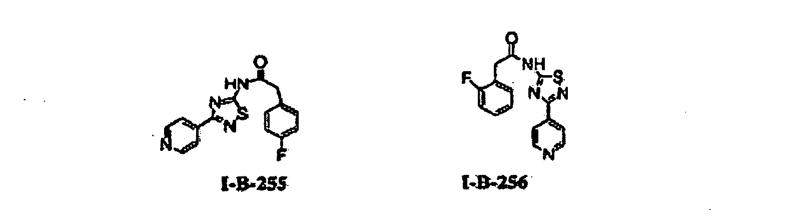

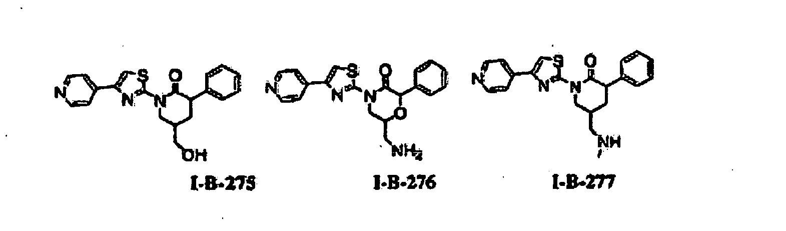

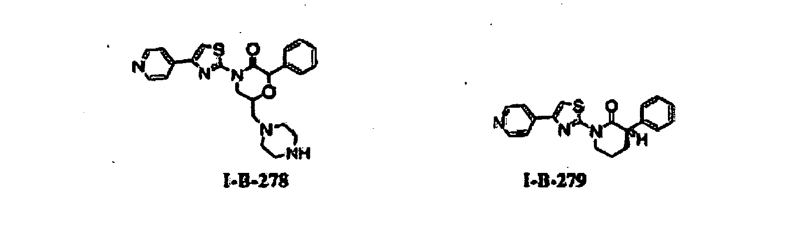

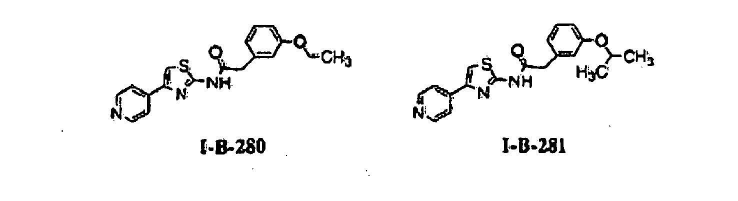

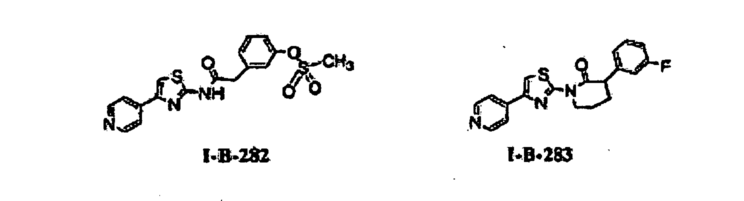

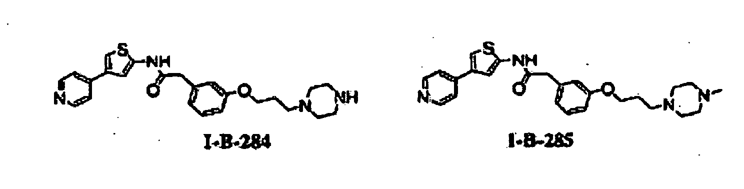

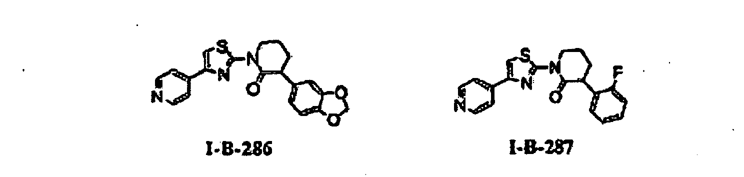

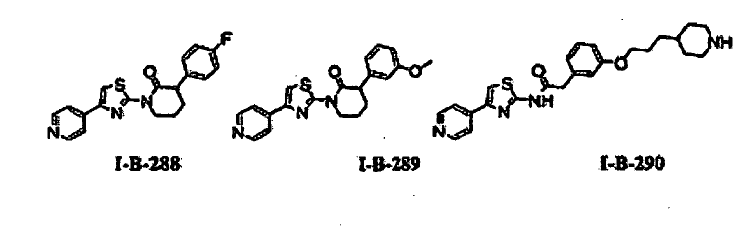

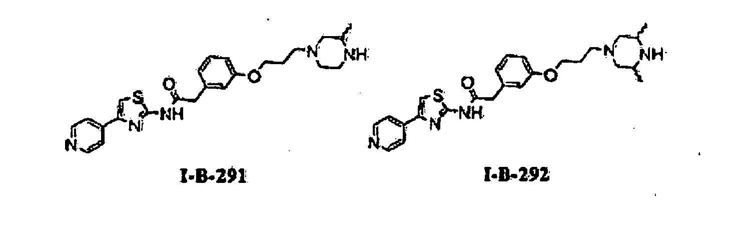

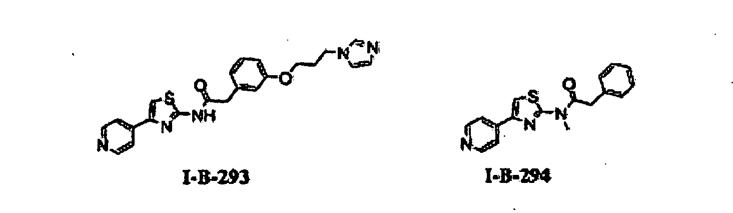

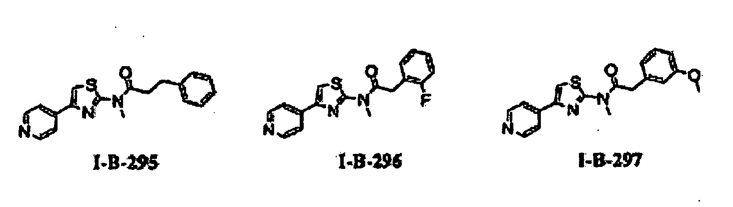

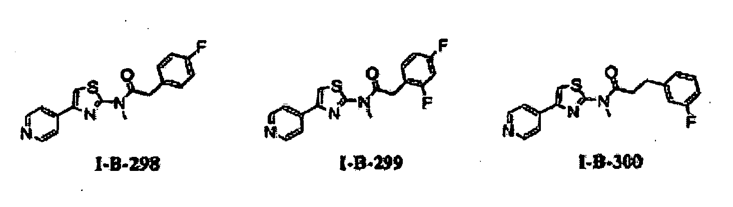

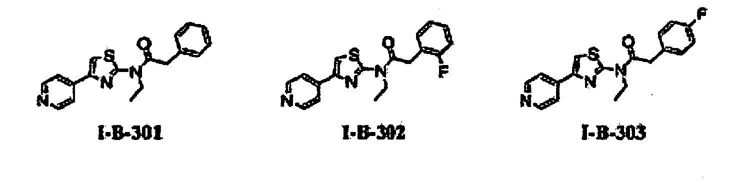

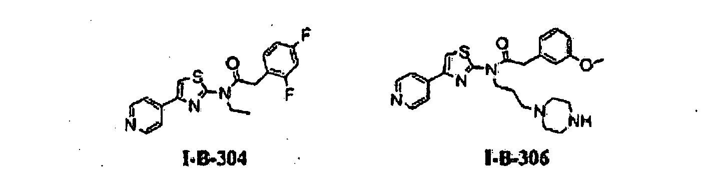

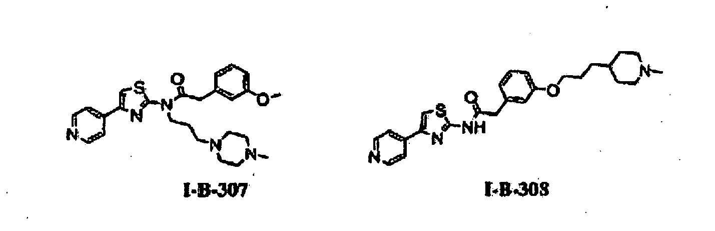

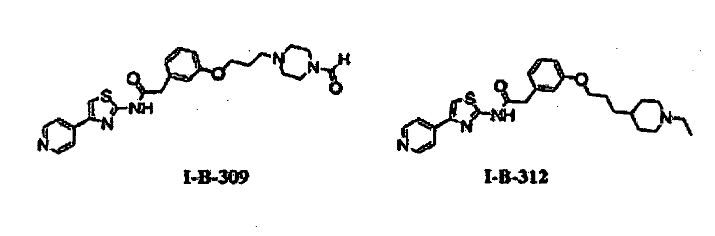

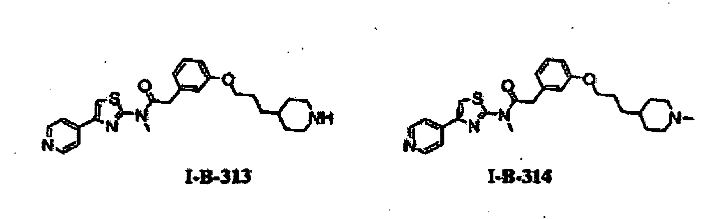

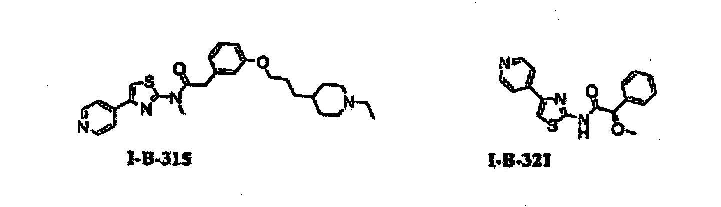

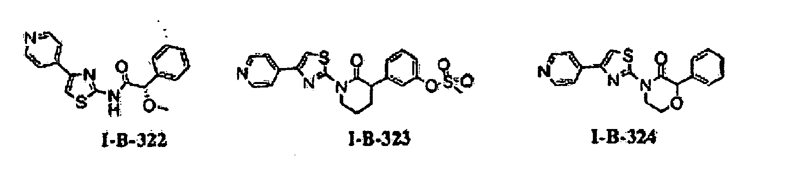

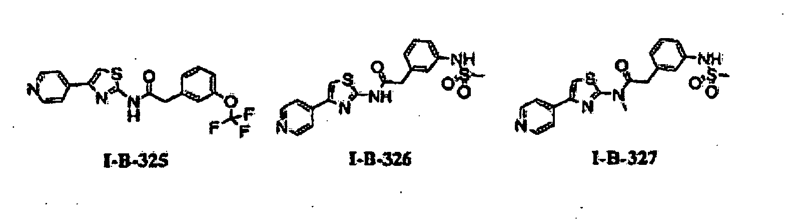

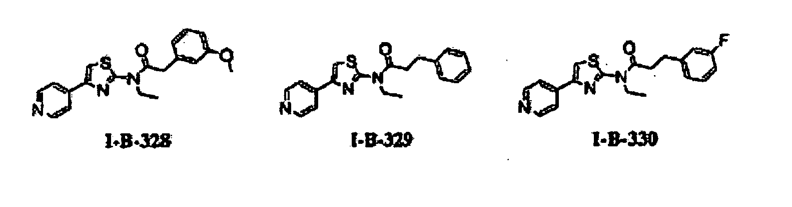

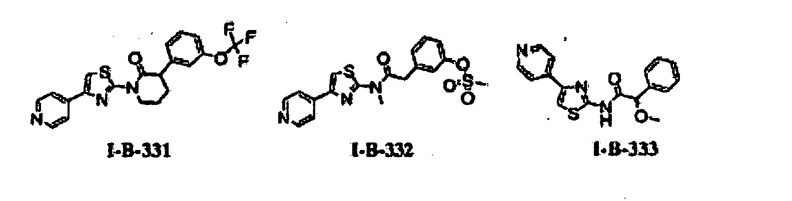

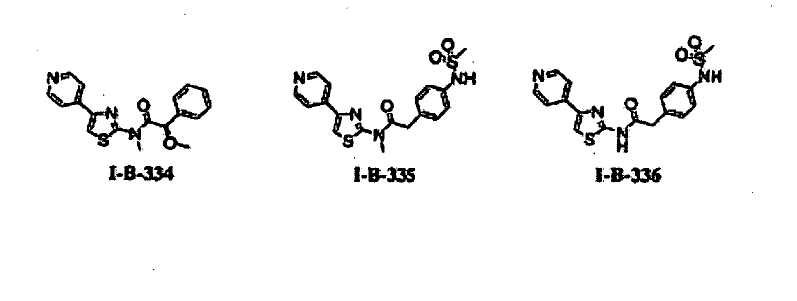

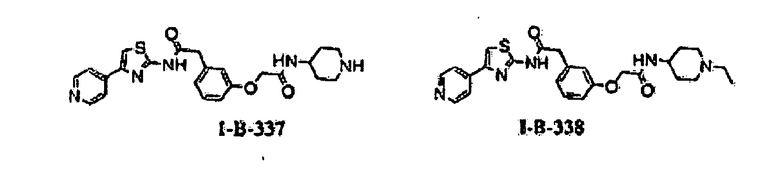

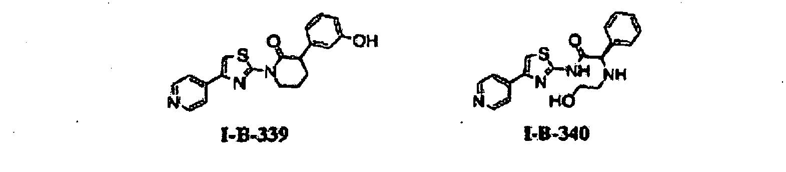

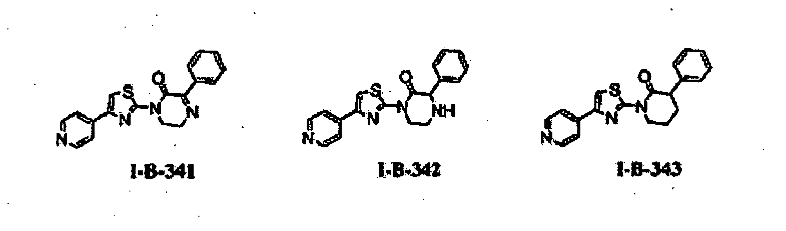

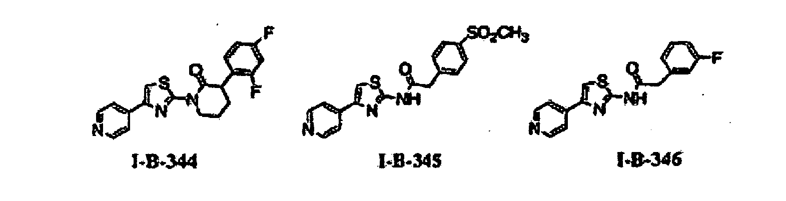

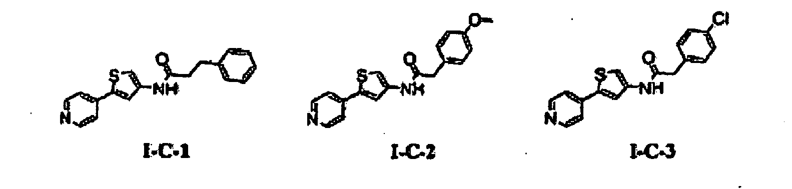

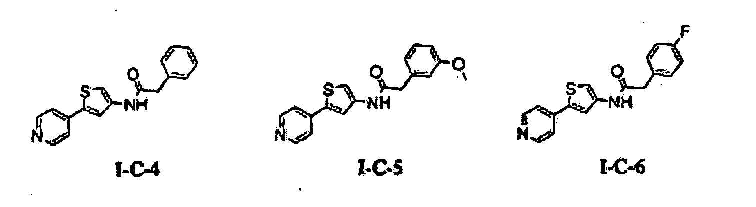

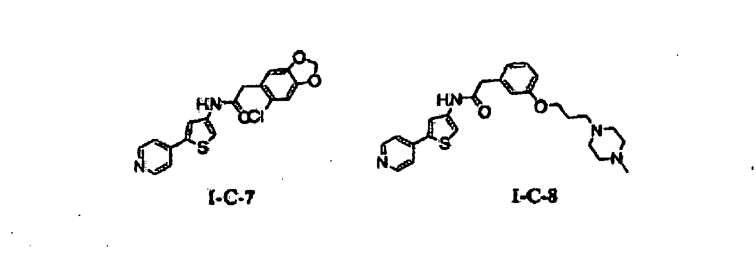

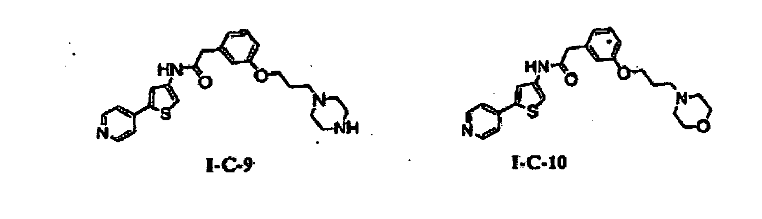

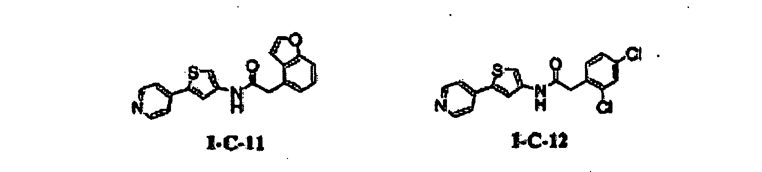

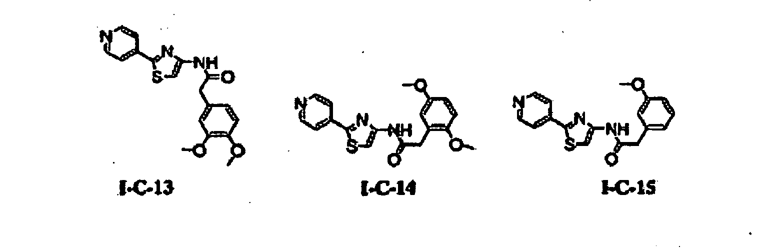

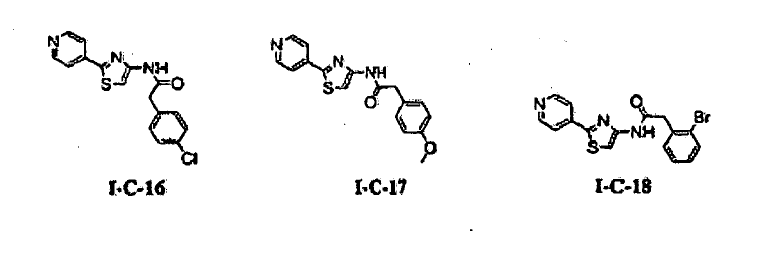

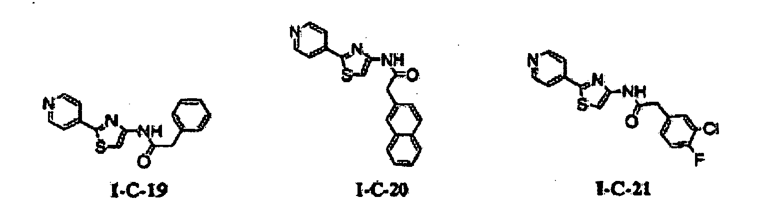

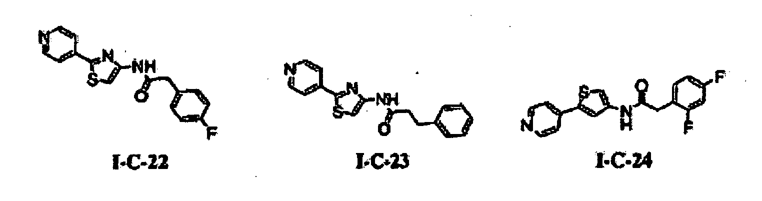

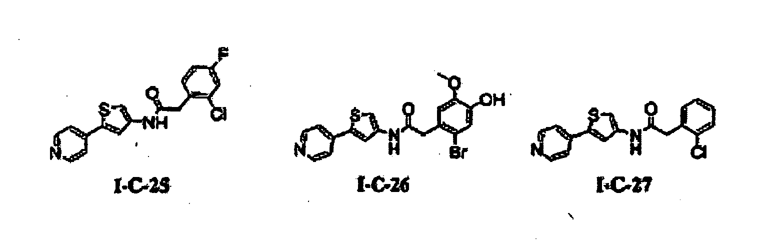

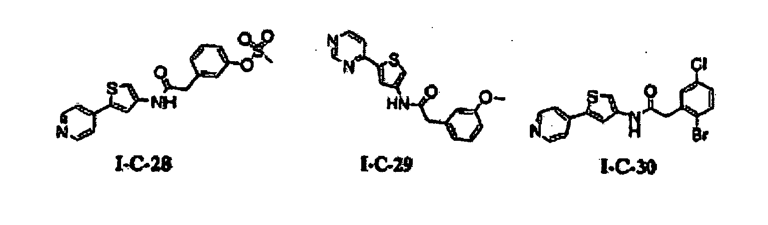

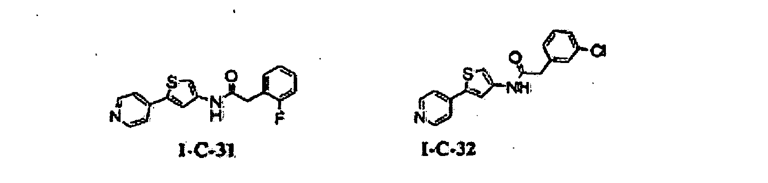

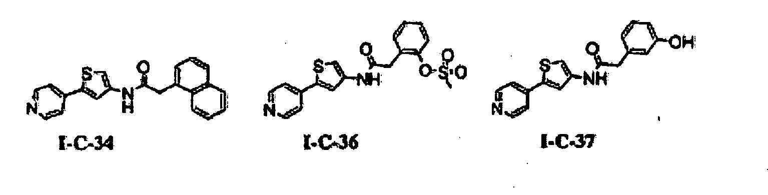

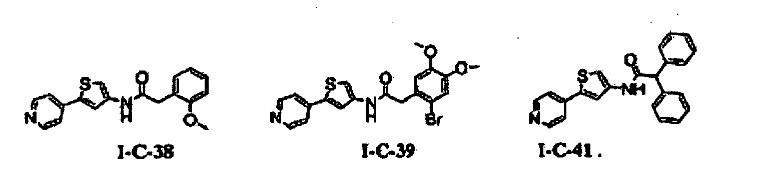

17. Zusammensetzung, umfassend eine wirksame Menge einer Verbindung nach einem der Ansprüche 1 bis 16, und einen pharmazeutisch annehmbaren Träger, ein pharmazeutisch annehmbares Adjuvans oder Vehikel.

18. Zusammensetzung nach Anspruch 17, zusätzlich umfassend ein therapeutisches Mittel, das ausgewählt ist aus einem chemotherapeutischen oder anti-proliferativen Mittel, einem entzündungshemmenden Mittel, einem immunmodulatorischen oder immunsuppressiven Mittel, einem neurotrophen Faktor, einem Mittel zur Behandlung einer kardiovaskulären Erkrankung, einem Mittel zur Behandlung destruktiver Knochenkrankheiten, einem Mittel zur Behandlung einer Lebererkrankung, einem antiviralen Mittel, einem Mittel zur Behandlung von Blutkrankheiten, einem Mittel zur Behandlung von Diabetes oder einem Mittel zur Behandlung von Immunmangelkrankheiten.

19. In-Vitro-Verfahren, das eine Verbindung der folgenden Formel verwendet:
oder ein pharmazeutisch annehmbares Salz davon,
wobei 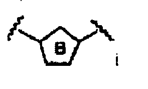 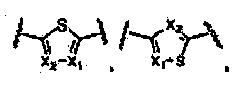 oder 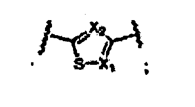 ist;
R¹ Halogen, CN, NO₂ oder VₘR ist;
Z¹ und Z³ jeweils unabhängig N oder CR^{Z} sind und Z² N oder CR¹ ist, vorausgesetzt, dass Z¹, Z² und Z³ nicht gleichzeitig N sind;
jede Präsenz von R^{Z} unabhängig Halogen, CN, NO₂ oder UₙR' ist;
R² UₙR' ist;
X¹ und X² jeweils unabhängig CR⁴ oder N sind;
jede Präsenz von R⁴ unabhängig Halogen, CN, NO₂ oder VₘR ist;
jede Präsenz von U oder V unabhängig eine optional substituierte C₁₋₆-Alkylidenkette ist, wobei bis zu zwei Methyleneinheiten der Kette optional und unabhängig durch -NR-, -S-, -O-, -CS-, -CO₂-, -OCO-, -CO-, -COCO-, -CONR-, -NRCO-, - NRCO₂-, -SO₂NR-, -NRSO₂-, -CONRNR-, -NRCONR-, -OCONR-, -NRNR-, - NRSO₂NR-, -SO-, -SO₂-, -PO-, -PO₂-, oder -POR- ersetzt sind;
m und n jeweils unabhängig 0 oder 1 sind;
jede Präsenz von R unabhängig Wasserstoff oder eine optional substituierte, aliphatische C₁₋₆-Gruppe ist; und jede Präsenz von R' unabhängig Wasserstoff oder eine optional substituierte aliphatische C₁₋₆-Gruppe, ein 3- bis 8-gliedriger, gesättigter, teilweise ungesättigter oder vollständig ungesättigter, monocyclischer Ring mit 0 bis 3 Heteroatomen ist, die unabhängig ausgewählt sind aus Stickstoff, Sauerstoff oder Schwefel, oder ein 8- bis 12-gliedriges gesättigtes, teilweise ungesättigtes oder vollständig ungesättigtes bicyclisches Ringsystem mit 0 bis 5 Heteroatomen, die unabhängig ausgewählt sind aus Stickstoff, Sauerstoff oder Schwefel; oder R und R', zweifache Präsenzen von R oder zweifache Präsenzen von R', gemeinsam mit dem Atom oder den Atomen genommen werden, an die sie gebunden sind, um einen optional substituierten, 3-bis 12-gliedrigen, gesättigten, teilweise ungesättigten oder vollständig ungesättigten monocyclischen oder bicyclischen Ring mit 0 bis 4 Heteroatomen zu bilden; die unabhängig ausgewählt sind aus Stickstoff, Sauerstoff oder Schwefel;
Q¹ -CO-, -SO₂-, -CONR- oder -SO₂NR- ist;
R³ Q²-Ar¹ ist,
oder R² und Q¹-R³, gemeinsam genommen mit dem Stickstoffatom, die cyclische Gruppe 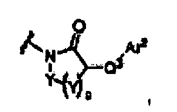 bilden, wobei s 1 oder 2 ist, jede Präsenz von Y unabhängig, wie Valenz und Stabilität zulassen, -CO-, -CS-, -SO₂-, -O-, -S-, NR⁵- oder -C(R⁵)₂- ist und R⁵ UₙR' ist;
Q² und Q³ jeweils unabhängig eine Bindung oder eine C₁₋₆-Alkylidenkette sind, wobei bis zu zwei Methyleneinheiten der Kette jeweils optional und unabhängig durch -NR'-, -S-, -O-, - CS-, -CO₂-, -OCO-, -CO-, -COCO-, -CONR'-, -NR'CO-, -NR'CO₂-, - SO₂NR'-, -NR'SO₂-, -CONR'NR'-, -NR'CONR', -OCONR'-, -NR'NR'-, - NR'SO₂NR'-, -SO-, -SO₂-, -PO-, -PO₂- oder -POR' ersetzt sind; und wobei jedes Kohlenstoffatom in der einen oder den mehreren Methyleneinheiten optional mit einer oder mehreren Präsenzen von R⁶ substituiert ist, wobei jede Präsenz von R⁶ unabhängig Halogen, CN, NO₂ oder UₙR' ist oder zwei Präsenzen von R⁶ oder R' und R⁶ gemeinsam genommen mit den Atomen, an die sie gebunden sind, einen optional substituierten, 3- bis 6-gliedrigen Cycloalkyl, Heterocyclyl-, Aryl- oder Heteroarylring bilden; und
Ar¹ und Ar² jeweils unabhängig ein 5- bis 8-gliedriger, gesättigter, teilweise ungesättigter oder vollständig ungesättigter, monocyclischer Ring mit 0 bis 3 Heteroatomen sind, die unabhängig ausgewählt sind aus Stickstoff, Sauerstoff oder Schwefel, oder ein 8- bis 12-gliedriges, gesättigtes, teilweise ungesättigtes oder vollständig ungesättigtes, bicyclisches Ringsystem mit 0 bis 5 Heteroatomen, die unabhängig ausgewählt sind aus Stickstoff, Sauerstoff oder Schwefel; wobei Ar¹ und Ar² jeweils optional mit 0 bis 5 unabhängigen Präsenzen von TR⁷ substituiert sind; wobei T eine Bindung oder eine C₁-C₆-Alkylidenkette ist, wobei bis zu zwei Methyleneinheiten von T optional und unabhängig durch -NR-, -S-, -O-, -CS-, -CO₂-, -OCO-, -CO-, -COCO-, -CONR-, -NRCO-, -NRCO₂-, -SO₂NR-, -NRSO₂-, -CONRNR- , -NRCONR-, -OCONR-, -NRNR-, -NRSO₂NR-, -SO-, -SO₂-, -PO-, -PO₂-, oder -POR- ersetzt sind; und jede Präsenz von R⁷ unabhängig R', Halogen, NO₂ oder CN ist;
oder ein pharmazeutisch annehmbares Salz oder eine Zusammensetzung davon, zum Hemmen der ROCK-, ERK-, GSK- oder AGC-Kinase-Aktivität in einer biologischen Probe.

20. In-Vitro-Verfahren nach Anspruch 19 zur Verwendung in der Hemmung der ROCK-Aktivität.

21. Verbindung mit der Formel: 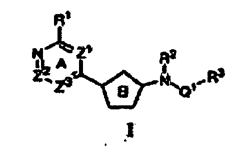 wobei 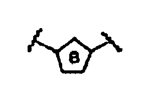 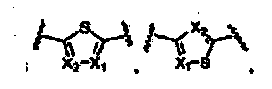 oder 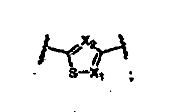 ist;
R¹ Halogen, CN, NO₂ oder VₘR ist;
Z¹ und Z³ jeweils unabhängig N oder CR^{Z} sind und Z² N oder CR¹ ist, vorausgesetzt, dass Z¹, Z² und Z³ nicht gleichzeitig N sind;
jede Präsenz von R^{z} unabhängig Halogen, CN, NO₂ oder UₙR' ist;
R² UₙR' ist;
X¹ und X² jeweils unabhängig CR⁴ oder N sind;
jede Präsenz von R⁴ unabhängig Halogen, CN, NO₂ oder VₘR ist;
jede Präsenz von U oder V unabhängig eine optional substituierte C₁₋₆-Alkylidenkette ist, wobei bis zu zwei Methyleneinheiten der Kette optional und unabhängig durch -NR-, -S-, -O-, -CS-, -CO₂-, -OCO-, -CO-, -COCO-, -CONR-, -NRCO-, - NRCO₂-, -SO₂NR-, -NRSO₂-, -CONRNR-, -NRCONR-, -OCONR-, -NRNR-, - NRSO₂NR-, -SO-, -SO₂-, -PO-, -PO₂- oder -POR- ersetzt sind;
m und n jeweils unabhängig 0 oder 1 sind;
jede Präsenz von R unabhängig Wasserstoff oder eine optional substituierte, aliphatische C₁₋₆-Gruppe ist; und jede Präsenz von R' unabhängig Wasserstoff oder eine optional substituierte aliphatische C₁₋₆-Gruppe, ein 3- bis 8-gliedriger, gesättigter, teilweise ungesättigter oder vollständig ungesättigter, monocyclischer Ring mit 0 bis 3 Heteroatomen ist, die unabhängig ausgewählt sind aus Stickstoff, Sauerstoff oder Schwefel, oder ein 8-bis 12-gliedriges, gesättigtes, teilweise ungesättigtes oder vollständig ungesättigtes, bicyclisches Ringsystem mit 0 bis 5 Heteroatomen, die unabhängig ausgewählt sind aus Stickstoff, Sauerstoff oder Schwefel; oder R und R', zweifache Präsenzen von R oder zweifache Präsenzen von R', gemeinsam mit dem Atom oder den Atomen genommen werden, an die sie gebunden sind, um einen optional substituierten, 3- bis 12-gliedrigen, gesättigten, teilweise ungesättigten oder vollständig ungesättigten, monocyclischen oder bicyclischen Ring mit 0 bis 4 Heteroatomen zu bilden, die unabhängig ausgewählt sind aus Stickstoff, Sauerstoff oder Schwefel;
Q¹ -CO-, -SO₂-, oder -SO₂NR- ist;
R³ Q²-Ar¹ ist;
oder R² und Q¹-R³, gemeinsam genommen mit dem Stickstoffatom, die zyklische Gruppe: 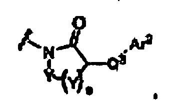 bilden, wobei s 1 oder 2 ist, jede Präsenz von Y unabhängig, wie Valenz und Stabilität zulassen, -CO-, -CS-, -SO₂-, -O-, -S-, NR⁵- oder -C(R⁵)₂- ist und R⁵ UₙR' ist;
Q² und Q³ jeweils unabhängig eine Bindung oder eine C₁₋₆-Alkylidenkette sind, wobei bis zu zwei Methyleneinheiten der Kette jeweils optional und unabhängig durch -S-, -O-, -CS-, -CO₂- , -OCO-, -CO-, -COCO-, -CONR'-, -NR'CO-, NR'CO₂-, -SO₂NR'-, - NR'SO₂-, -CONR'NR'-, -NR'CONR', -OCONR'-, -NR'NR'-, -NR'SO₂NR'-, -SO-, -SO₂-, -PO-, -PO₂- oder -POR' ersetzt sind; und wobei jedes Kohlenstoffatom in der einen oder den mehreren Methyleneinheiten optional mit einer oder mehreren Präsenzen von R⁶ substituiert ist, wobei jede Präsenz von R⁶ unabhängig Halogen, CN, NO₂ oder UₙR' ist, oder zwei Präsenzen von R⁶ oder R' und R⁶, gemeinsam genommen mit den Atomen, an die sie gebunden sind, einen optional substituierten, 3- bis 6-gliedrigen Cycloalkyl-, Heterocyclyl-, Aryl- oder Heteroarylring bilden; und
Ar¹ und Ar² jeweils unabhängig ein 5- bis 8-gliedriger, gesättigter, teilweise ungesättigter oder vollständig ungesättigter, monocyclischer Ring mit 0 bis 3 Heteroatomen sind, die unabhängig ausgewählt sind aus Stickstoff, Sauerstoff oder Schwefel, oder ein 8- bis 12-gliedriges, gesättigtes, teilweise ungesättigtes oder vollständig ungesättigtes, bicyclisches Ringsystem mit 0 bis 5 Heteroatomen, die unabhängig ausgewählt sind aus Stickstoff, Sauerstoff oder Schwefel; wobei Ar¹ und Ar² jeweils optional mit 0 bis 5 unabhängigen Präsenzen von TR⁷ substituiert sind; wobei T eine Bindung oder eine C₁-C₆-Alkylidenkette ist, wobei bis zu zwei Methyleneinheiten von T optional und unabhängig durch -NR-, -S-, -O-, -CS-, -CO₂-, -OCO-, -CO-, -COCO-, -CONR-, -NRCO-, -NRCO₂-, -SO₂NR-, -NRSO₂-, -CONRNR-, -NRCONR-, -OCONR-, -NRNR-, -NRSO₂NR-, -SO-, -SO₂-, -PO-, -PO₂-, oder -POR- ersetzt sind; und jede Präsenz von R⁷ unabhängig R', Halogen, NO₂ oder CN ist;
oder ein pharmazeutisch annehmbares Salz oder eine Zusammensetzung davon zur Verwendung in der Behandlung oder Milderung der Schwere einer Erkrankung, eines Zustandes oder einer Krankheit, die/der ausgewählt ist aus einer proliferativen Krankheit, einer Herzerkrankung, einer neurodegenerativen Krankheit, einer psychotischen Krankheit, einer Autoimmunerkrankung, einem Zustand, der mit einer Organtransplantation zusammenhängt, einer Entzündungserkrankung, einer immunologisch vermittelten Krankheit, einer Viruserkrankung oder einer Knochenkrankheit.

22. Verbindung, pharmazeutisch annehmbares Salz oder Zusammensetzung davon nach Anspruch 21 zur Verwendung mit einem zusätzlichen therapeutischen Mittel, das ausgewählt ist aus einem chemotherapeutischen oder anti-proliferativen Mittel, einem entzündungshemmenden Mittel, einem immunmodulatorischen oder immunsuppressiven Mittel, einem neurotrophen Faktor, einem anti-psychotischen Mittel, einem Mittel zur Behandlung einer kardiovaskulären Erkrankung, einem Mittel zur Behandlung destruktiver Knochenkrankheiten, einem Mittel zur Behandlung einer Lebererkrankung, einem antiviralen Mittel, einem Mittel zur Behandlung von Blutkrankheiten, eine Mittel zur Behandlung von Diabetes oder einem Mittel zur Behandlung von Immunmangelkrankheiten.

23. Verbindung, pharmazeutisch annehmbares Salz oder Zusammensetzung davon nach Anspruch 21 zur Verwendung in der Behandlung oder Milderung der Schwere einer Erkrankung, eines Zustandes oder einer Krankheit, wobei die Erkrankung, der Zustand oder die Krankheit Allergie, Asthma, Diabetes, Alzheimer-Krankheit, Huntington-Krankheit, Parkinson-Krankheit, AIDS-assoziierte Demenz, amyotrophe Lateral-Sklerose (AML, Lou-Gehrig-Syndrom), Multiple Sklerose (MS), Schizophrenie, Cardiomyozyten-Hypertrophie, Reperfusion/Ischämie (z.B. Schlaganfall), Alopezie, Krebs, Hepatomegalie, kadiovaskuläre Erkrankung, einschließlich Kardiomegalie, zystische Fibrose, Viruserkrankung, Autoimmunerkrankung, Atherosklerose, Restenose, Psoriasis, Entzündung, Hypertonie, Angina pectoris, zerebrovaskuläre Kontraktion, periphere Durchblutungsstörung, Frühgeburt, Arteriosklerose, Vasospasmus (zerebraler Vasospasmus, koronarer Vasospasmus), Retinopathie, erektile Dysfunktion (ED), AIDS, Osteoporose, Morbus Crohn und Colitis, Neuritenauswuchs, oder Raynaud-Syndrom ist; insbesondere Atherosklerose, Hypertonie, erektile Dysfunktion (ED), Reperfusion/Ischämie (z.B. Schlaganfall) oder Vasospasmus (zerebraler Vasospasmus, koronarer Vasospasmus).

24. Verwendung einer Verbindung nach einem der Ansprüche 1 bis 16 in der Herstellung eines Medikaments zur Behandlung oder Milderung der Schwere einer Erkrankung, eines Zustandes oder einer Krankheit, die/der ausgewählt ist aus einer proliferativen Krankheit, einer Herzerkrankung, einer neurodegenerativen Krankheit, einer psychotischen Krankheit, einer Autoimmunerkrankung, einem Zustand, der mit einer Organtransplantation zusammenhängt, einer Entzündungserkrankung, einer immunologisch vermittelten Krankheit, einer Viruserkrankung oder einer Knochenkrankheit; wie Allergie, Asthma, Diabetes, Alzheimer-Krankheit, Huntington-Krankheit, Parkinson-Krankheit, AIDS-assoziierte Demenz, amyotrophe Lateral-Sklerose (AML, Lou-Gehrig-Syndrom), Multiple Sklerose (MS), Schizophrenie, Cardiomyozyten-Hypertrophie, Reperfusion/Ischämie (z.B. Schlaganfall), Alopezie, Krebs, Hepatomegalie, kadiovaskuläre Erkrankung, einschließlich Kardiomegalie, zystische Fibrose, Viruserkrankung, Autoimmunerkrankung, Atherosklerose, Restenose, Psoriasis, Entzündung, Hypertonie, Angina pectoris, zerebrovaskuläre Kontraktion, periphere Durchblutungsstörung, Frühgeburt, Arteriosklerose, Vasospasmus (zerebraler Vasospasmus, koronarer Vasospasmus), Retinopathie, erektile Dysfunktion (ED), AIDS, Osteoporose, Morbus Crohn und Colitis, Neuritenauswuchs, oder Raynaud-Syndrom;
insbesondere Atherosklerose, Hypertonie, erektile Dysfunktion (ED), Reperfusion/Ischämie (z.B. Schlaganfall) oder Vasospasmus (zerebraler Vasospasmus, koronarer Vasospasmus).

## Revendications

1. Composé de formule I : 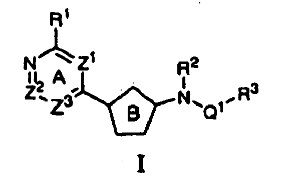 ou un sel pharmacologiquement admissible d'un tel composé, dans laquelle formule : 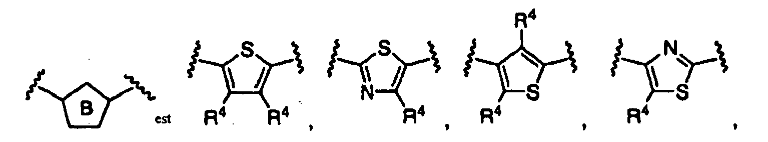 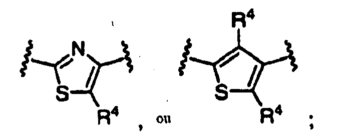 R¹ représente un halogène, CN, NO₂ ou VₘR ;
Z¹ et Z³ représentent chacun CR^{z} et Z² représente CR¹ ;
chaque occurrence de R^{z} représente indépendamment un halogène, CN, NO₂ ou UₙR' ;
R² représente UₙR' ;
chaque occurrence de R⁴ représente indépendamment un halogène, CN, NO₂ ou VₘR ;
chaque occurrence de U ou V représente indépendamment une chaîne alkylidène en C_{1 à 6} éventuellement substituée, où jusqu'à deux motifs méthylène de la chaîne sont éventuellement et indépendamment remplacés par -NR-, -S-, -O-, -CS-, -CO₂-, -OCO-, -CO-, -COCO-, -CONR-, -NRCO-, -NRCO₂-, -SO₂NR-, -NRSO₂-, -CONRNR-, -NRCONR-, -OCONR-, -NRNR-, -NRSO₂NR-, -SO-, -SO₂-, -PO-, -PO₂- ou -POR- ;
m et n représentent chacun indépendamment 0 ou 1 ;
chaque occurrence de R' représente indépendamment un hydrogène ou un groupe aliphatique en C_{1 à 6} éventuellement substitué ; et chaque occurrence de R représente indépendamment l'hydrogène ou un groupe aliphatique en C_{1 à 6} éventuellement substitué, un anneau monocyclique à 3 à 8 membres saturé, partiellement insaturé ou complètement insaturé comportant 0 à 3 hétéroatomes indépendamment choisis parmi l'azote, l'oxygène ou le soufre, ou un système d'anneau bicyclique à 8 à 12 membres saturé, partiellement insaturé ou complètement insaturé comportant 0 à 5 hétéroatomes indépendamment choisis parmi l'azote, l'oxygène ou le soufre ; ou R et R', deux occurrences de R, ou deux occurrences de R', sont pris ensemble avec le(s) atome(s) au(x)quel(s) ils sont liés pour former un anneau monocyclique ou bicyclique éventuellement substitué à 3 à 12 membres, saturé, partiellement insaturé ou complètement insaturé comportant 0 à 4 hétéroatomes indépendamment choisis parmi l'azote, l'oxygène ou le soufre ;
Q¹ représente -CO- ;
R³ représente Q²-Ar¹, où Q² représente -(CHR⁶)_{q}-, où q représente 1, 2 ou 3,
ou R² et Q¹-R³, pris ensemble avec l'atome d'azote intermédiaire, forment le groupe cyclique : 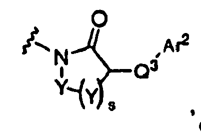 où s représente 1 ou 2, chaque occurrence de Y est indépendamment, comme la valence et la stabilité le permettent, -CO-, -CS-, -SO₂-, -O-, -S-, -NR⁵- ou -C(R⁵)₂-, et R⁵ représente UₙR' ;
Q³ est une liaison ou une chaîne alkylidène en C_{1 à 6}, où jusqu'à deux motifs méthylène de la chaîne sont chacun éventuellement et indépendamment remplacés par -NR'-, -S-, -O-, -CS-, -C₂-, -OCO-, -CO-, -COCO-, -CONR'-, -NR'CO-, -NR'CO₂-, -SO₂NR'-, -NR'SO₂-, -CONR'NR'-, -NR'CONR'-, -OCONR'-, -NR'NR'-, -NR'SO₂NR'-, -SO-, -SO₂-, -PO-, -PO₂- ou -POR'- ; et où n'importe quel atome de carbone dans l'un ou plusieurs motifs méthylène est éventuellement substitué par une ou deux occurrences de R⁶, où chaque occurrence de R⁶ représente indépendamment un halogène, CN, NO₂ ou UₙR', ou deux occurrences de R⁶, ou R' et R⁶, pris ensemble avec les atomes auxquels ils sont liés, forment un anneau cycloalkyle à 3 à 6 membres, hétérocyclyle, aryle ou hétéroaryle, éventuellement substitué ; et
Ar¹ représente : 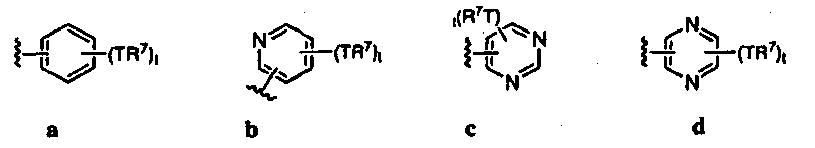 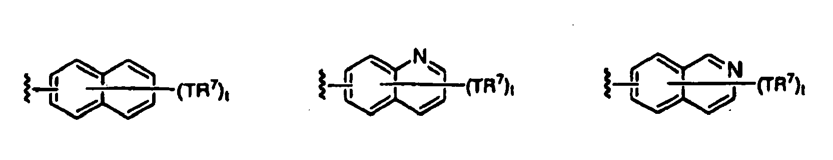 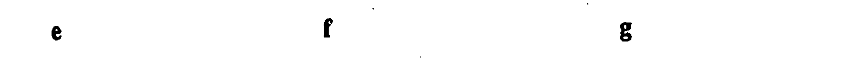 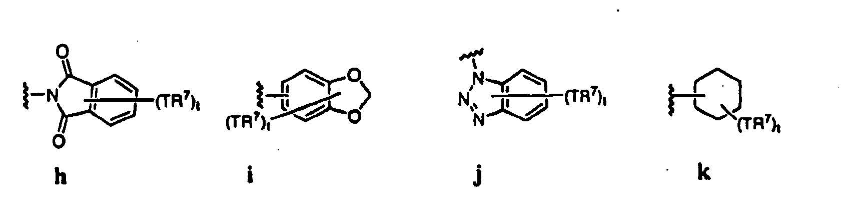 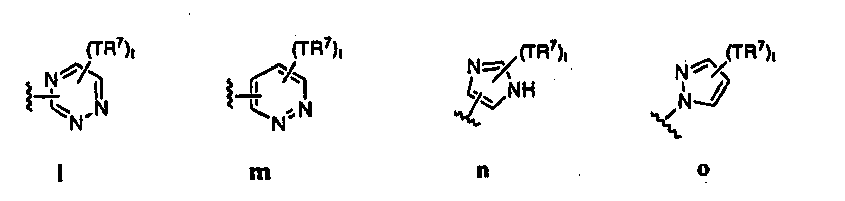 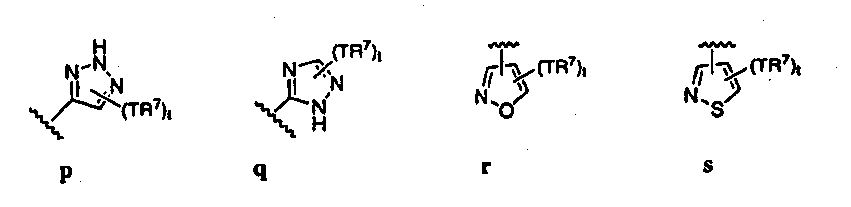 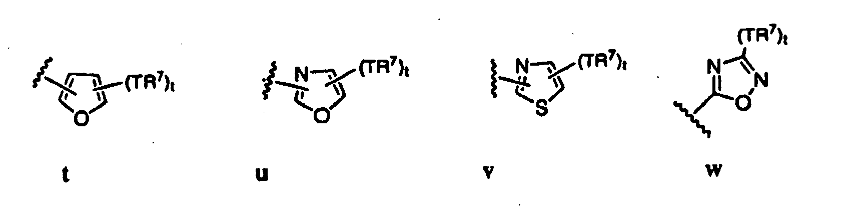 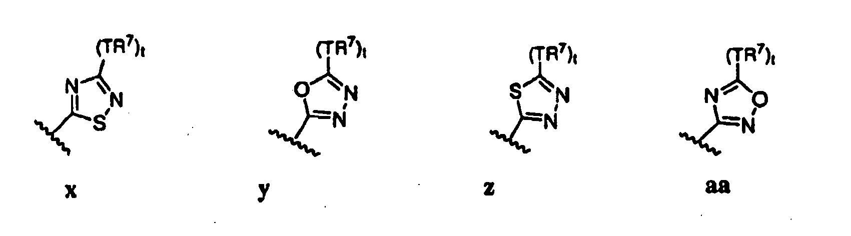 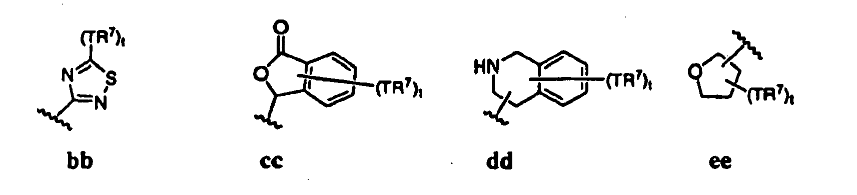 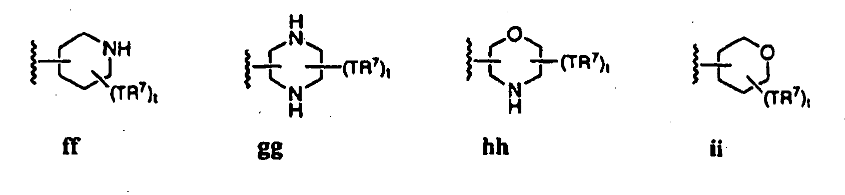 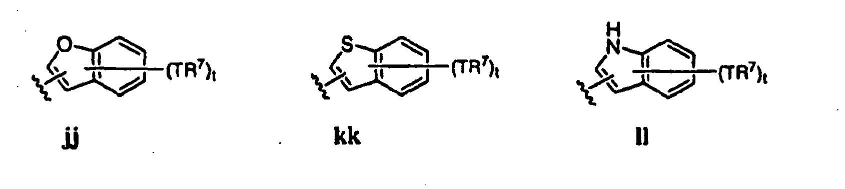 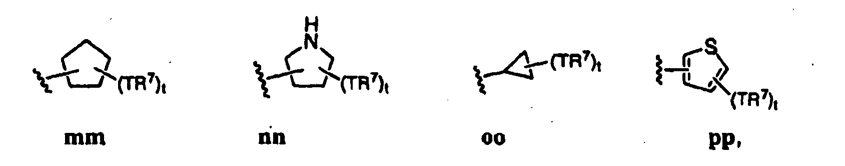 où t représente 0, 1, 2, 3, 4 ou 5, et où n'importe quel Ar¹ est lié à Q² à travers n'importe quel atome d'azote ou de carbone pouvant être substitué, et où un ou plusieurs atomes d'hydrogène sur n'importe quel atome d'azote ou de carbone pouvant être substitué est substitué avec une ou plusieurs occurrences indépendantes de TR⁷ ;
et Ar² représente un anneau monocyclique à 5 à 8 membres, saturé, partiellement insaturé ou complètement insaturé comportant 0 à 3 hétéroatomes indépendamment choisis parmi l'azote, l'oxygène ou le soufre, ou un système d'anneau bicyclique à 8 à 12 membres saturé, partiellement insaturé ou complètement insaturé comportant 0 à 5 hétéroatomes indépendamment choisis parmi l'azote, l'oxygène ou le soufre ; où Ar¹ et Ar² sont chacun éventuellement substitués avec 0 à 5 occurrences indépendantes de TR⁷ ; où T est une liaison ou représente une chaîne alkylidène en C₁ à C₆ où jusqu'à deux motifs méthylène de T sont éventuellement et indépendamment remplacés par -NR-, -S-, -O-, -CS-, -CO₂-, -OCO-, -CO-, -COCO-, -CONR-, -NRCO-, -NRCO₂-, -SO₂NR-, -NRSO₂-, -CONRNR-, -NRCONR-, -OCONR-, -NRNR-, -NRSO₂NR-, -SO-, -SO₂-, -PO-, -PO₂- ou -POR- ; et chaque occurrence de R⁷ représente indépendamment R', un halogène, NO₂ ou CN ;
à condition que :
pour le composé de structure : 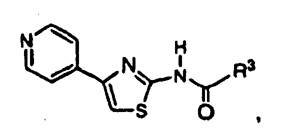 R³ ne représente pas l'un quelconque des groupes suivants : -CH₂(3-NHCOPh-phényle), -CH₂-pyrrolidine, benzyle non substitué, -CH₂-naphtyle, -CH₂CH₂-3-(4-Cl-phényle)-1-phényl-1-H-pyrazol-4-yle ou -CH₂(1,3-dioxo-iso-indole).

2. Composé selon la revendication 1, dans lequel :
(1) R² représente l'hydrogène, ou représente UₙR' où n vaut 1, et U représente une chaîne alkylidène en C_{1 à 6} dans laquelle un ou deux motifs méthylène sont éventuellement et indépendamment remplacés par O, NR, S ou C(O),
de préférence, où U représente -CH₂-, -CH₂CH₂-, -CH₂CH₂CH₂-, -CH₂CH₂CH₂CH₂-, -CH₂O-, -CH₂S-, -CH₂NR-, -CH₂CH₂O-, -CH₂CH₂S-, -CH₂CH₂NR-, -CH₂CH₂CH₂O-, -CH₂CH₂CH₂S-, -CH₂CH₂CH₂NR-, -CH₂CH₂CH₂CH₂O-, -CH₂CH₂CH₂CH₂S-, -CH₂CH₂CH₂CH₂NR-, -CH₂CH₂OCH₂CH₂-, -(CH₂)4NHCH₂-, -(CH₂)₃NHCH₂CH₂- ou -CH₂CH₂NHCH₂CH₂-, et, de préférence, R' est choisi parmi l'hydrogène, un alkyle en C_{1 à} C₄, un tétrahydropyranyle éventuellement substitué, un pyrrolidinyle, un pipéridinyle, un pipérazinyle, un morpholinyle, un thiomorpholinyle, un pyridinyle, un phényle ou un cyclohexyle, ou R et R', pris ensemble avec l'atome d'azote auquel ils sont liés, forment un anneau hétérocyclyle éventuellement substitué à 5 ou 6 membres ; ou
(2) Ar¹ représente : 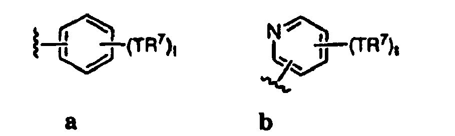 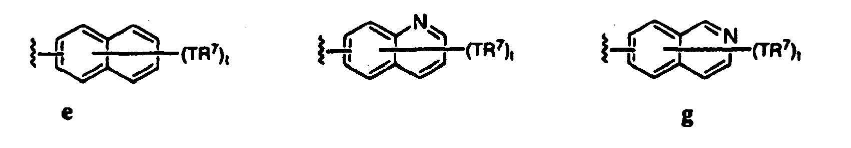 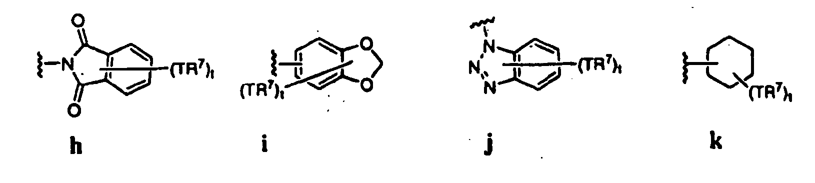 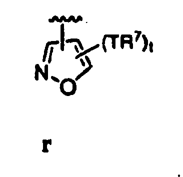 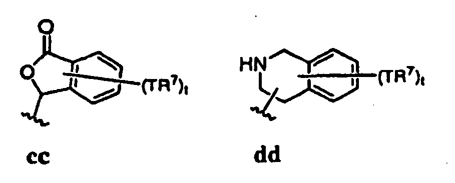 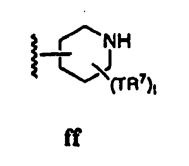 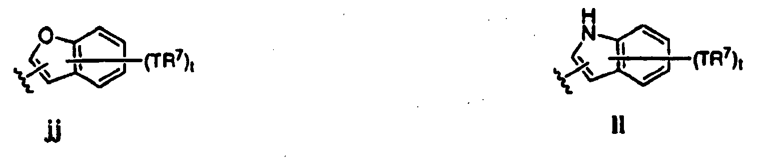 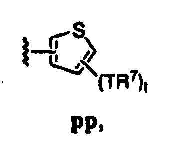
où t représente 0, 1, 2, 3, 4 ou 5, et où n'importe quel Ar¹ est lié à Q² à travers n'importe quel atome d'azote ou de carbone pouvant être substitué, et où un ou plusieurs atomes d'hydrogène sur n'importe quel atome d'azote ou de carbone pouvant être substitué est substitué avec une ou plusieurs occurrences indépendantes de TR⁷ ;
où
(a) T est une liaison ou est une chaîne alkylidène éventuellement substituée en C_{1 à 6} dans laquelle un ou deux motifs méthylène sont éventuellement et indépendamment remplacés par -O-, -NR-, -S-, -SO₂-,-COO-, -CO-, -OSO₂-, -NRSO₂, -CONR-, ou -SO₂NR-, et R⁷ représente R' ou un halogène ; ou
(b) chaque occurrence de TR⁷ représente indépendamment un alkyle en C_{1 à 3}, -OR', -SR', -CF₃, -OCF₃, -SCF₃, -F, -Cl, I, -Br, -COOR', -COR', -O(CH₂)₄N(R)(R'), -O(CH₂)₃N(R)(R'), -O(CH₂)₂N(R)(R'), -O(CH₂)N(R)(R'), -O(CH₂)₄CON(R)(R'), -O(CH₂)₃CON(R)(R'), -O(CH₂)₂CON(R)(R'), -O(CH₂)CON(R)(R'), -C(O)N(R)(R'), -(CH₂)₄OR'-(CH₂)₃OR', -(CH₂)₂OR', -CH₂OR', un phényle ou benzyle éventuellement substitué, -N(R)(R'), - (CH₂)₄N(R)(R'), -(CH₂)₃N(R)(R'), -(CH₂)₂N(R)(R'), -(CH₂)N(R)(R'), ou SO₂N(R)(R'), NRSO₂R', CON(R)(R') ou-OSO₂R'.

3. Composé selon la revendication 1, dans lequel R et Q¹-R³, pris ensemble avec l'atome d'azote intermédiaire, forment le groupe cyclique : 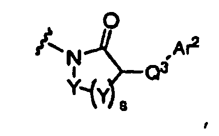 où s représente 1 ou 2, chaque occurrence de Y représente indépendamment, comme la valence et la stabilité le permettent, -CO-, -CS-, -SO₂-, -O-, -S-, -NR⁵- ou -C(R⁵)₂-, et R⁵ représente UₙR', et les composés de formule I-A-ii, I-B-ii et I-C-ii sont fournis : 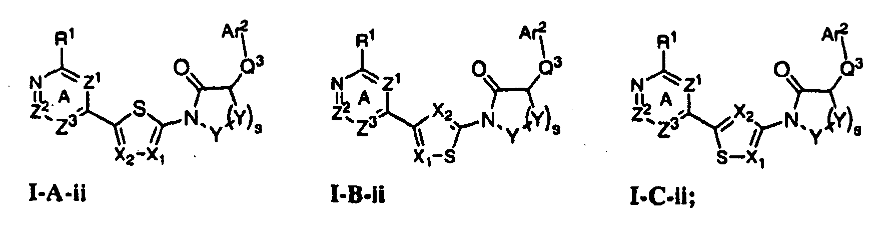 où :
(1) Q³ est une liaison directe, ou est -(CHR⁶)_{q}-, -(CHR⁶)_{q}O-, -(CHR⁶)_{q}S-, -(CHR⁶)_{q}S(O)₂-, -(CHR⁶)_{q}S(O)-, -(CHR⁶)_{q}NR- ou -(CHR⁶)_{q}C(O)-, où q représente 0, 1, 2 ou 3, et R⁶ représente R', -N(R)(R'), -(CH₂)₁₋₄N(R)(R'), -OR', -(CH₂)₁₋₄OR', -NR(CH₂)₁₋₄N(R)(R'), -NR(CH₂)₁₋₄SO₂R', -NR(CH₂)₁₋₄COOR' ou -NR(CH₂)₁₋₄COR', ou deux occurrences de R⁶, prises ensemble avec les atomes auxquels elles sont liées, forment un anneau éventuellement substitué à 3 à 6 membres, saturé, partiellement insaturé ou complètement insaturé ; par exemple, où : R⁶ représente CH₂OH, CH₂CH₂OH, OH, OMe, OEt, NH₂, NH(Me), NH(Et), N(Me)(Me), CH₂NH₂, CH₂CH₂NH₂, NHCO₂t-butyle, phényle, cyclopentyle, méthyle, éthyle, isopropyle, cyclopropyle, NH (CH₂)₃NH₂, NH (CH₂)₂NH₂, NH(CH₂)₂NHEt, NHCH₂pyridyl, NHSO₂phényle, NHC(O)CH₂C(O)Ot-butyle, NHC(O)CH₂NH₃ et NHCH₂-imidazol-4-yle ;
(2) Ar² représente : 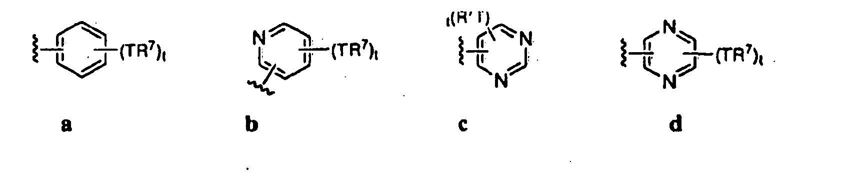 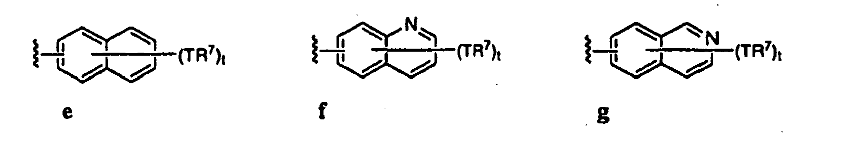 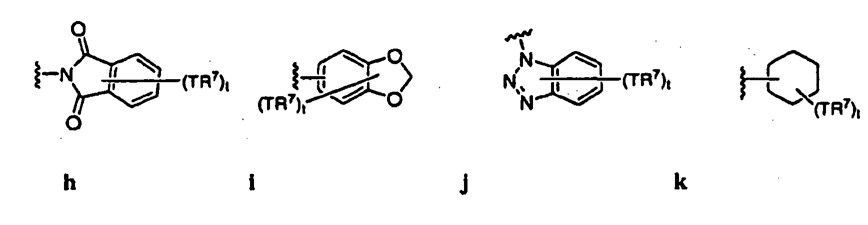 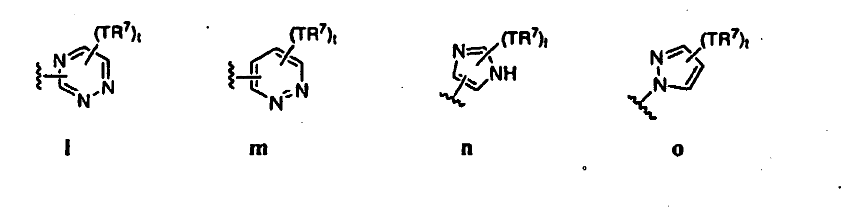 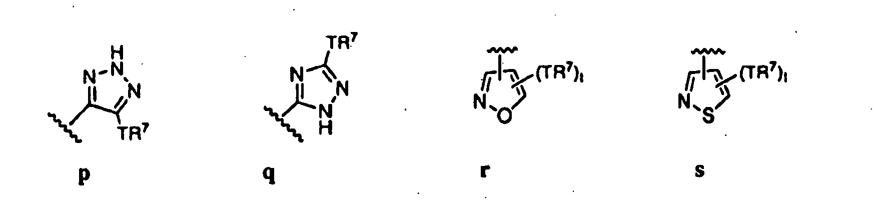 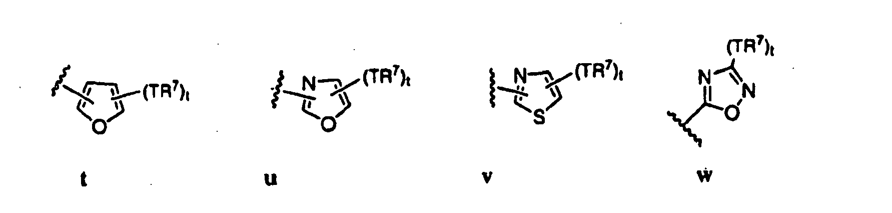 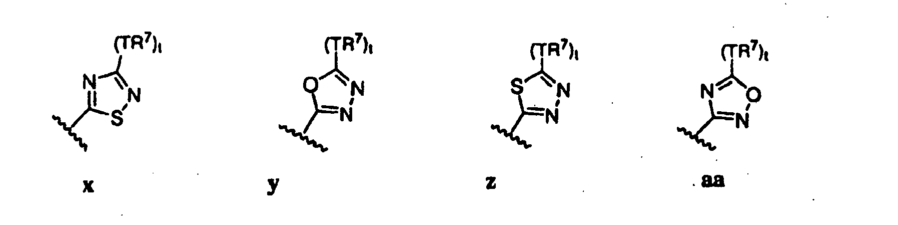 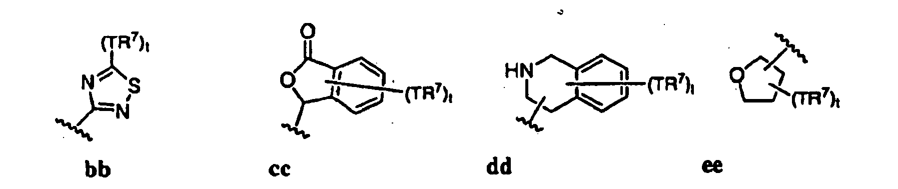 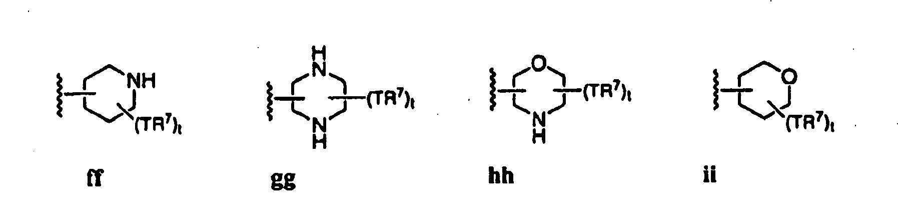 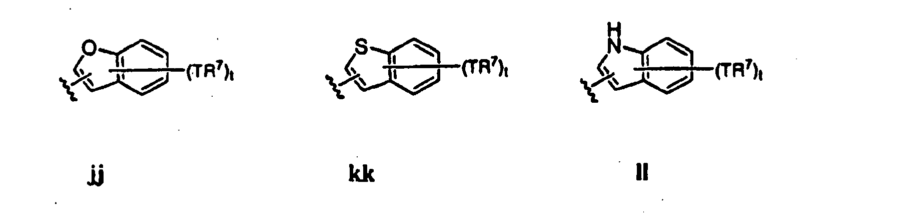 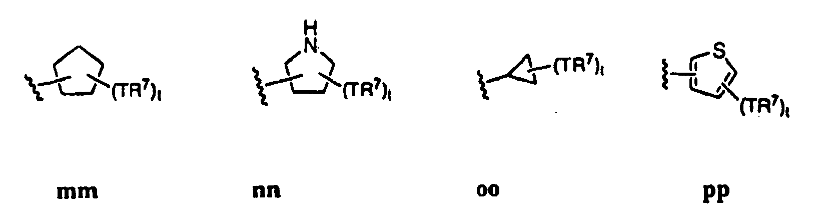 où t représente 0, 1, 2, 3, 4 ou 5, et où n'importe quel Ar₂ est lié à Q³ à travers n'importe quel atome d'azote ou de carbone pouvant être substitué, et où un ou plusieurs atomes d'hydrogène sur n'importe quel atome d'azote ou de carbone pouvant être substitué est substitué avec une ou plusieurs occurrences indépendantes de TR⁷ ; par exemple, où Ar₂ représente a, b, e, g, h, i, j, k, n, r, cc, dd, ff, jj, 11 ou pp ;
où :
(a) T est une liaison ou est une chaîne alkylidène éventuellement substituée en C_{1 à 6} dans laquelle un ou deux motifs méthylène sont éventuellement et indépendamment remplacés par -O-, -NR-, -S-, -SO₂-,-COO-, -CO-, -OSO₂-, -NRSO₂, -CONR-, ou -SO₂NR-, et R⁷ représente R' ou un halogène ; ou
(b) chaque occurrence de TR⁷ représente indépendamment un alkyle en C_{1 à 3}, -OR', -SR', -CF₃, -OCF₃, -SCF₃, -F, -Cl, I, -Br, -COOR', -COR', -O(CH₂)₄N(R)(R'), -O(CH₃)₃N(R)(R'), -O(CH₂)₂N(R)(R'), -O(CH₂)N(R)(R'), -O(CH₂)₄CON(R)(R'), -O(CH₂)₃CON(R)(R'), -O(CH₂)₂CON(R)(R'), -O(CH₂)CON(R)(R'), -C(O)N(R)(R'), -(CH₂)₄OR' -(CH₂)₃OR', -(CH₂)₃OR', -CH₂OR', un phényle ou benzyle éventuellement substitué, -N(R)(R'), -(CH₂)₄N(R)(R"), -(CH₂)₃N(R)(R'), -(CH₂)₂N(R)(R'). -(CH₂)N(R)(R'), ou SO₂N(R)(R'), NRSO₂R', CON(R)(R') ou -OSO₂R' ; ou
(3) R⁵ représente l'hydrogène, (CH₂)₃OR', (CH₂)₂OR', (CH₂)OR', (CH₂)₃N(R')2, (CH₂)₂N(R')₂, (CH₂)N(R')₂, ou un groupe aliphatique en C_{1 à 4}.

4. Composé selon la revendication 1, dans lequel chaque occurrence de R¹ représente indépendamment l'hydrogène, un halogène, un groupe aliphatique éventuellement substitué en C₁ à C₄, OR, SR, ou N(R)₂ ; de préférence dans lequel chaque occurrence de R¹ représente indépendamment l'hydrogène, un halogène, -CH₃, -CH₂CH₃, -OH, -OCH₃, -SCH₃, -NH₂, -N(CH₃)₂, -N(CH₂CH₃)₂, NH(CH₂)₂NHCH₃, NH(cyclopropyle), NH(CH₂)cyclopropyle ou NH(CH₂)₂N(CH3)₂.

5. Composé selon la revendication 1, dans lequel chaque occurrence de R₂ représente indépendamment l'hydrogène, un halogène, un groupe aliphatique en C₁ à C₄, OH, OR' ou N(R)(R') ;
de préférence dans lequel chaque occurrence de R^{Z} représente indépendamment l'hydrogène, un halogène, Me, OH, OMe, NH₂ ou N(Me)₂.

6. Composé selon la revendication 1, dans lequel les groupes R⁴ représentent chacun indépendamment l'hydrogène, un groupe aliphatique en C_{1 à 6}, CN, COR, C(=O)OR, C(=)N(R)₂ ou un halogène.

7. Composé selon la revendication 1, dans lequel

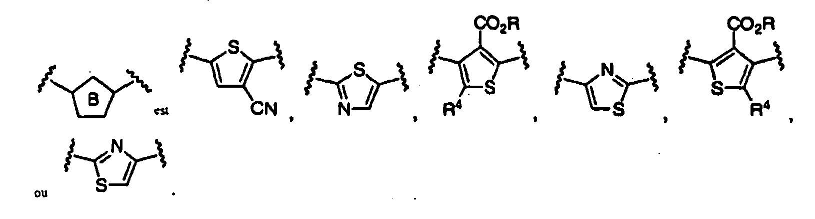

8. Composé selon la revendication 1, où ledit composé a une des formules XIV, XV, XVI, XVII, XVIII ou XIX :

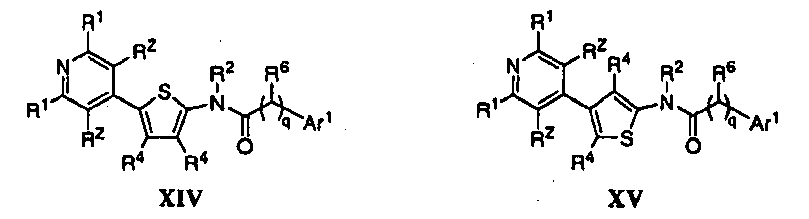

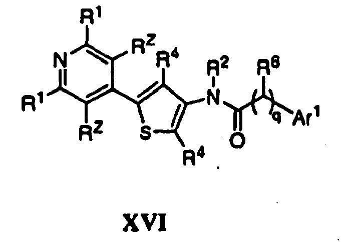

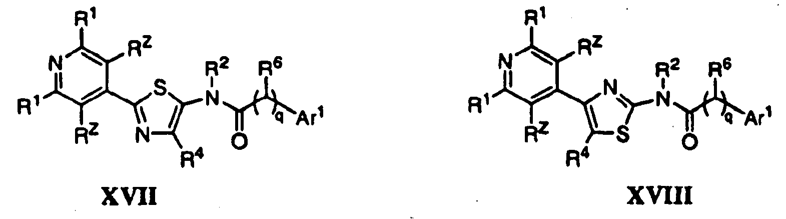

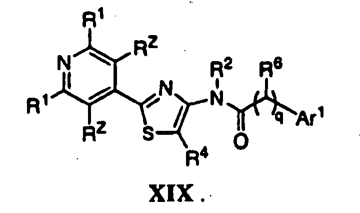

9. Composé selon la revendication 8, dans lequel les variables du composé sont choisies parmi un ou plusieurs des groupes suivants :
a) chaque occurrence de R¹ représente indépendamment l'hydrogène, un halogène, un groupe aliphatique éventuellement substitué en C₁ à C₄, OR, SR ou N(R)₂ ;
b) chaque occurrence de R¹ représente indépendamment l'hydrogène, un halogène, -CH₃, -CH₂CH₃, -OH, -OCH₃, -SCH₃, -NH₂, -N(CH₃)₂, -N(CH₂CH₃)₂, NH(CH₂)₂NHCH₃, NH(cyclopropyle), NH(CH₂)cyclopropyle ou NH(CH₂)₂N(CH₃)₂ ;
c) chaque occurrence de R^{z} représente indépendamment l'hydrogène, un halogène, un groupe aliphatique éventuellement substitué en C₁ à C₄, OH, O(R') ou N(R) (R') ;
d) chaque occurrence de R^{z} représente indépendamment l'hydrogène, un halogène, Me, OH, OMe, NH₂ ou N(Me)₂ ;
e) R² représente l'hydrogène ou représente UₙR', où n vaut 1, et U représente -CH₂-, -CH₂CH₂- , -CH₂CH₂CH₂-, -CH₂CH₂CH₂CH₂-, -CH₂O-, -CH₂S-, -CH₂NR-, -CH₂CH₂O-, -CH₂CH₂S-, -CH₂CH₂NR-, -CH₂CH₂CH₂O-, -CH₂CH₂CH₂S-, -CH₂CH₂CH₂NR-, -CH₂CH₂CH₂CH₂O-, -CH₂CH₂CH₂CH₂S-, -CH₂CH₂CH₂CH₂NR-, -CH₂CH₂OCH₂CH₂-, -(CH₂)₄NHCH₂-, -(CH₂)₃NHCH₂CH₂-, ou -CH₂CH₂NHCH₂CH₂-, et les groupes R' représentent l'hydrogène, un alkyle en C₁ à C₄, un tétrahydropyranyle éventuellement substitué, un pyrrolidinyle, un pipéridinyle, un pipérazinyle, un morpholinyle, un thiomorpholinyle, un pyridinyle, un phényle ou un cyclohexyle, ou R et R', pris ensemble avec l'atome d'azote auquel ils sont liés, forment un anneau hétérocyclyle éventuellement substitué à 5 ou 6 membres ;
f) chaque occurrence de R⁴ représente indépendamment l'hydrogène, un groupe aliphatique en C_{1 à 6}, CN, COR, COOR, CON(R)₂ ou un halogène ;
g) q vaut 1, 2 ou 3 ;
h) R⁶ représente R', -N(R)(R'), -(CH₂)₁₋₄N(R)(R'), -OR', -(CH₂)₁₋₄OR', -NR(CH₂)₁₋₄N(R)(R'), -NR(CH₂)₁₋₄SO₂R', -NR(CH₂)₁₋₄COOR' ou -NR(CH₂)₁₋₄COR', ou deux occurrences de R⁶, prises ensemble avec les atomes auxquelles elles sont liées, forment un anneau éventuellement substitué à 3 à 6 membres saturé, partiellement insaturé ou complètement insaturé ;
i) R⁶ représente CH₂OH, CH₂CH₂OH, OH, OMe, OEt, NH₂, NH(Me), NH(Et), N(Me)(Me), CH₂NH₂, CH₂CH₂NH₂, NHCO₂t-butyle, phényle, cyclopentyle, méthyle, éthyle, isopropyle, cyclopropyle, NH(CH₂)₃NH₂, NH(CH₂)₂NH₂, NH(CH₂)₂NHEt, NHCH₂pyridyl, NHSO₂phényle, NHC(O)CH₂C(O)Ot-butyle, NHC(O)CH₂NH₃ et NHCH₂-imidazol-4-yle ;
j) Ar¹ est un anneau a, b, e, g, h, i, j, k, r, cc, dd, ff, jj, 11 ou pp, où t représente 0, 1, 2 ou 3, et T est une liaison ou est une chaîne alkylidène éventuellement substituée en C_{1 à 6} dans laquelle un ou deux motifs méthylène sont éventuellement et indépendamment remplacés par -O-, -NR-, -S-, -SO₂-,-COO-, -CO-, -OSO₂-, -NRSO₂, -CONR-, ou -SO₂NR- et R₇ représente R' ou un halogène ; ou
k) Ar¹ est un anneau a, b, e, g, h, i, j, k, r, cc, dd, ff, jj, ll ou pp, dans lequel t représente 0, 1, 2 ou 3, et chaque occurrence de TR⁷ représente indépendamment un alkyle en C_{1 à 3}, -OR', -SR', -CF₃, -OCF₃, -SCF₃, -F, -Cl, I, -Br, -COOR', -COR', -O(CH₂)₄N(R)(R'), -O(CH₂)₃N(R)(R'), -O(CH₂)₂N(R)(R'), -O(CH₂)N(R)(R'), -O(CH₂)₄CON(R)(R'), -O(CH₂)₃CON(R)(R'), -O(CH₂)₂CON(R)(R'), -O(CH₂)CON(R)(R'), -C(O)N(R)(R'), -(CH₂)₄OR' - (CH₂)₃OR', -(CH₂)₂OR', -CH₂OR', un phényle ou benzyle éventuellement substitué, -N(R)(R'), -(CH₂)₄N(R)(R'), -(CH₂)₃N(R)(R'), -(CH₂)₂N(R)(R'), -(CH₂)N(R)(R'), ou SO₂N(R)(R'), NRSO₂R', CON(R)(R') ou -OSO₂R'.

10. Composé selon la revendication 8, dans lequel q vaut 1, et Ar¹ représente un phényle éventuellement substitué et un composé de formule générale XIV-A à XIX-A est fourni : 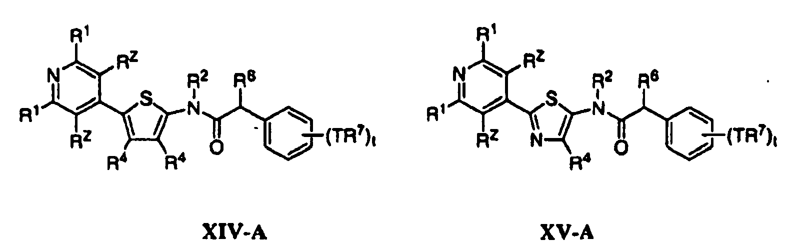 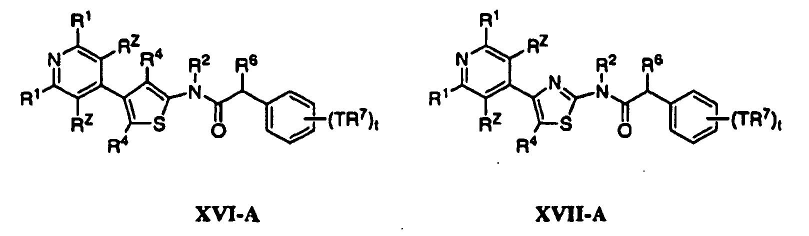 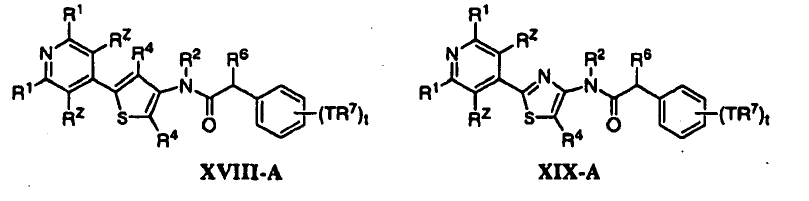 où
chaque occurrence de R¹ représente l'hydrogène ;
chaque occurrence de R^{Z} représente l'hydrogène ;
R² représente l'hydrogène ou représente UₙR', où n vaut 1, et U représente -CH₂-, -CH₂CH₂-, -CH₂CH₂CH₂-, -CH₂CH₂CH₂CH₂-, -CH₂O-, -CH₂S-, -CH₂NR-, -CH₂CH₂O-, -CH₂CH₂S-, -CH₂CH₂NR-, -CH₂CH₂CH₂O-, -CH₂CH₂CH₂S-, -CH₂CH₂CH₂NR-, -CH₂CH₂CH₂CH₂O-, -CH₂CH₂CH₂CH₂S-, -CH₂CH₂CH₂CH₂NR-, -CH₂CH₂OCH₂CH₂-, - (CH₂)₄NHCH₂-, - (CH₂)₃NHCH₂CH₂-, ou -CH₂CH₂NHCH₂CH₂-, et les groupes R' représentent l'hydrogène, un alkyle en C₁ à C₄, un tétrahydropyranyle éventuellement substitué, un pyrrolidinyle, un pipéridinyle, un pipérazinyle, un morpholinyle, un thiomorpholinyle, un pyridinyle, un phényle ou un cyclohexyle, ou R et R', pris ensemble avec l'atome d'azote auquel ils sont liés, forment un anneau hétérocyclyle éventuellement substitué à 5 ou 6 membres ;
chaque occurrence de R⁴ représente indépendamment l'hydrogène, un groupe aliphatique en C_{1 à 6}, CN, COR, COOR, CON(R)₂ ou un halogène ;
R⁶ représente R', -N(R)(R'), -(CH₂)₁₋₄N(R)(R'), -OR', -(CH₂)₁₋₄OR', -NR(CH₂)₁₋₄N(R)(R'), -NR(CH₂)₁₋₄SO2R', -NR(CH₂)₁₋₄COOR' ou -NR(CH₂)₁₋₄COR' ; et
t représente 0, 1, 2 ou 3, et chaque occurrence de TR⁷ représente indépendamment un alkyle en C_{1 à 3}, -OR', -SR', -CF₃, -OCF₃, -SCF₃, -F, -Cl, I, -Br, -COOR', -COR', -O(CH₂)₄N(R)(R'), -O(CH₂)₃N(R)(R'), -O(CH₂)₂N(R)(R'), -O(CH₂)N(R)(R'), -O(CH₂)₄CON(R)(R'), -O(CH₂)₃CON(R)(R'), -O(CH₂)₂CON(R)(R'), -O(CH₂)CON(R)(R'), -C(O)N(R)(R'), -(CH₂)₄OR' -(CH₂)₃OR', -(CH₂)₂OR', -CH₂OR', un phényle ou benzyle éventuellement substitué, -N(R)(R'), -(CH₂)₄N(R)(R'), -(CH₂)₃N(R)(R'), -(CH₂)₂N(R)(R'), -(CH₂)N(R)(R'), ou SO₂N(R)(R'), NRSO₂R', CON(R)(R') ou -OSO₂R'.

11. Composé selon la revendication 1, dans lequel
(1) R² et Q¹-R³, pris ensemble avec les atomes auxquels ils sont liés forment un groupe cyclique à 5 membres, et les composés ont la formule générale XX à XXXI : 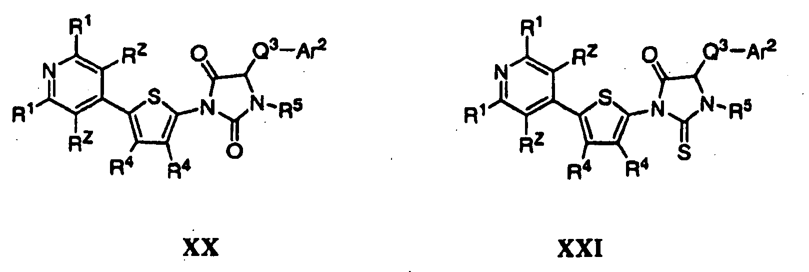 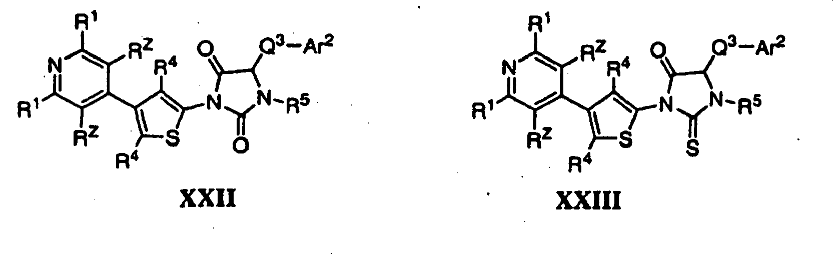 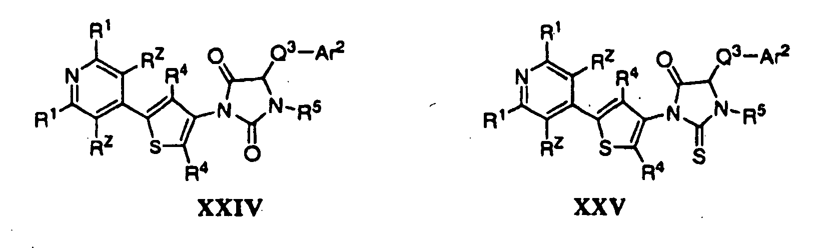 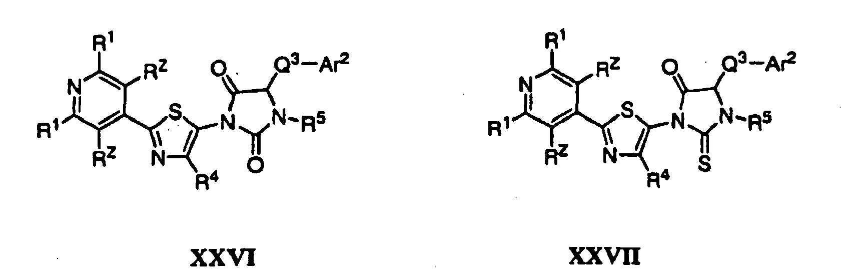 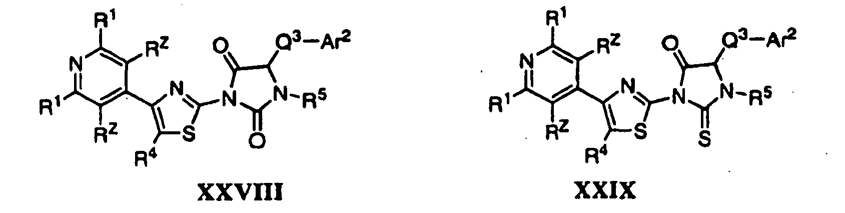 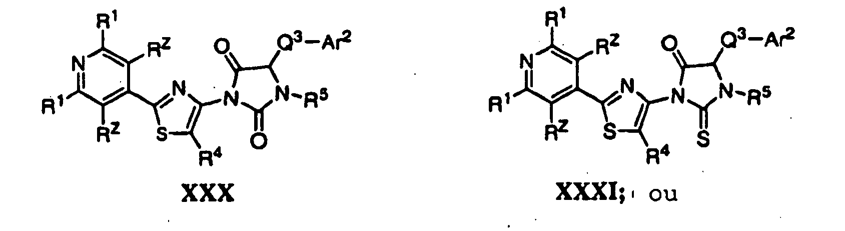
(2) R² et Q¹-R³, pris ensemble avec les atomes auxquels ils sont liés forment un groupe cyclique à 6 membres, et les composés ont la formule générale XXXII à XXXVII : 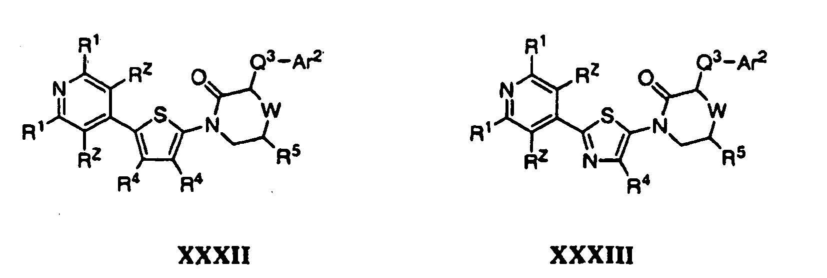 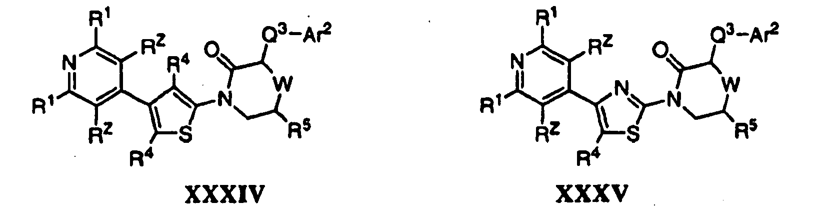 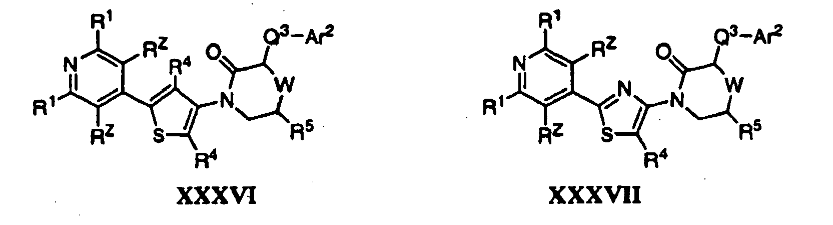
où W représente O, NR⁵ ou CHR⁵.

12. Composé selon la revendication 11, dans lequel les variables du composé sont choisies parmi un ou plusieurs des groupes suivants :
a) chaque occurrence de R¹ représente indépendamment l'hydrogène, un halogène, un groupe aliphatique éventuellement substitué en C₁ à C₄, OR, SR ou N(R)₂ ;
b) chaque occurrence de R^{Z} représente indépendamment l'hydrogène, un halogène, un groupe aliphatique éventuellement substitué en C₁ à C₄, OH, OR' ou N(R)(R') ;
c) chaque occurrence de R⁴ représente indépendamment l'hydrogène, un groupe aliphatique en C_{1 à 6}, CN, COR, COOR, CON(R)₂ ou un halogène ;
d) R⁵ représente l'hydrogène, (CH₂)₃OR', (CH₂)₂OR', (CH₂)OR', (CH₂)₃N(R')₂, (CH₂)₂N(R')₂, (CH₂)N(R')₂. ou un groupe aliphatique en C_{1 à 4}.
e) Q³ est une liaison directe, ou représente -(CHR⁶)_{q}-, -(CHR⁶)_{q}O-, -(CHR⁶)_{q}S-, -(CHR⁶)_{q}S(O)₂-, -(CHR⁶)_{q}S(O)-, -(CHR⁶)_{q}NR- ou -(CHR⁶)_{q}C(O)-, où q représente 0, 1, 2 ou 3 ; et
f) Ar² est un anneau a, b, e, g, h, i, j, k, n, r, cc, dd, ff, jj, 11 ou pp, où t représente 0, 1, 2 ou 3, et T est une liaison ou est une chaîne alkylidène éventuellement substituée en C_{1 à 6} dans laquelle un ou deux motifs méthylène sont éventuellement et indépendamment remplacés par -O-, -NR-, -S-, -SO₂-,-COO-, -CO-, -OSO₂-, -NRSO₂, -CONR-, ou -SO₂NR- et R⁷ représente R' ou un halogène ;

13. Composé selon la revendication 11, dans lequel les variables du composé sont choisies parmi un ou plusieurs des groupes suivants :
a) chaque occurrence de R¹ représente indépendamment l'hydrogène, un halogène, -CH₃, -CH₂CH₃, -OH, -OCH₃, -SCH₃, -NH₂, -N(CH₃)₂, -N(CH₂CH₃)₂, NH(CH₂)₂NHCH₃, NH(cyclopropyle), NH(CH₂)cyclopropyle ou NH(CH₂)₂N(CH₃)₂ ;
b) chaque occurrence de R^{z} représente indépendamment l'hydrogène, un halogène, Me, OH, OMe, NH₂ ou N(Me)₂ ;
c) chaque occurrence de R⁴ représente indépendamment l'hydrogène, un groupe aliphatique en C_{1 à 6}, CN, COR, COOR, CON(R)₂ ou un halogène ;
d) R⁵ représente l'hydrogène, (CH₂)₃OR' , (CH₂)₂OR', (CH₂)OR', (CH₂)₃N(R')₂, (CH₂)₂N(R')₂, (CH₂)N(R')₂, ou un groupe aliphatique en C_{1 à 4} ;
e) Q³ est une liaison directe, ou représente -(CHR⁶)_{q}-, -(CHR⁶)_{q}O-, -(CHR⁶)_{q}S-, -(CHR⁶)_{q}S(O)₂-, -(CHR⁶)_{q}S(O)-, - (CHR⁶)_{q}NR- ou , -(CHR⁶)_{q}C(O)-, où q représente 0, 1, 2 ou 3 ; et
f) Ar² est un anneau a, b, e, g, h, i, j, k, n, r, cc, dd, ff, jj, 11 ou pp, dans lequel t représente 0, 1, 2 ou 3, et chaque occurrence de TR⁷ représente indépendamment un alkyle en C_{1 à 3}, -OR', -SR', -CF₃, -OCF₃, -SCF₃, -F, -Cl, I, -Br, -COOR', -COR', -O(CH₂)₄N(R)(R'), -O(CH₂)₃N(R)(R'), -O(CH₂)₂N(R)(R'), -O(CH₂)N(R)(R'), -O(CH₂)₄CON(R)(R'), -O(CH₂)₃CON(R)(R'), -O(CH₂)₂CON(R)(R'), -O(CH₂)CON(R)(R'), -C(O)N(R)(R'), -(CH₂₎₄OR', -(CH₂)₃OR', -(CH₂)₂OR', -CH₂OR', un phényle ou benzyle éventuellement substitué, -N(R)(R'), -(CH₂)₄N(R)(R'), -(CH₂)₃N(R)(R'), -(CH₂)₂N(R)(R'), -(CH₂)N(R)(R'), ou SO₂N(R)(R'), NRSO₂R', CON(R)(R') ou-OSO₂R'.

14. Composé selon la revendication 11, dans lequel Ar² représente un phényle éventuellement substitué et les composés de formule générale XX-A à XXXVII sont fournis :

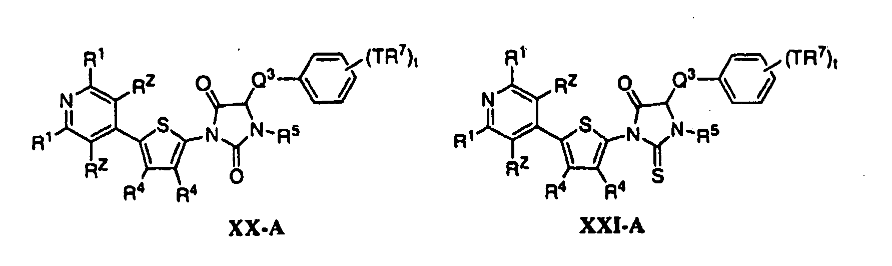

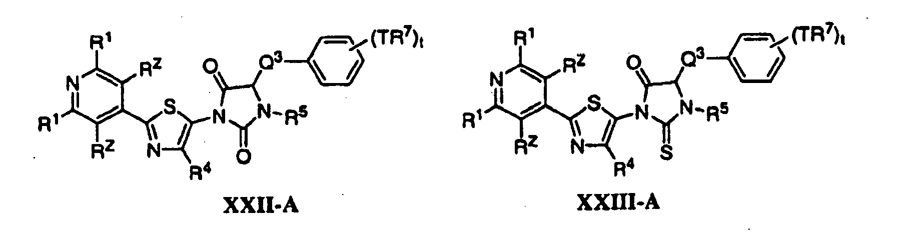

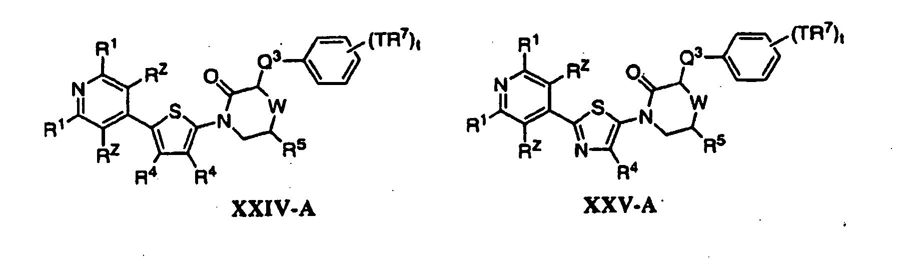

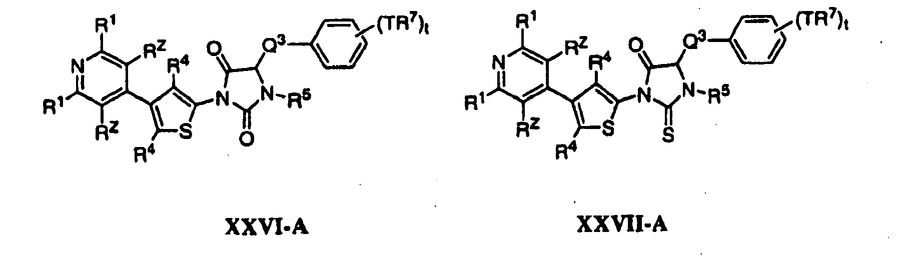

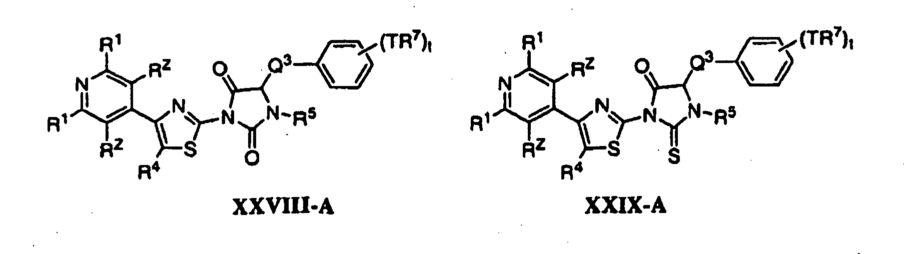

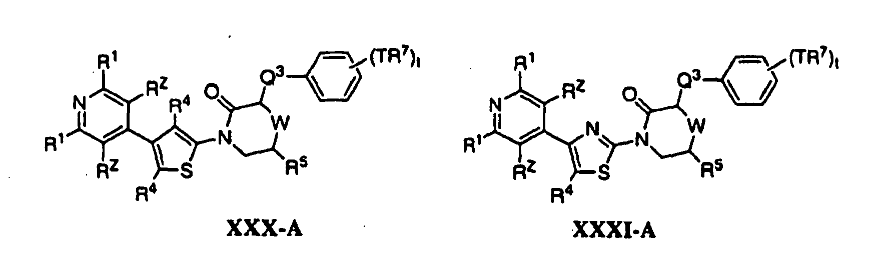

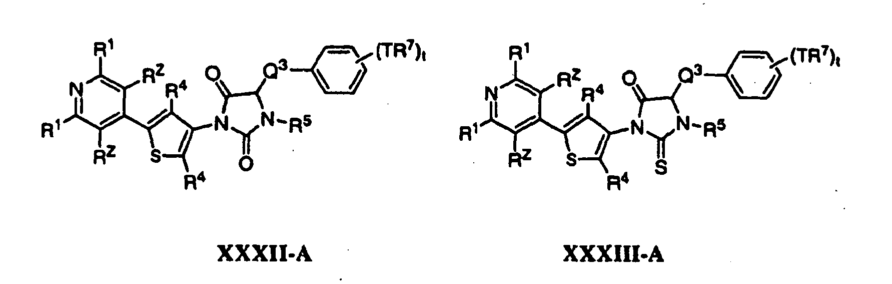

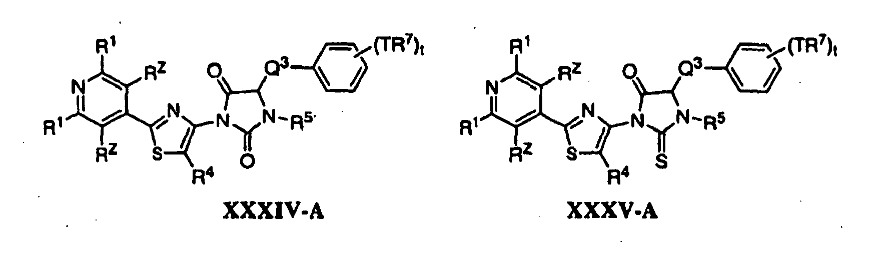

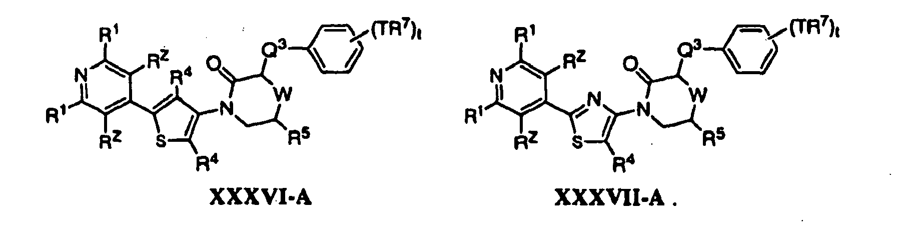

15. Composé selon la revendication 14, dans lequel les variables du composé sont choisies parmi :
chaque occurrence de R¹ représente l'hydrogène ;
chaque occurrence de R^{Z} représente l'hydrogène ;
chaque occurrence de R⁴ représente indépendamment l'hydrogène, un groupe aliphatique en C_{1 à 6}, CN, COR, COOR, CON(R)₂ ou un halogène ;
R⁵ représente l'hydrogène, (CH₂)₃OR', (CH₂)₂OR', (CH₂)OR', (CH₂)₃N(R')_{2,} (CH₂)₂N(R')₂, (CH₂)N(R')₂, ou un groupe aliphatique en C_{1 à 4}.
e) Q³ est une liaison directe, ou représente -(CHR⁶)_{q}-, -(CHR⁶)_{q}O-, -(CHR⁶)_{q}S-, -(CHR⁶)_{q}S(O)₂-, -(CHR⁶)_{q}S(O)-, -(CHR⁶)_{q}NR- ou -(CHR⁶)_{q}C(O)-, où q représente 0, 1, 2 ou 3 ; et
t représente 0, 1, 2 ou 3, et chaque occurrence de TR⁷ représente indépendamment un alkyle en C_{1 à 3}, -OR', -SR', -CF₃, -OCF₃, -SCF₃, -F, -Cl, I, -Br, -COOR', -COR', -O(CH₂)₄N(R)(R'), -O(CH₂)₃N(R)(R'), -O(CH₂)₂N(R)(R'), -O(CH₂)N(R)(R'), -O(CH₂)₄CON(R)(R'), -O(CH₂)₃CON(R)(R'), -O(CH₂)₂CON(R)(R'), -O(CH₂)CON(R)(R'), -C(O)N(R)(R'), -(CH₂)₄OR' -(CH₂)₃OR', -(CH₂)₂OR', -CH₂OR', un phényle ou benzyle éventuellement substitué, -N(R)(R'), -(CH₂)₄N(R)(R'), -(CH₂)₃N(R)(R'), -(CH₂)₂N(R)(R'), -(CH₂)N(R)(R'), ou SO₂N(R)(R'), NRSO₂R', CON(R)(R') ou -OSO₂R'.

16. Composé selon la revendication 1, ayant une des structures :

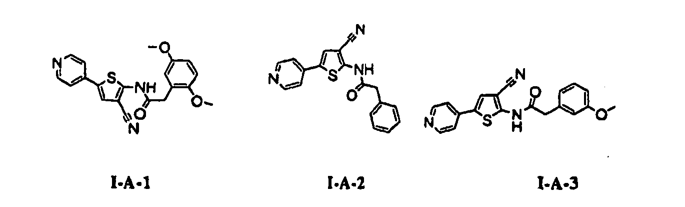

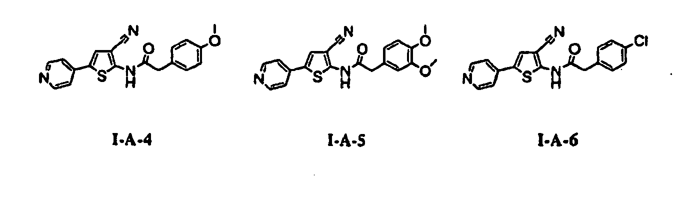

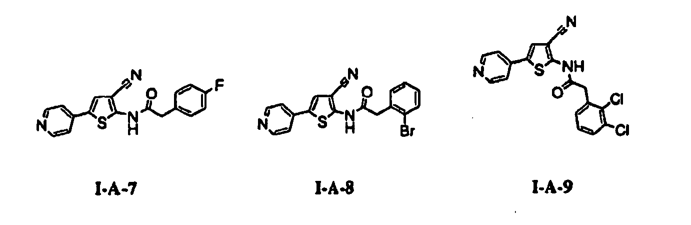

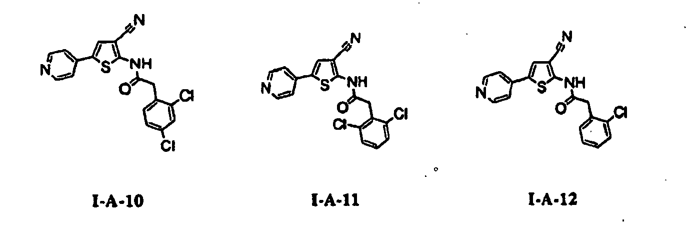

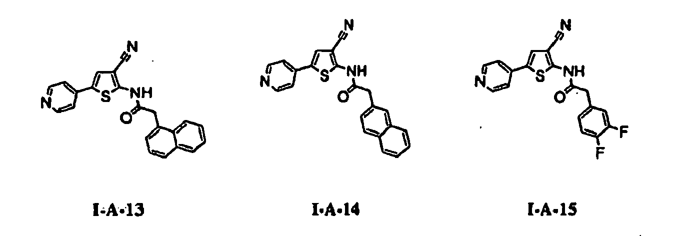

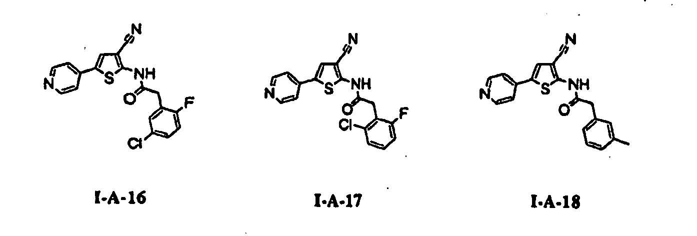

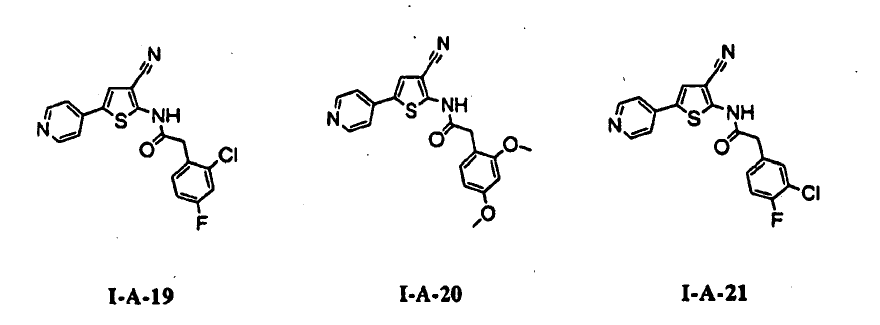

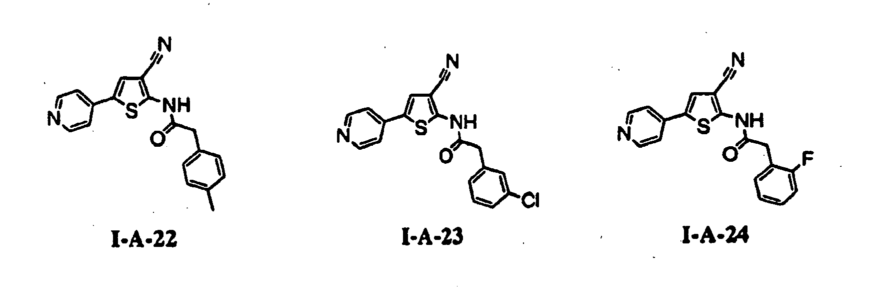

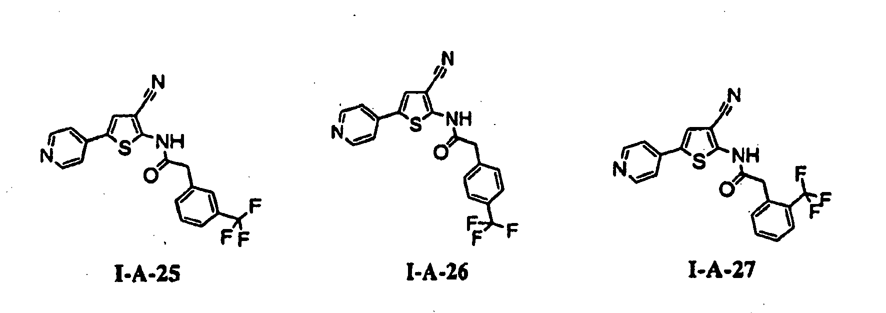

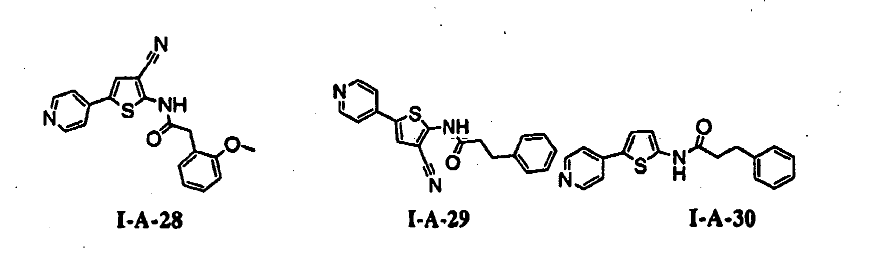

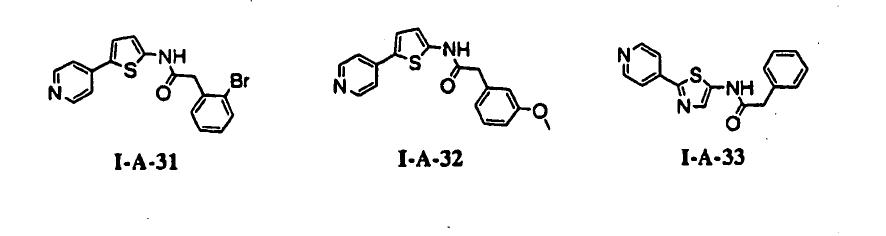

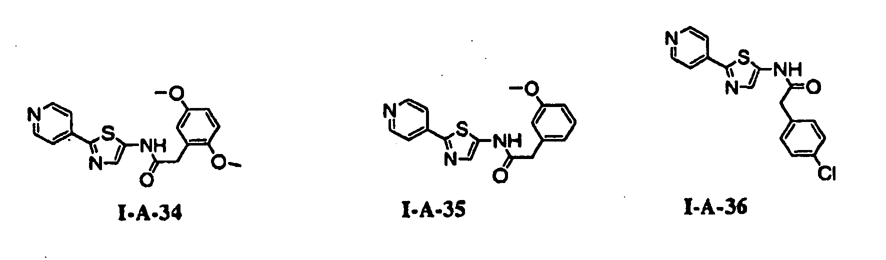

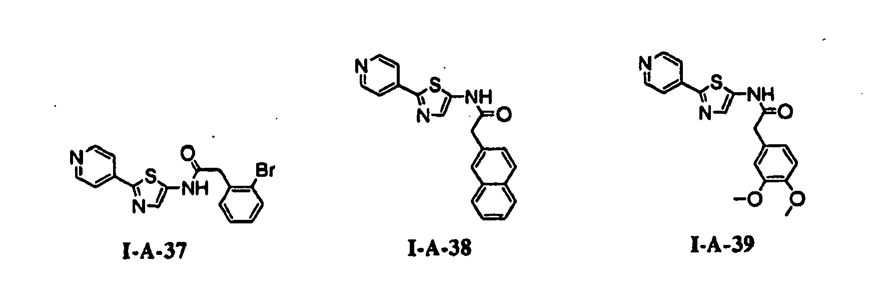

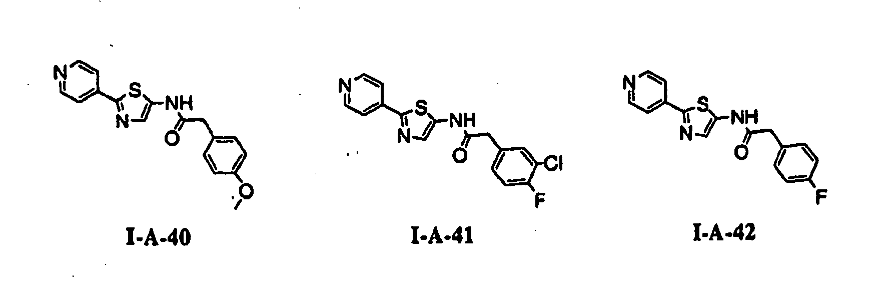

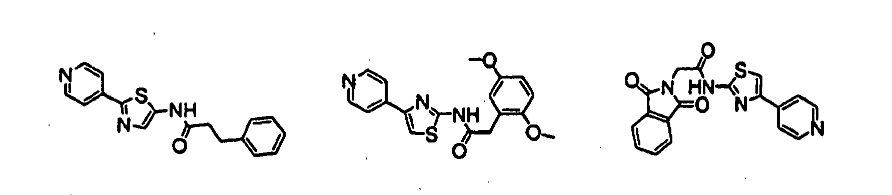

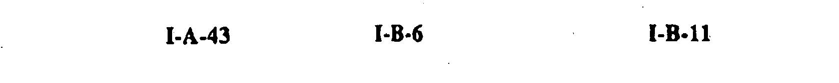

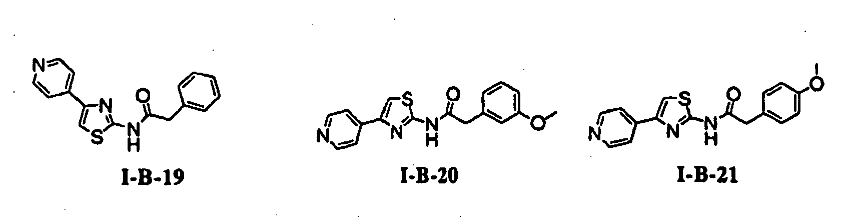

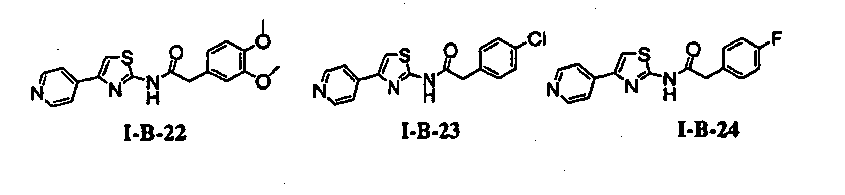

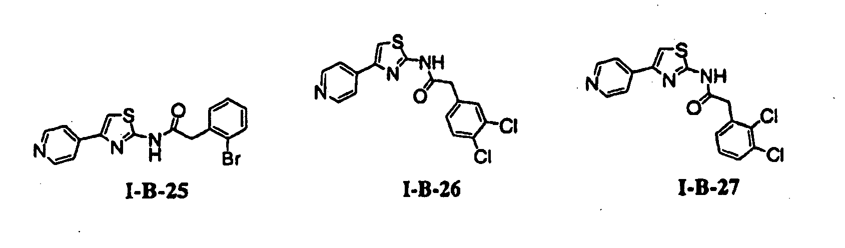

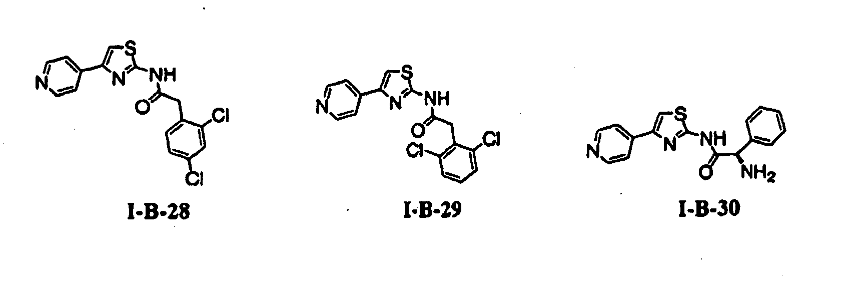

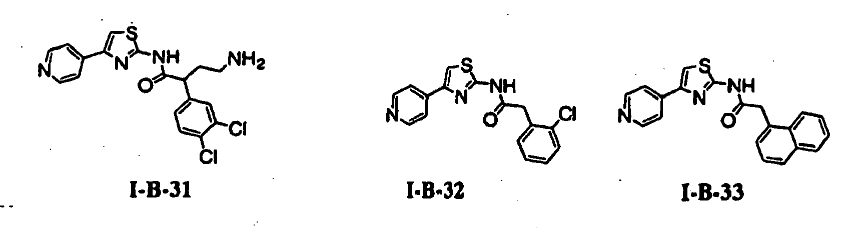

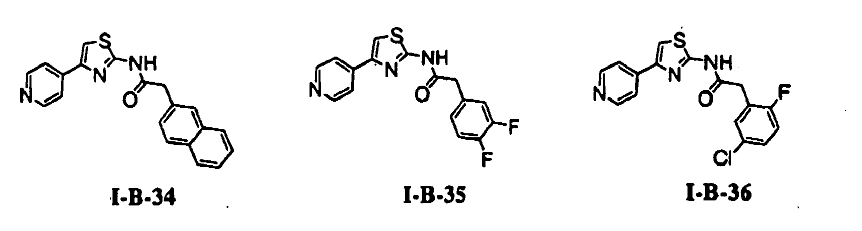

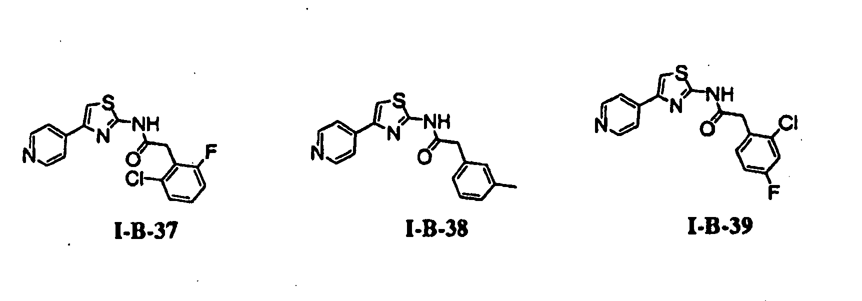

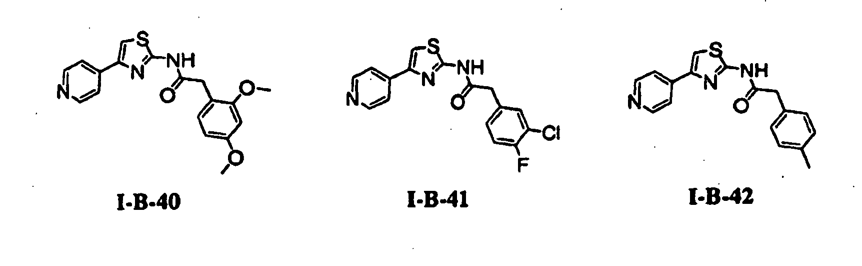

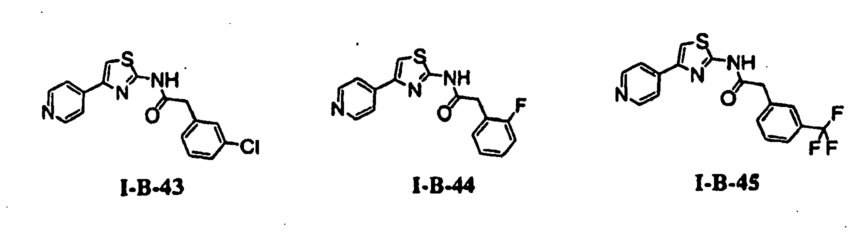

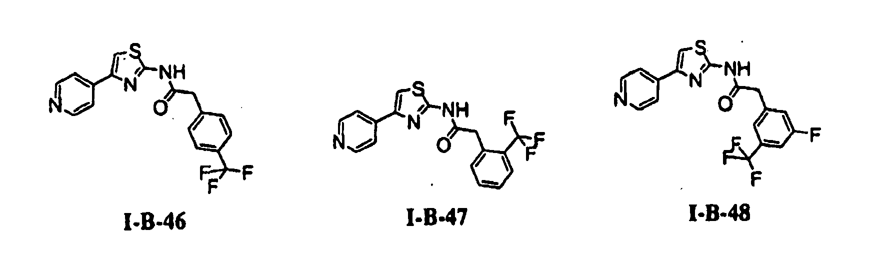

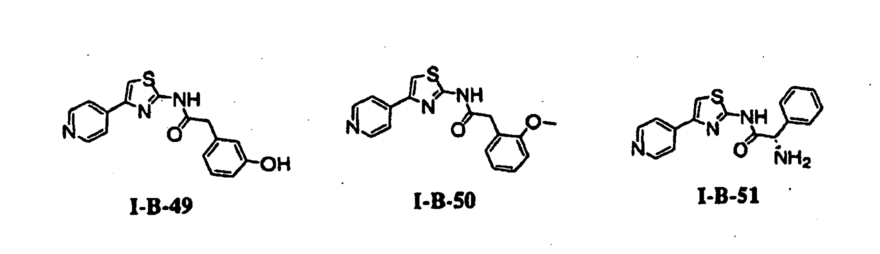

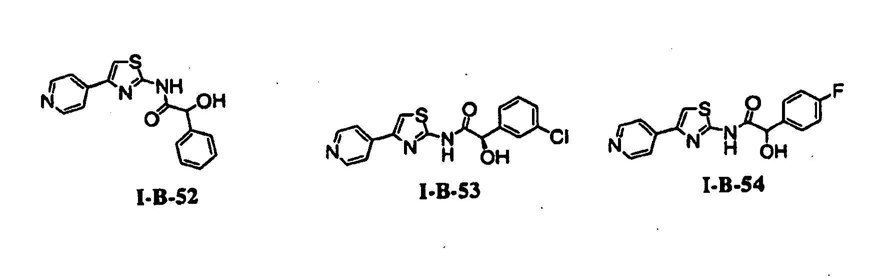

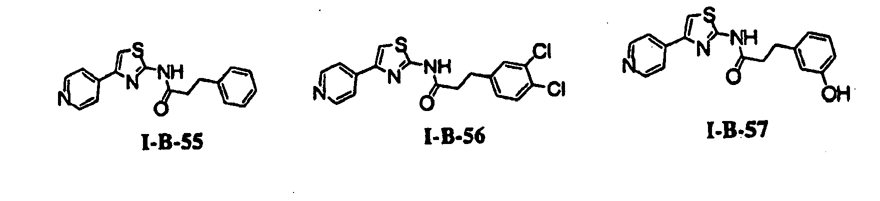

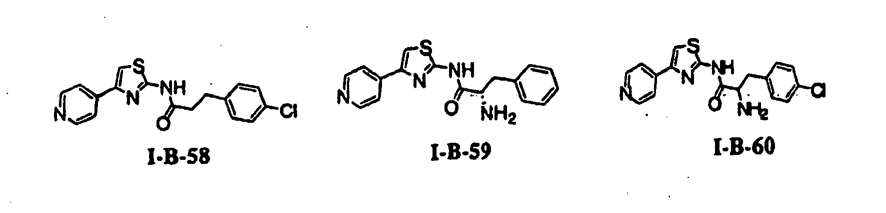

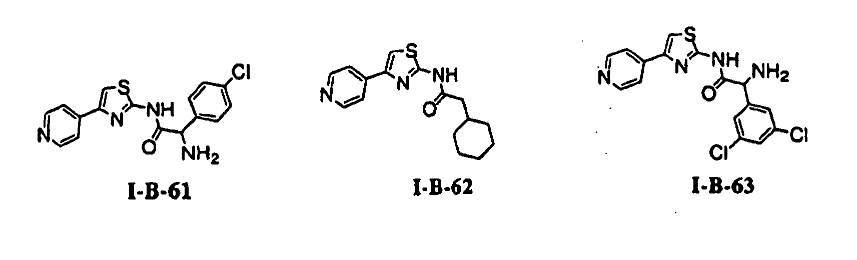

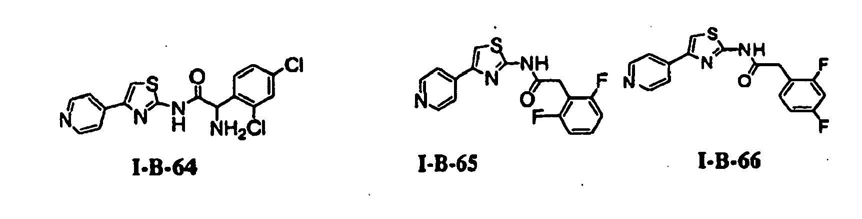

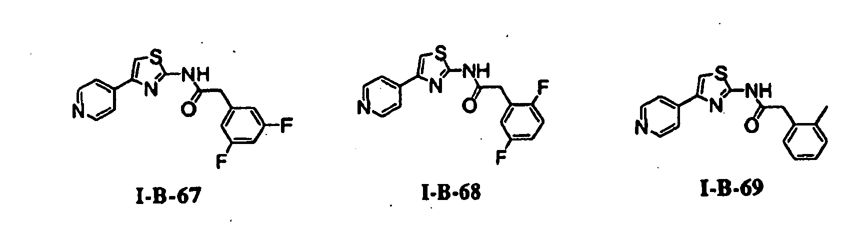

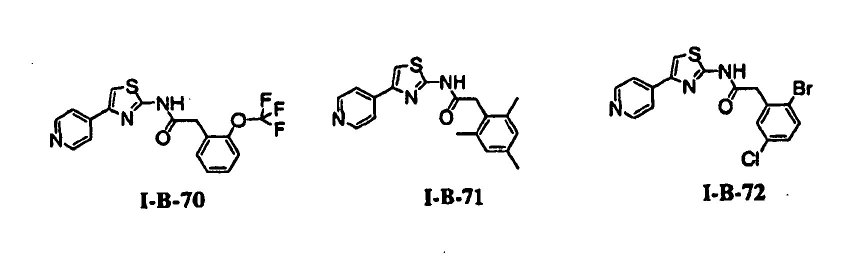

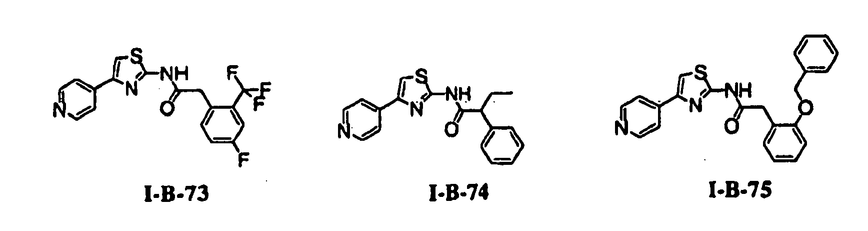

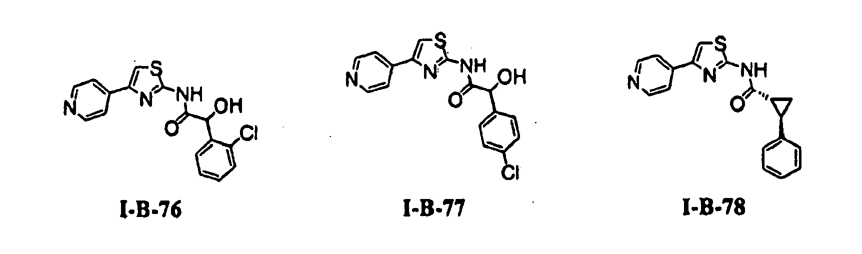

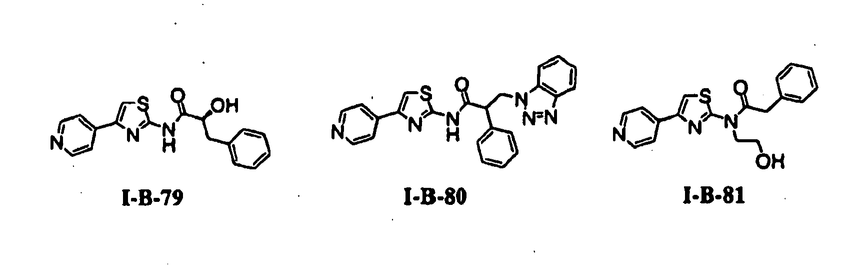

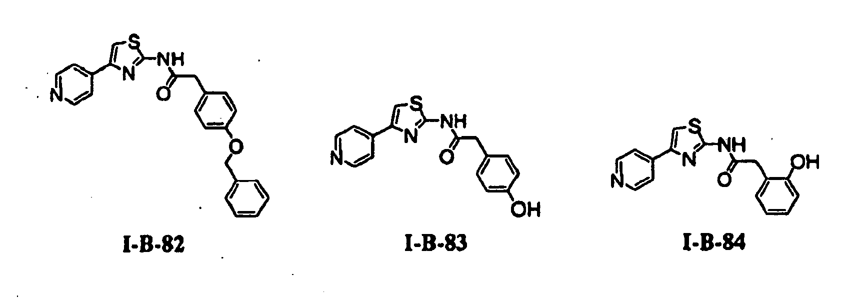

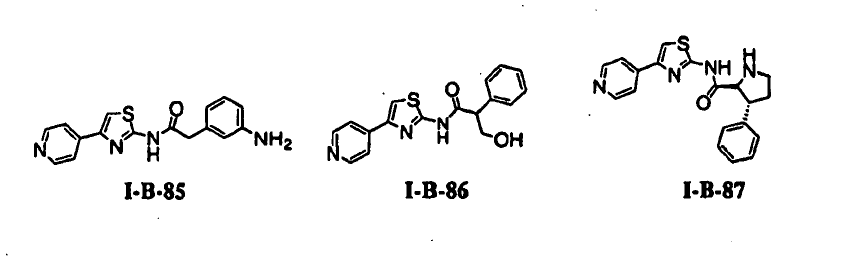

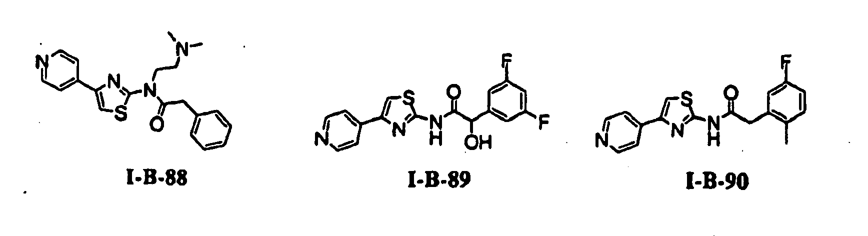

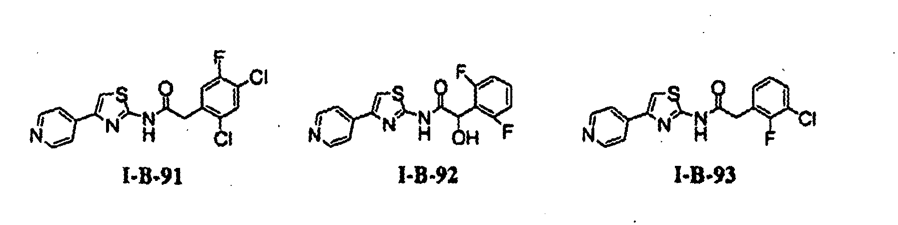

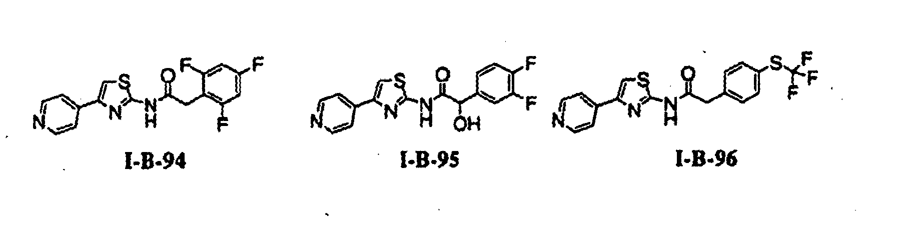

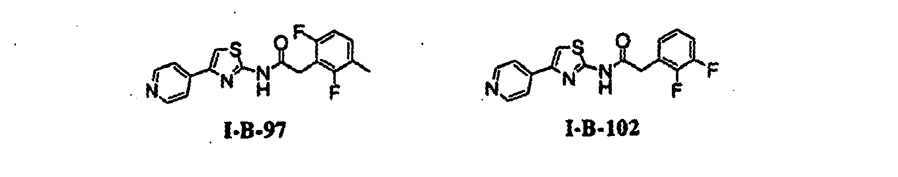

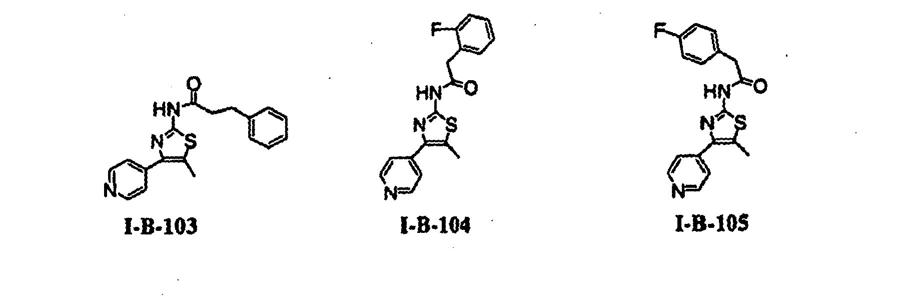

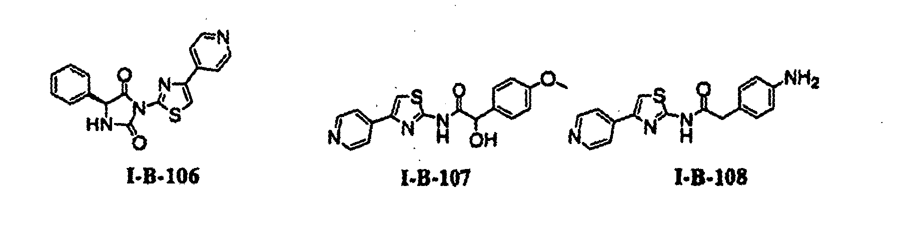

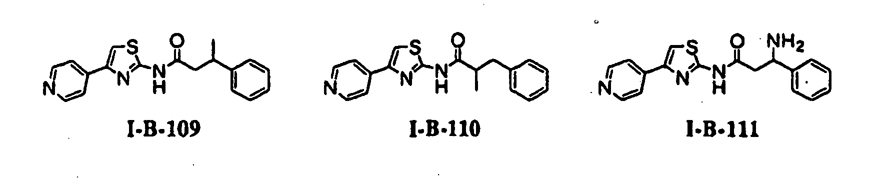

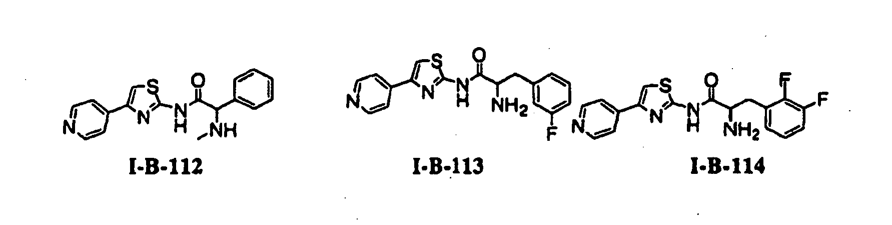

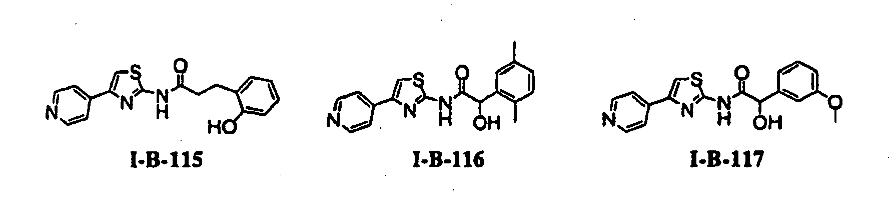

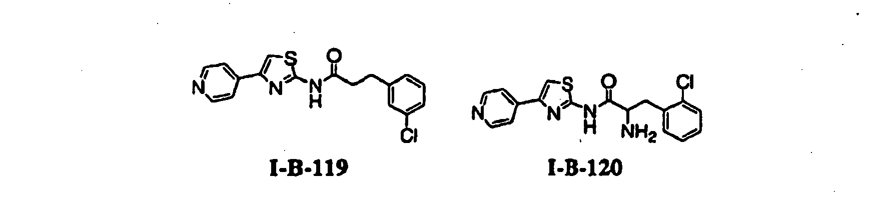

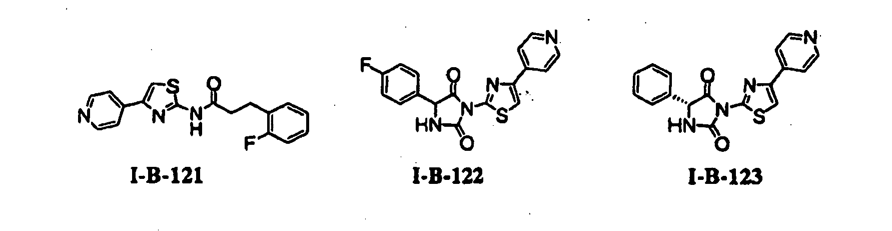

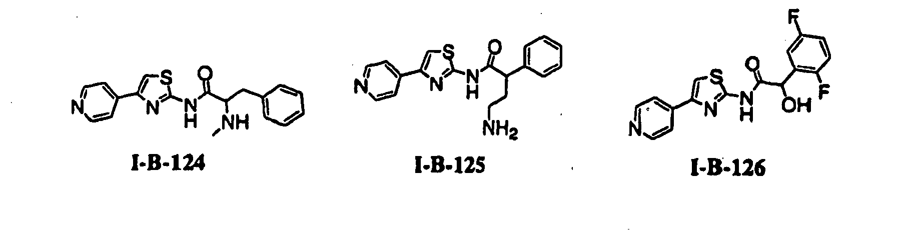

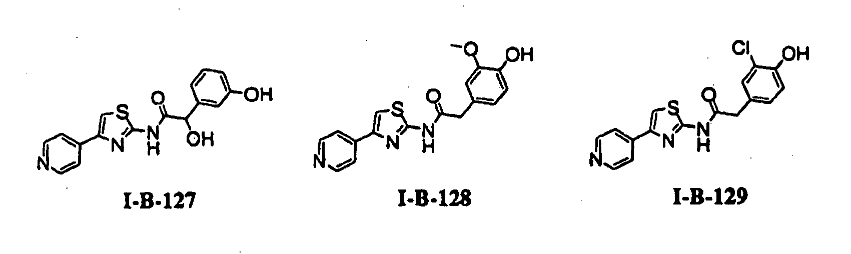

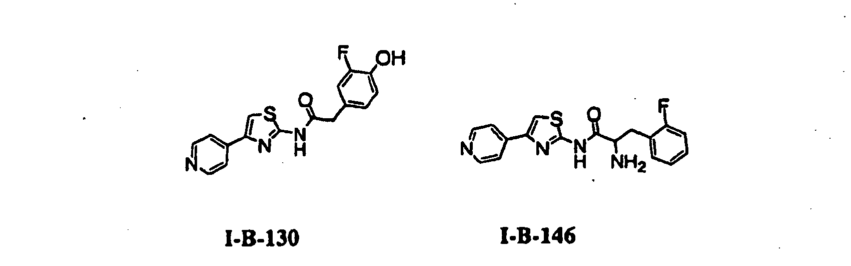

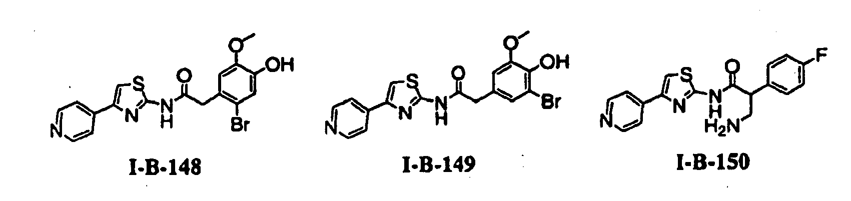

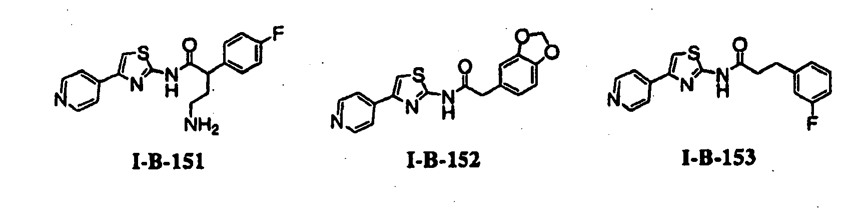

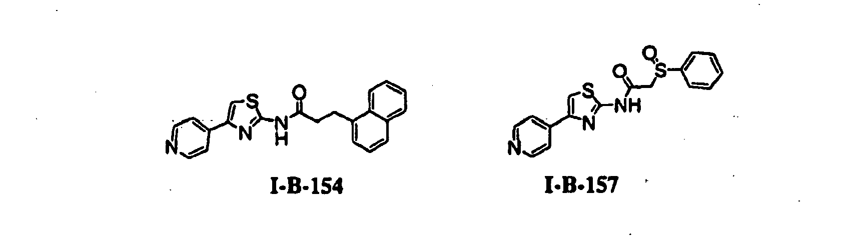

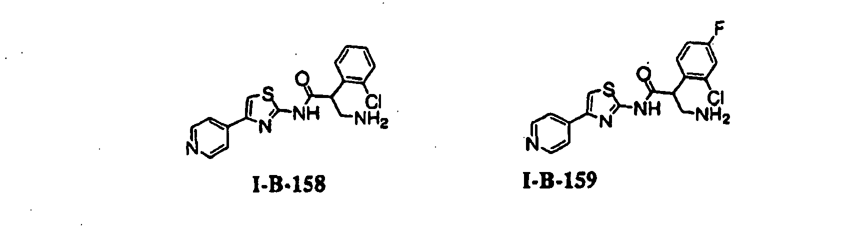

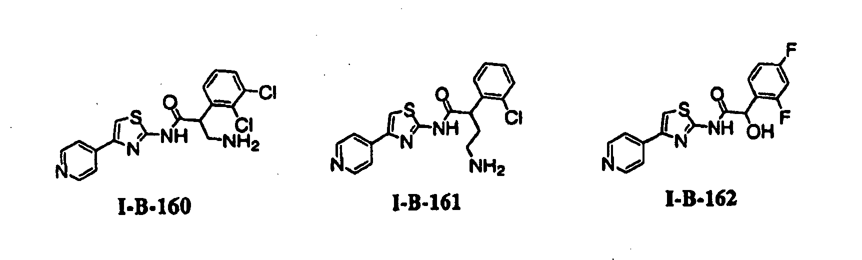

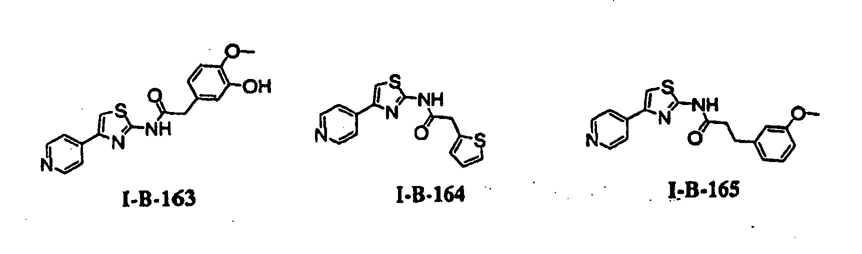

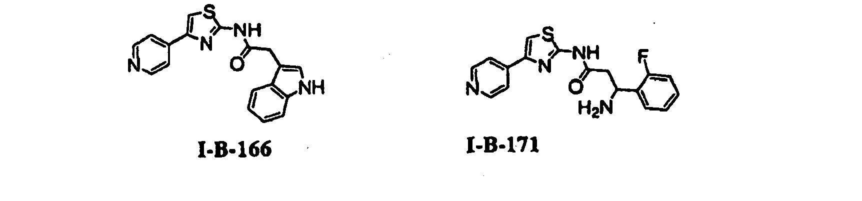

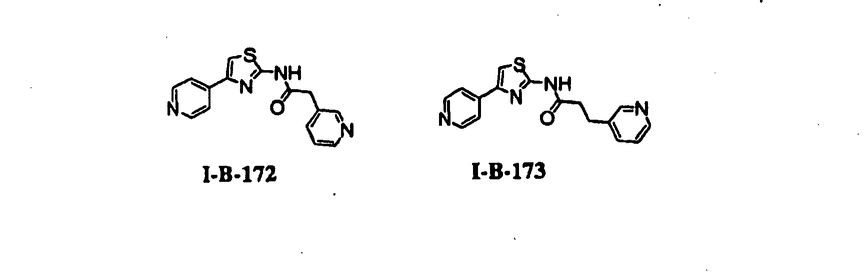

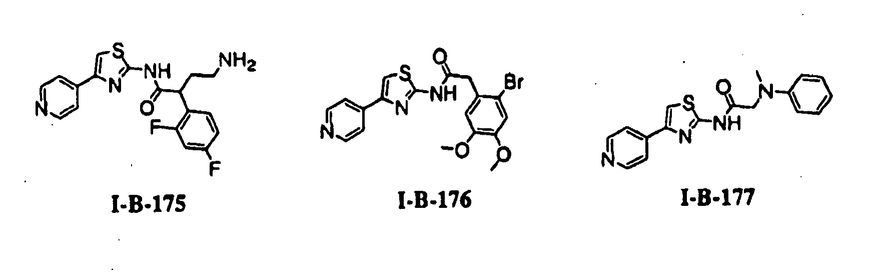

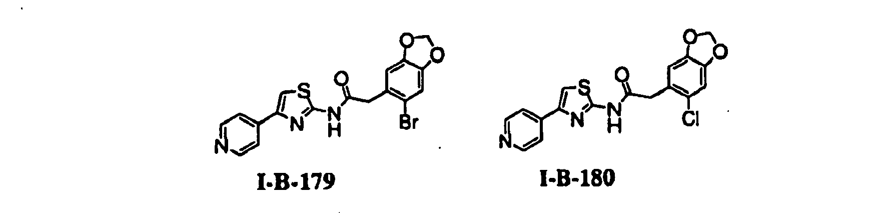

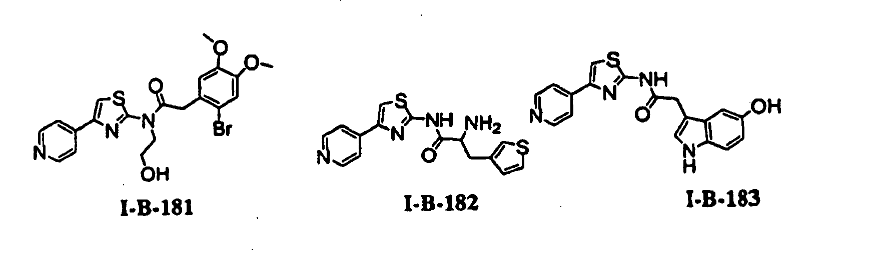

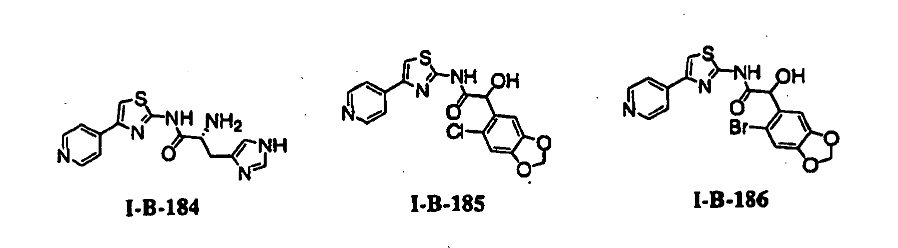

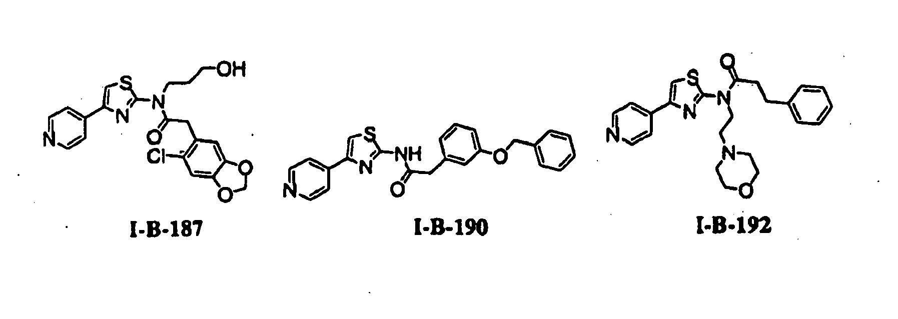

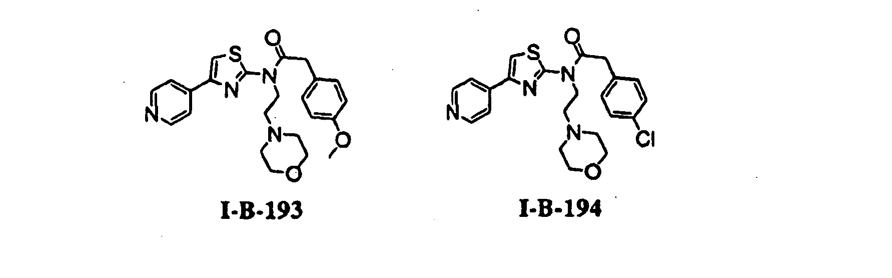

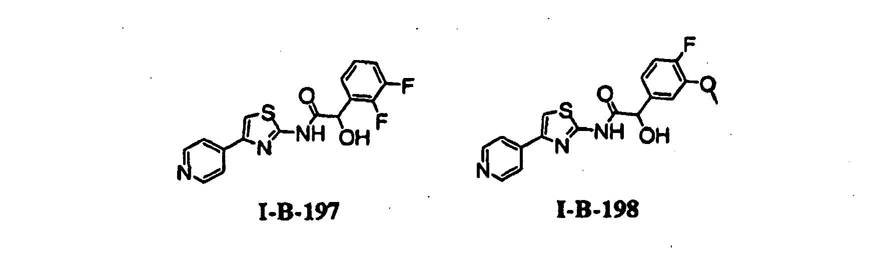

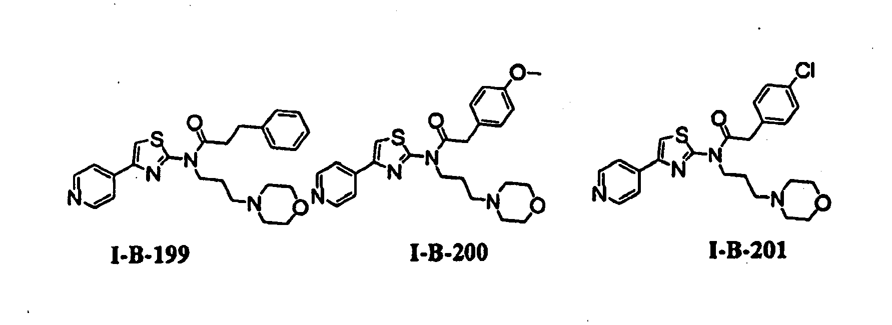

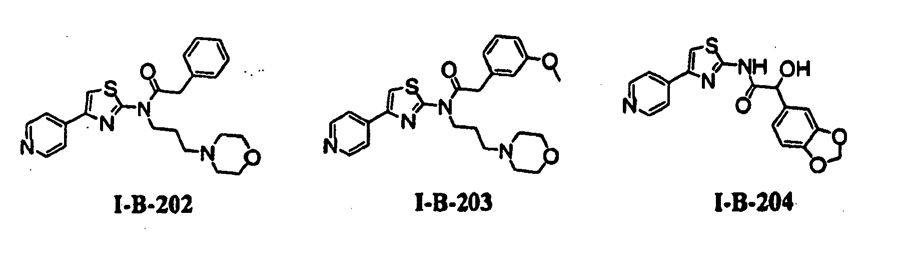

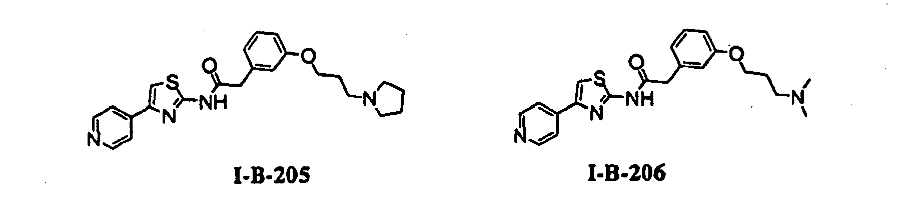

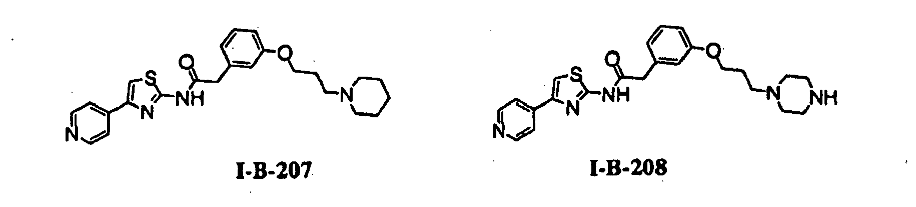

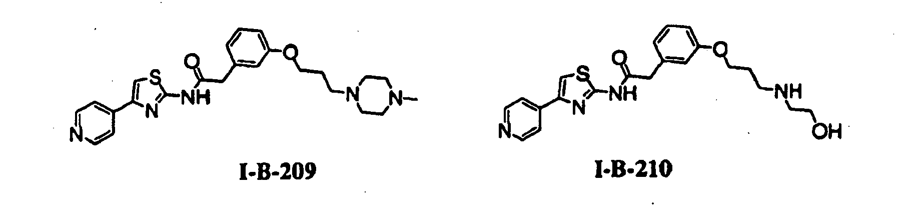

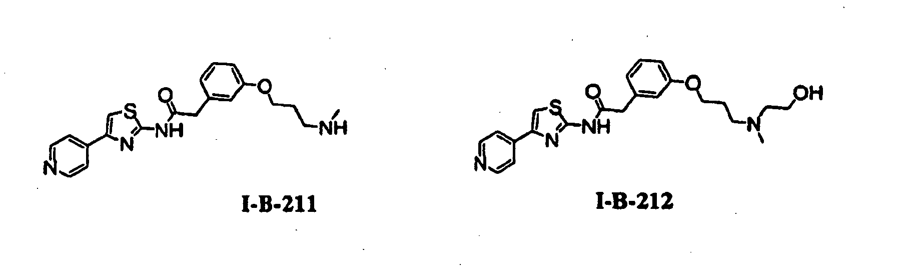

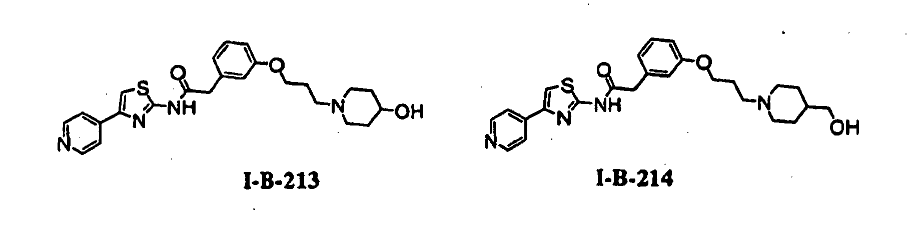

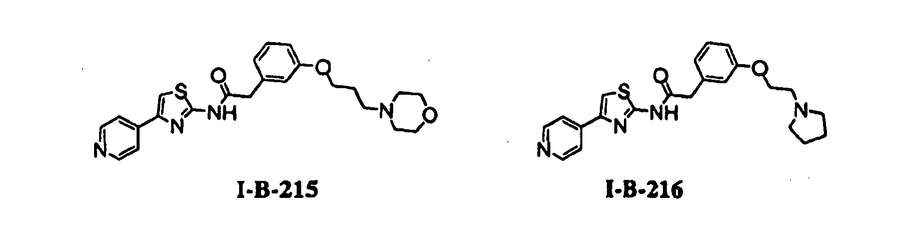

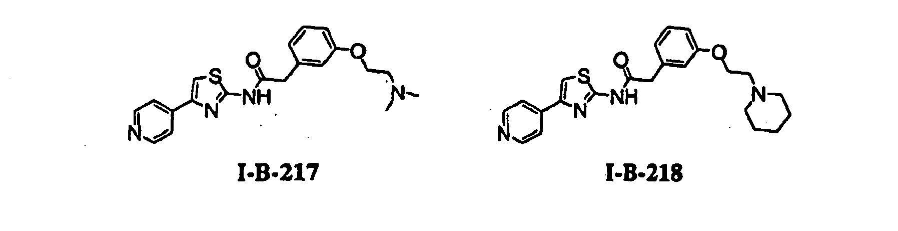

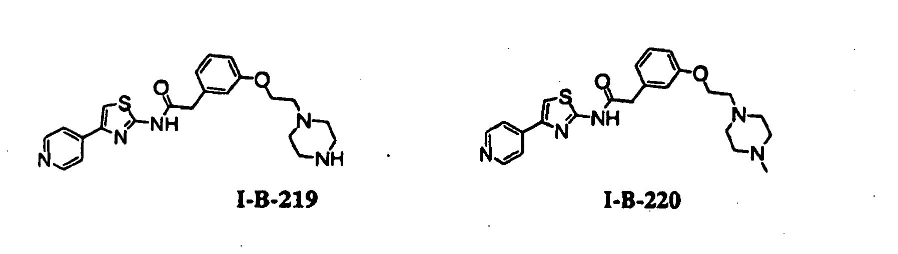

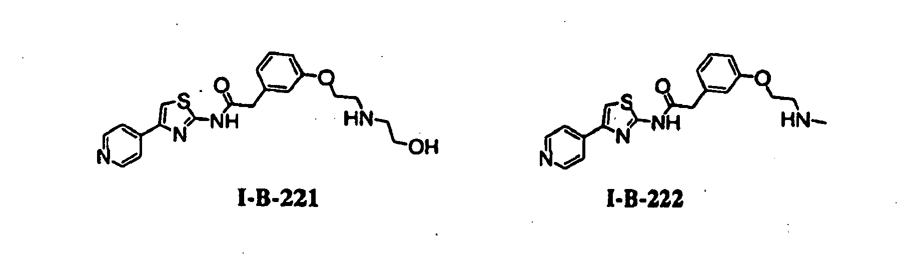

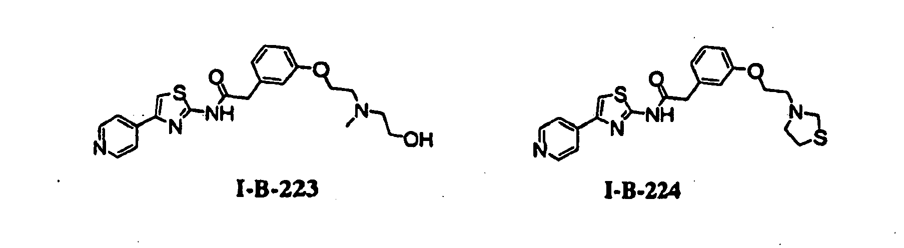

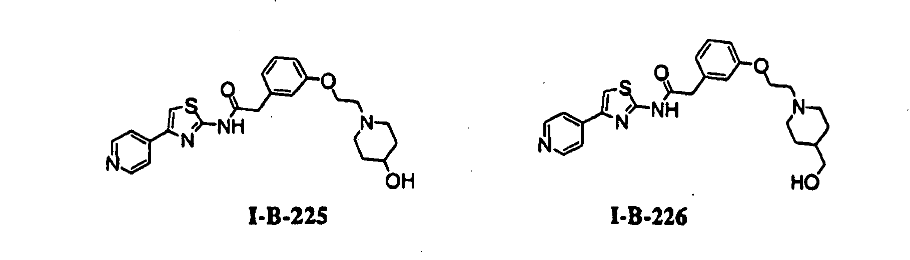

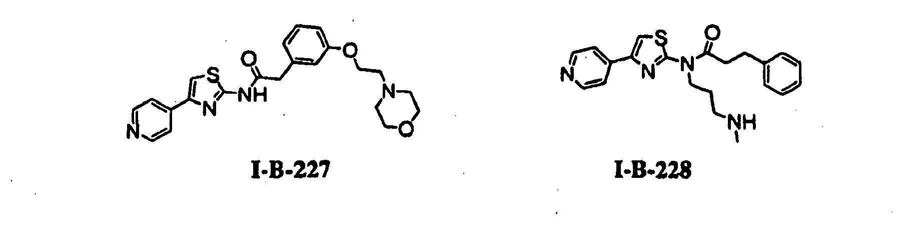

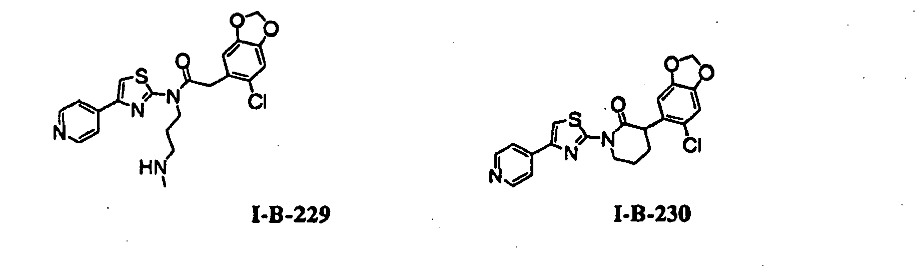

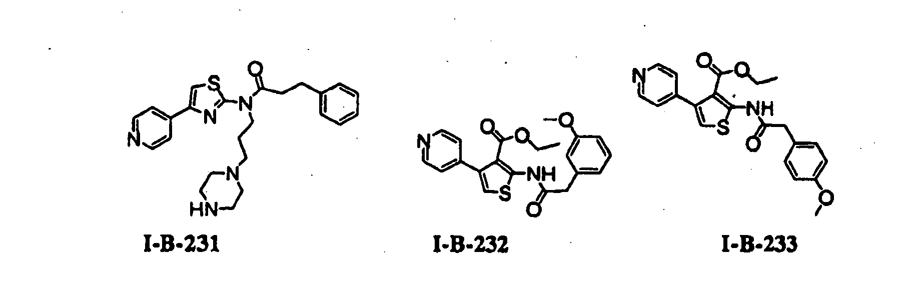

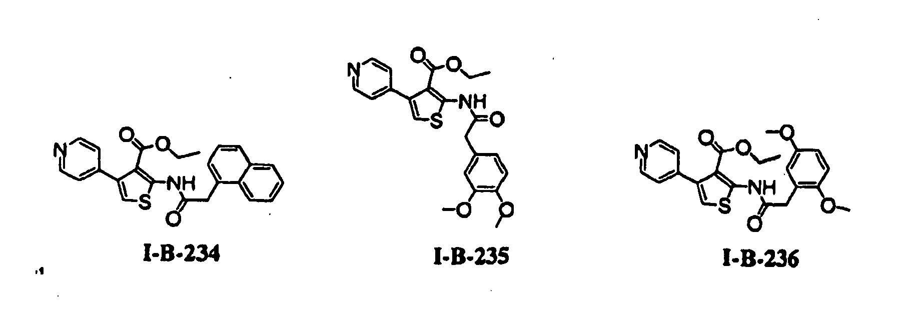

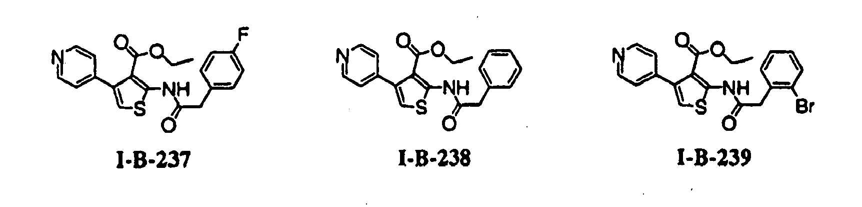

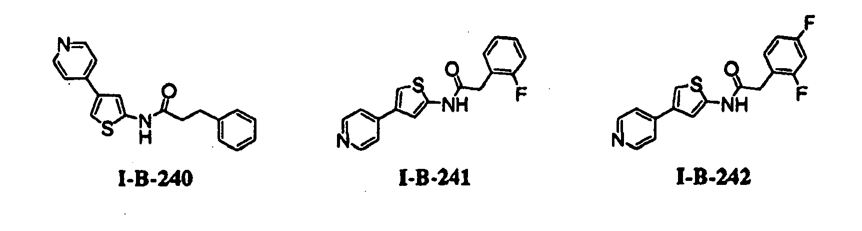

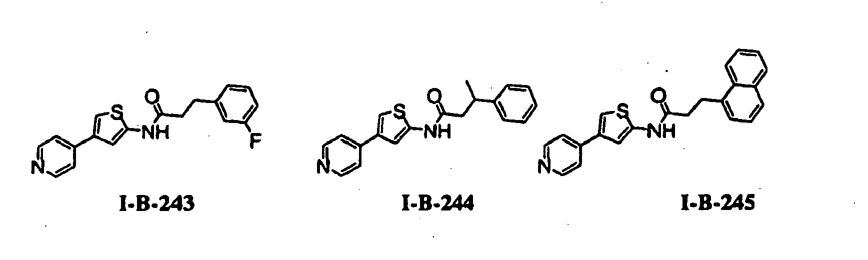

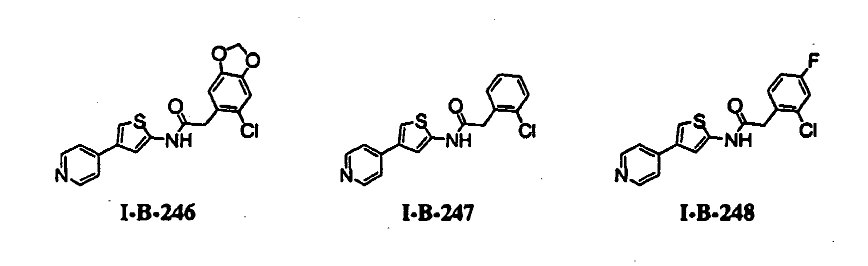

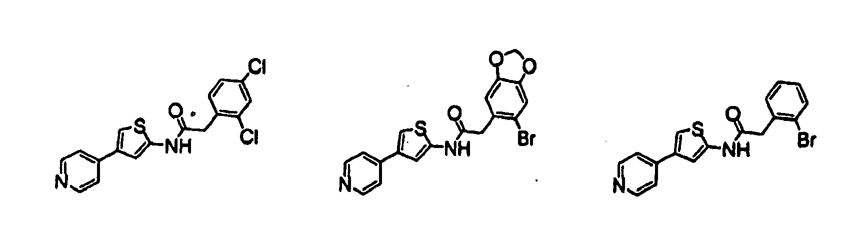

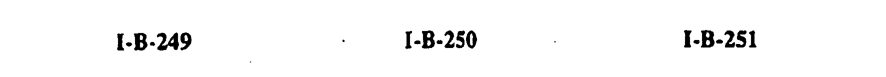

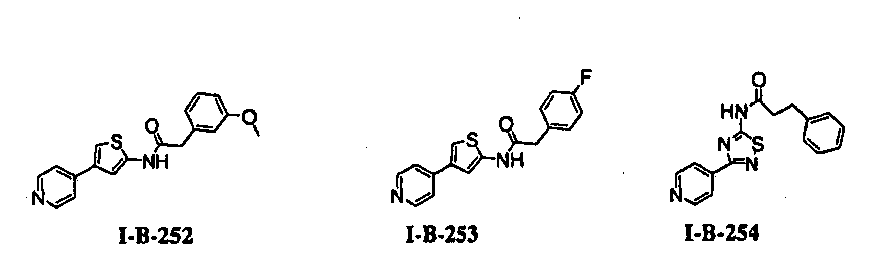

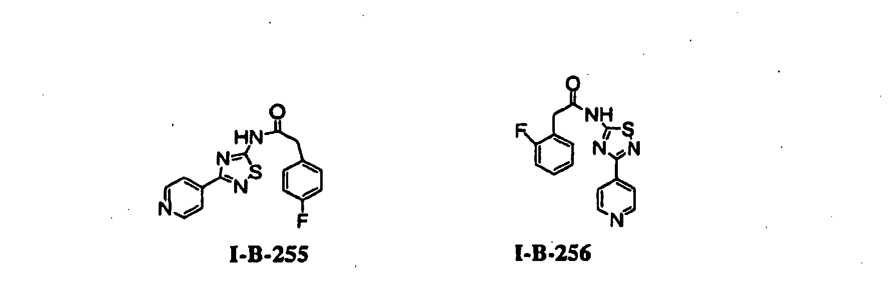

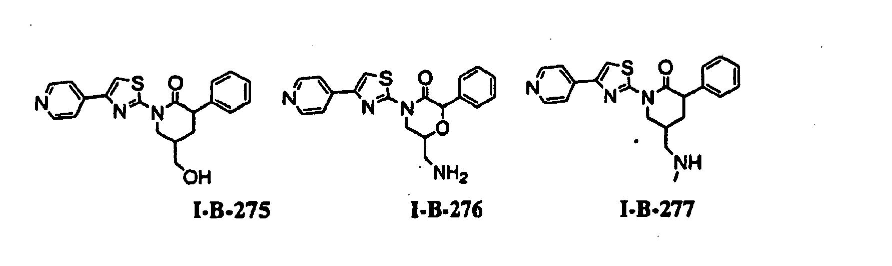

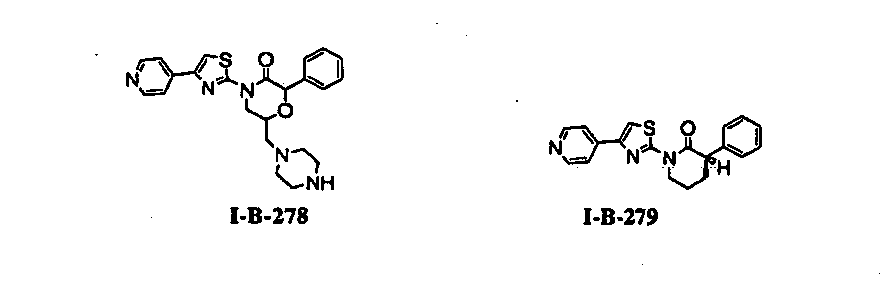

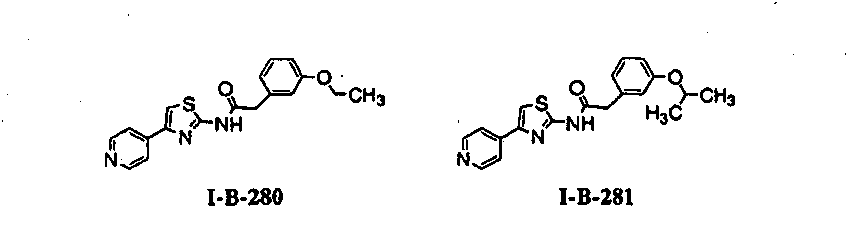

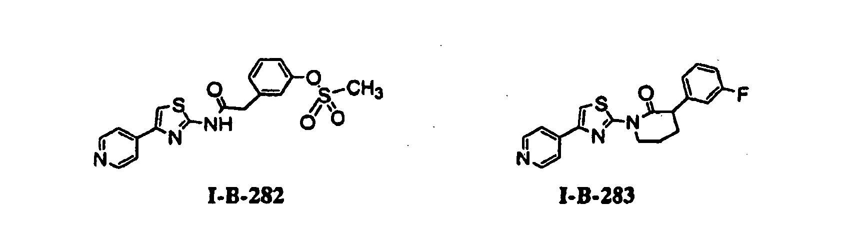

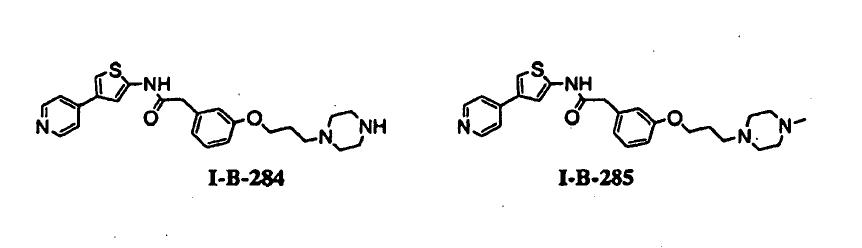

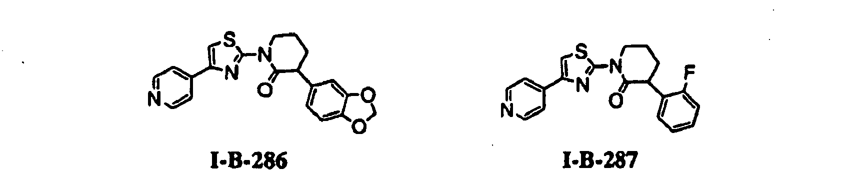

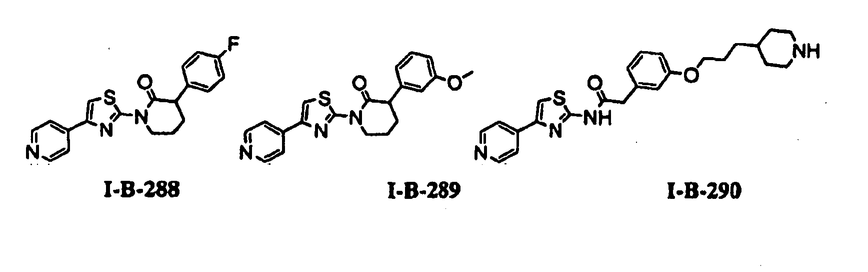

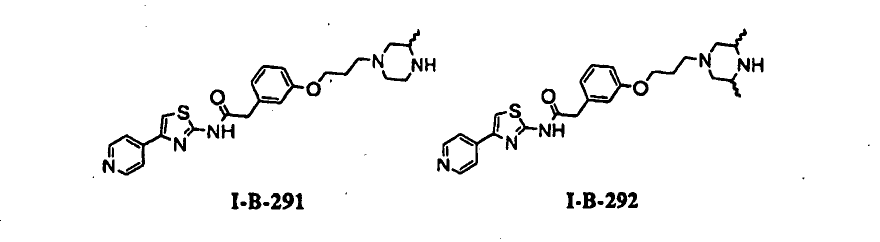

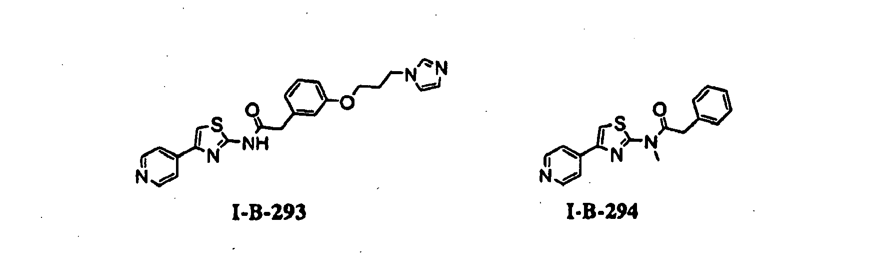

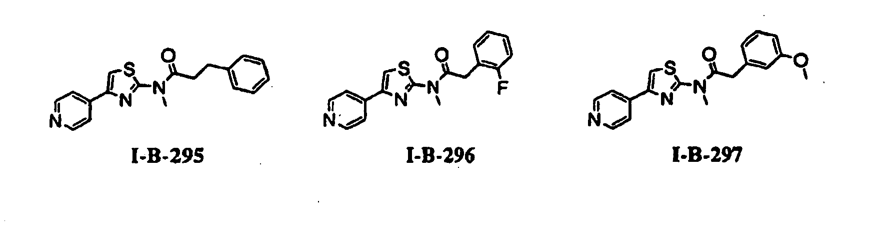

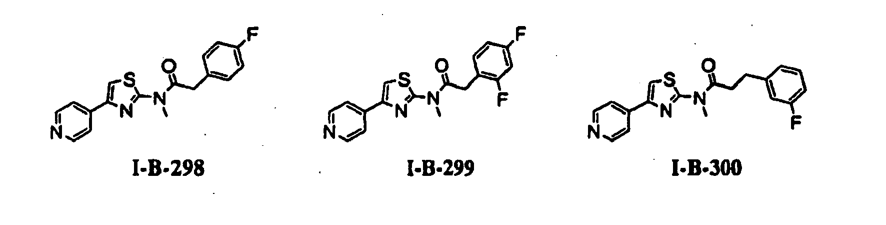

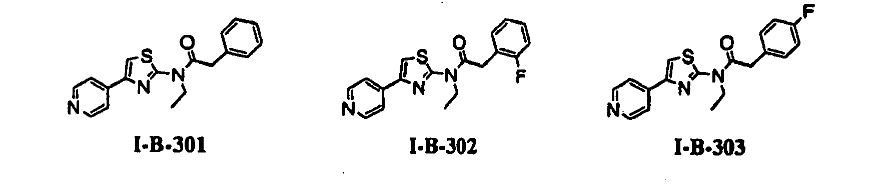

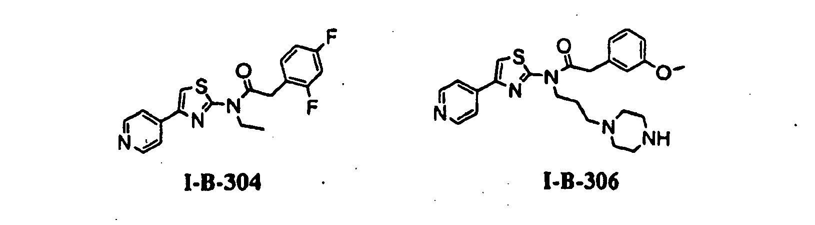

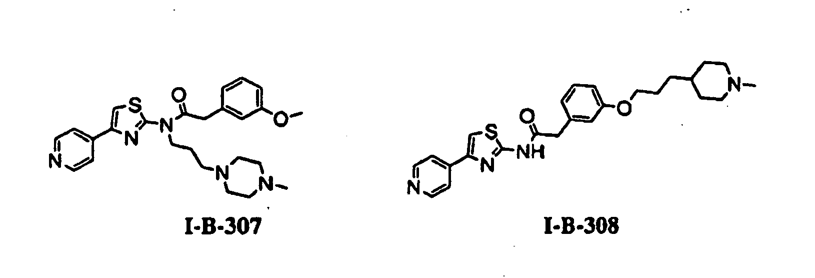

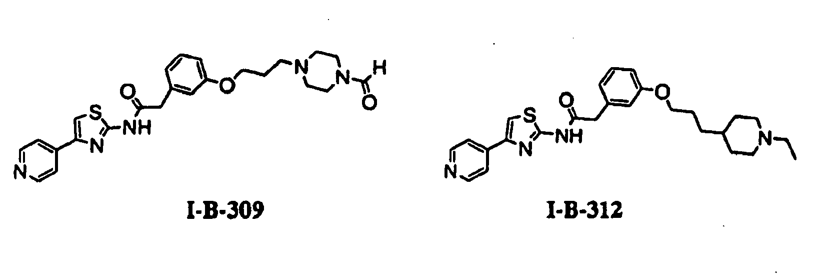

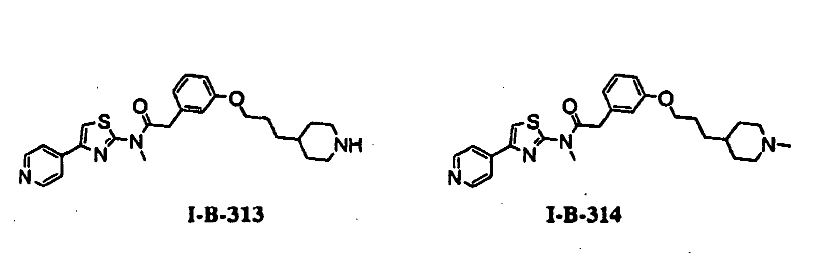

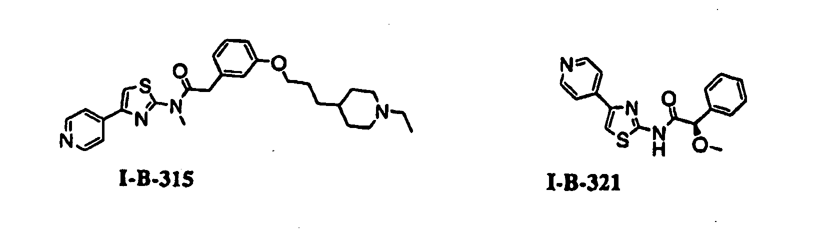

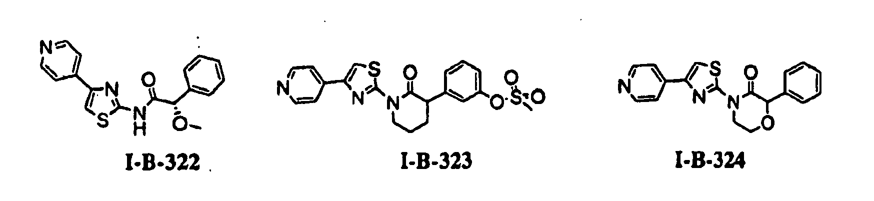

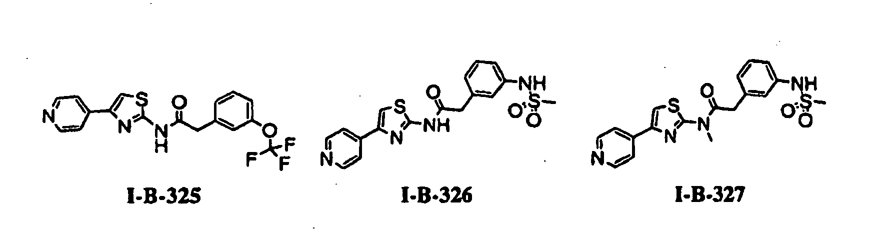

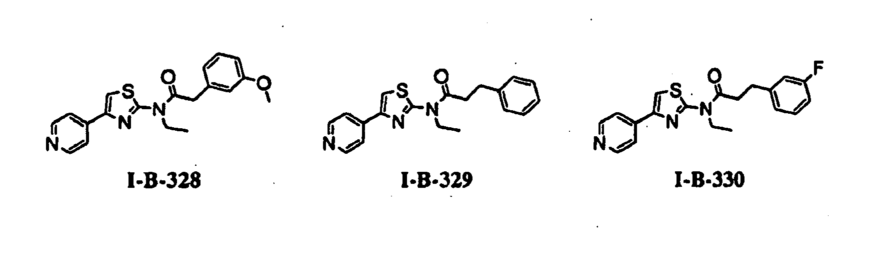

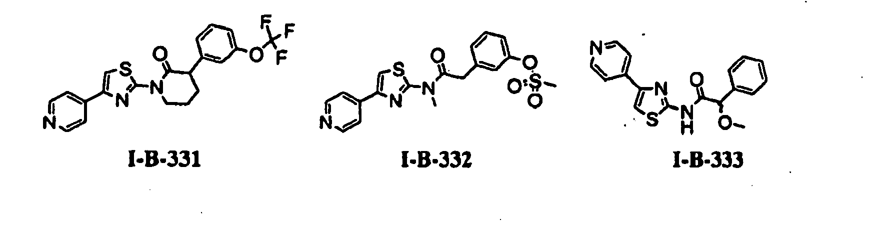

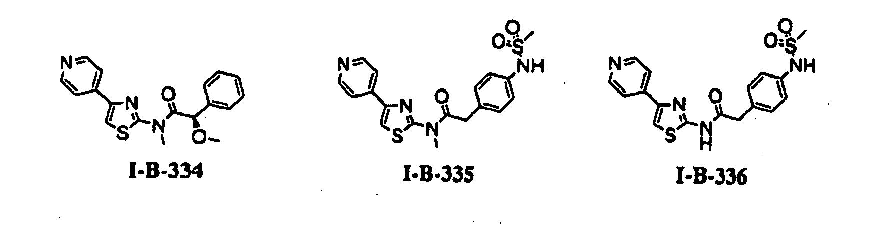

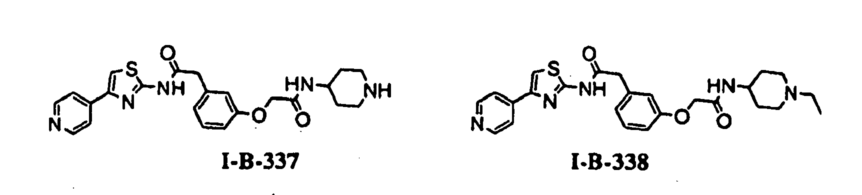

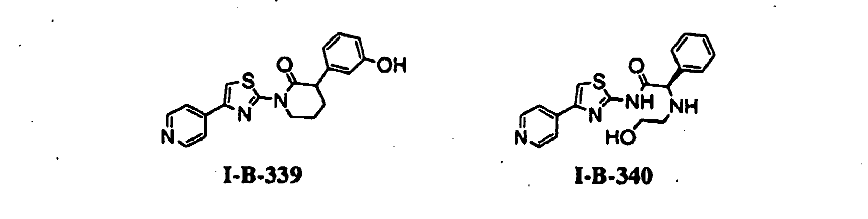

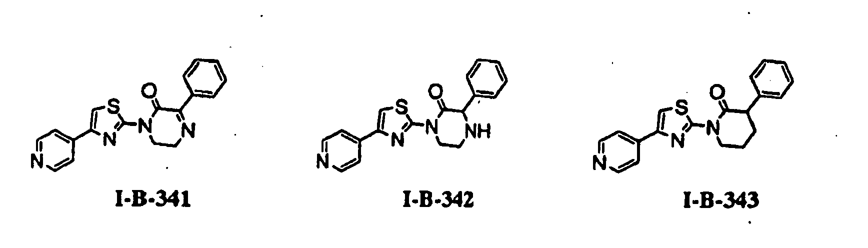

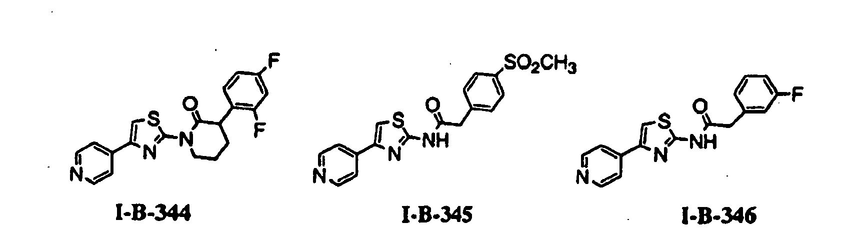

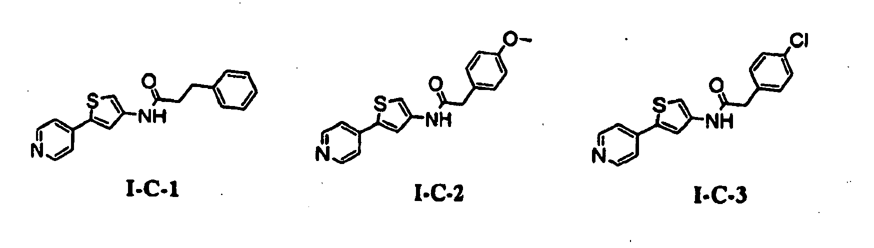

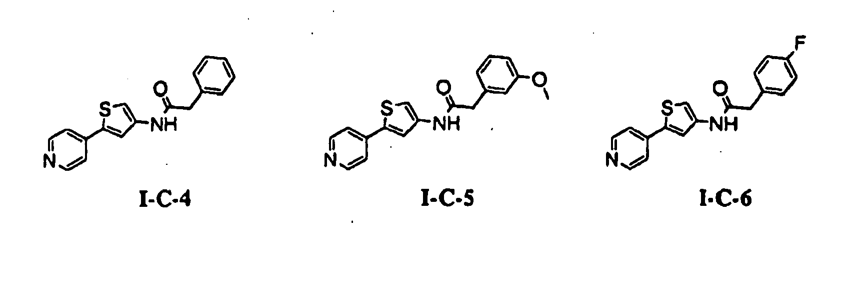

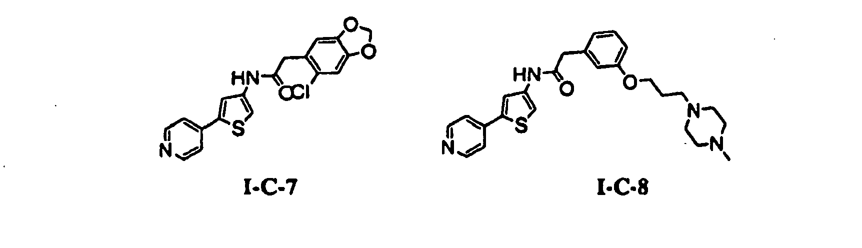

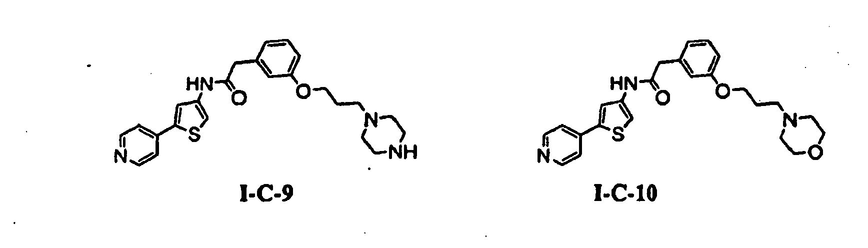

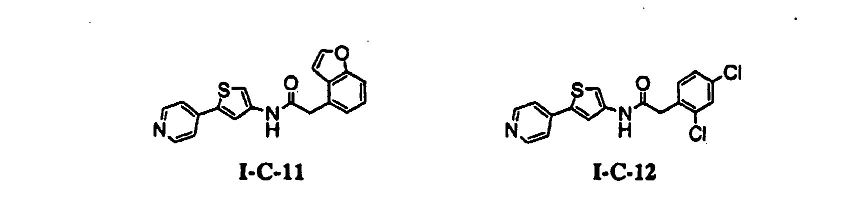

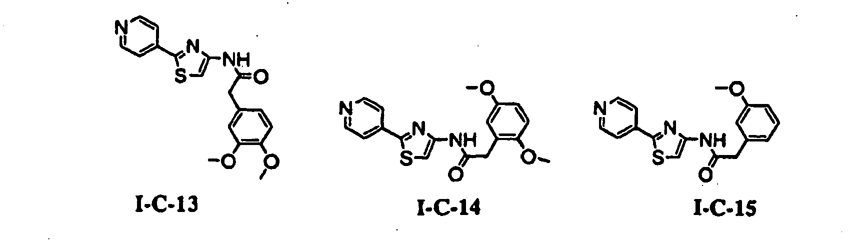

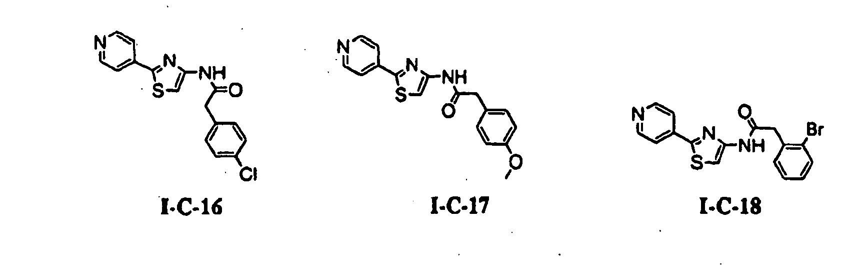

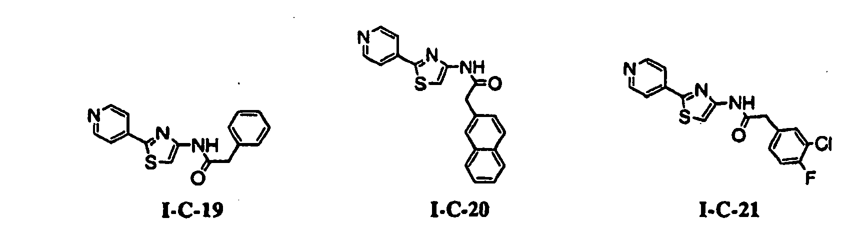

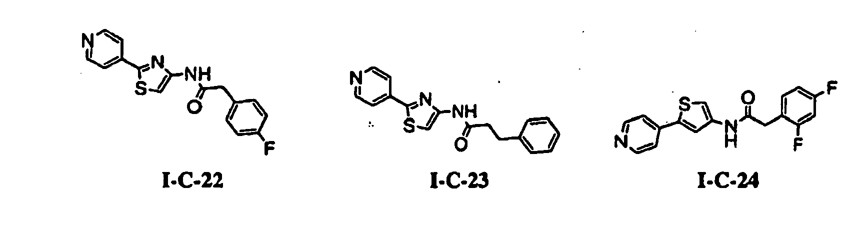

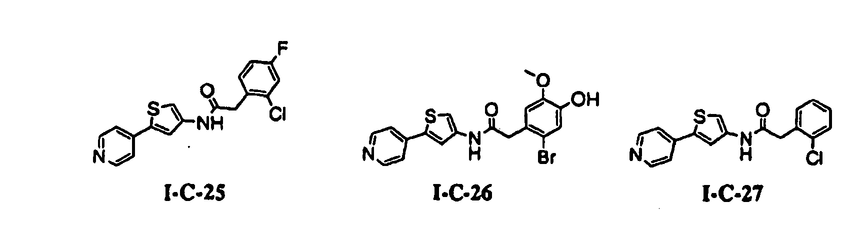

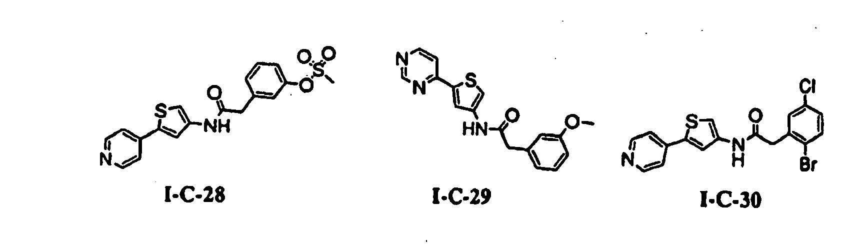

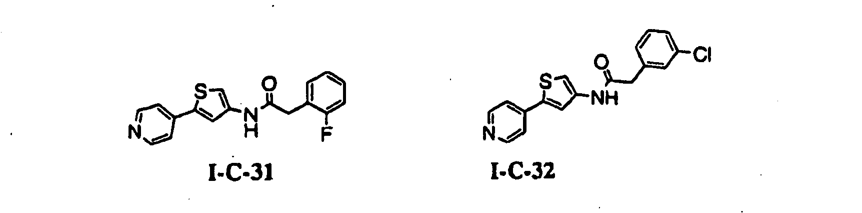

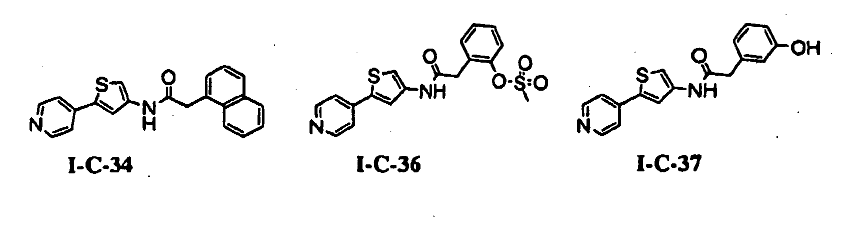

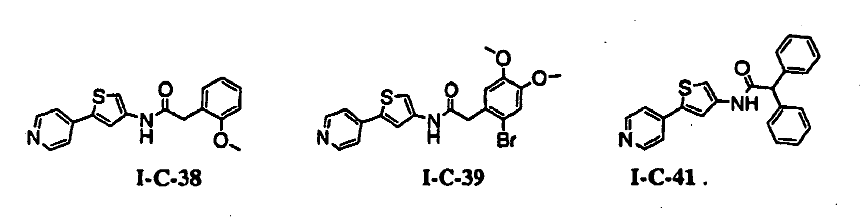

17. Composition comprenant une quantité efficace d'un composé selon l'une quelconque des revendications 1 à 16, et un vecteur, adjuvant ou véhicule acceptable sur le plan pharmaceutique.

18. Composition selon la revendication 17, comprenant en outre un agent thérapeutique choisi parmi un agent chimiothérapeutique ou antiprolifératif, un agent anti-inflammatoire, un agent immunorégulateur ou immunosuppressif, un facteur neurotrophique, un agent pour le traitement d'une maladie cardiovasculaire, un agent pour le traitement de troubles osseux destructifs, un agent pour le traitement d'un trouble hépatique, un agent antiviral, un agent pour le traitement de troubles sanguins, un agent pour le traitement du diabète, ou un agent pour le traitement de troubles immunodéficitaires.

19. Procédé in vitro utilisant un composé de formule : 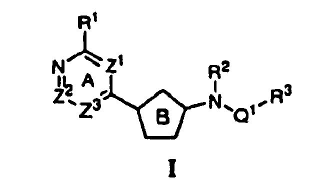 ou un sel pharmaceutiquement acceptable de celui-ci,
dans laquelle 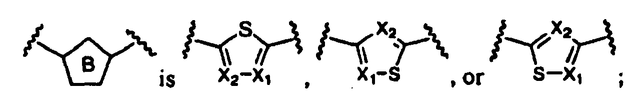 R¹ représente un halogène, CN, NO₂ ou VₘR ;
Z¹ et Z³ représentent chacun indépendamment N ou CR^{Z}, et Z² représente N ou CR¹, à condition que Z¹, Z² et Z³ ne représentent pas simultanément N ;
chaque occurrence de R^{z} représente indépendamment un halogène CN, NO₂ ou UₙR' ;
R² représente UₙR' ;
X¹ et X² représentent chacun indépendamment CR⁴ ou N ;
chaque occurrence de R⁴ représente indépendamment un halogène, CN, NO₂ ou VₘR ;
chaque occurrence de U ou V représente indépendamment une chaîne alkylidène en C_{1 à 6} éventuellement substituée, où jusqu'à deux motifs méthylène de la chaîne sont éventuellement et indépendamment remplacés par -NR-, -S-, -O-, -CS-, -CO₂-, -OCO-, -CO-, -COCO-, -CONR-, -NRCO-, -NRCO₂-, -SO₂NR-, -NRSO₂-, -CONRNR-, -NRCONR-, -OCONR-, -NRNR-, -NRSO₂NR-, -SO-, -SO₂-, -PO-, -PO₂- ou -POR- ;
m et n représentent chacun indépendamment 0 ou 1 ;
chaque occurrence de R représente indépendamment un hydrogène ou un groupe aliphatique en C_{1 à 6} éventuellement substitué ; et chaque occurrence de R' représente indépendamment l'hydrogène ou un groupe aliphatique en C_{1 à 6} éventuellement substitué, un anneau monocyclique à 3 à 8 membres saturé, partiellement insaturé ou complètement insaturé comportant 0 à 3 hétéroatomes indépendamment choisis parmi l'azote, l'oxygène ou le soufre, ou un système d'anneau bicyclique à 8 à 12 membres saturé, partiellement insaturé ou complètement insaturé comportant 0 à 5 hétéroatomes indépendamment choisis parmi l'azote, l'oxygène ou le soufre ; ou R et R', deux occurrences de R, ou deux occurrences de R', sont pris ensemble avec le(s) atome(s) au(x)quel(s) ils sont liés pour former un anneau monocyclique ou bicyclique éventuellement substitué à 3 à 12 membres, saturé, partiellement insaturé ou complètement insaturé comportant 0 à 4 hétéroatomes indépendamment choisis parmi l'azote, l'oxygène ou le soufre ;
Q¹ représente -CO-, -SO₂-, -CONR- ou -SO₂NR- ;
R³ représente Q²-Ar¹,
ou R² et Q¹-R³, pris ensemble avec l'atome d'azote, forment le groupe cyclique : 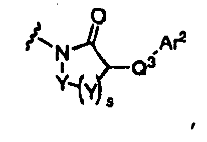 où s représente 1 ou 2, chaque occurrence de Y est indépendamment, comme la valence et la stabilité le permettent, -CO-, -CS-, -SO₂-, -O-, -S-, -NR⁵- ou -C(R⁵)₂-, et R⁵ représente UₙR' ;
Q² et Q³ représentent chacun indépendamment une liaison ou une chaîne alkylidène en C_{1 à 6}, où jusqu'à deux motifs méthylène de la chaîne sont chacun éventuellement et indépendamment remplacés par -NR'-, -S-, -O-, -CS-, -CO₂-, -OCO-, -CO-, -COCO-, -CONR'-, -NR'CO-, -NR'CO₂-, -SO₂NR'-, -NR'SO₂-, -CONR'NR'-, -NR'CONR'-, -OCONR'-, -NR'NR'-, -NR'SO₂NR'-, -SO-, -SO₂-, -PO-, -PO₂- ou -POR'- ; et où n'importe quel atome de carbone dans l'un ou plusieurs motifs méthylène est éventuellement substitué par une ou deux occurrences de R⁶, où chaque occurrence de R⁶ représente indépendamment un halogène, CN, NO₂ ou UₙR', ou deux occurrences de R⁶, ou R' et R⁶, pris ensemble avec les atomes auxquels ils sont liés, forment un anneau cycloalkyle à 3 à 6 membres, hétérocyclyle, aryle ou hétéroaryle, éventuellement substitué ; et
Ar¹ et Ar² représentent chacun indépendamment un anneau monocyclique à 5 à 8 membres saturé, partiellement insaturé ou complètement insaturé comportant 0 à 3 hétéroatomes indépendamment choisis parmi l'azote, l'oxygène ou le soufre, ou un système d'anneau bicyclique à 8 à 12 membres, saturé, partiellement insaturé ou complètement insaturé, comportant 0 à 5 hétéroatomes indépendamment choisis parmi l'azote, l'oxygène ou le soufre ; où Ar¹ et Ar² sont chacun éventuellement substitués avec 0 à 5 occurrences indépendantes de TR⁷ ; où T est une liaison ou représente une chaîne alkylidène en C₁ à C₆ où jusqu'à deux motifs méthylène de T sont éventuellement et indépendamment remplacés par -NR-, -S-, -O-, -CS-, -CO₂-, -OCO-, -CO-, -COCO-, -CONR-, -NRCO-, -NRCO₂-, -SO₂NR-, -NRSO₂-, -CONRNR-, -NRCONR-, -OCONR-, -NRNR-, -NRSO₂NR-, -SO-, -SO₂-, -PO-, -PO₂- ou -POR- ; et chaque occurrence de R⁷ représente indépendamment R', un halogène, NO₂ ou CN ;
ou un sel ou composition de celui-ci acceptable sur le plan pharmaceutique, pour inhiber l'activité de la kinase ROCK, ERK, GSK ou AGC dans un échantillon biologique.

20. Procédé in vitro selon la revendication 19, pour une utilisation dans l'inhibition de l'activité de ROCK.

21. Composé de formule : 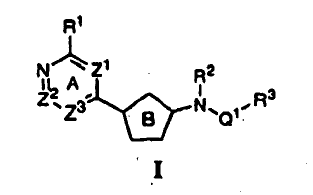 dans laquelle 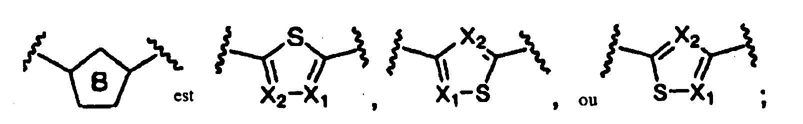 R¹ représente un halogène, CN, NO₂ ou VₘR ;
Z¹ et Z³ représentent chacun indépendamment N ou CR^{z}, et Z² représente N ou CR¹, à condition que Z¹, Z² et Z³ ne représentent pas simultanément N ;
chaque occurrence de R^{z} représente indépendamment un halogène, CN, NO₂ ou UₙR' ;
R² représente UₙR' ;
X¹ et X² représentent chacun indépendamment CR⁴ ou N ;
chaque occurrence de R⁴ représente indépendamment un halogène, CN, NO₂ ou VₘR ;
chaque occurrence de U ou V représente indépendamment une chaîne alkylidène en C_{1 à 6} éventuellement substituée, où jusqu'à deux motifs méthylène de la chaîne sont éventuellement et indépendamment remplacés par -NR-, -S-, -O-, -CS-, -CO₂-, -OCO-, -CO-, -COCO-, -CONR-, -NRCO-, -NRCO₂-, -SO₂NR-, -NRSO₂-, -CONRNR-, -NRCONR-, -OCONR-, -NRNR-, -NRSO₂NR-, -SO-, -SO₂-, -PO-, -PO₂- ou -POR- ;
m et n représentent chacun indépendamment 0 ou 1 ;
chaque occurrence de R représente indépendamment un hydrogène ou un groupe aliphatique en C_{1 à 6} éventuellement substitué ; et chaque occurrence de R' représente indépendamment l'hydrogène ou un groupe aliphatique en C_{1 à 6} éventuellement substitué, un anneau monocyclique à 3 à 8 membres saturé, partiellement insaturé ou complètement insaturé comportant 0 à 3 hétéroatomes indépendamment choisis parmi l'azote, l'oxygène ou le soufre, ou un système d'anneau bicyclique à 8 à 12 membres saturé, partiellement insaturé ou complètement insaturé comportant 0 à 5 hétéroatomes indépendamment choisis parmi l'azote, l'oxygène ou le soufre ; ou R et R', deux occurrences de R, ou deux occurrences de R', sont pris ensemble avec le(s) atome(s) au(x)quel(s) ils sont liés pour former un anneau monocyclique ou bicyclique éventuellement substitué à 3 à 12 membres, saturé, partiellement insaturé ou complètement insaturé comportant 0 à 4 hétéroatomes indépendamment choisis parmi l'azote, l'oxygène ou le soufre ;
Q¹ représente -CO-, -SO₂- ou -SO₂NR- ;
R³ représente Q²-Ar¹,
ou R² et Q¹-R³, pris ensemble avec l'atome d'azote, forment le groupe cyclique : 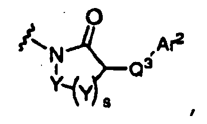 où s représente 1 ou 2, chaque occurrence de Y est indépendamment, comme la valence et la stabilité le permettent, -CO-, -CS-, -SO₂-, -O-, -S-, -NR⁵- ou -C(R⁵)₂-, et R⁵ représente UₙR' ;
Q² et Q³ représentent chacun indépendamment une liaison ou une chaîne alkylidène en C_{1 à 6}, où jusqu'à deux motifs méthylène de la chaîne sont chacun éventuellement et indépendamment remplacés par -S-, -O-, -CS-, -CO₂-, -OCO-, -CO-, -COCO-, -CONR'-, -NR'CO-, -NR'CO₂-, -SO₂NR'-, -NR'SO₂-, -CONR'NR'-, -NR'CONR'-, -OCONR'-, -NR'NR'-, -NR'SO₂NR'-, -SO-, -SO₂-, -PO-, -PO₂- ou -POR'- ; et où n'importe quel atome de carbone dans l'un ou plusieurs motifs méthylène est éventuellement substitué par une ou deux occurrences de R⁶, où chaque occurrence de R⁶ représente indépendamment un halogène, CN, NO₂ ou UₙR', ou deux occurrences de R⁶, ou R' et R⁶, pris ensemble avec les atomes auxquels ils sont liés, forment un anneau cycloalkyle à 3 à 6 membres, hétérocyclyle, aryle ou hétéroaryle, éventuellement substitué ; et
Ar¹ et Ar² représentent chacun indépendamment un anneau monocyclique à 5 à 8 membres, saturé, partiellement insaturé ou complètement insaturé, comportant 0 à 3 hétéroatomes indépendamment choisis parmi l'azote, l'oxygène ou le soufre, ou un système d'anneau bicyclique à 8 à 12 membres, saturé, partiellement insaturé ou complètement insaturé, comportant 0 à 5 hétéroatomes indépendamment choisis parmi l'azote, l'oxygène ou le soufre ; où Ar¹ et Ar² sont chacun éventuellement substitués avec 0 à 5 occurrences indépendantes de TR⁷ ; où T est une liaison ou représente une chaîne alkylidène en C₁ à C₆ où jusqu'à deux motifs méthylène de T sont éventuellement et indépendamment remplacés par -NR-, -S-, -O-, -CS-, -CO₂-, -OCO-, -CO-, -COCO-, -CONR-, -NRCO-, -NRCO₂-, -SO₂NR-, -NRSO₂-, -CONRNR-, -NRCONR-, -OCONR-, -NRNR-, -NRSO₂NR-, -SO-, -SO₂-, -PO-, -PO₂- ou -POR- ; et chaque occurrence de R⁷ représente indépendamment R', un halogène, NO₂ ou CN ;
ou un sel ou composition de celui-ci acceptable sur le plan pharmaceutique pour une utilisation dans le traitement ou l'atténuation de la gravité d'un état maladif ou trouble choisi parmi un trouble prolifératif, un trouble cardiaque, un trouble neurodégénératif, un trouble psychotique, un trouble auto-immun, une condition associée à une transplantation d'organe, un trouble inflammatoire, un trouble immunologiquement médié, une maladie virale, ou un trouble osseux.

22. Composé, sel ou composition de celui-ci acceptable sur le plan pharmaceutique selon la revendication 21, pour une utilisation avec un agent thérapeutique supplémentaire choisi parmi un agent chimiothérapeutique ou antiprolifératif, un agent anti-inflammatoire, un agent immunorégulateur ou immunosuppressif, un facteur neurotrophique, un agent antipsychotique, un agent pour le traitement d'une maladie cardiovasculaire, un agent pour le traitement de troubles osseux destructifs, un agent pour le traitement d'un trouble hépatique, un agent antiviral, un agent pour le traitement de troubles sanguins, un agent pour le traitement du diabète, ou un agent pour le traitement de troubles immunodéficitaires.

23. Composé, sel ou composition de celui-ci acceptable sur le plan pharmaceutique selon la revendication 21, pour une utilisation dans le traitement ou l'atténuation de la gravité d'une maladie, d'un état ou d'un trouble, dans lequel la maladie, l'état ou le trouble est une allergie, de l'asthme, du diabète, la maladie d'Alzheimer, la maladie d'Huntington, la maladie de Parkinson, la démence associée au SIDA, la sclérose latérale amyotrophique (SAL, maladie de Lou Gehrig), la sclérose en plaques (MS), la schizophrénie, l'hypertrophie cardiomyocyte, une ischémie/reperfusion (par exemple, accident cérébrovasculaire), la calvitie, le cancer, l'hépatomégalie, une maladie cardiovasculaire y compris la cardiomégalie, la mucoviscidose, une maladie virale, des maladies auto-immunes, l'athérosclérose, une resténose, le psoriasis, une inflammation, l'hypertension, une angine de poitrine, une contraction cérébrovasculaire, un trouble de la circulation périphérique, une naissance prématurée, l'artériosclérose, un angiospasme (angiospasme cérébral, angiospasme coronaire), une rétinopathie, la dysfonction érectile (DE), le SIDA, l'ostéoporose, la maladie de Crohn et la colite, une excroissance de névrite ou la maladie de Raynaud ; en particulier l'athérosclérose, l'hypertension, la dysfonction érectile (DE), une ischémie/reperfusion (par exemple, accident cérébrovasculaire), ou un angiospasme (angiospasme cérébral et un angiospasme coronaire).

24. Utilisation d'un composé selon l'une quelconque des revendications 1 à 16 dans la fabrication d'un médicament pour le traitement ou l'atténuation de la gravité d'une maladie, d'un trouble ou d'un état choisi parmi un trouble prolifératif, un trouble cardiaque, un trouble neurodégénératif, un trouble psychotique, un trouble auto-immun, une condition associée à une transplantation d'organe, un trouble inflammatoire, un trouble immunologiquement médié, une maladie virale ou un trouble osseux ; tel qu'une allergie, de l'asthme, du diabète, la maladie d'Alzheimer, la maladie d'Huntington, la maladie de Parkinson, la démence associée au SIDA, la sclérose latérale amyotrophique (SAL, maladie de Lou Gehrig), la sclérose en plaques (MS), la schizophrénie, l'hypertrophie cardiomyocyte, une ischémie/reperfusion (par exemple, accident cérébrovasculaire), la calvitie, le cancer, l'hépatomégalie, une maladie cardiovasculaire y compris la cardiomégalie, la mucoviscidose, une maladie virale, des maladies auto-immunes, l'athérosclérose, une resténose, le psoriasis, une inflammation, l'hypertension, une angine de poitrine, une contraction cérébrovasculaire, un trouble de la circulation périphérique, une naissance prématurée, l'artériosclérose, un angiospasme (angiospasme cérébral, angiospasme coronaire), une rétinopathie, la dysfonction érectile (DE), le SIDA, l'ostéoporose, la maladie de Crohn et la colite, une excroissance de névrite ou la maladie de Raynaud ;
en particulier, l'athérosclérose, l'hypertension, la dysfonction érectile (DE), une ischémie/reperfusion (par exemple, accident cérébrovasculaire), ou un angiospasme (angiospasme cérébral et un angiospasme coronaire).
